# EUROPEAN PATENT APPLICATION

(11) **EP 4 509 498 A1**
(43) Date of publication of application: **19.02.2025**
(21) Application number: 23788251.9
(22) Date of filing: 06.04.2023
(51) Int. Cl.: C07D 307/77, C07D 307/92, C07D 405/12, H10K 50/00, H10K 50/15

(54) **COMPOUND, MATERIAL FOR ORGANIC ELECTROLUMINESCENT ELEMENTS, ORGANIC ELECTROLUMINESCENT ELEMENT, AND ELECTRONIC DEVICE**

(30) Priority: 12.04.2022 JP 2022065500
(71) Applicant: Idemitsu Kosan Co.,Ltd., Tokyo 100-8321 (JP)
(72) Inventor: TAKAHASHI Yusuke, Tokyo 100-8321 (JP); ITOI Hiroaki, Tokyo 100-8321 (JP); TANAKA Shota, Tokyo 100-8321 (JP); FUKAMI Takuto, Tokyo 100-8321 (JP); SAWATO Tsukasa, Tokyo 100-8321 (JP)
(74) Representative: Hoffmann Eitle
(86) International application number: PCT/JP2023/014165
(87) International publication number: WO 2023/199832

(57) **Abstract**

A compound represented by the following formula (1): 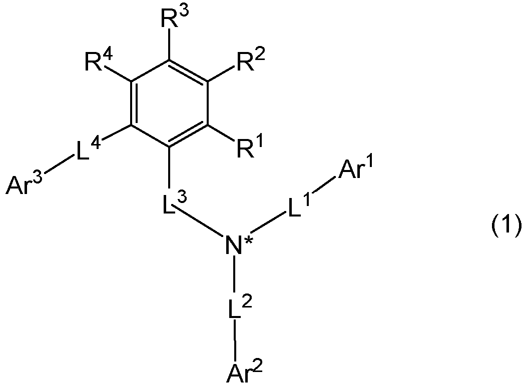 (each sign in the formula (1) is as defined in the description), an organic electroluminescent device containing the compound, and an electronic instrument including the organic electroluminescent device.

## Description

### Technical Field

The present invention relates to a compound, a material for organic electroluminescent devices, an organic electroluminescent device, and an electronic instrument including the organic electroluminescent device.

### Background Art

In general, an organic electroluminescent device (hereinafter sometimes referred to as "organic EL device") is composed of an anode, a cathode, and an organic layer interposed between the anode and the cathode. In application of a voltage between the two electrodes, electrons from the cathode side and holes from the anode side are injected into a light emitting region, and the injected electrons and holes are recombined in the light emitting region to generate an excited state, which then returns to the ground state to emit light. Accordingly, it is important, for providing a high-performance organic EL device, to develop a material that efficiently transports electrons or holes into a light emitting region, promotes recombination of the electrons and holes and efficiently issue excitons, and to find combinations thereof.

PTLs 1 to 4 disclose compounds for use as a material for organic electroluminescent devices.

### Citation List

### Patent Literature

PTL 1: WO 2022/009999 A
PTL 2: WO 2019/185061 A
PTL 3: CN 112430225 A
PTL 4: CN 108689972 A

### Summary of Invention

### Technical Problem

Although many compounds for organic EL devices have conventionally been reported, it is still desired to further enhance performance of an organic EL device.

The present invention has been made for solving the above problem, and has an object to provide a compound that further improves performance of an organic EL device, an organic EL device having further improved device performance by containing a specific compound, and an electronic instrument including such an organic EL device.

### Solution to Problem

As a result of intensive and extensive studies about the performance of organic EL devices containing the compounds disclosed in PTLs 1 to 4, the present inventors have found that an organic EL device containing a compound represented by the following formula (1) has further improved performance.

In an aspect, the present invention provides a compound represented by the following formula (1): 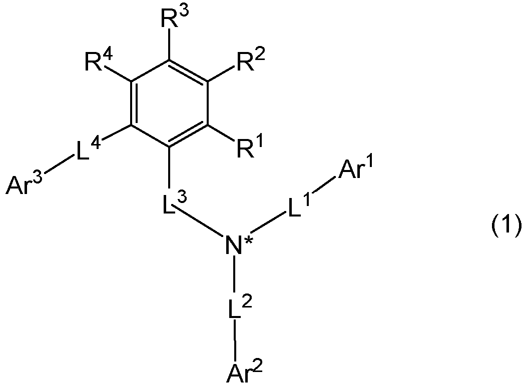 wherein
N* is a central nitrogen atom,
R¹ to R⁴ are each independently a hydrogen atom, an unsubstituted alkyl group having 1 to 6 carbon atoms, or an unsubstituted aryl group having 6 to 12 ring carbon atoms, adjacent two selected from R¹ to R⁴ are not bonded to each other, thus forming no ring,
L¹ to L⁴ are each independently a single bond, an unsubstituted arylene group having 6 to 30 ring carbon atoms or an unsubstituted divalent heterocyclic group having 5 to 30 ring atoms,
Ar¹ and Ar² are each independently a substituted or unsubstituted aryl group having 6 to 30 ring carbon atoms constituted only of 6-membered rings, a substituted or unsubstituted heterocyclic group having 5 to 30 ring atoms, or a group represented by the following formula (a): 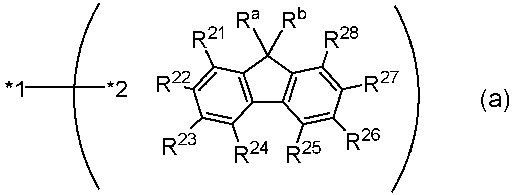 wherein
   * 1 is a bonding site to L¹ or L²,
   R^{a} and R^{b} are each independently a hydrogen atom, a substituted or unsubstituted alkyl group having 1 to 30 carbon atoms, a substituted or unsubstituted aryl group having 6 to 30 ring carbon atoms, or a substituted or unsubstituted heterocyclic group having 5 to 30 ring atoms,
   R^{a} and R^{b} may be bonded to each other to form a substituted or unsubstituted ring, or may not be bonded to each other, thus forming no ring,
   one selected from R²¹ to R²⁸ is a single bond bonded to *2, and R²¹ to R²⁸ that are not a single bond bonded to *2 are each independently a hydrogen atom, a substituted or unsubstituted alkyl group having 1 to 6 carbon atoms, a substituted or unsubstituted aryl group having 6 to 12 ring carbon atoms, or a substituted or unsubstituted heterocyclic group having 5 to 13 ring atoms,
   adjacent two selected from R²¹ to R²⁸ that are not the single bond may be bonded to each other to form one or more unsubstituted benzene rings, or may not be bonded to each other, thus forming no ring,
   provided that when R^{a} and R^{b} are bonded to each other to form a substituted or unsubstituted spirofluorene ring with a carbon atom at site 9 of a fluorene skeleton, one selected from R²² to R²⁷ is a single bond bonded to *2,
   when all of L¹ to L⁴ are single bonds, Ar¹ and Ar² are groups represented by the formula (a), and R²² or R²⁷ is a single bond bonded to *2, at least one selected from two R^{a}'s and two R^{b}'s is a substituted or unsubstituted aryl group having 6 to 30 ring carbon atoms;
   Ar³ is a group represented by the following formula (x): 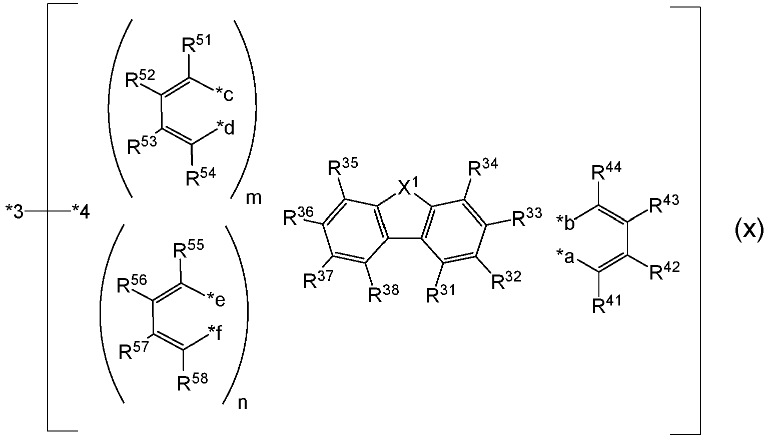 wherein
      *3 is a bonding site to L⁴,
      R³¹ to R³⁸, R⁴¹ to R⁴⁴, and R⁵¹ to R⁵⁸ are each independently a hydrogen atom, a halogen atom, a cyano group, a nitro group, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by - Si(R₉₀₁)(R₉₀₂)(R₉₀₃), a group represented by -O-(R₉₀₄), a group represented by -S-(R₉₀₅), a group represented by -N(R₉₀₆)(R₉₀₇), a substituted or unsubstituted aryl group having 6 to 50 ring carbon atoms, or a substituted or unsubstituted heterocyclic group having 5 to 50 ring atoms,
      R₉₀₁ to R₉₀₇ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms,
      in the case where two or more groups represented by R₉₀₁ exist, the two or more groups each represented by R₉₀₁ are the same as or different from each other,
      in the case where two or more groups represented by R₉₀₂ exist, the two or more groups each represented by R₉₀₂ are the same as or different from each other,
      in the case where two or more groups represented by R₉₀₃ exist, the two or more groups each represented by R₉₀₃ are the same as or different from each other,
      in the case where two or more groups represented by R₉₀₄ exist, the two or more groups each represented by R₉₀₄ are the same as or different from each other,
      in the case where two or more groups represented by R₉₀₅ exist, the two or more groups each represented by R₉₀₅ are the same as or different from each other,
      in the case where two or more groups represented by R₉₀₆ exist, the two or more groups each represented by R₉₀₆ are the same as or different from each other,
      in the case where two or more groups represented by R₉₀₇ exist, the two or more groups each represented by R₉₀₇ are the same as or different from each other,
      m is 0 or 1, and n is 0 or 1,
      when m and n are 0, one of R³¹ and R³², R³² and R³³, or R³³ and R³⁴ is a single bond bonded to *a, and the other is a single bond bonded to *b,
      one selected from R³¹ to R³⁸ that are not a single bond bonded to *a and *b, and R⁴¹ to R⁴⁴ is a single bond bonded to *4,
      when m is 1 and n is 0, one of R³¹ and R³², R³² and R³³, or R³³ and R³⁴ is a single bond bonded to *a, and the other is a single bond bonded to *b,
      one of R³⁵ and R³⁶, R³⁶ and R³⁷, or R³⁷ and R³⁸ is a single bond bonded to *c, and the other is a single bond bonded to *d,
      one selected from R³¹ to R³⁴ that are not a single bond bonded to *a and *b, R³⁵ to R³⁸ that are not a single bond bonded to *c and *d, R⁴¹ to R⁴⁴, and R⁵¹ to R⁵⁴ is a single bond bonded to *4,
      when m is 0 and n is 1, one of R³¹ and R³², R³² and R³³, or R³³ and R³⁴ is a single bond bonded to *a, and the other is a single bond bonded to *b,
      one of R³⁵ and R³⁶, R³⁶ and R³⁷, or R³⁷ and R³⁸ is a single bond bonded to *e, and the other is a single bond bonded to *f,
      one selected from R³¹ to R³⁴ that are not a single bond bonded to *a and *b, R³⁵ to R³⁸ that are not a single bond bonded to *e and *f, R⁴¹ to R⁴⁴, and R⁵⁵ to R⁵⁸ is a single bond bonded to *4,
      when m is 1 and n is 1, one of R³¹ and R³², R³² and R³³, or R³³ and R³⁴ is a single bond bonded to *a, and the other is a single bond bonded to *b,
      one in any one group of three groups, R³⁵ and R³⁶, R³⁶ and R³⁷, and R³⁷ and R³⁸, is a single bond bonded to *c, and the other is a single bond bonded to *d,
      one in any one group of the remaining two groups of R³⁵ and R³⁶, R³⁶ and R³⁷, or R³⁷ and R³⁸, is a single bond bonded to *e, and the other is a single bond bonded to *f,
      one selected from R³¹ to R³⁴ that are not a single bond bonded to *a and *b, R³⁵ to R³⁸ that are not a single bond bonded to *c to *f, R⁴¹ to R⁴⁴, and R⁵¹ to R⁵⁸ is a single bond bonded to *4,
      provided that when L³ and L⁴ are a single bond, m and n are 0, R³³ is a single bond bonded to *a, and R³⁴ is a single bond bonded to *b, or R³⁴ is a single bond bonded to *a and R³³ is a single bond bonded to *b, one selected from R³¹, R³², R³⁵ to R³⁷, and R⁴¹ to R⁴⁴ is a single bond bonded to *4,
      when L³ and L⁴ are a single bond, m and n are 0, R³¹ is a single bond bonded to *a, and R³² is a single bond bonded to *b, one selected from R³³ to R³⁸ and R⁴² to R⁴⁴ is a single bond bonded to *4,
      when L³ and L⁴ are a single bond, m and n are 0, R³² is a single bond bonded to *a, and R³¹ is a single bond bonded to *b, one selected from R³³ to R³⁸ and R⁴¹ to R⁴³ is a single bond bonded to *4;
      X¹ is an oxygen atom or a sulfur atom.

In another aspect, the present invention provides a material for organic EL devices, the material containing the compound represented by the formula (1).

In still another aspect, the present invention provides an organic electroluminescent device including a cathode, an anode, and an organic layer disposed between the cathode and the anode, the organic layer including a light emitting layer, at least one layer of the organic layer containing the compound represented by the formula (1).

In still yet another aspect, the present invention provides an electronic instrument including the organic electroluminescent device.

### Advantageous Effects of Invention

The organic EL device containing the compound represented by the formula (1) shows an improved device performance.

### Brief Description of Drawings

Fig. 1 is a schematic diagram illustrating an example of the layer configuration of the organic EL device according to an aspect of the present invention.
Fig. 2 is a schematic diagram illustrating another example of the layer configuration of the organic EL device according to an aspect of the present invention.
Fig. 3 is a schematic diagram illustrating still another example of the layer configuration of the organic EL device according to an aspect of the present invention.

### Description of Embodiments

### [Definitions]

In the description herein, the hydrogen atom encompasses isotopes thereof having different numbers of neutrons, i.e., protium, deuterium, and tritium.

In the description herein, the bonding site where the symbol, such as "R", or "D" representing a deuterium atom is not shown is assumed to have a hydrogen atom, i.e., a protium atom, a deuterium atom, or a tritium atom, bonded thereto.

In the description herein, the number of ring carbon atoms shows the number of carbon atoms among the atoms constituting the ring itself of a compound having a structure including atoms bonded to form a ring (such as a monocyclic compound, a condensed ring compound, a bridged compound, a carbocyclic compound, and a heterocyclic compound). In the case where the ring is substituted by a substituent, the carbon atom contained in the substituent is not included in the number of ring carbon atoms. The same definition is applied to the "number of ring carbon atoms" described hereinafter unless otherwise indicated. For example, a benzene ring has 6 ring carbon atoms, a naphthalene ring has 10 ring carbon atoms, a pyridine ring has 5 ring carbon atoms, and a furan ring has 4 ring carbon atoms. For example, 9,9-diphenylfluorenyl group has 13 ring carbon atoms, and 9,9'-spirobifluorenyl group has 25 ring carbon atoms.

In the case where a benzene ring has, for example, an alkyl group substituted thereon as a substituent, the number of carbon atoms of the alkyl group is not included in the number of ring carbon atoms of the benzene ring. Accordingly, a benzene ring having an alkyl group substituted thereon has 6 ring carbon atoms. In the case where a naphthalene ring has, for example, an alkyl group substituted thereon as a substituent, the number of carbon atoms of the alkyl group is not included in the number of ring carbon atoms of the naphthalene ring. Accordingly, a naphthalene ring having an alkyl group substituted thereon has 10 ring carbon atoms.

In the description herein, the number of ring atoms shows the number of atoms constituting the ring itself of a compound having a structure including atoms bonded to form a ring (such as a monocyclic ring, a condensed ring, and a set of rings) (such as a monocyclic compound, a condensed ring compound, a bridged compound, a carbocyclic compound, and a heterocyclic compound). The atom that does not constitute the ring (such as a hydrogen atom terminating the bond of the atom constituting the ring) and, in the case where the ring is substituted by a substituent, the atom contained in the substituent are not included in the number of ring atoms. The same definition is applied to the "number of ring atoms" described hereinafter unless otherwise indicated. For example, a pyridine ring has 6 ring atoms, a quinazoline ring has 10 ring atoms, and a furan ring has 5 ring atoms. For example, the number of hydrogen atoms bonded to a pyridine ring or atoms constituting a substituent is not included in the number of ring atoms of the pyridine ring. Accordingly, a pyridine ring having a hydrogen atom or a substituent bonded thereto has 6 ring atoms. For example, the number of hydrogen atoms bonded to carbon atoms of a quinazoline ring or atoms constituting a substituent is not included in the number of ring atoms of the quinazoline ring. Accordingly, a quinazoline ring having a hydrogen atom or a substituent bonded thereto has 10 ring atoms.

In the description herein, the expression "having XX to YY carbon atoms" in the expression "substituted or unsubstituted ZZ group having XX to YY carbon atoms" means the number of carbon atoms of the unsubstituted ZZ group, and, in the case where the ZZ group is substituted, the number of carbon atoms of the substituent is not included. Herein, "YY" is larger than "XX", "XX" represents an integer of 1 or more, and "YY" represents an integer of 2 or more.

In the description herein, the expression "having XX to YY atoms" in the expression "substituted or unsubstituted ZZ group having XX to YY atoms" means the number of atoms of the unsubstituted ZZ group, and, in the case where the ZZ group is substituted, the number of atoms of the substituent is not included. Herein, "YY" is larger than "XX", "XX" represents an integer of 1 or more, and "YY" represents an integer of 2 or more.

In the description herein, an unsubstituted ZZ group means the case where the "substituted or unsubstituted ZZ group" is an "unsubstituted ZZ group", and a substituted ZZ group means the case where the "substituted or unsubstituted ZZ group" is a "substituted ZZ group".

In the description herein, the expression "unsubstituted" in the expression "substituted or unsubstituted ZZ group" means that hydrogen atoms in the ZZ group are not substituted by a substituent. The hydrogen atoms in the "unsubstituted ZZ group" each are a protium atom, a deuterium atom, or a tritium atom.

In the description herein, the expression "substituted" in the expression "substituted or unsubstituted ZZ group" means that one or more hydrogen atom in the ZZ group is substituted by a substituent. The expression "substituted" in the expression "BB group substituted by an AA group" similarly means that one or more hydrogen atom in the BB group is substituted by the AA group.

### Substituents in Description

The substituents described in the description herein will be explained.

In the description herein, the number of ring carbon atoms of the "unsubstituted aryl group" is 6 to 50, preferably 6 to 30, and more preferably 6 to 18, unless otherwise indicated in the description.

In the description herein, the number of ring atoms of the "unsubstituted heterocyclic group" is 5 to 50, preferably 5 to 30, and more preferably 5 to 18, unless otherwise indicated in the description.

In the description herein, the number of carbon atoms of the "unsubstituted alkyl group" is 1 to 50, preferably 1 to 20, and more preferably 1 to 6, unless otherwise indicated in the description.

In the description herein, the number of carbon atoms of the "unsubstituted alkenyl group" is 2 to 50, preferably 2 to 20, and more preferably 2 to 6, unless otherwise indicated in the description.

In the description herein, the number of carbon atoms of the "unsubstituted alkynyl group" is 2 to 50, preferably 2 to 20, and more preferably 2 to 6, unless otherwise indicated in the description.

In the description herein, the number of ring carbon atoms of the "unsubstituted cycloalkyl group" is 3 to 50, preferably 3 to 20, and more preferably 3 to 6, unless otherwise indicated in the description.

In the description herein, the number of ring carbon atoms of the "unsubstituted arylene group" is 6 to 50, preferably 6 to 30, and more preferably 6 to 18, unless otherwise indicated in the description.

In the description herein, the number of ring atoms of the "unsubstituted divalent heterocyclic group" is 5 to 50, preferably 5 to 30, and more preferably 5 to 18, unless otherwise indicated in the description.

In the description herein, the number of carbon atoms of the "unsubstituted alkylene group" is 1 to 50, preferably 1 to 20, and more preferably 1 to 6, unless otherwise indicated in the description.

### Substituted or Unsubstituted Aryl Group

In the description herein, specific examples (set of specific examples G1) of the "substituted or unsubstituted aryl group" include the unsubstituted aryl groups (set of specific examples G1A) and the substituted aryl groups (set of specific examples G1B) shown below. (Herein, the unsubstituted aryl group means the case where the "substituted or unsubstituted aryl group" is an "unsubstituted aryl group", and the substituted aryl group means the case where the "substituted or unsubstituted aryl group" is a "substituted aryl group".) In the description herein, the simple expression "aryl group" encompasses both the "unsubstituted aryl group" and the "substituted aryl group".

The "substituted aryl group" means a group formed by substituting one or more hydrogen atom of the "unsubstituted aryl group" by a substituent. Examples of the "substituted aryl group" include groups formed by one or more hydrogen atom of each of the "unsubstituted aryl groups" in the set of specific examples G1A by a substituent, and the examples of the substituted aryl groups in the set of specific examples G1B. The examples of the "unsubstituted aryl group" and the examples of the "substituted aryl group" enumerated herein are mere examples, and the "substituted aryl group" in the description herein encompasses groups formed by substituting a hydrogen atom bonded to the carbon atom of the aryl group itself of each of the "substituted aryl groups" in the set of specific examples G1B by a substituent, and groups formed by substituting a hydrogen atom of the substituent of each of the "substituted aryl groups" in the set of specific examples G1B by a substituent.

### Unsubstituted Aryl Group (Set of Specific Examples G1A):

a phenyl group,
a p-biphenyl group,
a m-biphenyl group,
an o-biphenyl group,
a p-terphenyl-4-yl group,
a p-terphenyl-3-yl group,
a p-terphenyl-2-yl group,
a m-terphenyl-4-yl group,
a m-terphenyl-3-yl group,
a m-terphenyl-2-yl group,
an o-terphenyl-4-yl group,
an o-terphenyl-3-yl group,
an o-terphenyl-2-yl group,
a 1-naphthyl group,
a 2-naphthyl group,
an anthryl group,
a benzanthryl group,
a phenanthryl group,
a benzophenanthryl group,
a phenarenyl group,
a pyrenyl group,
a chrysenyl group,
a benzochrysenyl group,
a triphenylenyl group,
a benzotriphenylenyl group,
a tetracenyl group,
a pentacenyl group,
a fluorenyl group,
a 9,9'-spirobifluorenyl group,
a benzofluorenyl group,
a dibenzofluorenyl group,
a fluoranthenyl group,
a benzofluoranthenyl group,
a perylenyl group, and
monovalent aryl groups derived by removing one hydrogen atom from each of the ring structures represented by the following general formulae (TEMP-1) to (TEMP-15):

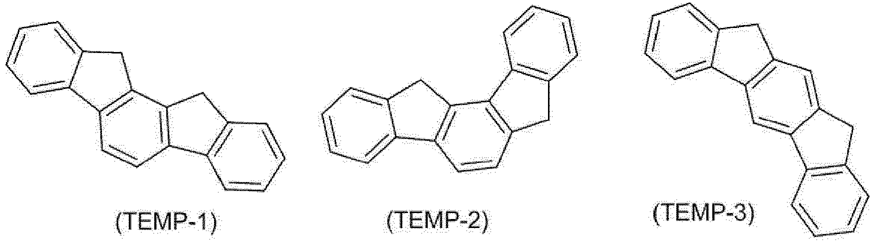

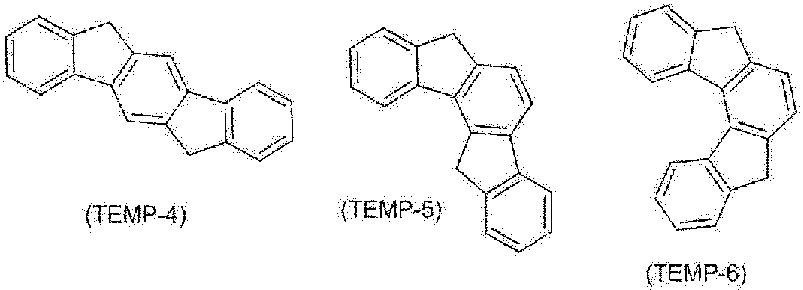

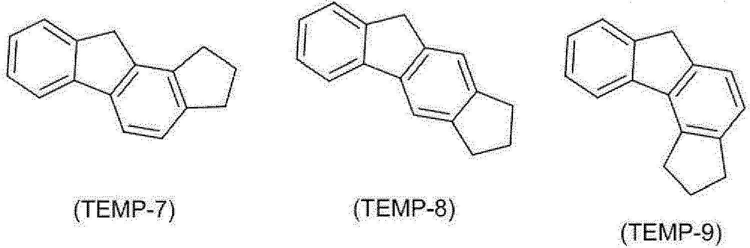

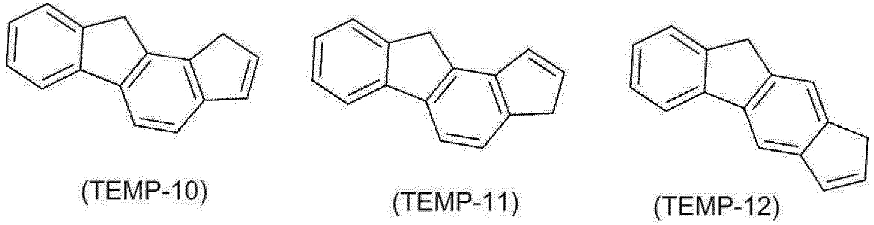

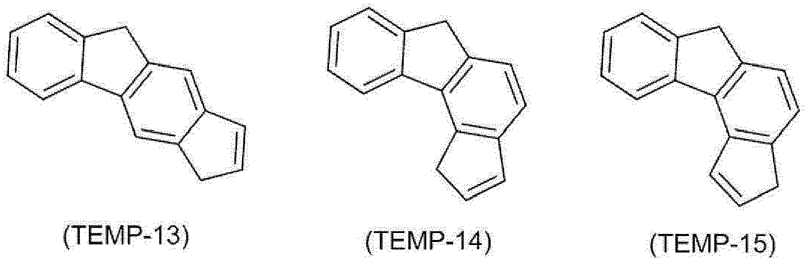

### Substituted Aryl Group (Set of Specific Examples G1B):

an o-tolyl group,
a m-tolyl group,
a p-tolyl group,
a p-xylyl group,
a m-xylyl group,
an o-xylyl group,
a p-isopropylphenyl group,
a m-isopropylphenyl group,
an o-isopropylphenyl group,
a p-t-butylphenyl group,
a m-t-butylphenyl group,
a o-t-butylphenyl group,
a 3,4,5-trimethylphenyl group,
a 9,9-dimethylfluorenyl group,
a 9,9-diphenylfluorenyl group,
a 9,9-bis(4-methylphenyl)fluorenyl group,
a 9,9-bis(4-isopropylphenyl)fluorenyl group,
a 9,9-bis(4-t-butylphenyl)fluorenyl group,
a cyanophenyl group,
a triphenylsilylphenyl group,
a trimethylsilylphenyl group,
a phenylnaphthyl group,
a naphthylphenyl group, and
groups formed by substituting one or more hydrogen atom of each of monovalent aryl groups derived from the ring structures represented by the general formulae (TEMP-1) to (TEMP-15) by a substituent.

### Substituted or Unsubstituted Heterocyclic Group

In the description herein, the "heterocyclic group" means a cyclic group containing at least one hetero atom in the ring atoms. Specific examples of the hetero atom include a nitrogen atom, an oxygen atom, a sulfur atom, a silicon atom, a phosphorus atom, and a boron atom.

In the description herein, the "heterocyclic group" is a monocyclic group or a condensed ring group.

In the description herein, the "heterocyclic group" is an aromatic heterocyclic group or a non-aromatic heterocyclic group.

In the description herein, specific examples (set of specific examples G2) of the "substituted or unsubstituted heterocyclic group" include the unsubstituted heterocyclic groups (set of specific examples G2A) and the substituted heterocyclic groups (set of specific examples G2B) shown below. (Herein, the unsubstituted heterocyclic group means the case where the "substituted or unsubstituted heterocyclic group" is an "unsubstituted heterocyclic group", and the substituted heterocyclic group means the case where the "substituted or unsubstituted heterocyclic group" is a "substituted heterocyclic group".) In the description herein, the simple expression "heterocyclic group" encompasses both the "unsubstituted heterocyclic group" and the "substituted heterocyclic group".

The "substituted heterocyclic group" means a group formed by substituting one or more hydrogen atom of the "unsubstituted heterocyclic group" by a substituent. Specific examples of the "substituted heterocyclic group" include groups formed by substituting a hydrogen atom of each of the "unsubstituted heterocyclic groups" in the set of specific examples G2A by a substituent, and the examples of the substituted heterocyclic groups in the set of specific examples G2B. The examples of the "unsubstituted heterocyclic group" and the examples of the "substituted heterocyclic group" enumerated herein are mere examples, and the "substituted heterocyclic group" in the description herein encompasses groups formed by substituting a hydrogen atom bonded to the ring atom of the heterocyclic group itself of each of the "substituted heterocyclic groups" in the set of specific examples G2B by a substituent, and groups formed by substituting a hydrogen atom of the substituent of each of the "substituted heterocyclic groups" in the set of specific examples G2B by a substituent.

The set of specific examples G2Aincludes, for example, the unsubstituted heterocyclic group containing a nitrogen atom (set of specific examples G2A1), the unsubstituted heterocyclic group containing an oxygen atom (set of specific examples G2A2), the unsubstituted heterocyclic group containing a sulfur atom (set of specific examples G2A3), and monovalent heterocyclic groups derived by removing one hydrogen atom from each of the ring structures represented by the following general formulae (TEMP-16) to (TEMP-33) (set of specific examples G2A4).

The set of specific examples G2B includes, for example, the substituted heterocyclic groups containing a nitrogen atom (set of specific examples G2B 1), the substituted heterocyclic groups containing an oxygen atom (set of specific examples G2B2), the substituted heterocyclic groups containing a sulfur atom (set of specific examples G2B3), and groups formed by substituting one or more hydrogen atom of each of monovalent heterocyclic groups derived from the ring structures represented by the following general formulae (TEMP-16) to (TEMP-33) by a substituent (set of specific examples G2B4).

### Unsubstituted Heterocyclic Group containing Nitrogen Atom (Set of Specific Examples G2A1):

a pyrrolyl group,
an imidazolyl group,
a pyrazolyl group,
a triazolyl group,
a tetrazolyl group,
an oxazolyl group,
an isoxazolyl group,
an oxadiazolyl group,
a thiazolyl group,
an isothiazolyl group,
a thiadiazolyl group,
a pyridyl group,
a pyridazinyl group,
a pyrimidinyl group,
a pyrazinyl group,
a triazinyl group,
an indolyl group,
an isoindolyl group,
an indolizinyl group,
a quinolizinyl group,
a quinolyl group,
an isoquinolyl group,
a cinnolinyl group,
a phthalazinyl group,
a quinazolinyl group,
a quinoxalinyl group,
a benzimidazolyl group,
an indazolyl group,
a phenanthrolinyl group,
a phenanthridinyl group,
an acridinyl group,
a phenazinyl group,
a carbazolyl group,
a benzocarbazolyl group,
a morpholino group,
a phenoxazinyl group,
a phenothiazinyl group,
an azacarbazolyl group, and
a diazacarbazolyl group.

### Unsubstituted Heterocyclic Group containing Oxygen Atom (Set of Specific Examples G2A2):

a furyl group,
an oxazolyl group,
an isoxazolyl group,
an oxadiazolyl group,
a xanthenyl group,
a benzofuranyl group,
an isobenzofuranyl group,
a dibenzofuranyl group,
a naphthobenzofuranyl group,
a benzoxazolyl group,
a benzisoxazolyl group,
a phenoxazinyl group,
a morpholino group,
a dinaphthofuranyl group,
an azadibenzofuranyl group,
a diazadibenzofuranyl group,
an azanaphthobenzofuranyl group, and
a diazanaphthobenzofuranyl group.

### Unsubstituted Heterocyclic Group containing Sulfur Atom (Set of Specific Examples G2A3):

a thienyl group,
a thiazolyl group,
an isothiazolyl group,
a thiadiazolyl group,
a benzothiophenyl group (benzothienyl group),
an isobenzothiophenyl group (isobenzothienyl group),
a dibenzothiophenyl group (dibenzothienyl group),
a naphthobenzothiophenyl group (naphthobenzothienyl group),
a benzothiazolyl group,
a benzisothiazolyl group,
a phenothiazinyl group,
a dinaphthothiophenyl group (dinaphthothienyl group),
an azadibenzothiophenyl group (azadibenzothienyl group),
a diazadibenzothiophenyl group (diazadibenzothienyl group),
an azanaphthobenzothiophenyl group (azanaphthobenzothienyl group), and
a diazanaphthobenzothiophenyl group (diazanaphthobenzothienyl group).

### Monovalent Heterocyclic Group derived by removing One Hydrogen Atom from Ring Structures represented by General Formulae (TEMP-16) to (TEMP-33) (Set of Specific Examples G2A4)

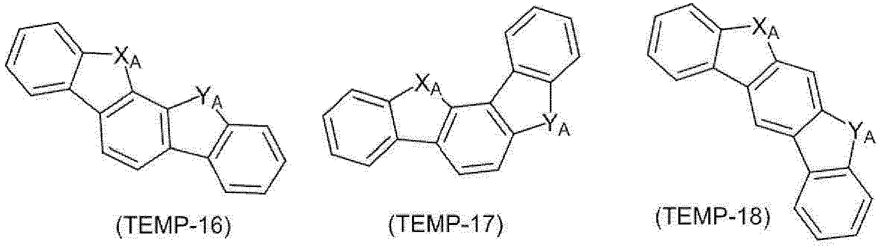

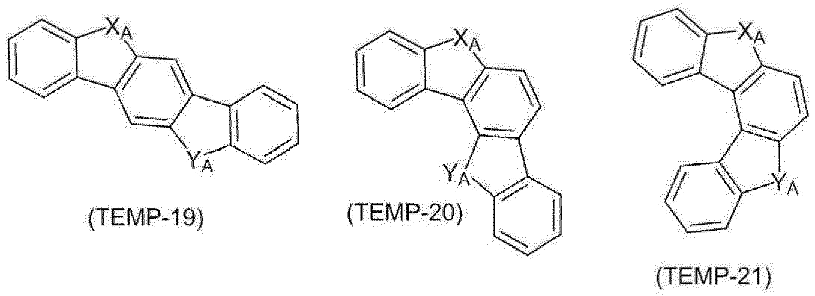

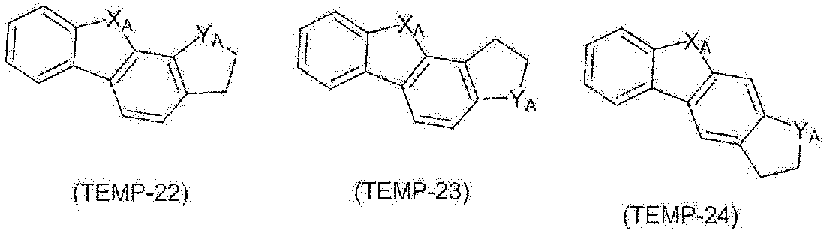

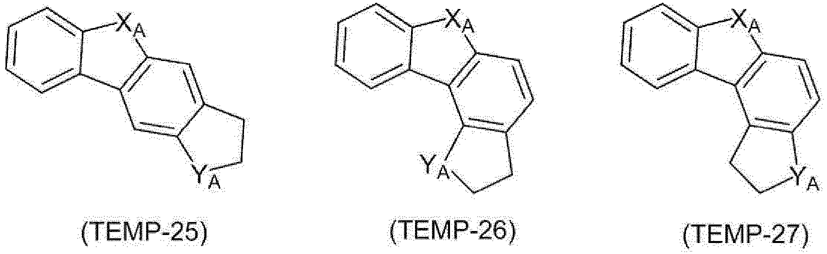

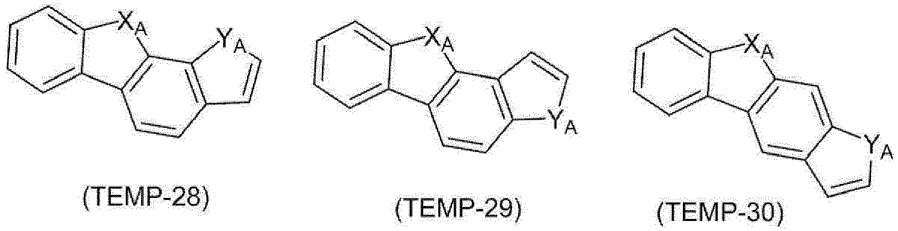

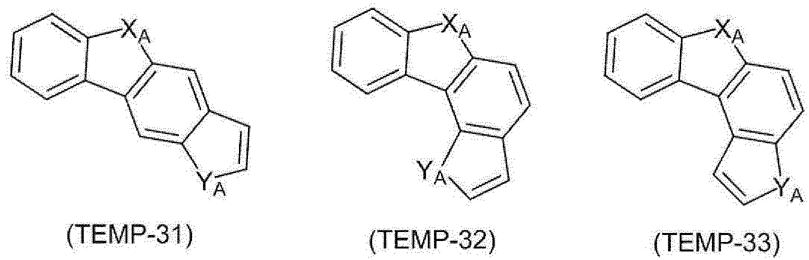

In the general formulae (TEMP-16) to (TEMP-33), X_{A} and Y_{A} each independently represent an oxygen atom, a sulfur atom, NH, or CH₂, provided that at least one of X_{A} and Y_{A} represents an oxygen atom, a sulfur atom, or NH.

In the general formulae (TEMP-16) to (TEMP-33), in the case where at least one of X_{A} and Y_{A} represents NH or CH₂, the monovalent heterocyclic groups derived from the ring structures represented by the general formulae (TEMP-16) to (TEMP-33) include monovalent groups formed by removing one hydrogen atom from the NH or CH₂.

Substituted Heterocyclic Group containing Nitrogen Atom (Set of Specific Examples G2B1):
a (9-phenyl)carbazolyl group,
a (9-biphenylyl)carbazolyl group,
a (9-phenyl)phenylcarbazolyl group,
a (9-naphthyl)carbazolyl group,
a diphenylcarbazol-9-yl group,
a phenylcarbazol-9-yl group,
a methylbenzimidazolyl group,
an ethylbenzimidazolyl group,
a phenyltriazinyl group,
a biphenyltriazinyl group,
a diphenyltriazinyl group,
a phenylquinazolinyl group, and
a biphenylquinazolinyl group.

### Substituted Heterocyclic Group containing Oxygen Atom (Set of Specific Examples G2B2):

a phenyldibenzofuranyl group,
a methyldibenzofuranyl group,
a t-butyldibenzofuranyl group, and
a monovalent residual group of spiro[9H-xanthene-9,9'-[9H]fluorene].

### Substituted Heterocyclic Group containing Sulfur Atom (Set of Specific Examples G2B3):

a phenyldibenzothiophenyl group,
a methyldibenzothiophenyl group,
a t-butyldibenzothiophenyl group, and
a monovalent residual group of spiro[9H-thioxanthene-9,9'-[9H]fluorene].

### Group formed by substituting one or more Hydrogen Atom of Monovalent Heterocyclic Group derived from Ring Structures represented by General Formulae (TEMP-16) to (TEMP-33) by Substituent (Set of Specific Examples G2B4)

The "one or more hydrogen atom of the monovalent heterocyclic group" means one or more hydrogen atom selected from the hydrogen atom bonded to the ring carbon atom of the monovalent heterocyclic group, the hydrogen atom bonded to the nitrogen atom in the case where at least one of X_{A} and Y_{A} represents NH, and the hydrogen atom of the methylene group in the case where one of X_{A} and Y_{A} represents CH₂.

### Substituted or Unsubstituted Alkyl Group

In the description herein, specific examples (set of specific examples G3) of the "substituted or unsubstituted alkyl group" include the unsubstituted alkyl groups (set of specific examples G3A) and the substituted alkyl groups (set of specific examples G3B) shown below. (Herein, the unsubstituted alkyl group means the case where the "substituted or unsubstituted alkyl group" is an "unsubstituted alkyl group", and the substituted alkyl group means the case where the "substituted or unsubstituted alkyl group" is a "substituted alkyl group".) In the description herein, the simple expression "alkyl group" encompasses both the "unsubstituted alkyl group" and the "substituted alkyl group".

The "substituted alkyl group" means a group formed by substituting one or more hydrogen atom of the "unsubstituted alkyl group" by a substituent. Specific examples of the "substituted alkyl group" include groups formed by substituting one or more hydrogen atom of each of the "unsubstituted alkyl groups" (set of specific examples G3A) by a substituent, and the examples of the substituted alkyl groups (set of specific examples G3B). In the description herein, the alkyl group in the "unsubstituted alkyl group" means a chain-like alkyl group. Accordingly, the "unsubstituted alkyl group" encompasses an "unsubstituted linear alkyl group" and an "unsubstituted branched alkyl group". The examples of the "unsubstituted alkyl group" and the examples of the "substituted alkyl group" enumerated herein are mere examples, and the "substituted alkyl group" in the description herein encompasses groups formed by substituting a hydrogen atom of the alkyl group itself of each of the "substituted alkyl groups" in the set of specific examples G3B by a substituent, and groups formed by substituting a hydrogen atom of the substituent of each of the "substituted alkyl groups" in the set of specific examples G3B by a substituent.

### Unsubstituted Alkyl Group (Set of Specific Examples G3A):

a methyl group,
an ethyl group,
a n-propyl group,
an isopropyl group,
a n-butyl group,
an isobutyl group,
a s-butyl group, and
a t-butyl group.

### Substituted Alkyl Group (Set of Specific Examples G3B):

a heptafluoropropyl group (including isomers),
a pentafluoroethyl group,
a 2,2,2-trifluoroethyl group, and
a trifluoromethyl group.

### Substituted or Unsubstituted Alkenyl Group

In the description herein, specific examples (set of specific examples G4) of the "substituted or unsubstituted alkenyl group" include the unsubstituted alkenyl groups (set of specific examples G4A) and the substituted alkenyl groups (set of specific examples G4B) shown below. (Herein, the unsubstituted alkenyl group means the case where the "substituted or unsubstituted alkenyl group" is an "unsubstituted alkenyl group", and the substituted alkenyl group means the case where the "substituted or unsubstituted alkenyl group" is a "substituted alkenyl group".) In the description herein, the simple expression "alkenyl group" encompasses both the "unsubstituted alkenyl group" and the "substituted alkenyl group".

The "substituted alkenyl group" means a group formed by substituting one or more hydrogen atom of the "unsubstituted alkenyl group" by a substituent. Specific examples of the "substituted alkenyl group" include the "unsubstituted alkenyl groups" (set of specific examples G4A) that each have a substituent, and the examples of the substituted alkenyl groups (set of specific examples G4B). The examples of the "unsubstituted alkenyl group" and the examples of the "substituted alkenyl group" enumerated herein are mere examples, and the "substituted alkenyl group" in the description herein encompasses groups formed by substituting a hydrogen atom of the alkenyl group itself of each of the "substituted alkenyl groups" in the set of specific examples G4B by a substituent, and groups formed by substituting a hydrogen atom of the substituent of each of the "substituted alkenyl groups" in the set of specific examples G4B by a substituent.

### Unsubstituted Alkenyl Group (Set of Specific Examples G4A):

a vinyl group,
an allyl group,
a 1-butenyl group,
a 2-butenyl group, and
a 3-butenyl group.

### Substituted Alkenyl Group (Set of Specific Examples G4B):

a 1,3-butanedienyl group,
a 1-methylvinyl group,
a 1-methylallyl group,
a 1,1-dimethylallyl group,
a 2-methylallyl group, and
a 1,2-dimethylallyl group.

### Substituted or Unsubstituted Alkynyl Group

In the description herein, specific examples (set of specific examples G5) of the "substituted or unsubstituted alkynyl group" include the unsubstituted alkynyl group (set of specific examples G5A) shown below. (Herein, the unsubstituted alkynyl group means the case where the "substituted or unsubstituted alkynyl group" is an "unsubstituted alkynyl group".) In the description herein, the simple expression "alkynyl group" encompasses both the "unsubstituted alkynyl group" and the "substituted alkynyl group".

The "substituted alkynyl group" means a group formed by substituting one or more hydrogen atom of the "unsubstituted alkynyl group" by a substituent. Specific examples of the "substituted alkenyl group" include groups formed by substituting one or more hydrogen atom of the "unsubstituted alkynyl group" (set of specific examples G5A) by a substituent.

Unsubstituted Alkynyl Group (Set of Specific Examples G5A):
an ethynyl group.

### Substituted or Unsubstituted Cycloalkyl Group

In the description herein, specific examples (set of specific examples G6) of the "substituted or unsubstituted cycloalkyl group" include the unsubstituted cycloalkyl groups (set of specific examples G6A) and the substituted cycloalkyl group (set of specific examples G6B) shown below. (Herein, the unsubstituted cycloalkyl group means the case where the "substituted or unsubstituted cycloalkyl group" is an "unsubstituted cycloalkyl group", and the substituted cycloalkyl group means the case where the "substituted or unsubstituted cycloalkyl group" is a "substituted cycloalkyl group".) In the description herein, the simple expression "cycloalkyl group" encompasses both the "unsubstituted cycloalkyl group" and the "substituted cycloalkyl group".

The "substituted cycloalkyl group" means a group formed by substituting one or more hydrogen atom of the "unsubstituted cycloalkyl group" by a substituent. Specific examples of the "substituted cycloalkyl group" include groups formed by substituting one or more hydrogen atom of each of the "unsubstituted cycloalkyl groups" (set of specific examples G6A) by a substituent, and the example of the substituted cycloalkyl group (set of specific examples G6B). The examples of the "unsubstituted cycloalkyl group" and the examples of the "substituted cycloalkyl group" enumerated herein are mere examples, and the "substituted cycloalkyl group" in the description herein encompasses groups formed by substituting one or more hydrogen atom bonded to the carbon atoms of the cycloalkyl group itself of the "substituted cycloalkyl group" in the set of specific examples G6B by a substituent, and groups formed by substituting a hydrogen atom of the substituent of the "substituted cycloalkyl group" in the set of specific examples G6B by a substituent.

### Unsubstituted Cycloalkyl Group (Set of Specific Examples G6A):

a cyclopropyl group,
a cyclobutyl group,
a cyclopentyl group,
a cyclohexyl group,
a 1-adamantyl group,
a 2-adamantyl group,
a 1-norbornyl group, and
a 2-norbornyl group.

Substituted Cycloalkyl Group (Set of Specific Examples G6B):
a 4-methylcyclohexyl group.

### Group represented by -Si(R₉₀₁)(R₉₀₂)(R₉₀₃)

In the description herein, specific examples (set of specific examples G7) of the group represented by -Si(R₉₀₁)(R₉₀₂)(R₉₀₃) include:

-Si(G1)(G1)(G1),

-Si(G1)(G2)(G2),

-Si(G1)(G1)(G2),

-Si(G2)(G2)(G2),

-Si(G3)(G3)(G3),

and

-Si(G6)(G6)(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.
Plural groups represented by G1 in -Si(G1)(G1)(G1) are the same as or different from each other.
Plural groups represented by G2 in -Si(G1)(G2)(G2) are the same as or different from each other.
Plural groups represented by G1 in -Si(G1)(G1)(G2) are the same as or different from each other.
Plural groups represented by G2 in -Si(G2)(G2)(G2) are the same as or different from each other.
Plural groups represented by G3 in -Si(G3)(G3)(G3) are the same as or different from each other.
Plural groups represented by G6 in -Si(G6)(G6)(G6) are the same as or different from each other.

### Group represented by -O-(R₉₀₄)

In the description herein, specific examples (set of specific examples G8) of the group represented by -O-(R₉₀₄) include:

-O(G1),

-O(G2),

-O(G3),

and

-O(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

### Group represented by -S-(R₉₀₅)

In the description herein, specific examples (set of specific examples G9) of the group represented by -S-(R₉₀₅) include:

-S(G1),

-S(G2),

-S(G3),

and

-S(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

### Group represented by -N(R₉₀₆)(R₉₀₇)

In the description herein, specific examples (set of specific examples G10) of the group represented by -N(R₉₀₆)(R₉₀₇) include:

-N(G1)(G1),

-N(G2)(G2),

-N(G1)(G2),

-N(G3)(G3),

and

-N(G6)(G6).

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.
Plural groups represented by G1 in -N(G1)(G1) are the same as or different from each other.
Plural groups represented by G2 in -N(G2)(G2) are the same as or different from each other.
Plural groups represented by G3 in -N(G3)(G3) are the same as or different from each other.
Plural groups represented by G6 in -N(G6)(G6) are the same as or different from each other.

### Halogen Atom

In the description herein, specific examples (set of specific examples G11) of the "halogen atom" include a fluorine atom, a chlorine atom, a bromine atom, and an iodine atom.

### Substituted or Unsubstituted Fluoroalkyl Group

In the description herein, the "substituted or unsubstituted fluoroalkyl group" means a group formed by substituting at least one hydrogen atom bonded to the carbon atom constituting the alkyl group in the "substituted or unsubstituted alkyl group" by a fluorine atom, and encompasses a group formed by substituting all the hydrogen atoms bonded to the carbon atoms constituting the alkyl group in the "substituted or unsubstituted alkyl group" by fluorine atoms (i.e., a perfluoroalkyl group). The number of carbon atoms of the "unsubstituted fluoroalkyl group" is 1 to 50, preferably 1 to 30, and more preferably 1 to 18, unless otherwise indicated in the description. The "substituted fluoroalkyl group" means a group formed by substituting one or more hydrogen atom of the "fluoroalkyl group" by a substituent. In the description herein, the "substituted fluoroalkyl group" encompasses a group formed by substituting one or more hydrogen atom bonded to the carbon atom of the alkyl chain in the "substituted fluoroalkyl group" by a substituent, and a group formed by substituting one or more hydrogen atom of the substituent in the "substituted fluoroalkyl group" by a substituent. Specific examples of the "unsubstituted fluoroalkyl group" include examples of groups formed by substituting one or more hydrogen atom in each of the "alkyl group" (set of specific examples G3) by a fluorine atom.

### Substituted or Unsubstituted Haloalkyl Group

In the description herein, the "substituted or unsubstituted haloalkyl group" means a group formed by substituting at least one hydrogen atom bonded to the carbon atom constituting the alkyl group in the "substituted or unsubstituted alkyl group" by a halogen atom, and encompasses a group formed by substituting all the hydrogen atoms bonded to the carbon atoms constituting the alkyl group in the "substituted or unsubstituted alkyl group" by halogen atoms. The number of carbon atoms of the "unsubstituted haloalkyl group" is 1 to 50, preferably 1 to 30, and more preferably 1 to 18, unless otherwise indicated in the description. The "substituted haloalkyl group" means a group formed by substituting one or more hydrogen atom of the "haloalkyl group" by a substituent. In the description herein, the "substituted haloalkyl group" encompasses a group formed by substituting one or more hydrogen atom bonded to the carbon atom of the alkyl chain in the "substituted haloalkyl group" by a substituent, and a group formed by substituting one or more hydrogen atom of the substituent in the "substituted haloalkyl group" by a substituent. Specific examples of the "unsubstituted haloalkyl group" include examples of groups formed by substituting one or more hydrogen atom in each of the "alkyl group" (set of specific examples G3) by a halogen atom. A haloalkyl group may be referred to as a halogenated alkyl group in some cases.

### Substituted or Unsubstituted Alkoxy Group

In the description herein, specific examples of the "substituted or unsubstituted alkoxy group" include a group represented by -O(G3), wherein G3 represents the "substituted or unsubstituted alkyl group" described in the set of specific examples G3. The number of carbon atoms of the "unsubstituted alkoxy group" is 1 to 50, preferably 1 to 30, and more preferably 1 to 18, unless otherwise indicated in the description.

### Substituted or Unsubstituted Alkylthio Group

In the description herein, specific examples of the "substituted or unsubstituted alkylthio group" include a group represented by -S(G3), wherein G3 represents the "substituted or unsubstituted alkyl group" described in the set of specific examples G3. The number of carbon atoms of the "unsubstituted alkylthio group" is 1 to 50, preferably 1 to 30, and more preferably 1 to 18, unless otherwise indicated in the description.

### Substituted or Unsubstituted Aryloxy Group

In the description herein, specific examples of the "substituted or unsubstituted aryloxy group" include a group represented by -O(G1), wherein G1 represents the "substituted or unsubstituted aryl group" described in the set of specific examples G1. The number of ring carbon atoms of the "unsubstituted aryloxy group" is 6 to 50, preferably 6 to 30, and more preferably 6 to 18, unless otherwise indicated in the description.

### Substituted or Unsubstituted Arylthio Group

In the description herein, specific examples of the "substituted or unsubstituted arylthio group" include a group represented by -S(G1), wherein G1 represents the "substituted or unsubstituted aryl group" described in the set of specific examples G1. The number of ring carbon atoms of the "unsubstituted arylthio group" is 6 to 50, preferably 6 to 30, and more preferably 6 to 18, unless otherwise indicated in the description.

### Substituted or Unsubstituted Trialkylsilyl Group

In the description herein, specific examples of the "trialkylsilyl group" include a group represented by -Si(G3)(G3)(G3), wherein G3 represents the "substituted or unsubstituted alkyl group" described in the set of specific examples G3. Plural groups represented by G3 in - Si(G3)(G3)(G3) are the same as or different from each other. The number of carbon atoms of each of alkyl groups of the "substituted or unsubstituted trialkylsilyl group" is 1 to 50, preferably 1 to 20, and more preferably 1 to 6, unless otherwise indicated in the description.

### Substituted or Unsubstituted Aralkyl Group

In the description herein, specific examples of the "substituted or unsubstituted aralkyl group" include a group represented by -(G3)-(G1), wherein G3 represents the "substituted or unsubstituted alkyl group" described in the set of specific examples G3, and G1 represents the "substituted or unsubstituted aryl group" described in the set of specific examples G1. Accordingly, the "aralkyl group" is a group formed by substituting a hydrogen atom of an "alkyl group" by an "aryl group" as a substituent, and is one embodiment of the "substituted alkyl group". The "unsubstituted aralkyl group" is an "unsubstituted alkyl group" that is substituted by an "unsubstituted aryl group", and the number of carbon atoms of the "unsubstituted aralkyl group" is 7 to 50, preferably 7 to 30, and more preferably 7 to 18, unless otherwise indicated in the description.

Specific examples of the "substituted or unsubstituted aralkyl group" include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, and a 2-β-naphthylisopropyl group.

In the description herein, the substituted or unsubstituted aryl group is preferably a phenyl group, a p-biphenyl group, a m-biphenyl group, an o-biphenyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group, a 9,9-diphenylfluorenyl group, and the like, unless otherwise indicated in the description.

In the description herein, the substituted or unsubstituted heterocyclic group is preferably a pyridyl group, a pyrimidinyl group, a triazinyl group, a quinolyl group, an isoquinolyl group, a quinazolinyl group, a benzimidazolyl group, a phenanthrolinyl group, a carbazolyl group (e.g., a 1-carbazolyl, group, a 2-carbazolyl, group, a 3-carbazolyl, group, a 4-carbazolyl, group, or a 9-carbazolyl, group), a benzocarbazolyl group, an azacarbazolyl group, a diazacarbazolyl group, a dibenzofuranyl group, a naphthobenzofuranly group, an azadibenzofuranyl group, a diazadibenzofuranyl group, a dibenzothiophenyl group, a naphthobenzothiophenyl group, an azadibenzothiophenyl group, a diazadibenzothiophenyl group, a (9-phenyl)carbazolyl group (e.g., a (9-phenyl)carbazol-1-yl group, a (9-phenyl)carbazol-2-yl group, a (9-phenyl)carbazol-3-yl group, or a (9-phenyl)carbazol-4-yl group), a (9-biphenylyl)carbazolyl group, a (9-phenyl)phenylcarbazolyl group, a diphenylcarbazol-9-yl group, a phenylcarbazol-9-yl group, a phenyltriazinyl group, a biphenylyltriazinyl group, a diphenyltriazinyl group, a phenyldibenzofuranyl group, a phenyldibenzothiophenyl group, and the like, unless otherwise indicated in the description.

In the description herein, the carbazolyl group is specifically any one of the following groups unless otherwise indicated in the description.

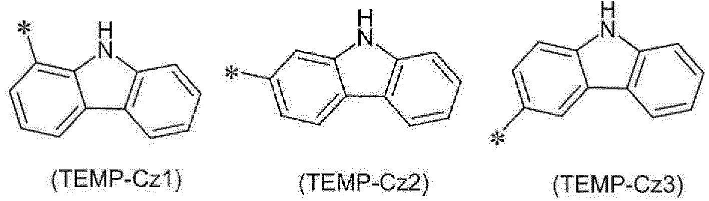

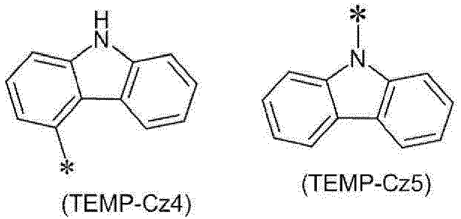

In the description herein, the (9-phenyl)carbazolyl group is specifically any one of the following groups unless otherwise indicated in the description.

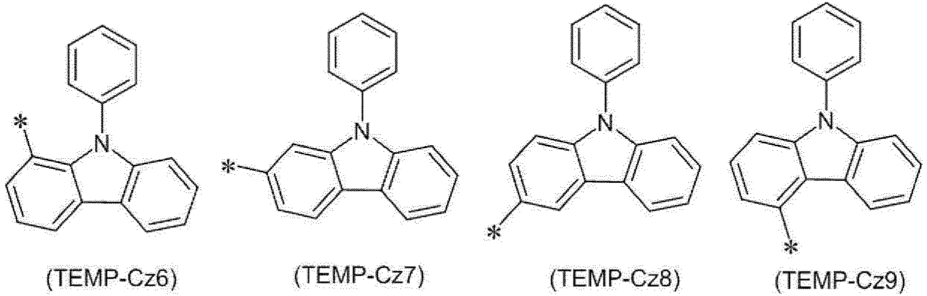

In the general formulae (TEMP-Cz1) to (TEMP-Cz9), * represents a bonding site.

In the description herein, the dibenzofuranyl group and the dibenzothiophenyl group are specifically any one of the following groups unless otherwise indicated in the description.

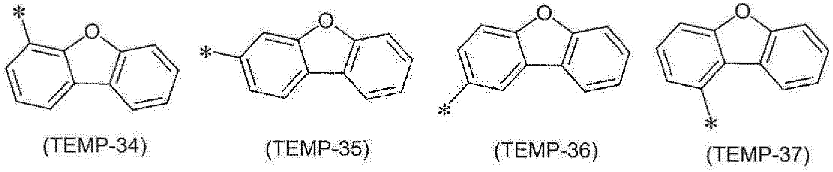

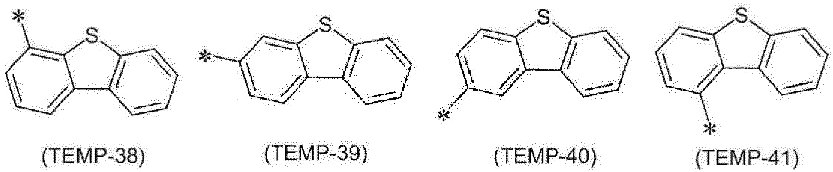

In the general formulae (TEMP-34) to (TEMP-41), * represents a bonding site.

In the description herein, the substituted or unsubstituted alkyl group is preferably a methyl group, an ethyl group, a propyl group, an isopropyl group, a n-butyl group, an isobutyl group, a t-butyl group, or the like unless otherwise indicated in the description.

### Substituted or Unsubstituted Arylene Group

In the description herein, the "substituted or unsubstituted arylene group" is a divalent group derived by removing one hydrogen atom on the aryl ring from the "substituted or unsubstituted aryl group" described above unless otherwise indicated in the description. Specific examples (set of specific examples G12) of the "substituted or unsubstituted arylene group" include divalent groups derived by removing one hydrogen atom on the aryl ring from the "substituted or unsubstituted aryl groups" described in the set of specific examples G1.

### Substituted or Unsubstituted Divalent Heterocyclic Group

In the description herein, the "substituted or unsubstituted divalent heterocyclic group" is a divalent group derived by removing one hydrogen atom on the heterocyclic ring from the "substituted or unsubstituted heterocyclic group" described above unless otherwise indicated in the description. Specific examples (set of specific examples G13) of the "substituted or unsubstituted divalent heterocyclic group" include divalent groups derived by removing one hydrogen atom on the heterocyclic ring from the "substituted or unsubstituted heterocyclic groups" described in the set of specific examples G2.

### Substituted or Unsubstituted Alkylene Group

In the description herein, the "substituted or unsubstituted alkylene group" is a divalent group derived by removing one hydrogen atom on the alkyl chain from the "substituted or unsubstituted alkyl group" described above unless otherwise indicated in the description. Specific examples (set of specific examples G14) of the "substituted or unsubstituted alkylene group" include divalent groups derived by removing one hydrogen atom on the alkyl chain from the "substituted or unsubstituted alkyl groups" described in the set of specific examples G3.

In the description herein, the substituted or unsubstituted arylene group is preferably any one of the groups represented by the following general formulae (TEMP-42) to (TEMP-68) unless otherwise indicated in the description.

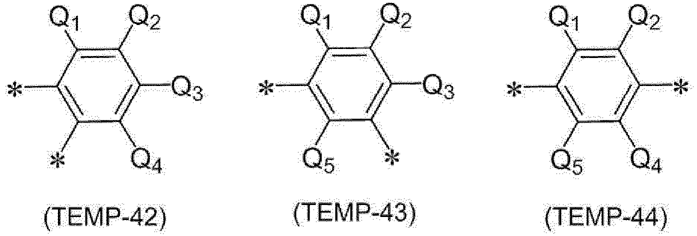

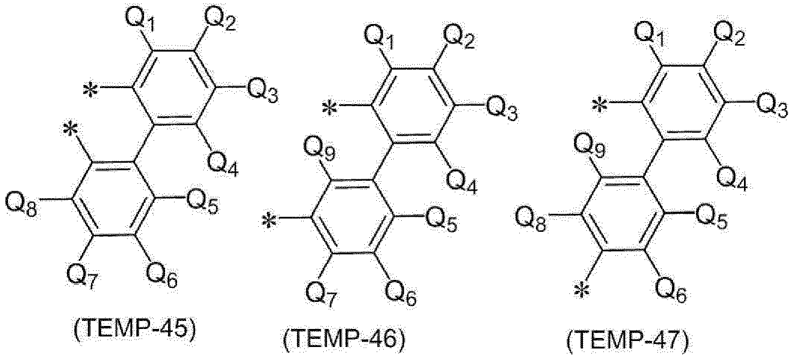

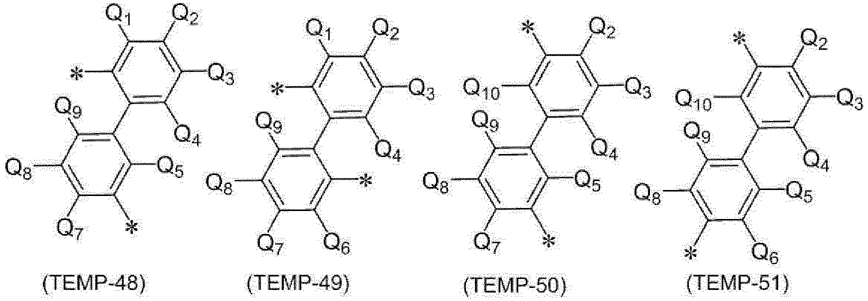

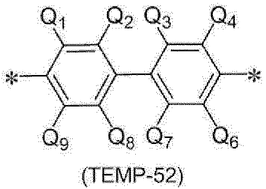

In the general formulae (TEMP-42) to (TEMP-52), Q₁ to Q₁₀ each independently represent a hydrogen atom or a substituent.

In the general formulae (TEMP-42) to (TEMP-52), * represents a bonding site.

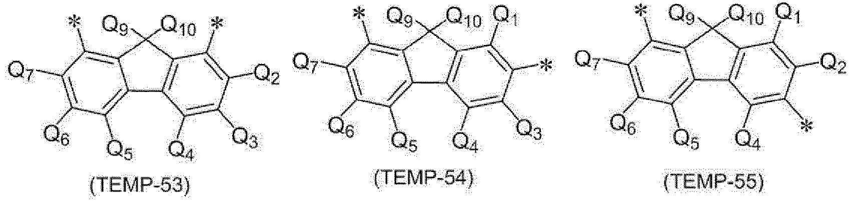

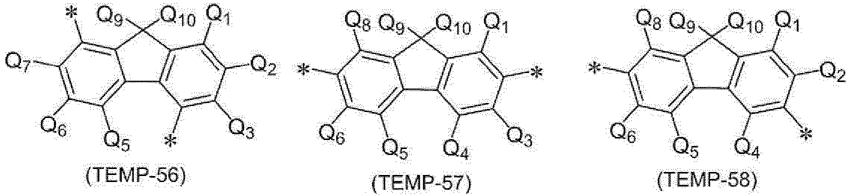

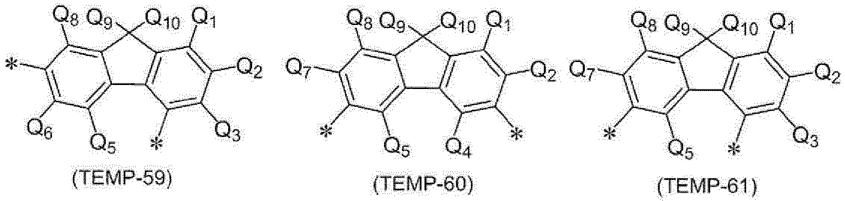

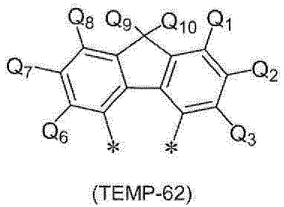

In the general formulae (TEMP-53) to (TEMP-62), Q₁ to Q₁₀ each independently represent a hydrogen atom or a substituent.

The formulae Q₉ and Q₁₀ may be bonded to each other to form a ring via a single bond.

In the general formulae (TEMP-53) to (TEMP-62), * represents a bonding site.

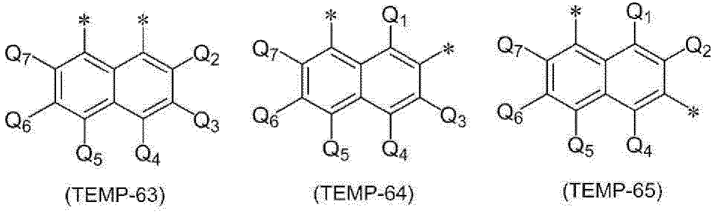

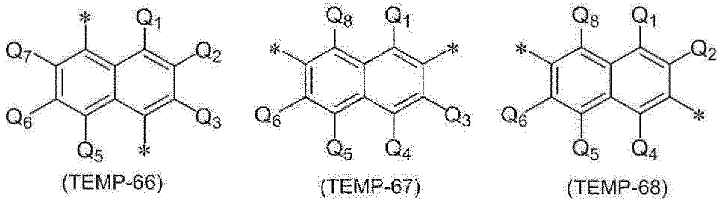

In the general formulae (TEMP-63) to (TEMP-68), Q₁ to Q₈ each independently represent a hydrogen atom or a substituent.

In the general formulae (TEMP-63) to (TEMP-68), * represents a bonding site.

In the description herein, the substituted or unsubstituted divalent heterocyclic group is preferably the groups represented by the following general formulae (TEMP-69) to (TEMP-102) unless otherwise indicated in the description.

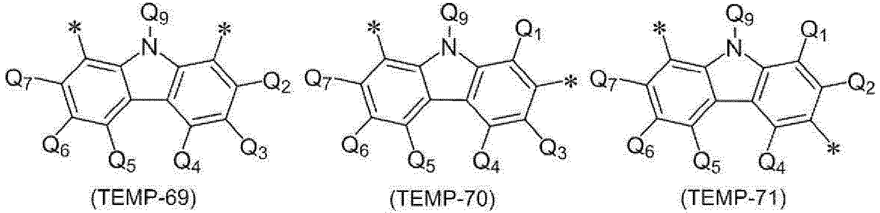

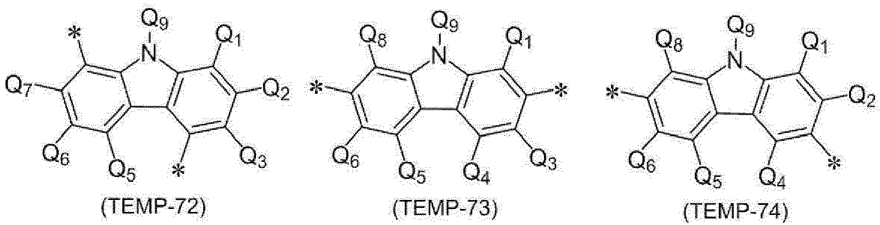

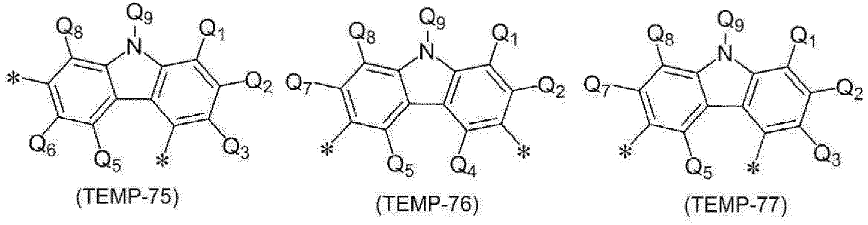

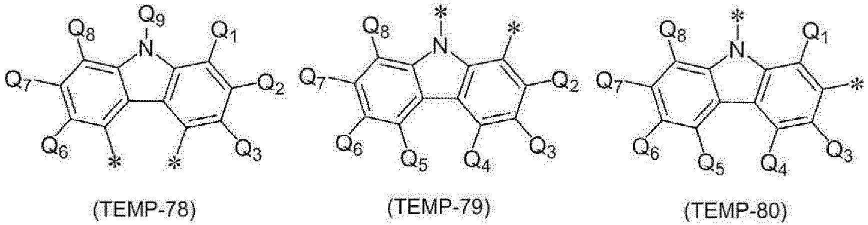

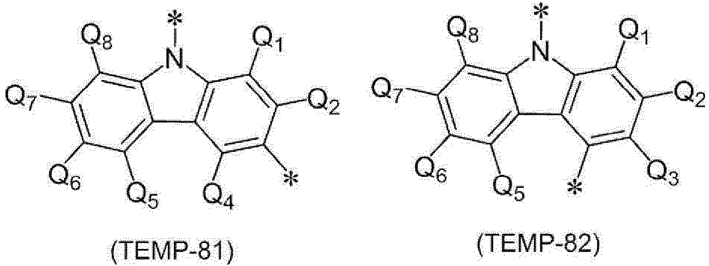

In the general formulae (TEMP-69) to (TEMP-82), Q₁ to Q₉ each independently represent a hydrogen atom or a substituent.

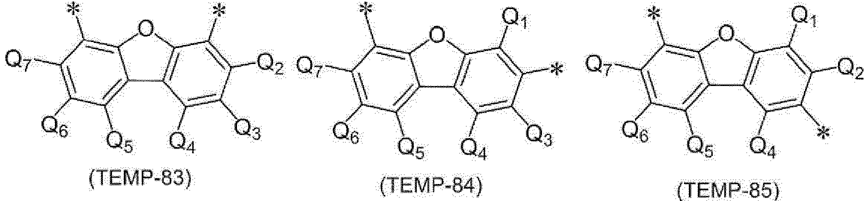

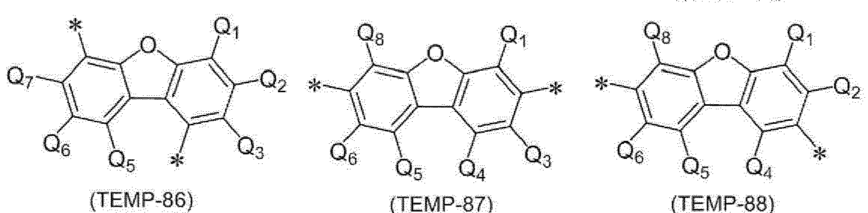

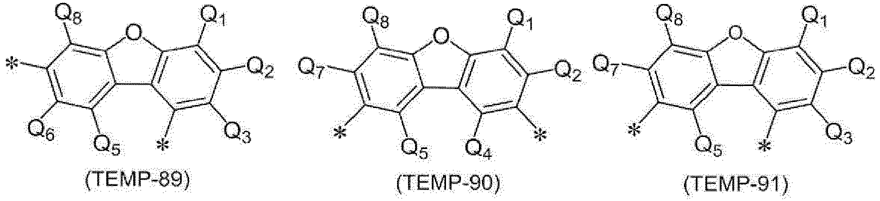

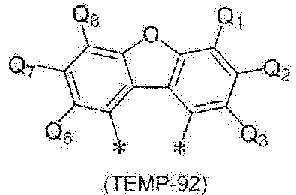

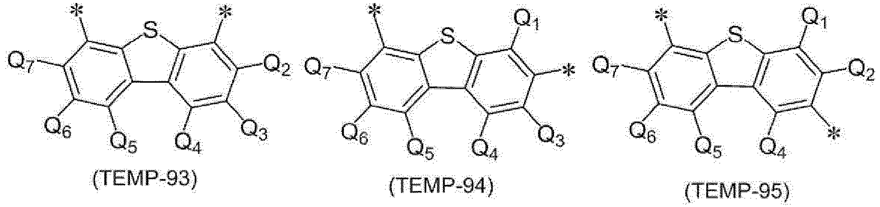

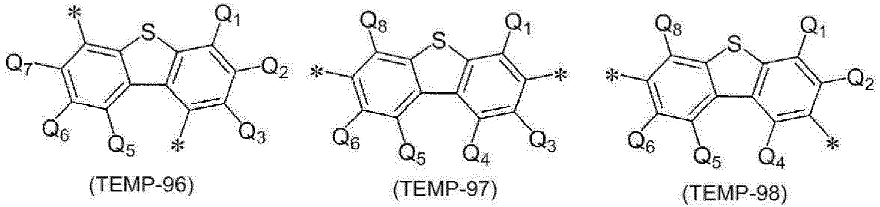

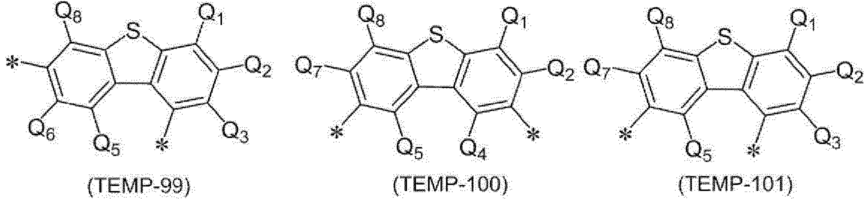

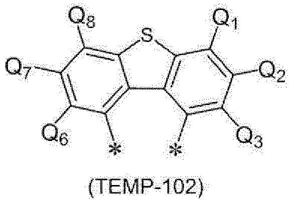

In the general formulae (TEMP-83) to (TEMP-102), Q₁ to Q₈ each independently represent a hydrogen atom or a substituent.

The above are the explanation of the "substituents in the description herein".

### Case forming Ring by bonding

In the description herein, the case where "one or more combinations of combinations each including adjacent two or more each are bonded to each other to form a substituted or unsubstituted monocyclic ring, or each are bonded to each other to form a substituted or unsubstituted condensed ring, or each are not bonded to each other" means a case where "one or more combinations of combinations each including adjacent two or more each are bonded to each other to form a substituted or unsubstituted monocyclic ring", a case where "one or more combinations of combinations each including adjacent two or more each are bonded to each other to form a substituted or unsubstituted condensed ring", and a case where "one or more combinations of combinations each including adj acent two or more each are not bonded to each other".

In the description herein, the case where "one or more combinations of combinations each including adjacent two or more each are bonded to each other to form a substituted or unsubstituted monocyclic ring" and the case where "one or more combinations of combinations each including adjacent two or more each are bonded to each other to form a substituted or unsubstituted condensed ring" (which may be hereinafter collectively referred to as a "case forming a ring by bonding") will be explained below. The cases will be explained for the anthracene compound represented by the following general formula (TEMP-103) having an anthracene core skeleton as an example.

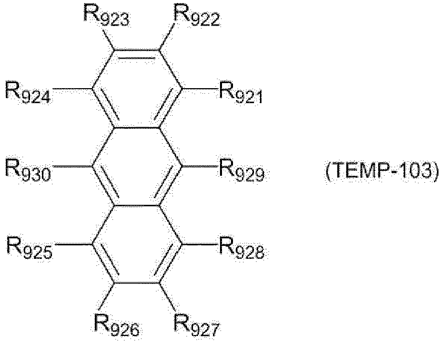

For example, in the case where "one or more combinations of combinations each including adjacent two or more each are bonded to each other to form a ring" among R₉₂₁ to R₉₃₀, the combinations each including adjacent two as one combination include a combination of R₉₂₁ and R₉₂₂, a combination of R₉₂₂ and R₉₂₃, a combination of R₉₂₃ and R₉₂₄, a combination of R₉₂₄ and R₉₃₀, a combination of R₉₃₀ and R₉₂₅, a combination of R₉₂₅ and R₉₂₆, a combination of R₉₂₆ and R₉₂₇, a combination of R₉₂₇ and R₉₂₈, a combination of R₉₂₈ and R₉₂₉, and a combination of R₉₂₉ and R₉₂₁.

The "one or more combinations" mean that two or more combinations each including adjacent two or more may form rings simultaneously. For example, in the case where R₉₂₁ and R₉₂₂ are bonded to each other to form a ring Q_{A}, and simultaneously R₉₂₅ and R₉₂₆ are bonded to each other to form a ring Q_{B}, the anthracene compound represented by the general formula (TEMP-103) is represented by the following general formula (TEMP-104).

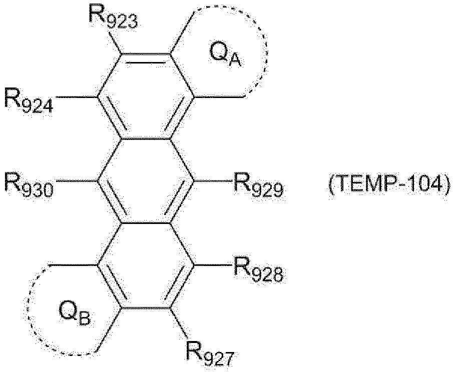

The case where the "combination including adjacent two or more forms rings" encompasses not only the case where adjacent two included in the combination are bonded as in the aforementioned example, but also the case where adjacent three or more included in the combination are bonded. For example, this case means that R₉₂₁ and R₉₂₂ are bonded to each other to form a ring Q_{A}, R₉₂₂ and R₉₂₃ are bonded to each other to form a ring Qc, and adjacent three (R₉₂₁, R₉₂₂, and R₉₂₃) included in the combination are bonded to each other to form rings, which are condensed to the anthracene core skeleton, and in this case, the anthracene compound represented by the general formula (TEMP-103) is represented by the following general formula (TEMP-105). In the following general formula (TEMP-105), the ring Q_{A} and the ring Qc share R₉₂₂.

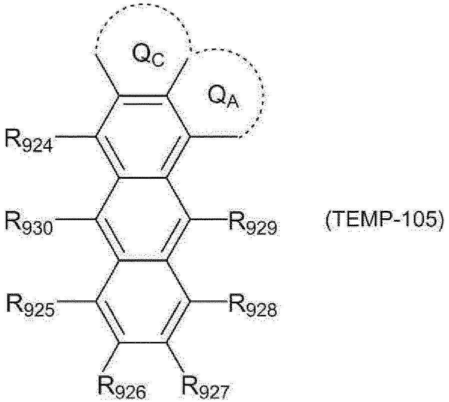

The formed "monocyclic ring" or "condensed ring" may be a saturated ring or an unsaturated ring in terms of structure of the formed ring itself. In the case where the "one combination including adjacent two" forms a "monocyclic ring" or a "condensed ring", the "monocyclic ring" or the "condensed ring" may form a saturated ring or an unsaturated ring. For example, the ring Q_{A} and the ring Q_{B} formed in the general formula (TEMP-104) each are a "monocyclic ring" or a "condensed ring". The ring Q_{A} and the ring Qc formed in the general formula (TEMP-105) each are a "condensed ring". The ring Q_{A} and the ring Qc in the general formula (TEMP-105) form a condensed ring through condensation of the ring Q_{A} and the ring Qc. In the case where the ring Q_{A} in the general formula (TMEP-104) is a benzene ring, the ring Q_{A} is a monocyclic ring. In the case where the ring Q_{A} in the general formula (TMEP-104) is a naphthalene ring, the ring Q_{A} is a condensed ring.

The "unsaturated ring" means an aromatic hydrocarbon ring or an aromatic heterocyclic ring. The "saturated ring" means an aliphatic hydrocarbon ring or a non-aromatic heterocyclic ring.

Specific examples of the aromatic hydrocarbon ring include the structures formed by terminating the groups exemplified as the specific examples in the set of specific examples G1 with a hydrogen atom.

Specific examples of the aromatic heterocyclic ring include the structures formed by terminating the aromatic heterocyclic groups exemplified as the specific examples in the set of specific examples G2 with a hydrogen atom.

Specific examples of the aliphatic hydrocarbon ring include the structures formed by terminating the groups exemplified as the specific examples in the set of specific examples G6 with a hydrogen atom.

The expression "to form a ring" means that the ring is formed only with the plural atoms of the core structure or with the plural atoms of the core structure and one or more arbitrary element. For example, the ring Q_{A} formed by bonding R₉₂₁ and R₉₂₂ each other shown in the general formula (TEMP-104) means a ring formed with the carbon atom of the anthracene skeleton bonded to R₉₂₁, the carbon atom of the anthracene skeleton bonded to R₉₂₂, and one or more arbitrary element. As a specific example, in the case where the ring Q_{A} is formed with R₉₂₁ and R₉₂₂, and in the case where a monocyclic unsaturated ring is formed with the carbon atom of the anthracene skeleton bonded to R₉₂₁, the carbon atom of the anthracene skeleton bonded to R₉₂₂, and four carbon atoms, the ring formed with R₉₂₁ and R₉₂₂ is a benzene ring.

Herein, the "arbitrary element" is preferably at least one kind of an element selected from the group consisting of a carbon element, a nitrogen element, an oxygen element, and a sulfur element, unless otherwise indicated in the description. For the arbitrary element (for example, for a carbon element or a nitrogen element), a bond that does not form a ring may be terminated with a hydrogen atom or the like, and may be substituted by an "arbitrary substituent" described later. In the case where an arbitrary element other than a carbon element is contained, the formed ring is a heterocyclic ring.

The number of the "one or more arbitrary element" constituting the monocyclic ring or the condensed ring is preferably 2 or more and 15 or less, more preferably 3 or more and 12 or less, and further preferably 3 or more and 5 or less, unless otherwise indicated in the description.

What is preferred between the "monocyclic ring" and the "condensed ring" is the "monocyclic ring" unless otherwise indicated in the description.

What is preferred between the "saturated ring" and the "unsaturated ring" is the "unsaturated ring" unless otherwise indicated in the description.

The "monocyclic ring" is preferably a benzene ring unless otherwise indicated in the description.

The "unsaturated ring" is preferably a benzene ring unless otherwise indicated in the description.

In the case where the "one or more combinations of combinations each including adjacent two or more" each are "bonded to each other to form a substituted or unsubstituted monocyclic ring", or each are "bonded to each other to form a substituted or unsubstituted condensed ring", it is preferred that the one or more combinations of combinations each including adjacent two or more each are bonded to each other to form a substituted or unsubstituted "unsaturated ring" containing the plural atoms of the core skeleton and 1 or more and 15 or less at least one kind of an element selected from the group consisting of a carbon element, a nitrogen element, an oxygen element, and a sulfur element, unless otherwise indicated in the description.

In the case where the "monocyclic ring" or the "condensed ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

In the case where the "saturated ring" or the "unsaturated ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

The above are the explanation of the case where "one or more combinations of combinations each including adjacent two or more" each are "bonded to each other to form a substituted or unsubstituted monocyclic ring", and the case where "one or more combinations of combinations each including adjacent two or more" each are "bonded to each other to form a substituted or unsubstituted condensed ring" (i.e., the "case forming a ring by bonding").

### Substituent for "Substituted or Unsubstituted"

In one embodiment in the description herein, the substituent for the case of "substituted or unsubstituted" (which may be hereinafter referred to as an "arbitrary substituent") is, for example, a group selected from the group consisting of
an unsubstituted alkyl group having 1 to 50 carbon atoms,
an unsubstituted alkenyl group having 2 to 50 carbon atoms,
an unsubstituted alkynyl group having 2 to 50 carbon atoms,
an unsubstituted cycloalkyl group having 3 to 50 ring carbon atoms,

   -Si(R₉₀₁)(R₉₀₂)(R₉₀₃),

   -O-(R₉₀₄),

   -S-(R₉₀₅),

   -N(R₉₀₆)(R₉₀₇),
a halogen atom, a cyano group, a nitro group,
an unsubstituted aryl group having 6 to 50 ring carbon atoms, and
an unsubstituted heterocyclic group having 5 to 50 ring atoms,
wherein R₉₀₁ to R₉₀₇ each independently represent
a hydrogen atom,
a substituted or unsubstituted alkyl group having 1 to 50 carbon atoms
a substituted or unsubstituted cycloalkyl group having 3 to 50 ring carbon atoms,
a substituted or unsubstituted aryl group having 6 to 50 ring carbon atoms, or
a substituted or unsubstituted heterocyclic group having 5 to 50 ring atoms.

In the case where two or more groups each represented by R₉₀₁ exist, the two or more groups each represented by R₉₀₁ are the same as or different from each other,
in the case where two or more groups each represented by R₉₀₂ exist, the two or more groups each represented by R₉₀₂ are the same as or different from each other,
in the case where two or more groups each represented by R₉₀₃ exist, the two or more groups each represented by R₉₀₃ are the same as or different from each other,
in the case where two or more groups each represented by R₉₀₄ exist, the two or more groups each represented by R₉₀₄ are the same as or different from each other,
in the case where two or more groups each represented by R₉₀₅ exist, the two or more groups each represented by R₉₀₅ are the same as or different from each other,
in the case where two or more groups each represented by R₉₀₆ exist, the two or more groups each represented by R₉₀₆ are the same as or different from each other, and
in the case where two or more groups each represented by R₉₀₇ exist, the two or more groups each represented by R₉₀₇ are the same as or different from each other.

In one embodiment, the substituent for the case of "substituted or unsubstituted" may be a group selected from the group consisting of
an alkyl group having 1 to 50 carbon atoms,
an aryl group having 6 to 50 ring carbon atoms, and
a heterocyclic group having 5 to 50 ring atoms.

In one embodiment, the substituent for the case of "substituted or unsubstituted" may be a group selected from the group consisting of
an alkyl group having 1 to 18 carbon atoms,
an aryl group having 6 to 18 ring carbon atoms, and
a heterocyclic group having 5 to 18 ring atoms.

The specific examples of the groups for the arbitrary substituent described above are the specific examples of the substituent described in the section "Substituents in Description" described above.

In the description herein, the arbitrary adjacent substituents may form a "saturated ring" or an "unsaturated ring", preferably form a substituted or unsubstituted saturated 5-membered ring, a substituted or unsubstituted saturated 6-membered ring, a substituted or unsubstituted unsaturated 5-membered ring, or a substituted or unsubstituted unsaturated 6-membered ring, and more preferably form a benzene ring, unless otherwise indicated.

In the description herein, the arbitrary substituent may further have a substituent unless otherwise indicated in the description. The definition of the substituent that the arbitrary substituent further has may be the same as the arbitrary substituent.

In the description herein, a numerical range shown by "AA to BB" means a range including the numerical value AA as the former of "AA to BB" as the lower limit value and the numerical value BB as the latter of "A A to BB" as the upper limit value.

The compound of the present invention will be described below.

The compound of the present invention is represented by the formula (1). Hereinafter, signs in the formula (1) and each formula, which will be described later, included in the formula (1) will be described. Unless otherwise specified, the same signs have the same meaning.

The compound of the present invention represented by the formula (1) and each formula, which will be described later, included in the formula (1) is sometimes referred to as "the inventive compound".

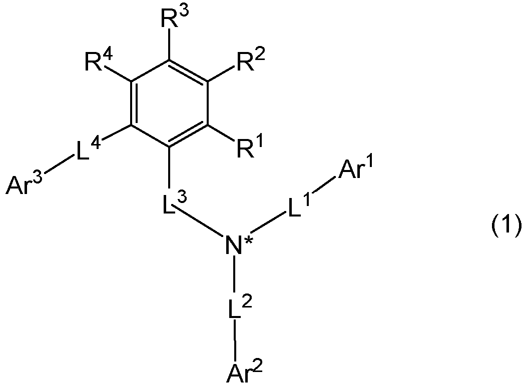

N* is a central nitrogen atom.

R¹ to R⁴ are each independently a hydrogen atom, an unsubstituted alkyl group having 1 to 6 carbon atoms, or an unsubstituted aryl group having 6 to 12 ring carbon atoms.

Adjacent two selected from R¹ to R⁴ are not bonded to each other, thus forming no ring.

The unsubstituted alkyl group having 1 to 6 carbon atoms represented by R¹ to R⁴ is, for example, a methyl group, an ethyl group, an n-propyl group, an isopropyl group, an n-butyl group, an isobutyl group, an s-butyl group, a t-butyl group, a pentyl group, or a hexyl group, preferably a methyl group, an ethyl group, an n-propyl group, an isopropyl group, an n-butyl group, an isobutyl group, an s-butyl group, or a t-butyl group, and more preferably a methyl group or a t-butyl group.

The unsubstituted aryl group having 6 to 12 ring carbon atoms represented by R¹ to R⁴ is, for example, a phenyl group, a biphenylyl group, a biphenylenyl group, or a naphthyl group, preferably a phenyl group, a 2-, 3-, or 4-biphenylyl group, or a 1- or 2-naphthyl group, and more preferably a phenyl group.

All of R¹ to R⁴ may be a hydrogen atom.

L¹ to L⁴ are each independently a single bond, an unsubstituted arylene group having 6 to 30, preferably 6 to 25, more preferably 6 to 12 ring carbon atoms, or an unsubstituted divalent heterocyclic group having 5 to 30, preferably 5 to 18, more preferably 5 to 13 ring atoms.

L¹ to L⁴ are each independently preferably a single bond, or an unsubstituted arylene group having 6 to 30 ring carbon atoms.

In an aspect of the present invention, L³ is preferably a single bond.

In another aspect of the present invention, L⁴ is preferably a single bond.

The unsubstituted arylene group having 6 to 30 ring carbon atoms represented by L¹ to L⁴ refers to a divalent group obtained by removing one hydrogen atom from an unsubstituted aryl group having 6 to 30 ring carbon atoms. The unsubstituted aryl group having 6 to 30 ring carbon atoms is, for example, a phenyl group, a biphenylyl group, a terphenylyl group, a biphenylenyl group, a naphthyl group, an anthryl group, a benzanthryl group, a phenanthryl group, a benzophenanthryl group, a phenalenyl group, a picenyl group, a pentaphenyl group, a pyrenyl group, a chrysenyl group, a benzocrysenyl group , a fluorenyl group, a fluoranthenyl group, a perylenyl group, or a triphenylenyl group, preferably a phenyl group, a biphenylyl group, a terphenylyl group, or a naphthyl group, more preferably a phenyl group, a 2-, 3-, or 4-biphenylyl group, a 2-, 3-, or 4-o-terphenylyl group, a 2-, 3-, or 4-m-terphenylyl group, a 2-, 3-, or 4-p-terphenylyl group, or a 1- or 2-naphthyl group, still more preferably a phenyl group, a 2-, 3-, or 4-biphenylyl group, or a 1- or 2-naphthyl group, and particularly preferably a phenyl group.

The unsubstituted divalent heterocyclic group having 5 to 30 ring atoms represented by L¹ to L⁴ refers to a divalent group obtained by removing one hydrogen atom from an unsubstituted heterocyclic group.

The unsubstituted aromatic heterocyclic group having 5 to 30 ring atoms represented by L¹ to L⁴ is, for example, a pyrrolyl group, a furyl group, a thienyl group, a pyridyl group, an imidazopyridyl group, a pyridazinyl group, a pyrimidinyl group, a pyrazinyl group, a triazinyl group, an imidazolyl group, an oxazolyl group, a thiazolyl group, a pyrazolyl group, an isoxazolyl group, an isothiazolyl group, an oxadiazolyl group, a thiadiazolyl group, a triazolyl group, a tetrazolyl group, an indolyl group, an isoindolyl group, an indolizinyl group, a quinolidinyl group, a quinolyl group, an isoquinolyl group, a cinnolyl group, a phthalazinyl group, a quinazolinyl group, a quinoxalinyl group, a benzimidazolyl group, a benzoxazolyl group, a benzothiazolyl group, an indazolyl group, a benzisoxazolyl group, a benzisothiazolyl group, a phenanthridinyl group, an acridinyl group, a phenanthrolinyl group, a phenazinyl group, a phenothiazinyl group, a phenoxazinyl group, a xanthenyl group, a benzofuranyl group, an isobenzofuranyl group, a naphthobenzofuranyl group, a dibenzofuranyl group, a benzothiophenyl group (benzothienyl group, hereinafter the same), an isobenzothiophenyl group (isobenzothienyl group, hereinafter the same), a naphthobenzothiophenyl group (naphthobenzothienyl group, hereinafter the same), a dibenzothiophenyl group (dibenzothienyl group, hereinafter the same), or a carbazolyl group, and preferably a benzofuranyl group, an isobenzofuranyl group, a naphthobenzofuranyl group, a dibenzofuranyl group, a benzothiophenyl group, an isobenzothiophenyl group, a naphthobenzothiophenyl group, a dibenzothiophenyl group, or a carbazolyl group (9-carbazolyl group, or a 1-, 2-, 3- or 4-carbazolyl group).

Ar¹ and Ar² are each independently a substituted or unsubstituted aryl group having 6 to 30, preferably 6 to 25, more preferably 6 to 12 ring carbon atoms constituted only of 6-membered rings, a substituted or unsubstituted heterocyclic group having 5 to 30, preferably 5 to 18, more preferably 5 to 13 ring atoms, or a group represented by the following formula (a).

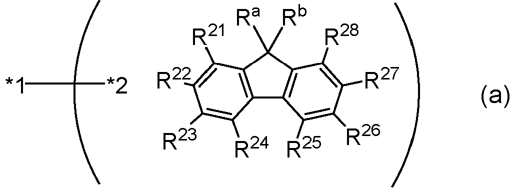

In the formula (a), *1 is a bonding site to L¹ or L².

R^{a} and R^{b} are each independently a hydrogen atom, a substituted or unsubstituted alkyl group having 1 to 30, preferably 1 to 18, more preferably 1 to 6 carbon atoms, a substituted or unsubstituted aryl group having 6 to 30, preferably 6 to 25, more preferably 6 to 12 ring carbon atoms, or a substituted or unsubstituted heterocyclic group having 5 to 30, preferably 5 to 18, more preferably 5 to 13 ring atoms.

R^{a} and R^{b} may be bonded to each other to form a substituted or unsubstituted ring, or may not be bonded to each other, thus forming no ring.

R^{a} and R^{b} are each independently preferably a substituted or unsubstituted alkyl group having 1 to 30 carbon atoms, or a substituted or unsubstituted aryl group having 6 to 30 ring carbon atoms.

The unsubstituted alkyl group having 1 to 30 carbon atoms represented by R^{a} and R^{b} is, for example, a methyl group, an ethyl group, an n-propyl group, an isopropyl group, an n-butyl group, an isobutyl group, an s-butyl group, a t-butyl group, a pentyl group, a hexyl group, a heptyl group, an octyl group, a nonyl group, a decyl group, an undecyl group, or a dodecyl group, preferably a methyl group, an ethyl group, an n-propyl group, an isopropyl group, an n-butyl group, an isobutyl group, an s-butyl group, a t-butyl group, or a pentyl group, more preferably a methyl group, an ethyl group, an n-propyl group, an isopropyl group, an n-butyl group, an isobutyl group, an s-butyl group, or a t-butyl group, and still more preferably a methyl group or a t-butyl group.

Details of the unsubstituted aryl group having 6 to 30 ring carbon atoms represented by R^{a} and R^{b} are as described for L¹ to L⁴.

Details of the unsubstituted heterocyclic group having 5 to 30 ring atoms represented by R^{a} and R^{b} are as described for L¹ to L⁴.

The unsubstituted monocyclic ring formed by R^{a} and R^{b} is, for example, a benzene ring, a cyclopentane ring, or a cyclohexane ring.

The unsubstituted condensed ring formed by R^{a} and R^{b} is, for example, a naphthalene ring or an anthracene ring.

In addition, when R^{a} and R^{b} are bonded to each other to form an unsubstituted monocyclic ring or an unsubstituted condensed ring, R^{a} and R^{b} may form a ring with a fluorene skeleton to which they are bonded to form a spiro ring. The spiro ring is a hydrocarbon ring or a heterocyclic ring, and is selected from a monocyclic ring, a condensed ring, a bridged bicyclo ring, and a bridged tricyclo ring. Although examples of substituted or unsubstituted spiro rings are shown below, the invention is not limited thereto. * indicates a bonding site of the fluorene skeleton to the benzene ring.

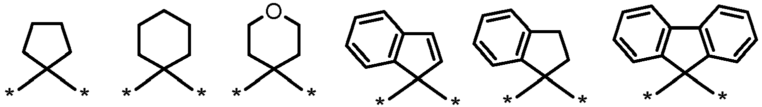

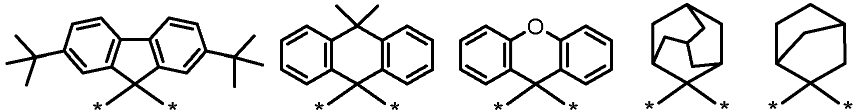

One selected from R²¹ to R²⁸ is a single bond bonded to *2, and R²¹ to R²⁸ that are not a single bond bonded to *2 are each independently a hydrogen atom, a substituted or unsubstituted alkyl group having 1 to 6 carbon atoms, a substituted or unsubstituted aryl group having 6 to 12 ring carbon atoms, or a substituted or unsubstituted heterocyclic group having 5 to 13 ring atoms,
adjacent two selected from R²¹ to R²⁸ that are not the single bond may be bonded to each other to form one or more unsubstituted benzene rings, or may not be bonded to each other, thus forming no ring.

Details of the unsubstituted alkyl group having 1 to 6 carbon atoms represented by R²¹ to R²⁸ that is not the single bond bonded to *2 are as described for R¹ to R⁴.

Details of the unsubstituted aryl group having 6 to 12 ring carbon atoms represented by R²¹ to R²⁸ that is not the single bond bonded to *2 are as described for L¹ to L⁴ except that the number of ring carbon atoms is 6 to 12.

Details of the unsubstituted heterocyclic group having 5 to 13 ring atoms represented by R²¹ to R²⁸ that is not the single bond bonded to *2 are as described for L¹ to L⁴ except that the number of ring atoms is 5 to 13.

When R^{a} and R^{b} are bonded to each other to form a substituted or unsubstituted spirofluorene ring with a carbon atom at site 9 of a fluorene skeleton, one selected from R²² to R²⁷ is a single bond bonded to *2,
when all of L¹ to L⁴ are single bonds, Ar¹ and Ar² are groups represented by the formula (a), and R²² or R²⁷ is a single bond bonded to *2, at least one selected from two R^{a}'s and two R^{b}'s is a substituted or unsubstituted aryl group having 6 to 30 ring carbon atoms.

All of R²¹ to R²⁸ that are not a single bond bonded to *2 may be a hydrogen atom.

Details of the unsubstituted aryl group having 6 to 30 ring carbon atoms constituted only of 6-membered rings represented by Ar¹ and Ar² are as described for L¹ to L⁴ except that it is an aryl group constituted only of 6-membered rings.

Details of the unsubstituted heterocyclic group having 5 to 30 ring atoms represented by Ar¹ and Ar² are as described for L¹ to L⁴.

Ar¹ and Ar² are each independently preferably a substituted or unsubstituted aryl group having 6 to 30 ring carbon atoms constituted only of 6-membered rings, or a substituted or unsubstituted heterocyclic group having 5 to 30 ring atoms, and more preferably a substituted or unsubstituted aryl group having 6 to 30 ring carbon atoms constituted only of 6-membered rings.

Ar¹ and Ar² are each independently preferably a group represented by any of the following formulas (1a) to (1e), and more preferably a group represented by any of the following formulas (1a), (1b) and (1d).

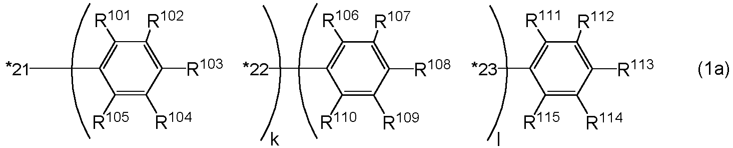

In the formula (1a),
*21 is a bonding site to L¹ or L².

R¹⁰¹ to R¹¹⁰ are each independently a hydrogen atom, an unsubstituted alkyl group having 1 to 6 carbon atoms, or an unsubstituted aryl group having 6 to 12 ring carbon atoms, provided that one selected from R¹⁰¹ to R¹⁰⁵ is a single bond bonded to *22, and one selected from R¹⁰⁶ to R¹¹⁰ is a single bond bonded to *23.

Adjacent two selected from R¹⁰¹ to R¹⁰⁵ that are not the single bond are not bonded to each other, thus forming no ring,
adjacent two selected from R¹⁰⁶ to R¹¹⁰ that are not the single bond are not bonded to each other, thus forming no ring.

Details of the unsubstituted alkyl group having 1 to 6 carbon atoms represented by R¹⁰¹ to R¹¹⁰ are as described for R¹ to R⁴.

Details of the unsubstituted aryl group having 6 to 12 ring carbon atoms represented by R¹⁰¹ to R¹¹⁰ are as described for L¹ to L⁴ except that the number of ring carbon atoms is 6 to 12.

All of R¹⁰¹ to R¹¹⁰ that are not a single bond bonded to *22 or a single bond bonded to *23 may be a hydrogen atom.

k is 0 or 1, 1 is 0 or 1,
k + l is an integer of 0 to 2.

In one aspect of the present invention, k is 0 and l is 0. In this case, *23 represents *21, and the formula (1a) is represented by the following formula.

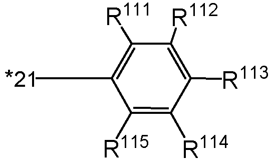

In another aspect of the present invention, k is 1 and l is 0. In this case, *23 represents *22, and the formula (1a) is represented by the following formula.

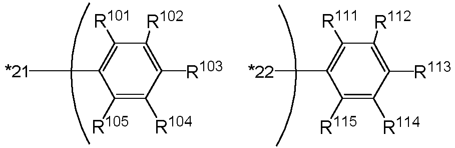

In another aspect of the present invention, k is 0 and l is 1. In this case, *22 represents *21, and the formula (1a) is represented by the following formula.

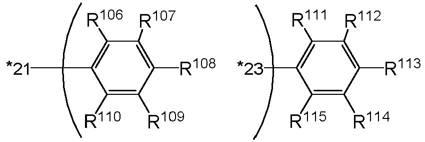

In another aspect of the present invention, k is 1 and l is 1. In this case, the formula (1a) is represented by the following formula.

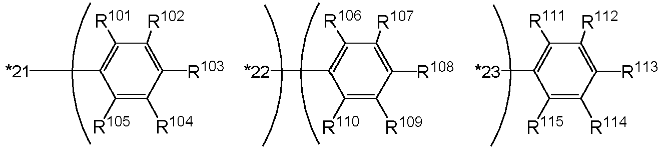

In one aspect of the present invention, k + l is preferably 1.

R¹¹¹ to R¹¹⁵ are each independently a hydrogen atom, a substituted or unsubstituted alkyl group having 1 to 6 carbon atoms, a substituted or unsubstituted aryl group having 6 to 12 ring carbon atoms, or a substituted or unsubstituted heterocyclic group having 5 to 13 ring atoms.

Adjacent two selected from R¹¹¹ to R¹¹⁵ may be bonded to each other to form one or more unsubstituted benzene rings, or may not be bonded to each other, thus forming no ring.

Details of the unsubstituted alkyl group having 1 to 6 carbon atoms represented by R¹¹¹ to R¹¹⁵ are as described for R¹ to R⁴.

Details of the unsubstituted aryl group having 6 to 12 ring carbon atoms represented by R¹¹¹ to R¹¹⁵ are as described for L¹ to L⁴ except that the number of ring carbon atoms is 6 to 12.

Details of the unsubstituted heterocyclic group having 5 to 13 ring atoms represented by R¹¹¹ to R¹¹⁵ are as described for L¹ to L⁴ except that the number of ring atoms is 5 to 13.

All of R¹¹¹ to R¹¹⁵ may be a hydrogen atom.

A group represented by the formula (1a) is preferably represented by the following formula. In the following formula, R is omitted for simplification.

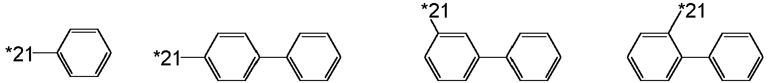

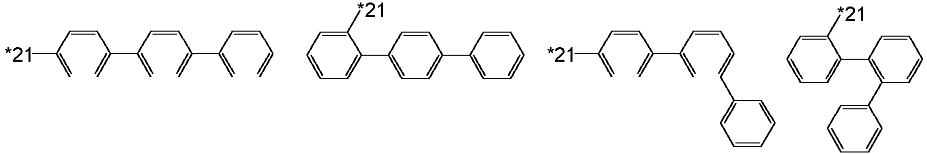

The formula (1b) is represented by the following formula.

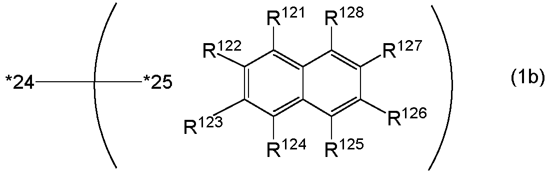

In the formula (1b),
*24 is a bonding site to L¹ or L².

R¹²¹ to R¹²⁸ are each independently a hydrogen atom, a substituted or unsubstituted alkyl group having 1 to 6 carbon atoms, or a substituted or unsubstituted aryl group having 6 to 12 ring carbon atoms,
provided that one selected from R¹²¹ to R¹²⁸ is a single bond bonded to *25, and adjacent two selected from R¹²¹ to R¹²⁸ that are not the single bond are not bonded to each other, thus forming no ring.

Details of the unsubstituted alkyl group having 1 to 6 carbon atoms represented by R¹²¹ to R¹²⁸ are as described for R¹ to R⁴.

Details of the unsubstituted aryl group having 6 to 12 ring carbon atoms represented by R¹²¹ to R¹²⁸ are as described for L¹ to L⁴ except that the number of ring carbon atoms is 6 to 12.

In one aspect of the present invention, R¹²¹ is preferably a single bond bonded to *25, and in another aspect, R¹²² is preferably a single bond bonded to *25.

All of R¹²¹ to R¹²⁸ that are not a single bond bonded to *25 may be a hydrogen atom.

The formula (1c) is represented by the following formula.

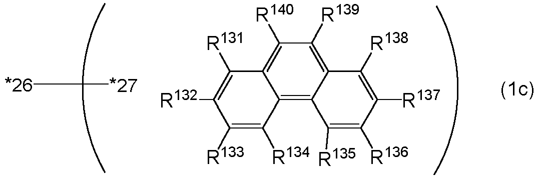

In the formula (1c),
*26 is a bonding site to L¹ or L².

R¹³¹ to R¹⁴⁰ are each independently a hydrogen atom, a substituted or unsubstituted alkyl group having 1 to 6 carbon atoms, or a substituted or unsubstituted aryl group having 6 to 12 ring carbon atoms,
provided that one selected from R¹³¹ to R¹⁴⁰ is a single bond bonded to *27, and adjacent two selected from R¹³¹ to R¹⁴⁰ that are not the single bond are not bonded to each other, thus forming no ring.

Details of the unsubstituted alkyl group having 1 to 6 carbon atoms represented by R¹³¹ to R¹⁴⁰ are as described for R¹ to R⁴.

Details of the unsubstituted aryl group having 6 to 12 ring carbon atoms represented by R¹³¹ to R¹⁴⁰ are as described for L¹ to L⁴ except that the number of ring carbon atoms is 6 to 12.

One selected from R¹³¹, R¹³², and R¹⁴⁰ is preferably a single bond bonded to *27. In one aspect of the present invention, R¹³¹ is a single bond bonded to *27, in another aspect, R¹³² is a single bond bonded to *27, and in still another aspect, R¹⁴⁰ is a single bond bonded to *27.

All of R¹³¹ to R¹⁴⁰ that are not a single bond bonded to *27 may be a hydrogen atom.

The formula (1d) is represented by the following formula.

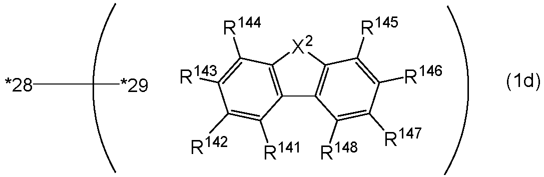

In the formula (1d),
*26 is a bonding site to L¹ or L².

X² is an oxygen atom, a sulfur atom, or NR^{A}.

X² is preferably an oxygen atom or NR^{A}.

R^{A} is a substituted or unsubstituted alkyl group having 1 to 6 carbon atoms or a substituted or unsubstituted aryl group having 6 to 12 ring carbon atoms.

Details of the unsubstituted alkyl group having 1 to 6 carbon atoms represented by R^{A} are as described for R¹ to R⁴.

Details of the unsubstituted aryl group having 6 to 12 ring carbon atoms represented by R^{A} are as described for L¹ to L⁴ except that the number of ring carbon atoms is 6 to 12.

R¹⁴¹ to R¹⁴⁸ are each independently a hydrogen atom, a substituted or unsubstituted alkyl group having 1 to 6 carbon atoms, a substituted or unsubstituted aryl group having 6 to 12 ring carbon atoms, or a substituted or unsubstituted heterocyclic group having 5 to 13 ring atoms, provided that one selected from R¹⁴¹ to R¹⁴⁸ and R^{A} is a single bond bonded to *29.

At least one adjacent two selected from R¹⁴¹ to R¹⁴⁸ that are not the single bond may be bonded to each other to form one or more unsubstituted benzene rings, or may not be bonded to each other, thus forming no ring.

Details of the unsubstituted alkyl group having 1 to 6 carbon atoms represented by R¹⁴¹ to R¹⁴⁸ are as described for R¹ to R⁴.

Details of the unsubstituted aryl group having 6 to 12 ring carbon atoms represented by R¹⁴¹ to R¹⁴⁸ are as described for L¹ to L⁴ except that the number of ring carbon atoms is 6 to 12.

Details of the unsubstituted heterocyclic group having 5 to 13 ring atoms represented by R¹⁴¹ to R¹⁴⁸ are as described for L¹ to L⁴ except that the number of ring atoms is 5 to 13.

All of R¹⁴¹ to R¹⁴⁸ and R^{A} that are not a single bond bonded to *29 may be a hydrogen atom.

When X² is an oxygen atom or a sulfur atom, it is preferable that one selected from R¹⁴¹ to R¹⁴⁴ is a single bond bonded to *29.

When X² is NR^{A}, it is preferable that one selected from R¹⁴¹ to R¹⁴⁴ and R^{A} is a single bond bonded to *29.

It is particularly preferable that R^{A} is a single bond bonded to *29, an unsubstituted phenyl group, or a naphthyl group.

The formula (1e) is represented by the following formula.

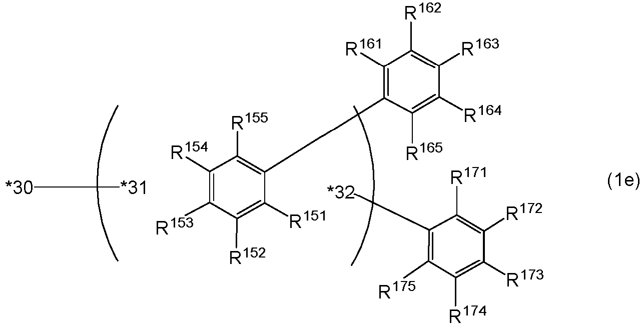

In the formula (1e),
*30 is a bonding site to L¹ or L².

R¹⁵¹ to R¹⁵⁵ are each independently a hydrogen atom, an unsubstituted alkyl group having 1 to 6 carbon atoms, or an unsubstituted phenyl group,
provided that one selected from R¹⁵¹ to R¹⁵⁵ is a single bond bonded to *31, and another one selected from R¹⁵¹ to R¹⁵⁵ is a single bond bonded to *32.

Adjacent two selected from R¹⁵¹ to R¹⁵⁵ that are neither a single bond bonded to *31 nor a single bond bonded to *32 are not bonded to each other, thus forming no ring.

Details of the unsubstituted alkyl group having 1 to 6 carbon atoms represented by R¹⁵¹ to R¹⁵⁵ are as described for R¹ to R⁴.

All of R¹⁵¹ to R¹⁵⁵ that are neither a single bond bonded to *31 nor a single bond bonded to *32 may be a hydrogen atom.

R¹⁶¹ to R¹⁶⁵ and R¹⁷¹ to R¹⁷⁵ are each independently a hydrogen atom or an unsubstituted alkyl group having 1 to 6 carbon atoms,
provided that at least one adjacent two selected from R¹⁶¹ to R¹⁶⁵ may be bonded to each other to form one or more unsubstituted benzene rings, or may not be bonded to each other, thus forming no ring.

At least one adjacent two selected from R¹⁷¹ to R¹⁷⁵ may be bonded to each other to form one or more unsubstituted benzene rings, or may not be bonded to each other, thus forming no ring.

Details of the unsubstituted alkyl group having 1 to 6 carbon atoms represented by R¹⁶¹ to R¹⁶⁵ and R¹⁷¹ to R¹⁷⁵ are as described for R¹ to R⁴.

All of R¹⁶¹ to R¹⁶⁵ and R¹⁷¹ to R¹⁷⁵ may be a hydrogen atom.

The formula (1e) includes groups represented by the following formulas (1e-1) to (1e-5), with the formulas (1e-1), (1e-2) or (1e-4) being preferred.

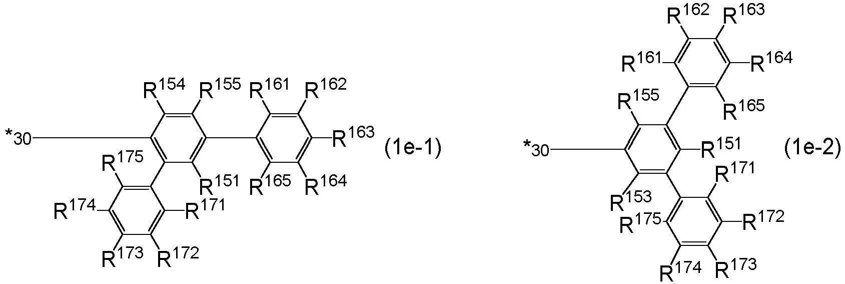

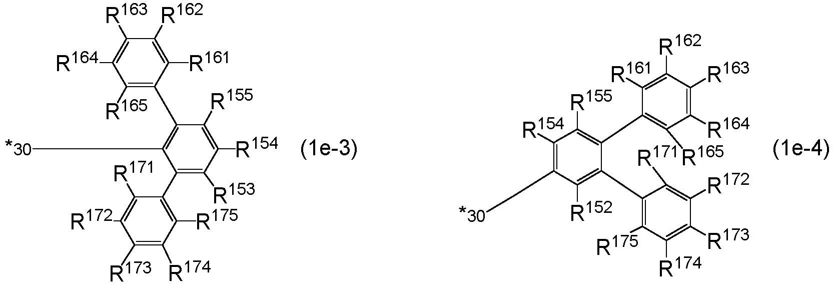

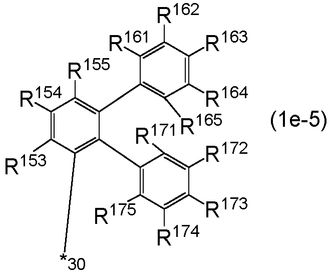

Ar³ is a group represented by the following formula (x).

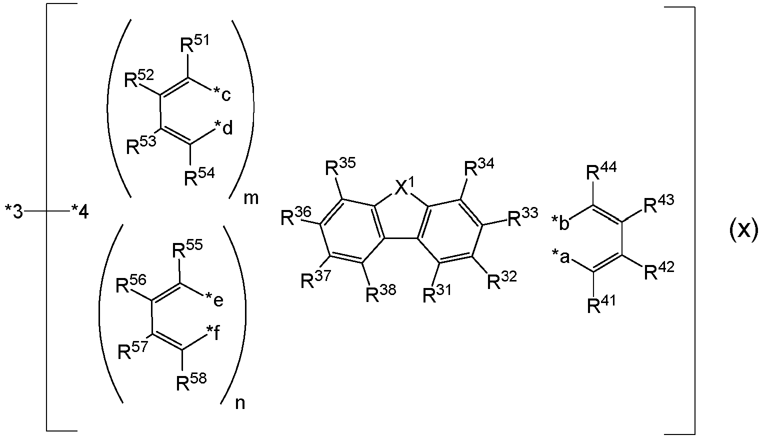

In the formula (x),
*3 is a bonding site to L⁴.

R³¹ to R³⁸, R⁴¹ to R⁴⁴, and R⁵¹ to R⁵⁸ are each independently a hydrogen atom, a halogen atom, a cyano group, a nitro group, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by - Si(R₉₀₁)(R₉₀₂)(R₉₀₃), a group represented by -O-(R₉₀₄), a group represented by -S-(R₉₀₅), a group represented by -N(R₉₀₆)(R₉₀₇), a substituted or unsubstituted aryl group having 6 to 50 ring carbon atoms, or a substituted or unsubstituted heterocyclic group having 5 to 50 ring atoms,

R⁹⁰¹ to R⁹⁰⁷ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms,
in the case where two or more groups represented by R₉₀₁ exist, the two or more groups each represented by R₉₀₁ are the same as or different from each other,
in the case where two or more groups represented by R₉₀₂ exist, the two or more groups each represented by R₉₀₂ are the same as or different from each other,
in the case where two or more groups represented by R₉₀₃ exist, the two or more groups each represented by R₉₀₃ are the same as or different from each other,
in the case where two or more groups represented by R₉₀₄ exist, the two or more groups each represented by R₉₀₄ are the same as or different from each other,
in the case where two or more groups represented by R₉₀₅ exist, the two or more groups each represented by R₉₀₅ are the same as or different from each other,
in the case where two or more groups represented by R₉₀₆ exist, the two or more groups each represented by R₉₀₆ are the same as or different from each other,
in the case where two or more groups represented by R₉₀₇ exist, the two or more groups each represented by R₉₀₇ are the same as or different from each other.

R³¹ to R³⁸, R⁴¹ to R⁴⁴, and R⁵¹ to R⁵⁸ are each independently preferably a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms, more preferably a hydrogen atom, a substituted or unsubstituted alkyl group having 1 to 30 carbon atoms, or a substituted or unsubstituted aryl group having 6 to 30 ring carbon atoms, further preferably a hydrogen atom, a substituted or unsubstituted alkyl group having 1 to 12 carbon atoms, or a substituted or unsubstituted aryl group having 6 to 12 ring carbon atoms, and furthermore preferably a hydrogen atom.

Details of the halogen atom represented by R³¹ to R³⁸, R⁴¹ to R⁴⁴, and R⁵¹ to R⁵⁸ are as described in the section "Substituents in Description", and preferred is a fluorine atom.

Details of the substituted or unsubstituted alkyl group having 1 to 50 carbon atoms represented by R³¹ to R³⁸, R⁴¹ to R⁴⁴, and R⁵¹ to R⁵⁸ are as described in the section "Substituents in Description".

The unsubstituted alkyl group represented by R³¹ to R³⁸, R⁴¹ to R⁴⁴, and R⁵¹ to R⁵⁸ is preferably a methyl group, an ethyl group, an n-propyl group, an isopropyl group, an n-butyl group, an isobutyl group, an s-butyl group, or a t-butyl group, more preferably a methyl group, an ethyl group, an isopropyl group, or a t-butyl group, and still more preferably a methyl group or a t-butyl group.

Details of the substituted or unsubstituted alkenyl group having 2 to 50 ring carbon atoms represented by R³¹ to R³⁸, R⁴¹ to R⁴⁴, and R⁵¹ to R⁵⁸ are as described in the section "Substituents in Description".

Details of the substituted or unsubstituted alkynyl group having 2 to 50 ring carbon atoms represented by R³¹ to R³⁸, R⁴¹ to R⁴⁴, and R⁵¹ to R⁵⁸ are as described in the section "Substituents in Description".

Details of the substituted or unsubstituted cycloalkyl group having 3 to 50 ring carbon atoms represented by R³¹ to R³⁸, R⁴¹ to R⁴⁴, and R⁵¹ to R⁵⁸ are as described in the section "Substituents in Description".

The unsubstituted cycloalkyl group represented by R³¹ to R³⁸, R⁴¹ to R⁴⁴, and R⁵¹ to R⁵⁸ is preferably a cyclopentyl group, a cyclohexyl group, a 1-adamantyl group, a 2-adamantyl group, a 1-norbornyl group, or a 2-norbornyl group, and more preferably a cyclopentyl group or a cyclohexyl group.

Details of the -Si(R₉₀₁)(R₉₀₂)(R₉₀₃) represented by R³¹ to R³⁸, R⁴¹ to R⁴⁴, and R⁵¹ to R⁵⁸, the group represented by -O-(R₉₀₄), the group represented by -S-(R₉₀₅), and the group represented by -N(R₉₀₆)(R₉₀₇) are as described in the section "Substituents in Description".

Details of the substituted or unsubstituted aryl group having 6 to 50 ring carbon atoms represented by R³¹ to R³⁸, R⁴¹ to R⁴⁴, and R⁵¹ to R⁵⁸ are as described in the section "Substituents in Description".

The unsubstituted aryl group represented by R¹ to R⁸ and R¹¹ to R¹⁸ is preferably a phenyl group, a biphenyl group, or a phenanthryl group, more preferably a phenyl group, a biphenyl group, or a naphthyl group, and further preferably a phenyl group.

Details of the substituted or unsubstituted heterocyclic group having 5 to 50 ring atoms represented by R³¹ to R³⁸, R⁴¹ to R⁴⁴, and R⁵¹ to R⁵⁸ are as described in the section "Substituents in Description".

The unsubstituted heterocyclic group represented by R³¹ to R³⁸, R⁴¹ to R⁴⁴, and R⁵¹ to R⁵⁸ is preferably a dibenzofuranyl group or a dibenzothiophenyl group.

m is 0 or 1, and n is 0 or 1.

When m and n are 0, one of R³¹ and R³², R³² and R³³, or R³³ and R³⁴ is a single bond bonded to *a, and the other is a single bond bonded to *b,
one selected from R³¹ to R³⁸ that are not a single bond bonded to *a and *b, and R⁴¹ to R⁴⁴ is a single bond bonded to *4.

When m is 1 and n is 0, one of R³¹ and R³², R³² and R³³, or R³³ and R³⁴ is a single bond bonded to *a, and the other is a single bond bonded to *b,
one of R³⁵ and R³⁶, R³⁶ and R³⁷, or R³⁷ and R³⁸ is a single bond bonded to *c, and the other is a single bond bonded to *d,
one selected from R³¹ to R³⁴ that are not a single bond bonded to *a and *b, R³⁵ to R³⁸ that are not a single bond bonded to *c and *d, R⁴¹ to R⁴⁴, and R⁵¹ to R⁵⁴ is a single bond bonded to *4.

When m is 0 and n is 1, one of R³¹ and R³², R³² and R³³, or R³³ and R³⁴ is a single bond bonded to *a, and the other is a single bond bonded to *b,
one of R³⁵ and R³⁶, R³⁶ and R³⁷, or R³⁷ and R³⁸ is a single bond bonded to *e, and the other is a single bond bonded to *f,
one selected from R³¹ to R³⁴ that are not a single bond bonded to *a and *b, R³⁵ to R³⁸ that are not a single bond bonded to *e and *f, R⁴¹ to R⁴⁴, and R⁵⁵ to R⁵⁸ is a single bond bonded to *4.

When m is 1 and n is 1, one of R³¹ and R³², R³² and R³³, or R³³ and R³⁴ is a single bond bonded to *a, and the other is a single bond bonded to *b,
one in any one group of three groups, R³⁵ and R³⁶, R³⁶ and R³⁷, and R³⁷ and R³⁸, is a single bond bonded to *c, and the other is a single bond bonded to *d,
one in any one group of the remaining two groups of R³⁵ and R³⁶, R³⁶ and R³⁷, or R³⁷ and R³⁸, is a single bond bonded to *e, and the other is a single bond bonded to *f,
one selected from R³¹ to R³⁴ that are not a single bond bonded to *a and *b, R³⁵ to R³⁸ that are not a single bond bonded to *c to *f, R⁴¹ to R⁴⁴, and R⁵¹ to R⁵⁸ is a single bond bonded to *4,
provided that when L³ and L⁴ are a single bond, m and n are 0, R³³ is a single bond bonded to *a, and R³⁴ is a single bond bonded to *b, or R³⁴ is a single bond bonded to *a and R³³ is a single bond bonded to *b, one selected from R³¹, R³², R³⁵ to R³⁷, and R⁴¹ to R⁴⁴ is a single bond bonded to *4,
when L³ and L⁴ are a single bond, m and n are 0, R³¹ is a single bond bonded to *a, and R³² is a single bond bonded to *b, one selected from R³³ to R³⁸ and R⁴² to R⁴⁴ is a single bond bonded to *4,
when L³ and L⁴ are a single bond, m and n are 0, R³² is a single bond bonded to *a, and R³¹ is a single bond bonded to *b, one selected from R³³ to R³⁸ and R⁴¹ to R⁴³ is a single bond bonded to *4.

In one aspect of the present invention, it is preferable that m is 0.

In another aspect of the present invention, it is preferable that n is 0.

In still another aspect of the present invention, it is preferable that m is 0 and n is 0.

When m is 0 and n is 1, it is preferable that one selected from R³¹ to R³³ that are not a single bond bonded to *a and *b, and R³⁶ to R³⁸ is a single bond bonded to *4, it is more preferable that one selected from R³¹ and R³² that are not a single bond bonded to *a and *b, and R³⁷ and R³⁸ is a single bond bonded to *4, and it is still more preferable that R³² or R³⁷ that is not a single bond bonded to *a and *b is a single bond bonded to *4.

X¹ is an oxygen atom or a sulfur atom.

X¹ is preferably an oxygen atom.

As described above, a "hydrogen atom" as used herein includes a light hydrogen atom, a deuterium atom, and a tritium atom. Thus, the inventive compound may contain a naturally-derived deuterium atom.

A deuterium atom may also be intentionally introduced in the compound (1) by using a deuterated compound in a part or all of raw material compounds. Thus, in an aspect of the present invention, the compound (1) contains at least one deuterium atom. Specifically, the inventive compound may be a compound represented by the formula (1), at least one of the hydrogen atoms contained in the compound being a deuterium atom.

At least one hydrogen atom selected from the following hydrogen atoms may be a deuterium atom (in the following, "substituted or unsubstituted", the number of carbon atoms, and the number of atoms are omitted):
a hydrogen atom represented by R¹ to R⁴ of the formula (1);
a hydrogen atom thereof when R¹ to R⁴ of the formula (1) are an alkyl group or an aryl group;
a hydrogen atom thereof when L¹ to L⁴ of the formula (1) are an arylene group or a divalent heterocyclic group;
a hydrogen atom thereof when Ar¹ and Ar² of the formula (1) are an aryl group constituted only of 6-membered rings or a heterocyclic group;
a hydrogen atom represented by R^{a} and R^{b} of the formula (a);
a hydrogen atom thereof when R^{a} and R^{b} of the formula (a) are an alkyl group, an aryl group, or a heterocyclic group;
a hydrogen atom represented by R²¹ to R²⁸ that are not a single bond bonded to *2 in the formula (a);
a hydrogen atom thereof when R²¹ to R²⁸ that are not a single bond bonded to *2 in the formula (a) are an alkyl group, an aryl group, or a heterocyclic group;
a hydrogen atom represented by R³¹ to R³⁸, R⁴¹ to R⁴⁴, and R⁵¹ to R⁵⁸ that are not a single bond bonded to *a to *f in the formula (x); and
a hydrogen atom thereof when R³¹ to R³⁸, R⁴¹ to R⁴⁴, and R⁵¹ to R⁵⁸ that are not a single bond bonded to *a to *f in the formula (x) are an alkyl group, an alkenyl group, an alkynyl group, a cycloalkyl group, a group represented by -Si(R₉₀₁)(R₉₀₂)(R₉₀₃), a group represented by -O-(R₉₀₄), a group represented -S-(R₉₀₅), a group represented by -N(R₉₀₆)(R₉₀₇), an aryl group or a heterocyclic group.

The deuteration ratio of the inventive compound depends on the deuteration ratio of the raw material compound used. Even when a raw material having a predetermined deuteration ratio is used, light hydrogen isomers may be contained at a certain naturally-derived ratio. Thus, the aspects of the deuteration ratio of the inventive compound shown below are a ratio obtained by taking a minor amount of naturally-derived isomers into account based on a ratio obtained by simply counting the number of deuterium atoms shown by the chemical formula.

The deuteration ratio of the inventive compound is preferably 1% or more, more preferably 3% or more, further preferably 5% or more, furthermore preferably 10% or more, and still furthermore preferably 50% or more.

The inventive compound may be a mixture containing a deuterated compound and a non-deuterated compound or a mixture of two or more compounds having different deuteration ratios. The deuteration ratio of such a mixture is preferably 1% or more, more preferably 3% or more, further preferably 5% or more, furthermore preferably 10% or more, still furthermore preferably 50% or more, and less than 100%.

The ratio of the number of the deuterium atoms based on the number of all the hydrogen atoms in the inventive compound is preferably 1% or more, more preferably 3% or more, further preferably 5% or more, furthermore preferably 10% or more, and 100% or less.

When a "substituted or unsubstituted XX group" included in the definitions of the above formulas is a substituted XX group, details of the substituent are as described in "Substituents for 'Substituted or Unsubstituted"', and is preferably an alkyl group having 1 to 6 carbon atoms, an aryl group having 6 to 12 ring carbon atoms, or an aromatic heterocyclic group having 5 to 13 ring atoms, and more preferably an alkyl group having 1 to 6 carbon atoms or an aryl group having 6 to 12 ring carbon atoms. Details of each group are as described above.

The inventive compound can be easily produced by a person skilled in the art with reference to synthetic examples described below and known synthetic methods.

Specific examples of the inventive compound will be shown below, but were not limited to the exemplified compounds.

In the following specific examples, D represents a deuterium atom.

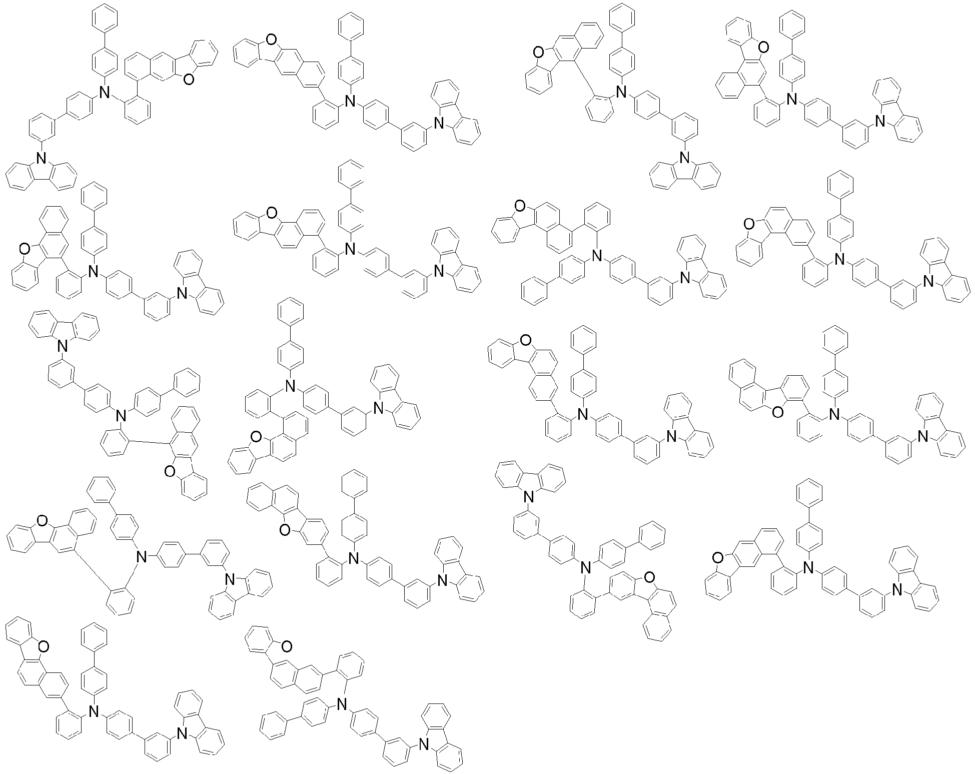

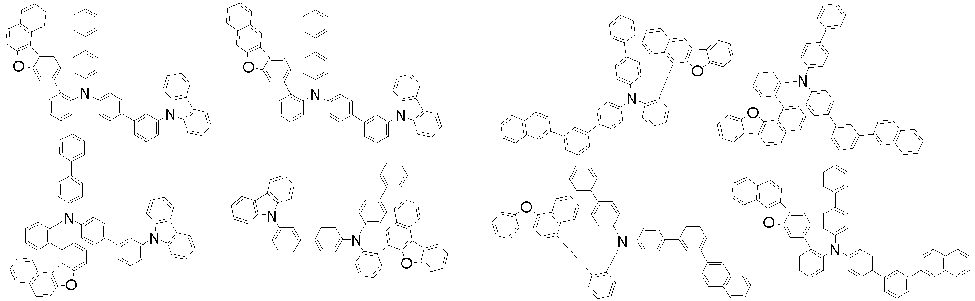

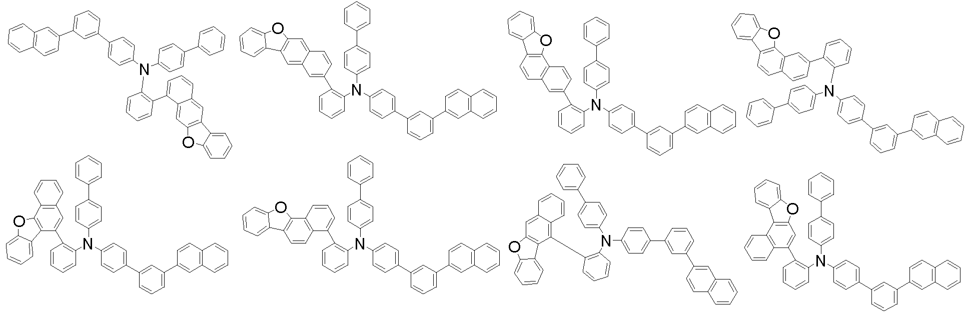

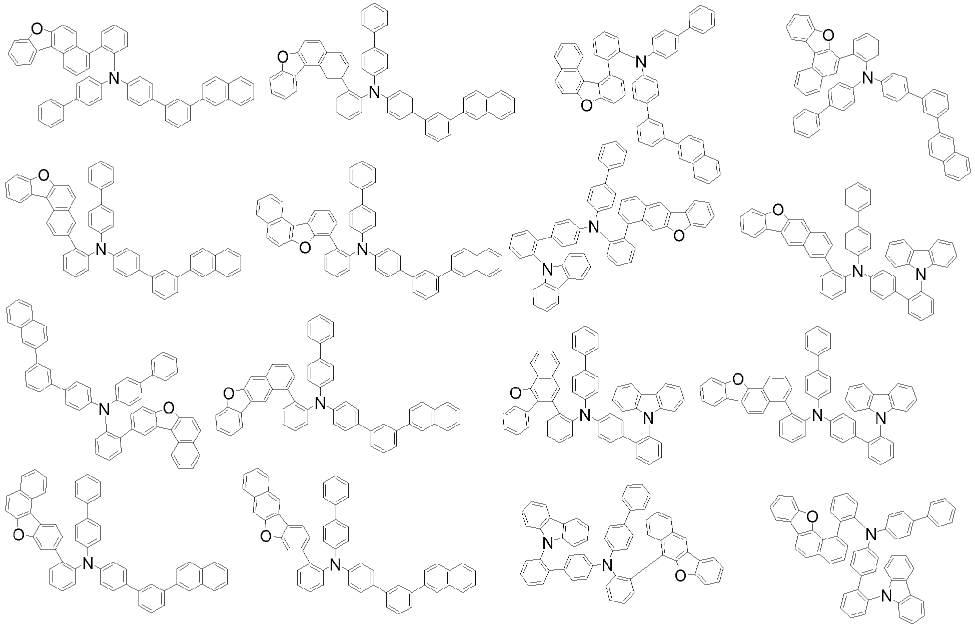

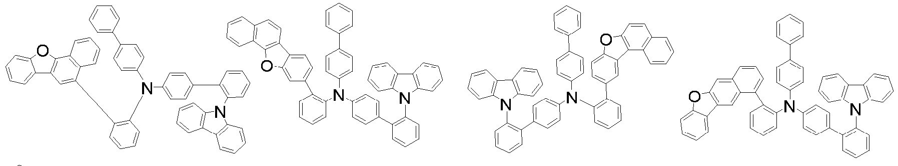

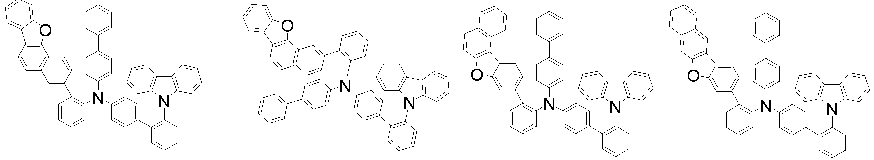

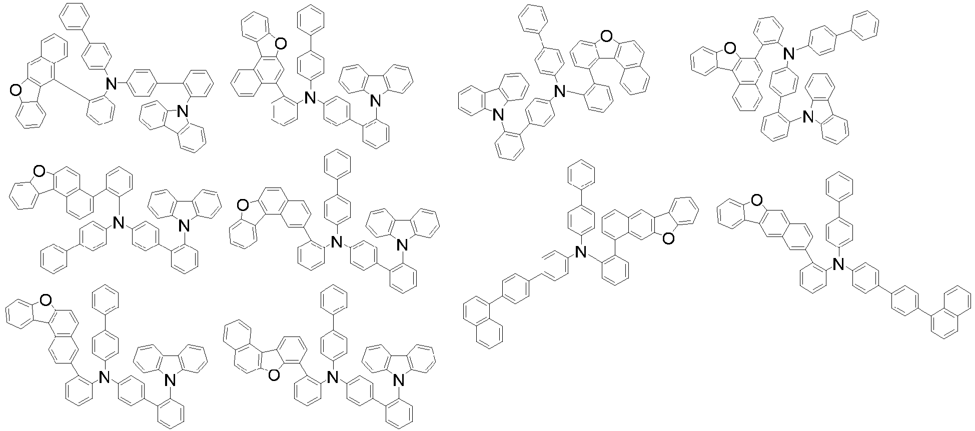

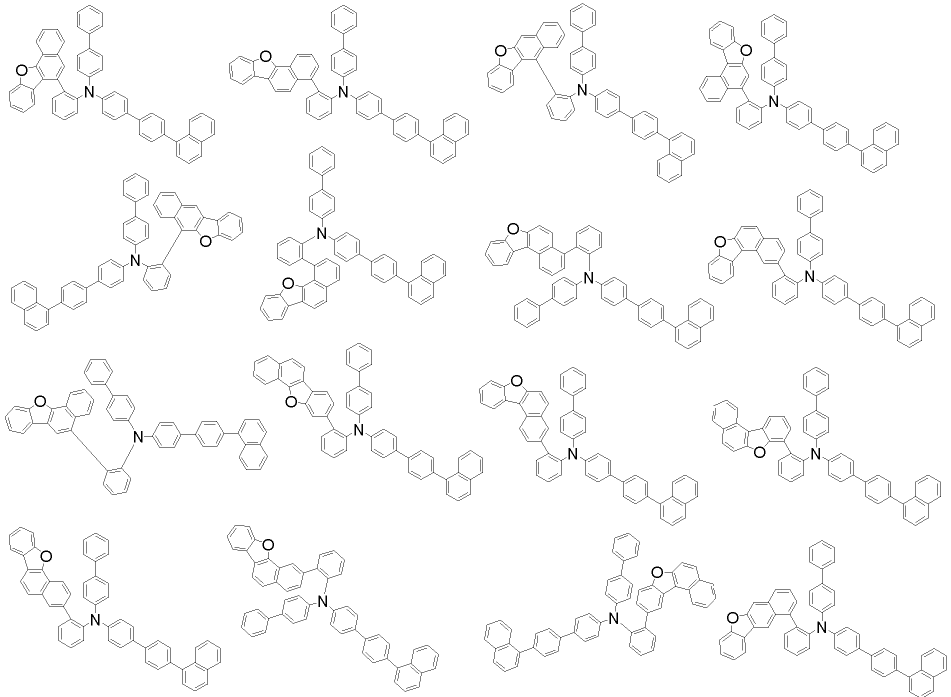

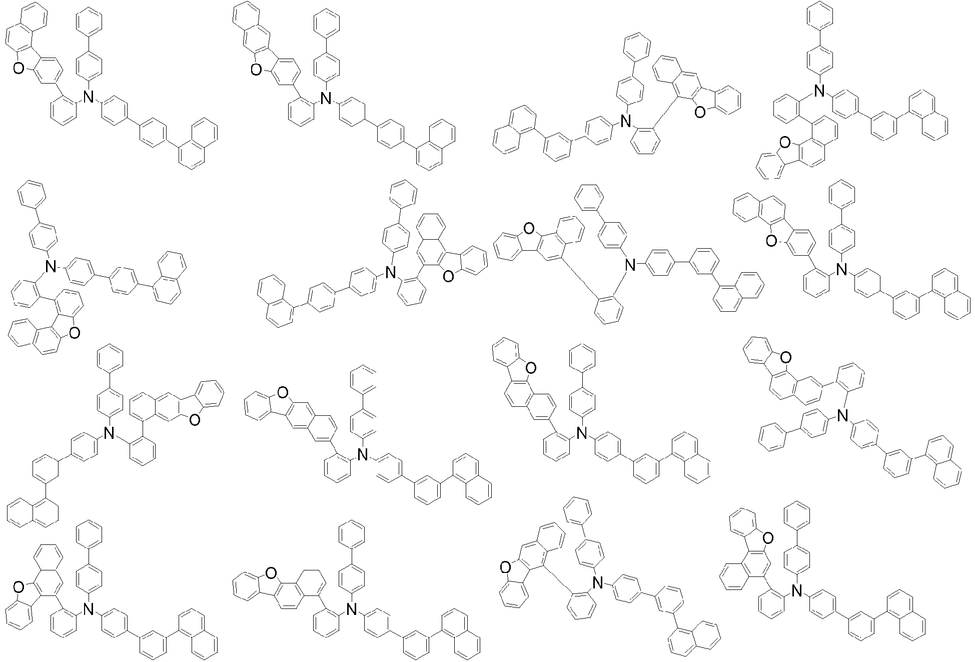

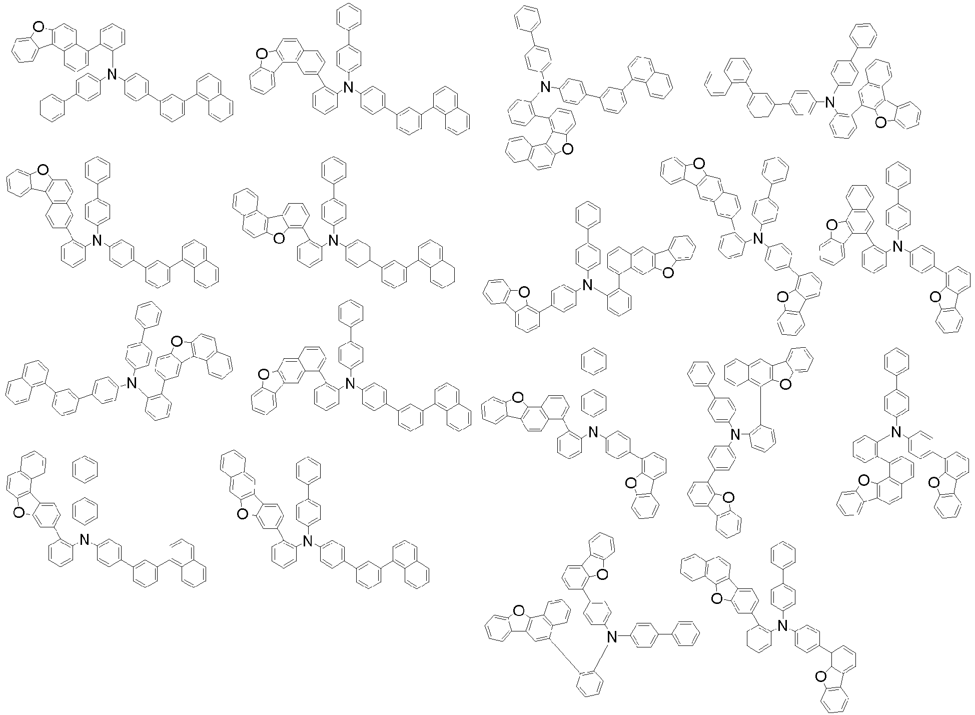

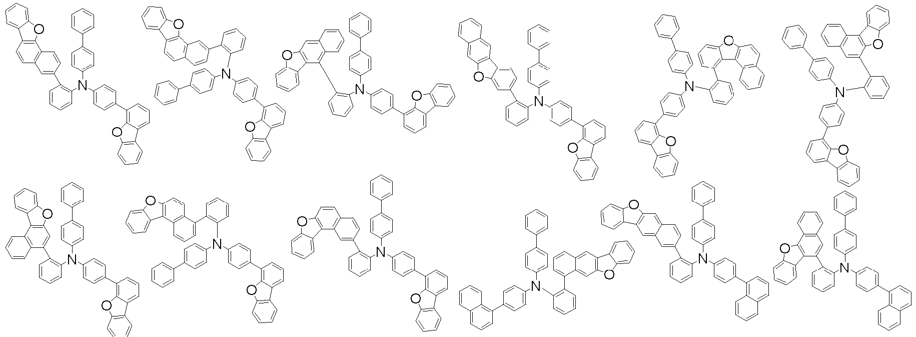

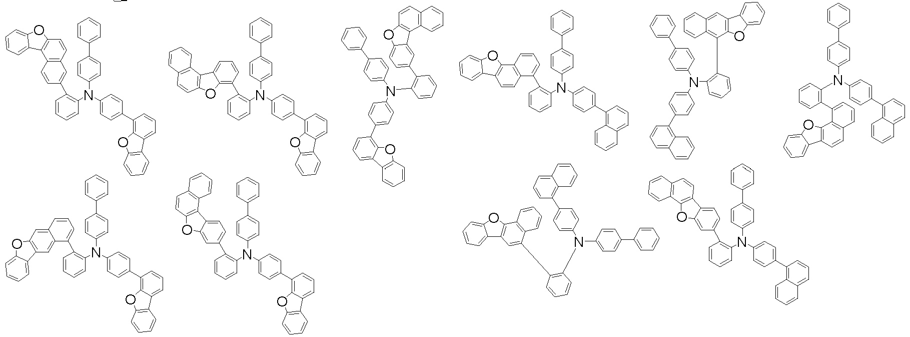

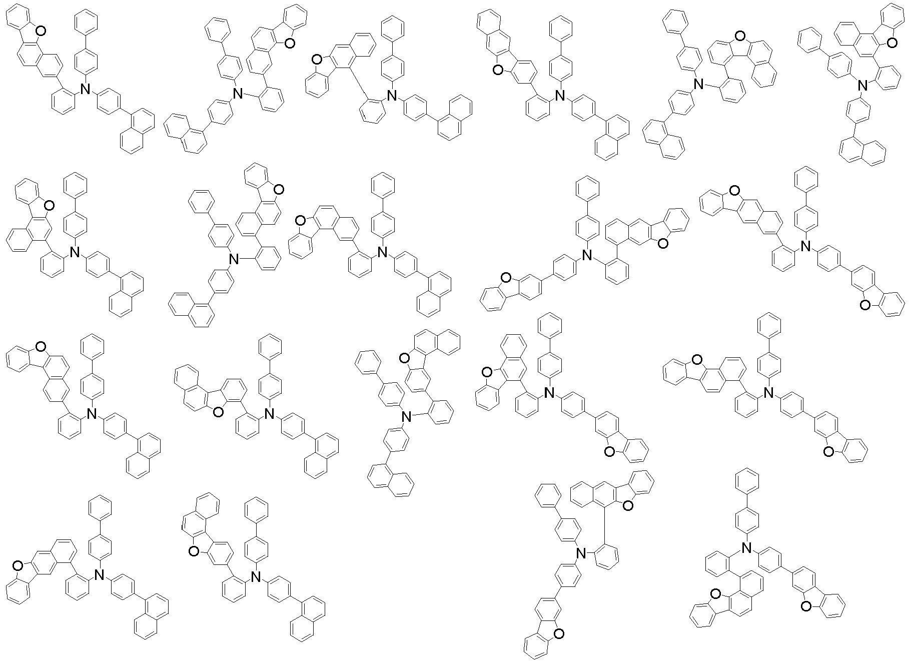

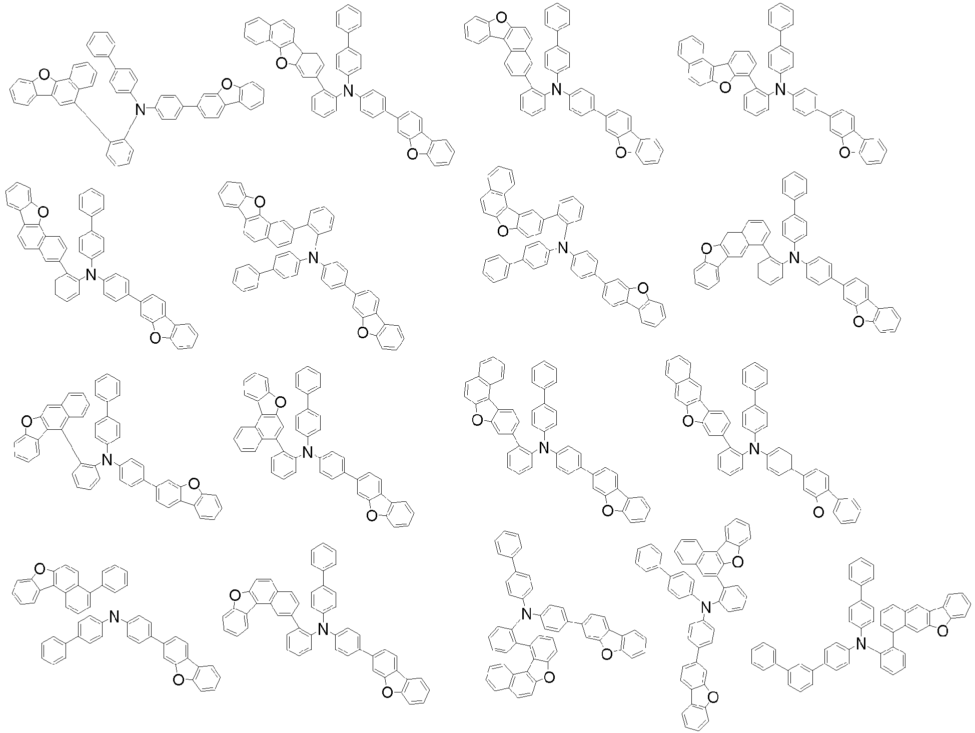

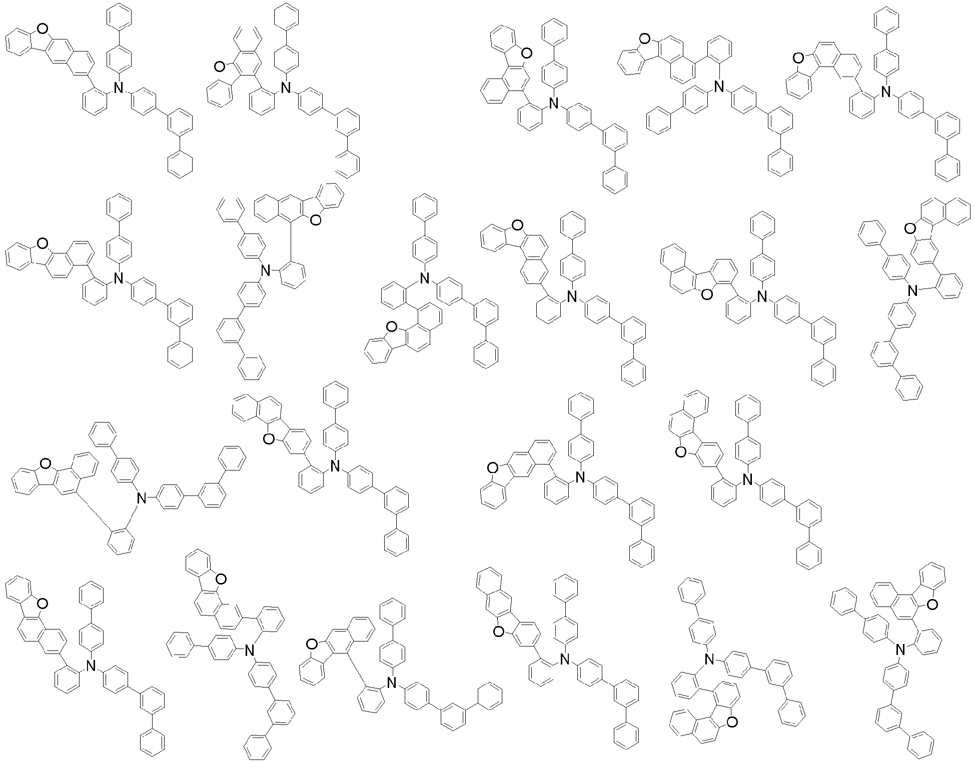

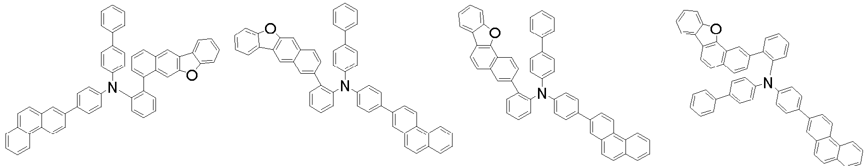

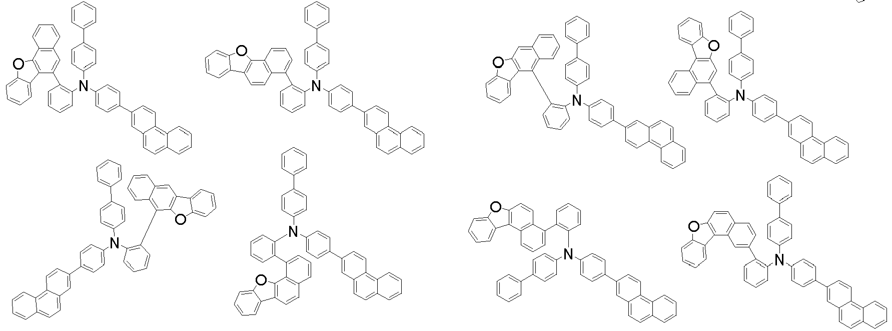

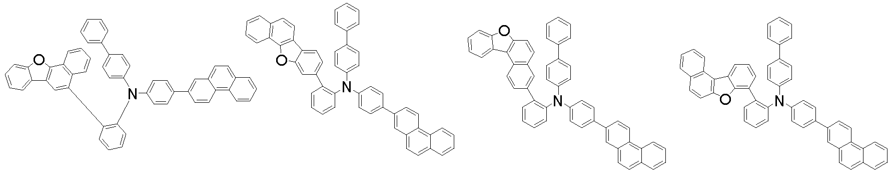

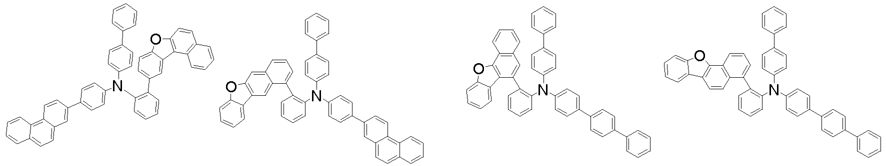

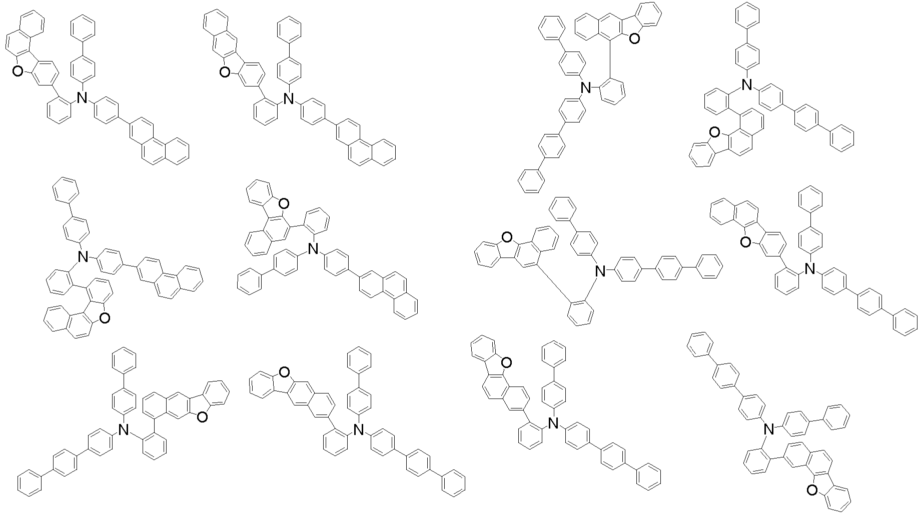

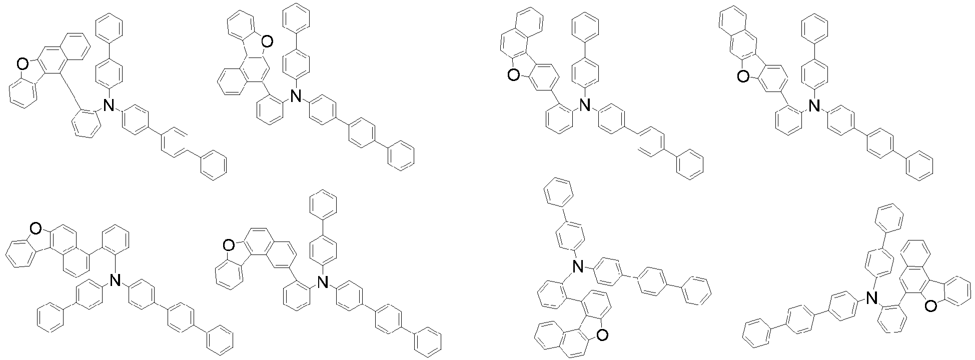

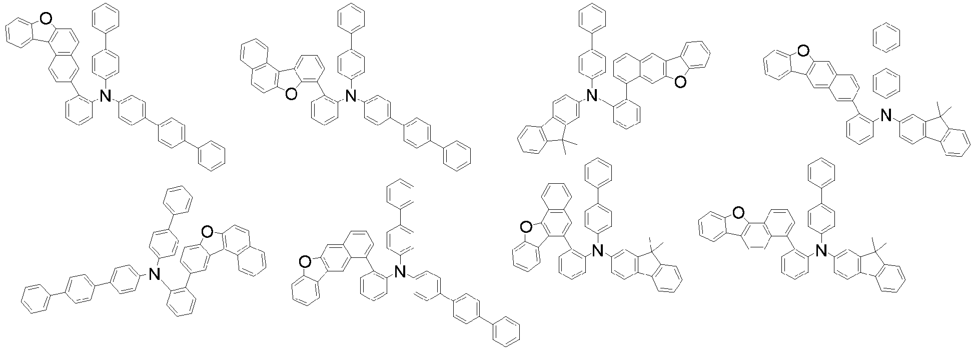

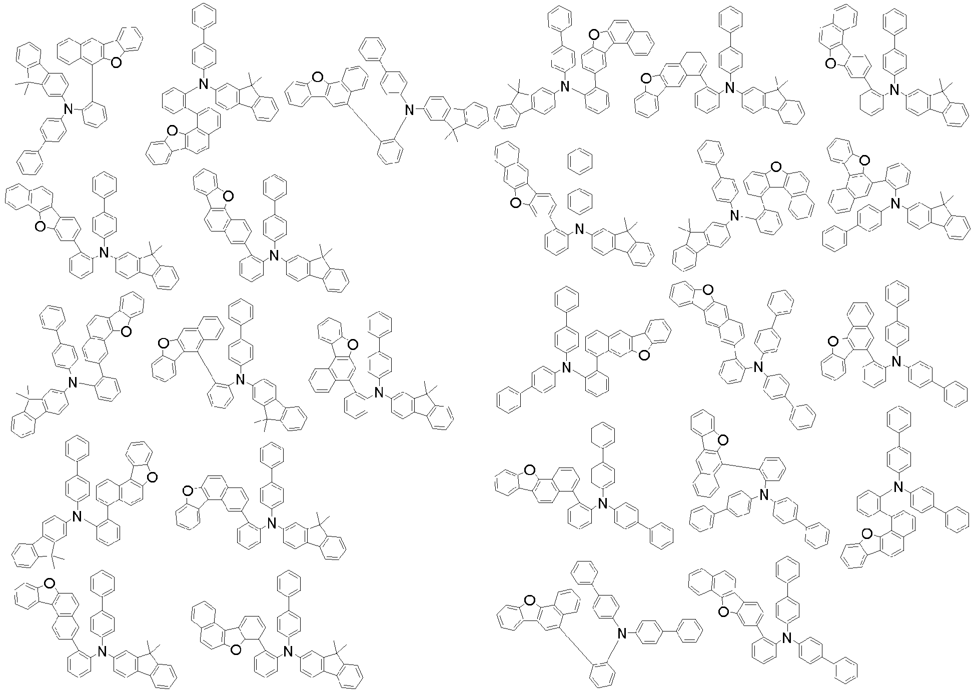

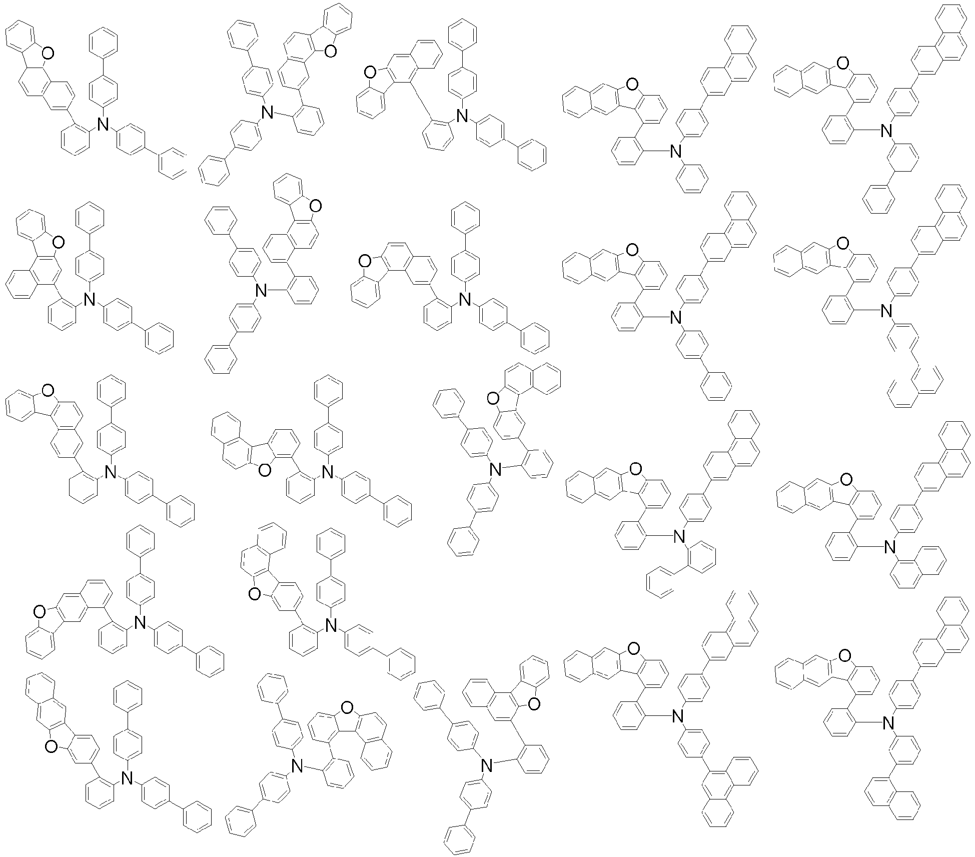

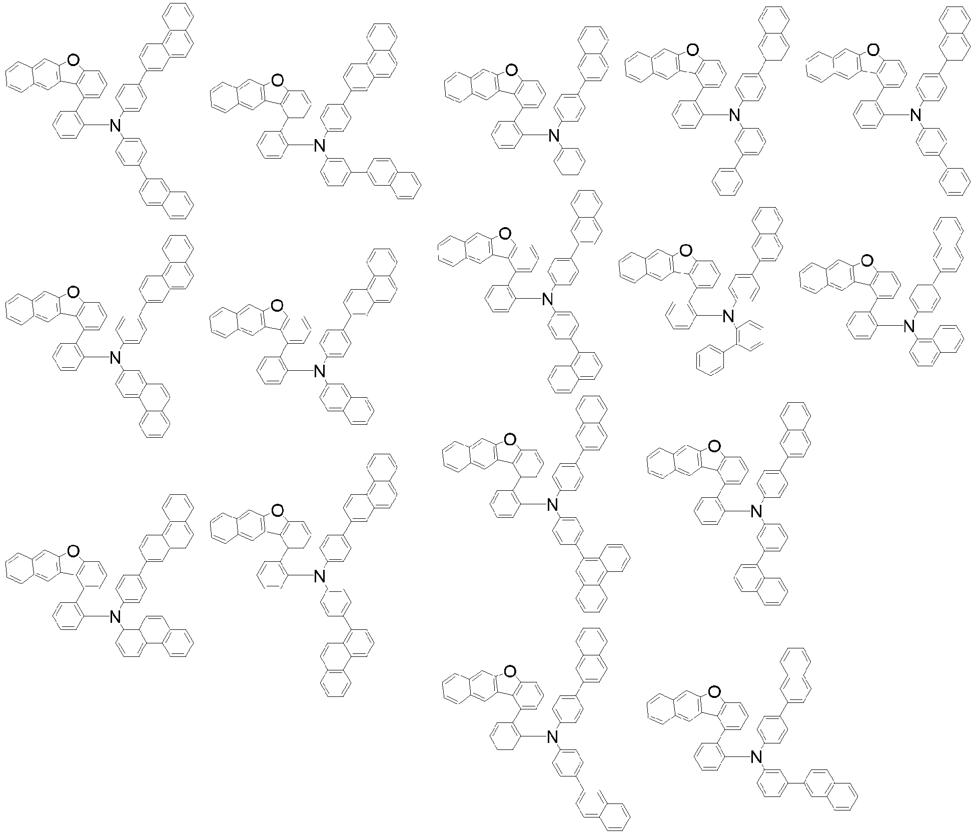

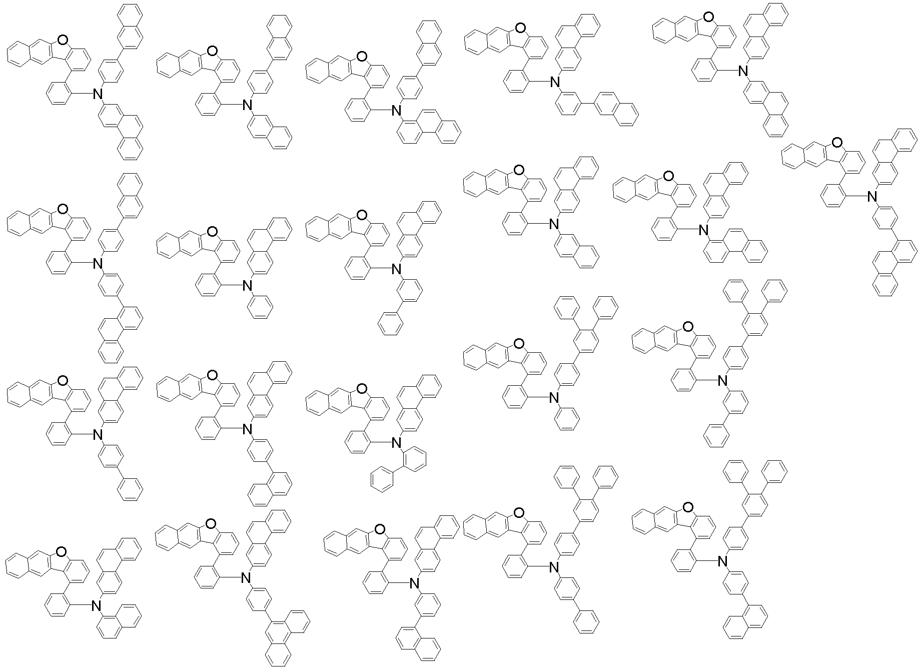

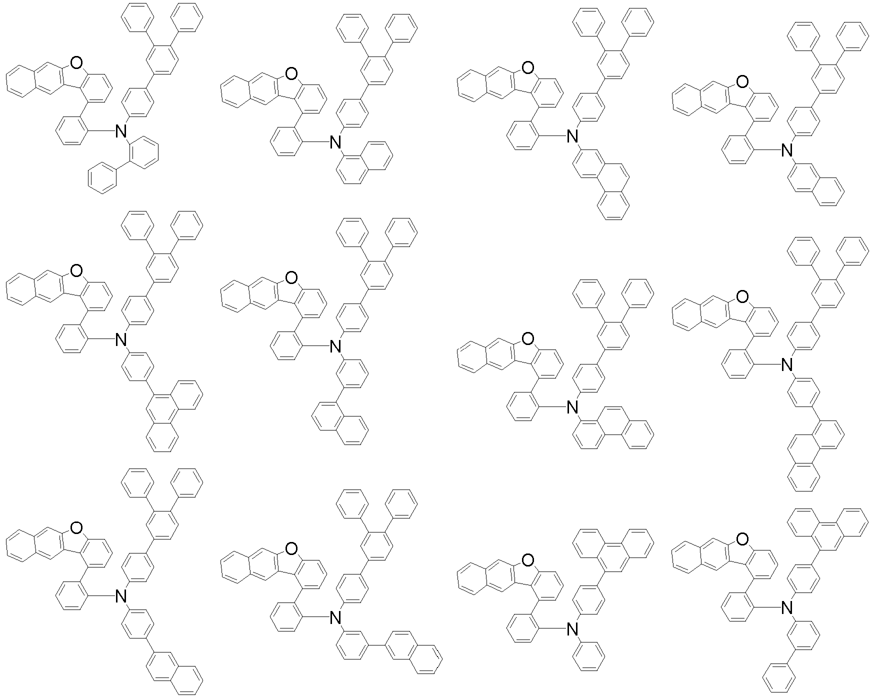

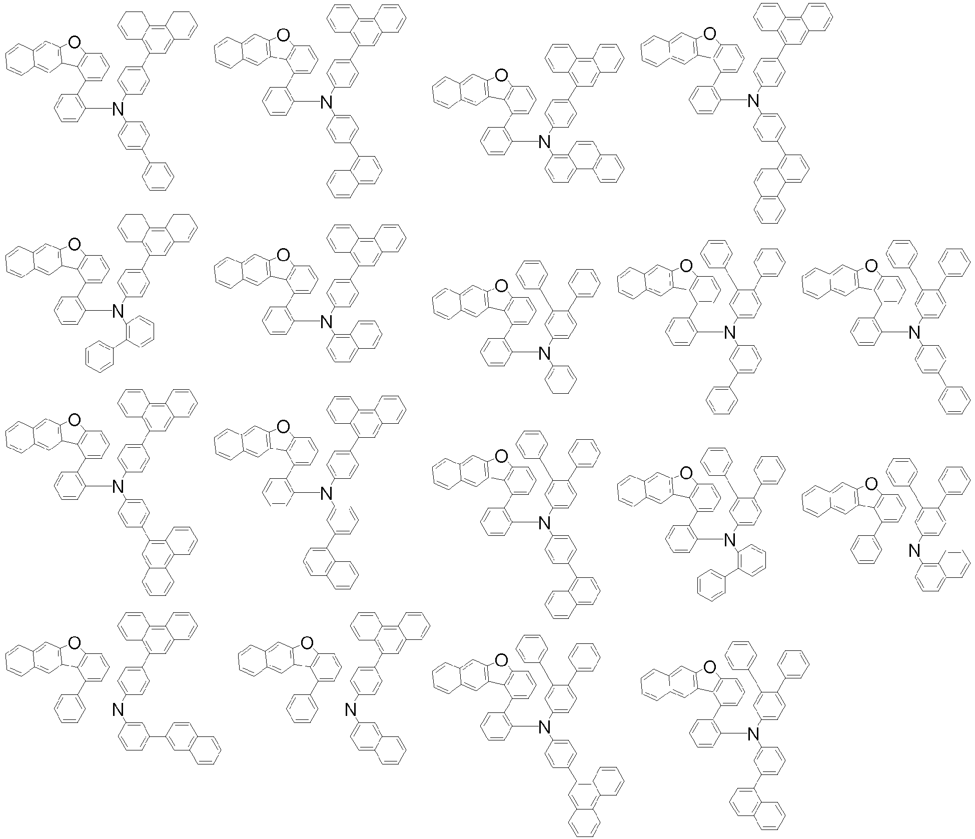

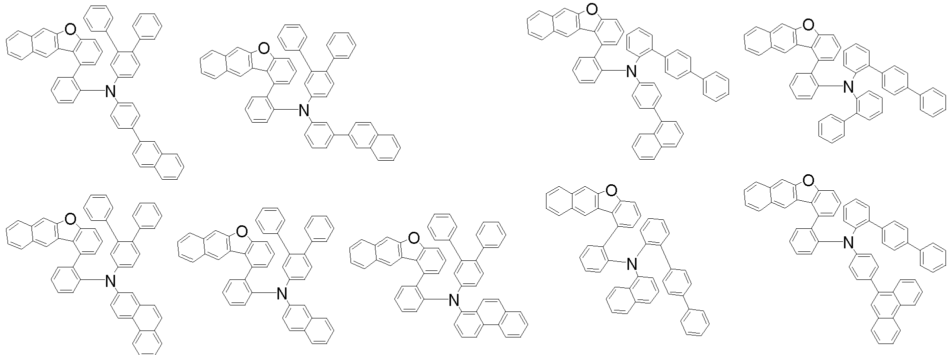

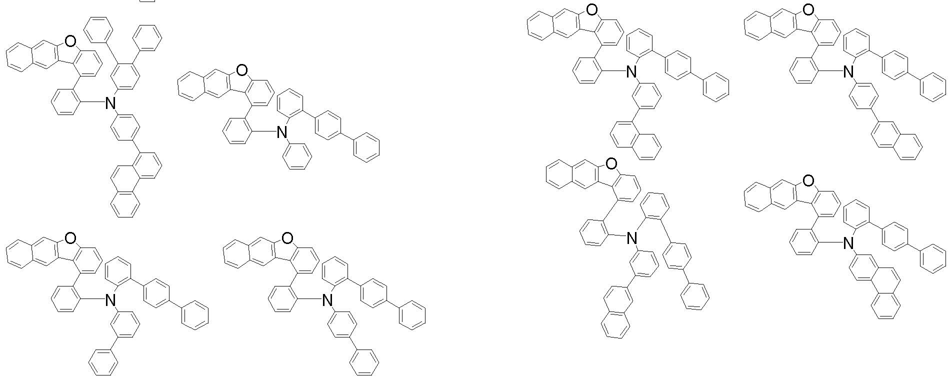

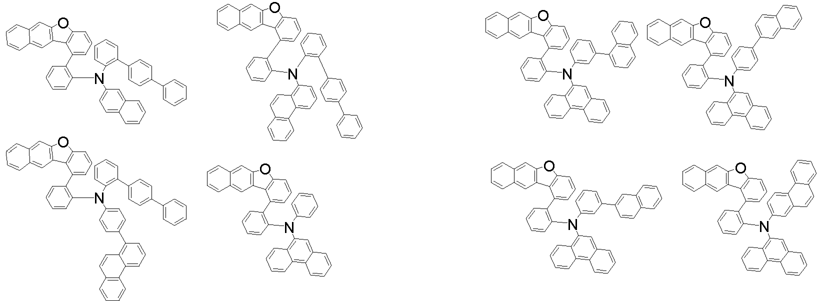

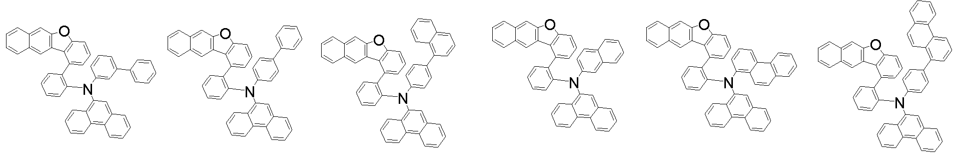

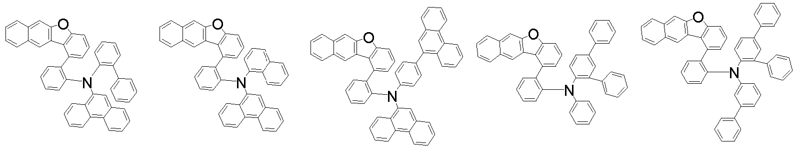

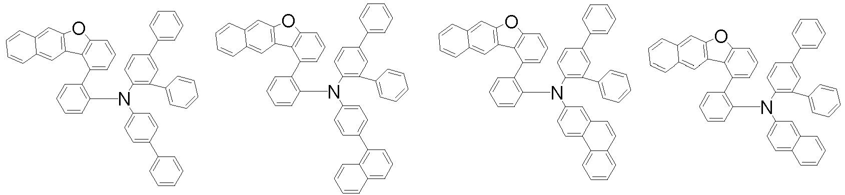

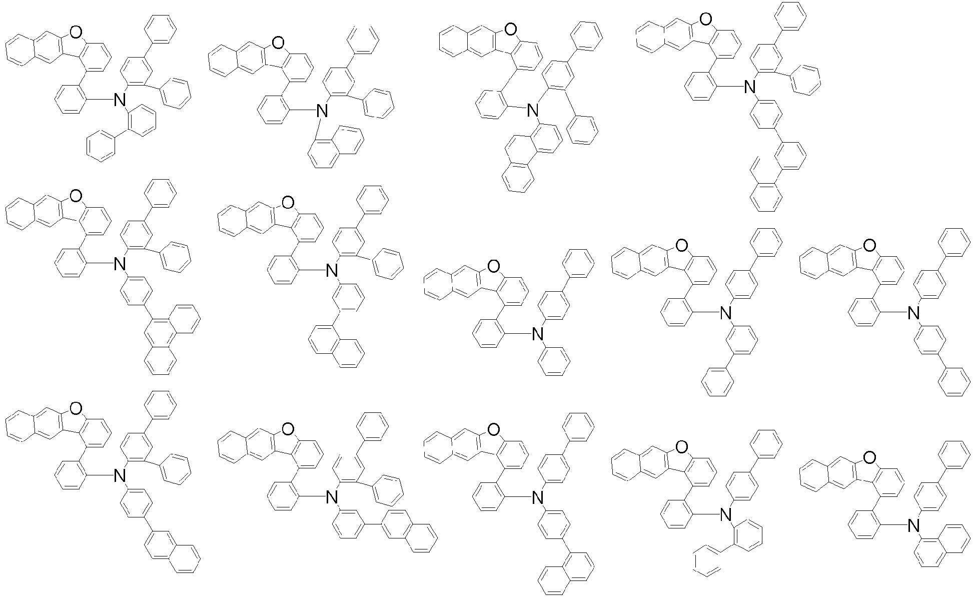

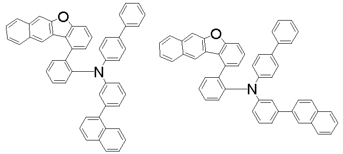

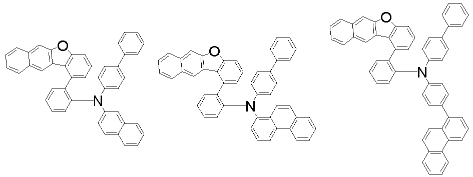

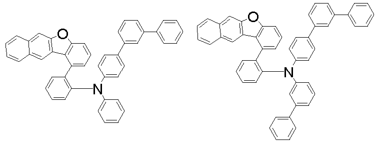

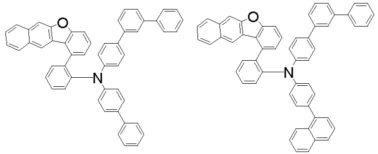

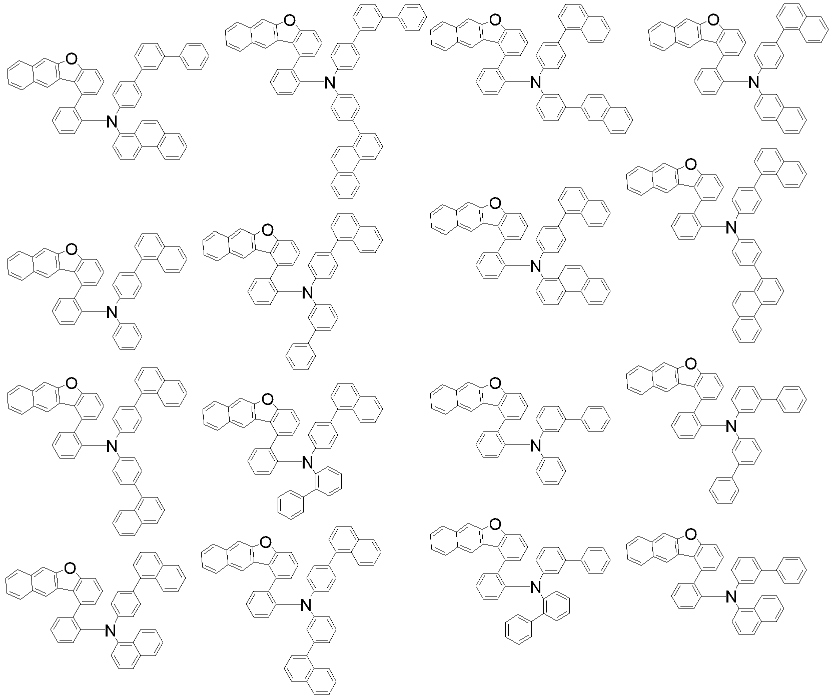

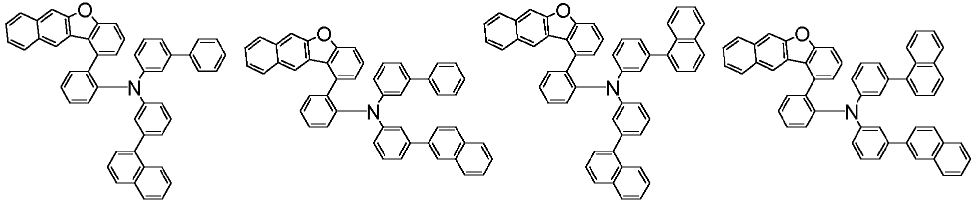

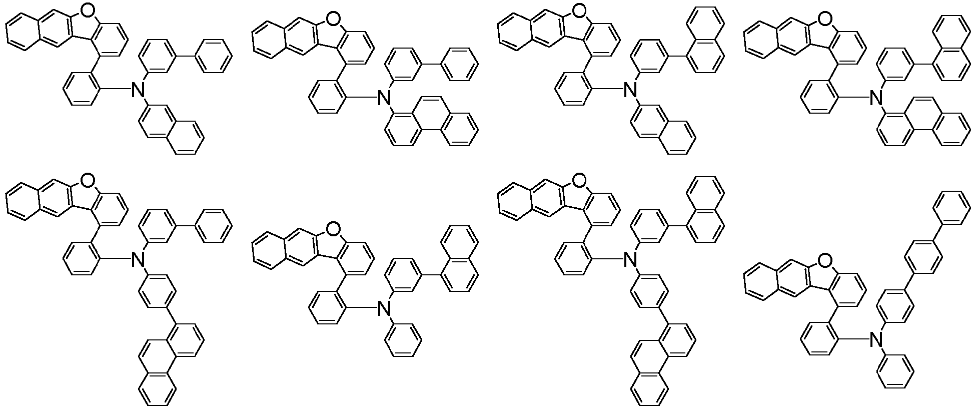

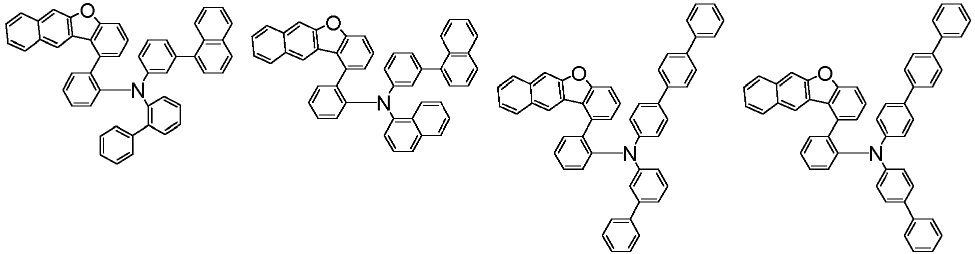

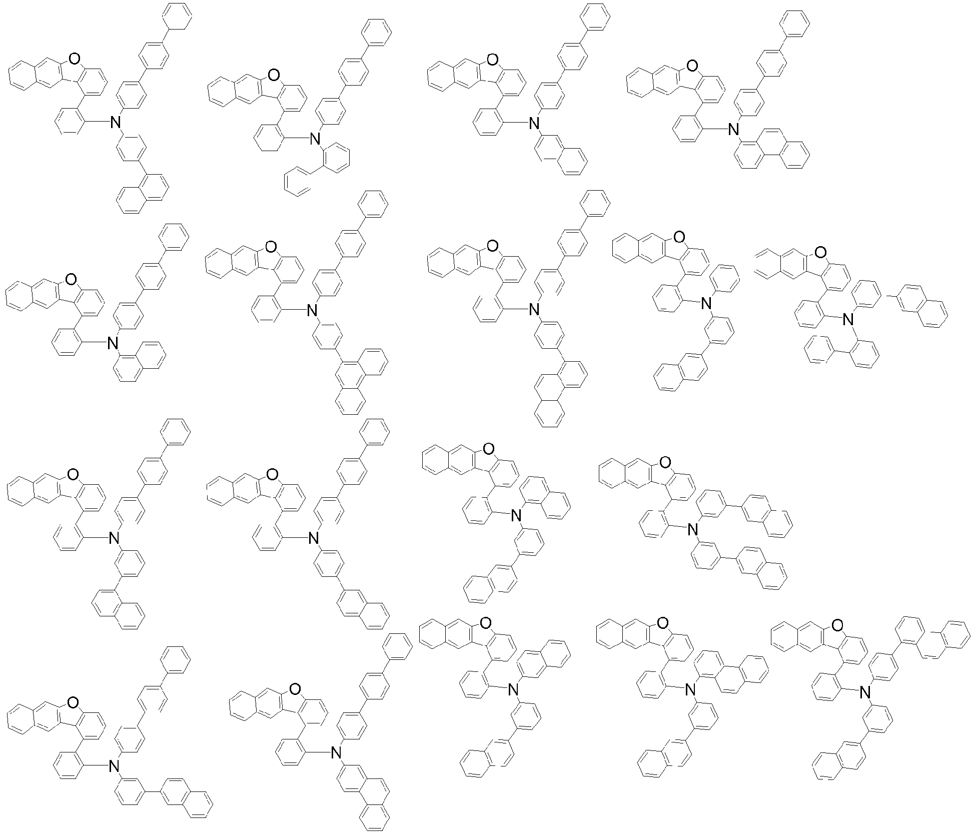

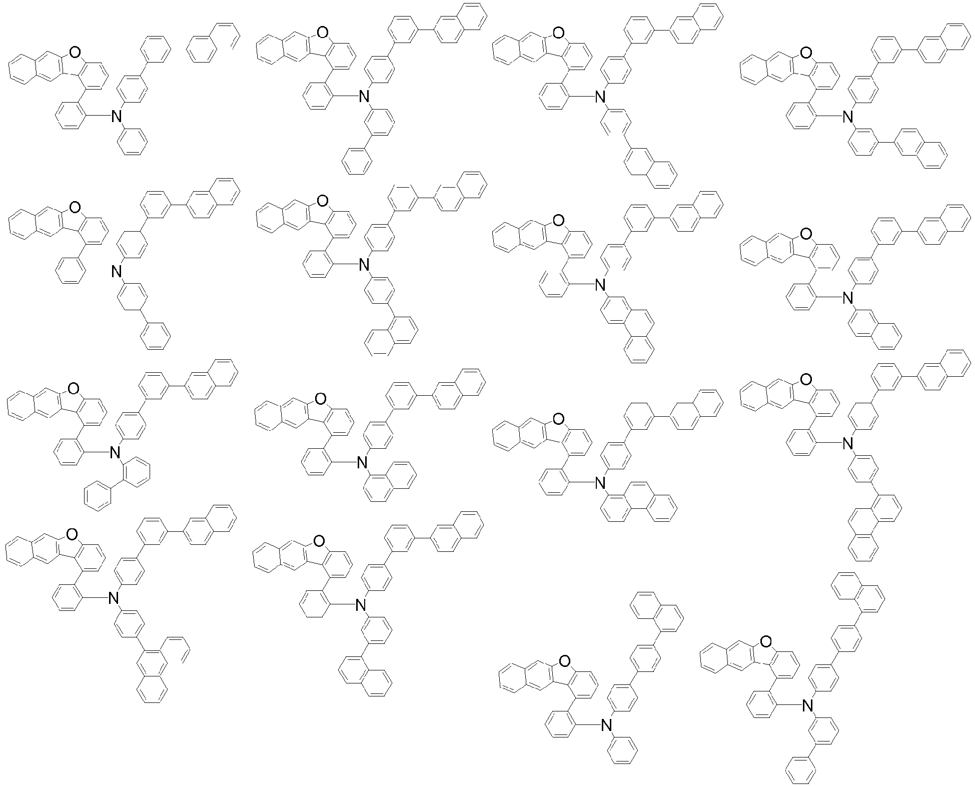

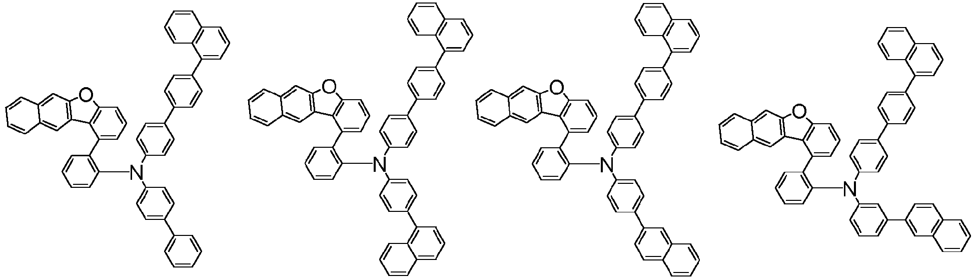

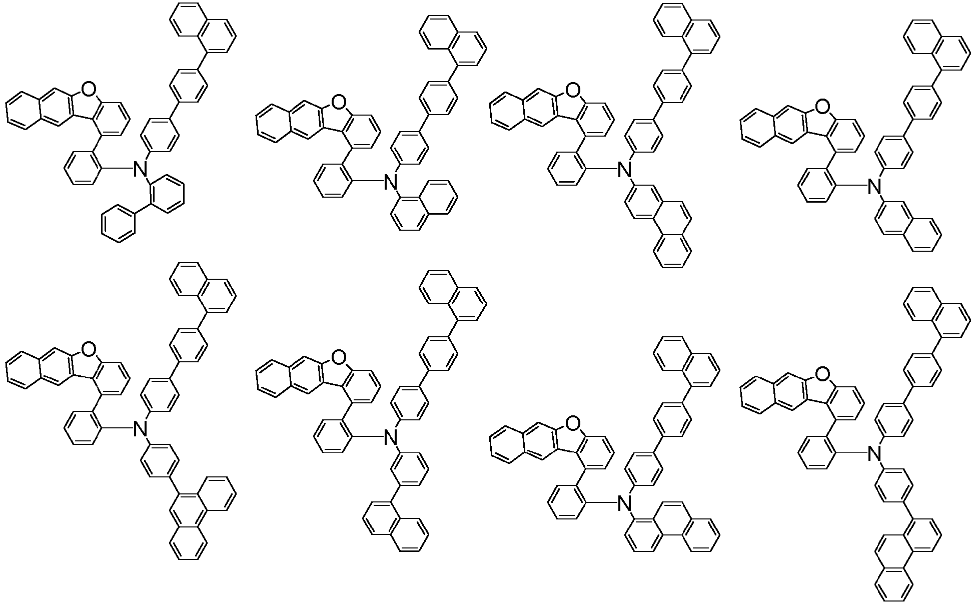

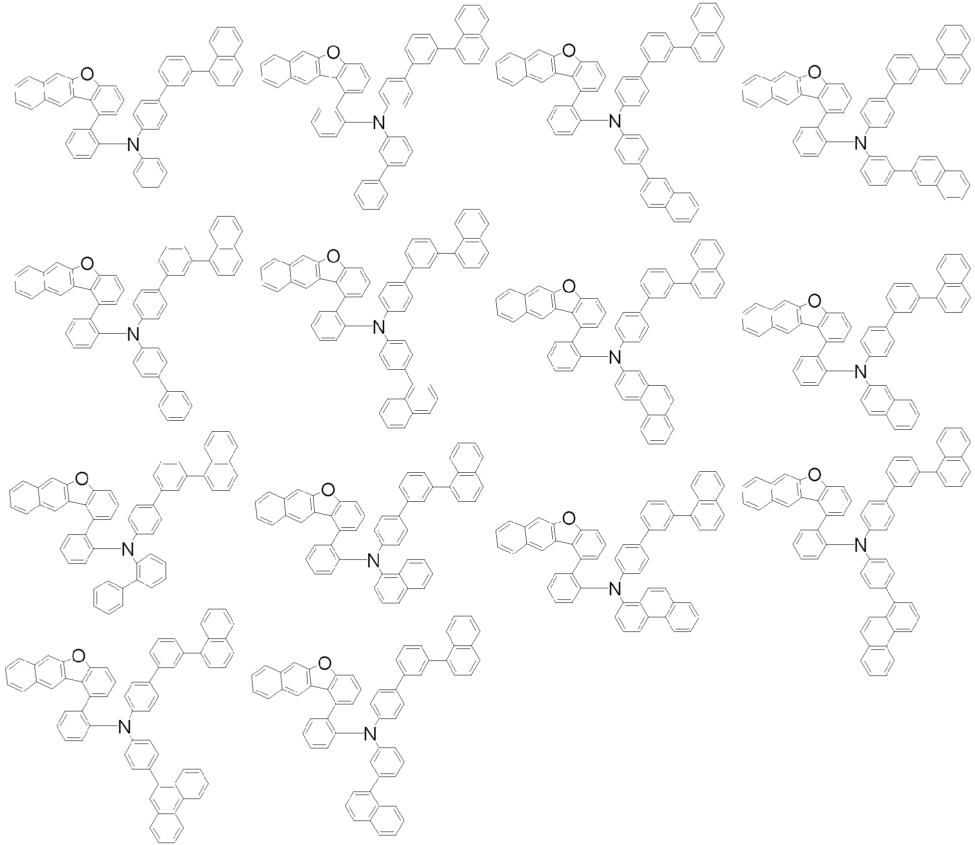

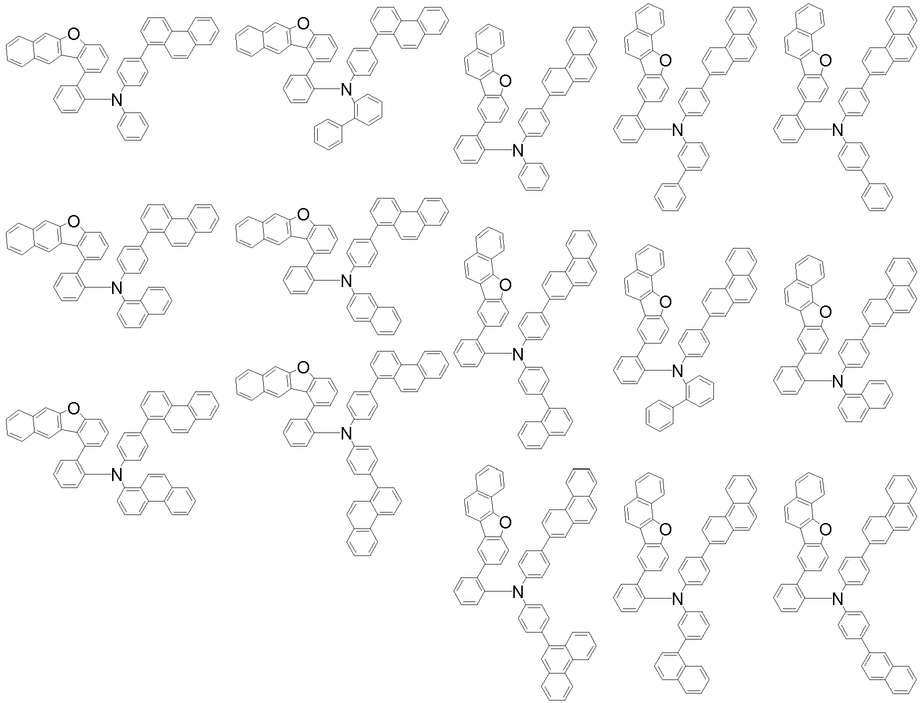

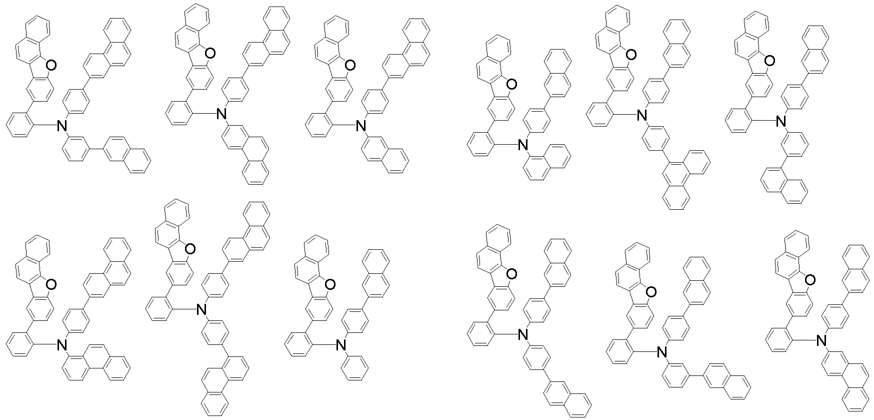

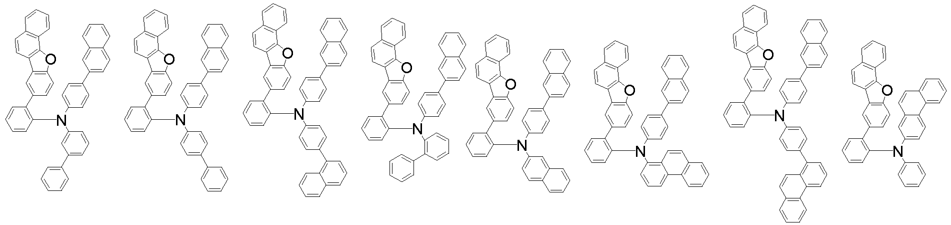

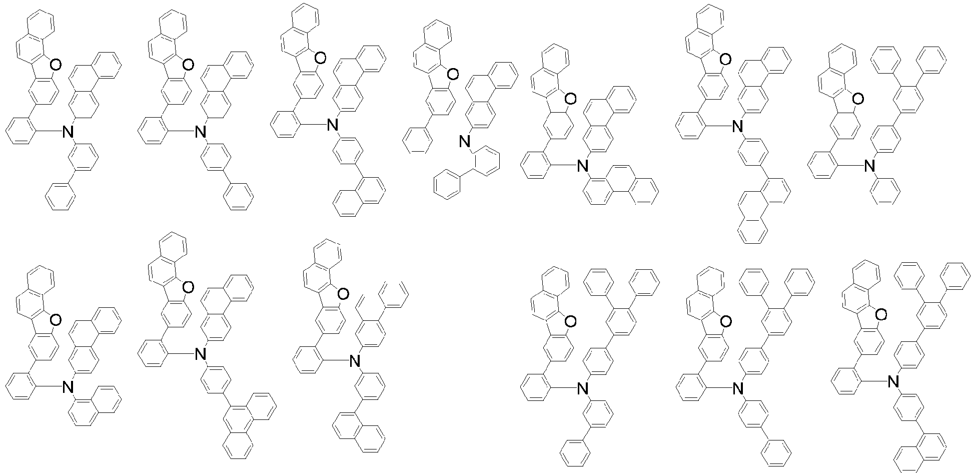

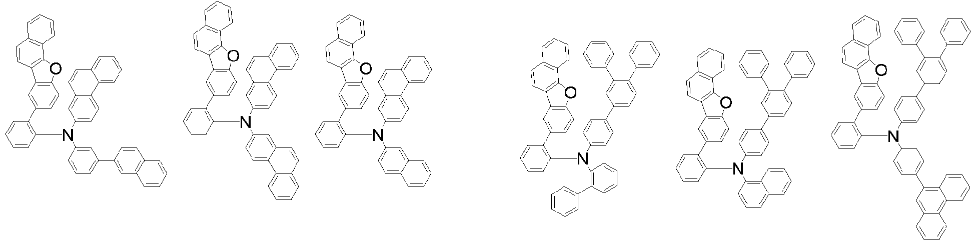

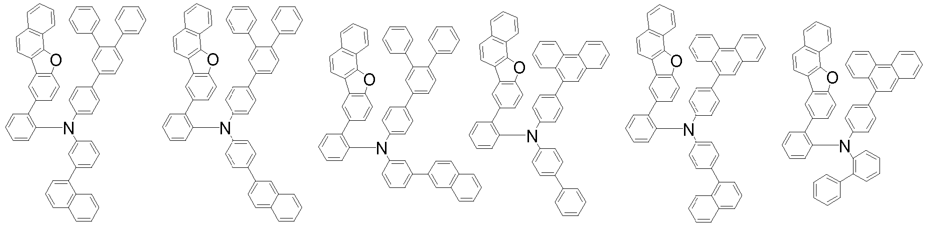

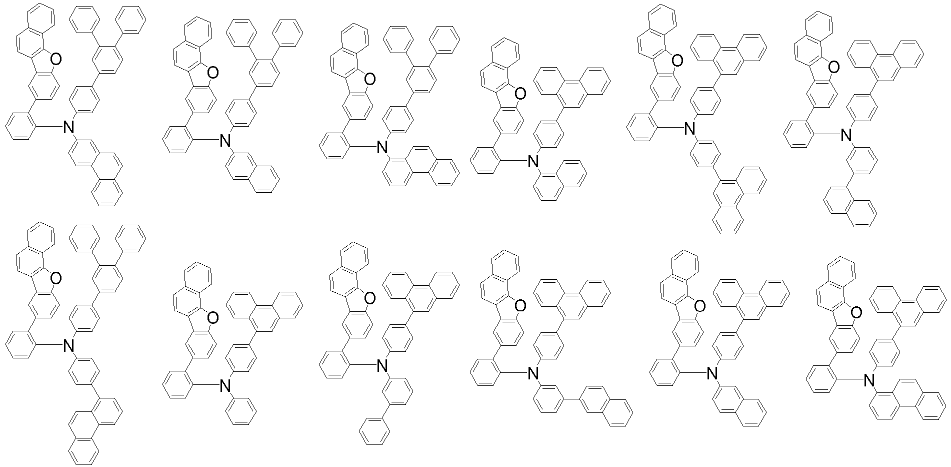

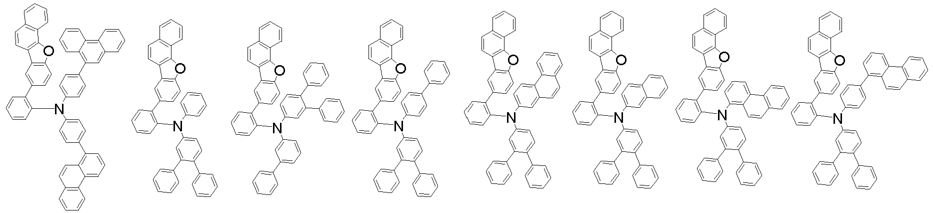

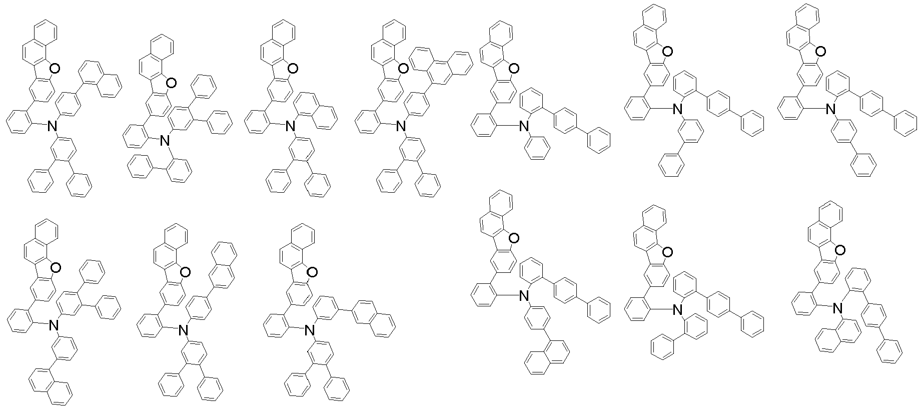

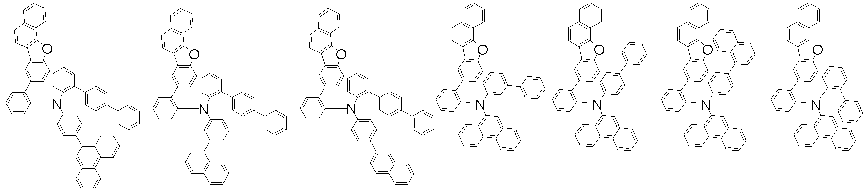

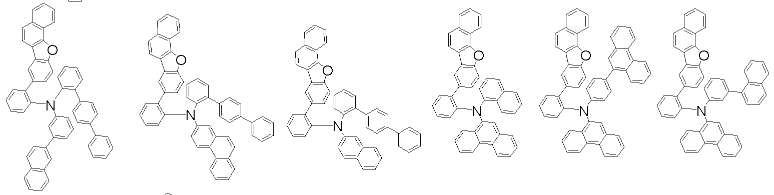

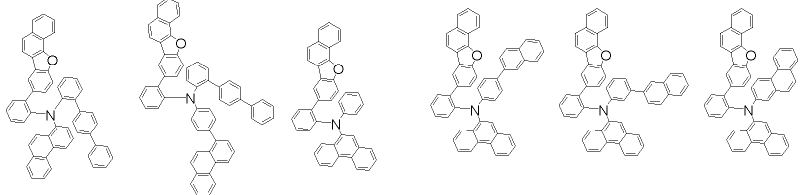

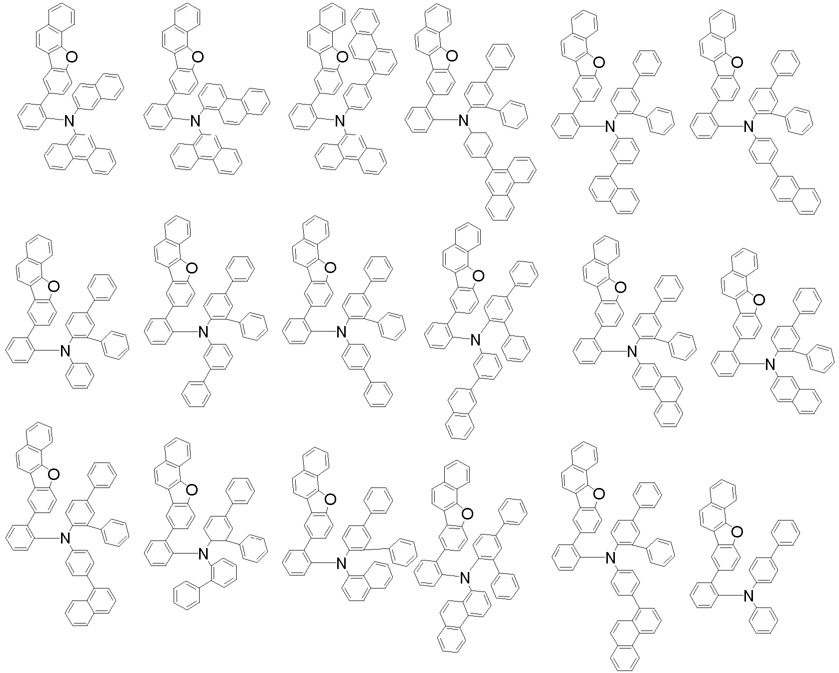

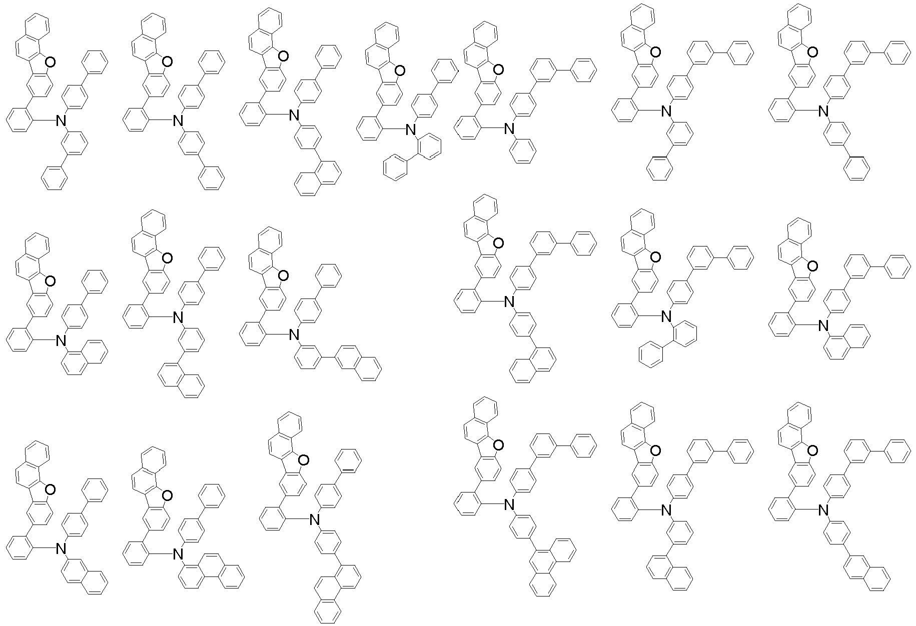

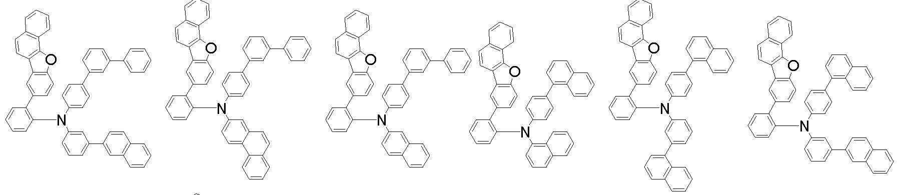

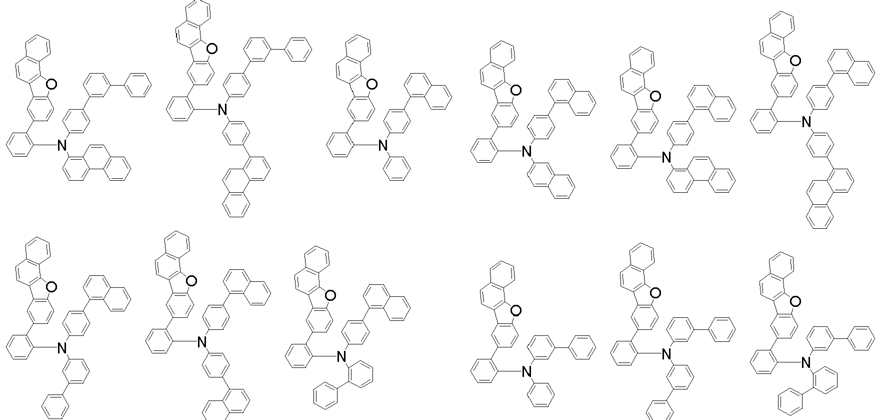

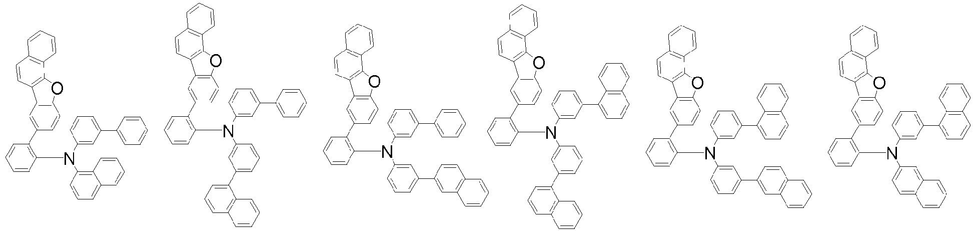

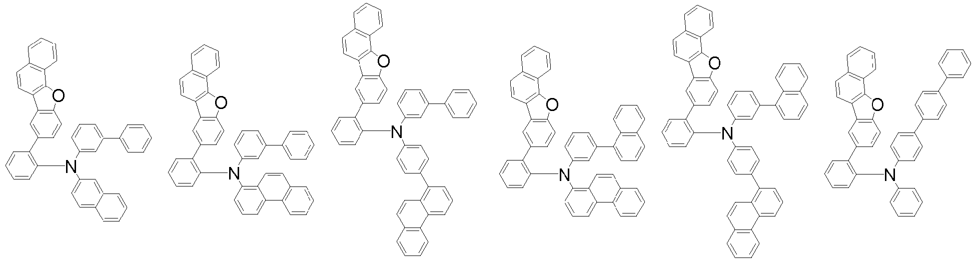

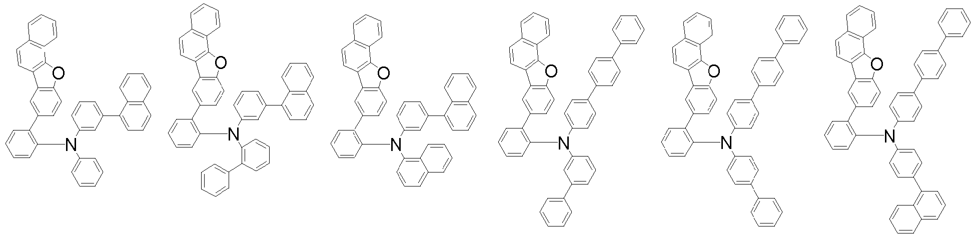

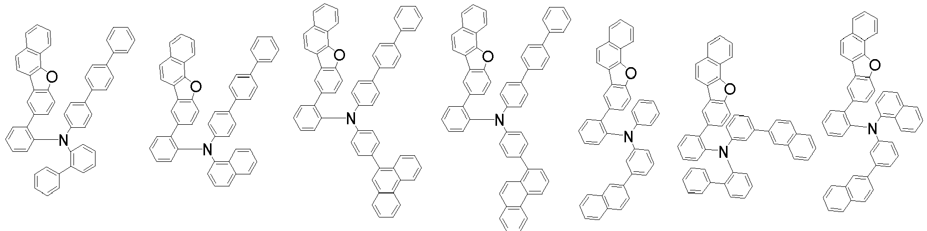

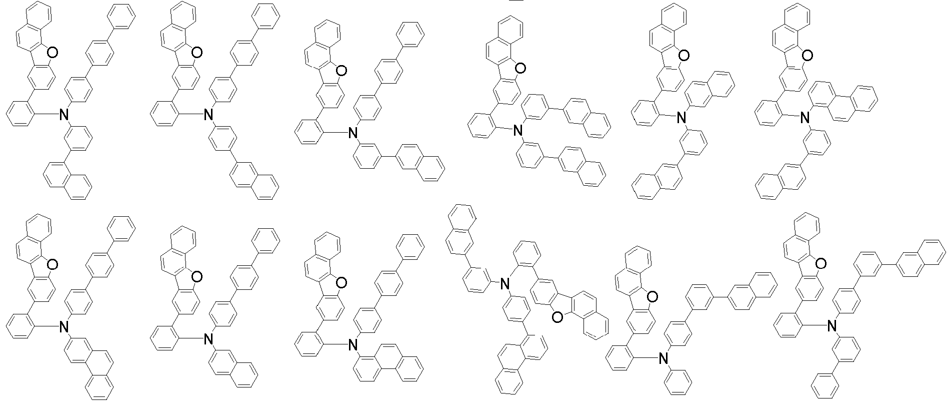

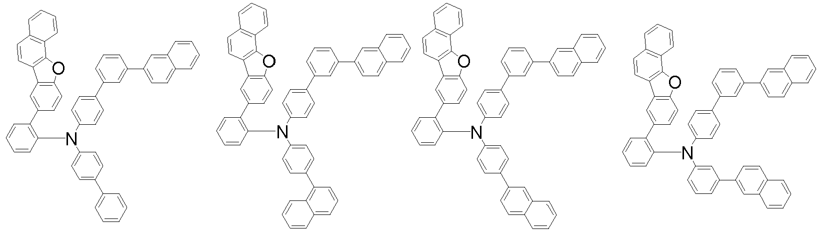

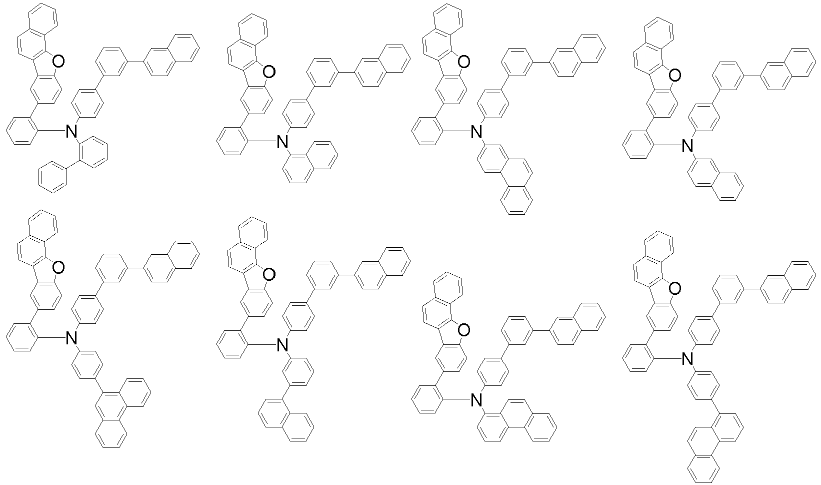

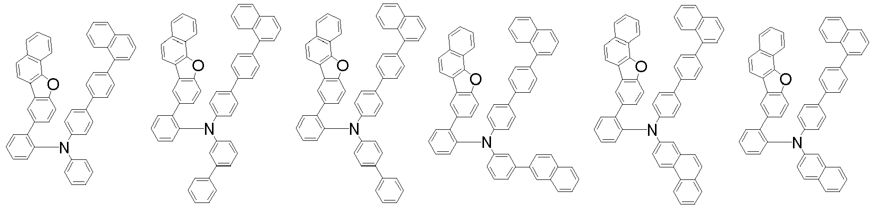

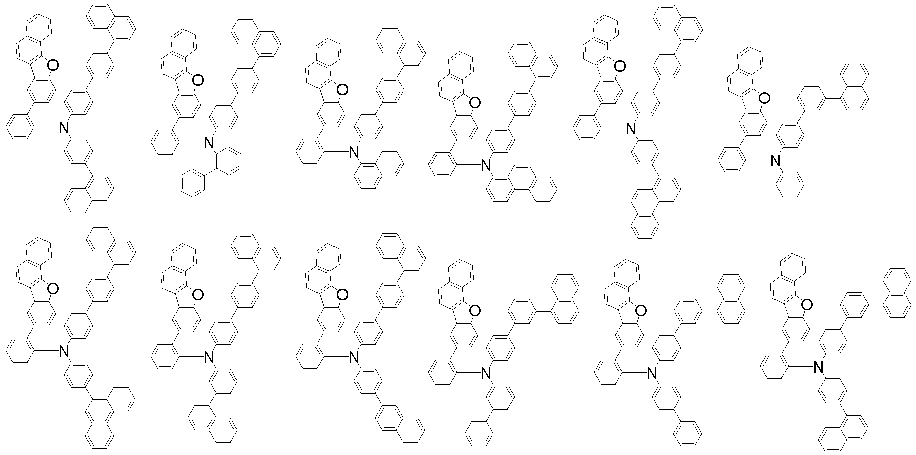

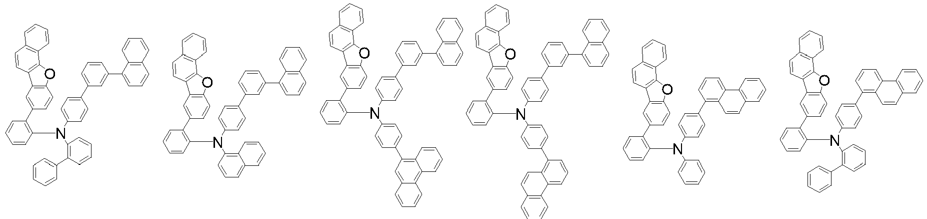

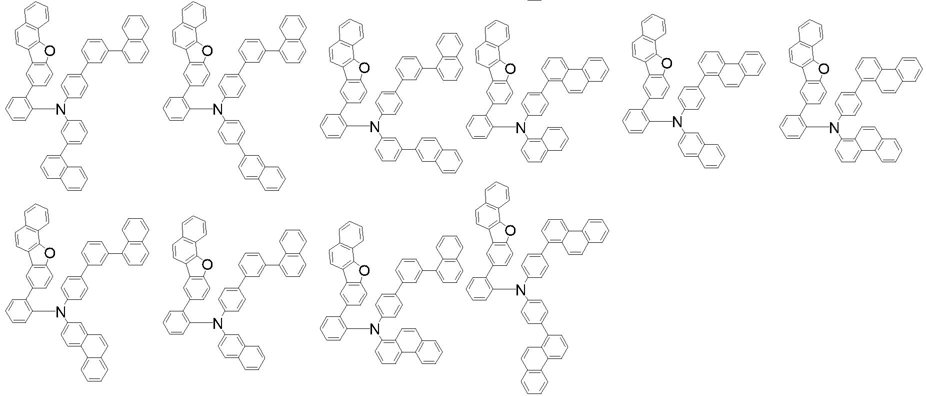

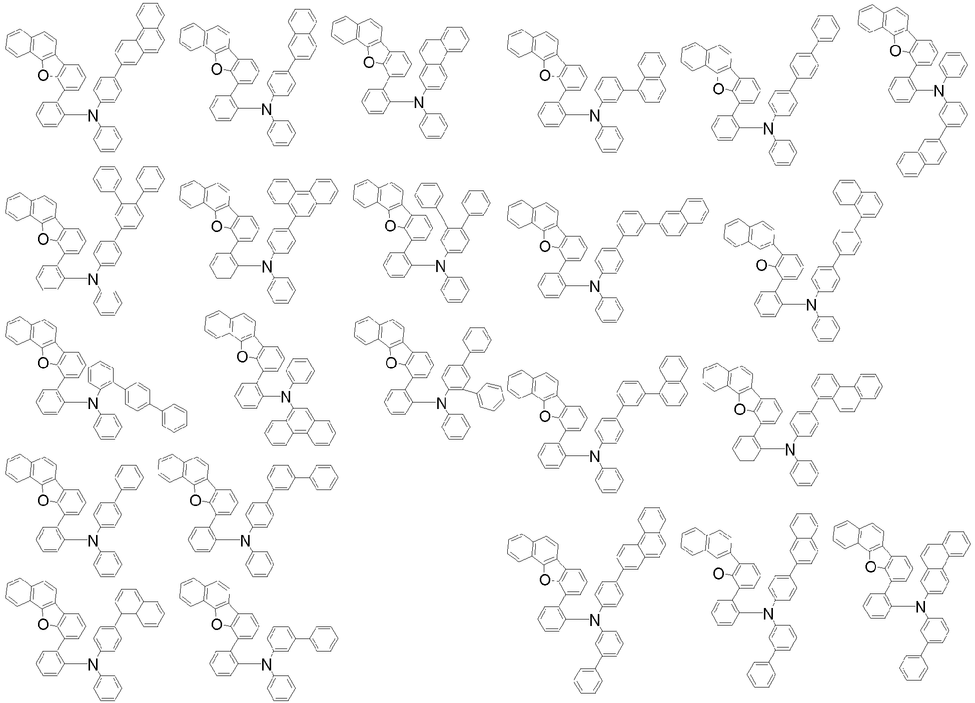

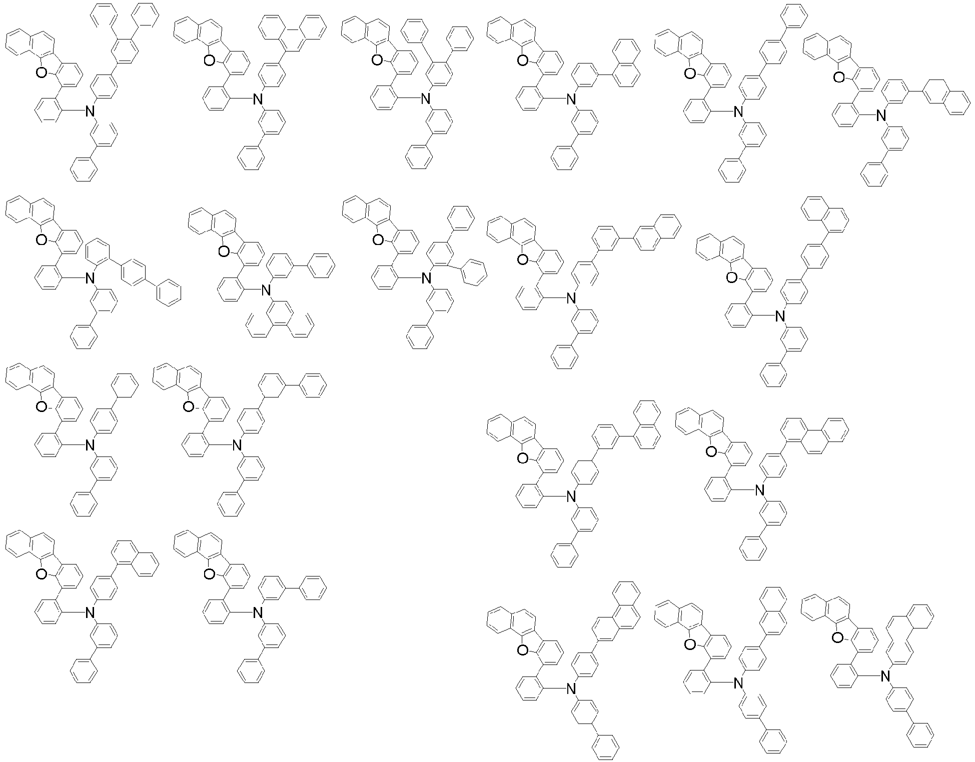

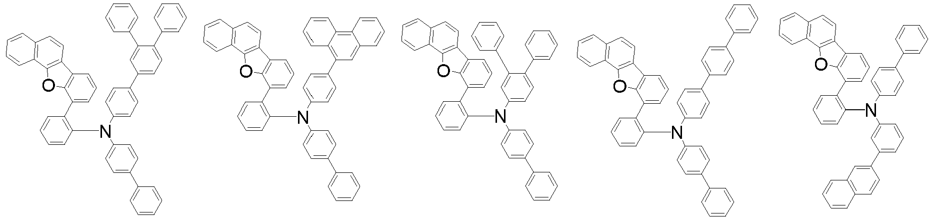

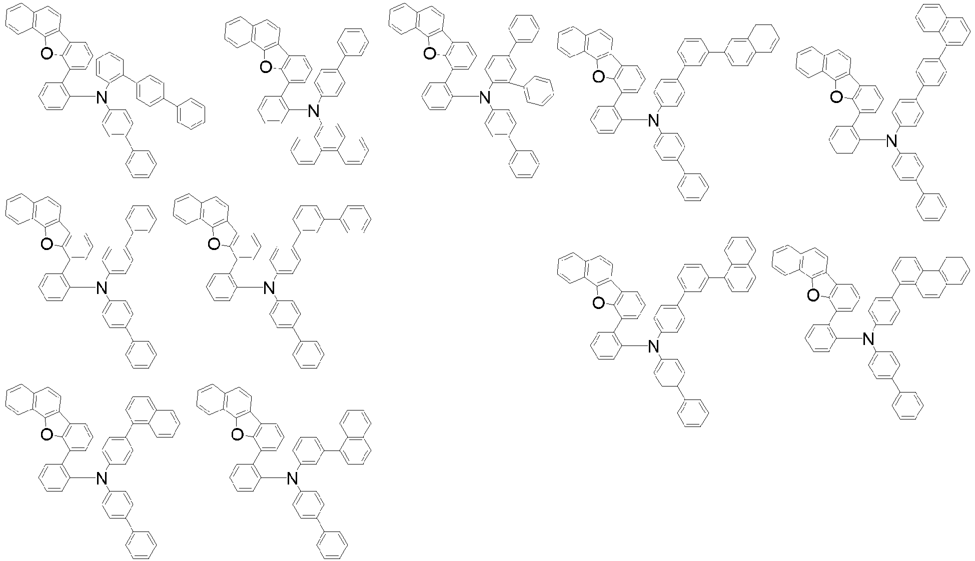

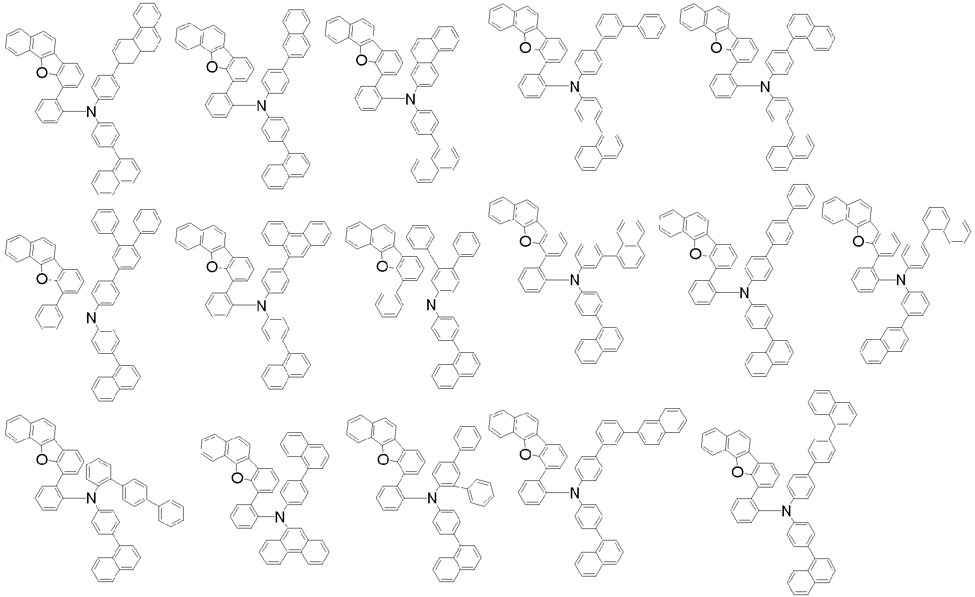

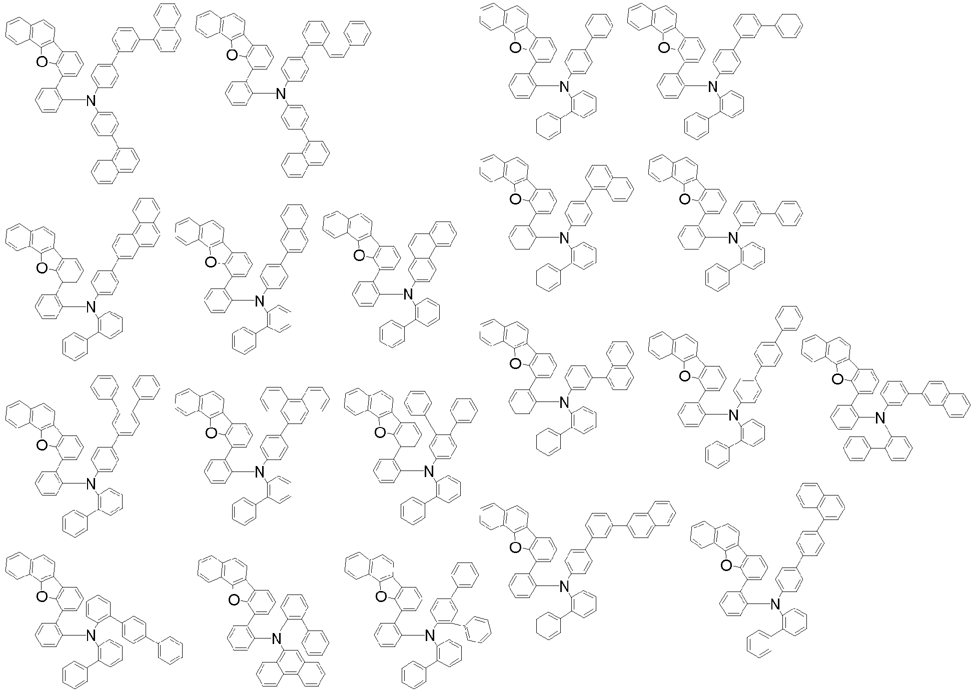

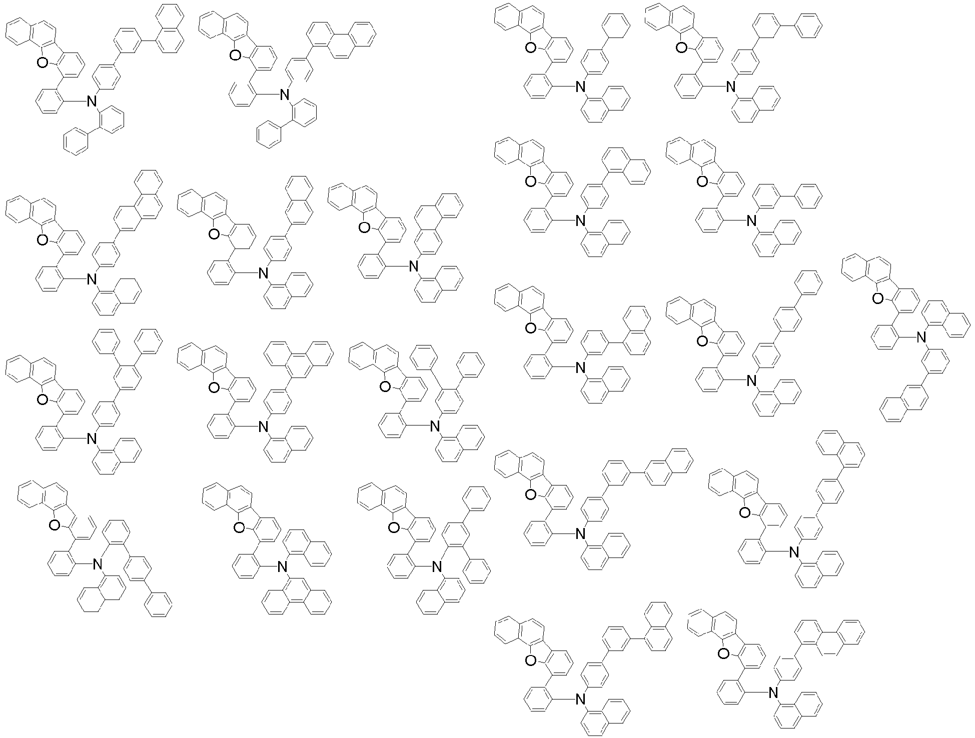

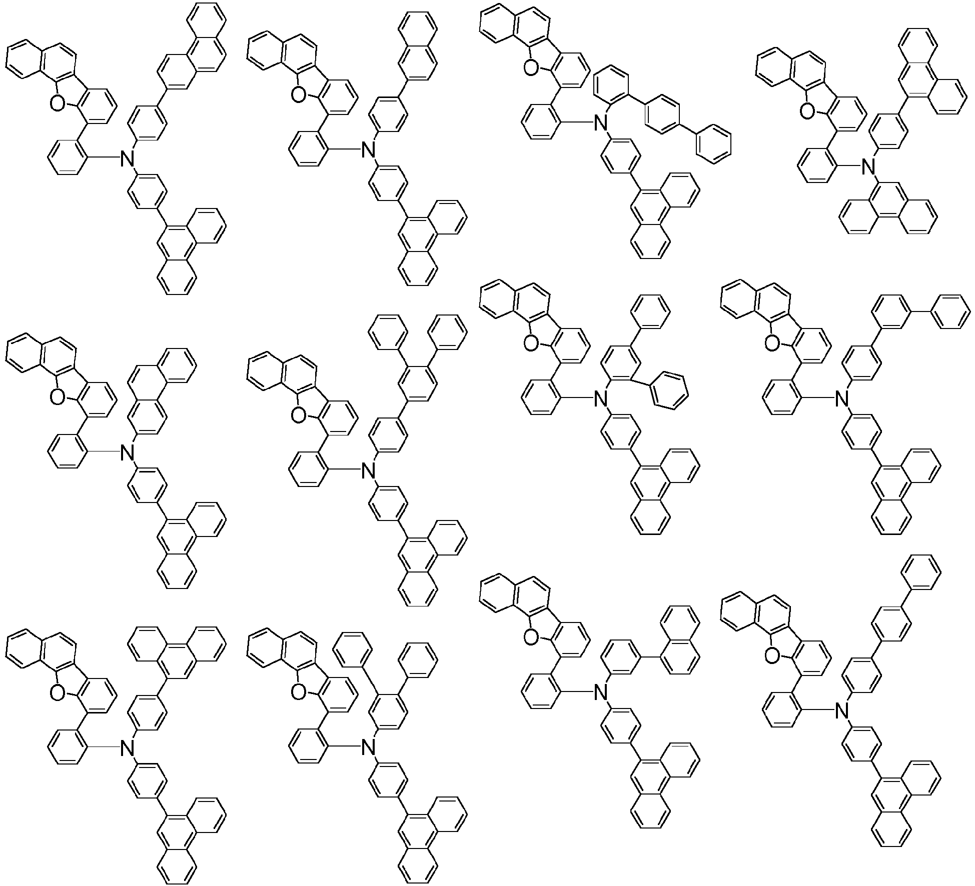

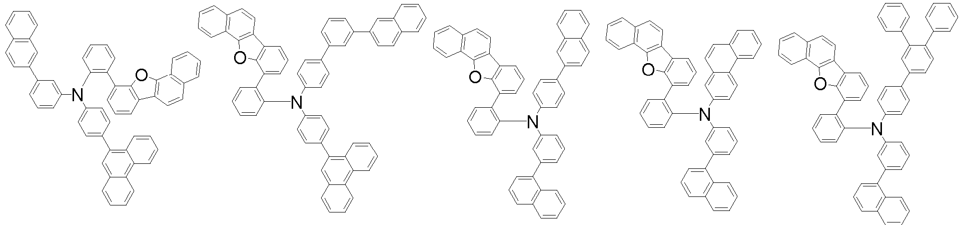

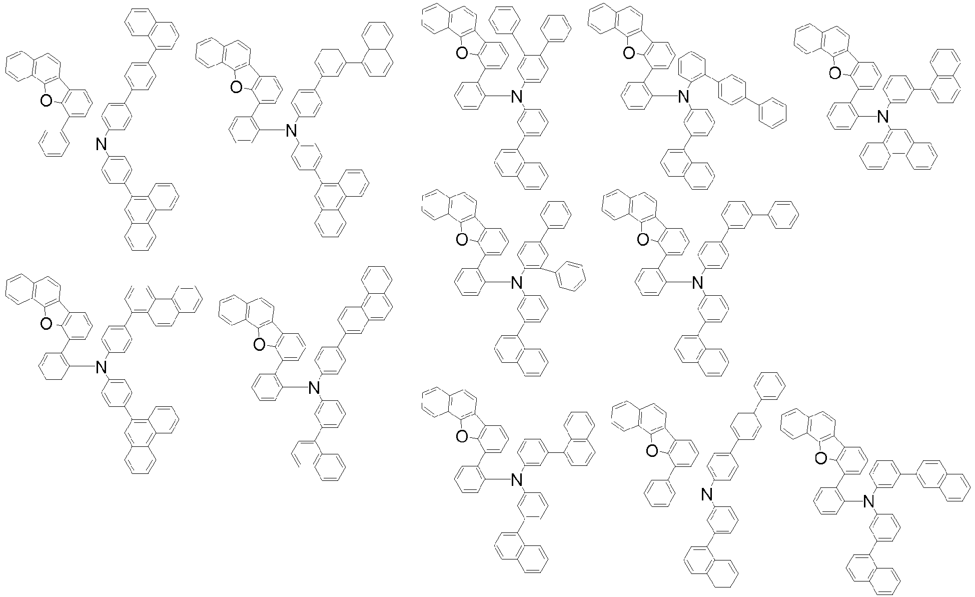

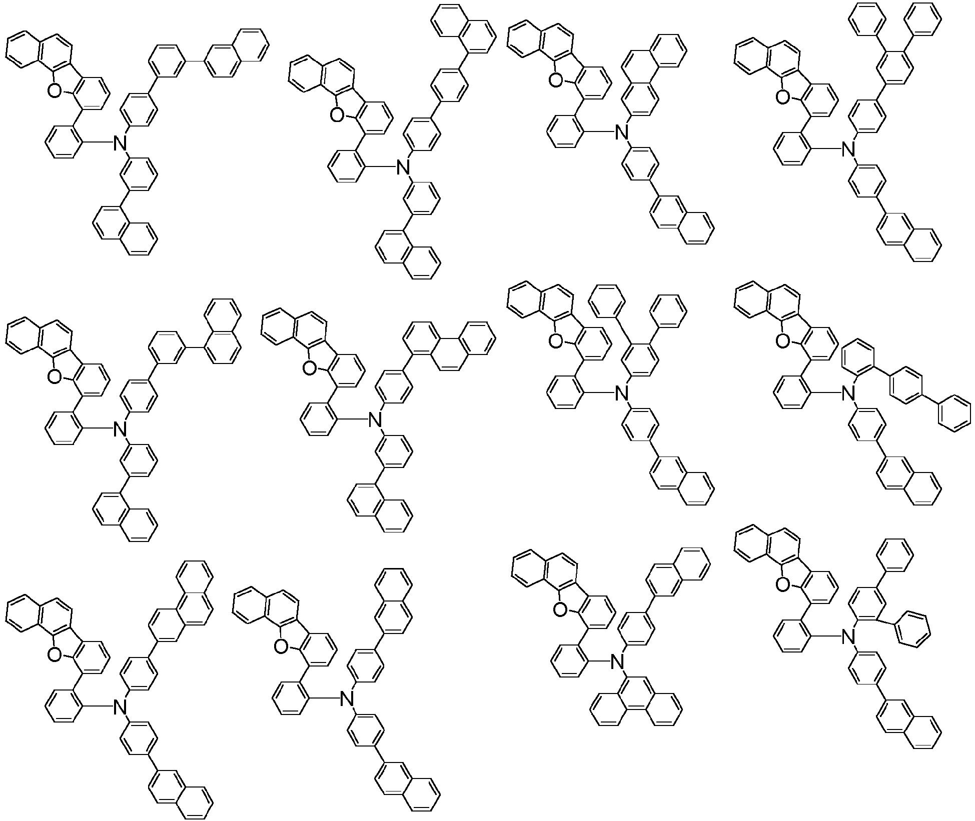

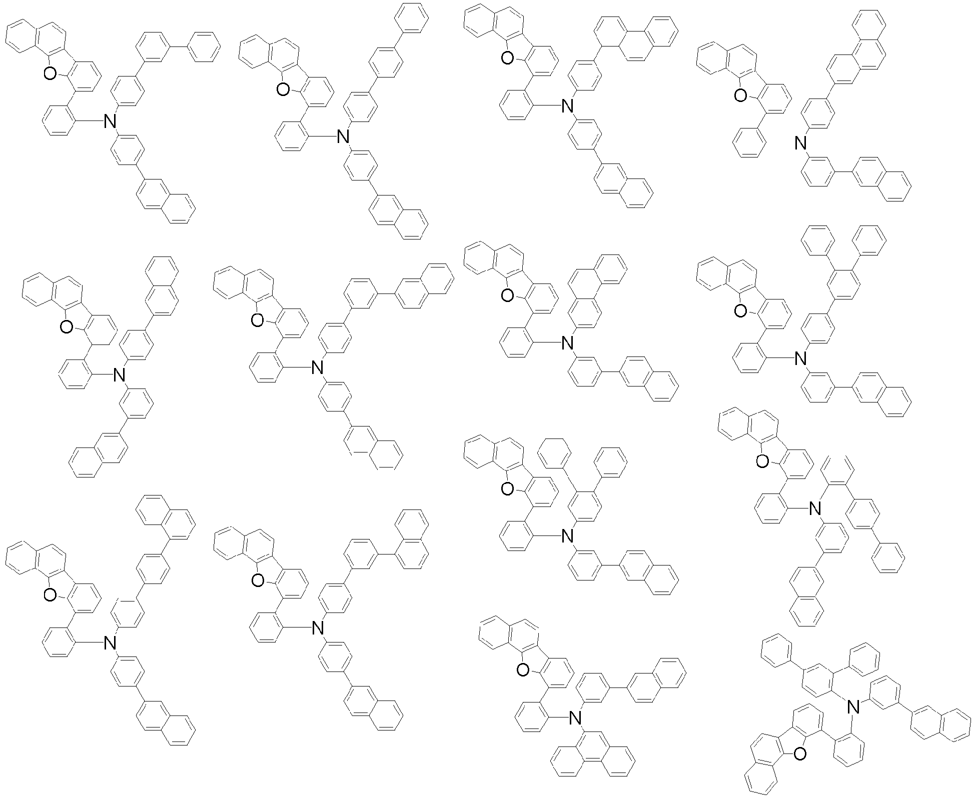

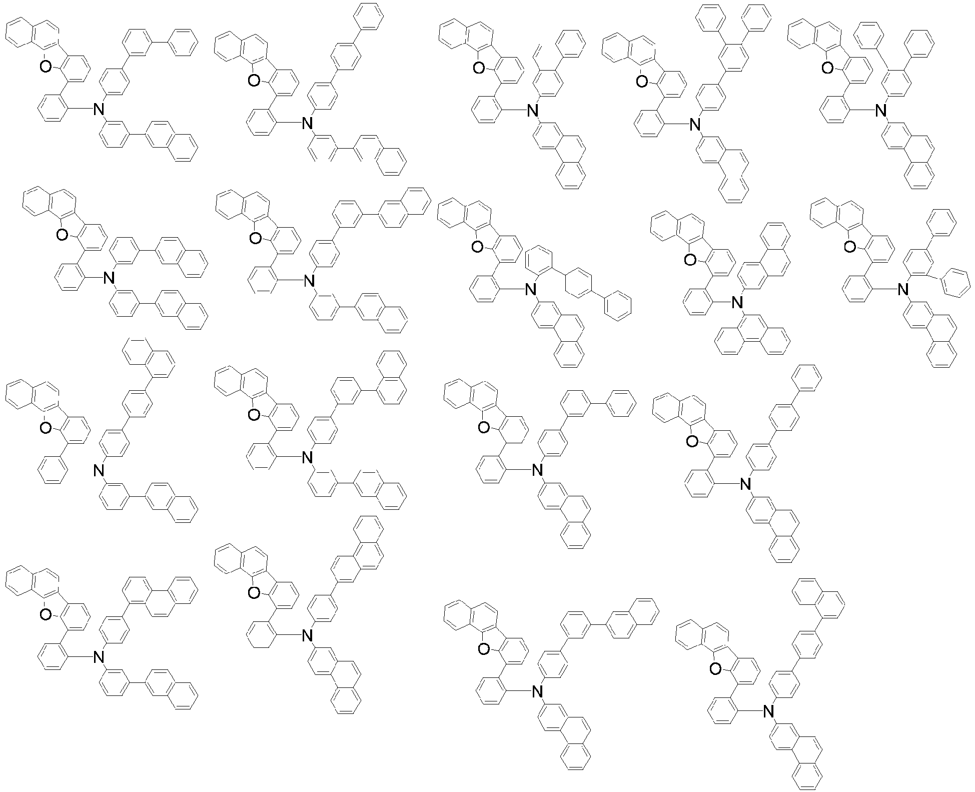

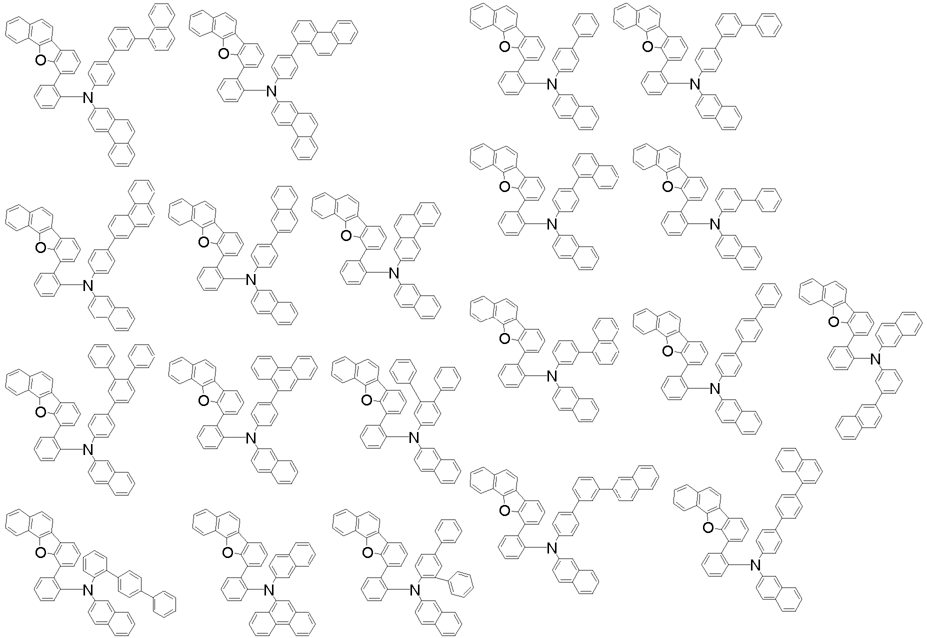

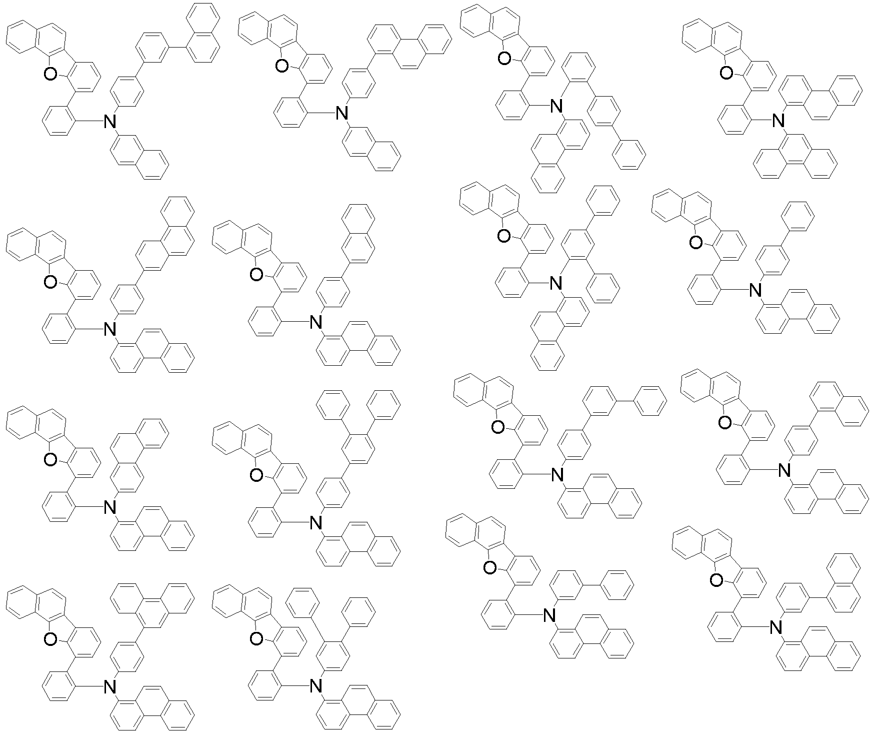

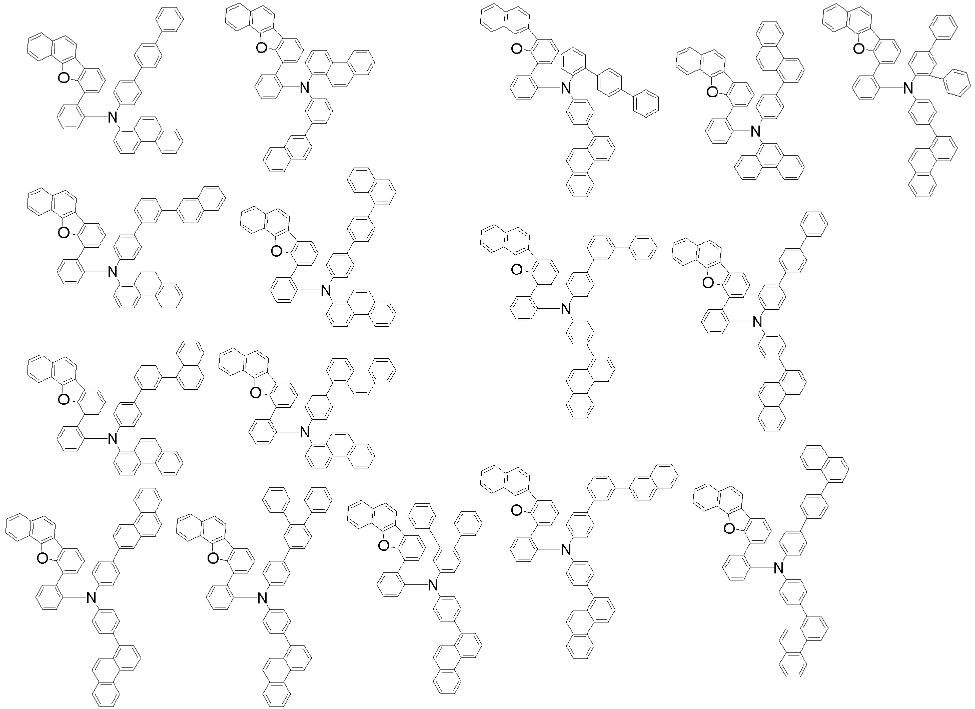

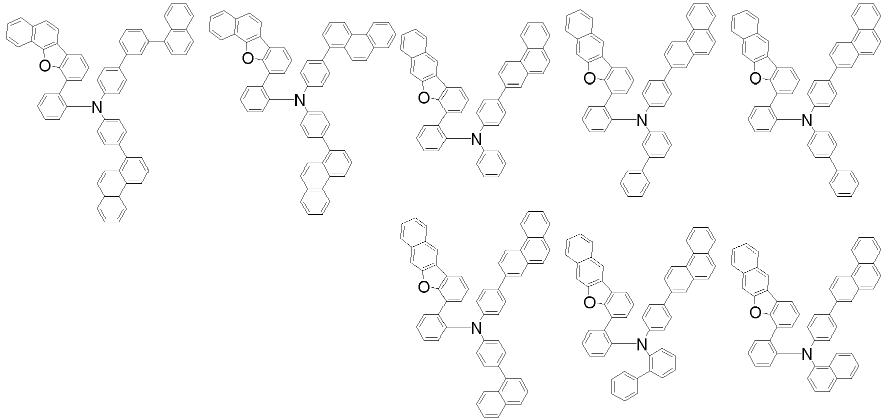

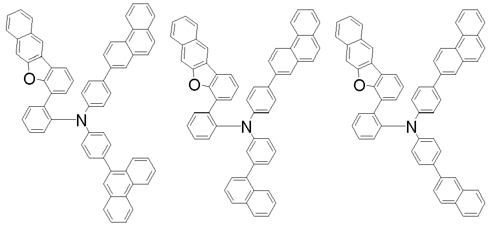

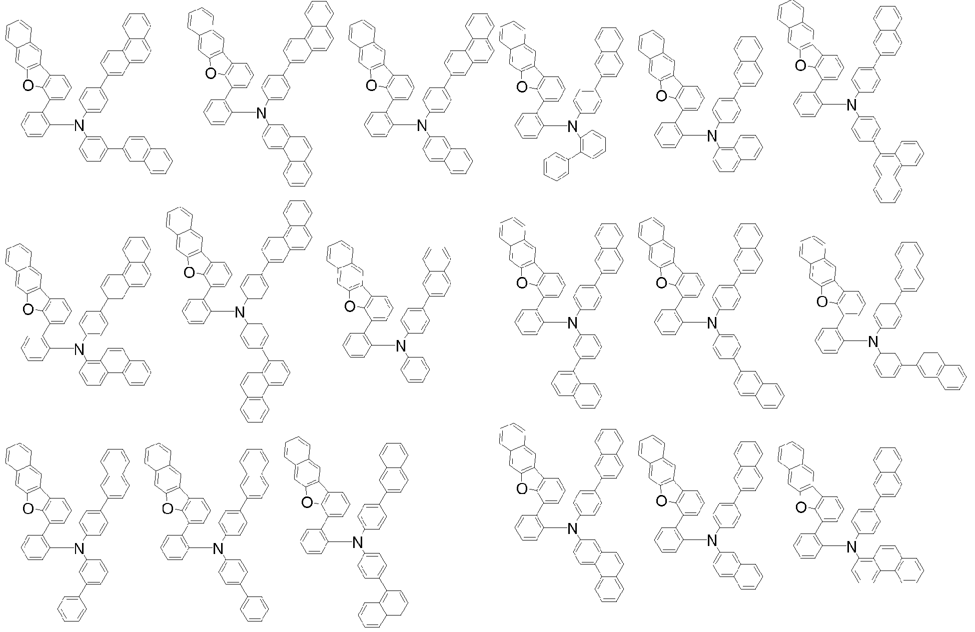

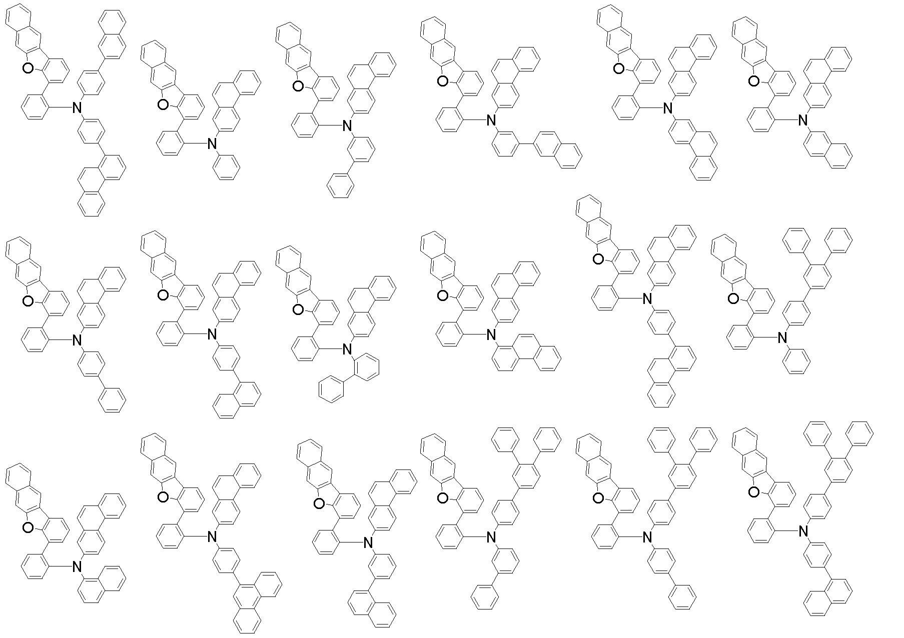

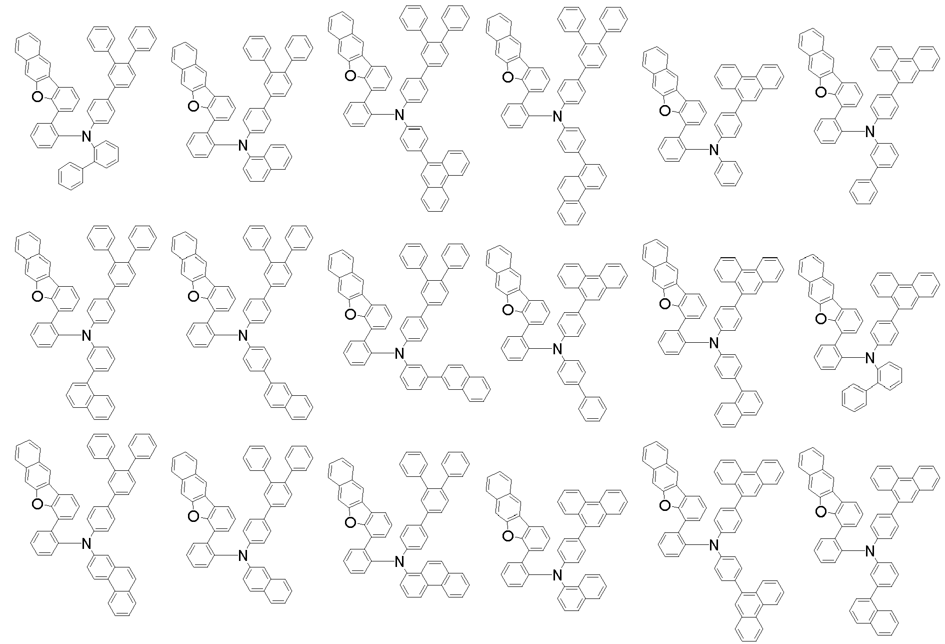

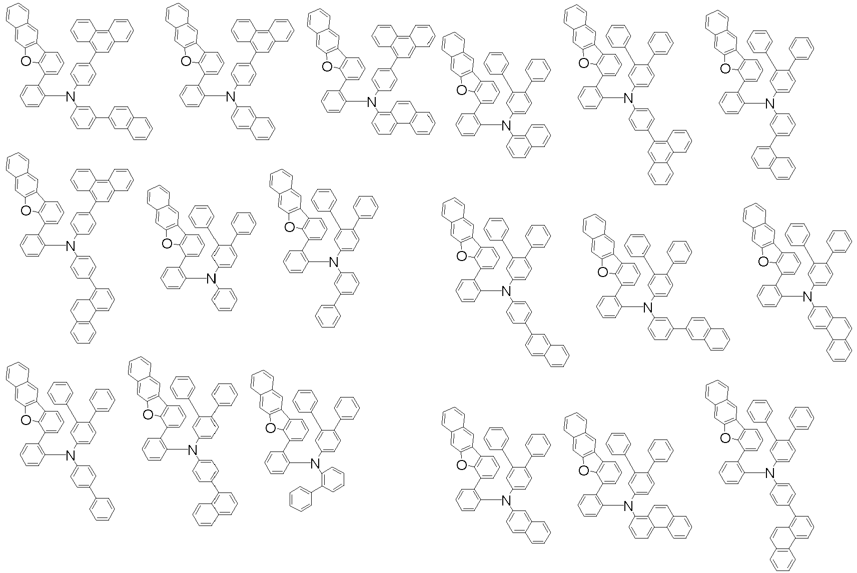

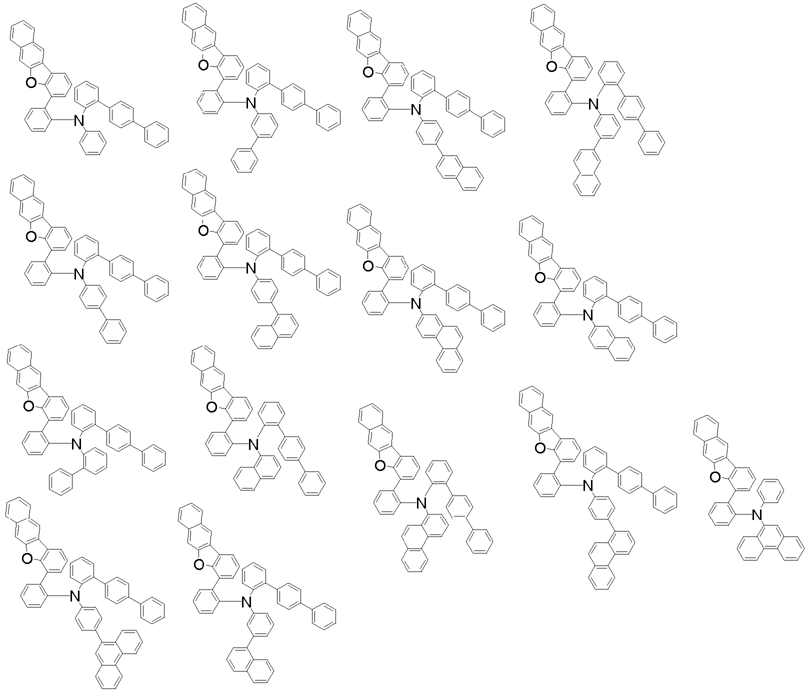

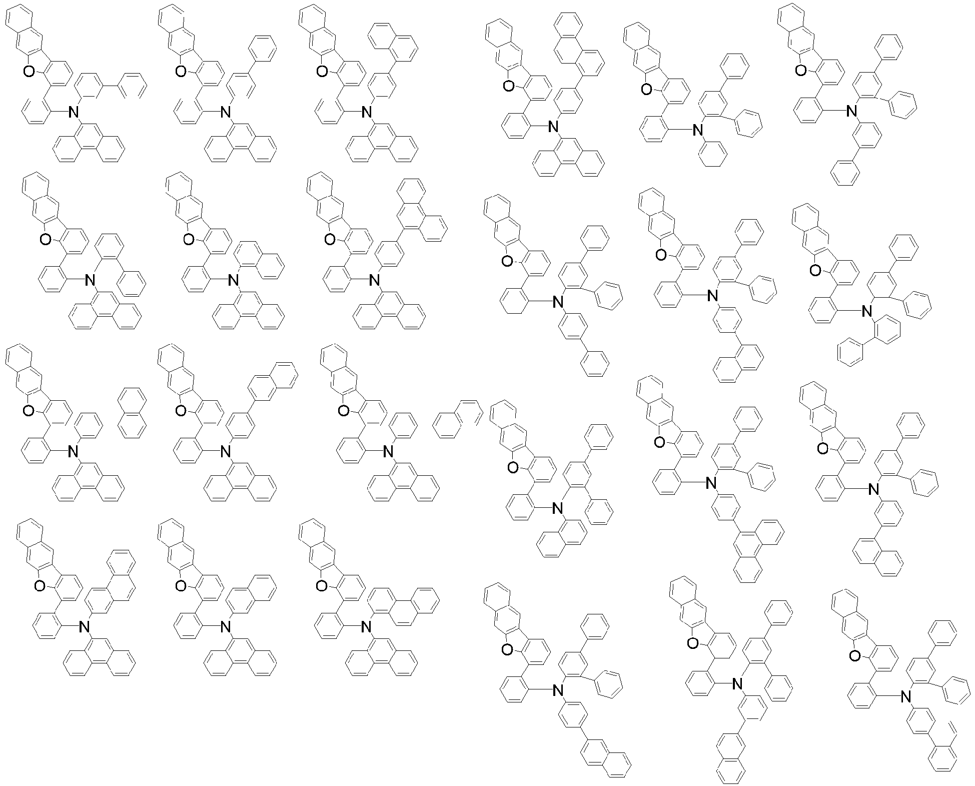

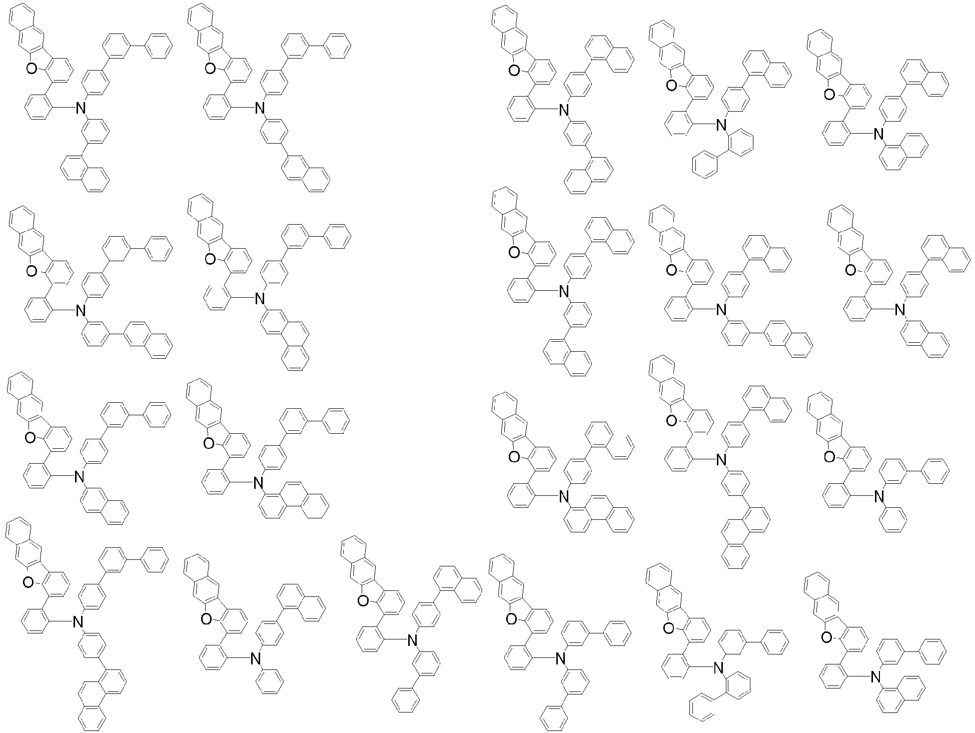

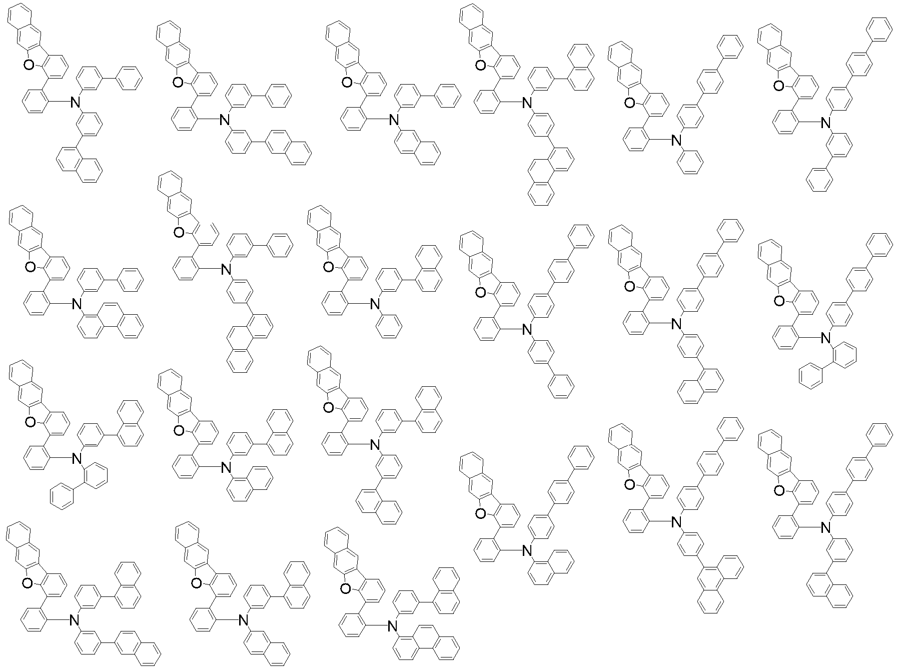

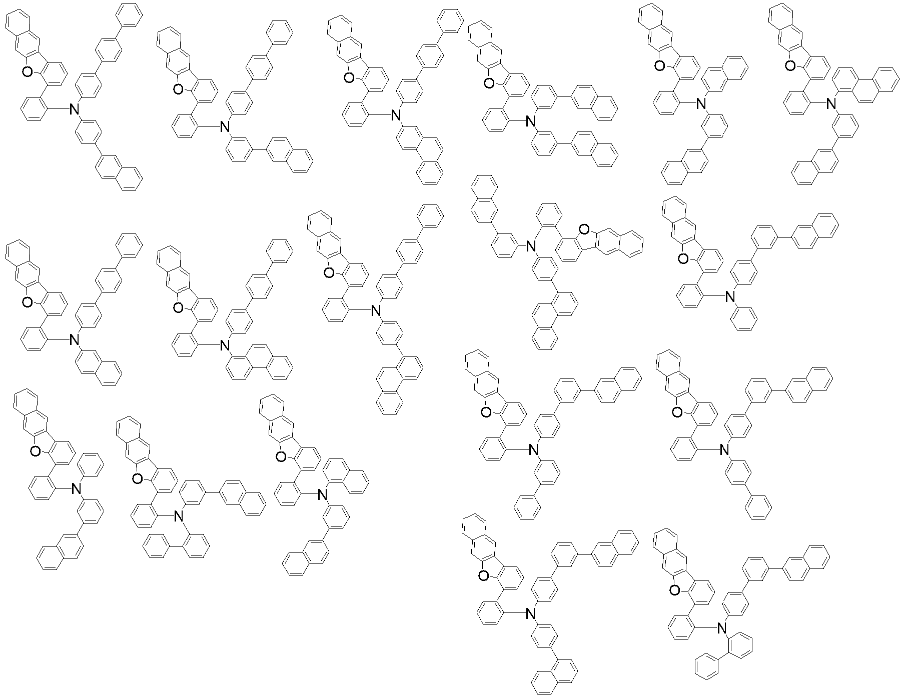

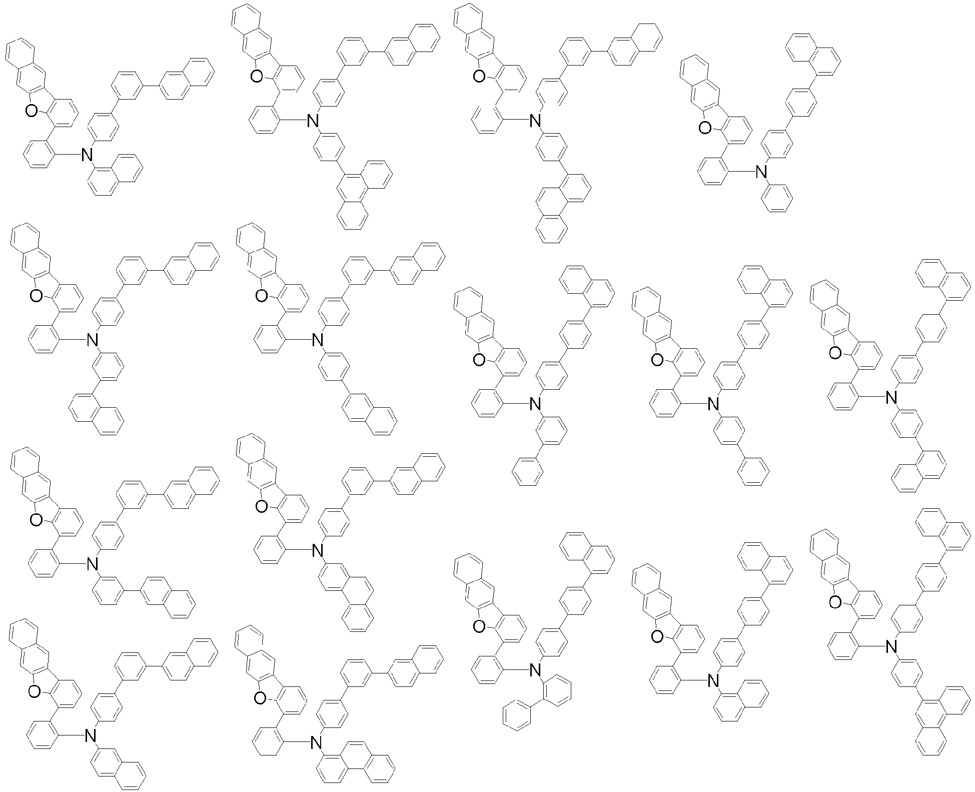

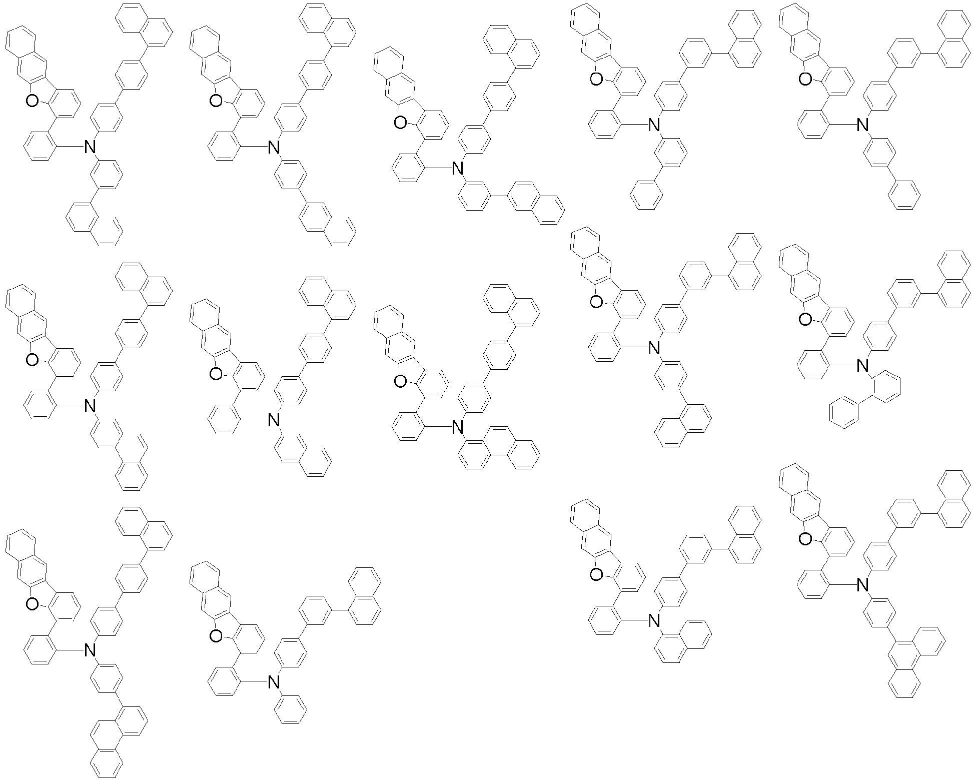

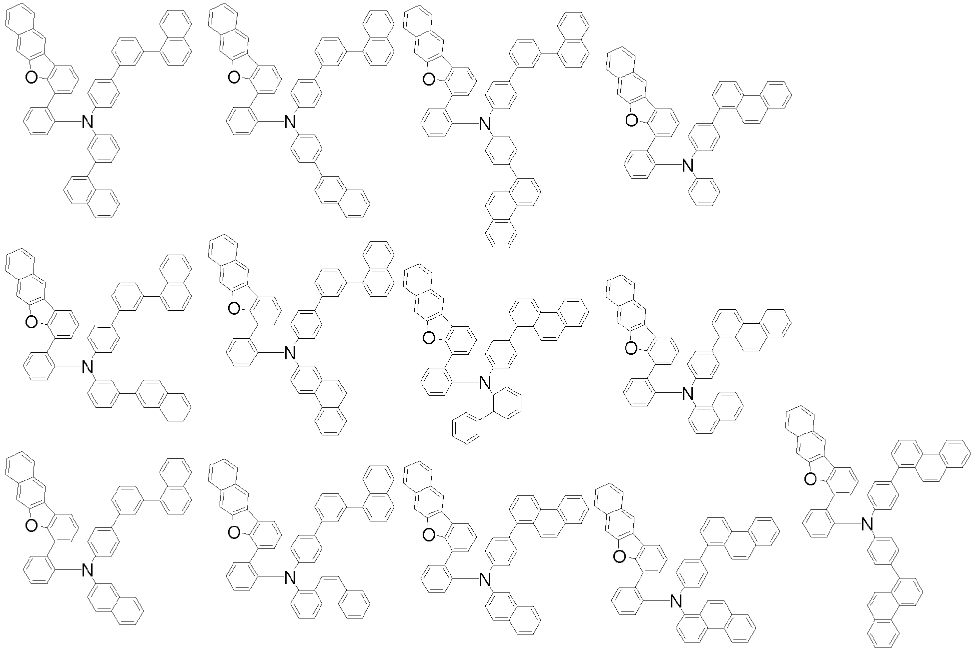

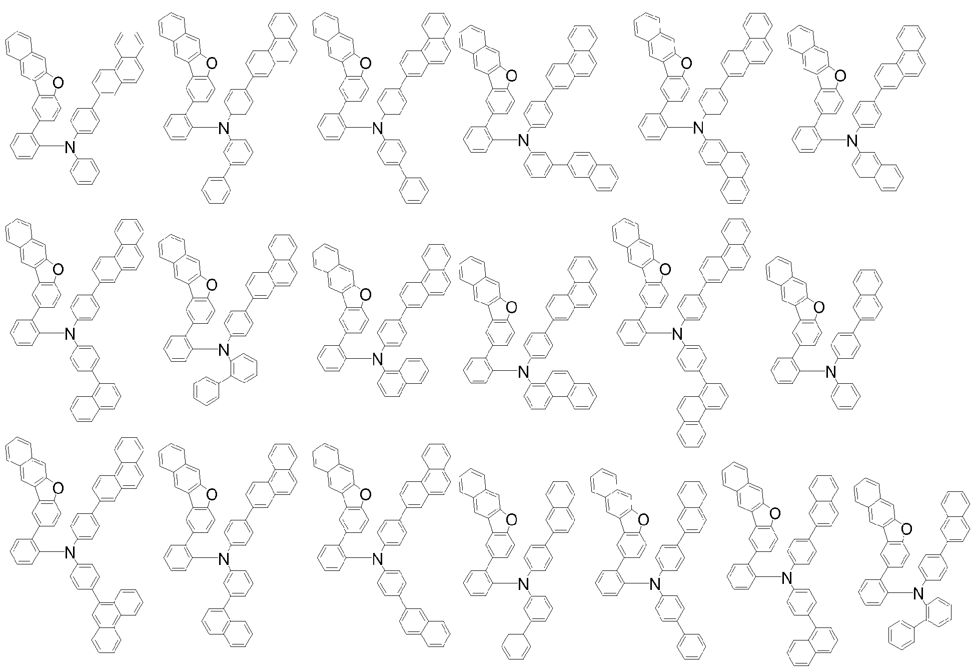

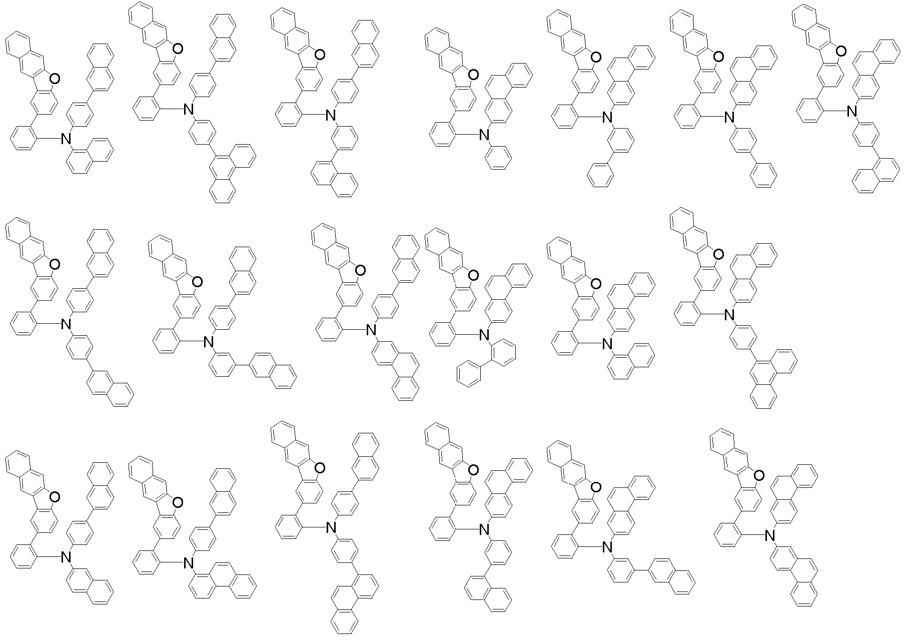

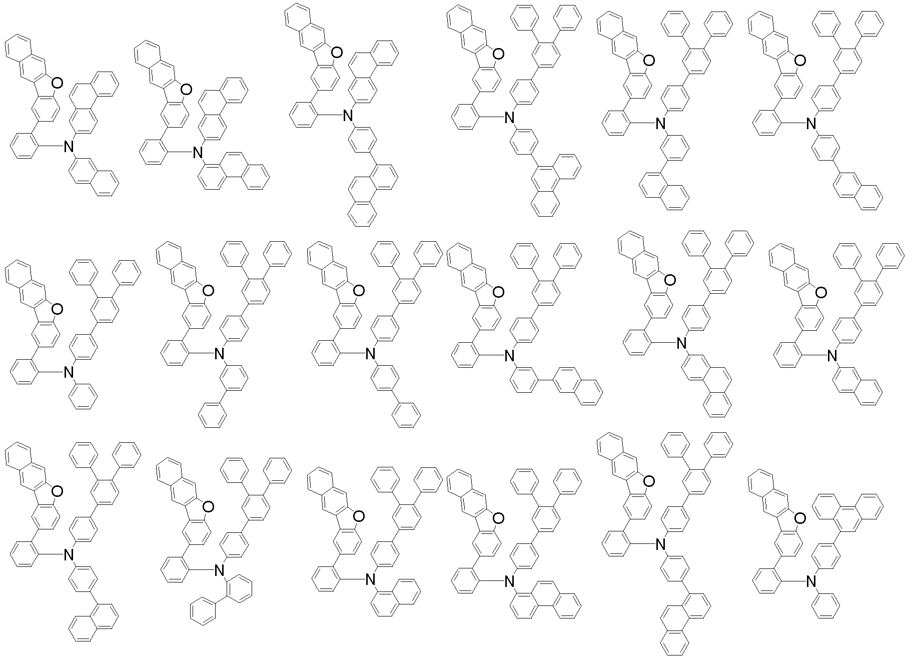

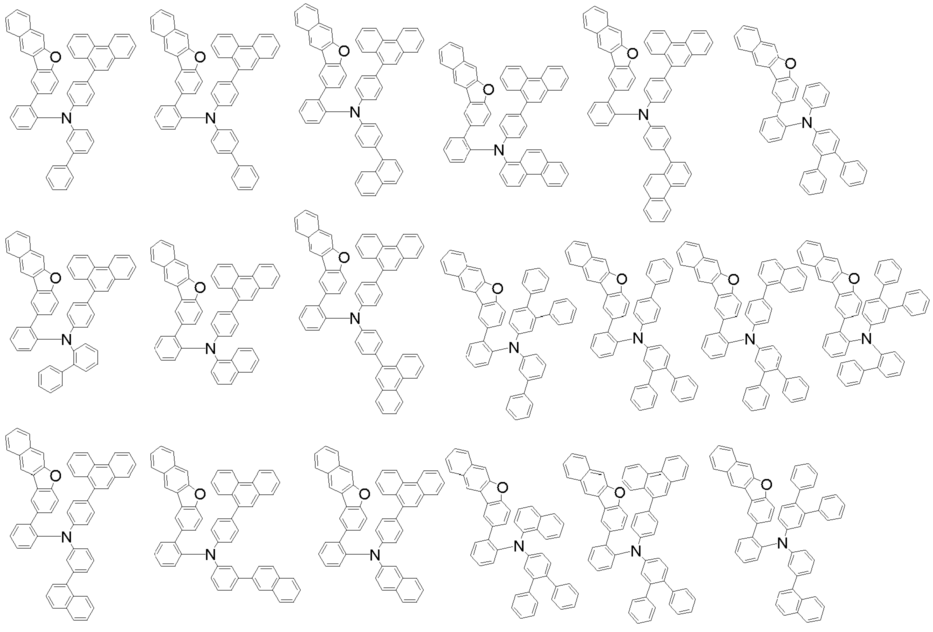

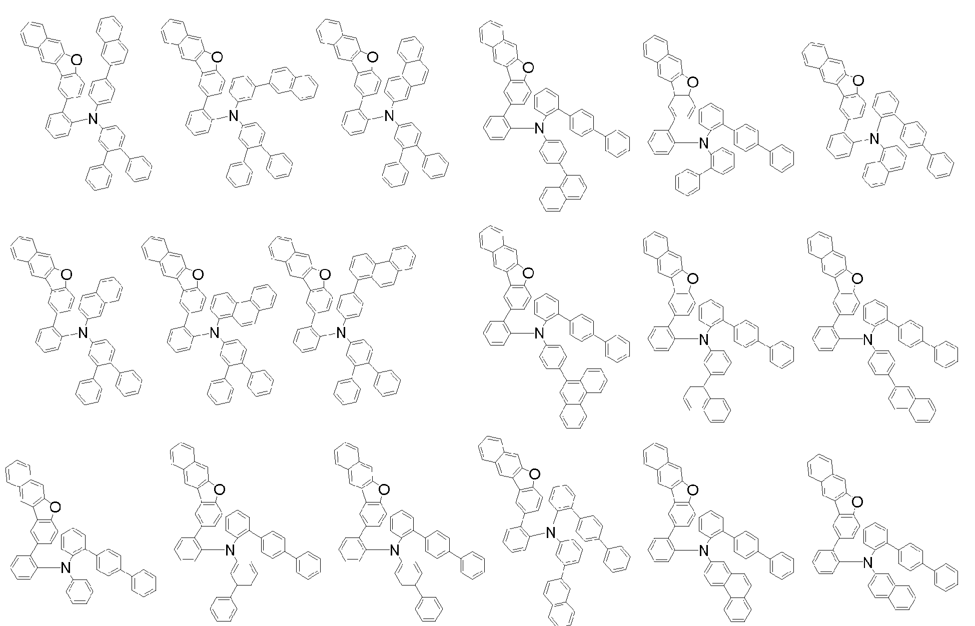

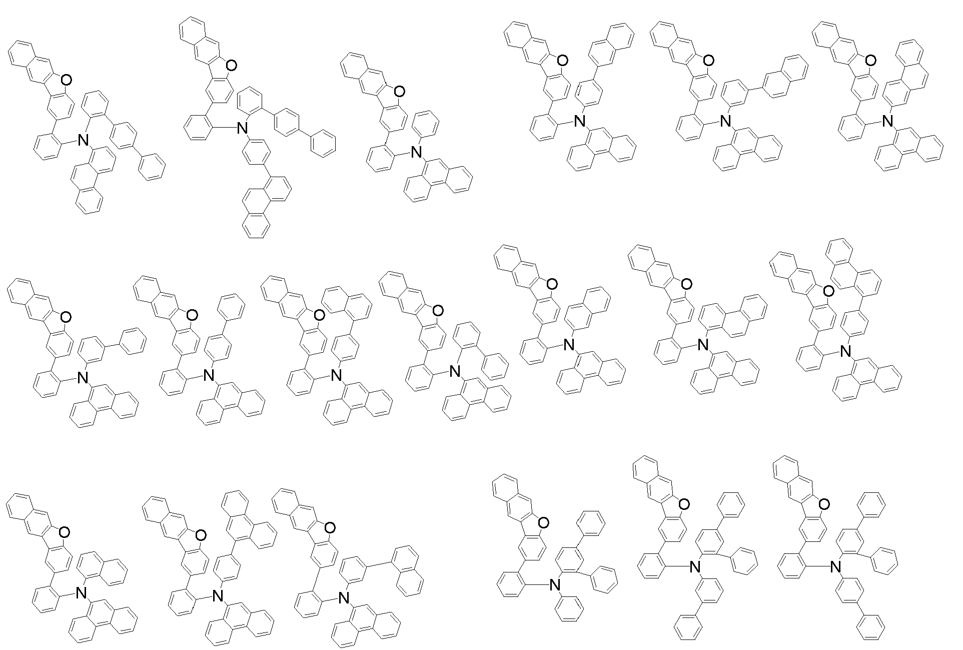

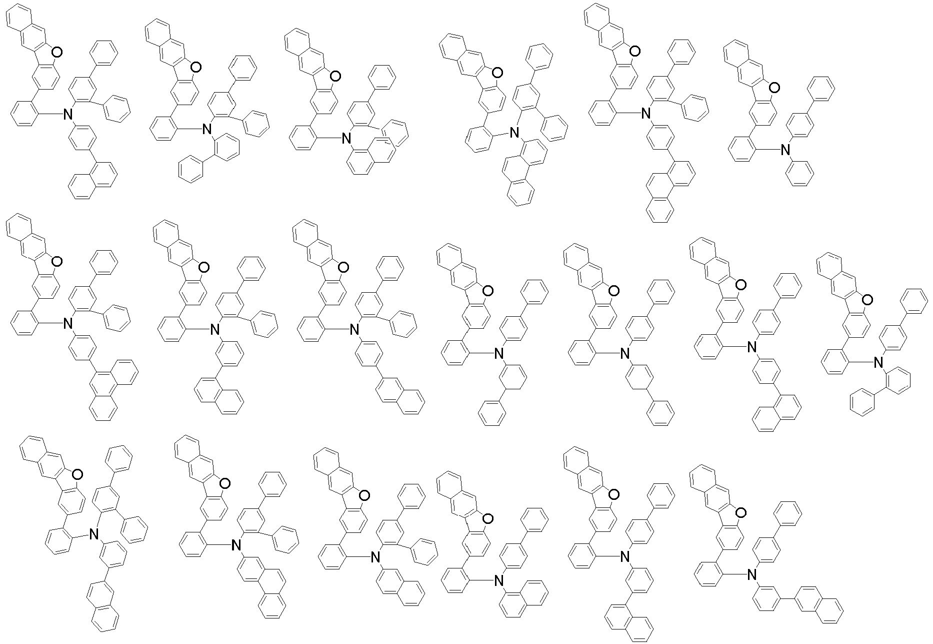

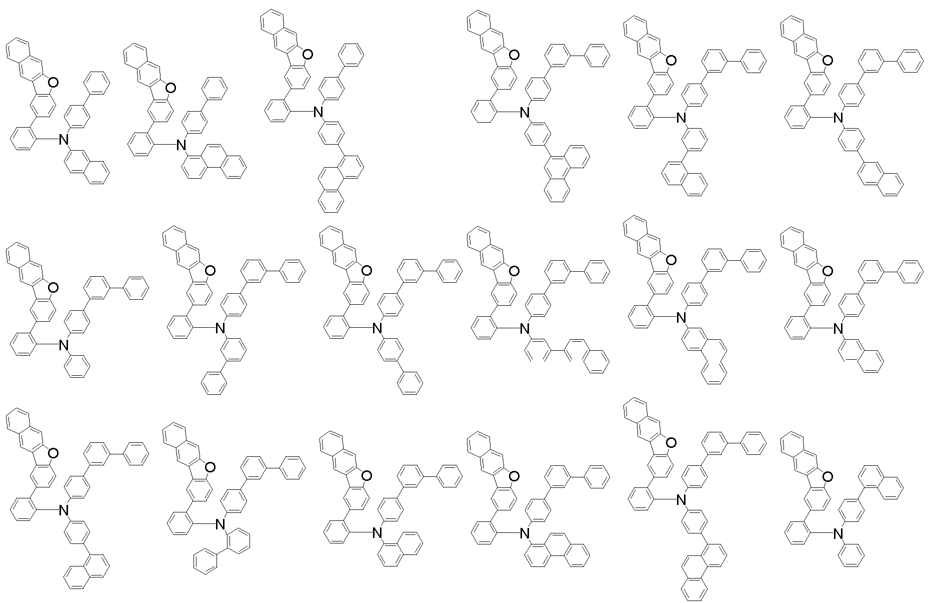

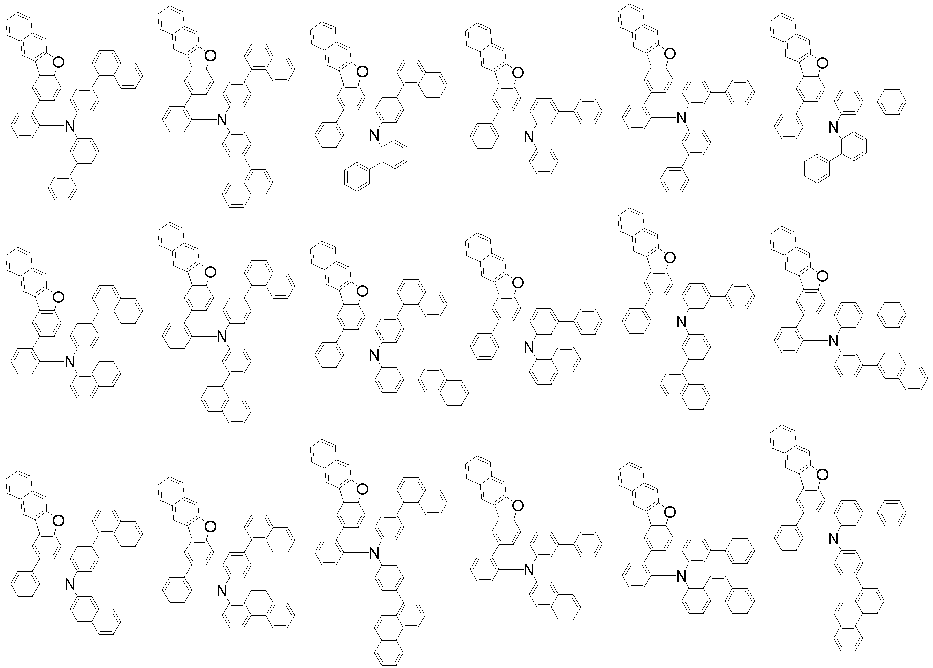

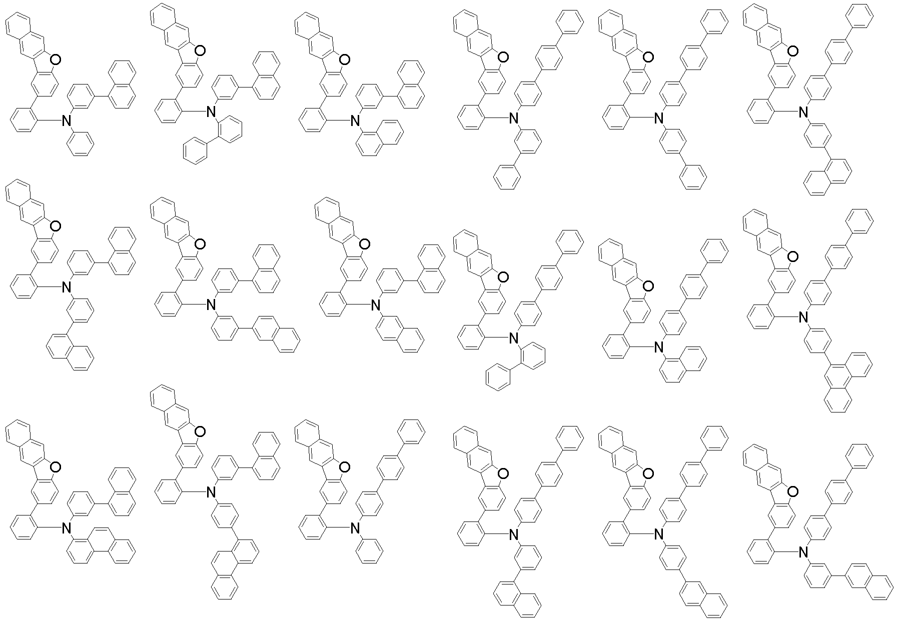

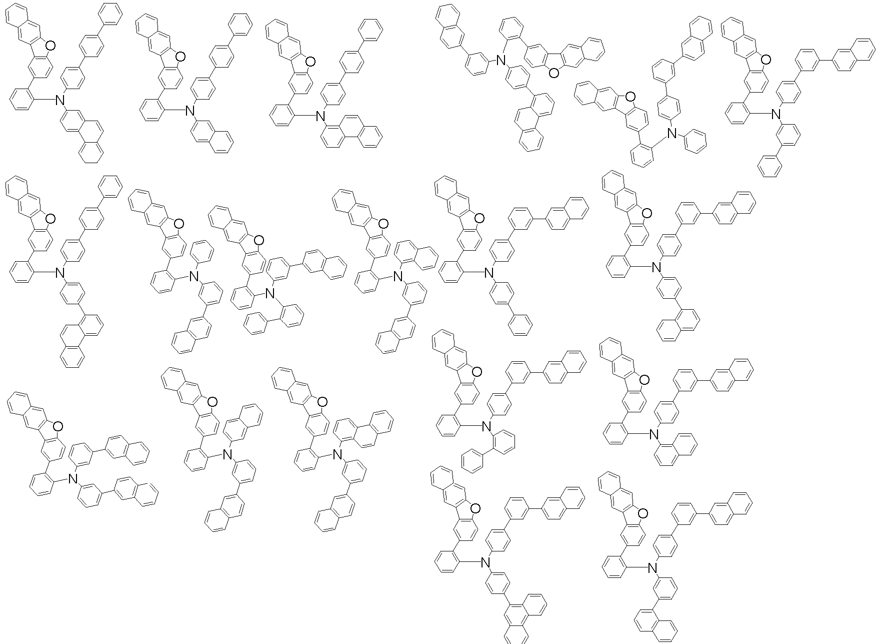

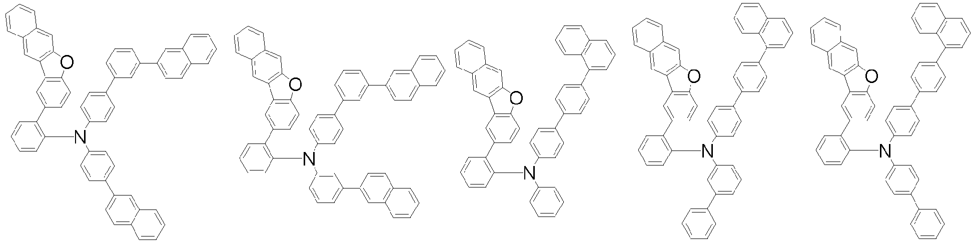

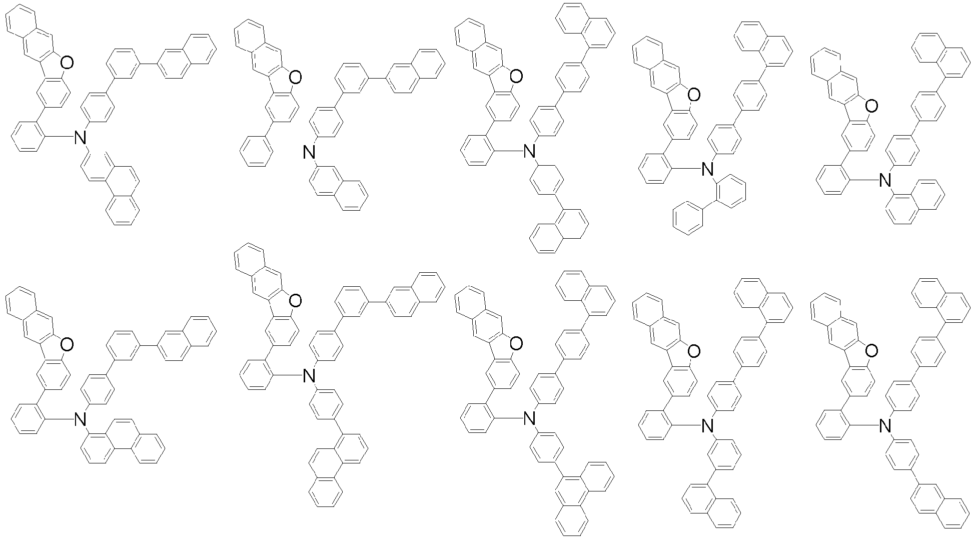

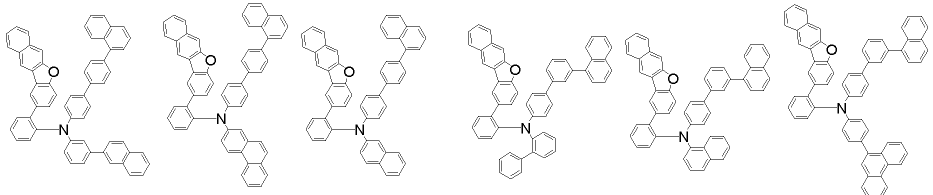

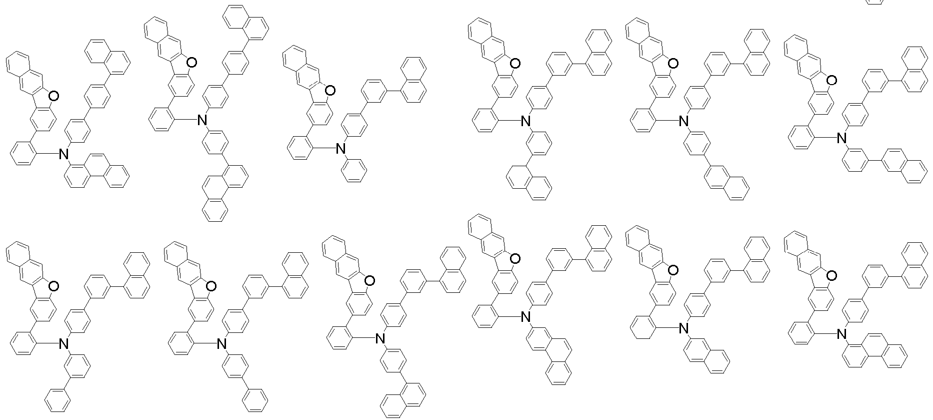

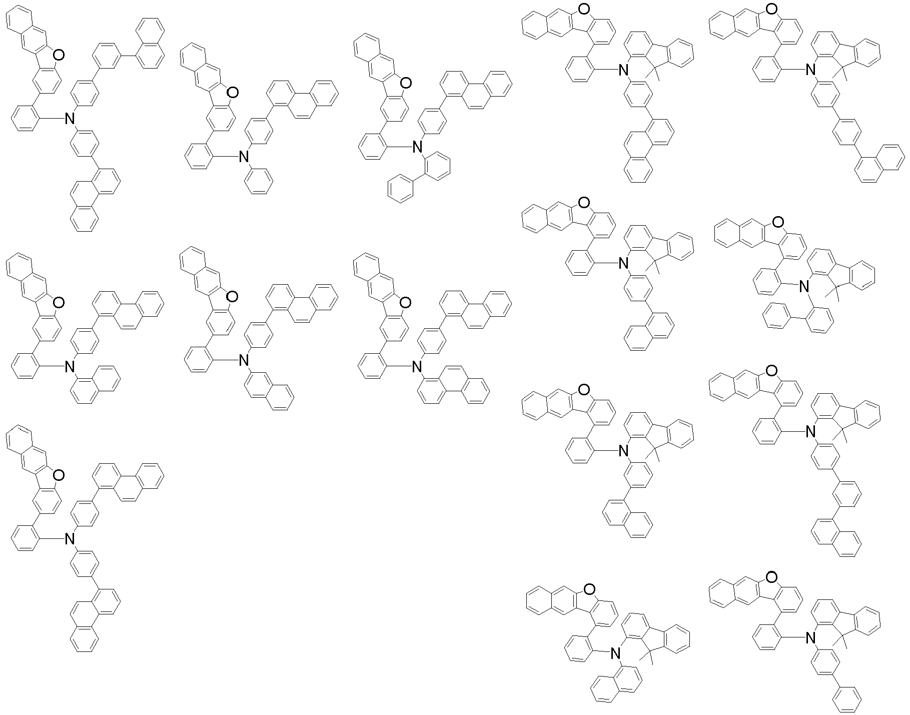

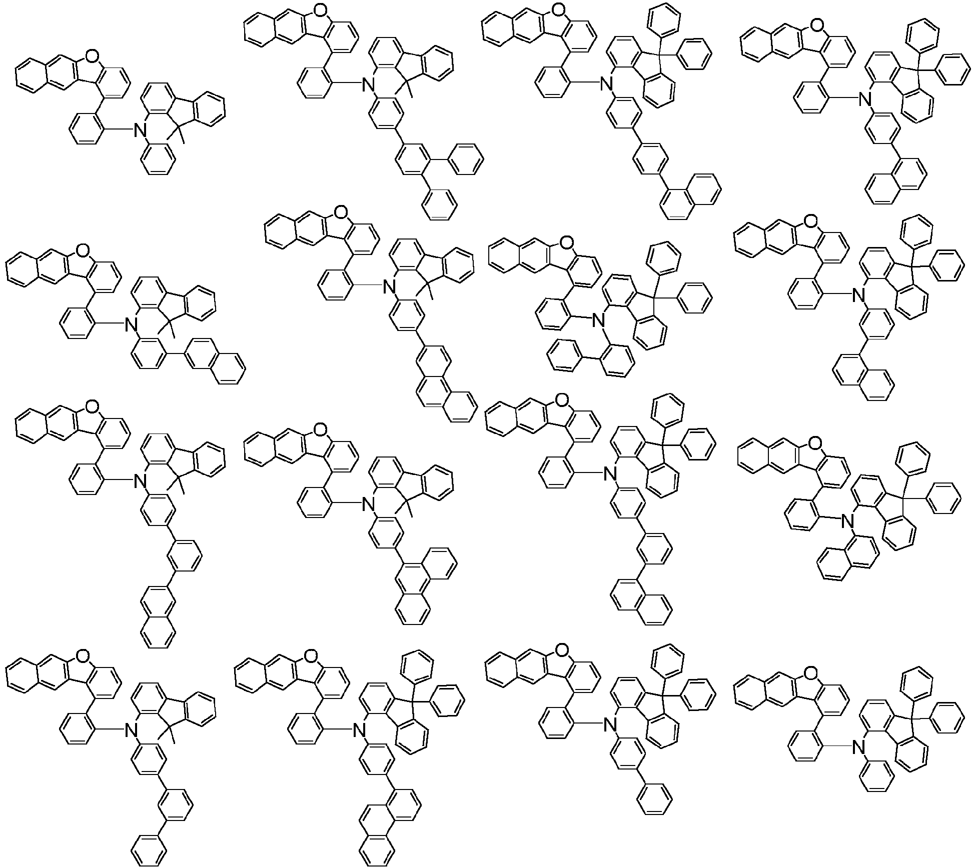

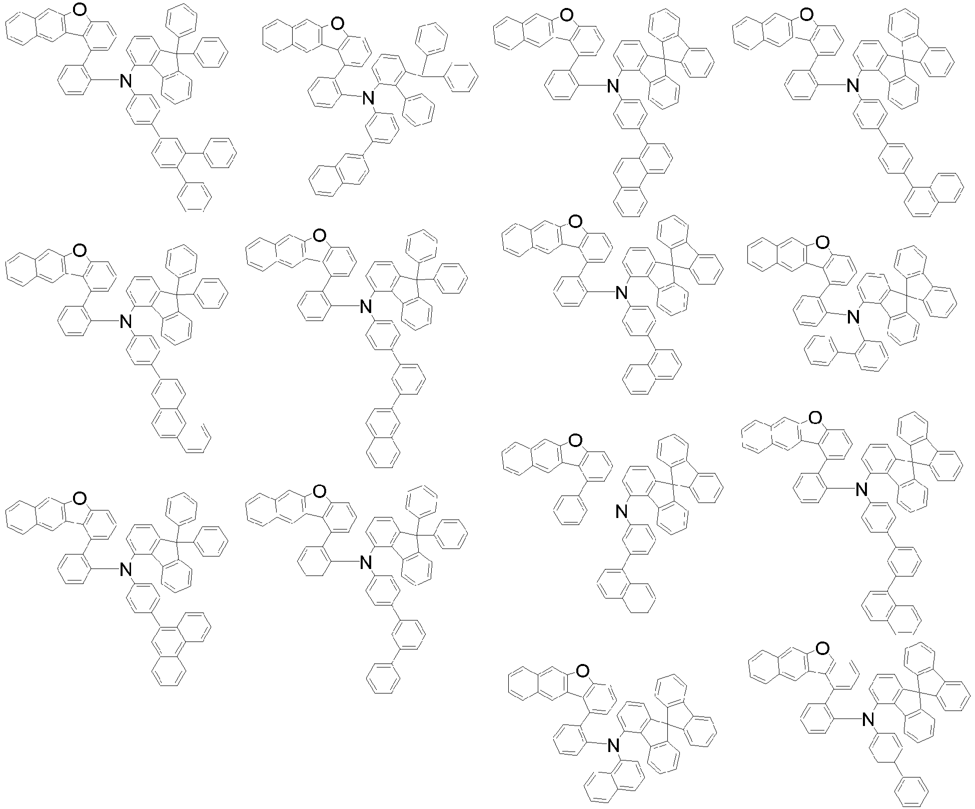

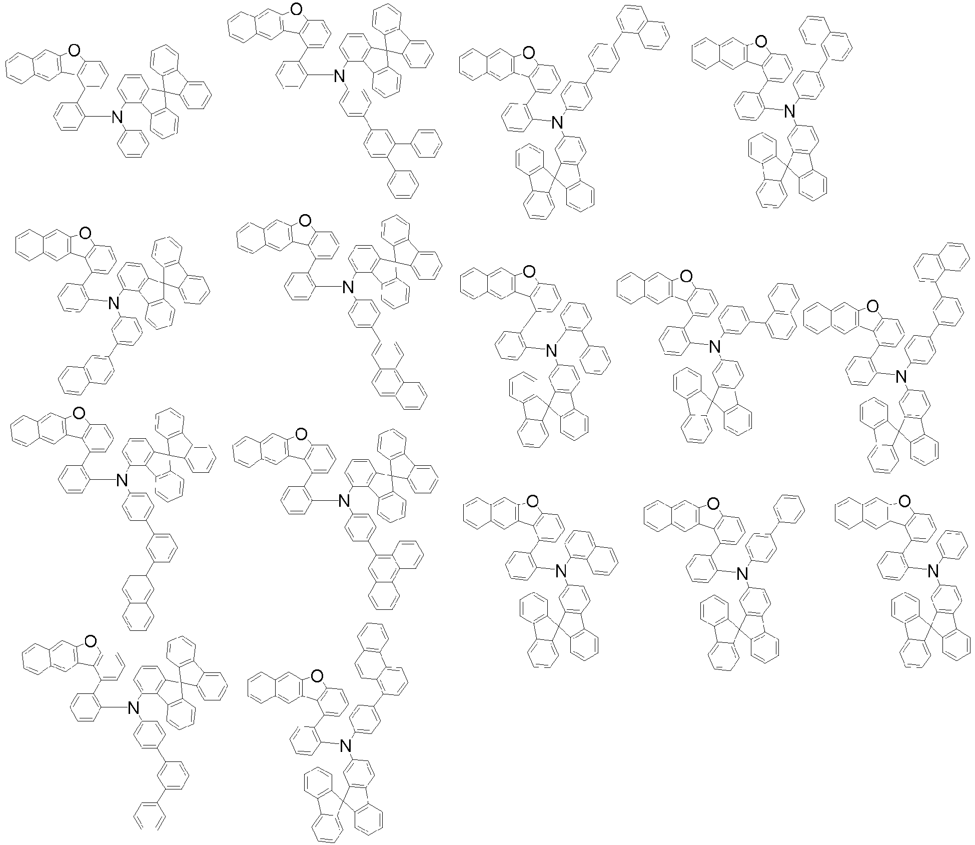

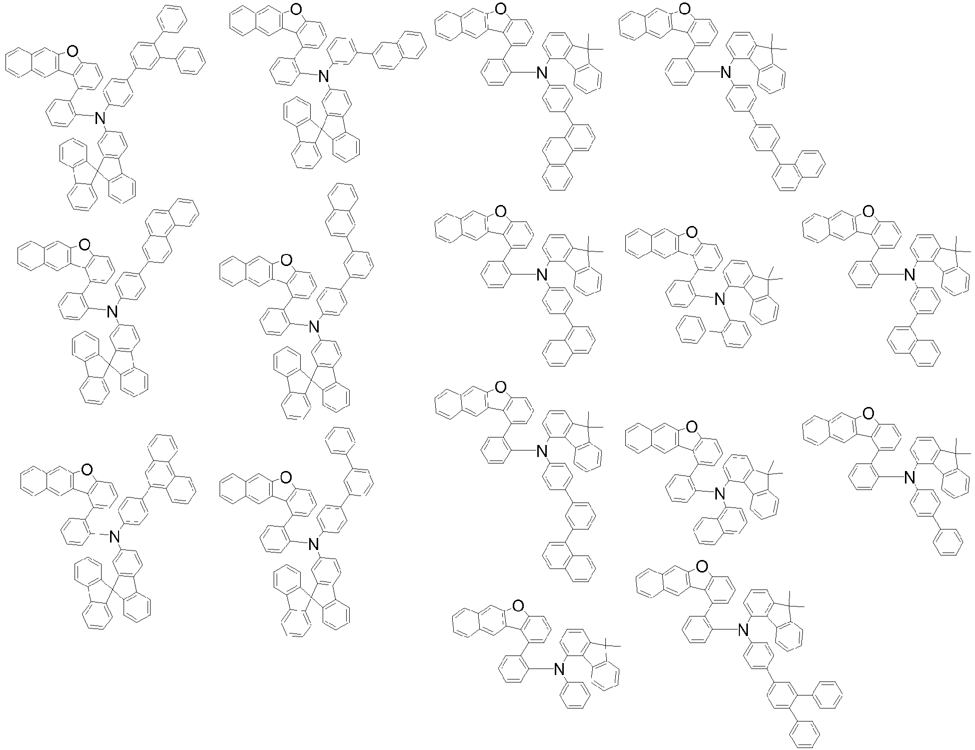

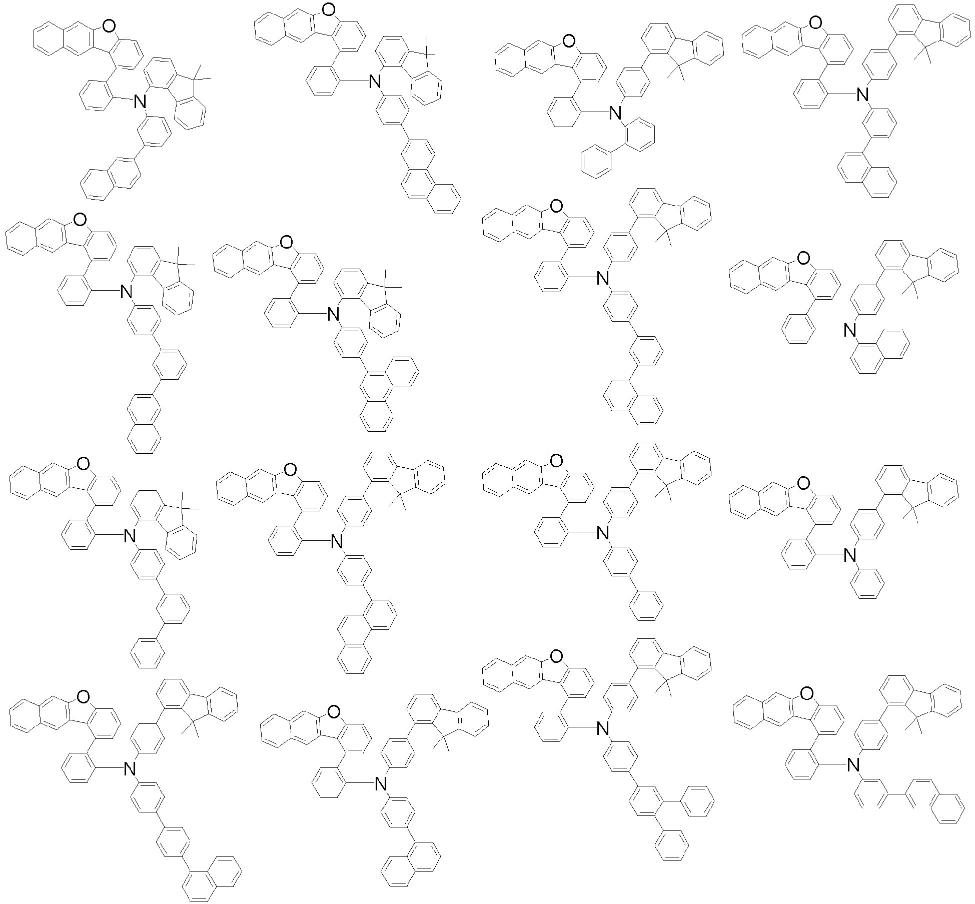

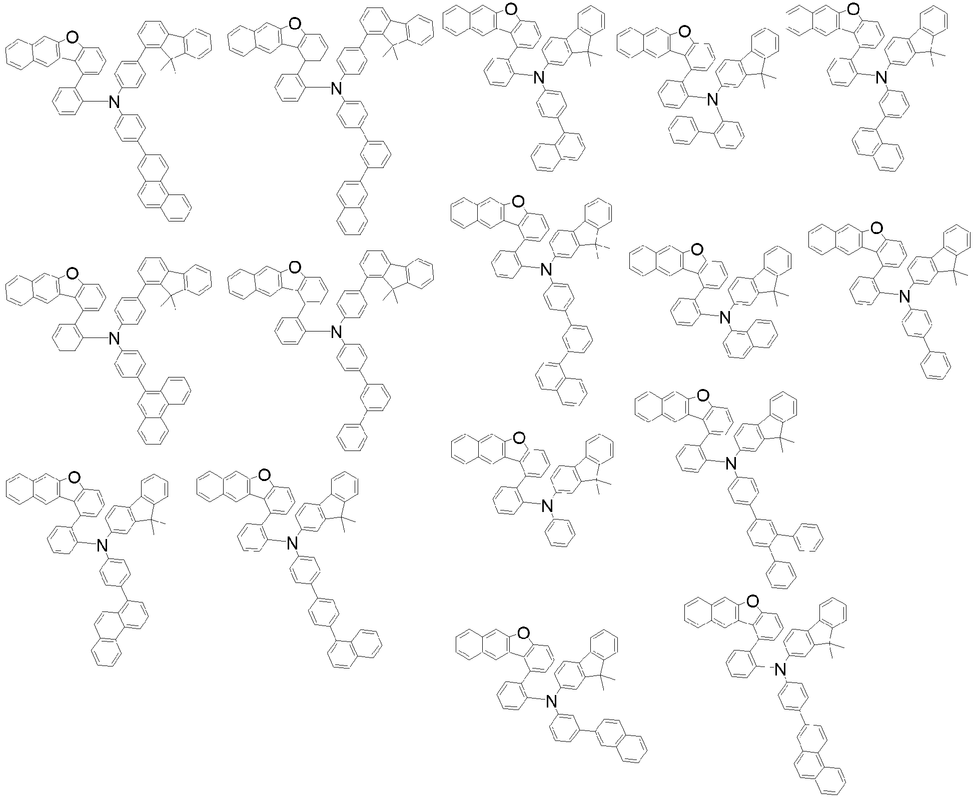

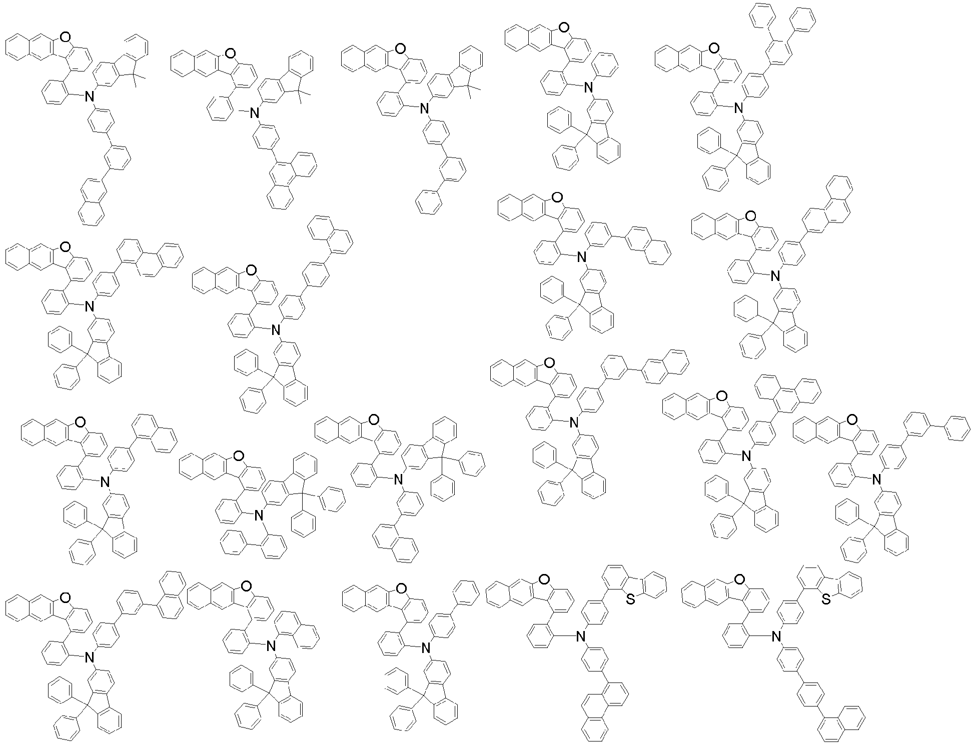

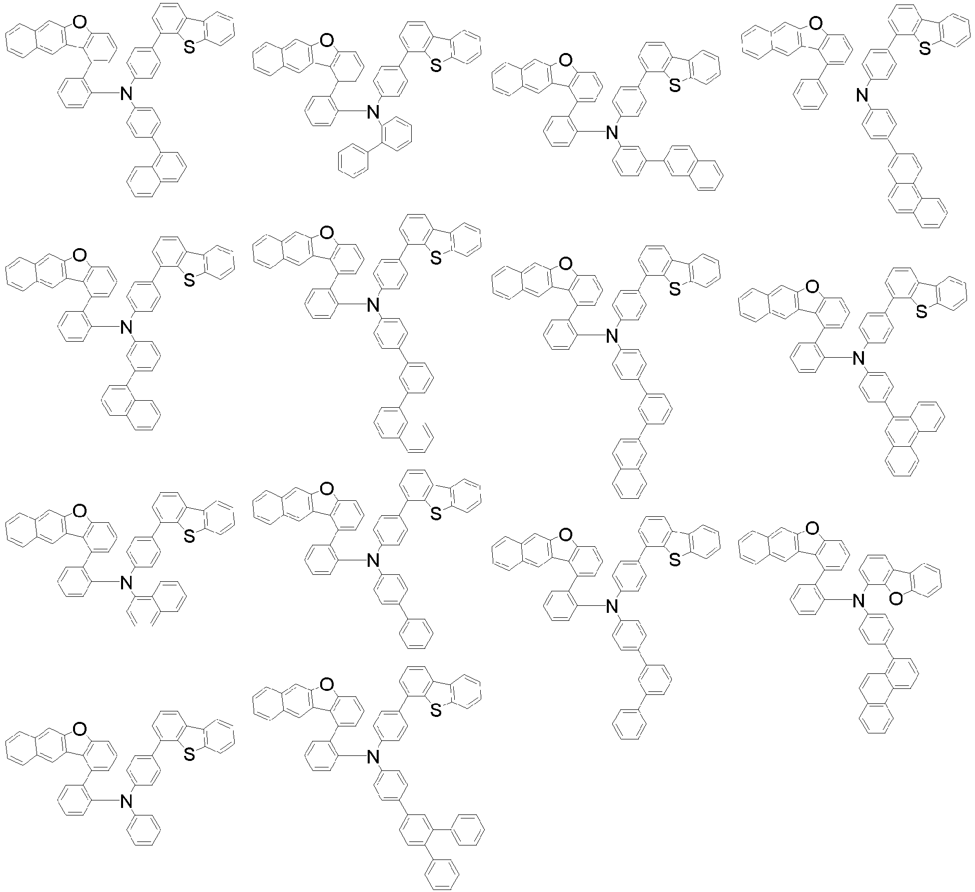

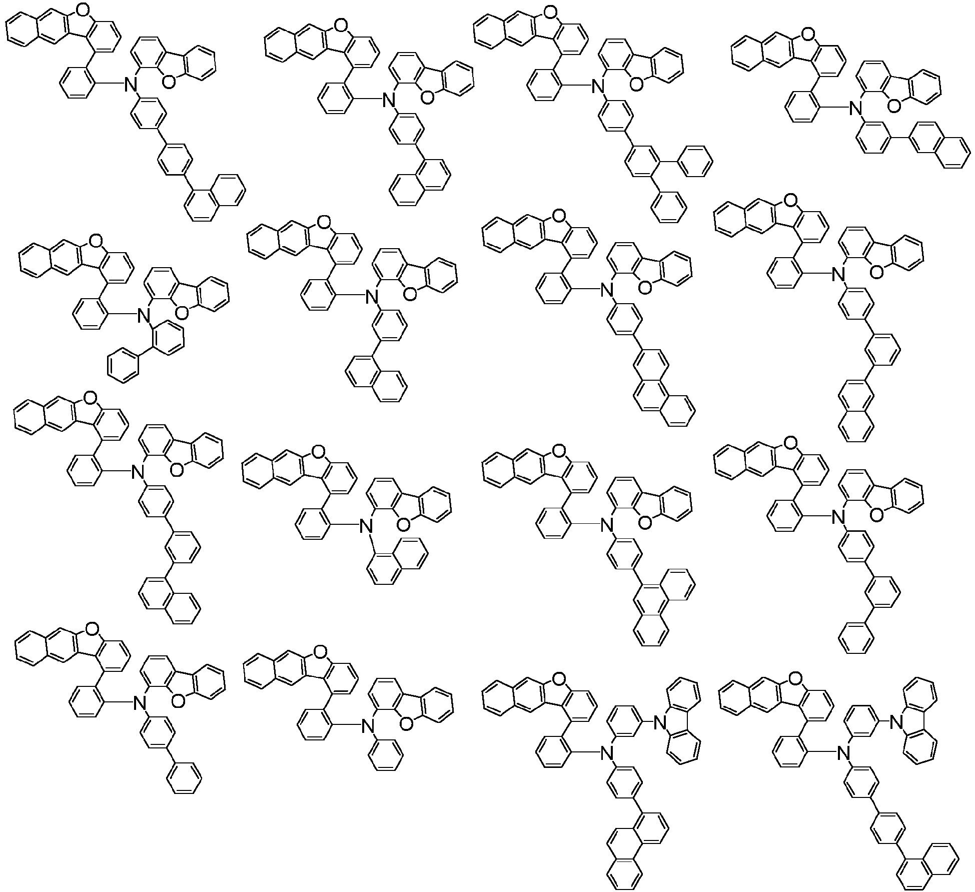

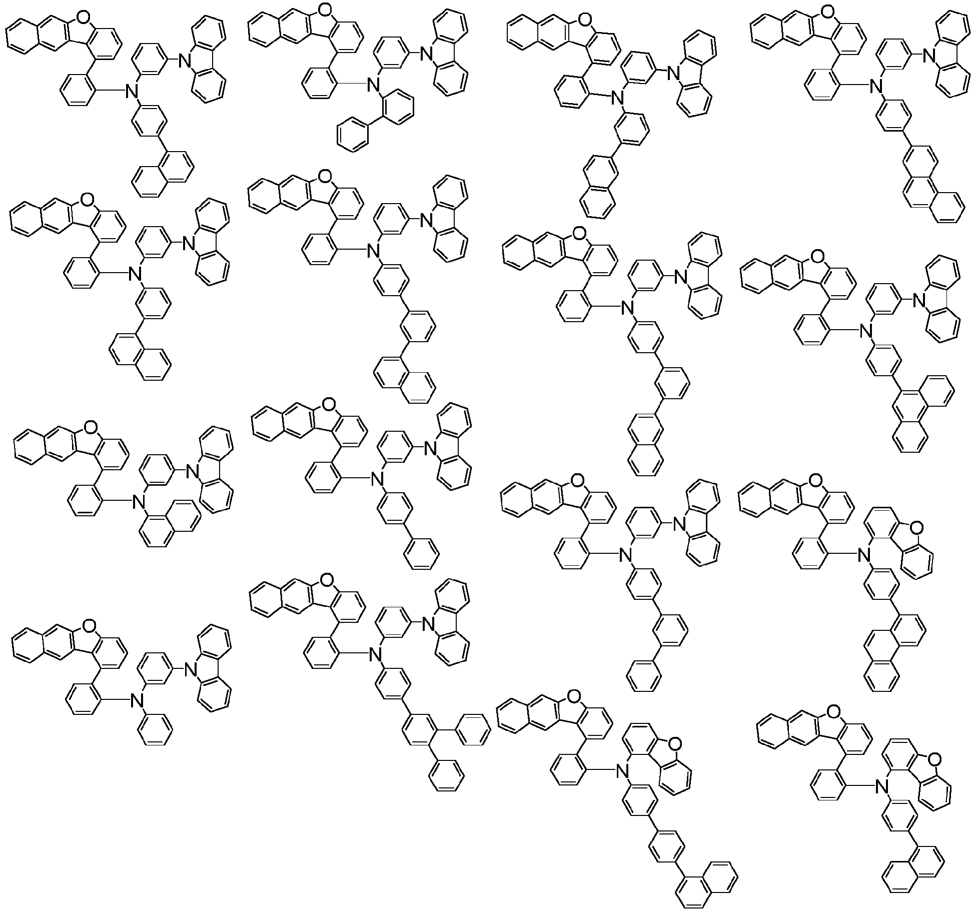

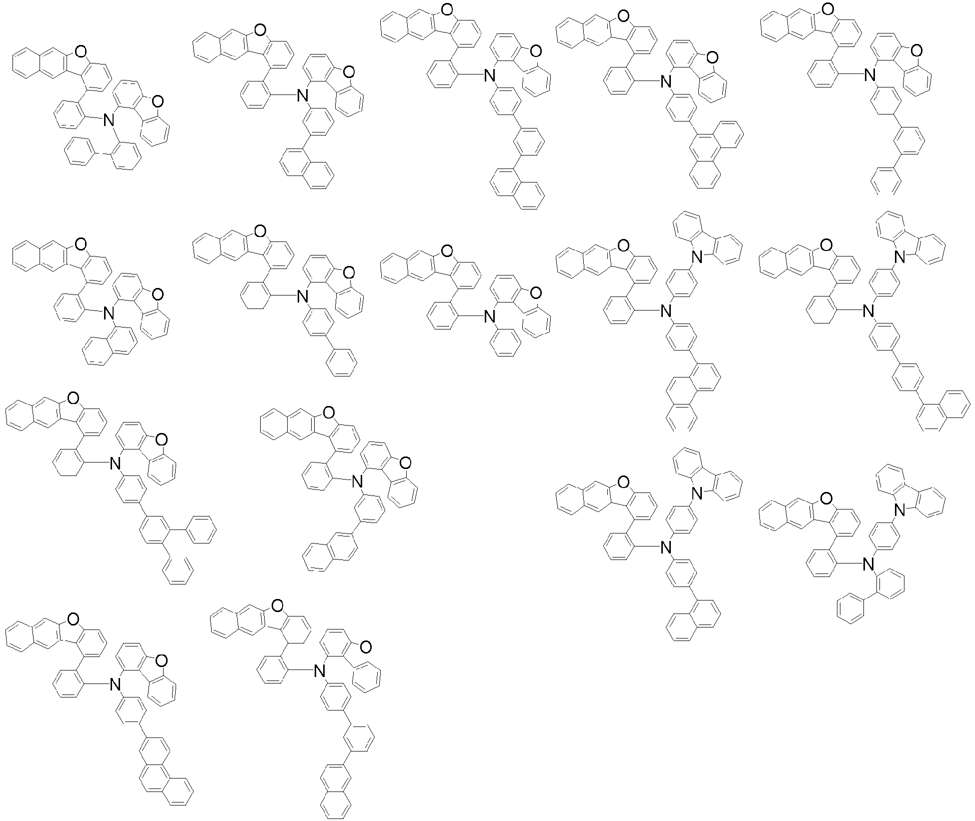

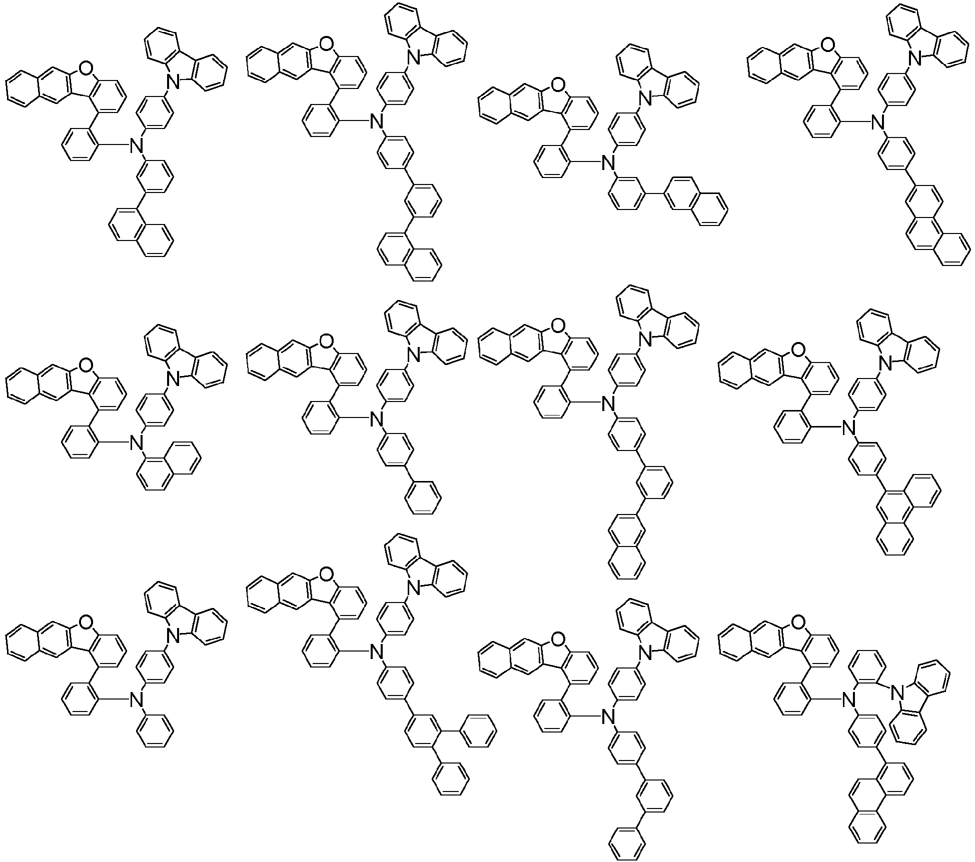

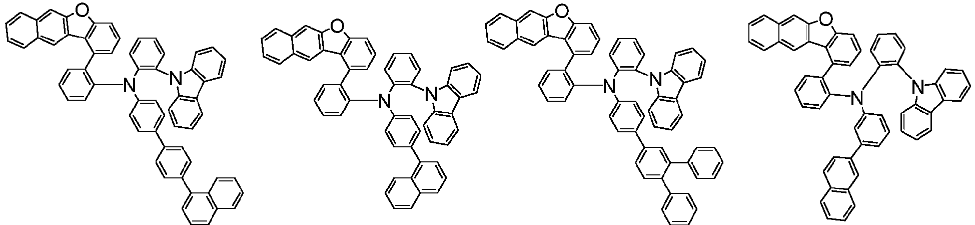

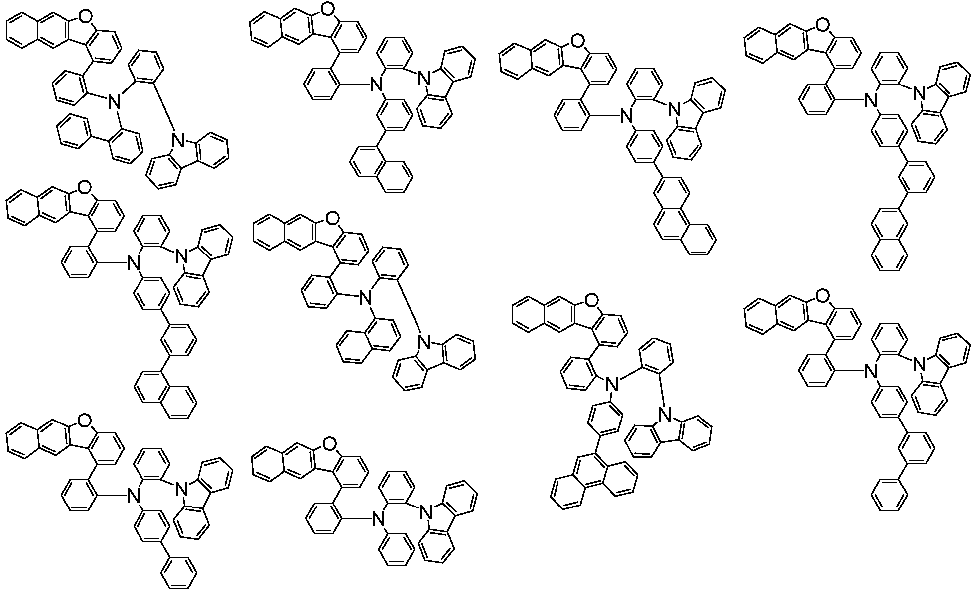

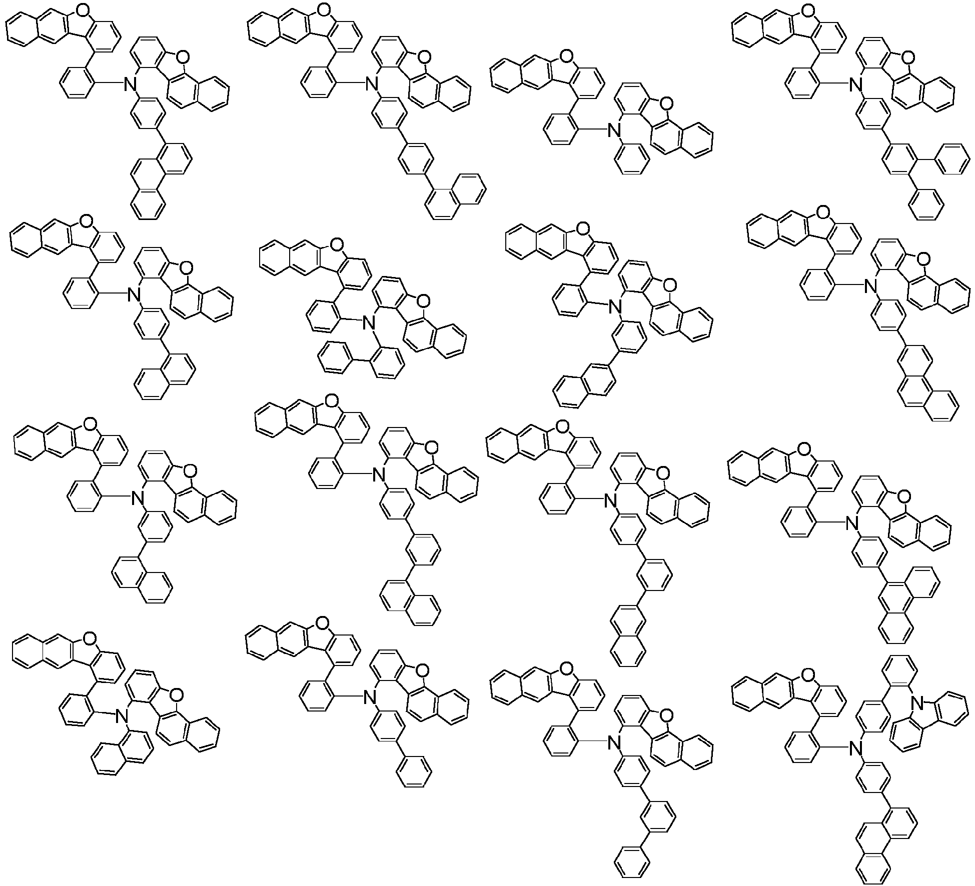

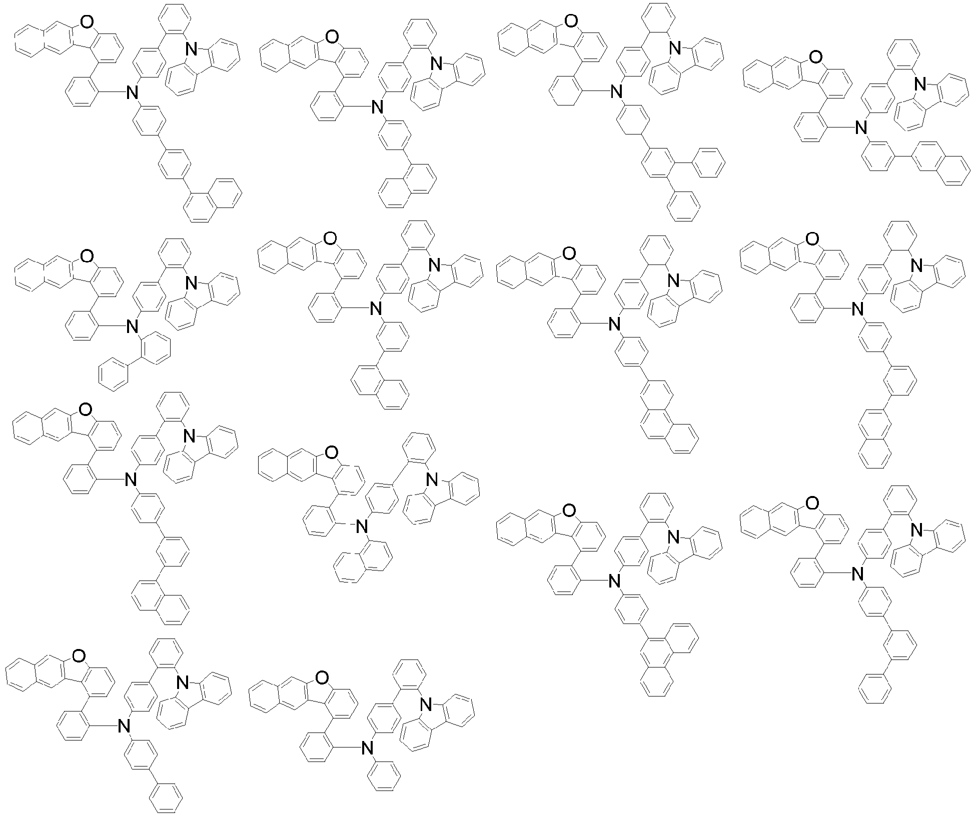

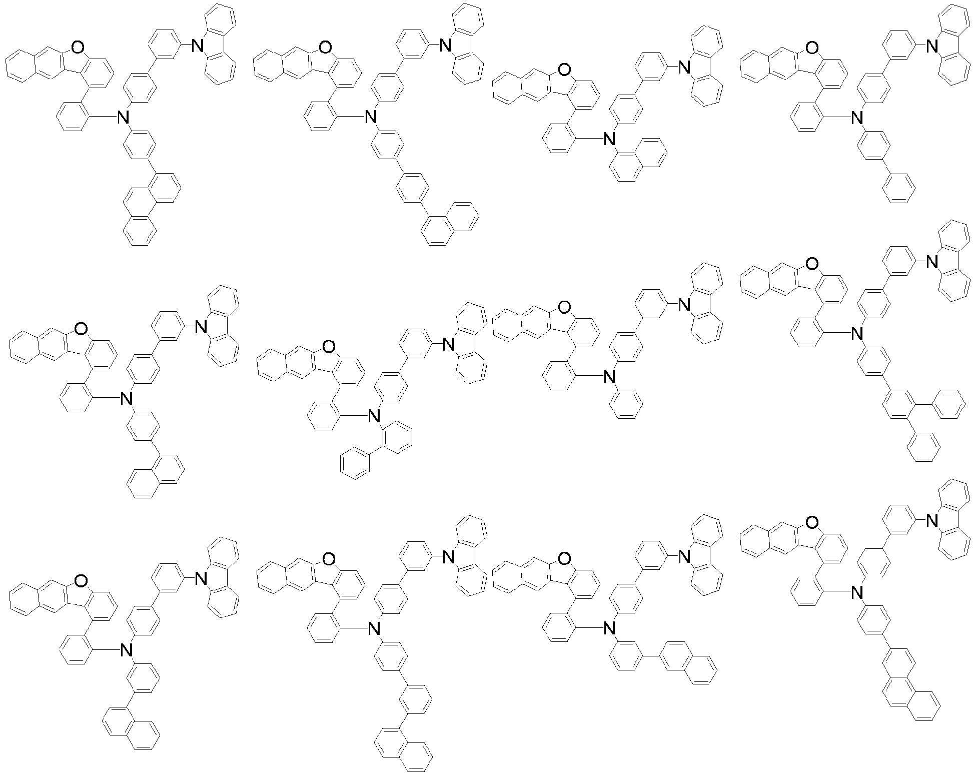

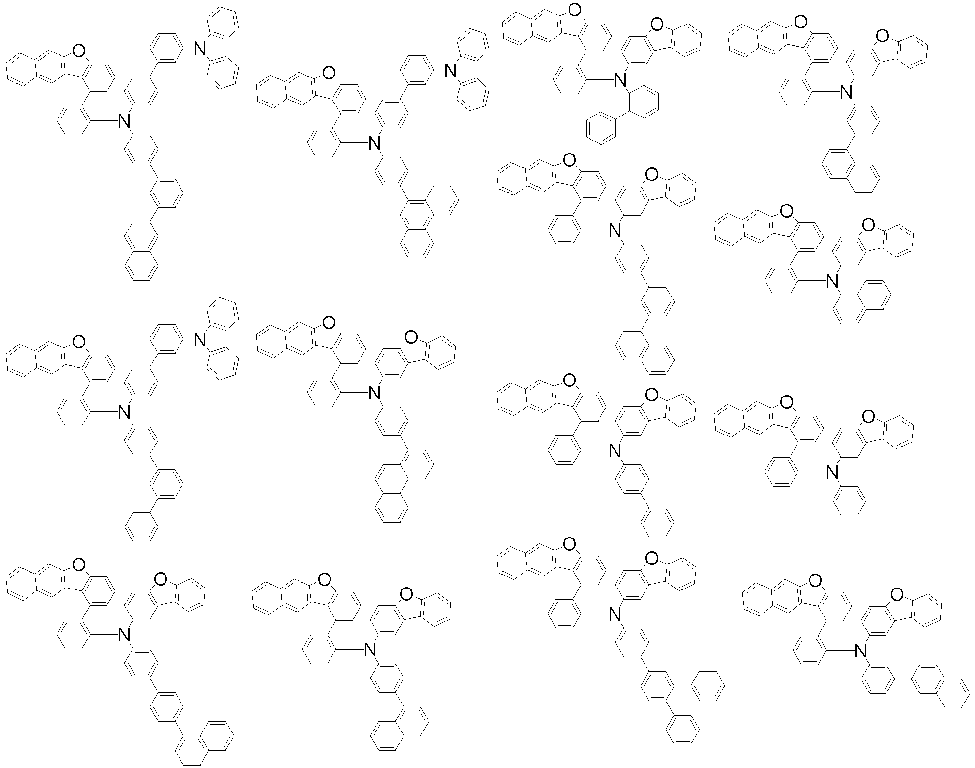

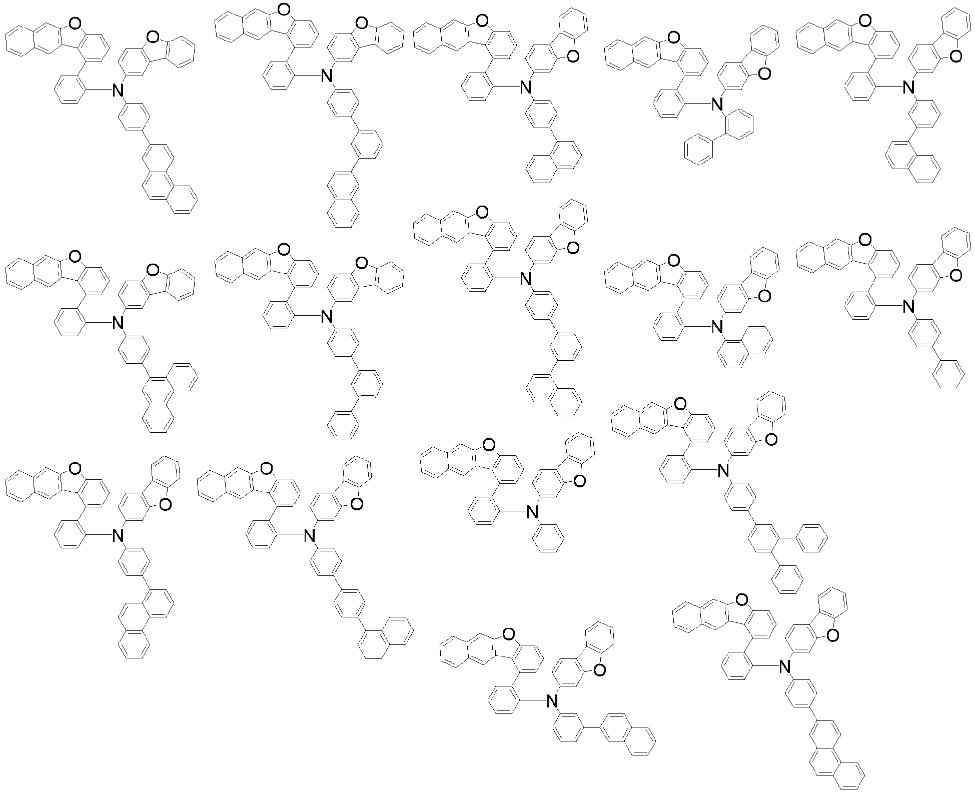

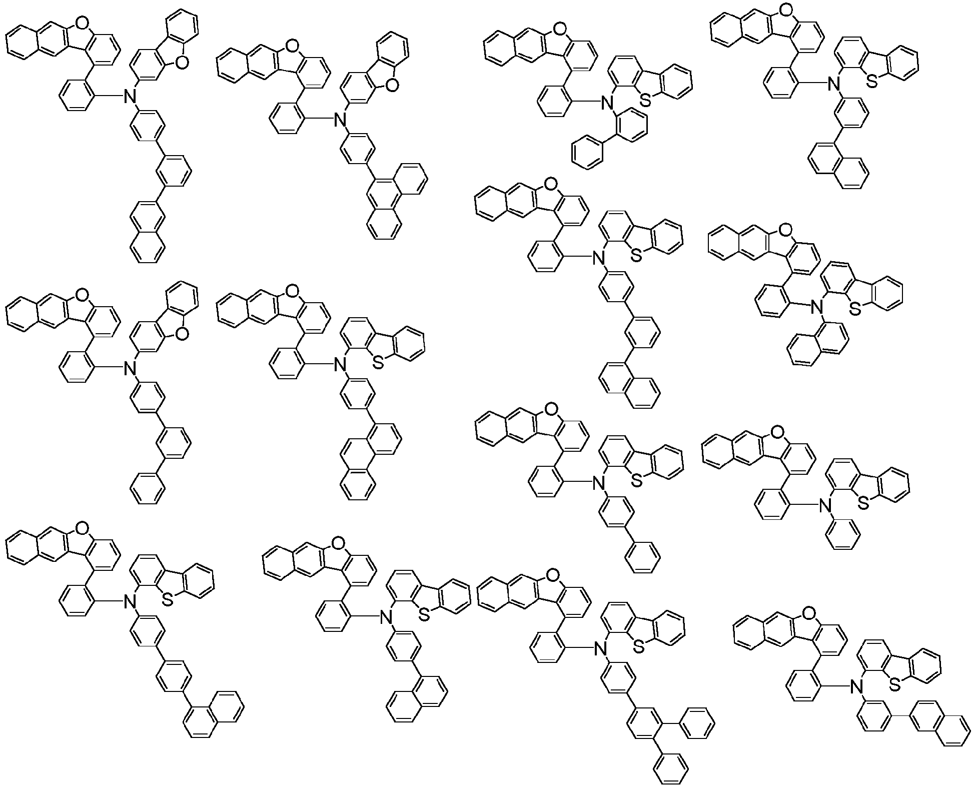

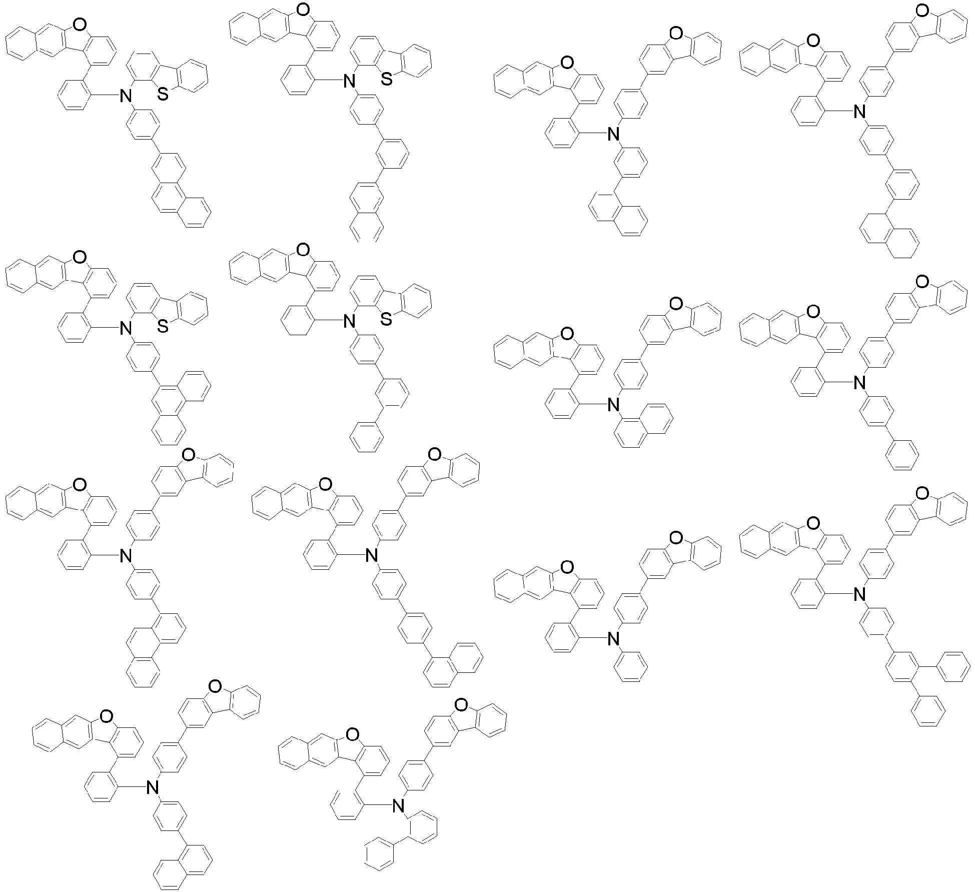

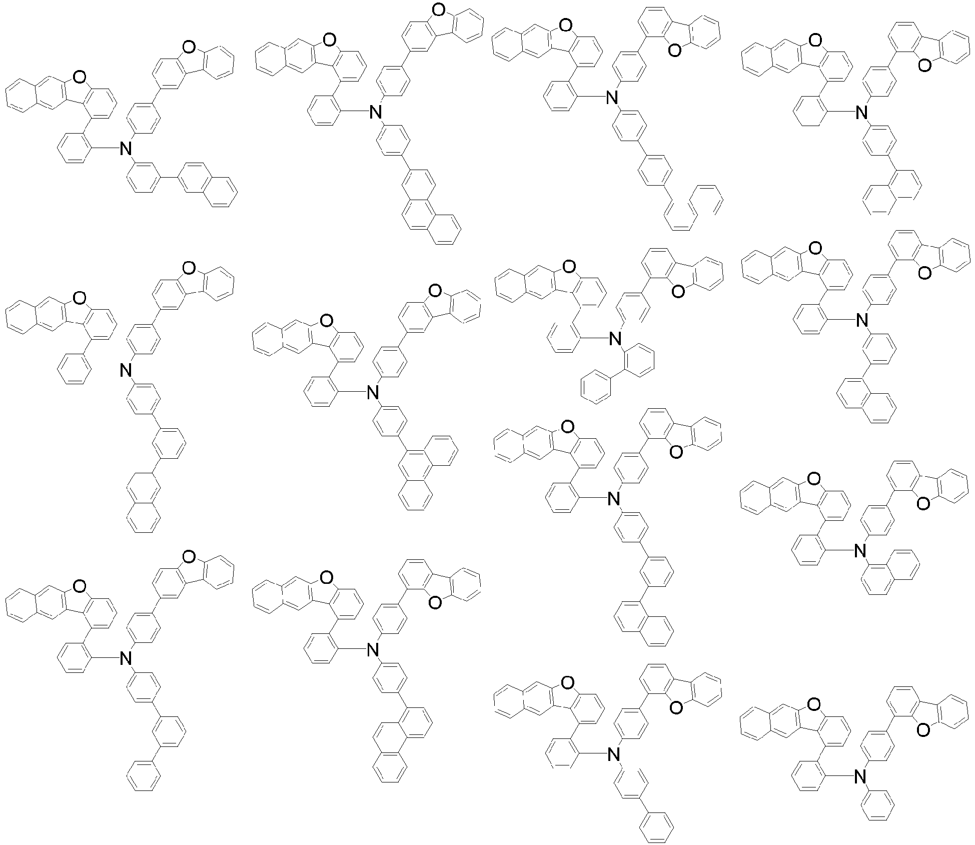

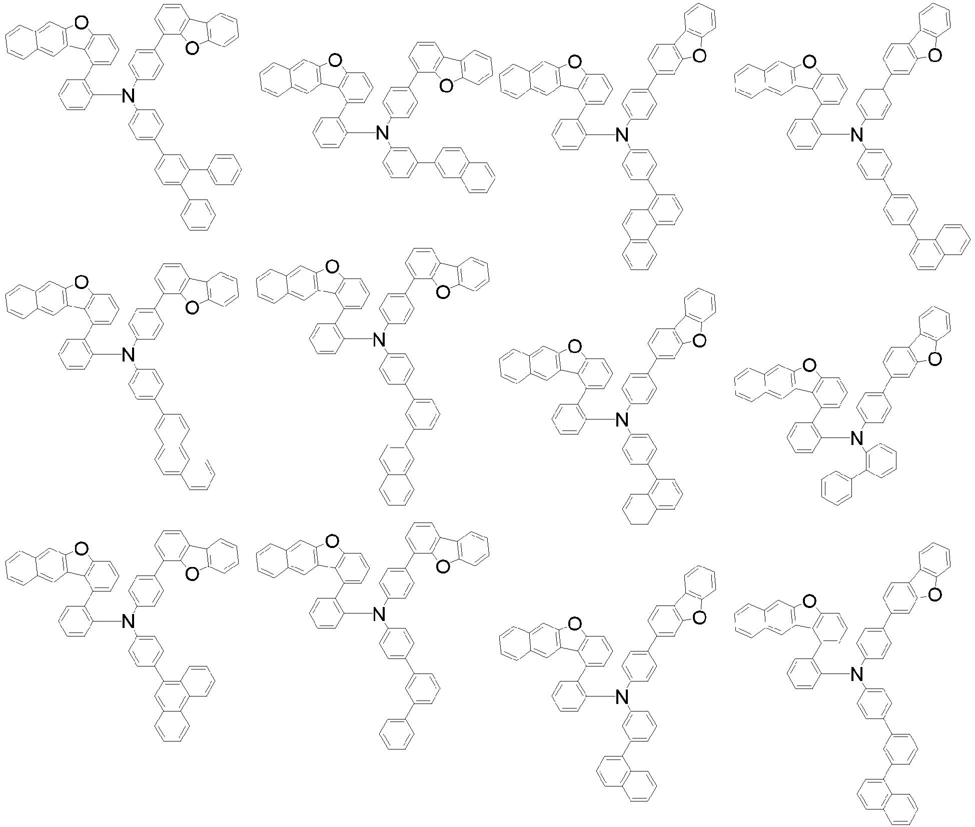

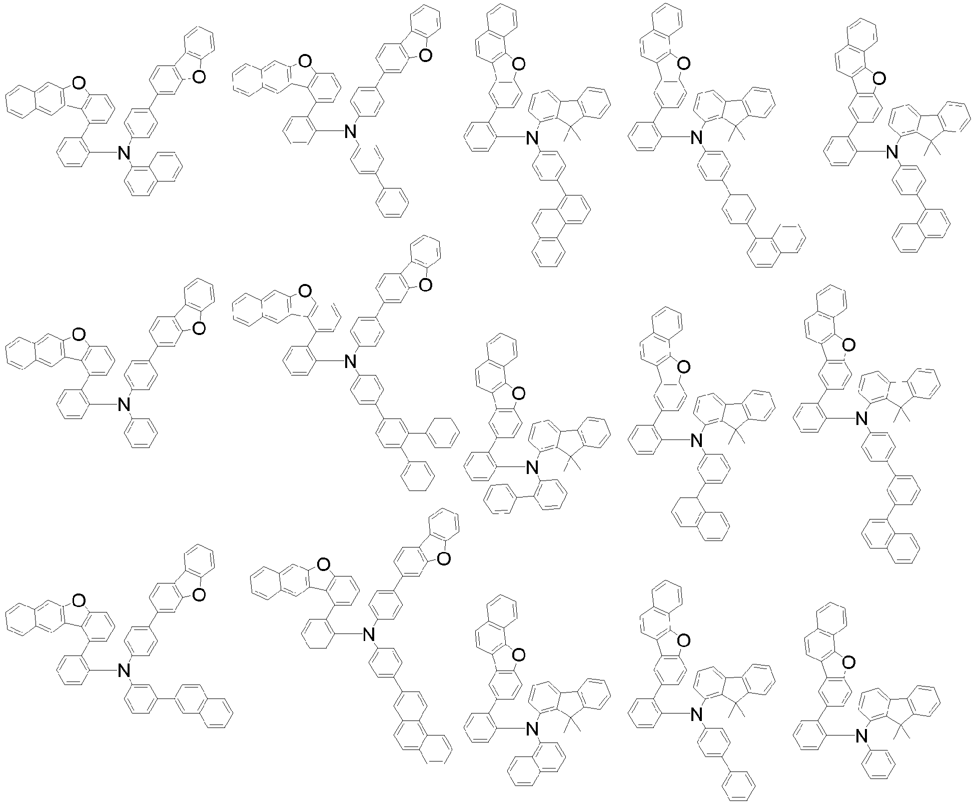

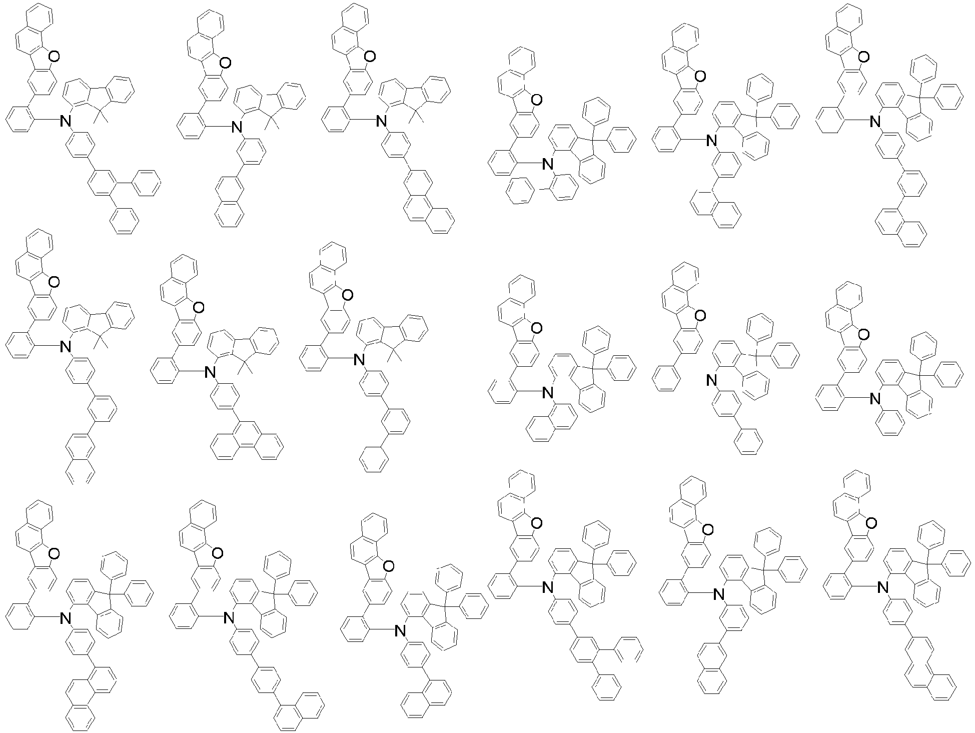

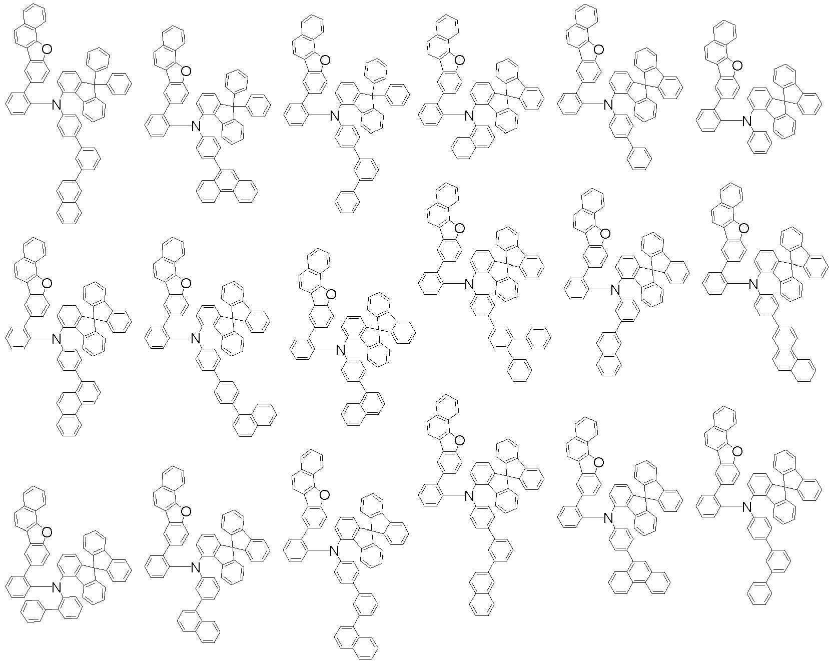

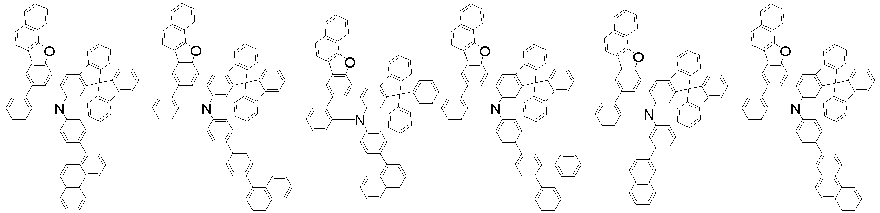

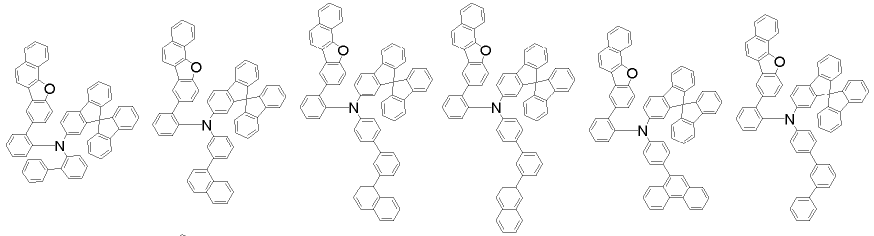

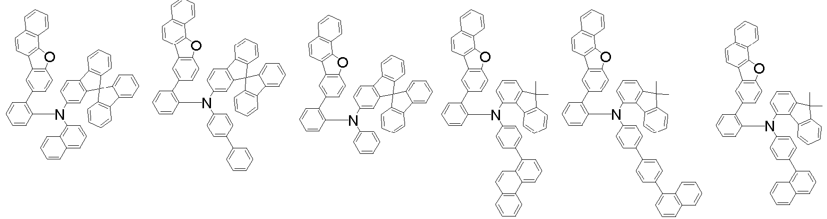

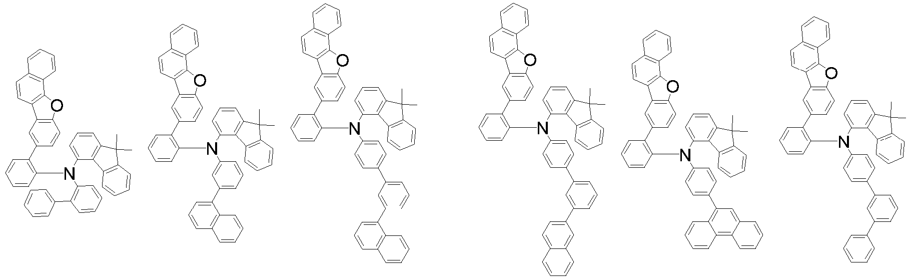

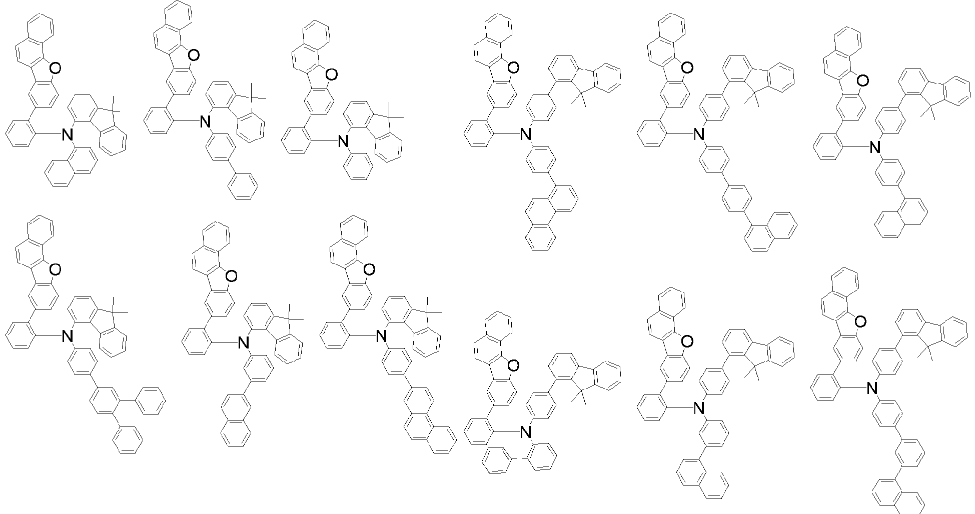

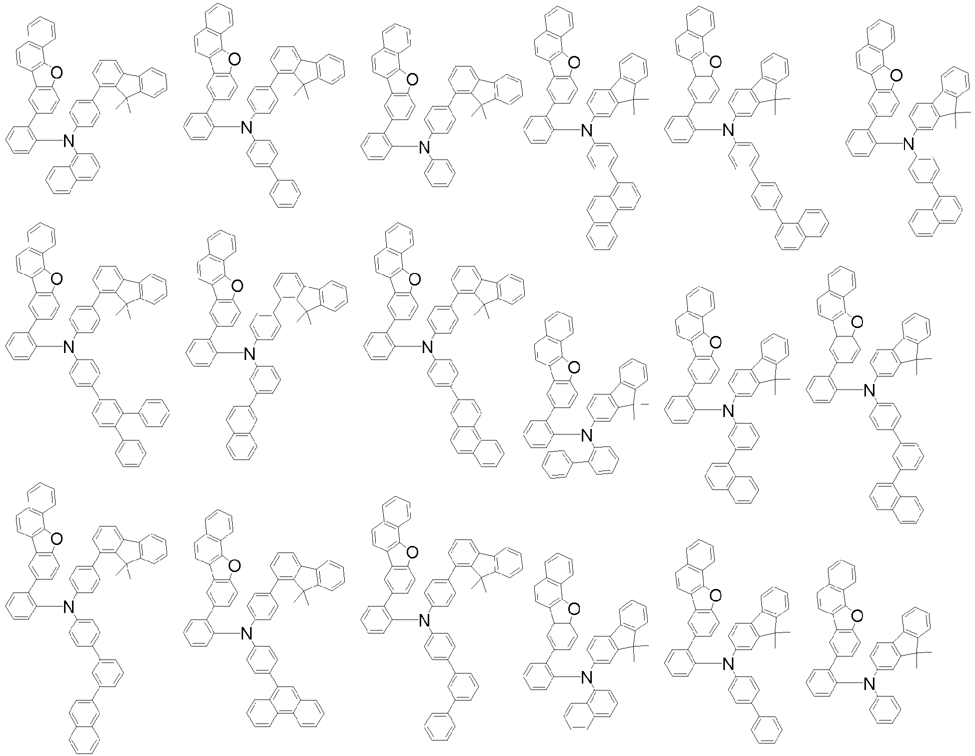

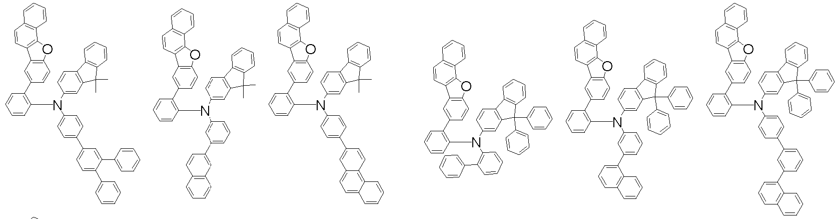

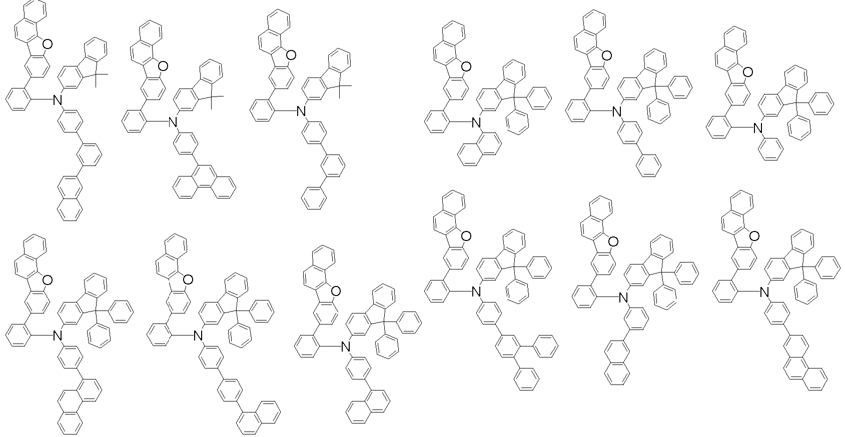

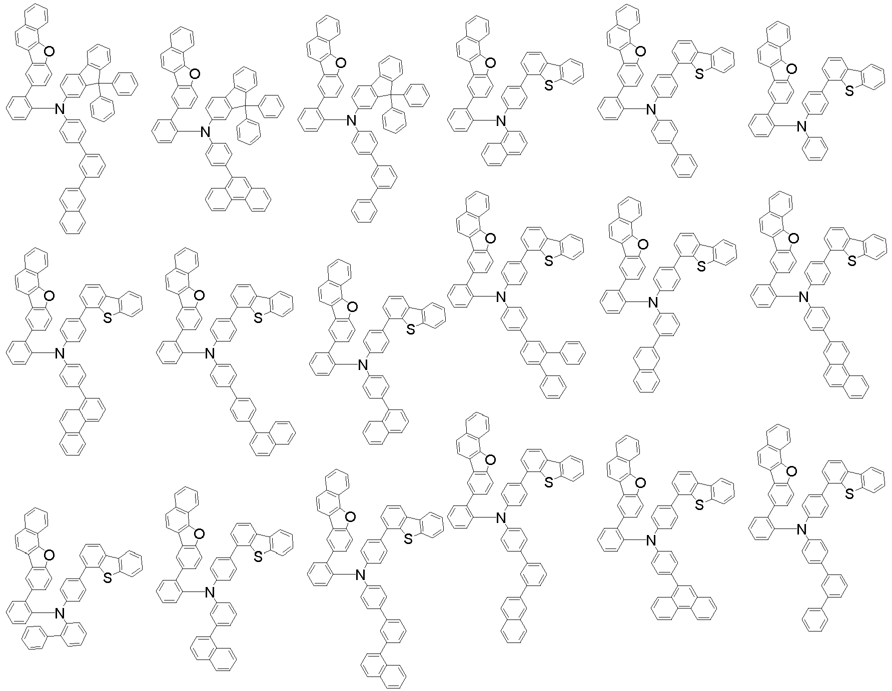

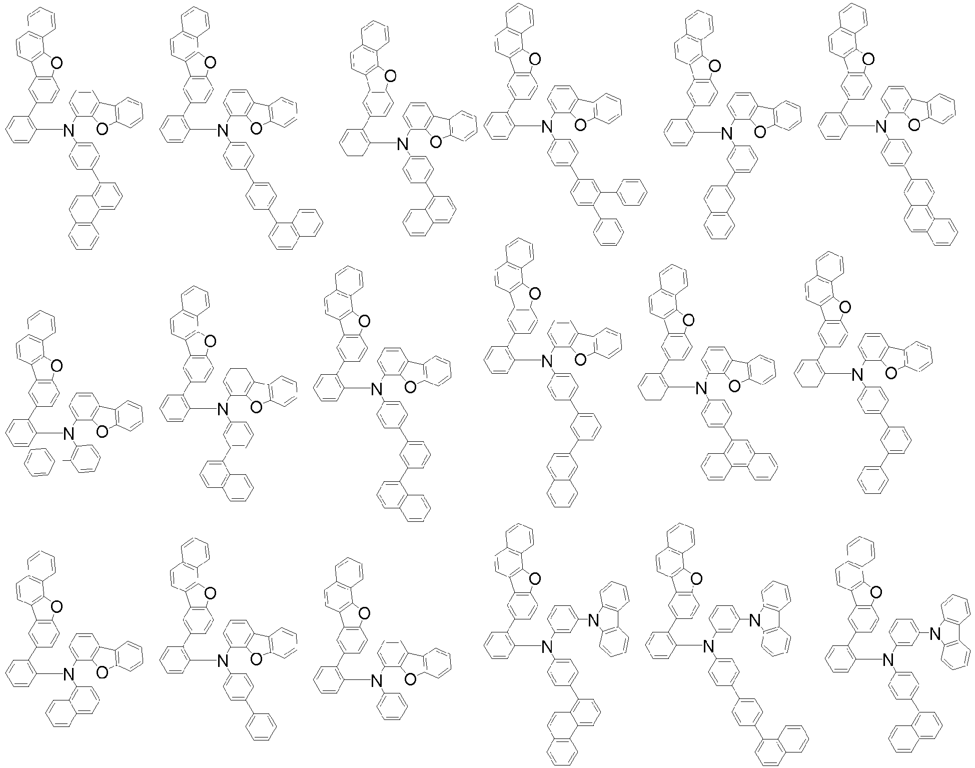

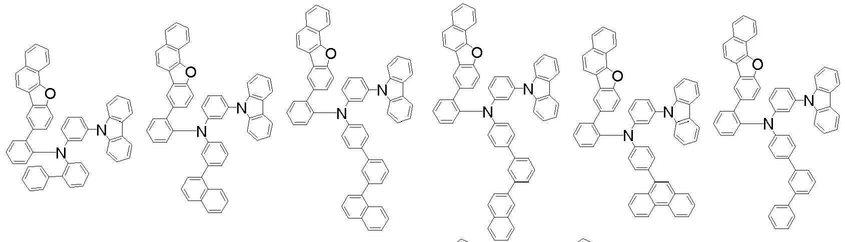

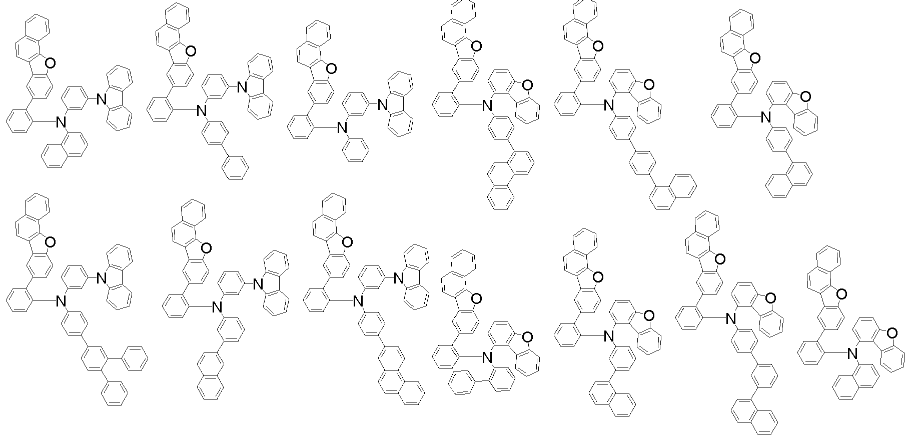

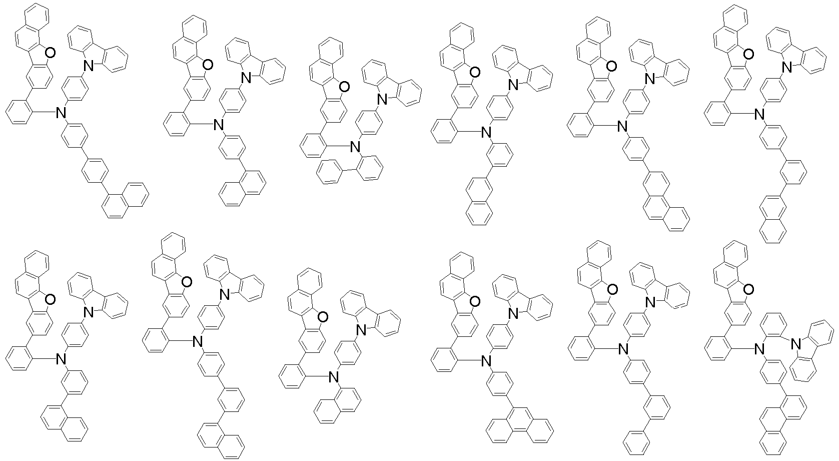

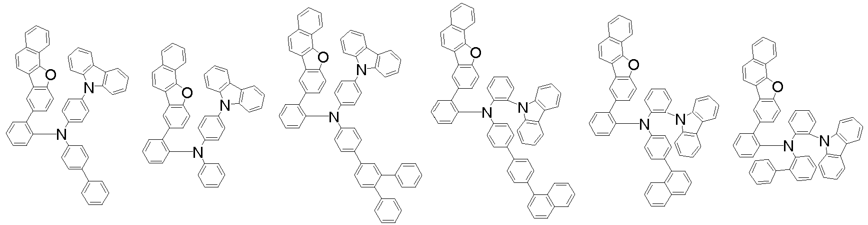

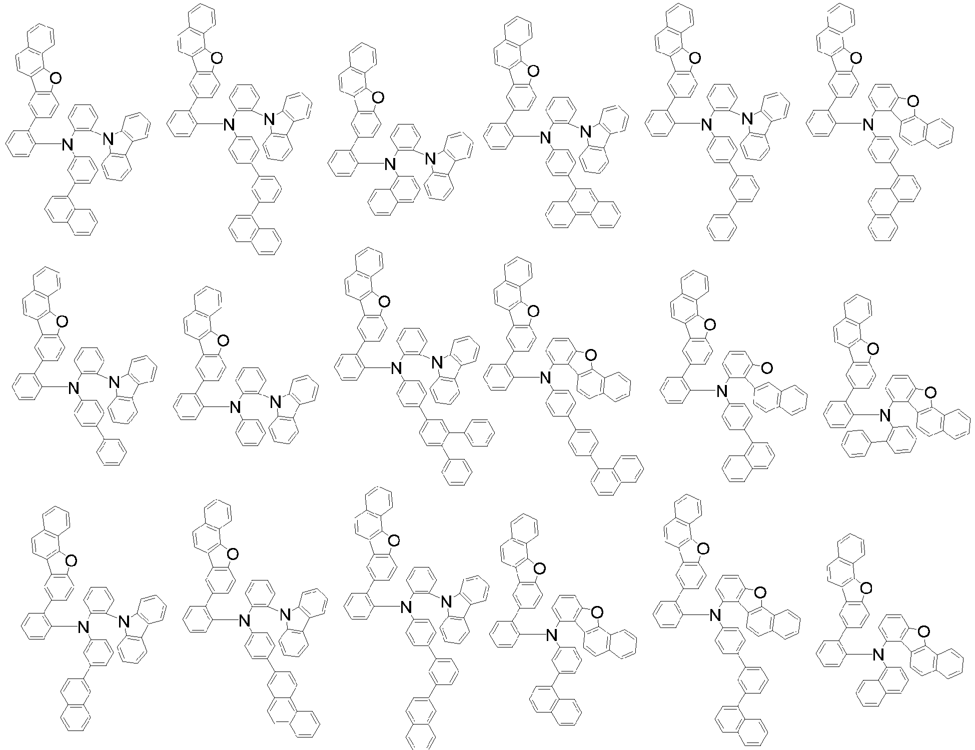

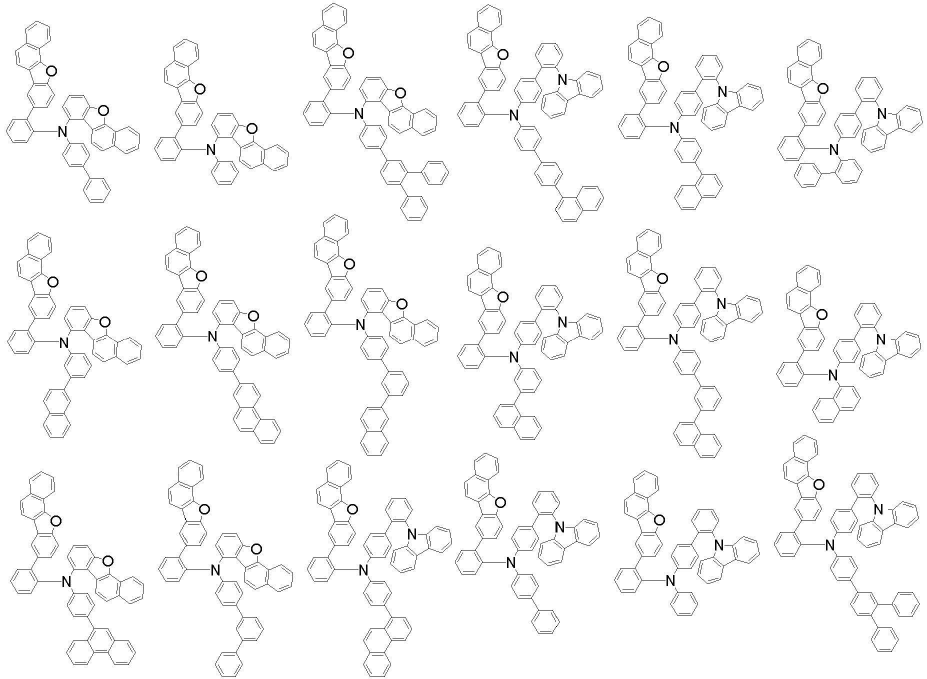

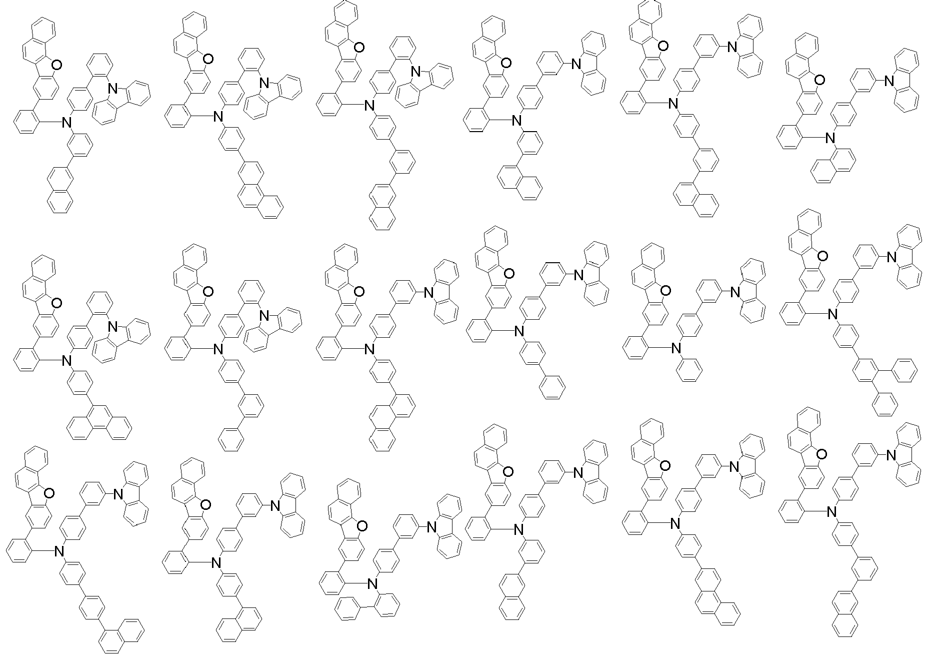

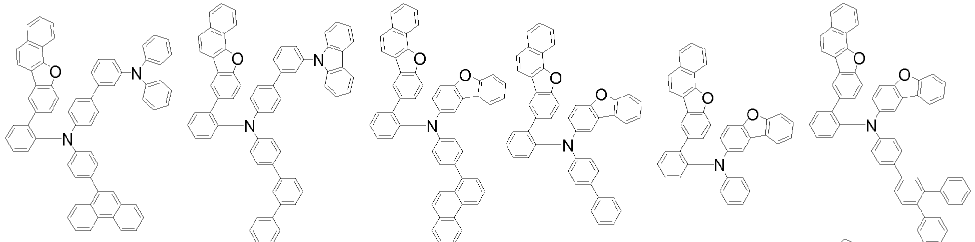

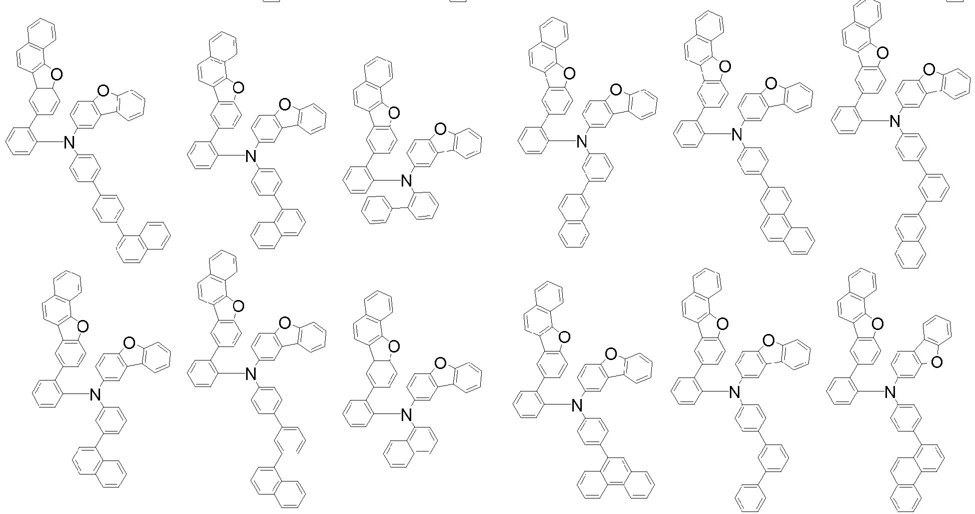

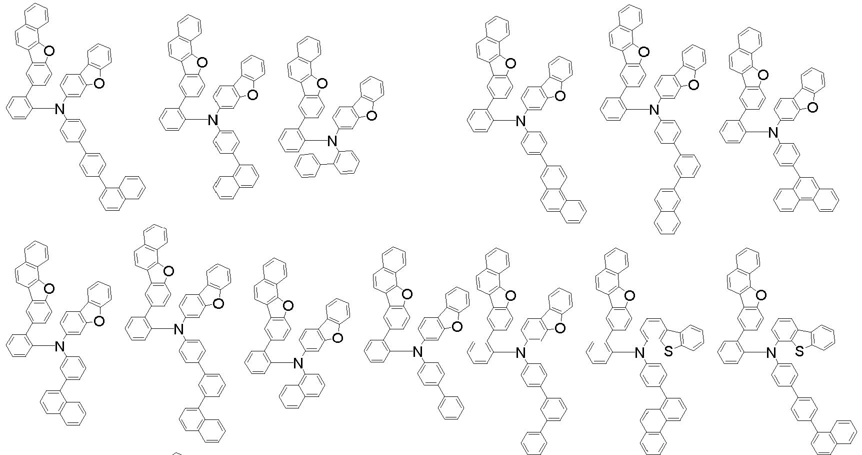

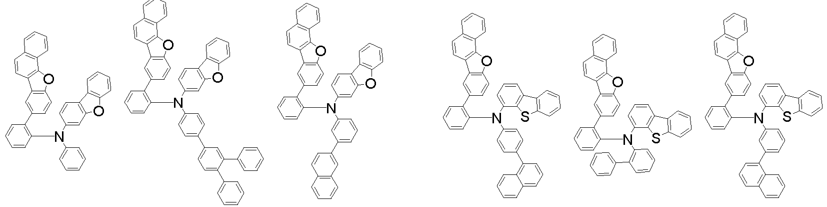

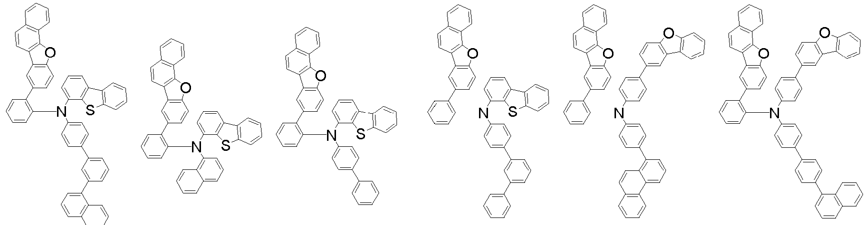

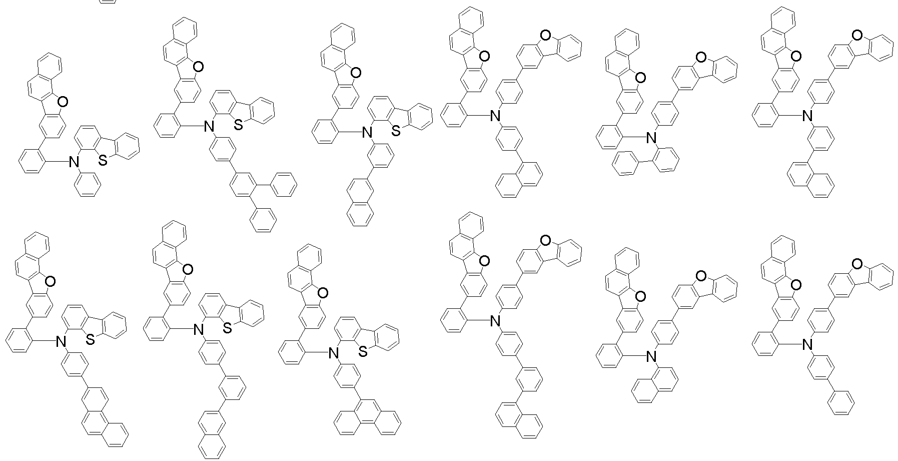

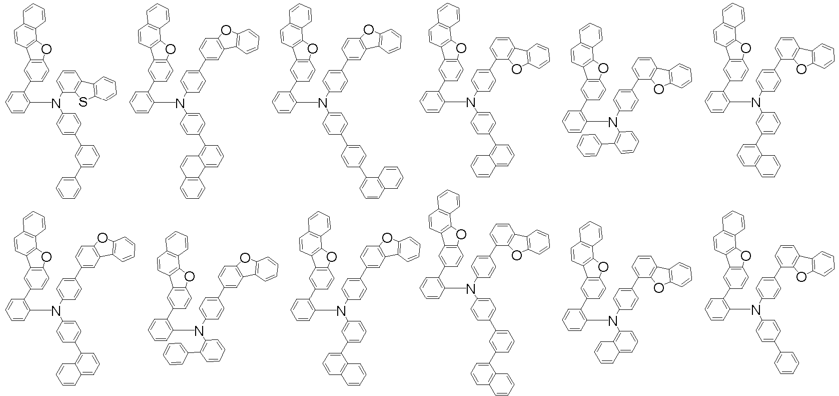

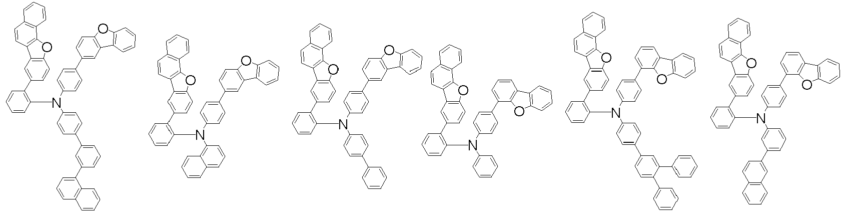

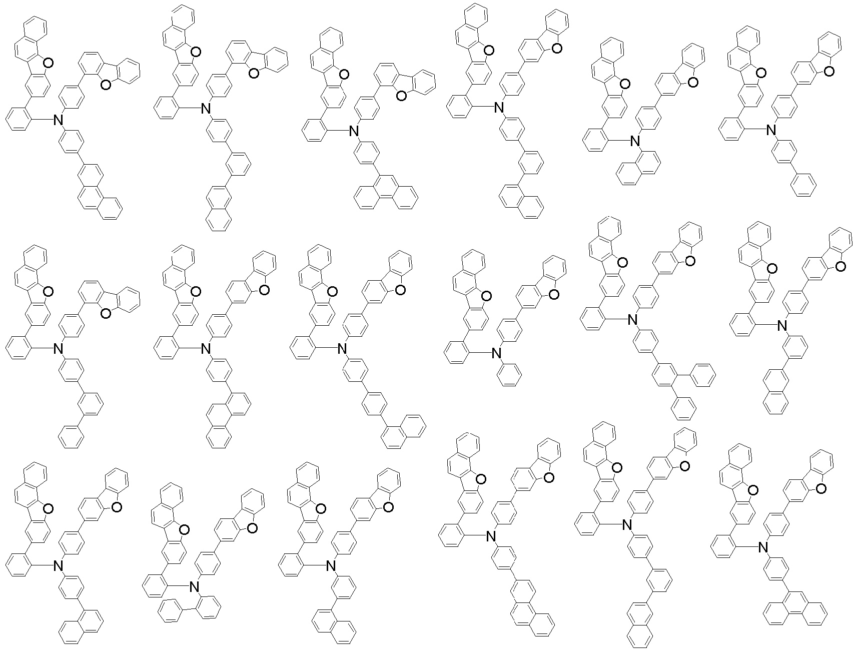

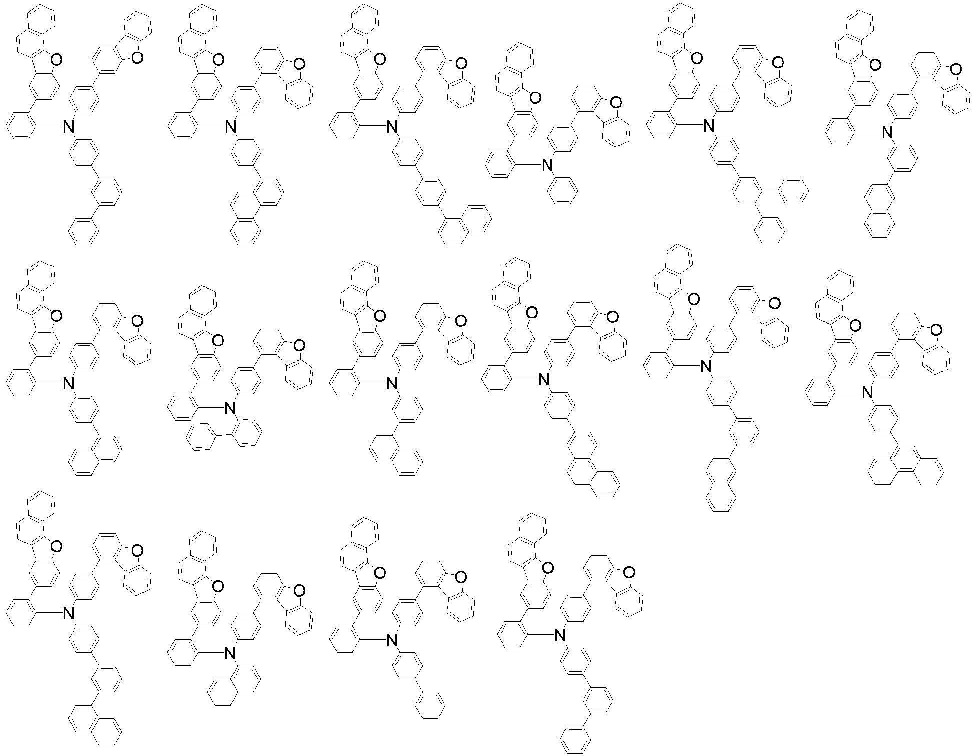

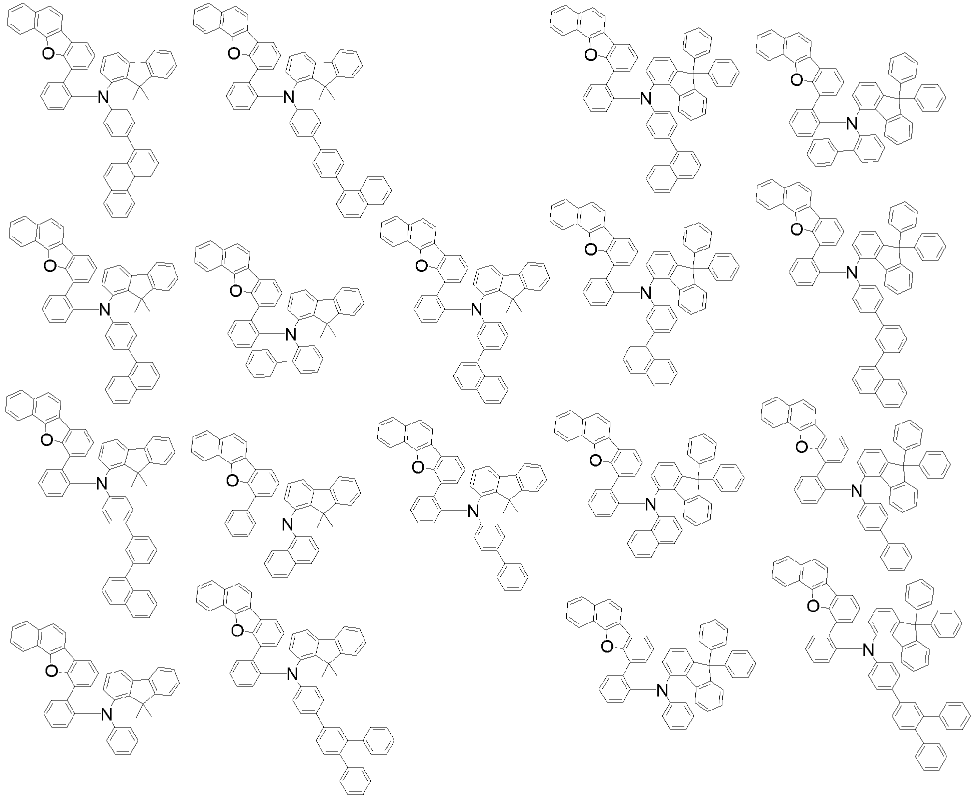

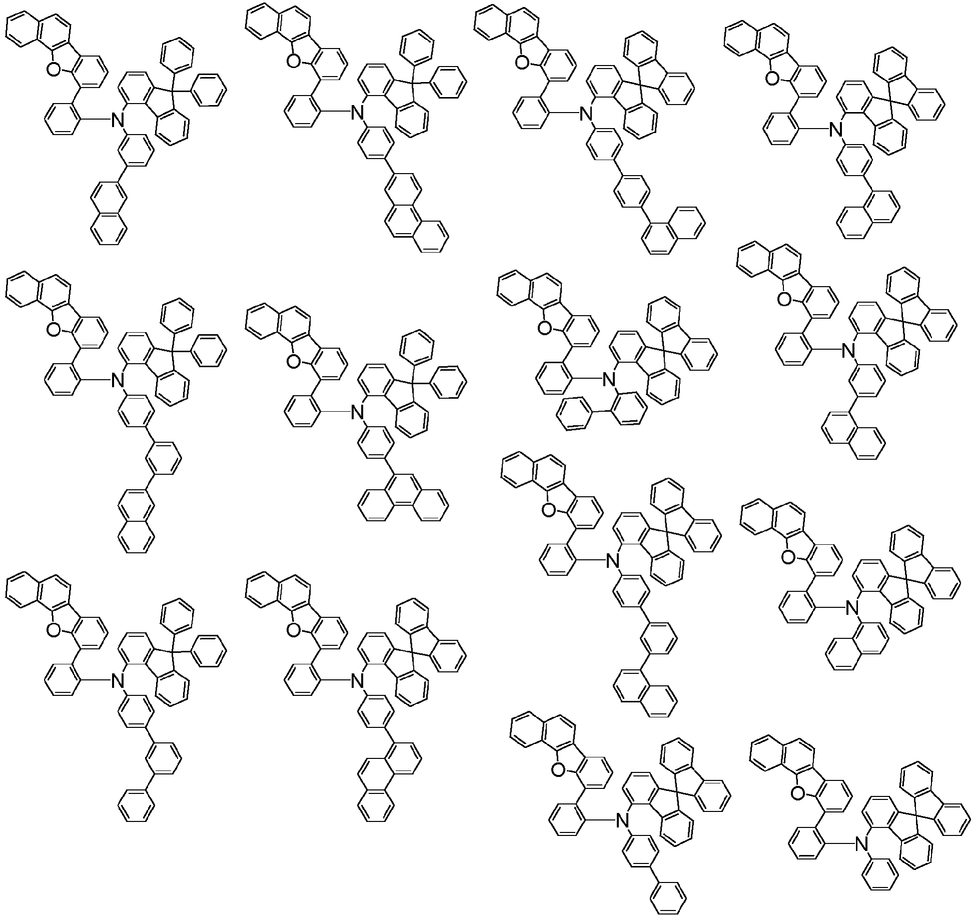

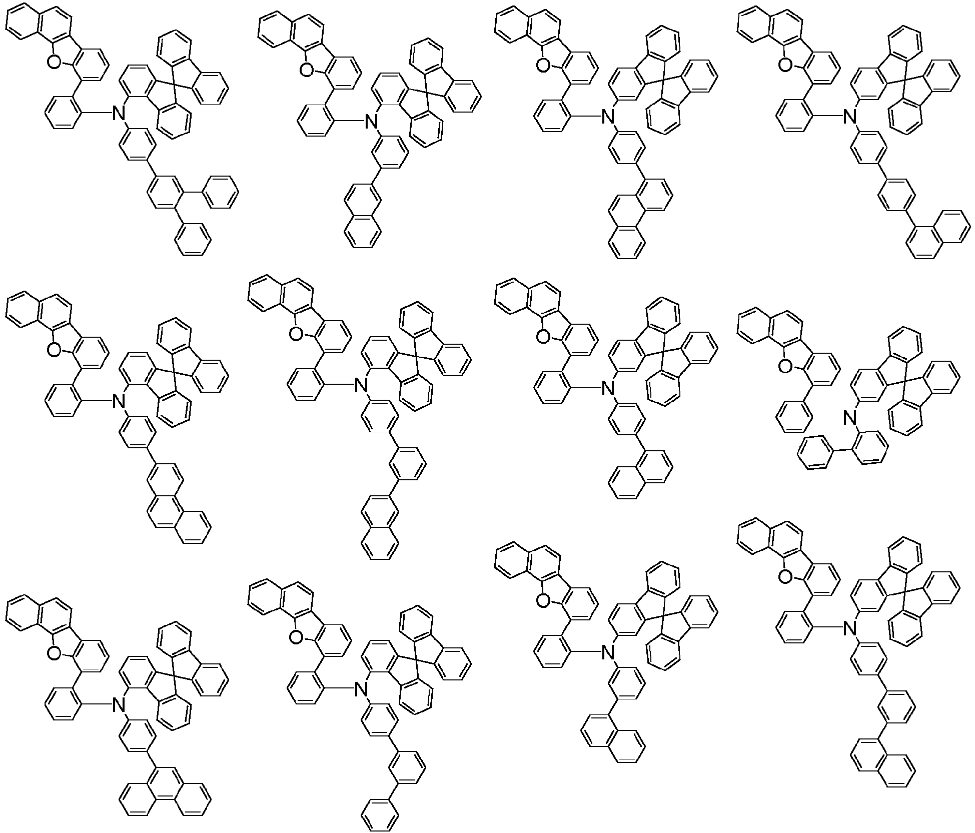

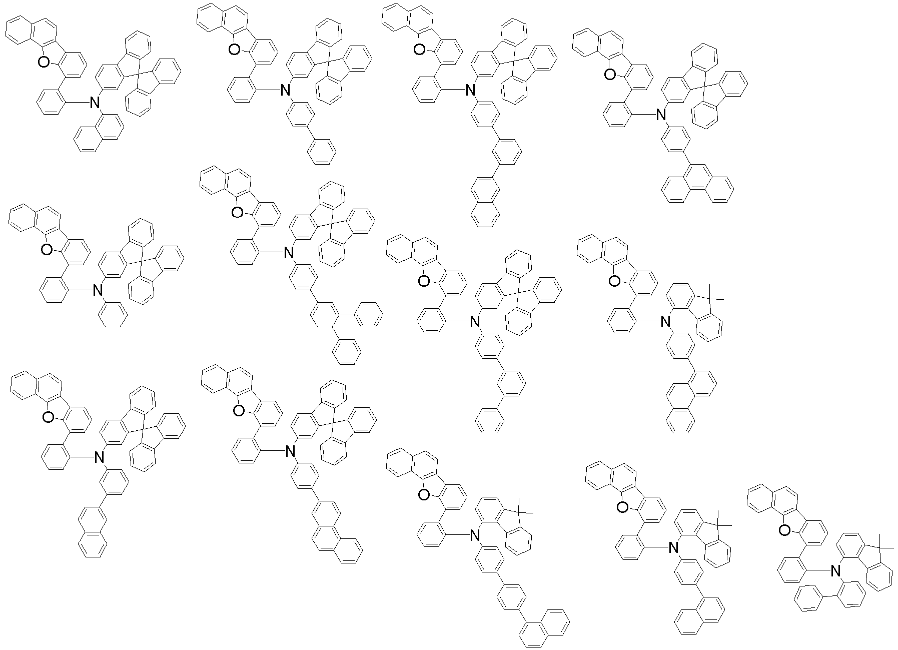

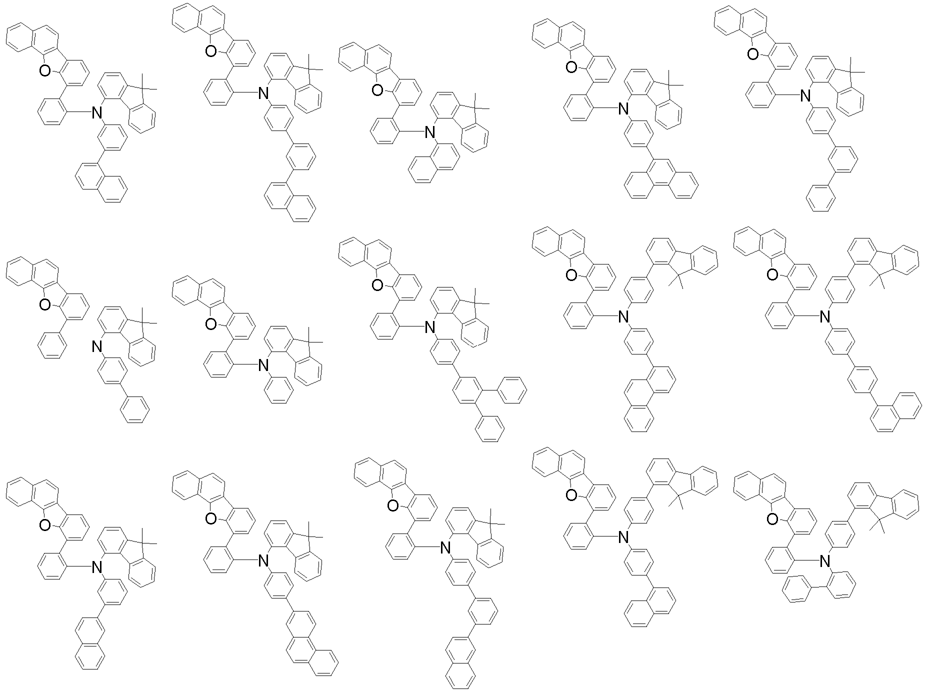

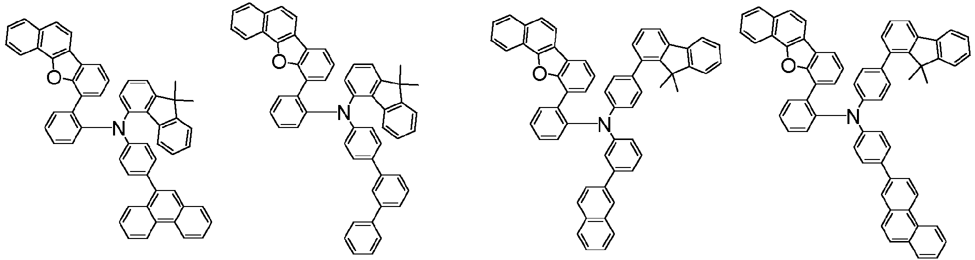

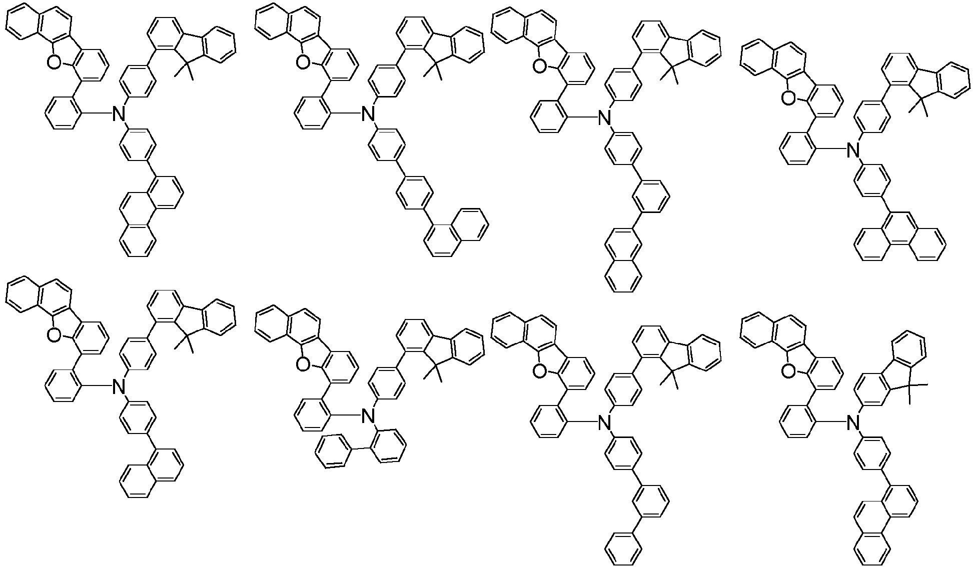

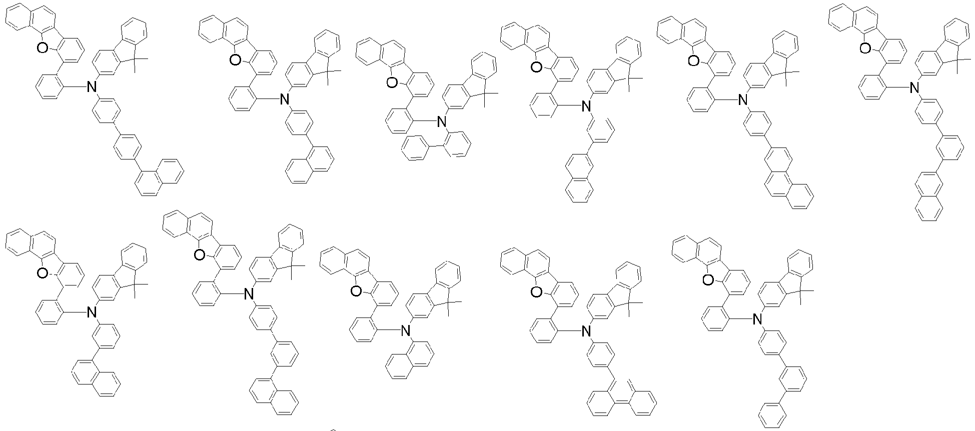

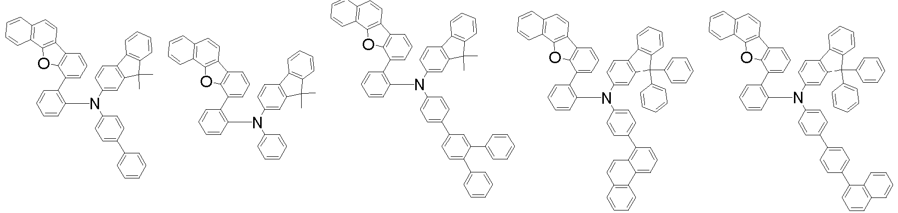

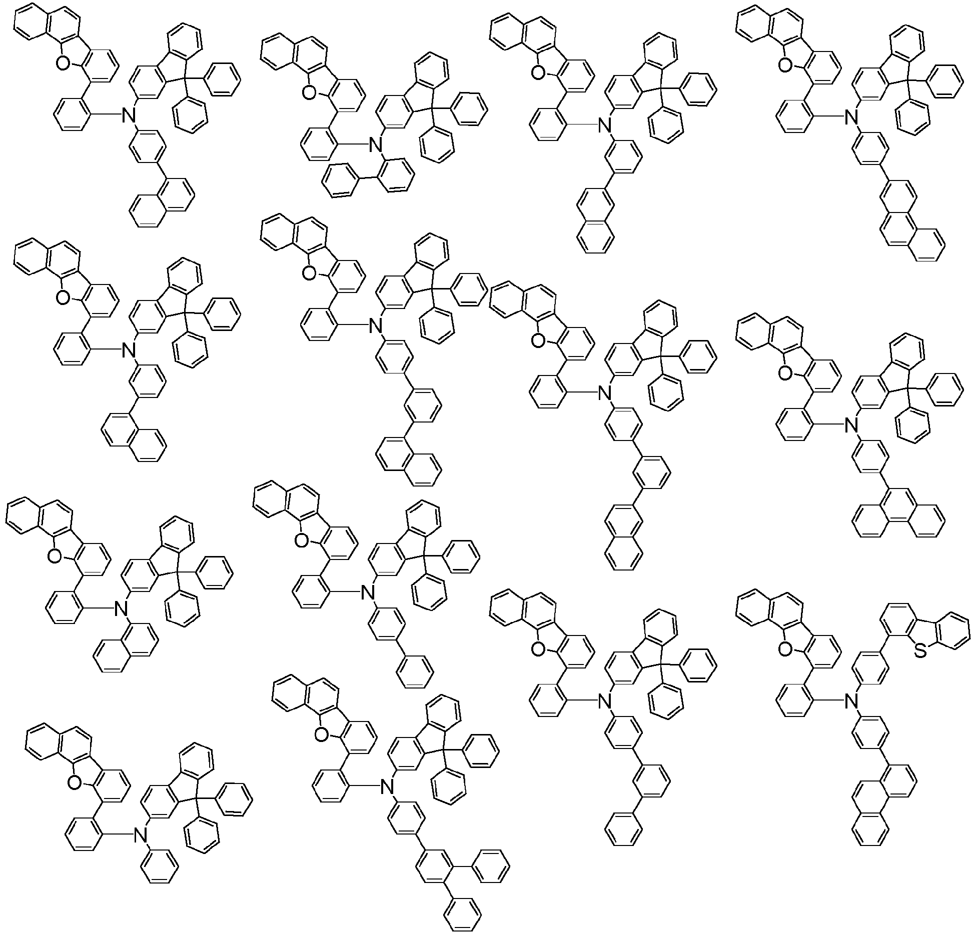

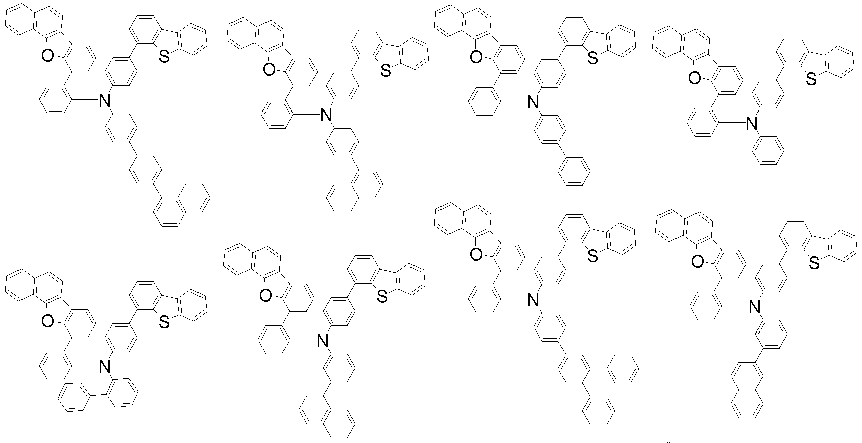

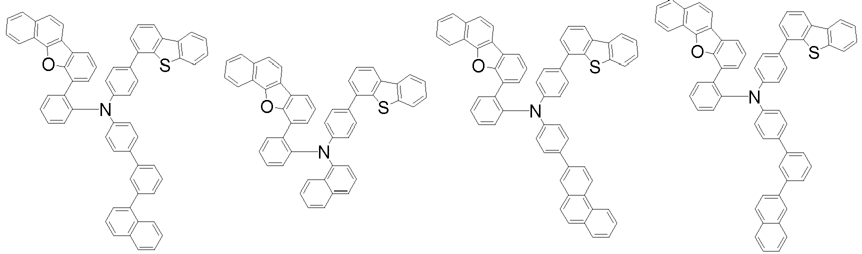

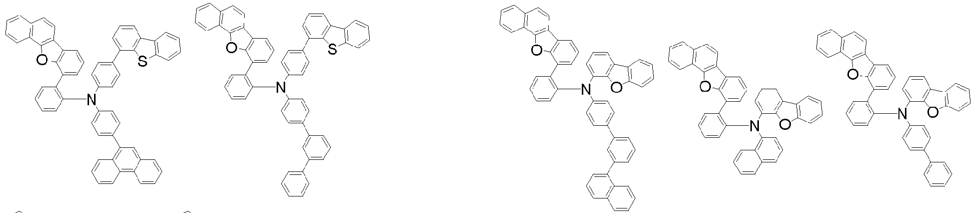

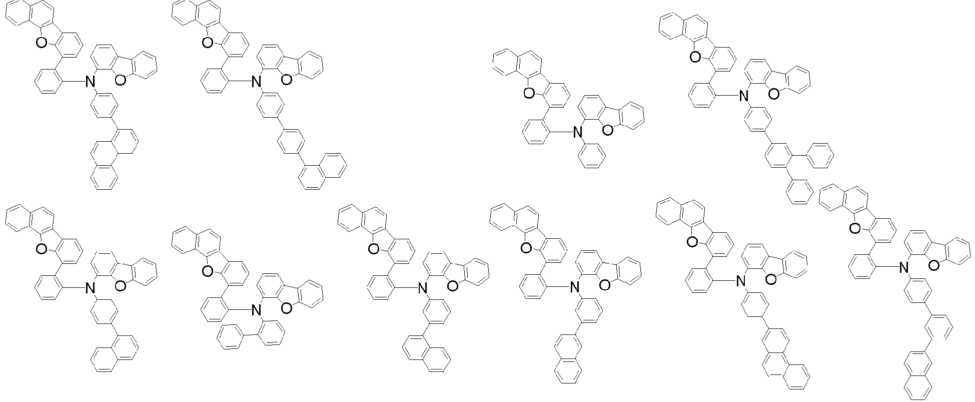

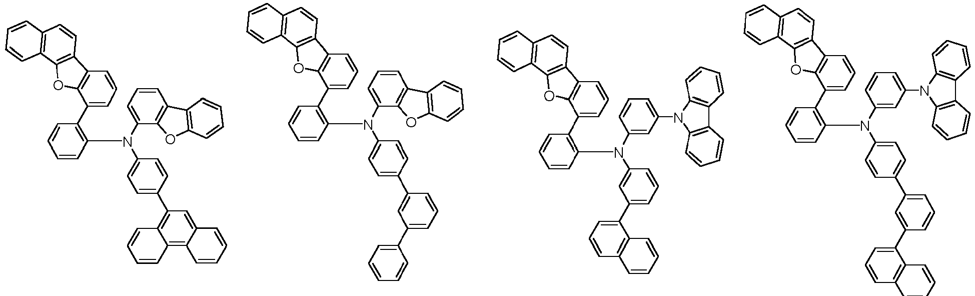

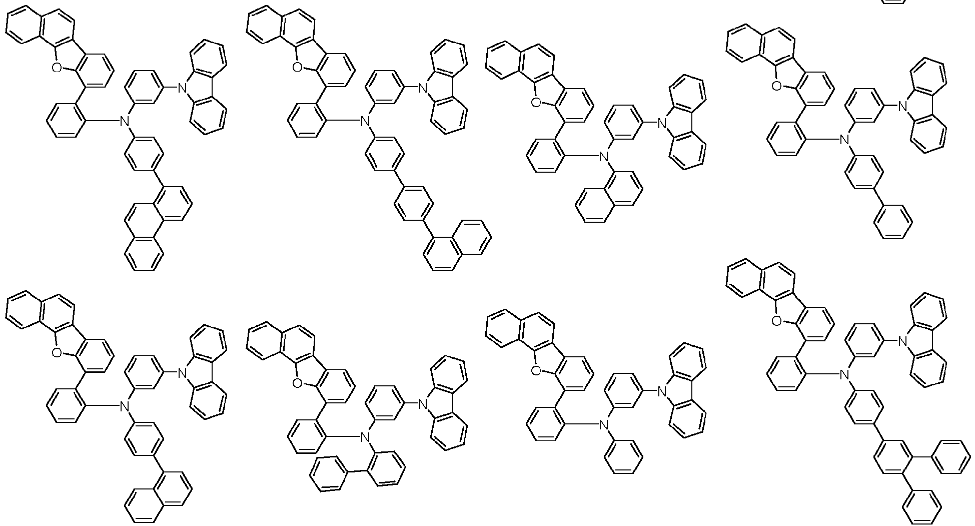

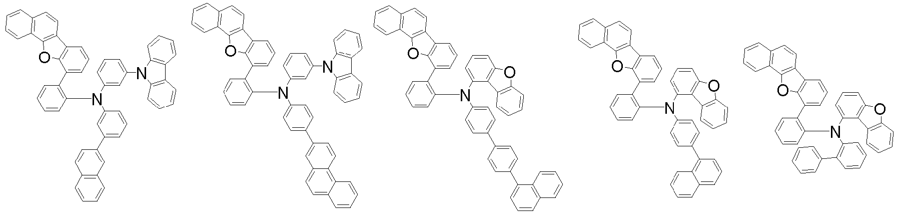

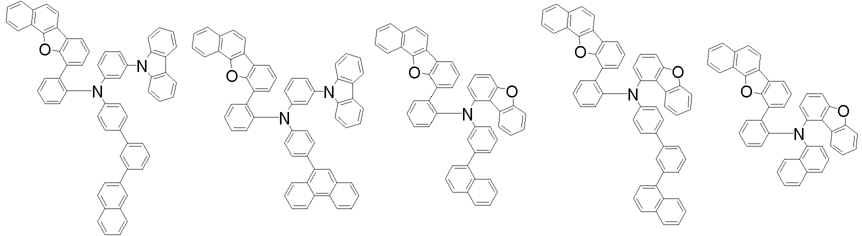

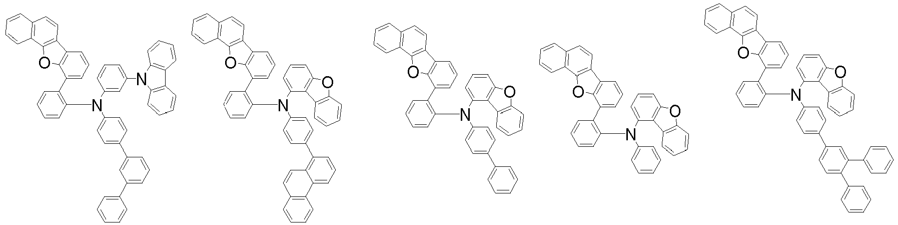

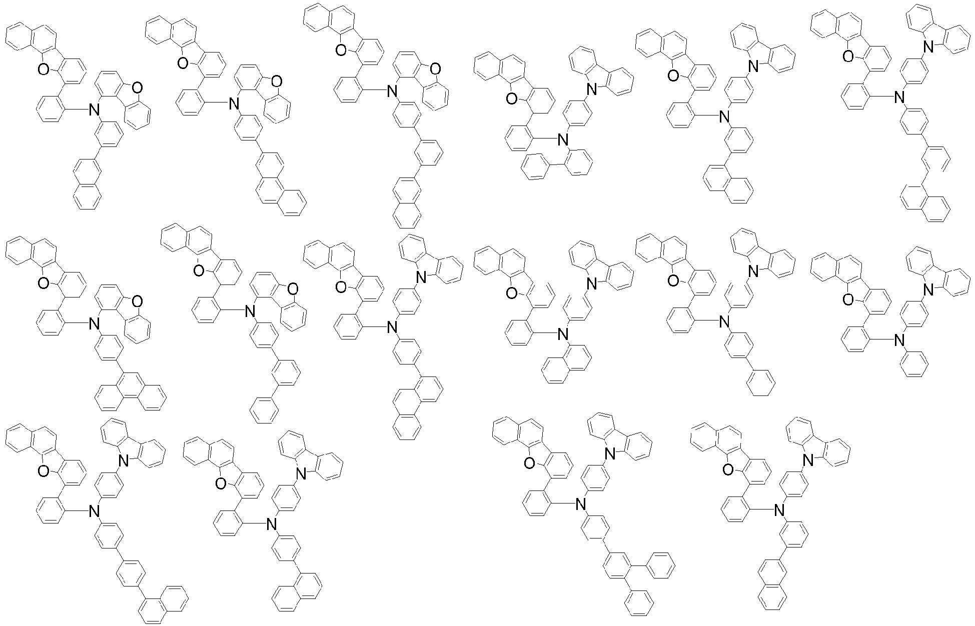

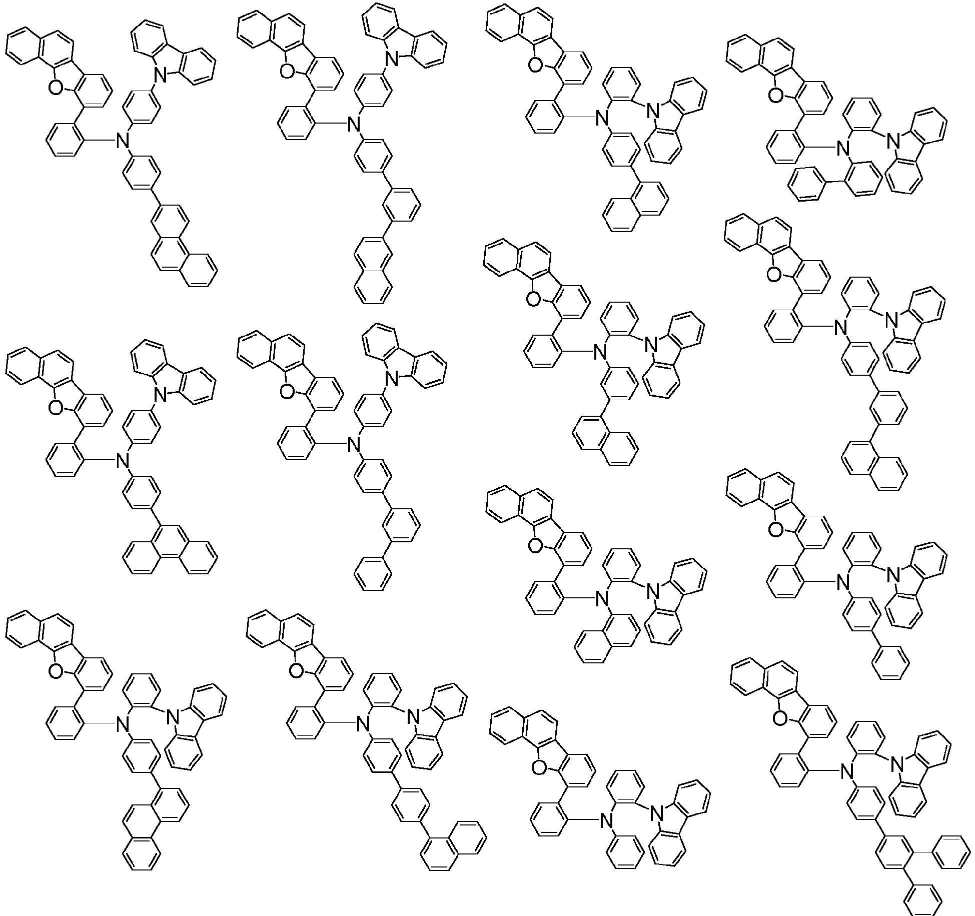

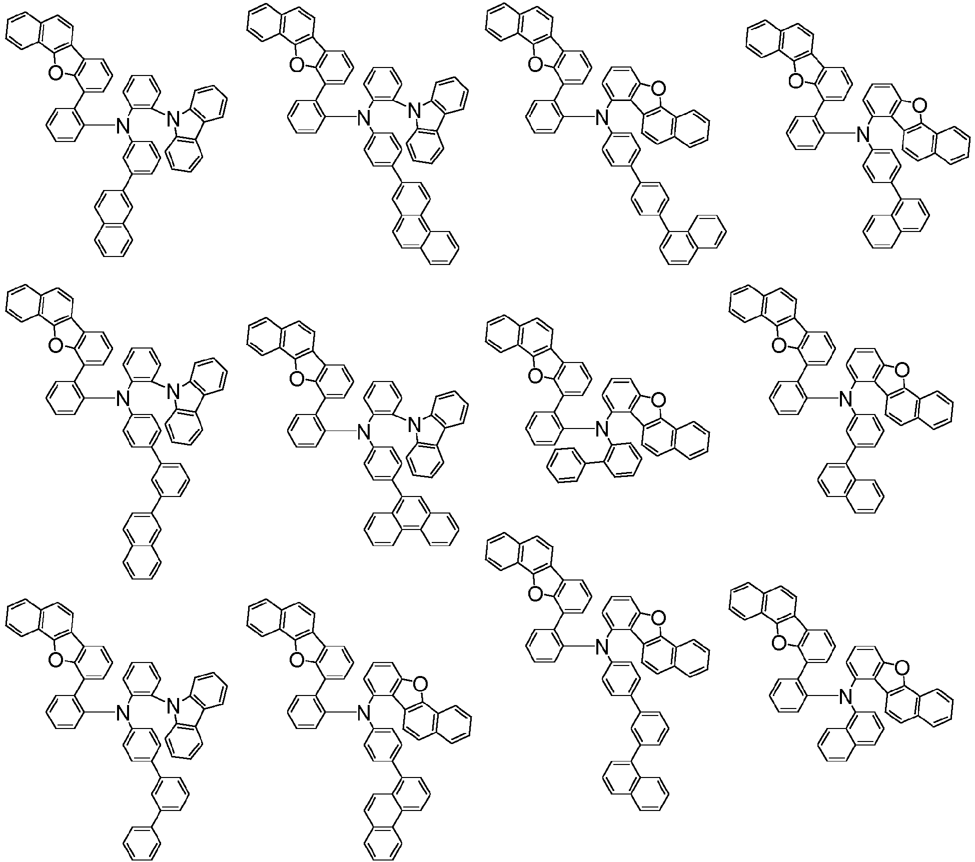

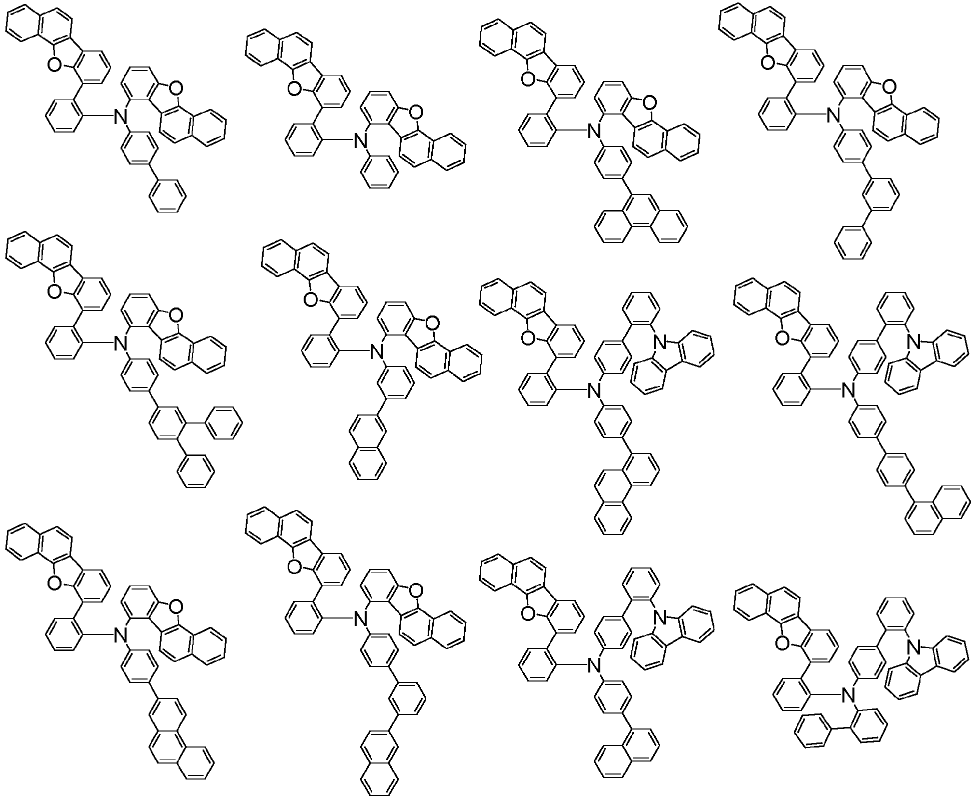

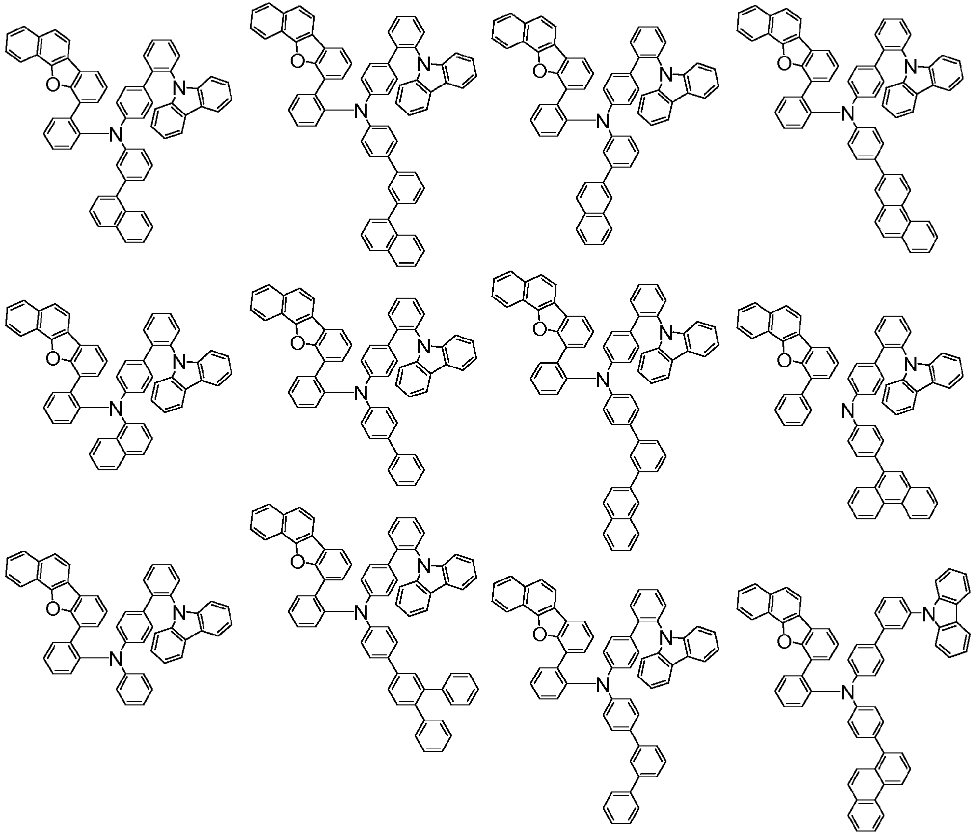

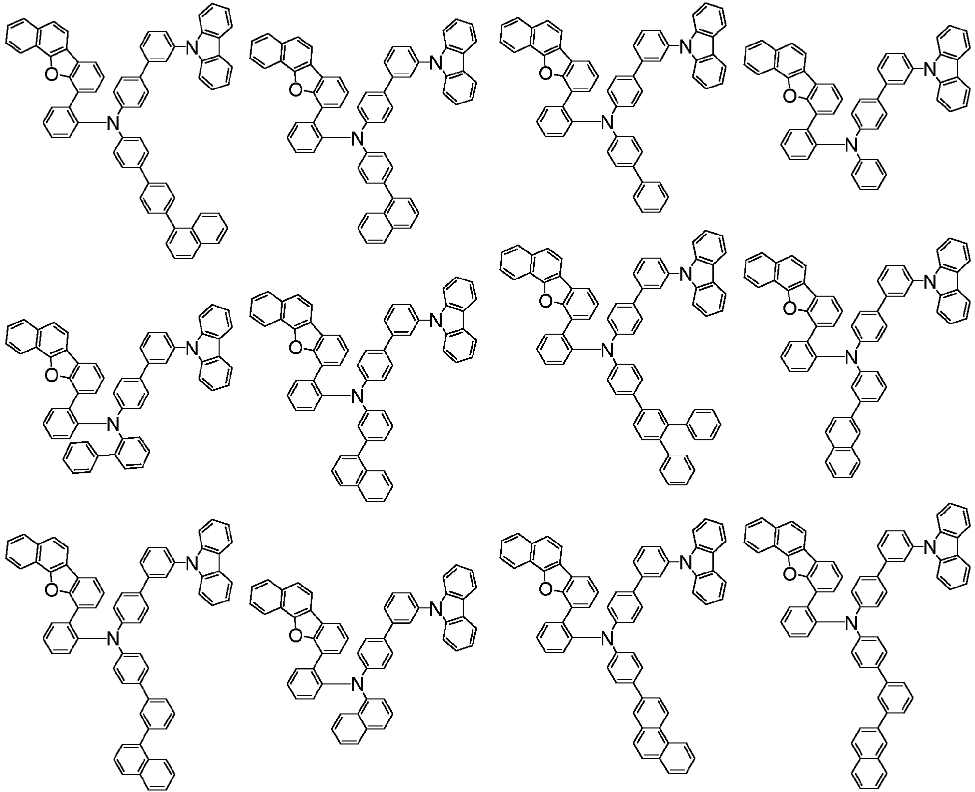

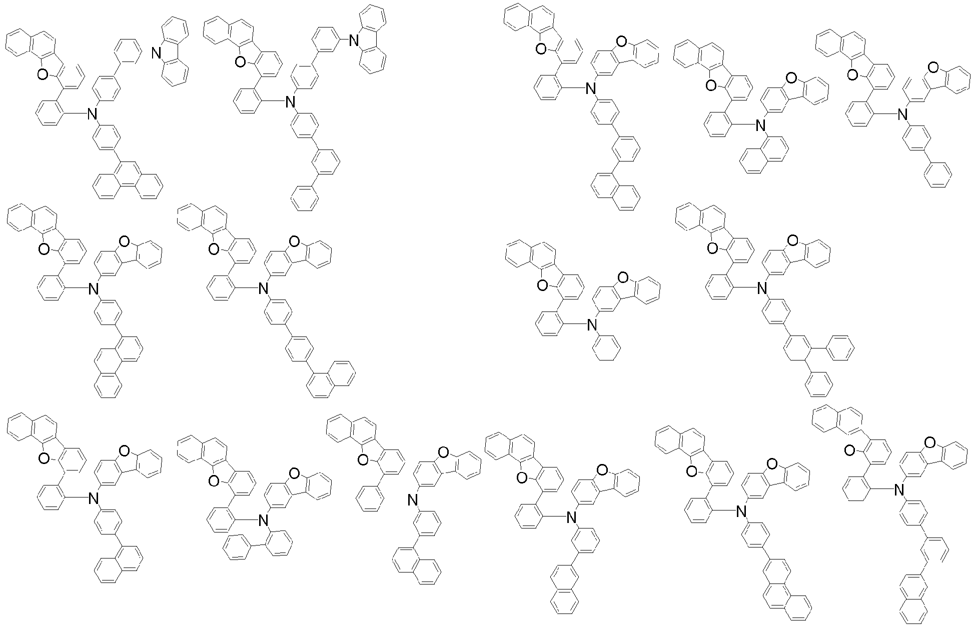

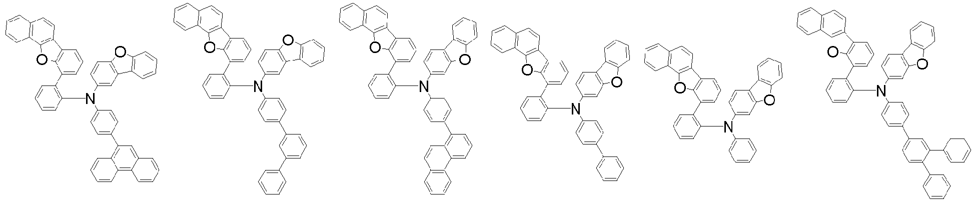

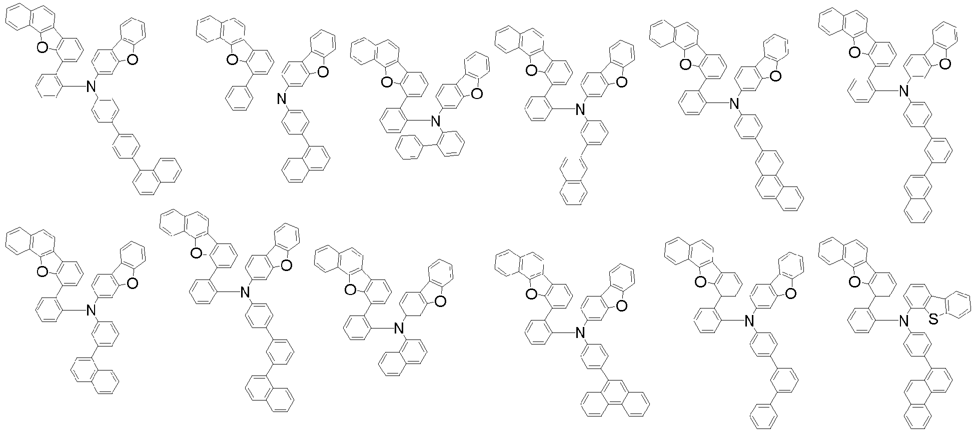

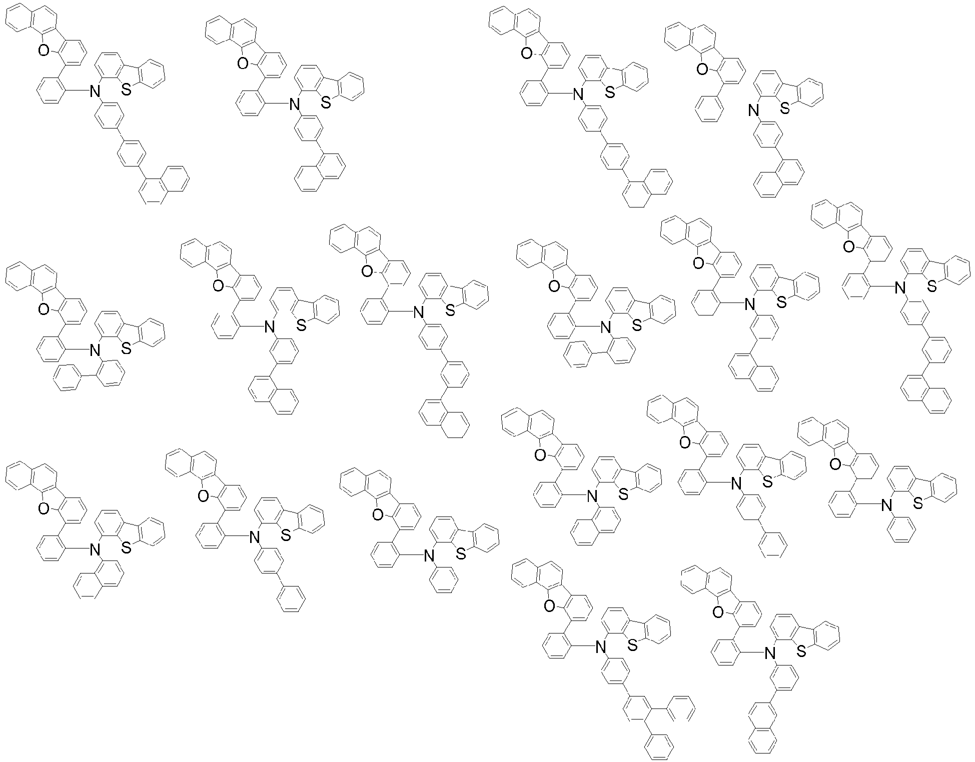

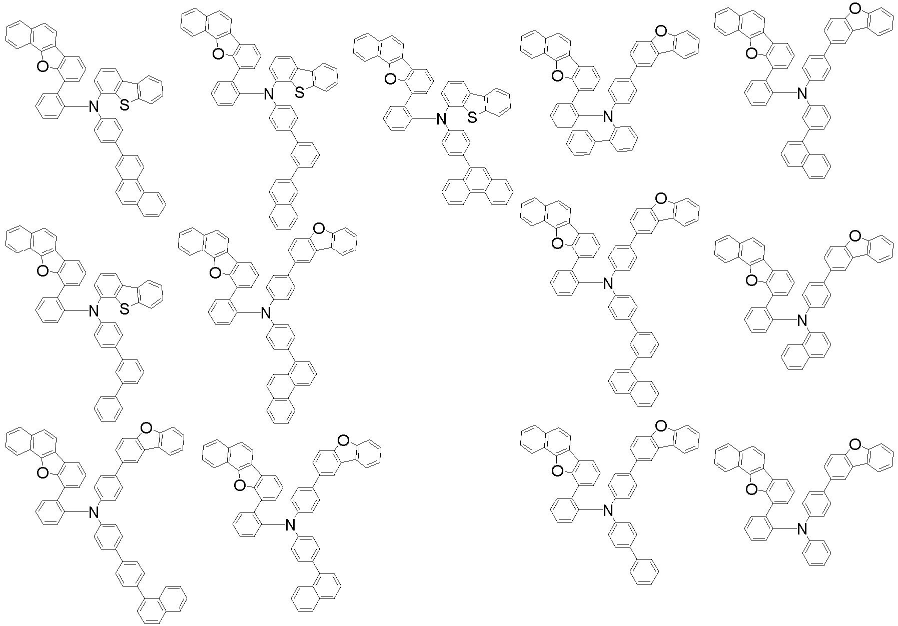

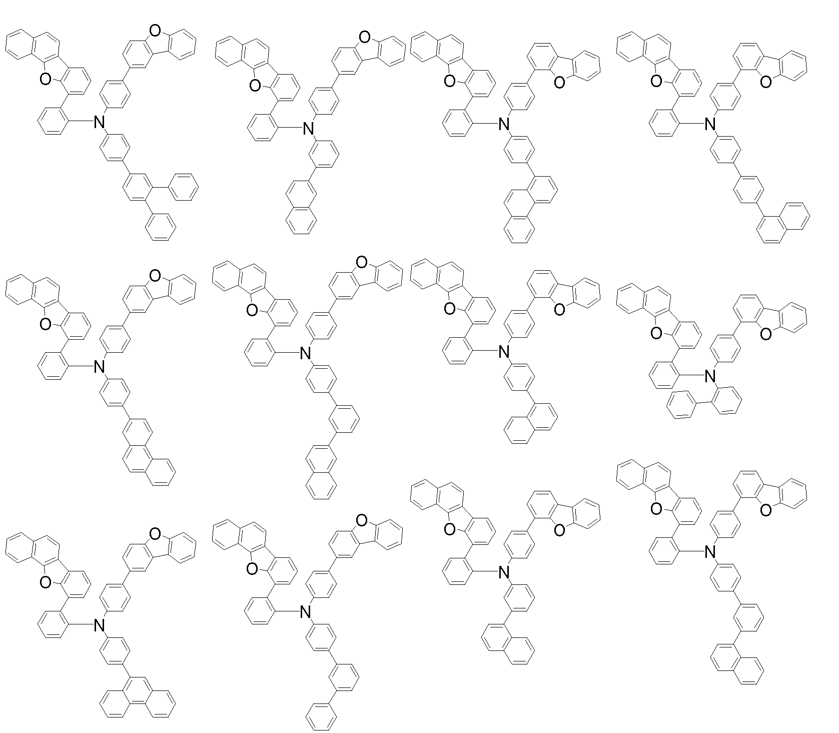

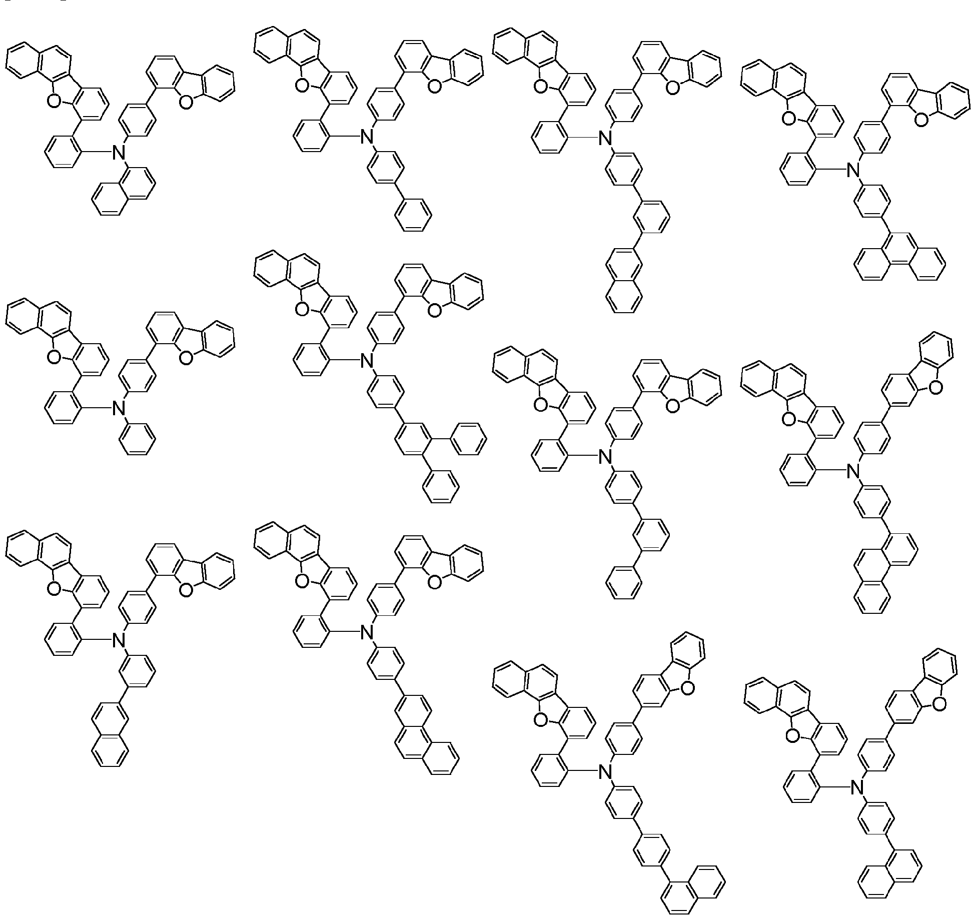

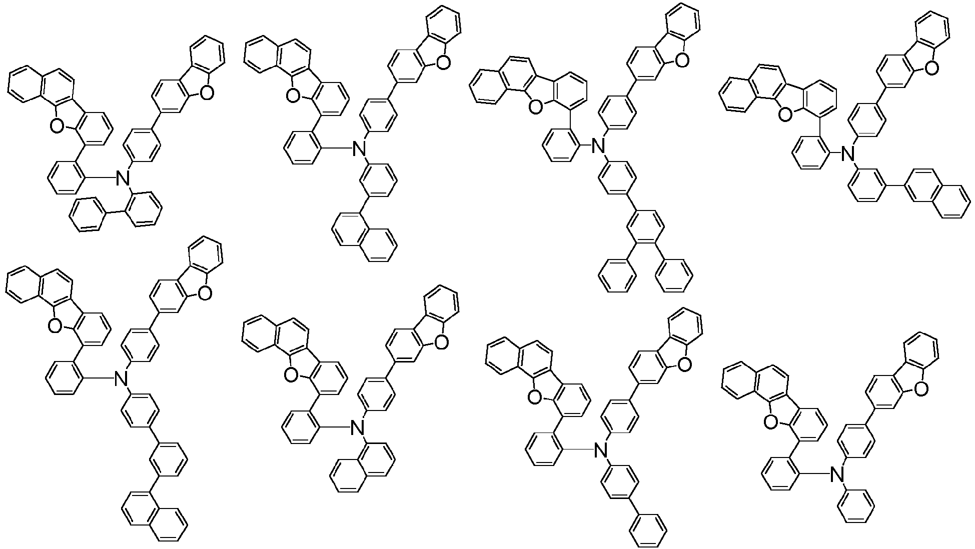

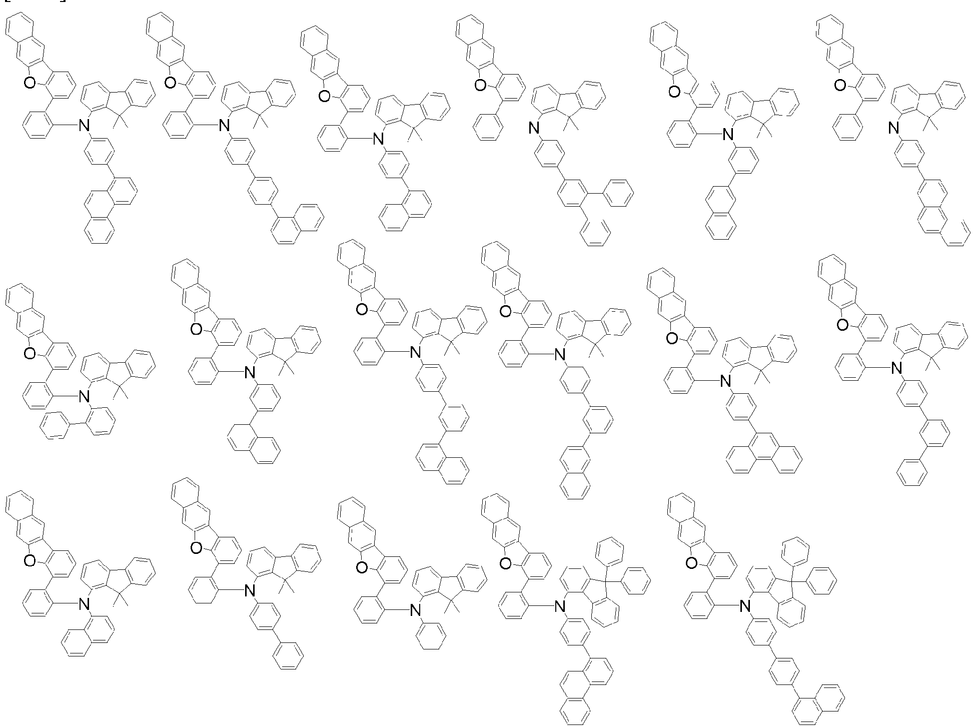

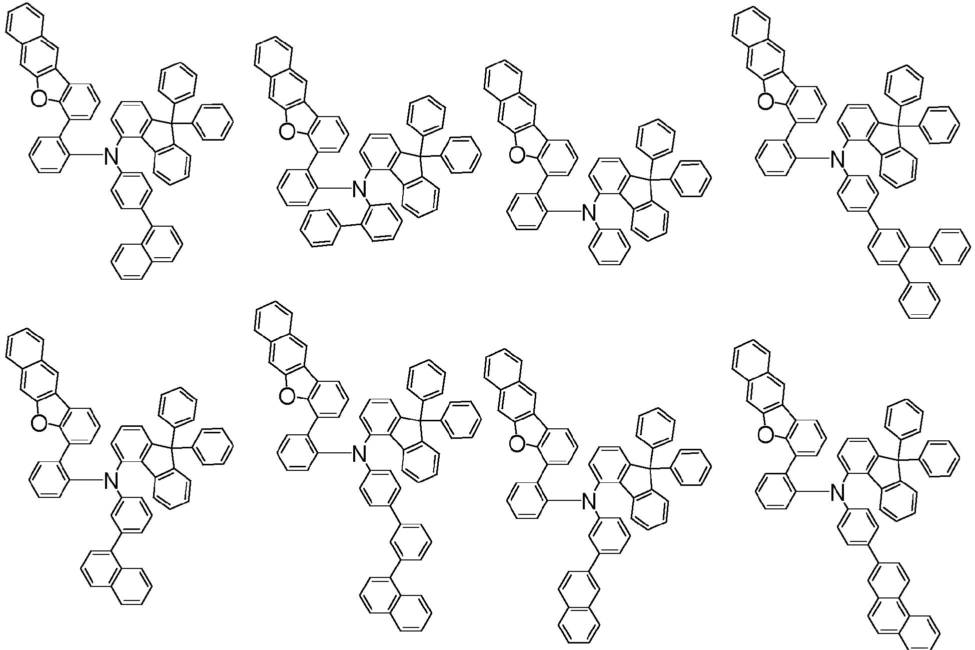

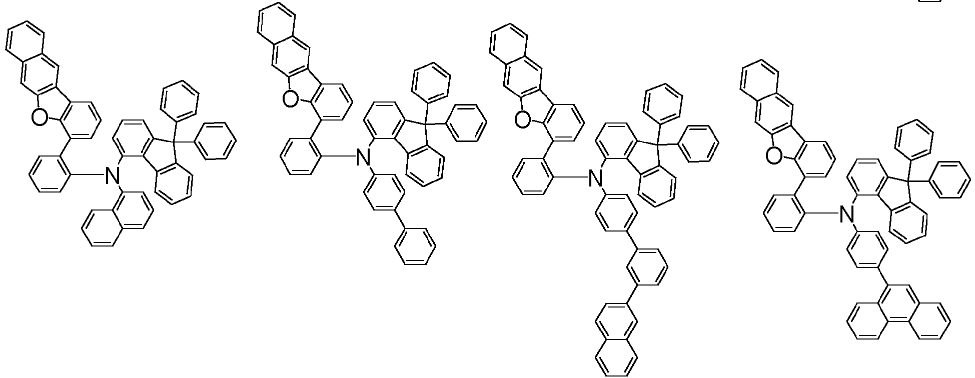

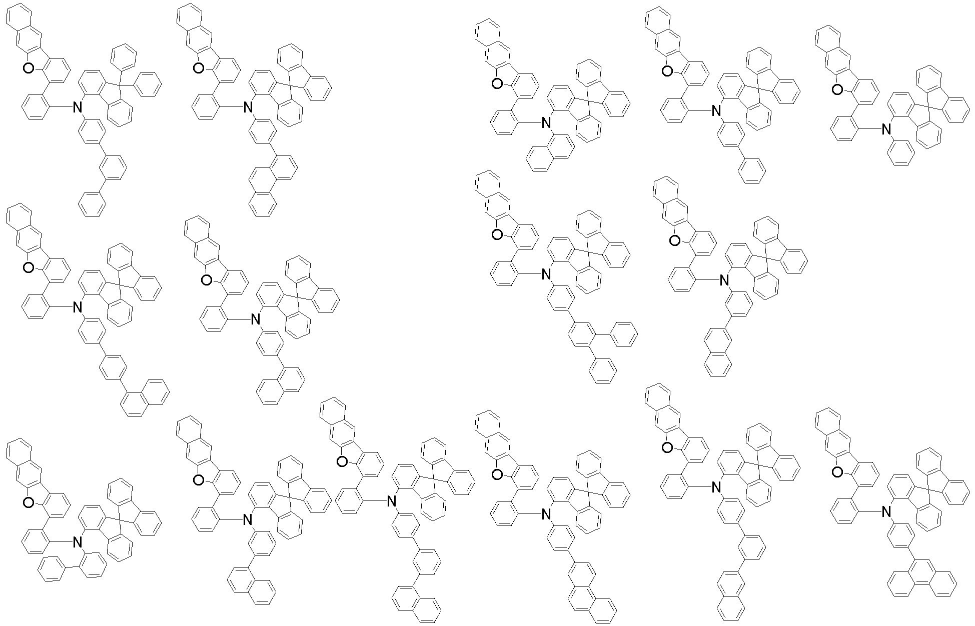

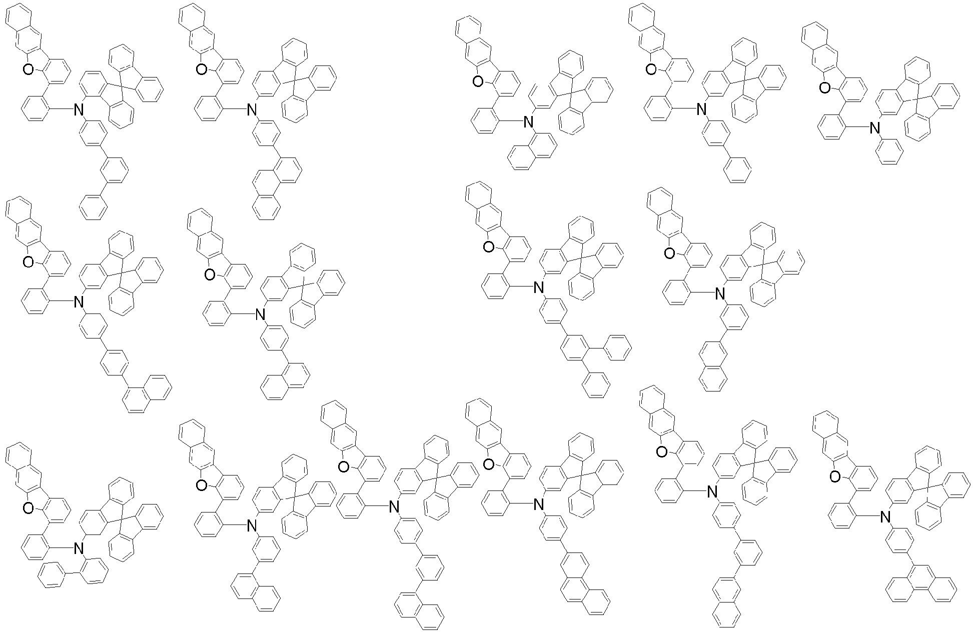

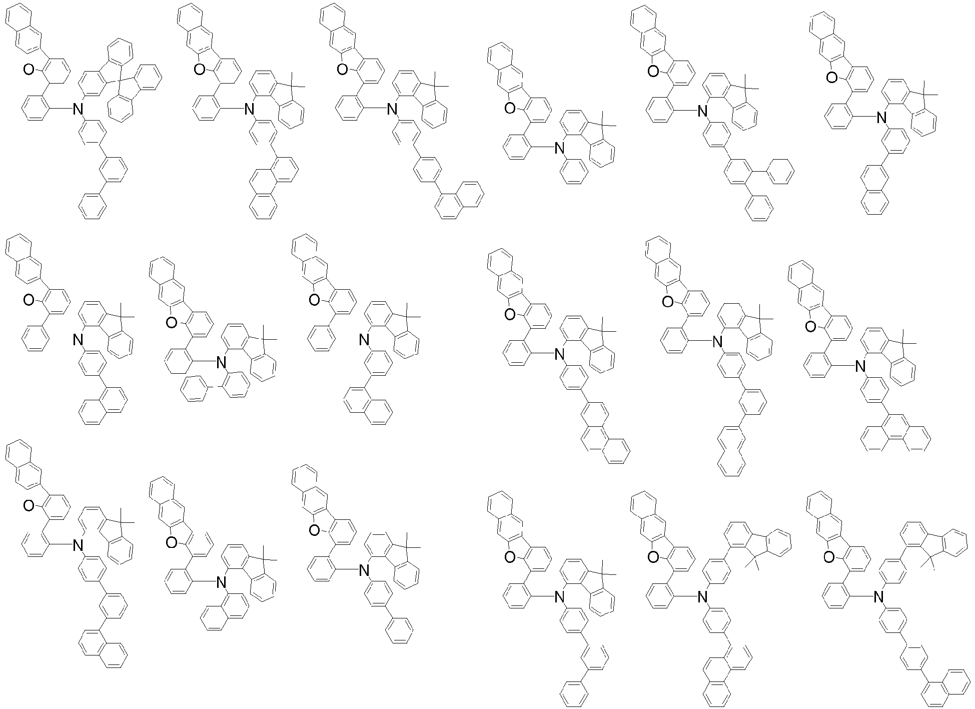

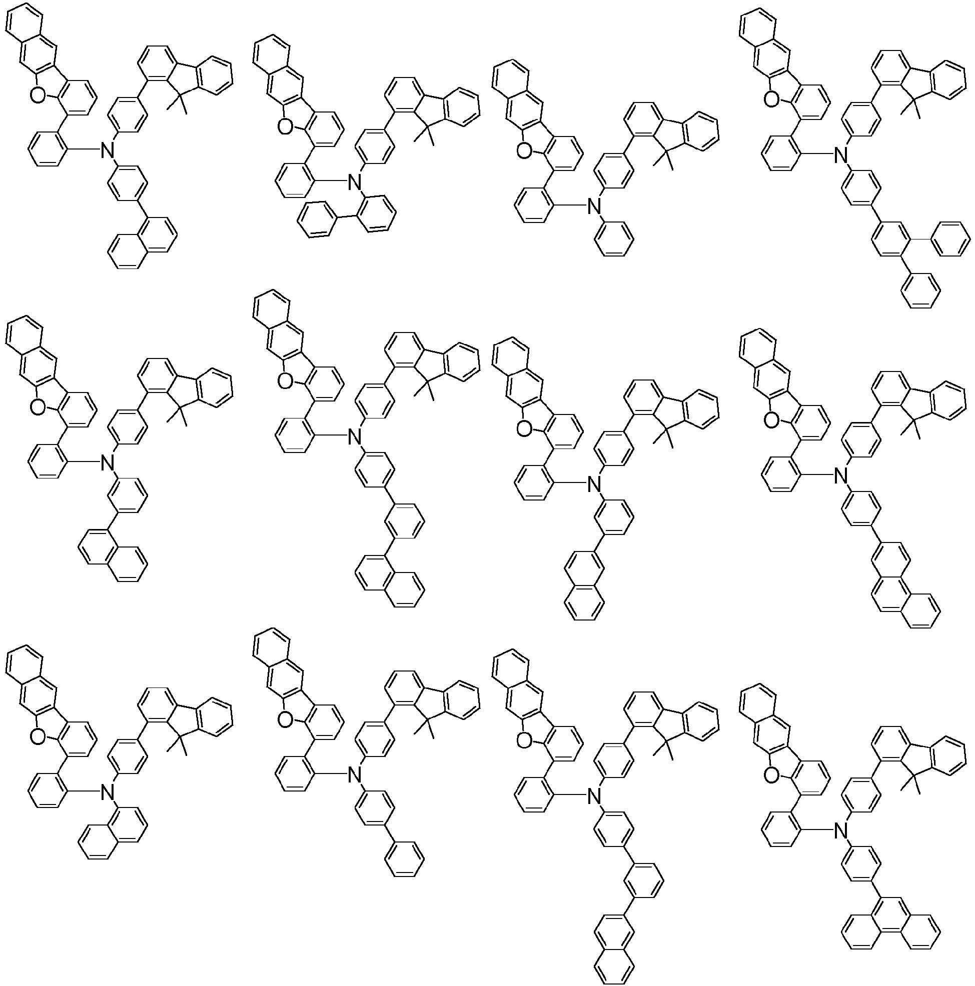

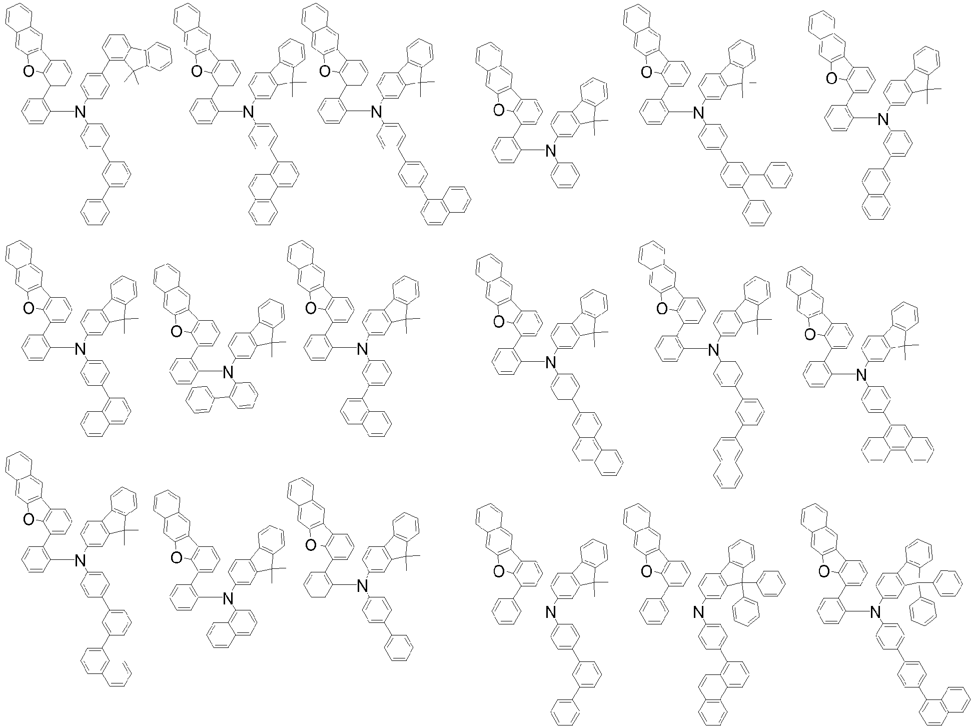

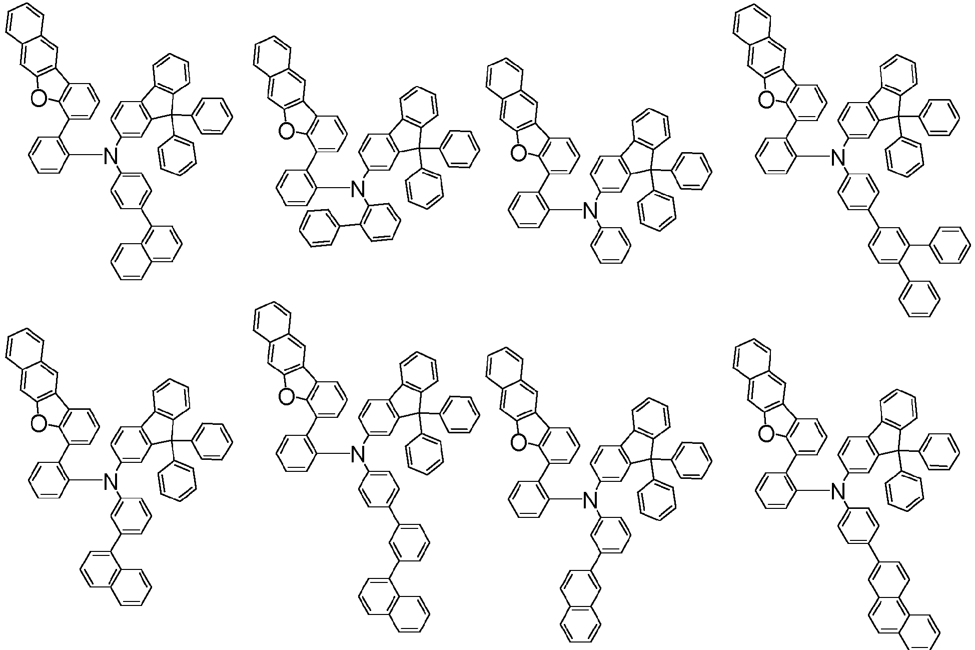

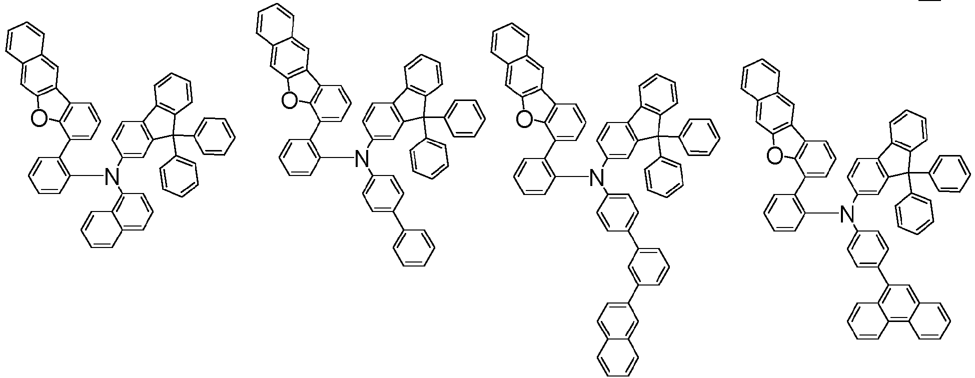

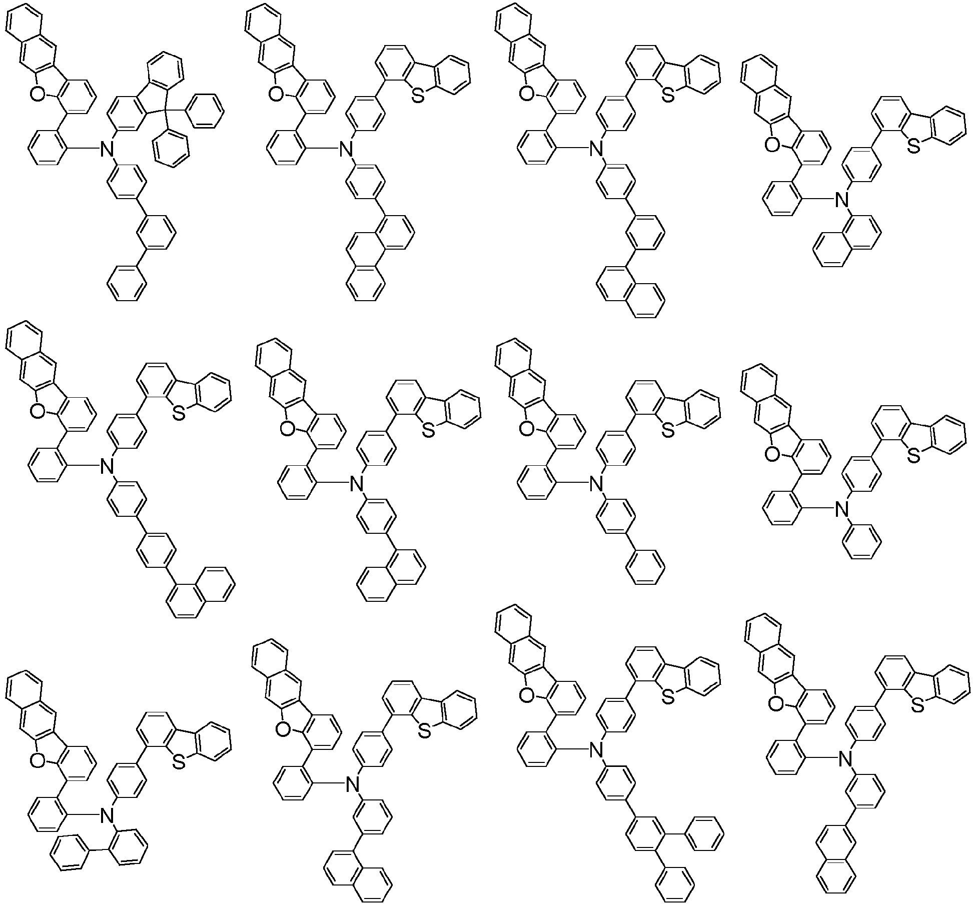

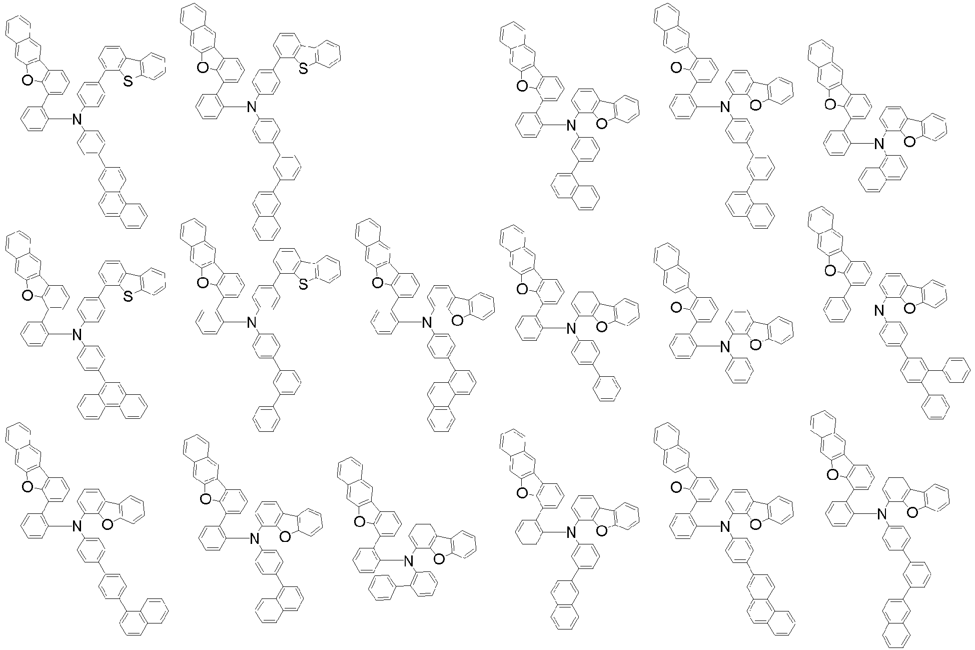

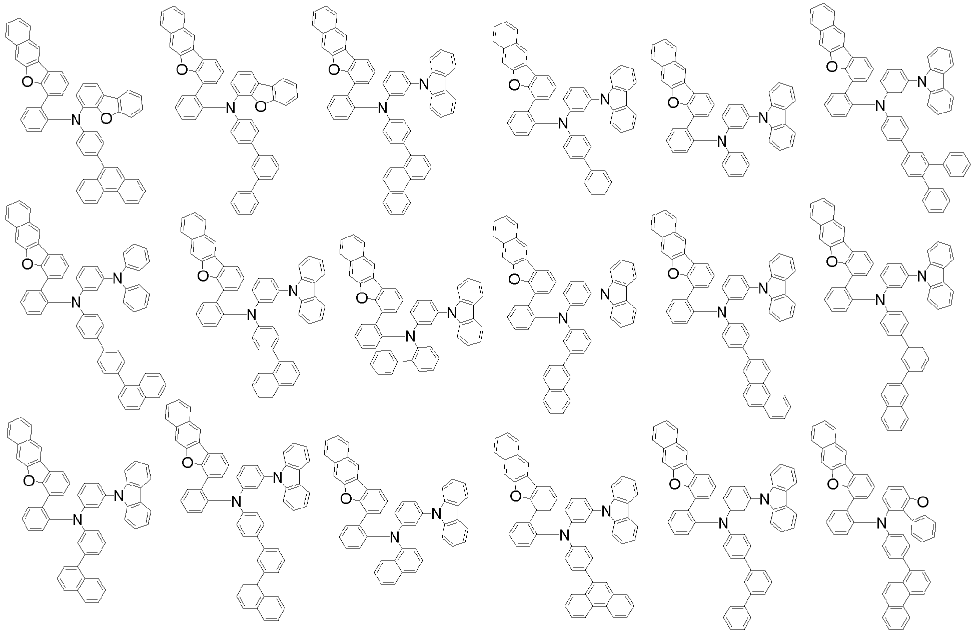

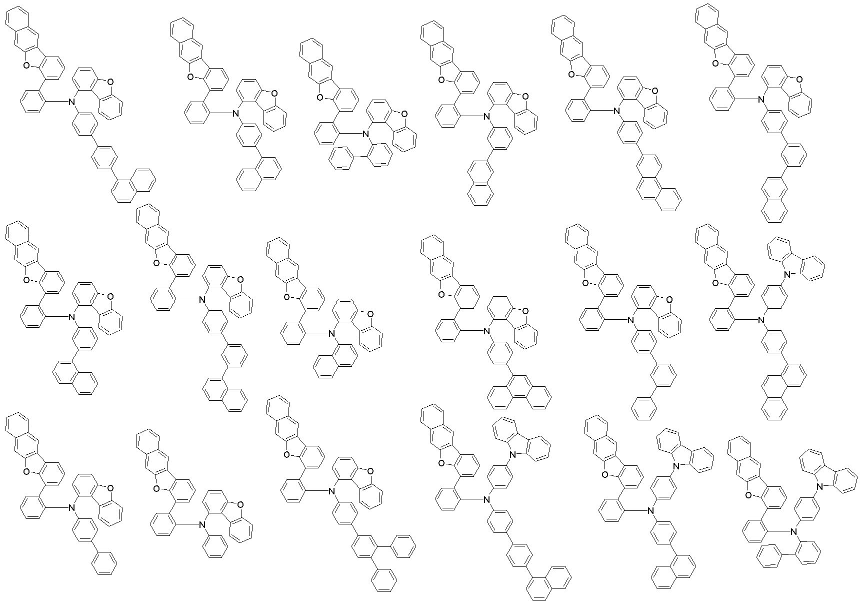

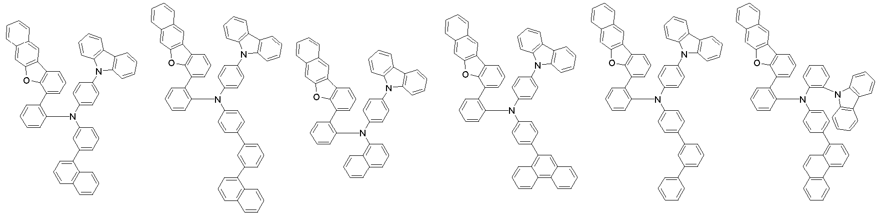

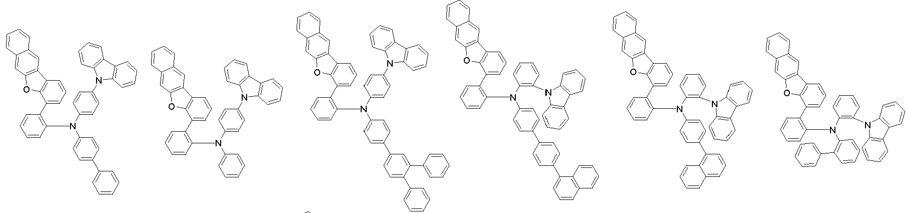

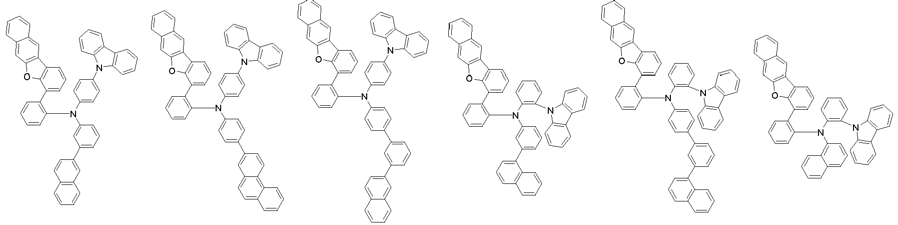

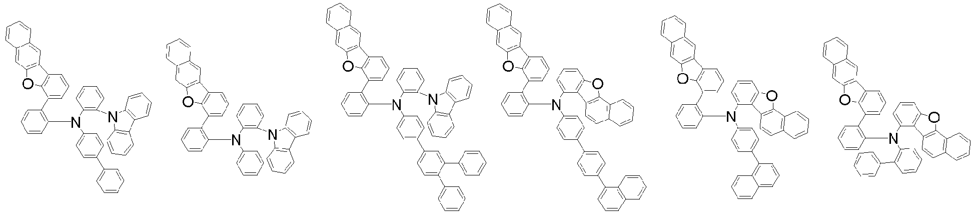

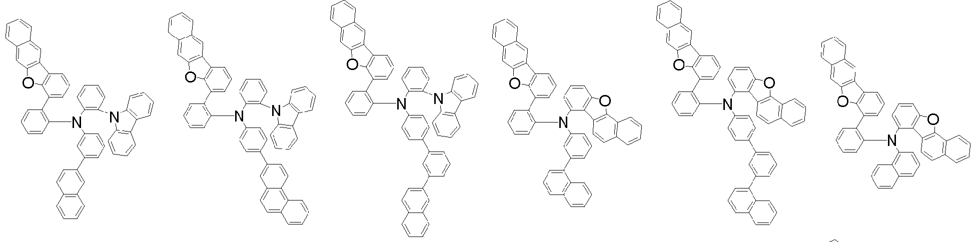

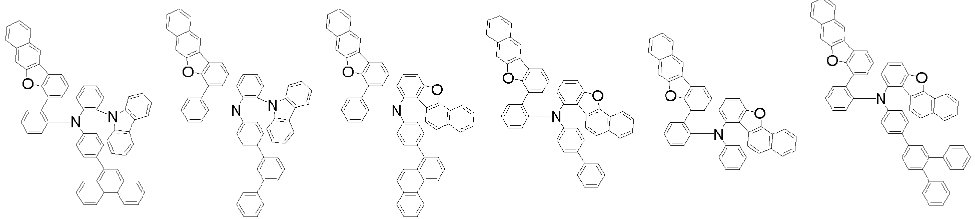

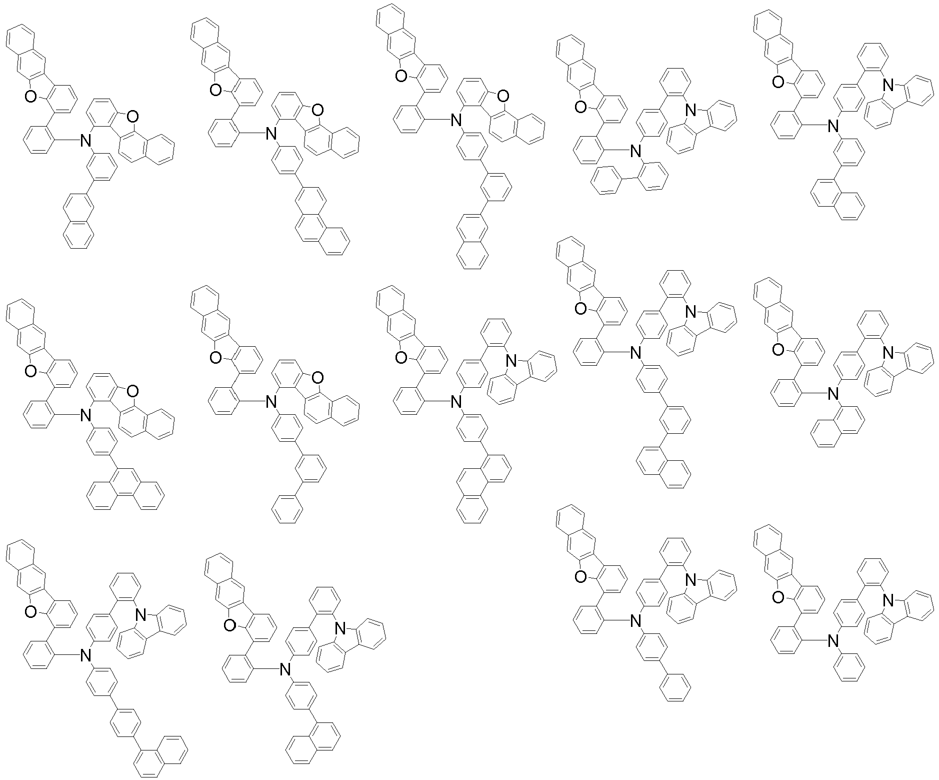

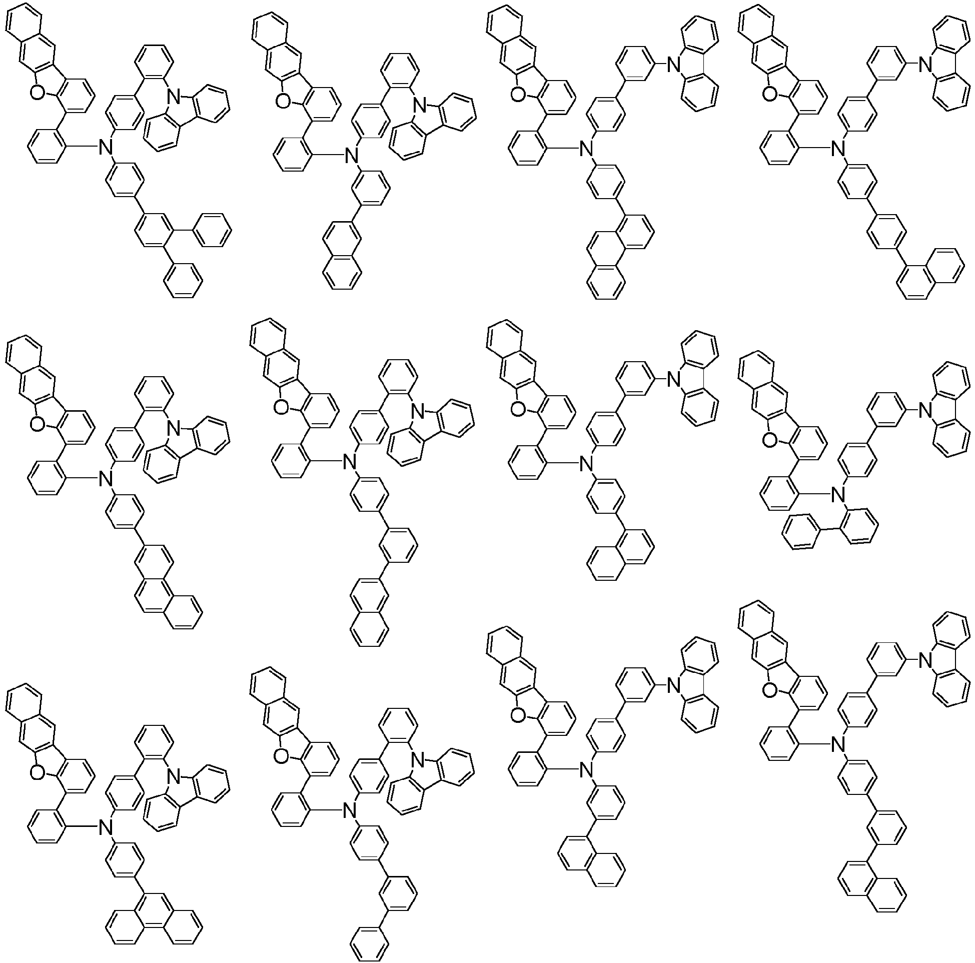

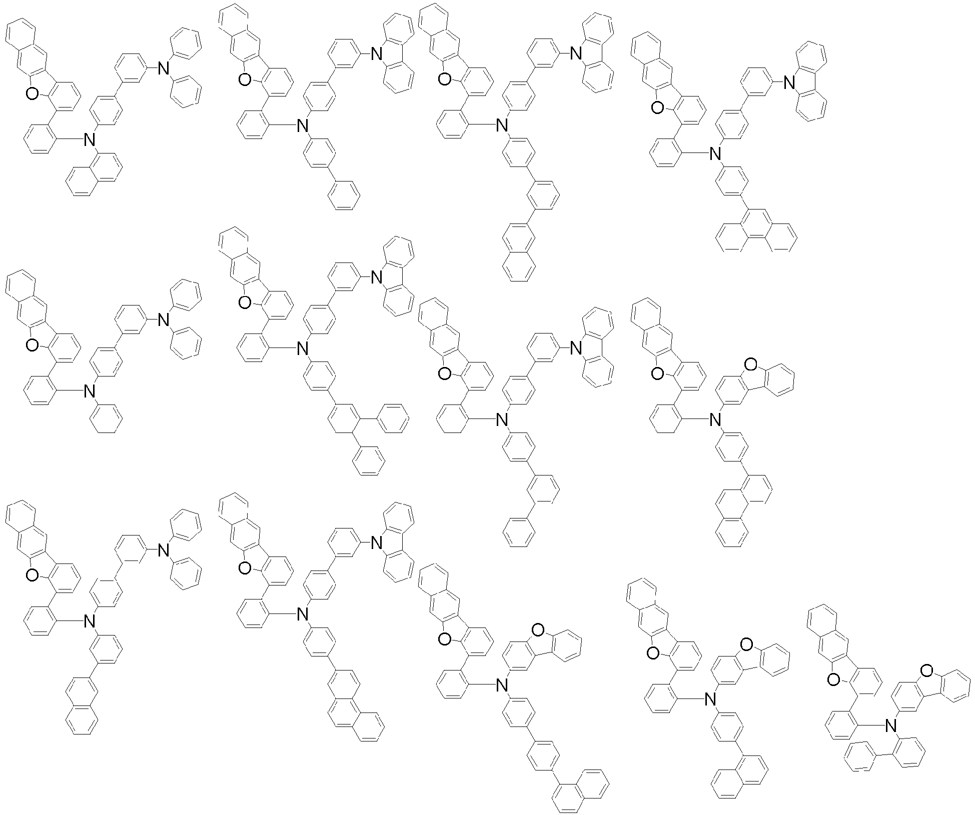

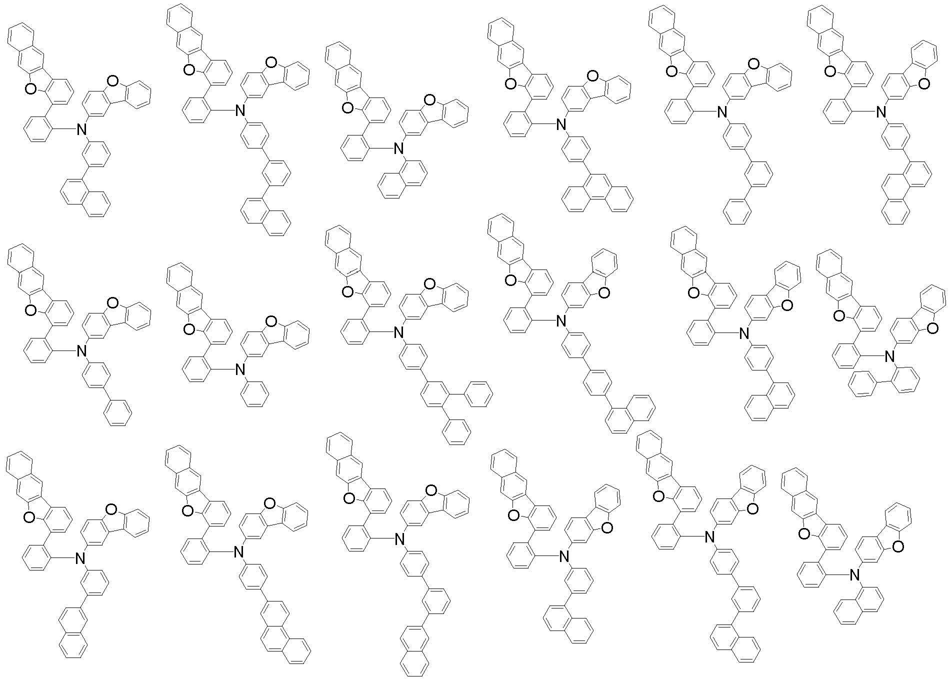

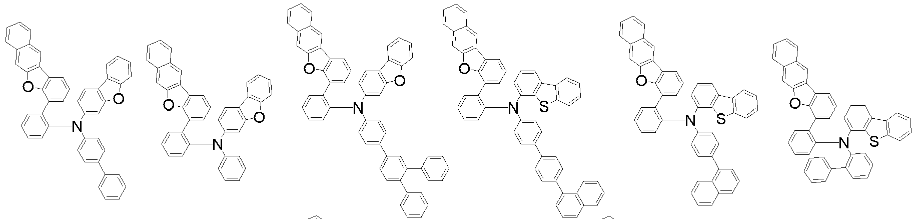

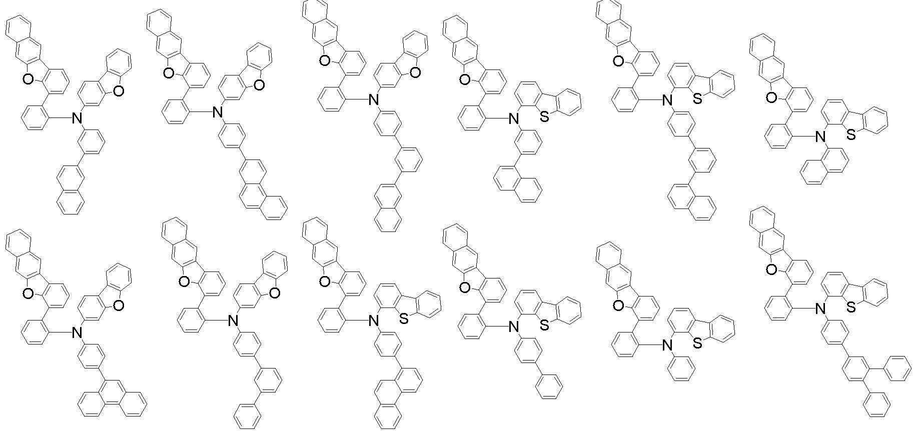

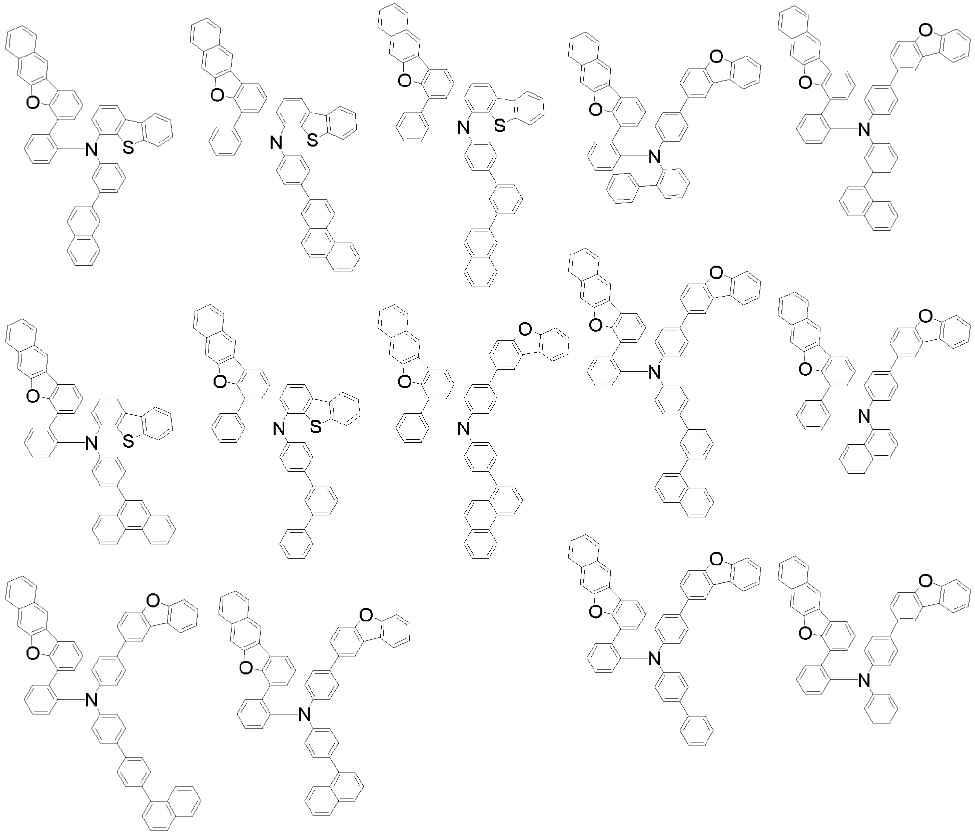

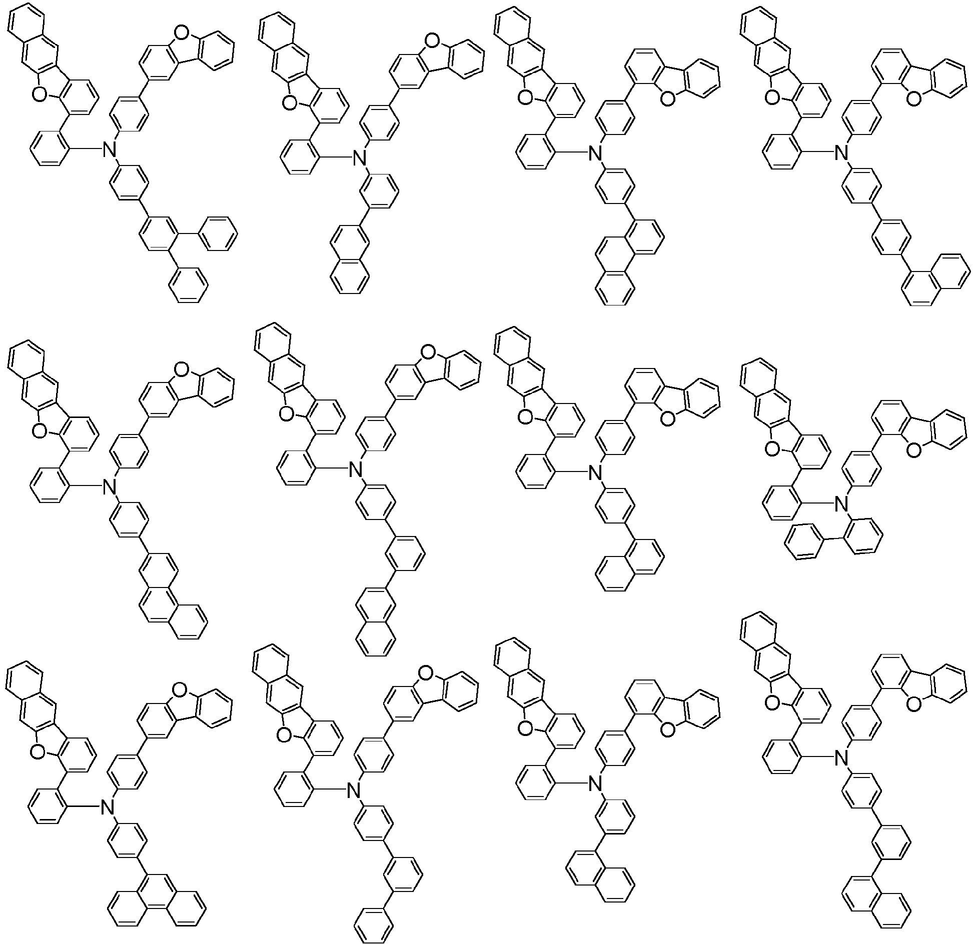

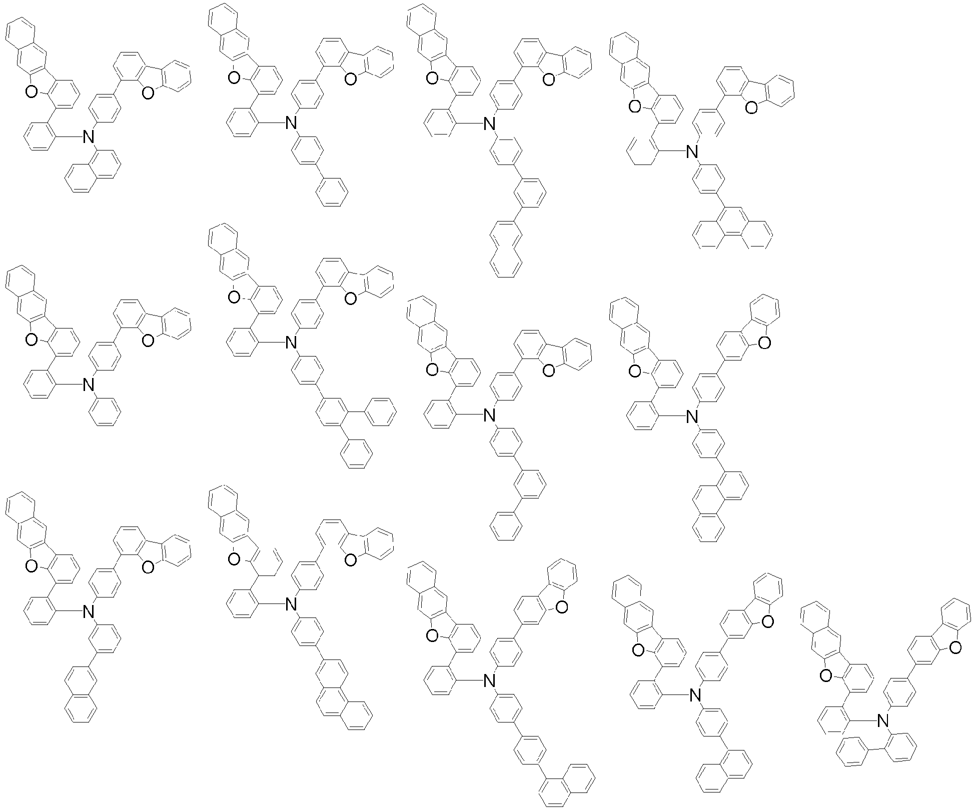

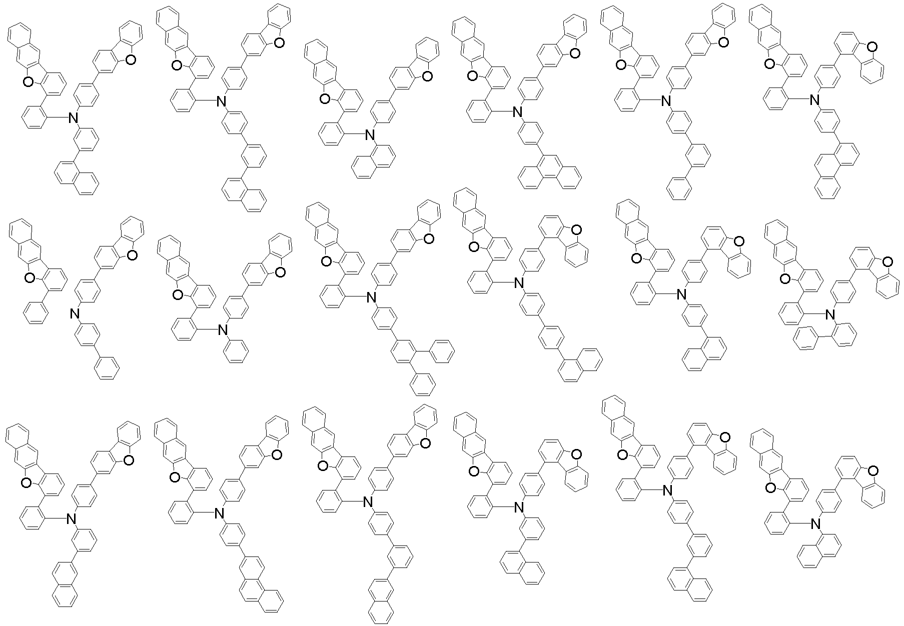

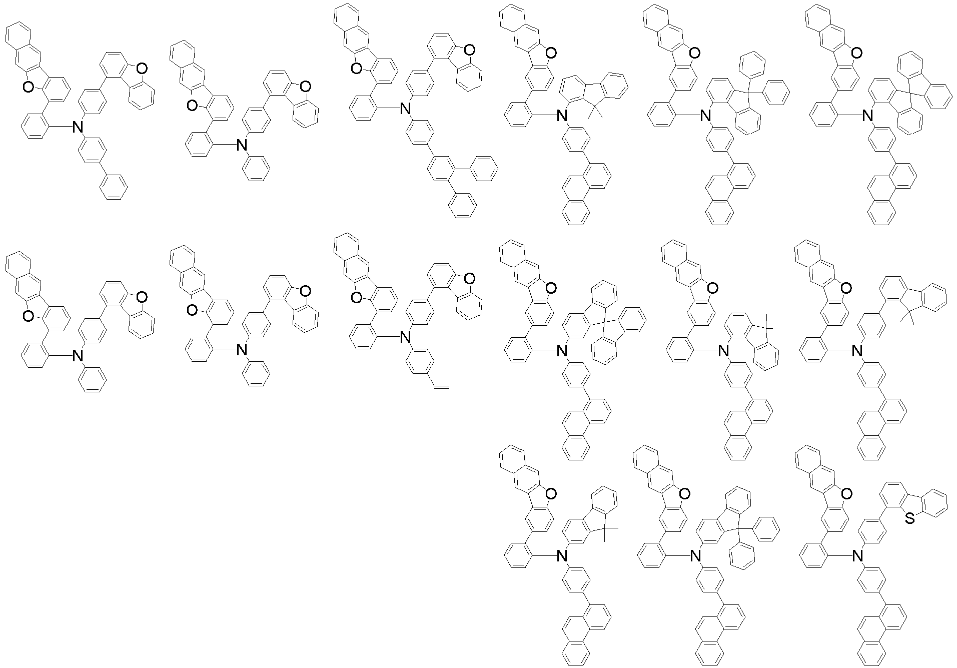

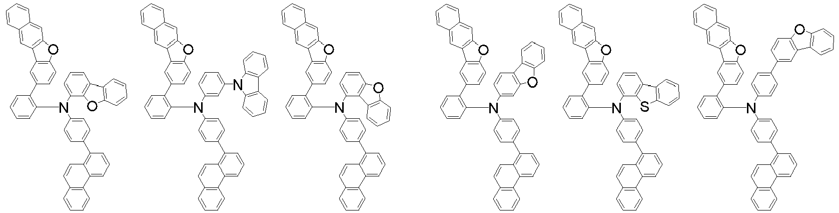

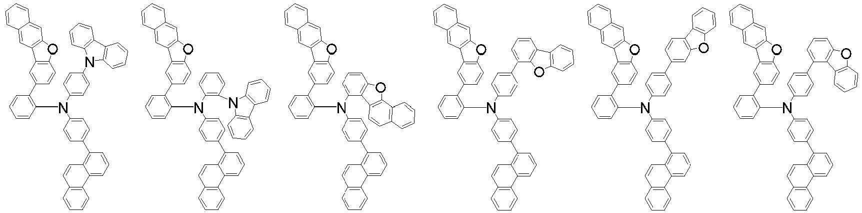

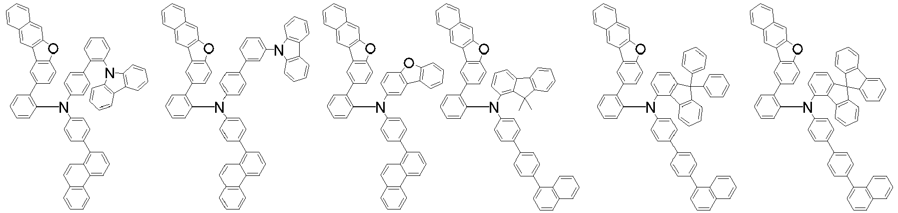

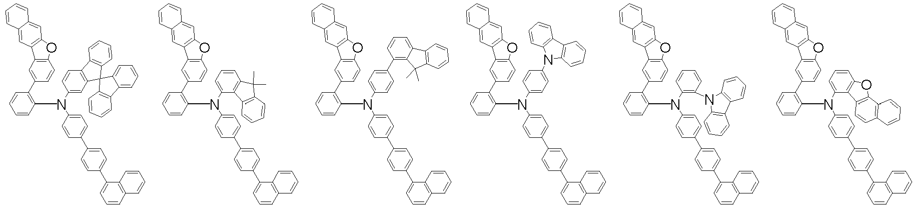

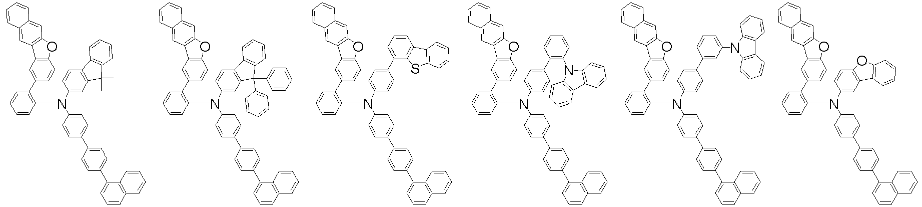

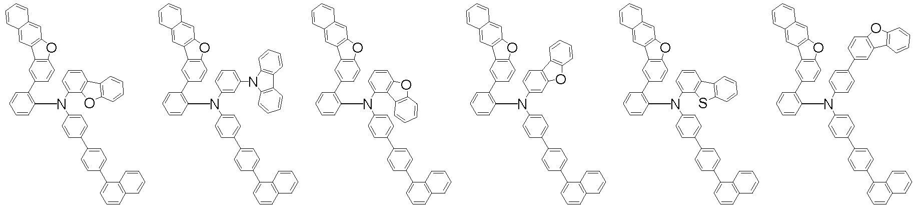

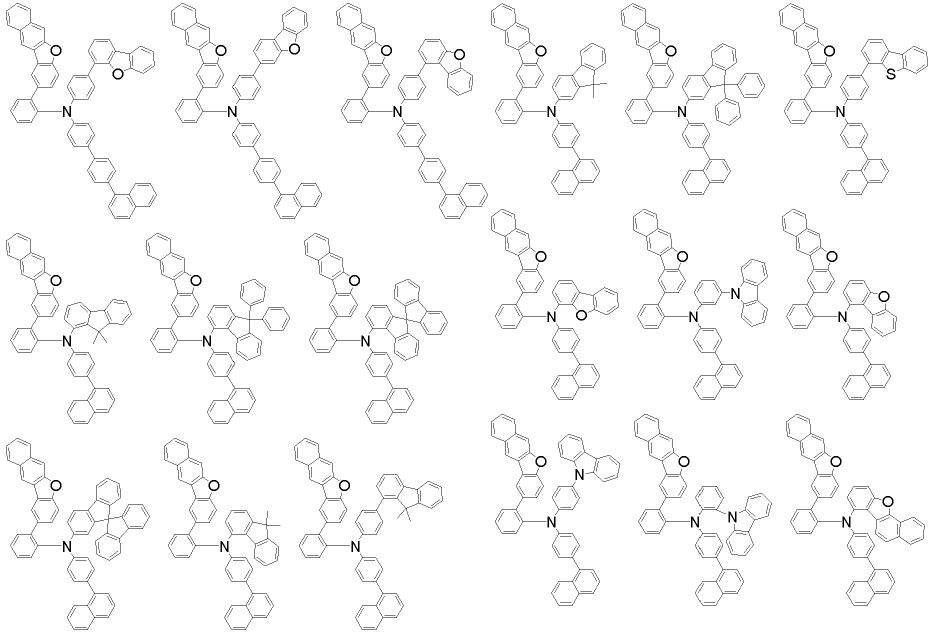

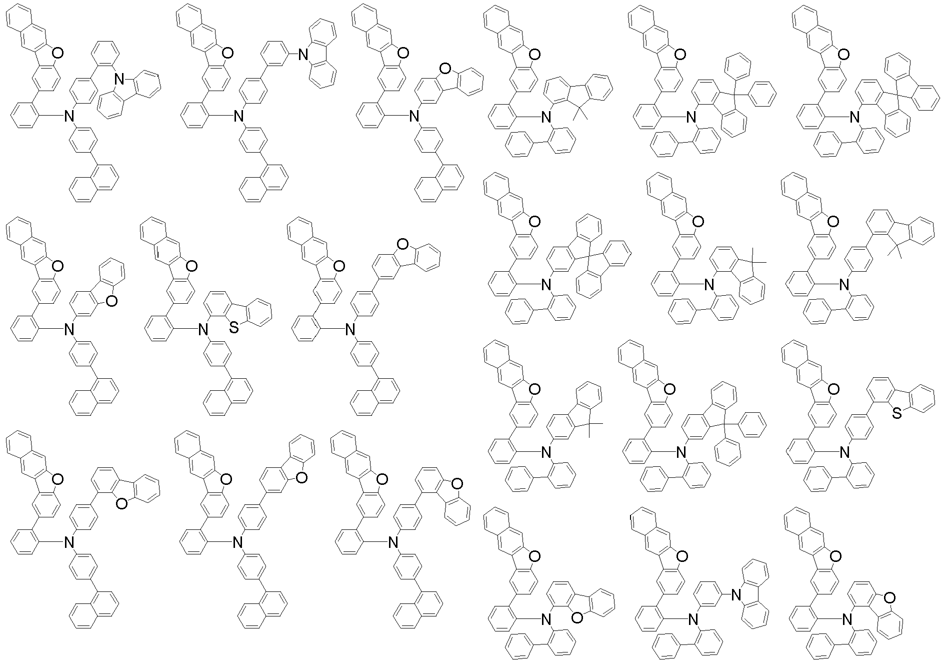

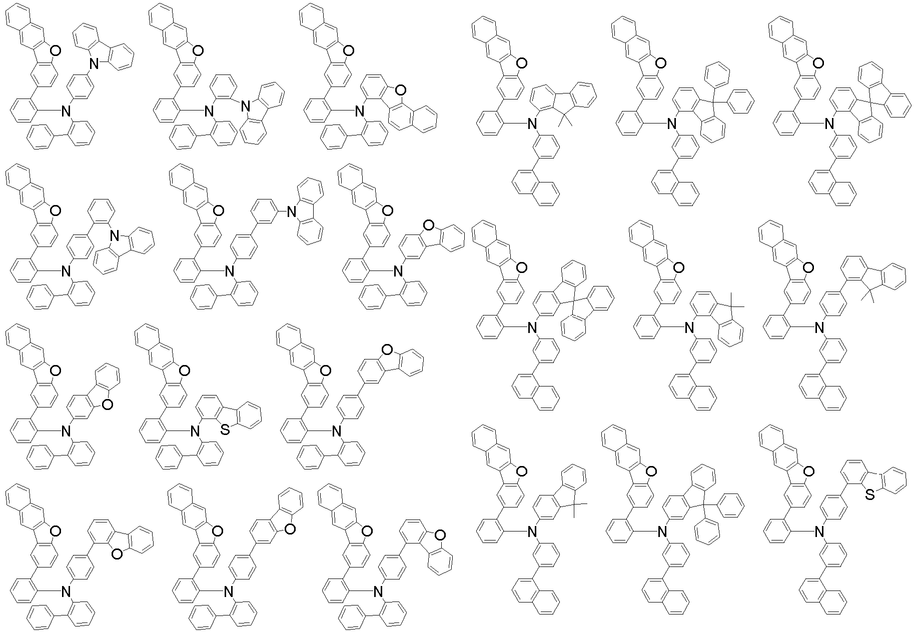

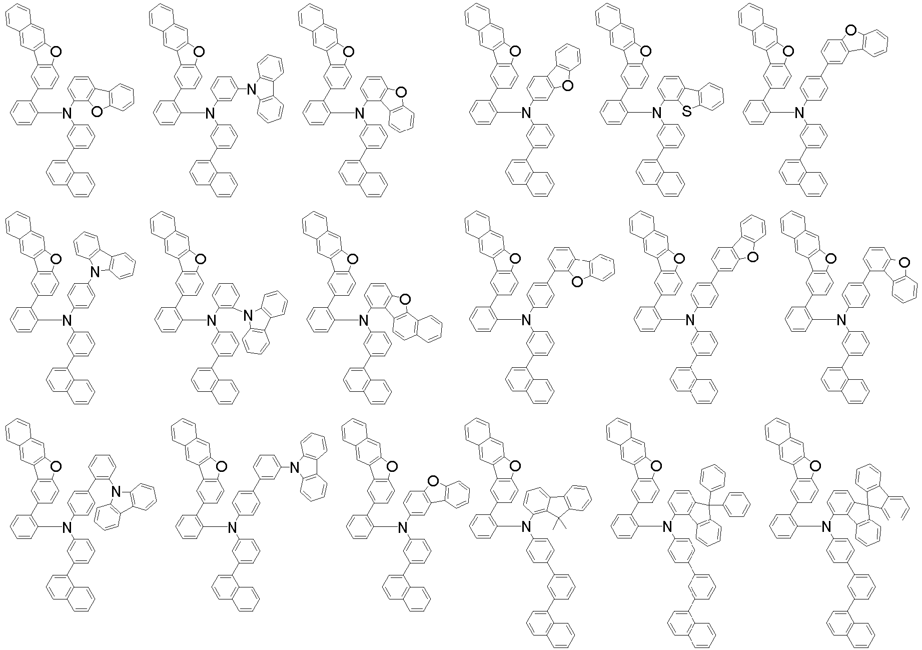

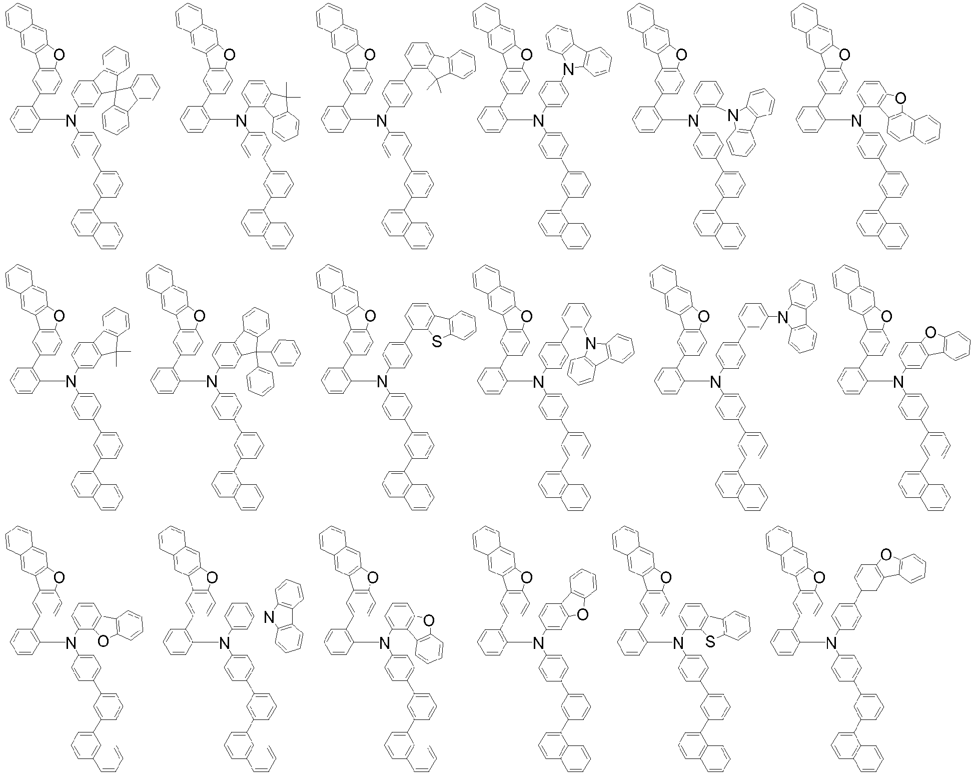

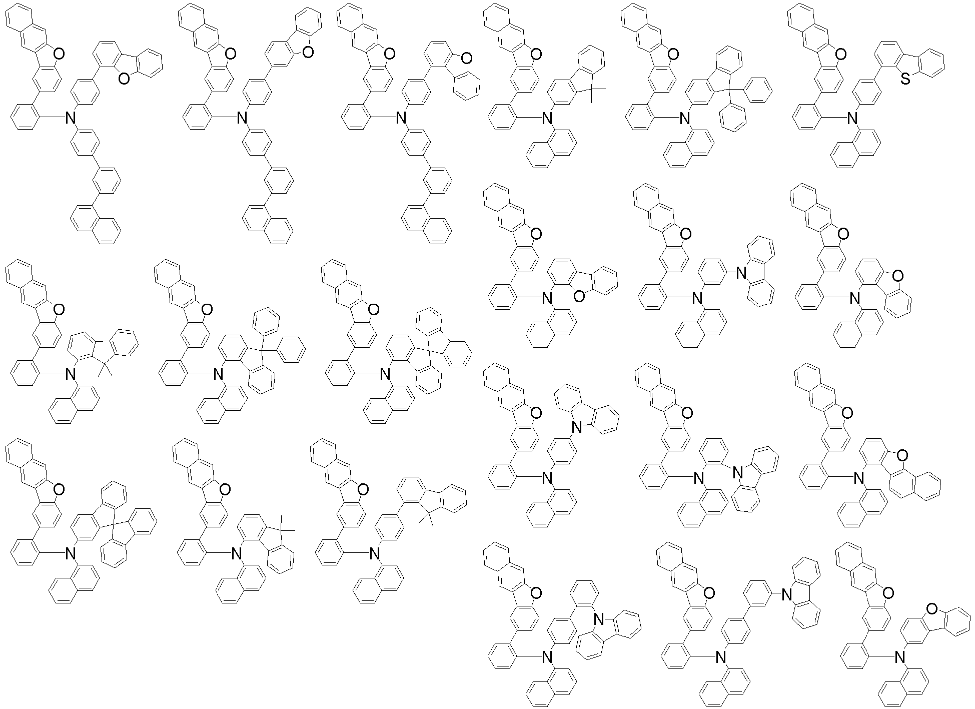

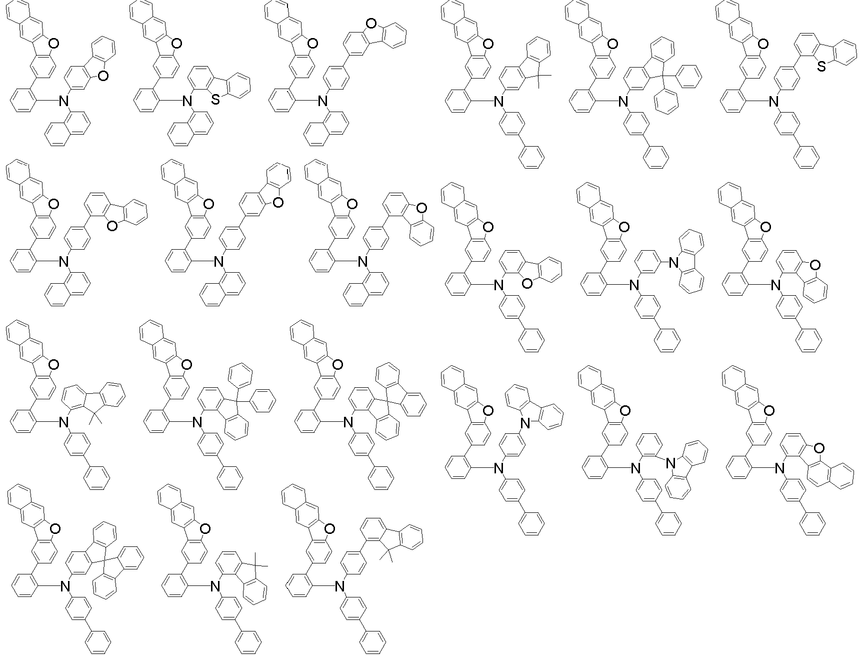

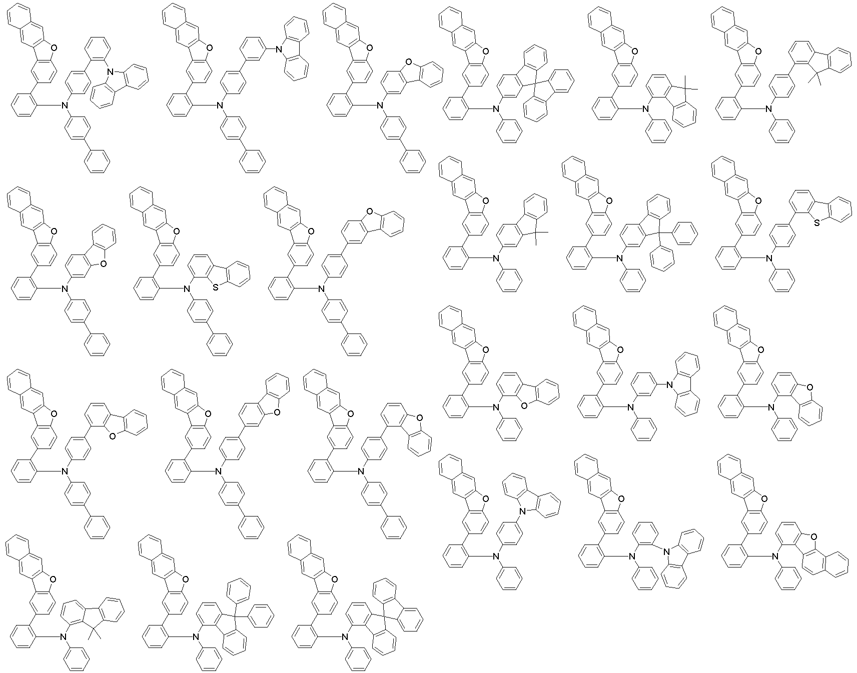

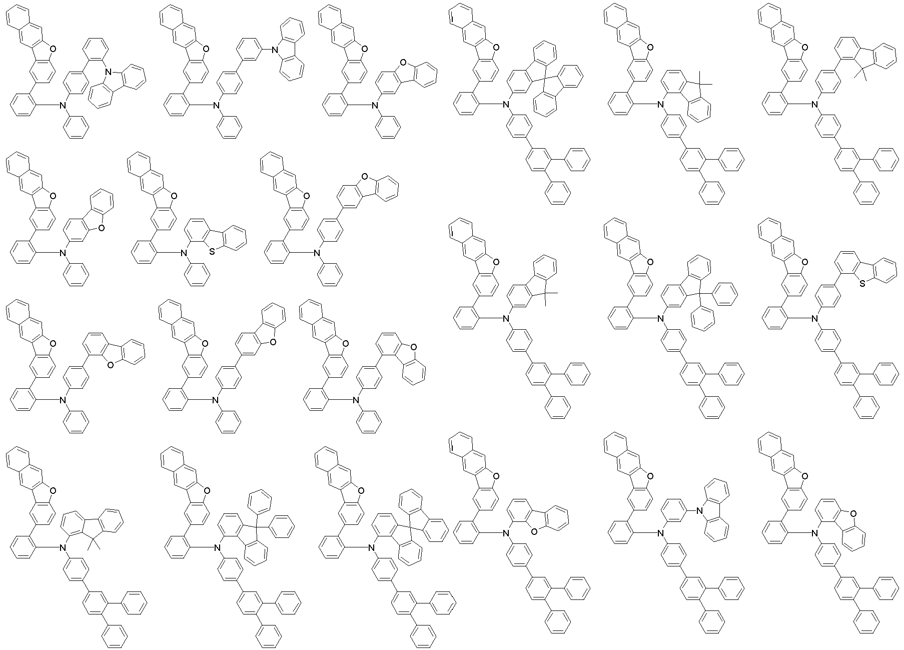

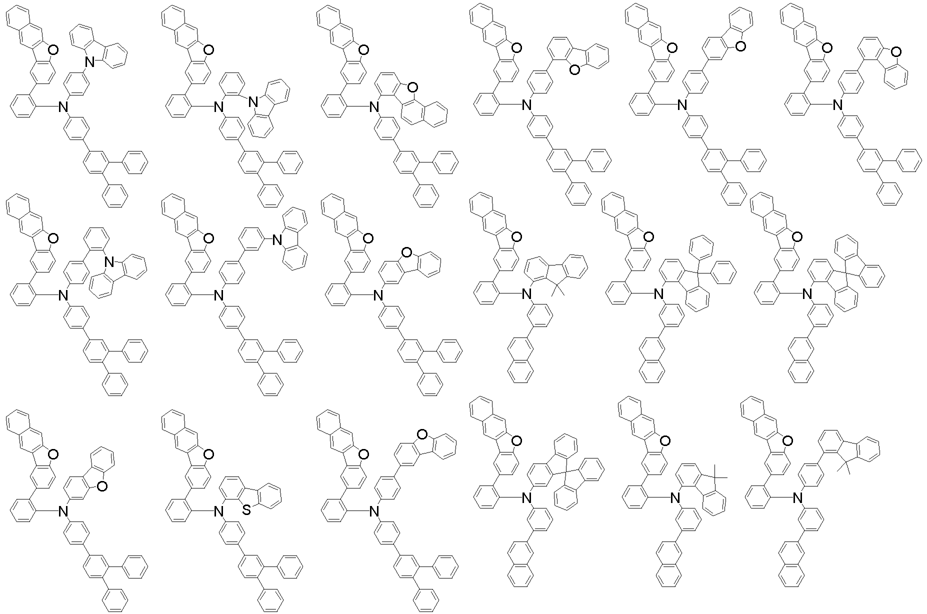

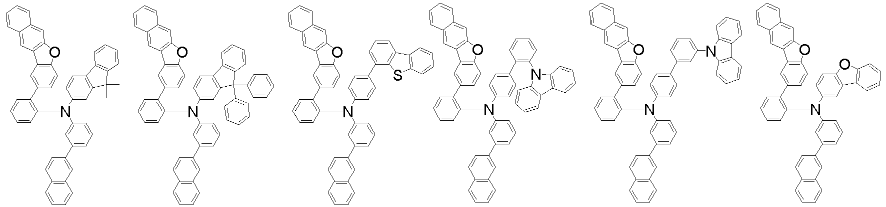

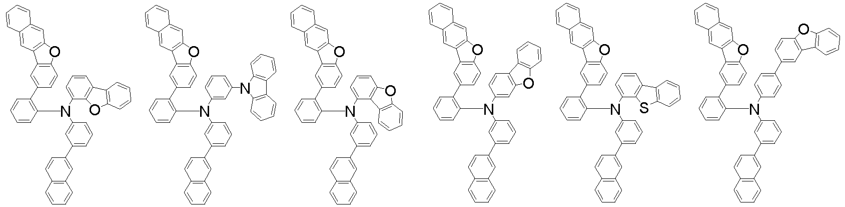

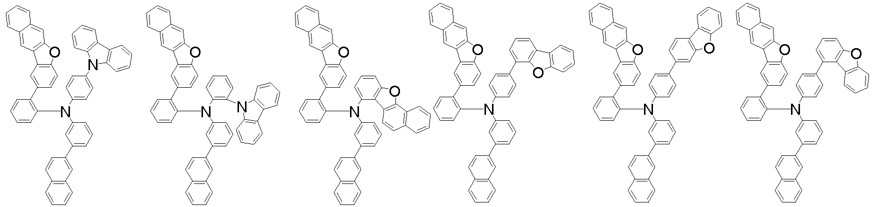

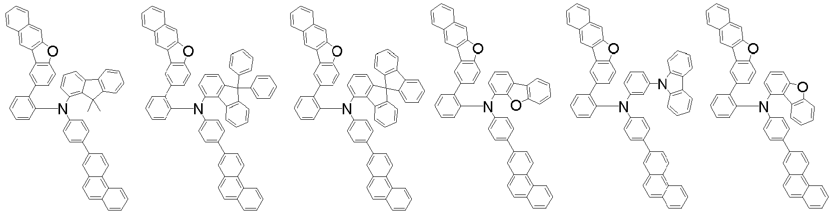

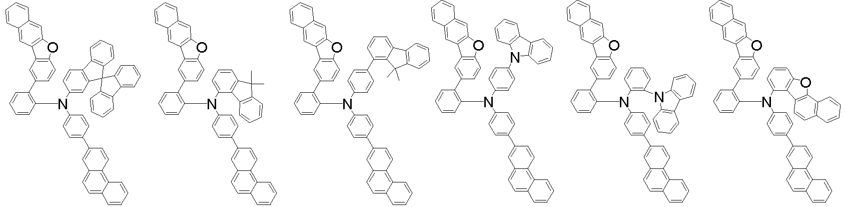

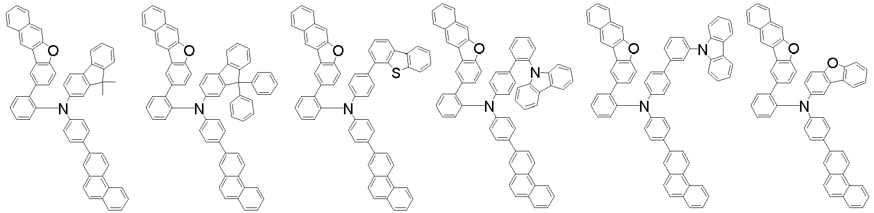

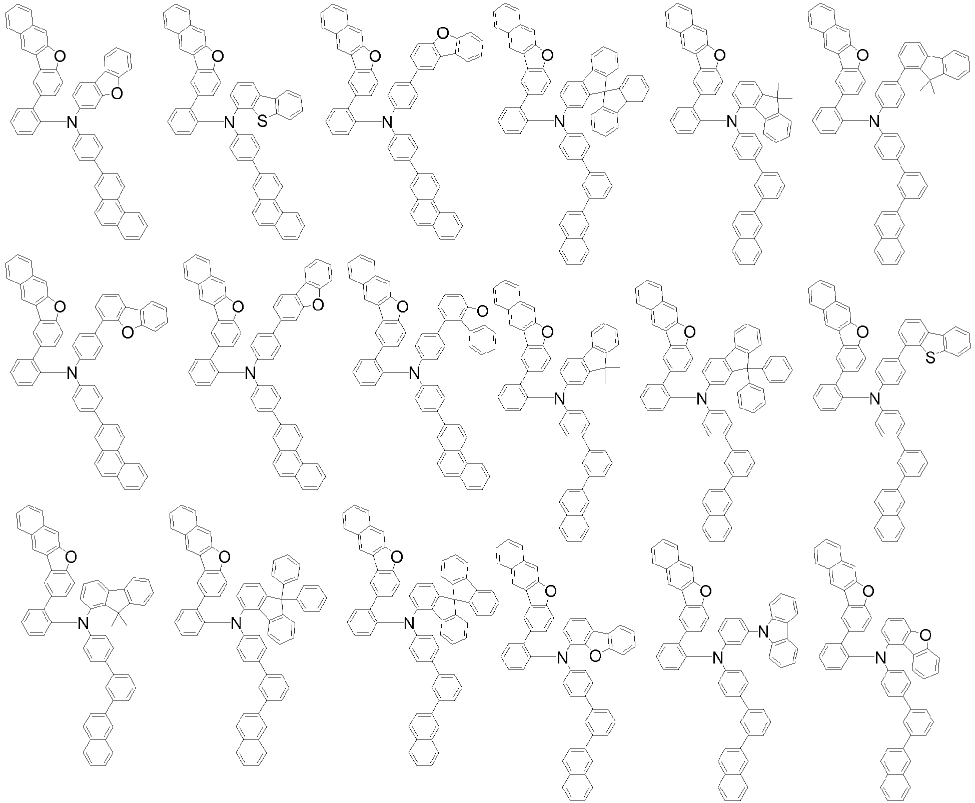

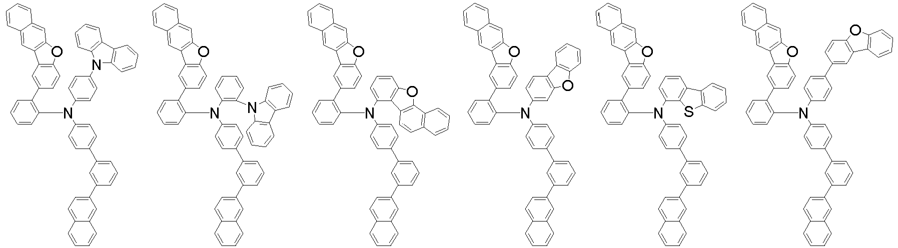

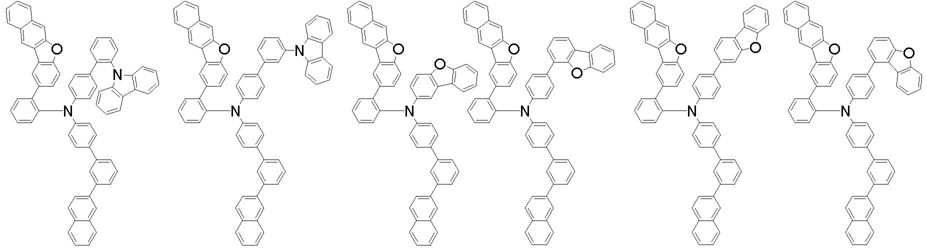

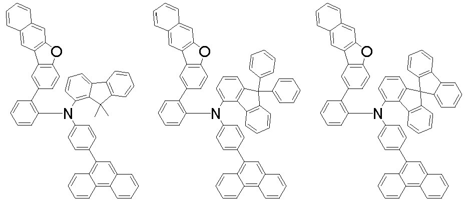

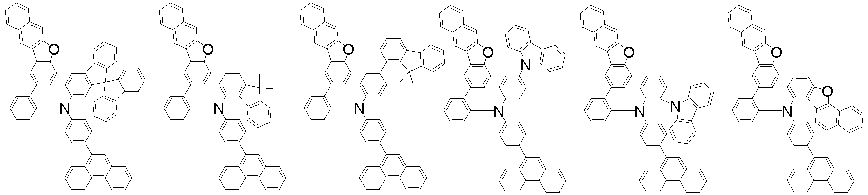

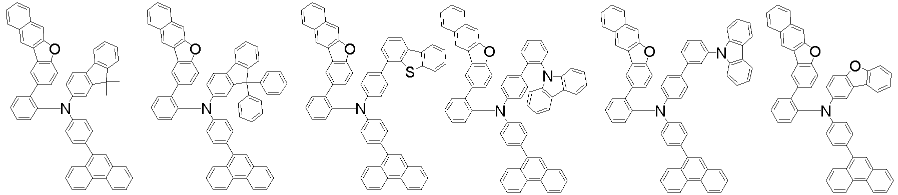

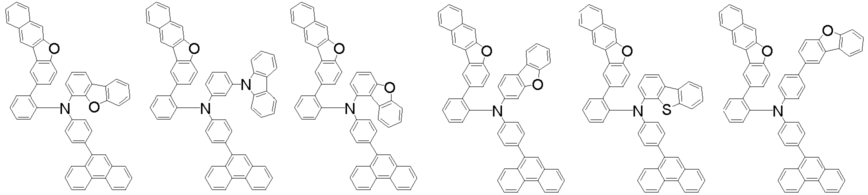

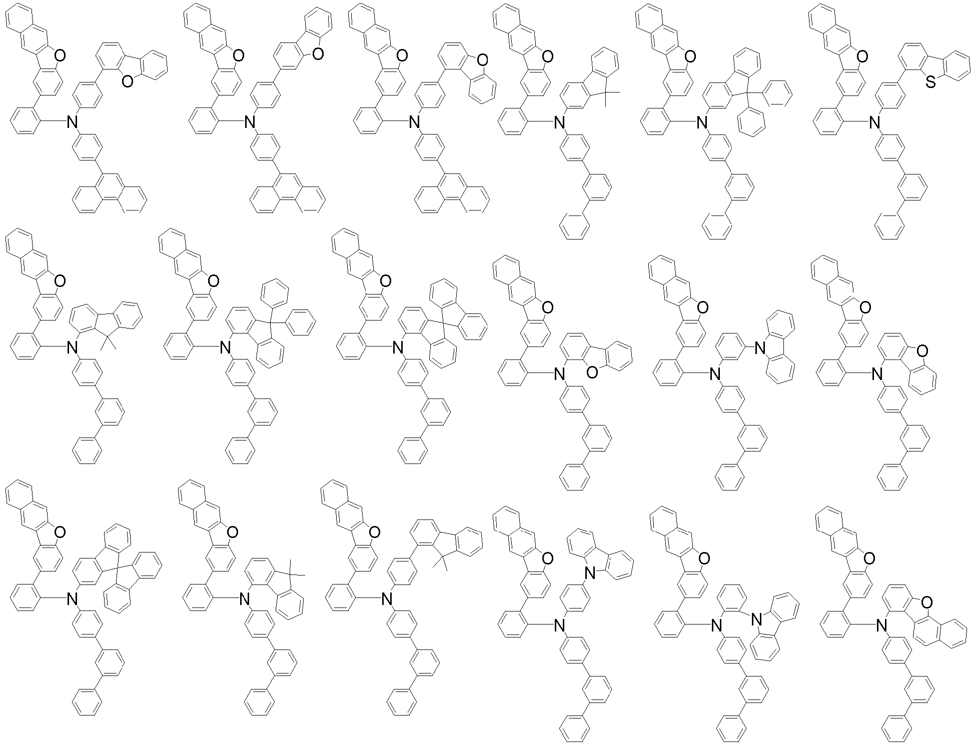

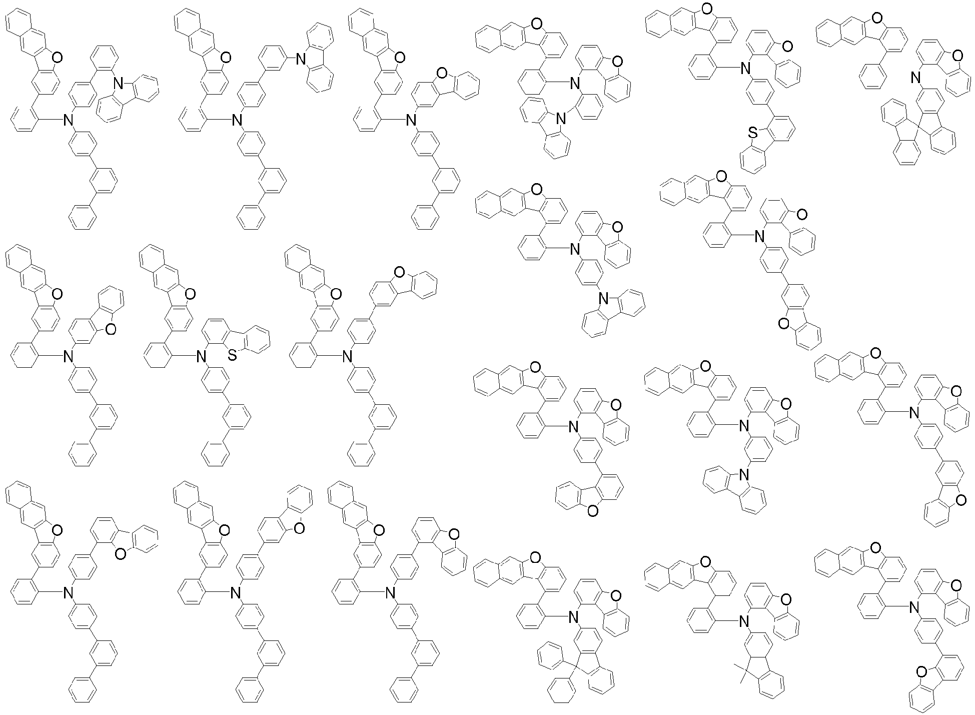

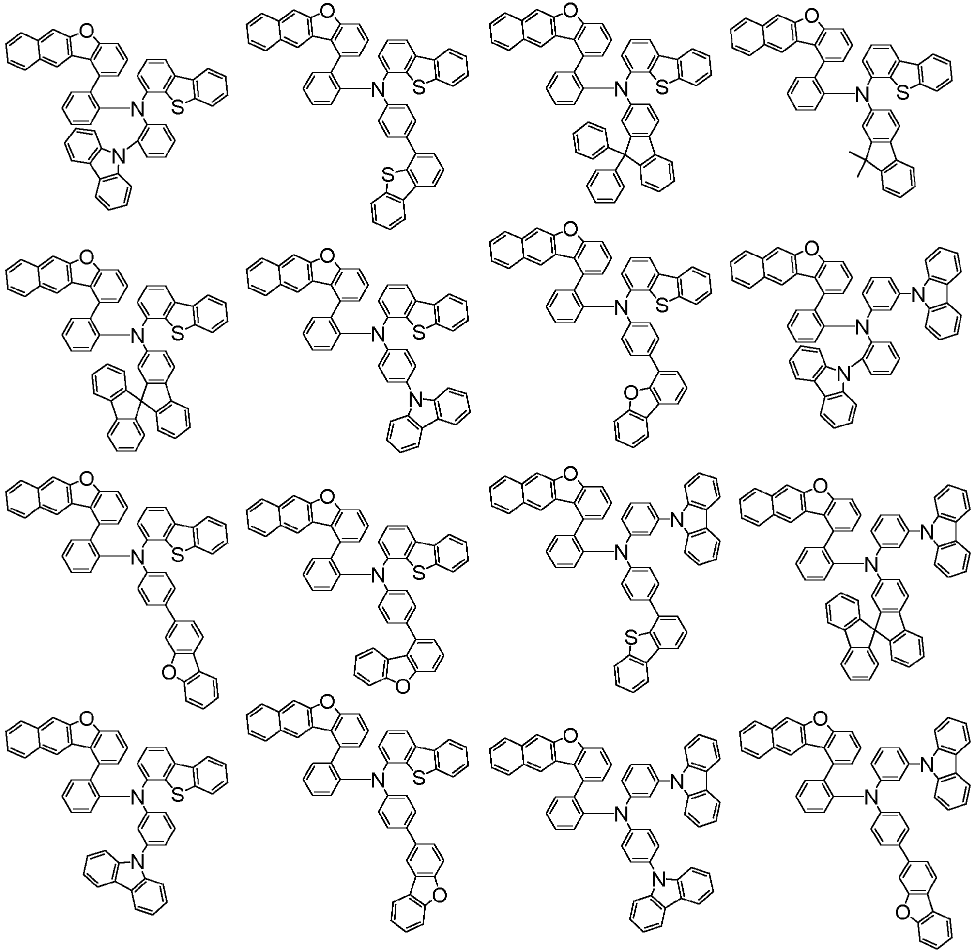

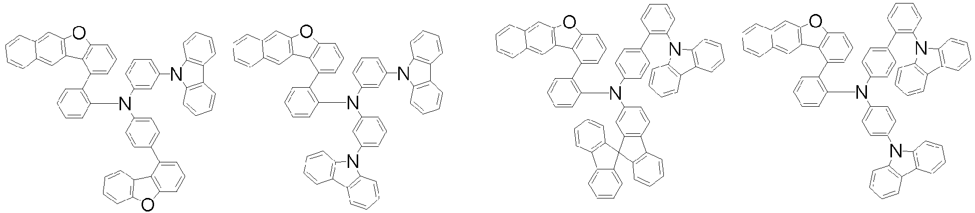

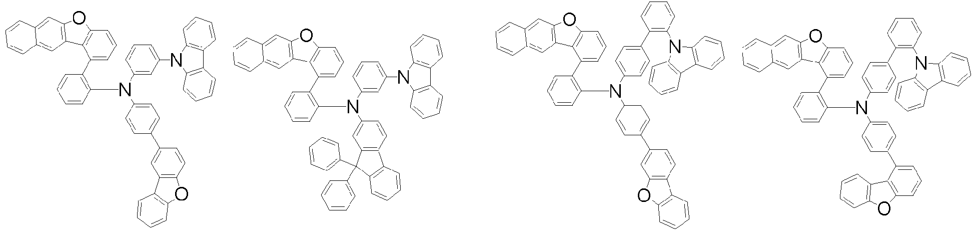

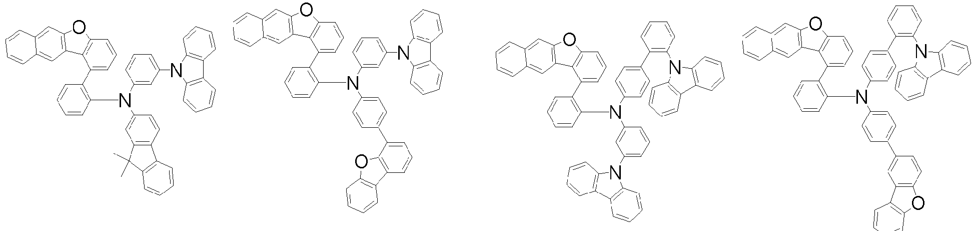

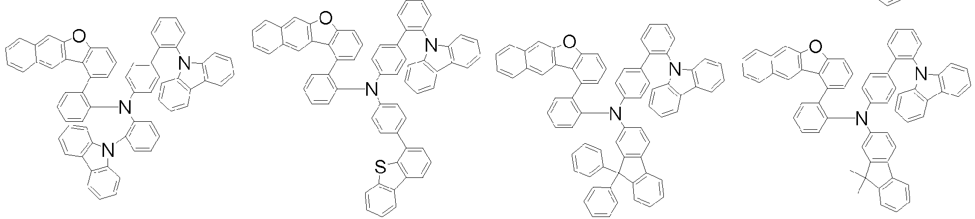

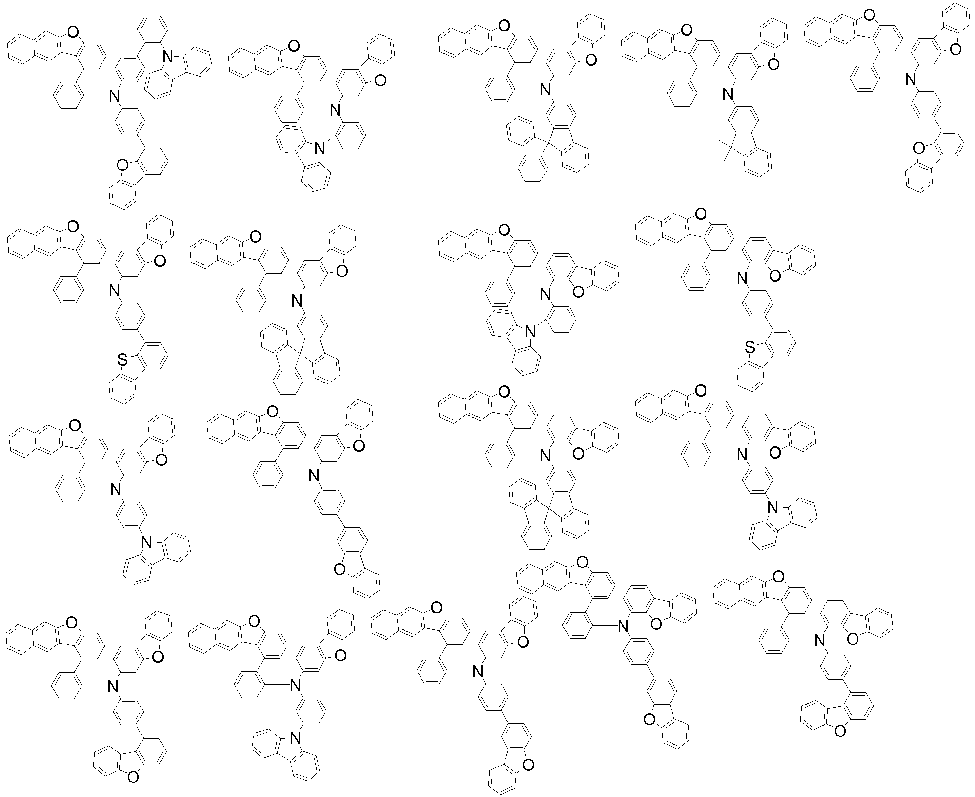

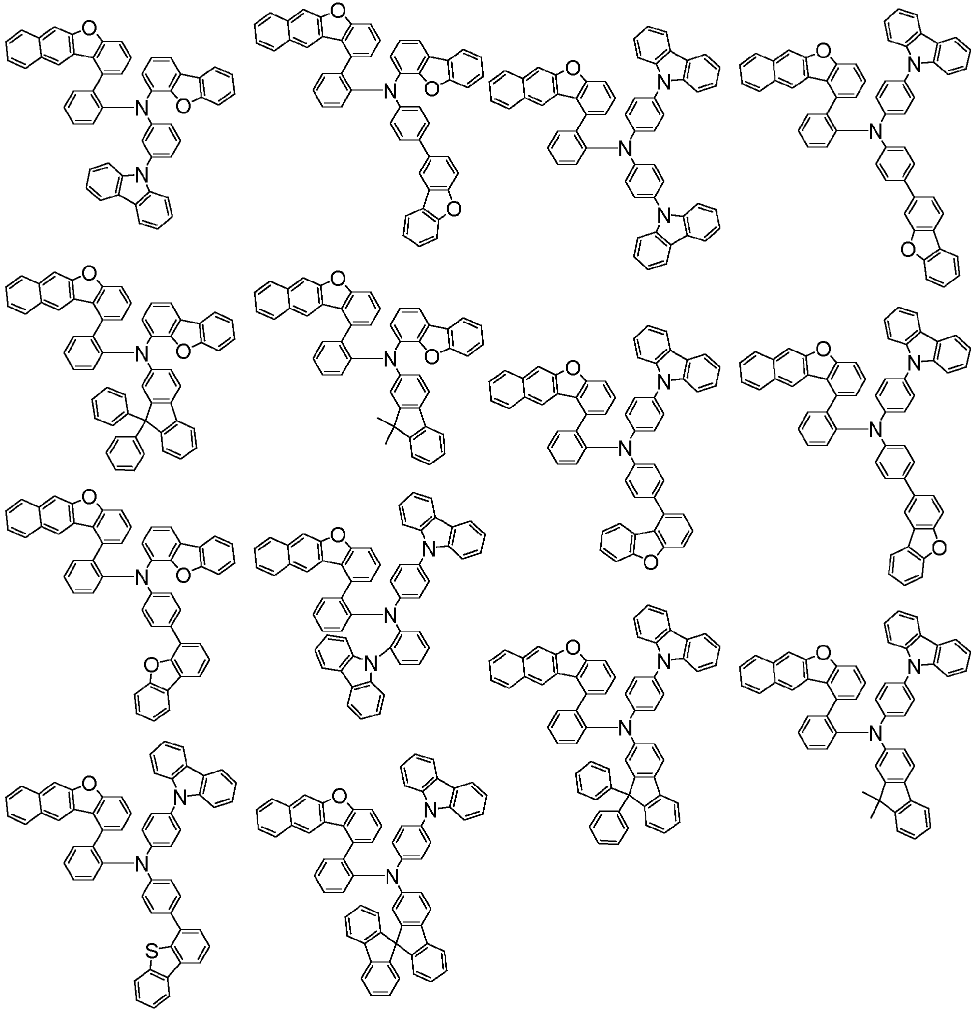

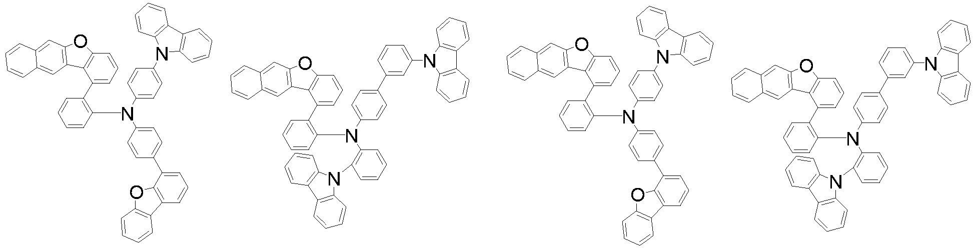

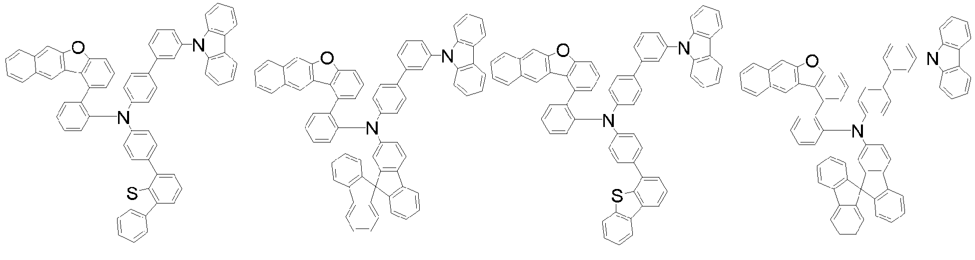

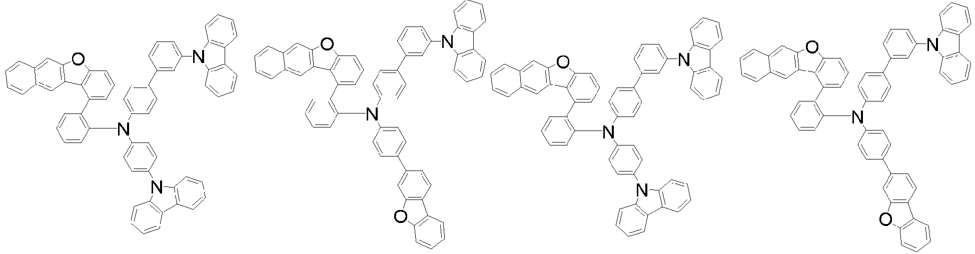

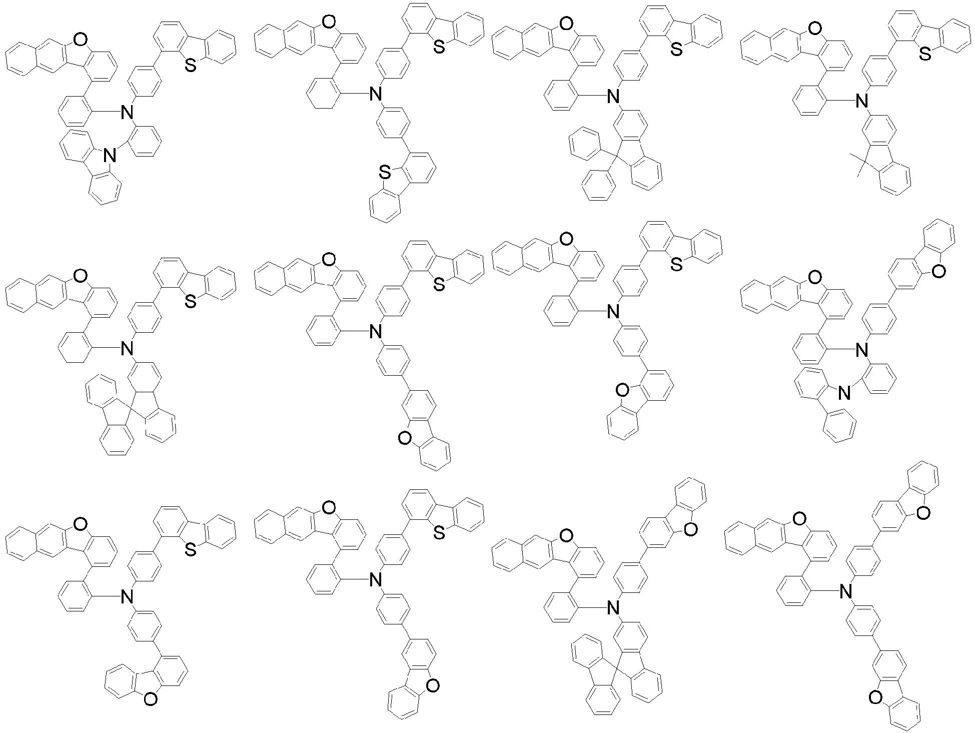

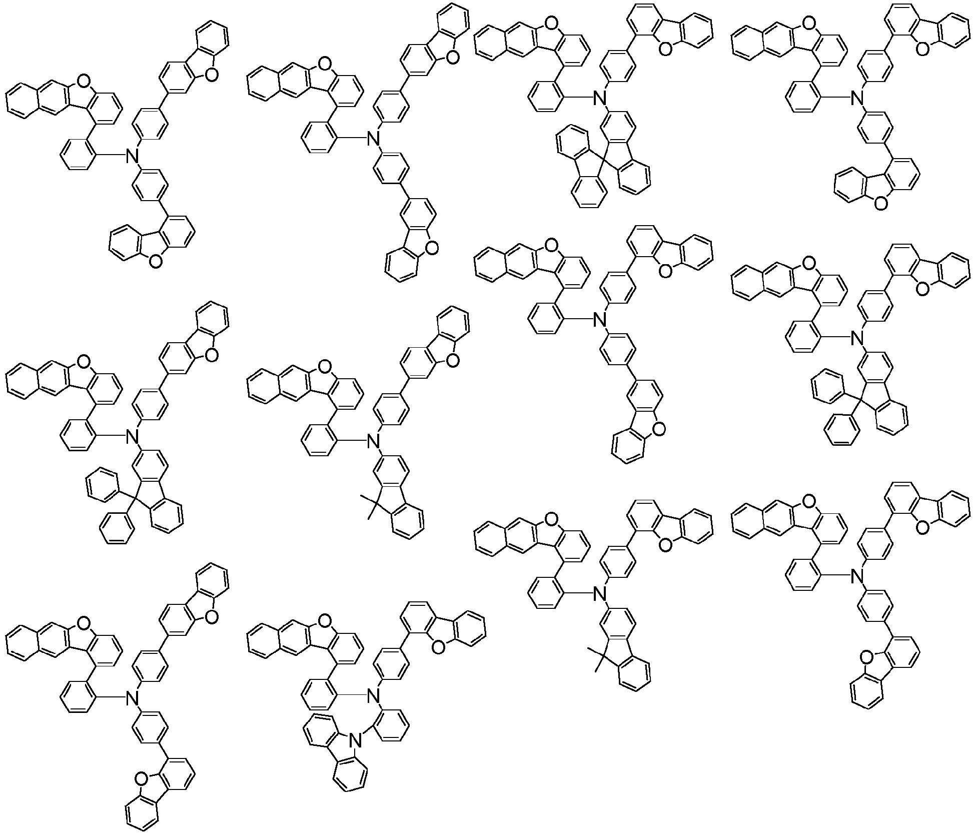

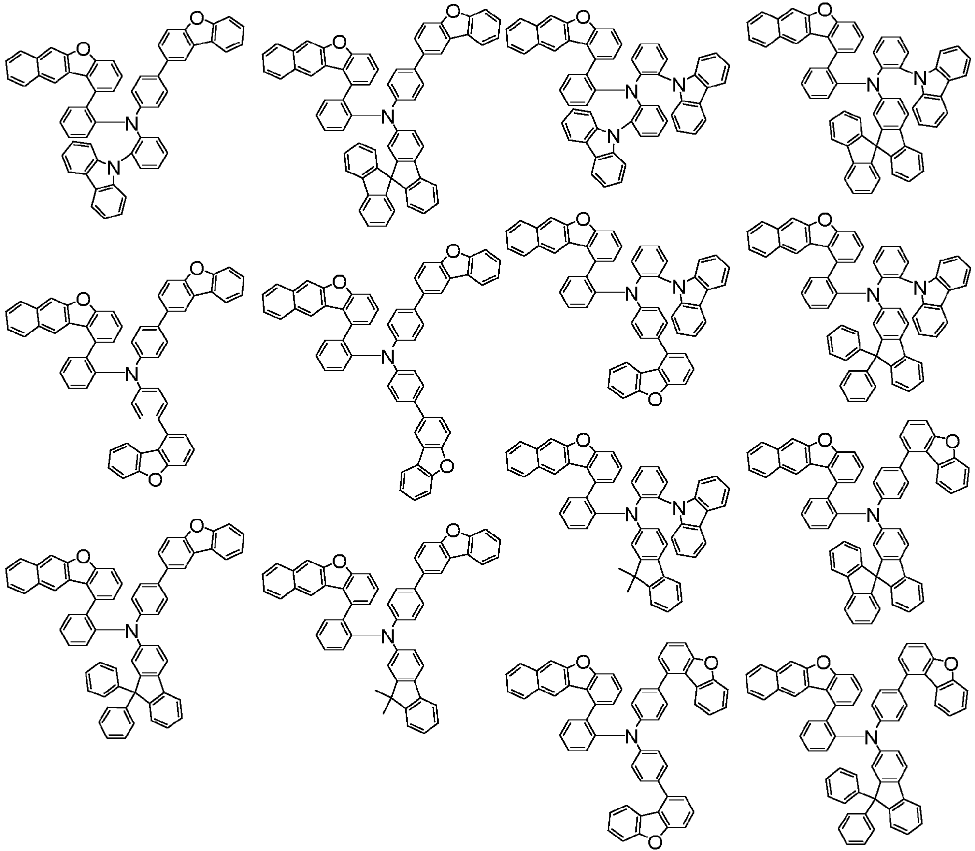

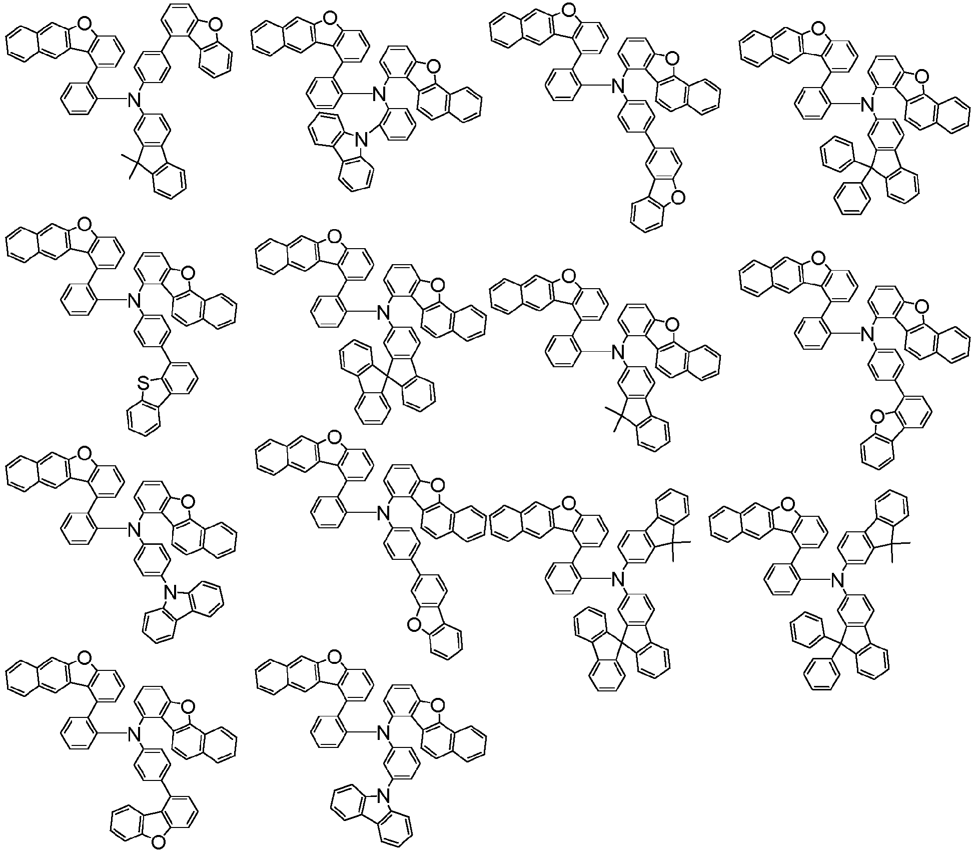

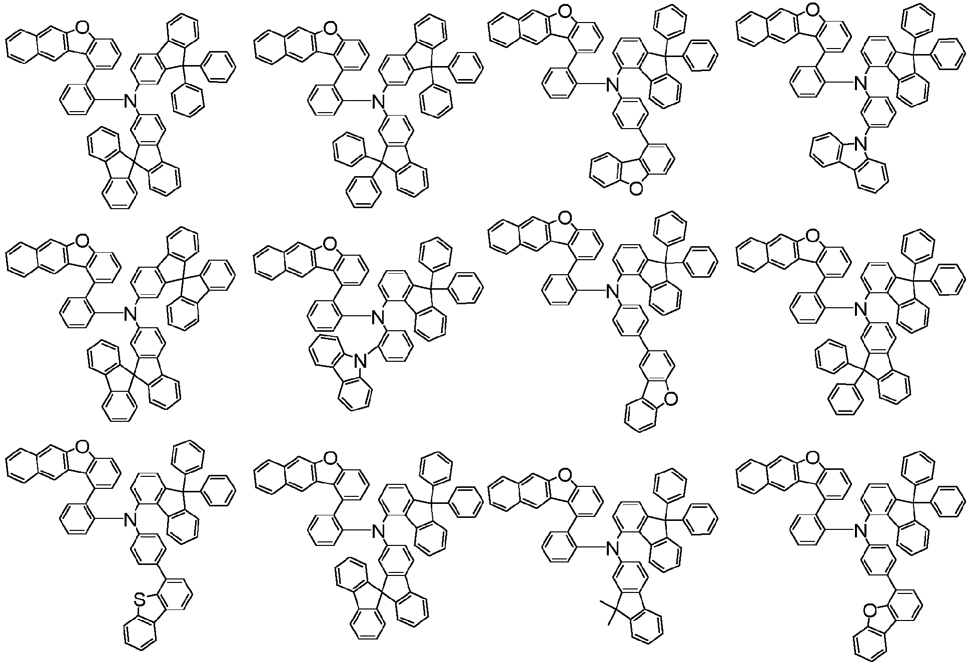

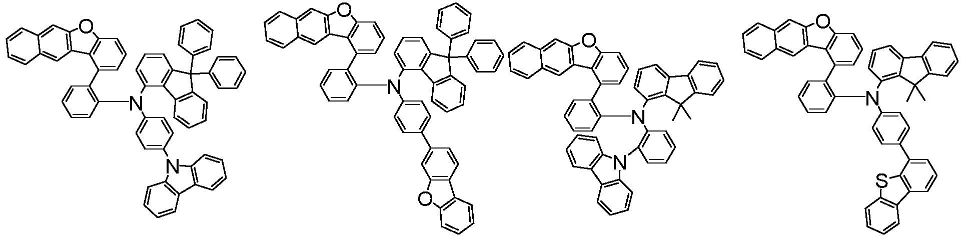

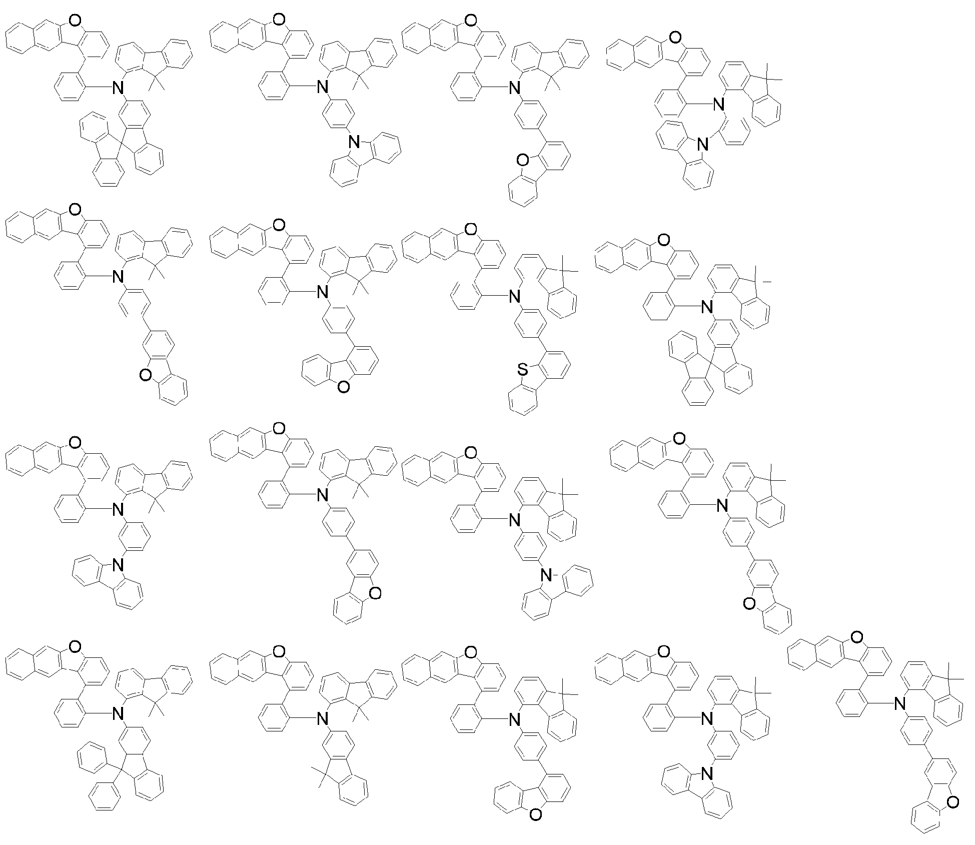

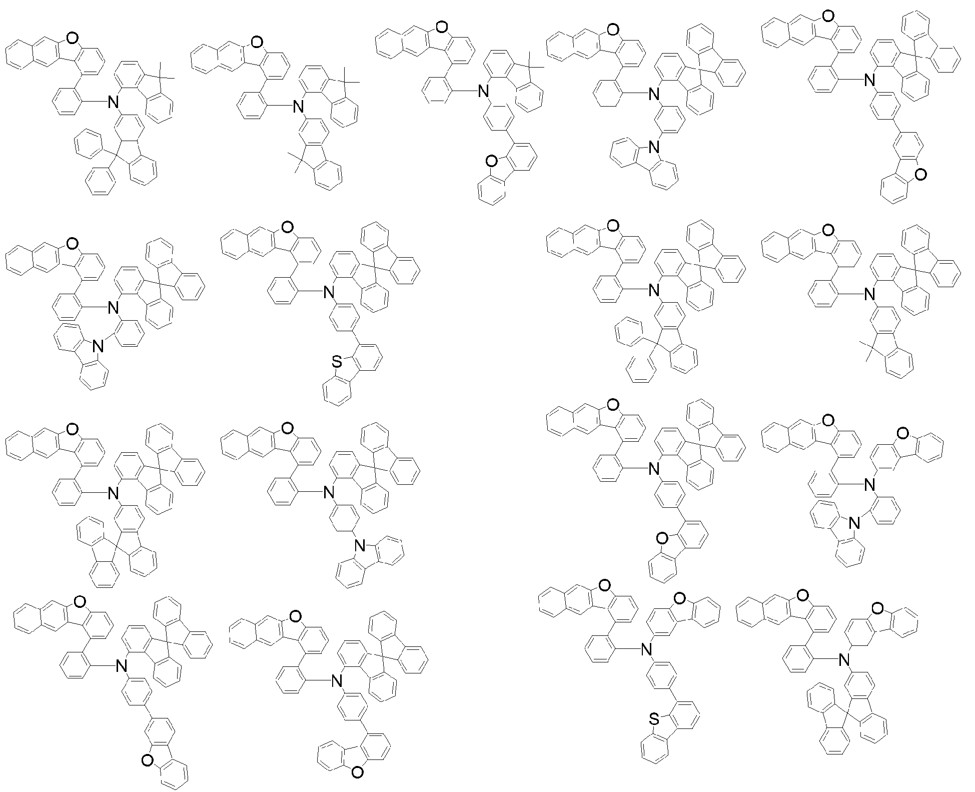

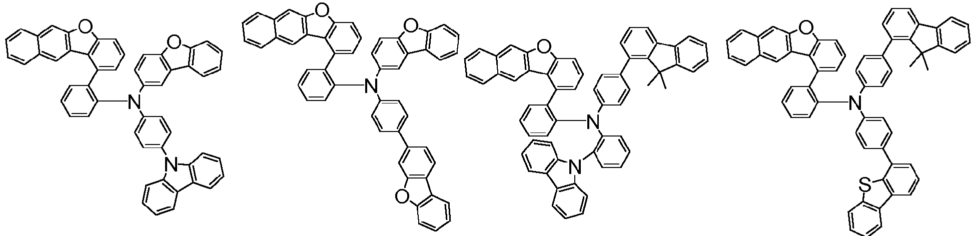

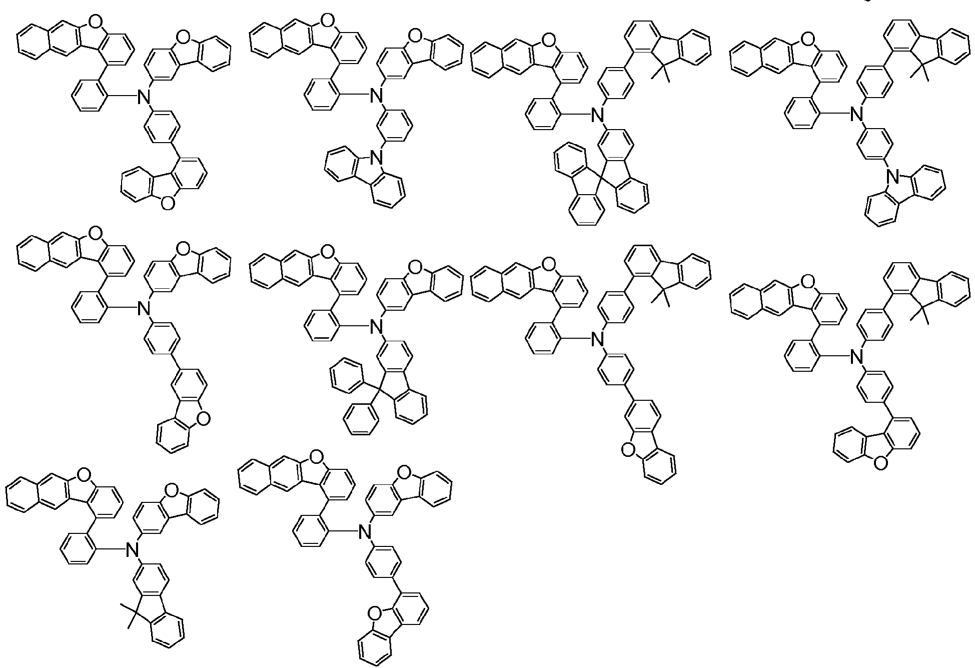

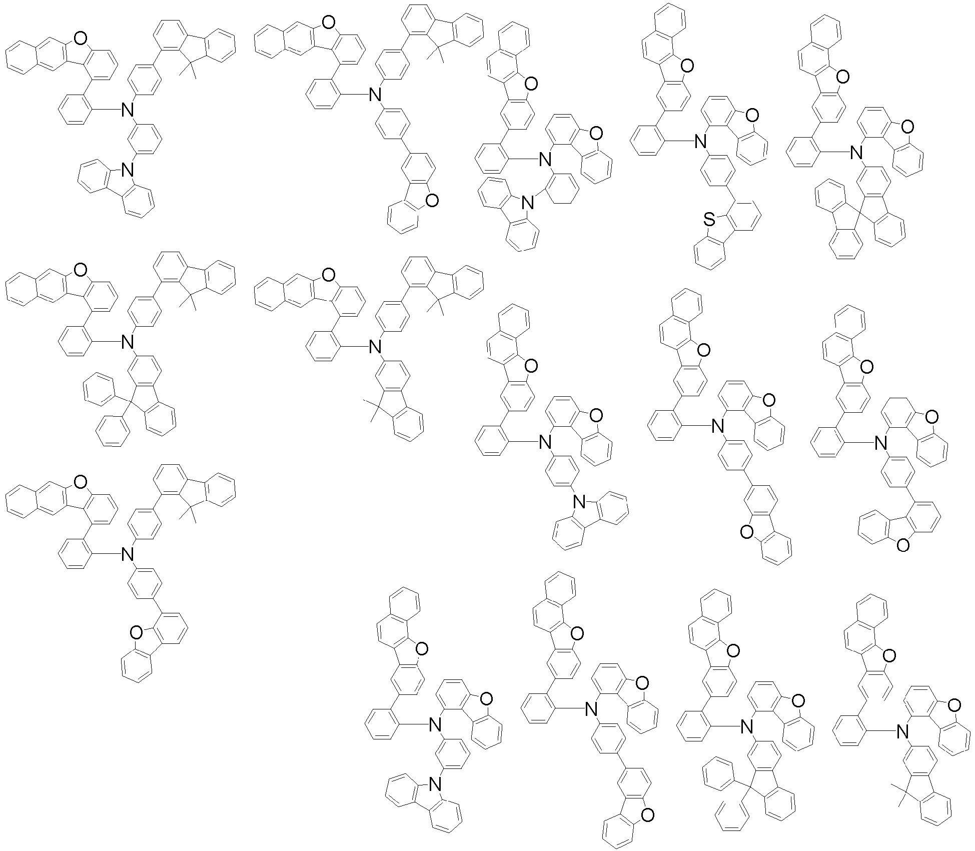

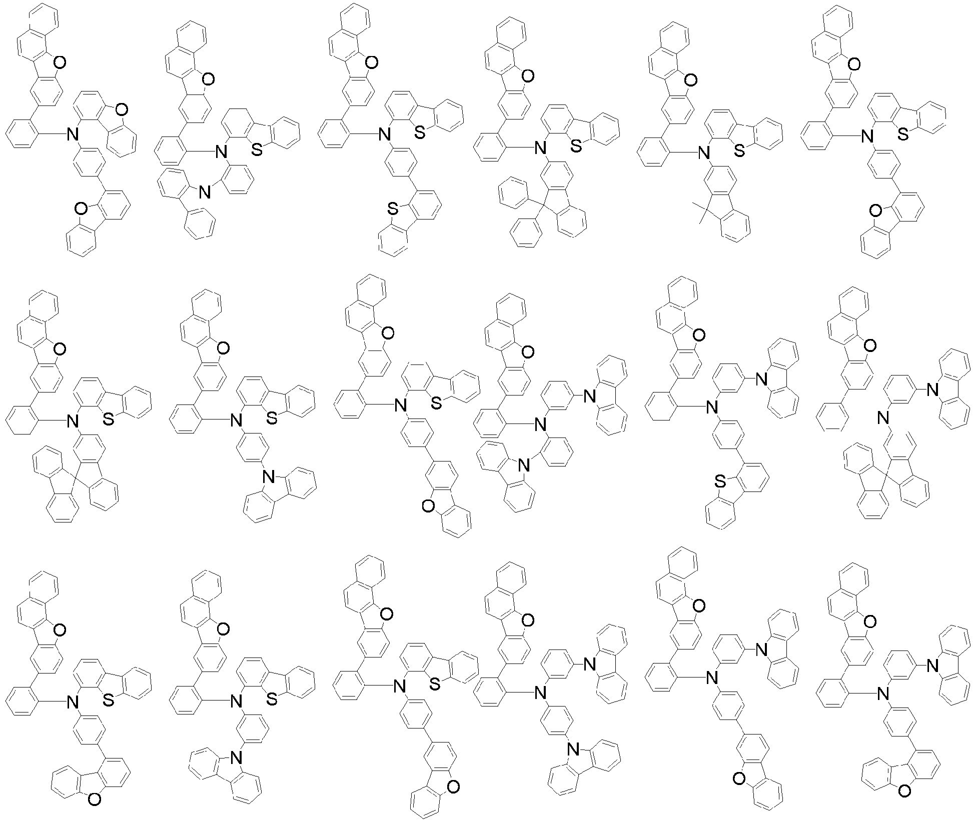

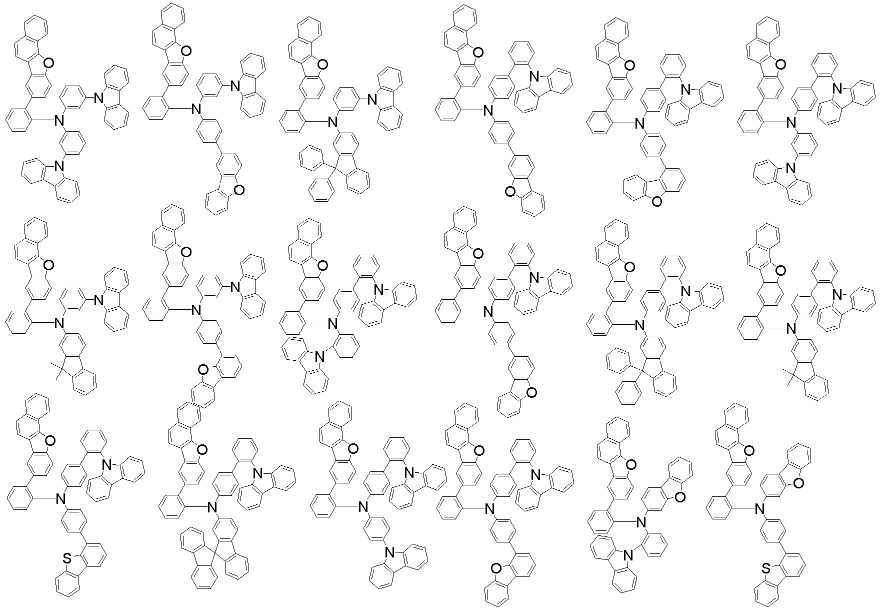

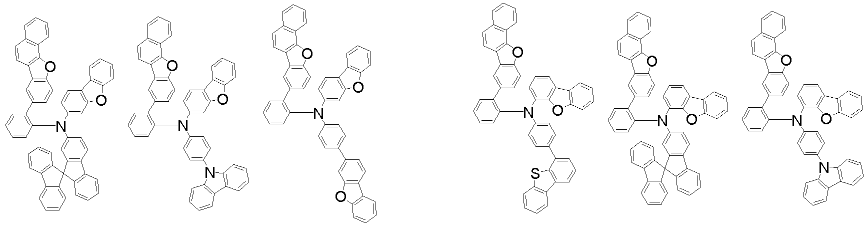

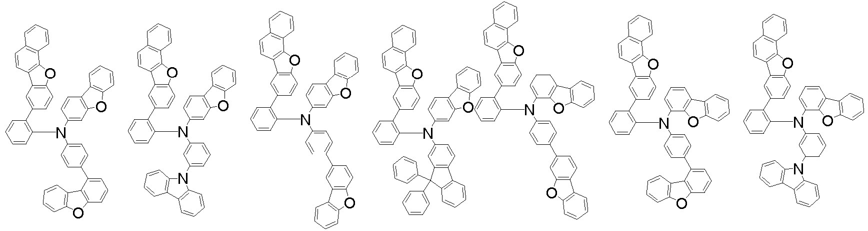

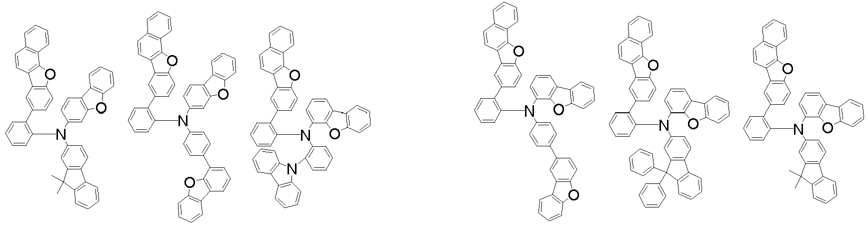

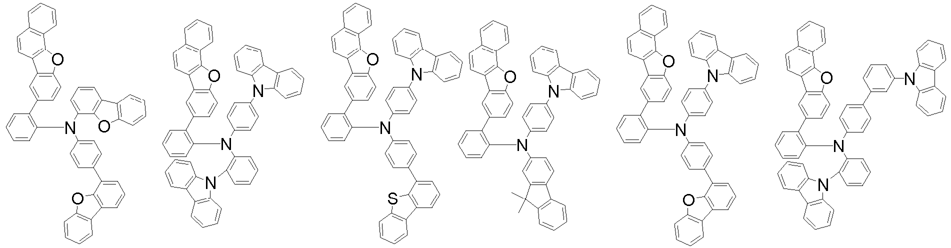

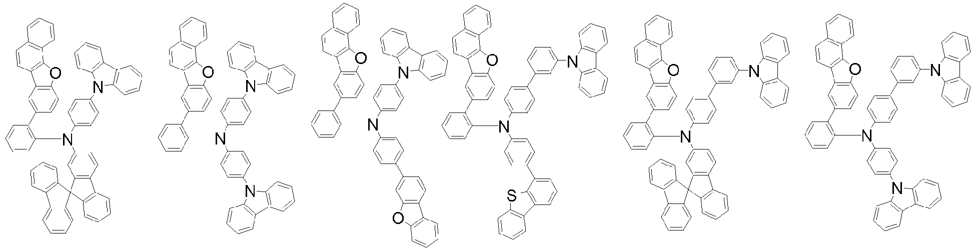

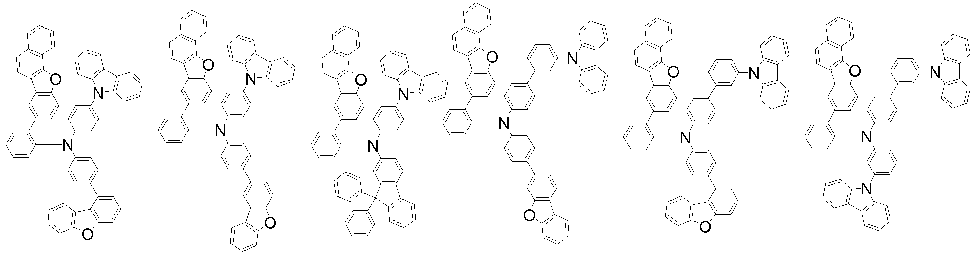

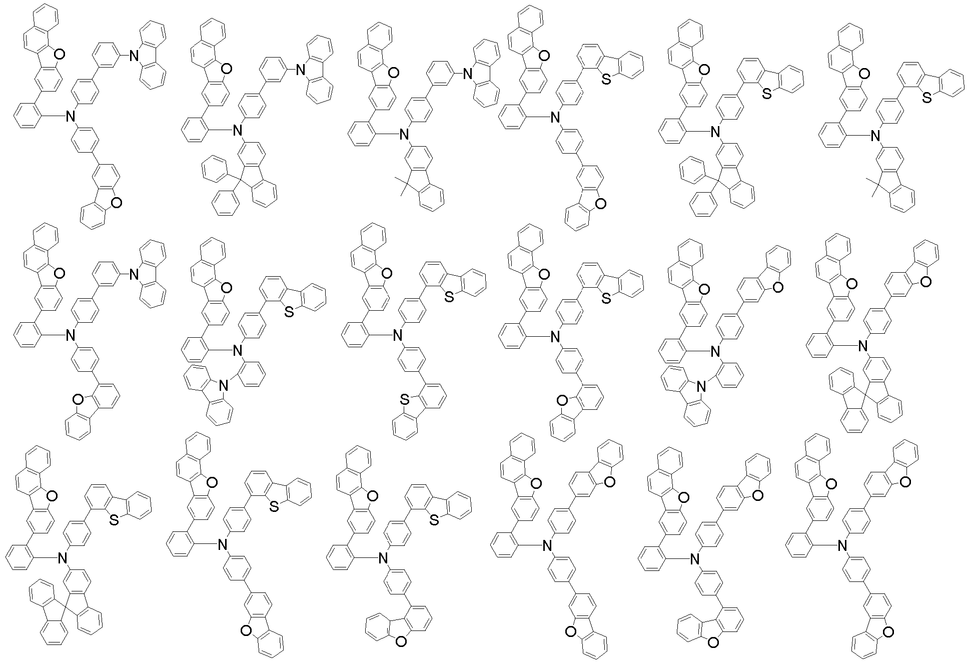

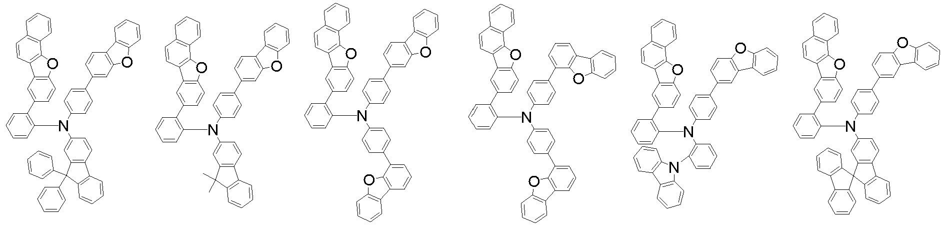

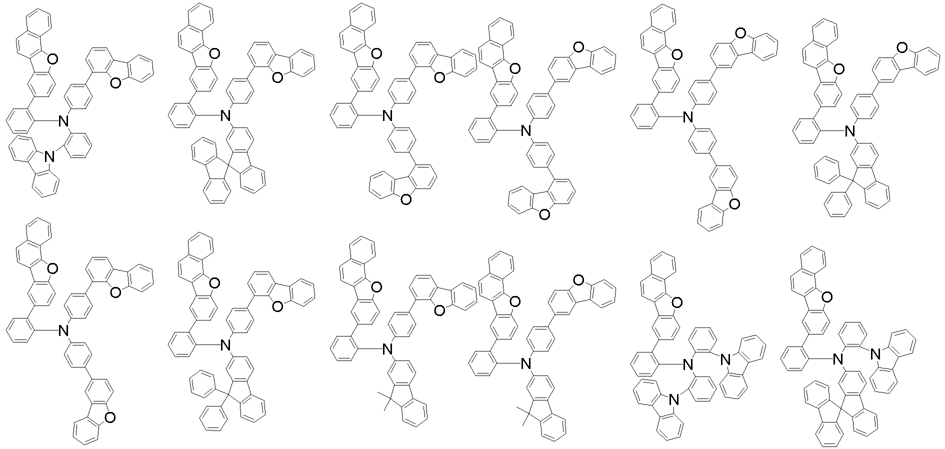

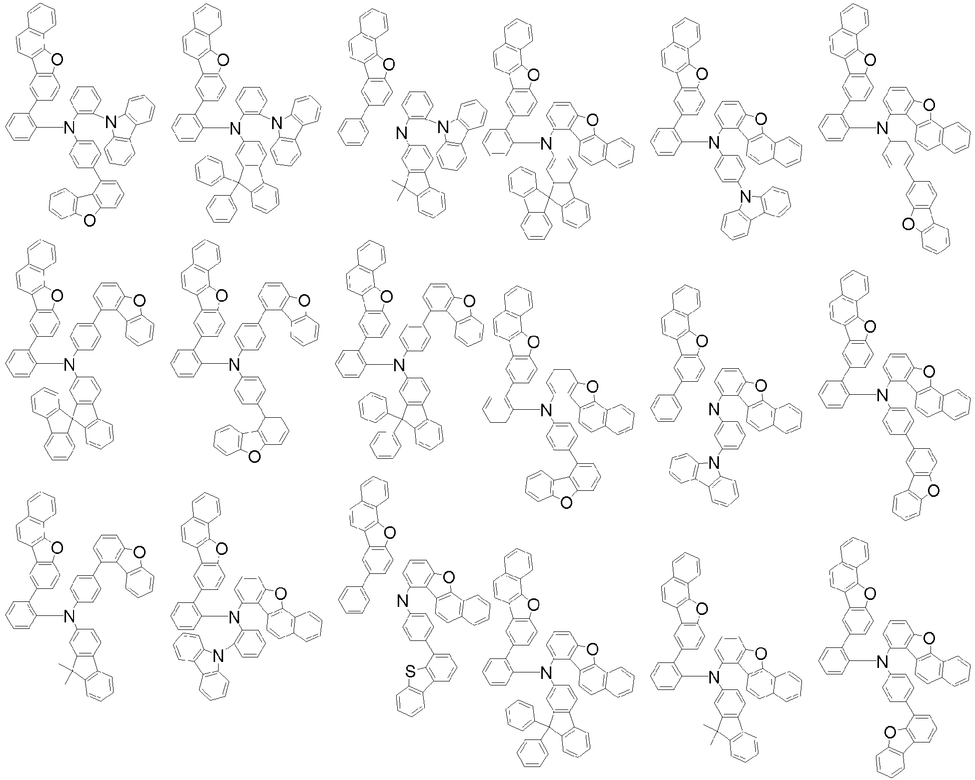

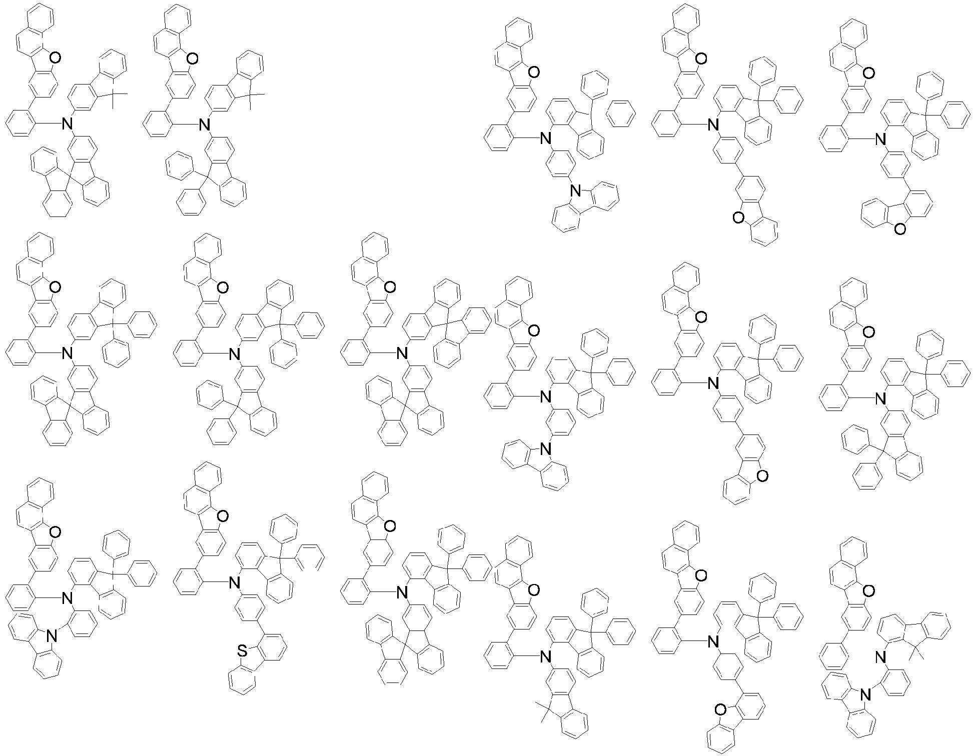

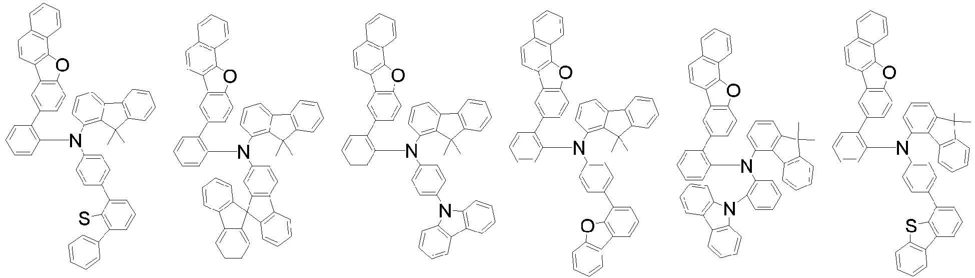

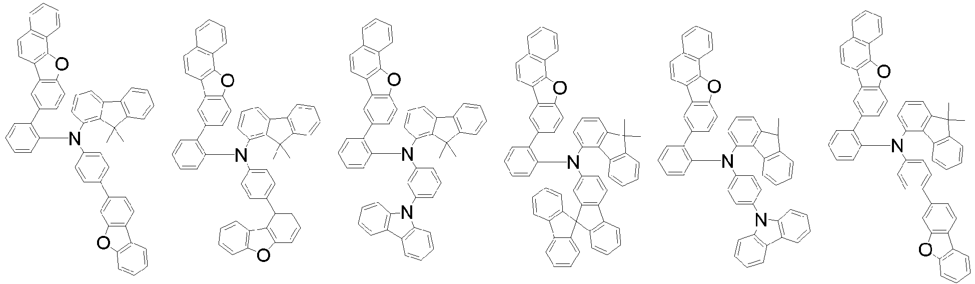

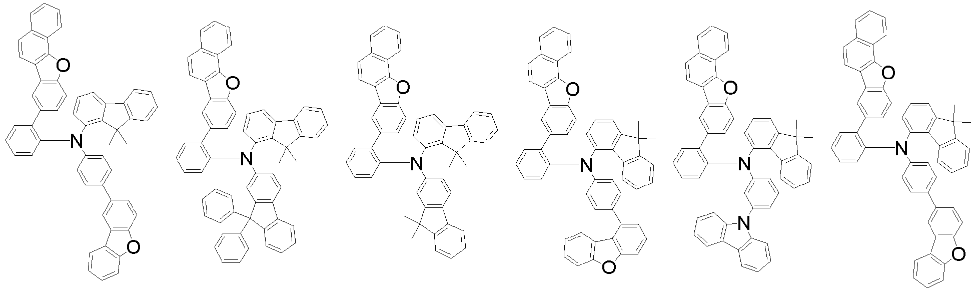

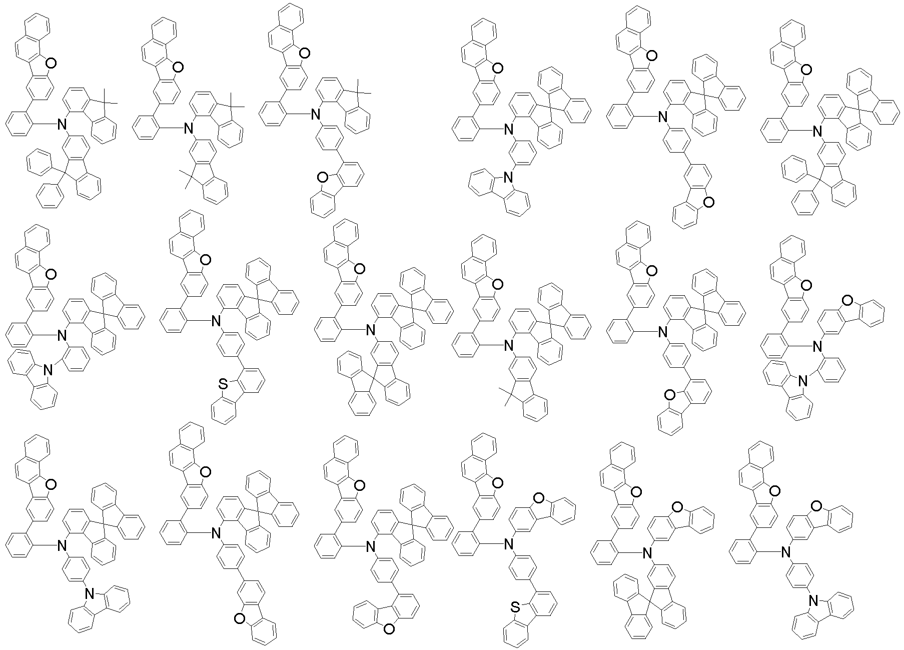

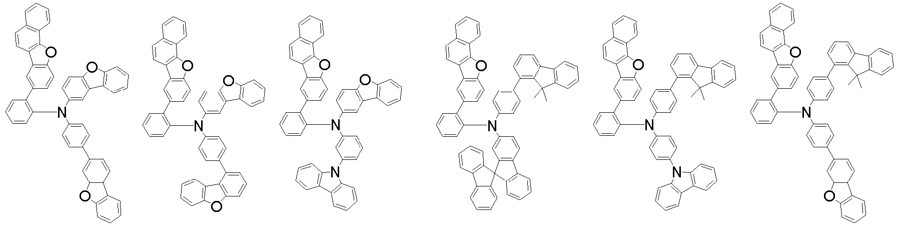

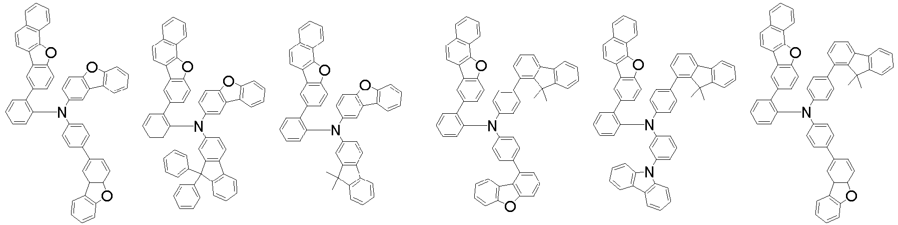

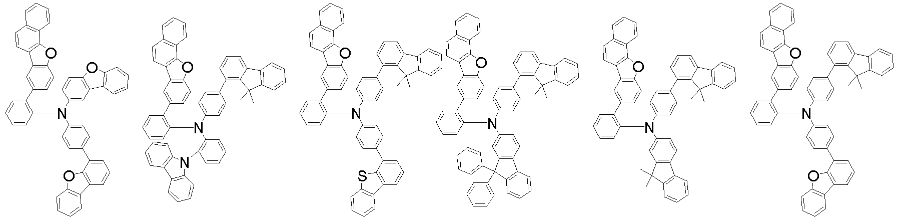

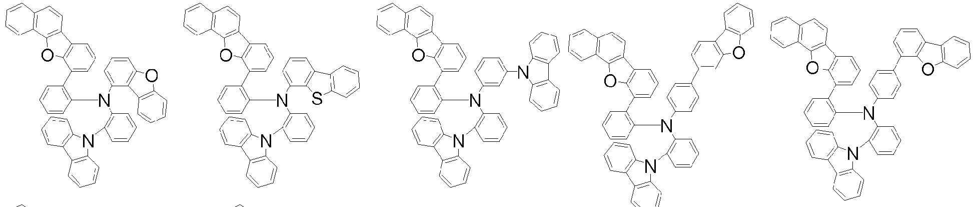

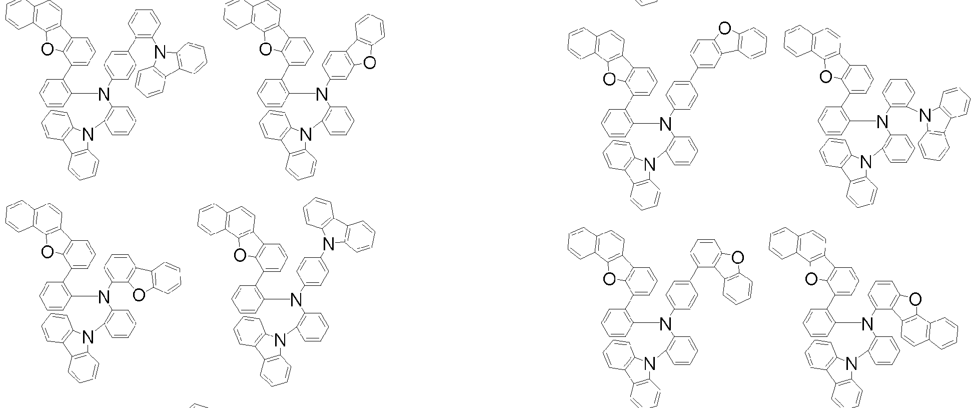

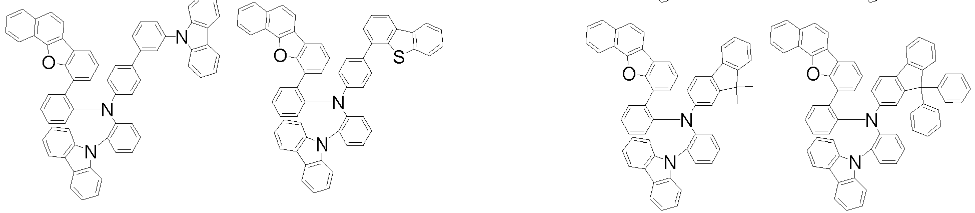

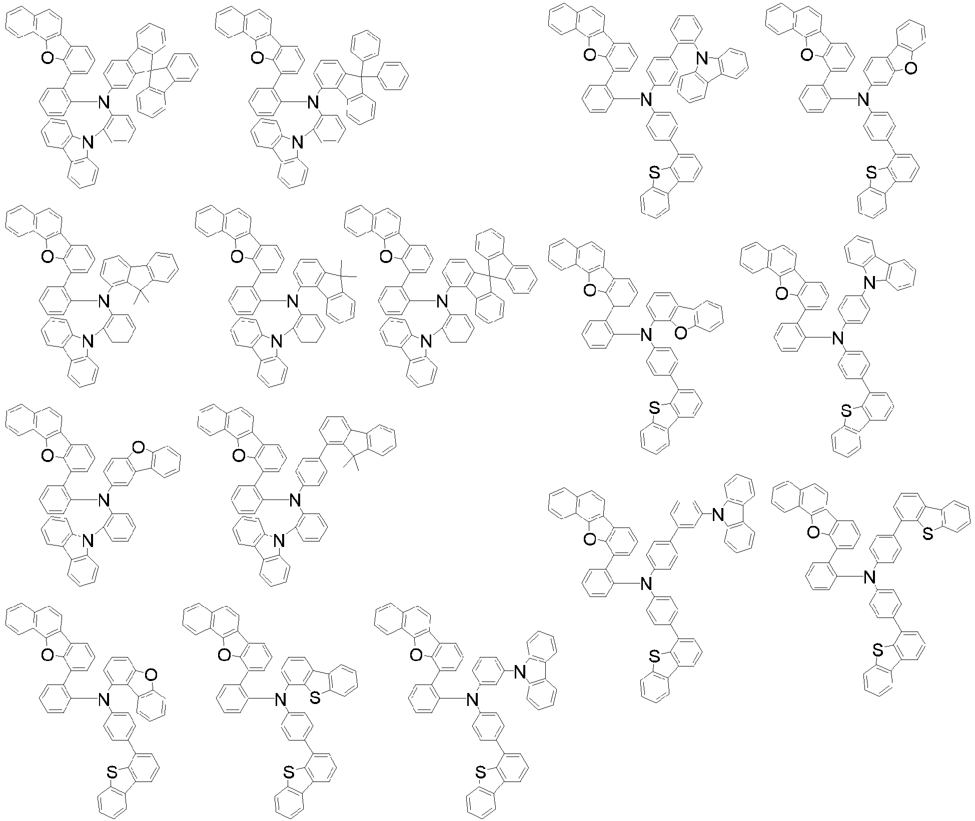

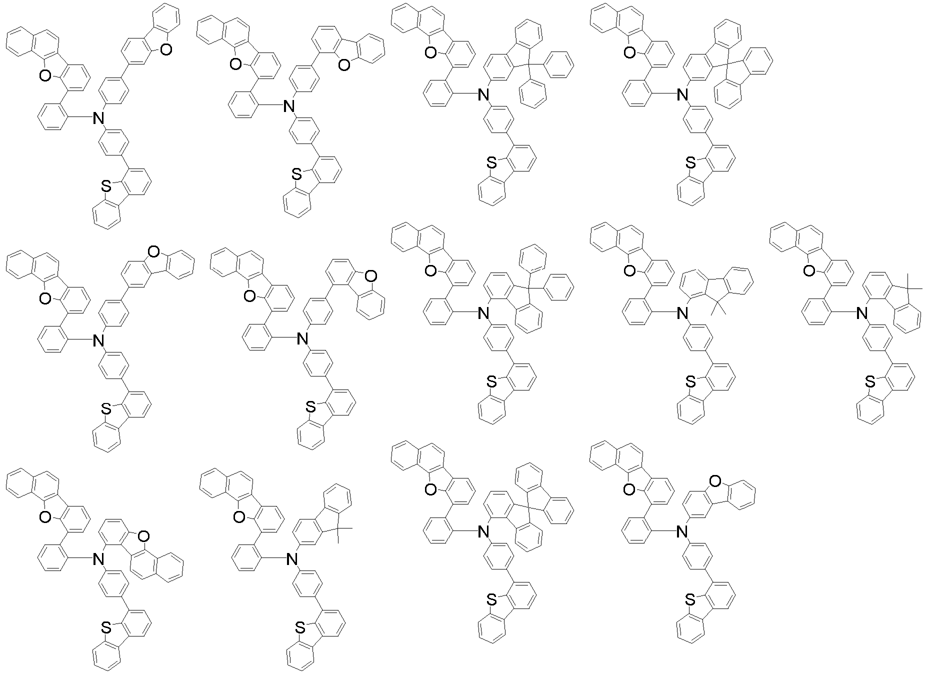

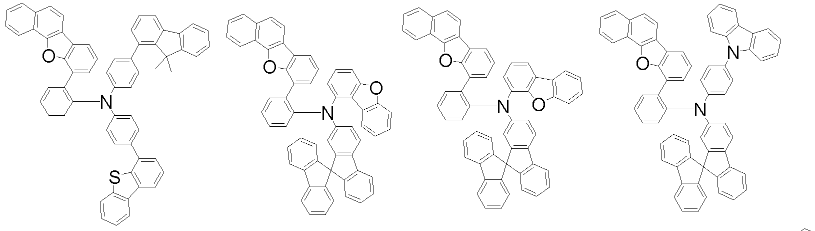

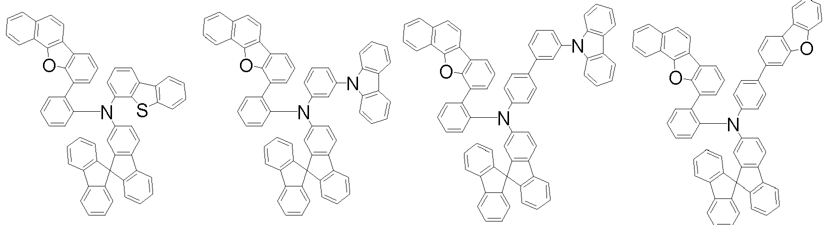

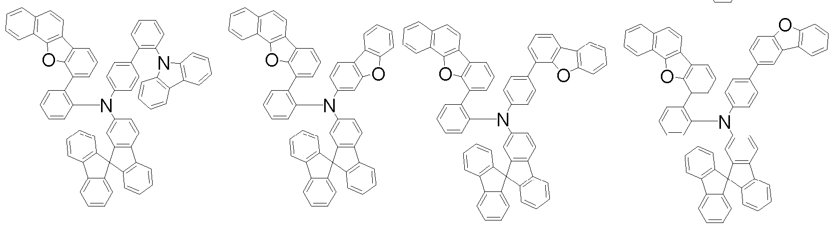

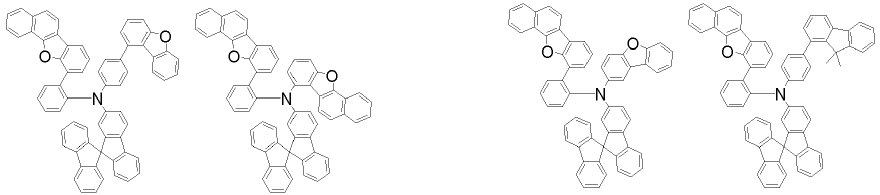

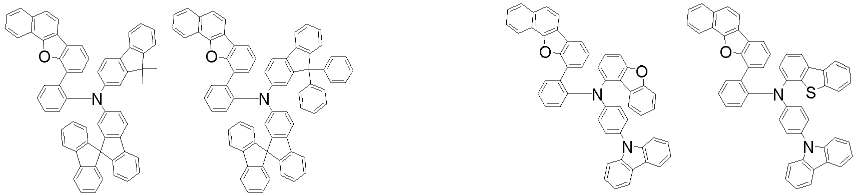

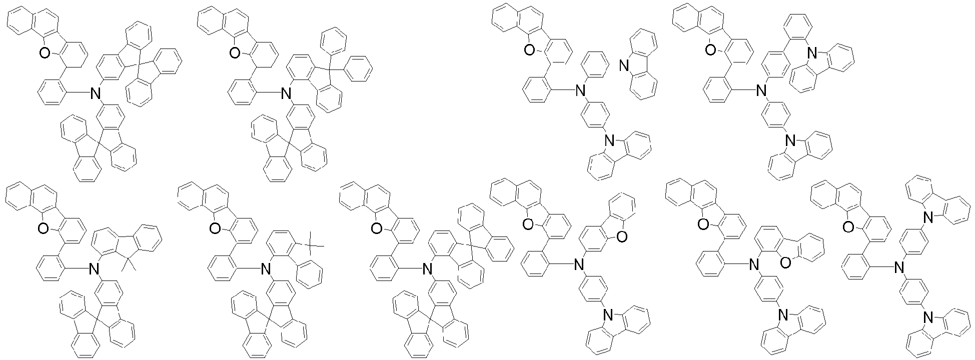

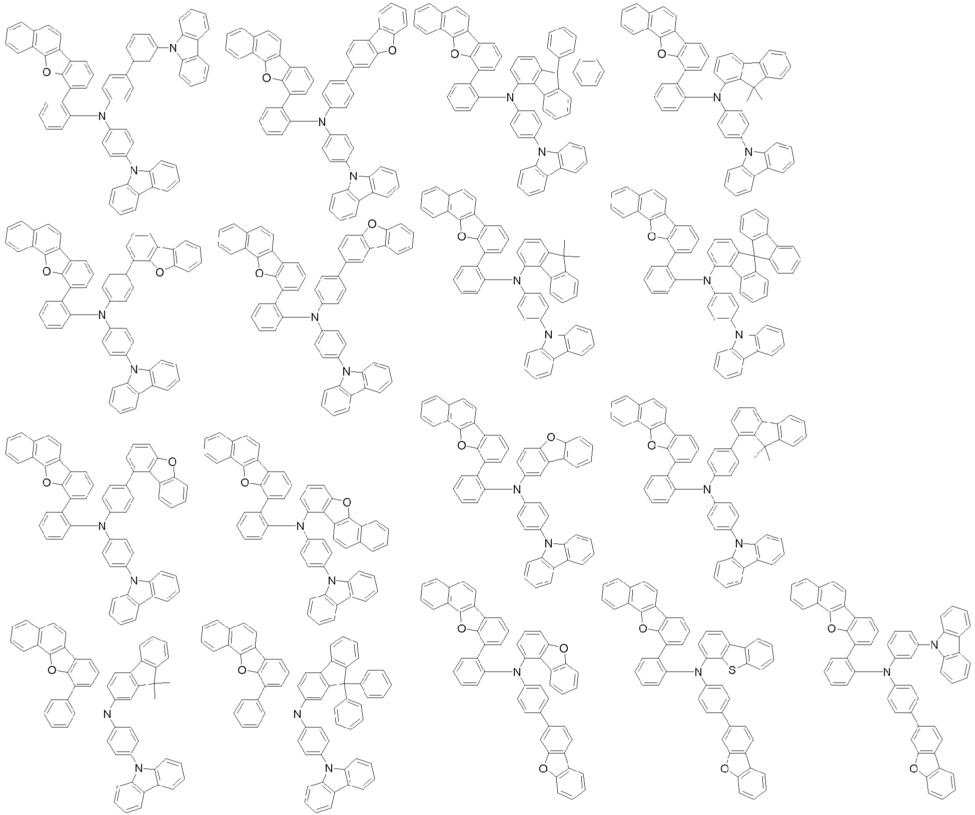

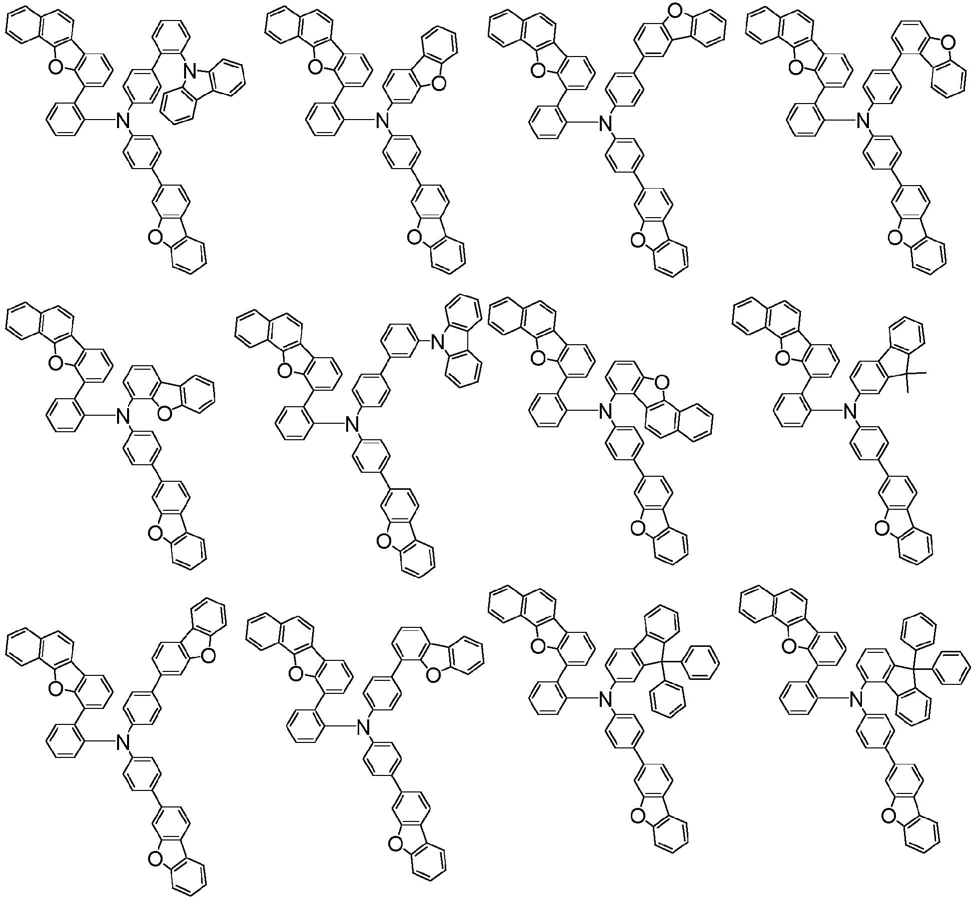

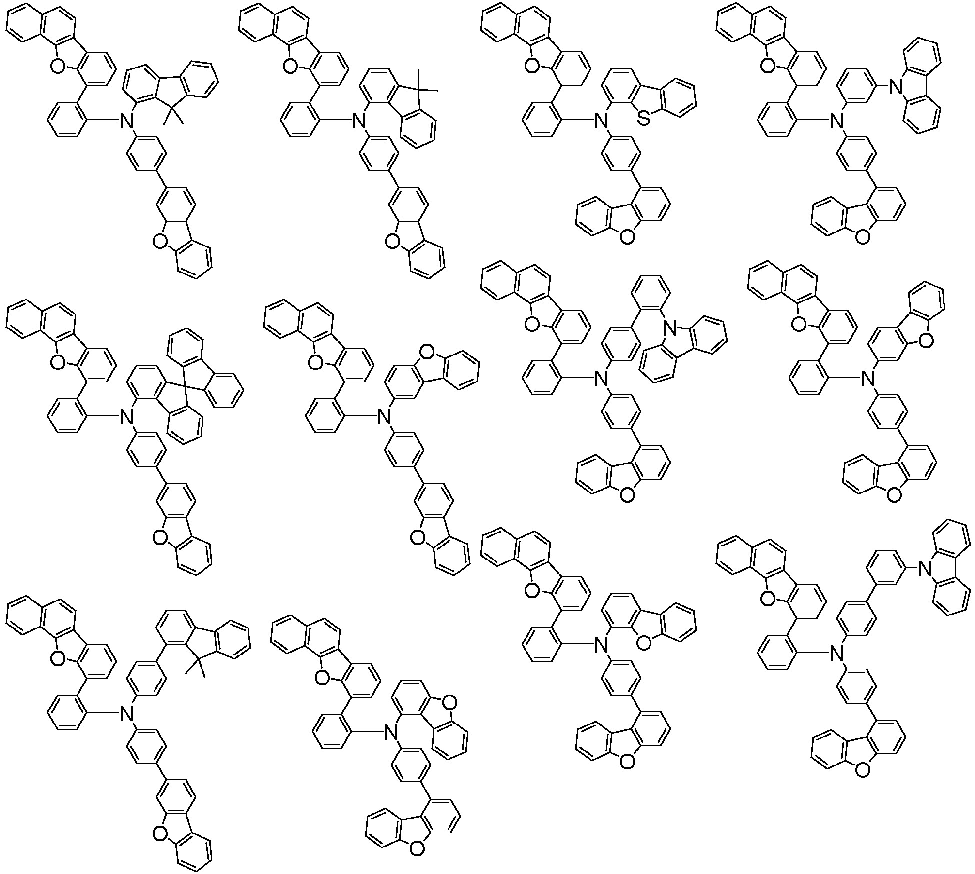

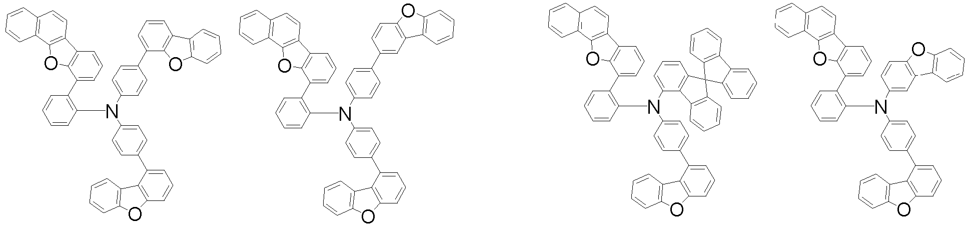

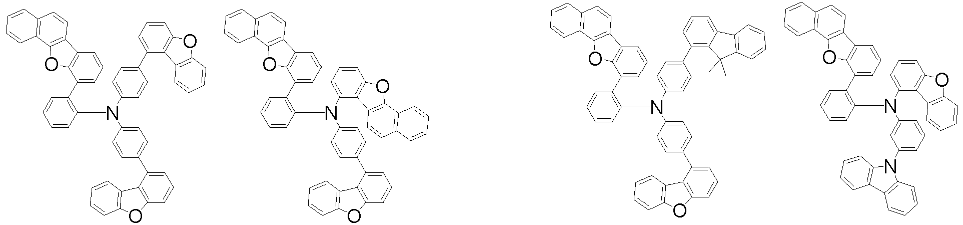

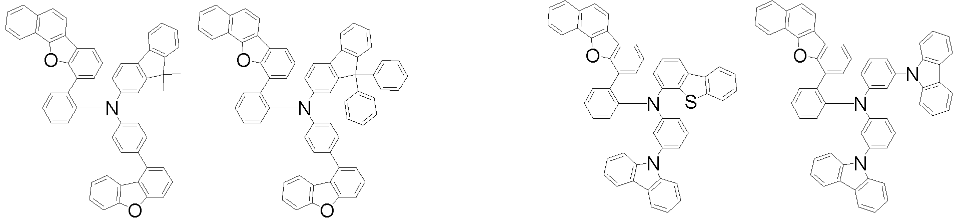

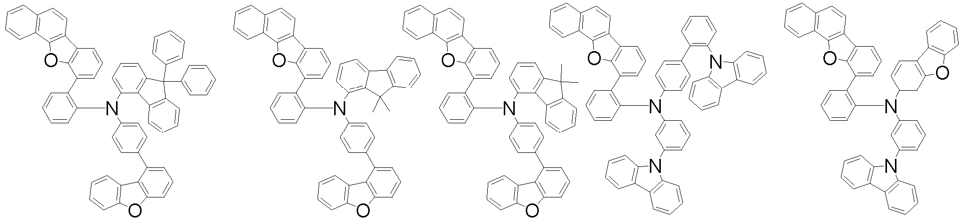

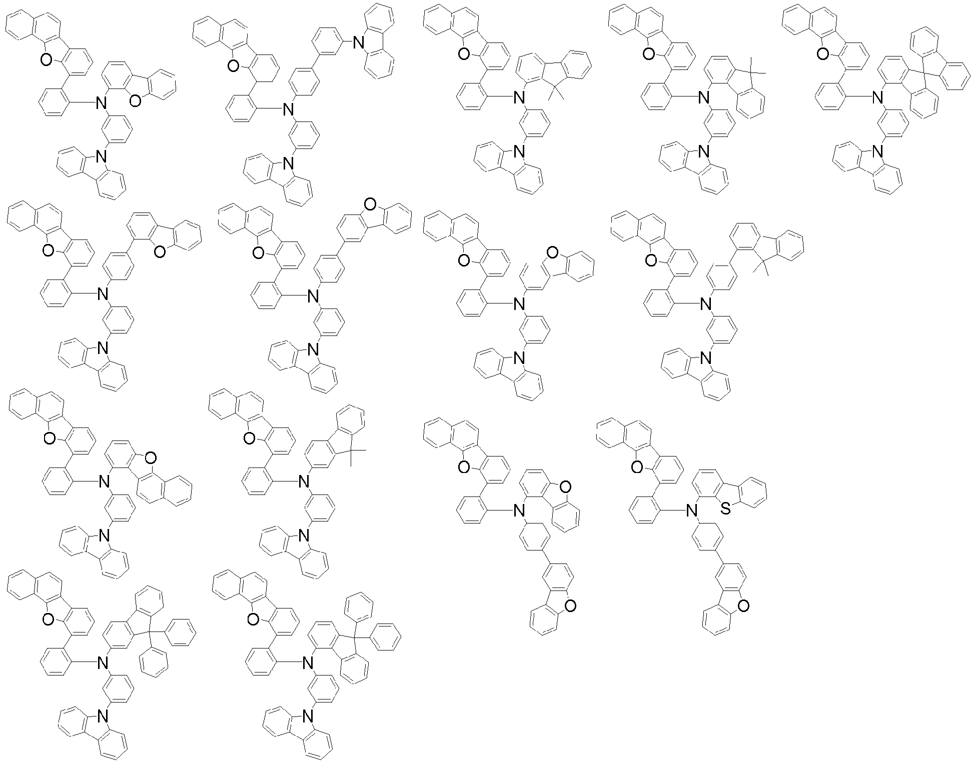

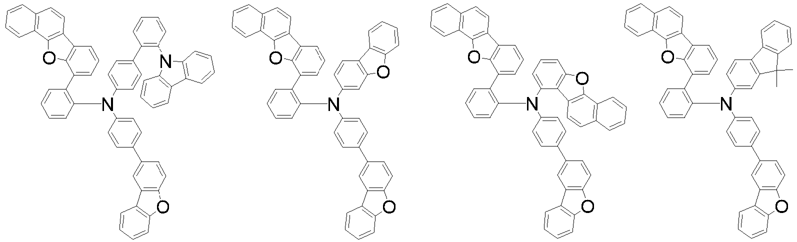

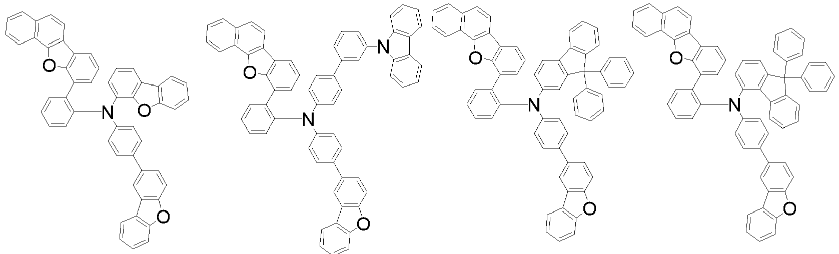

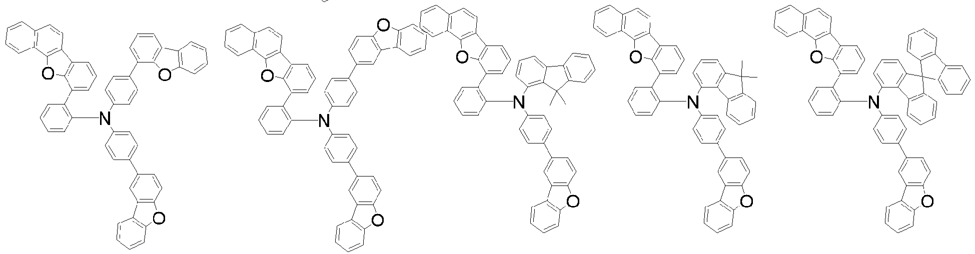

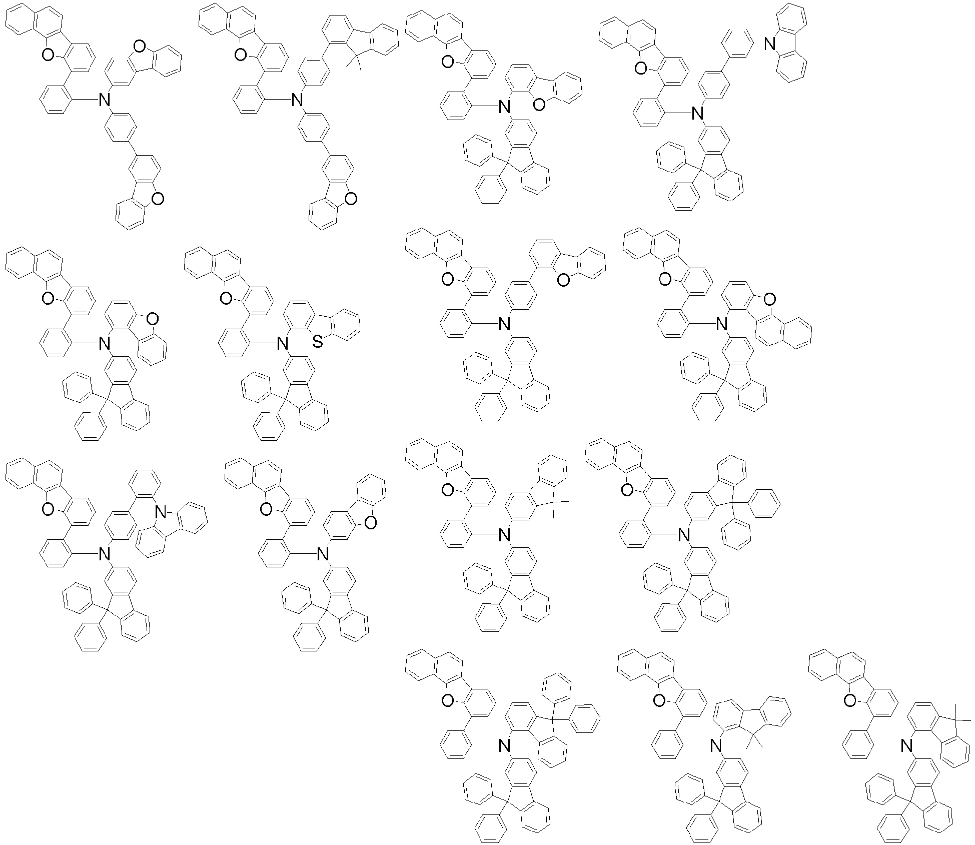

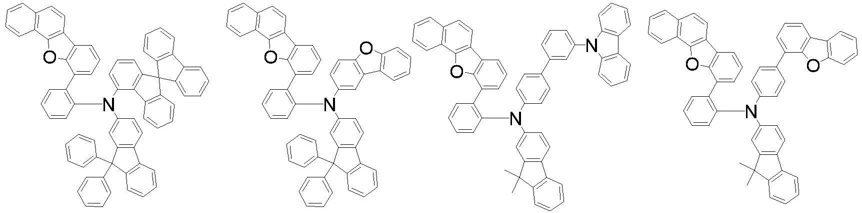

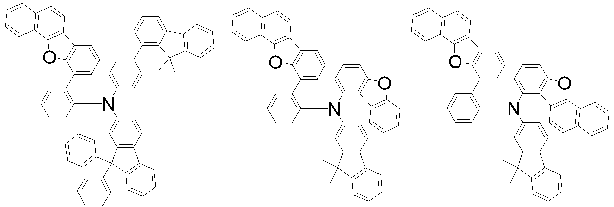

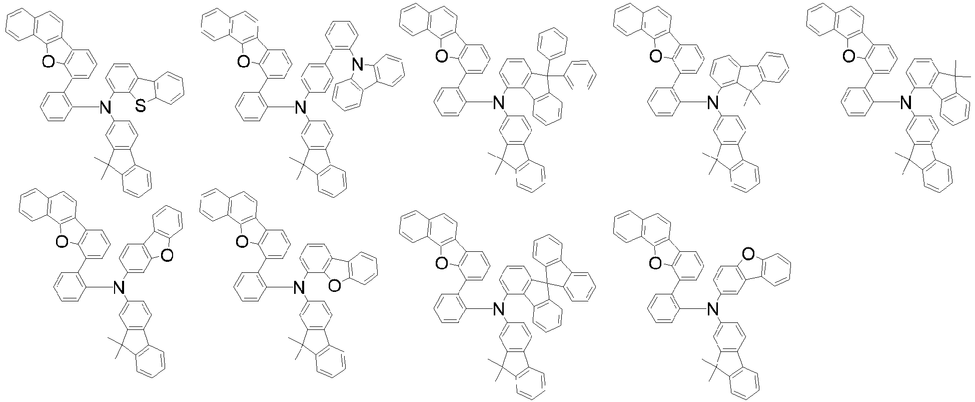

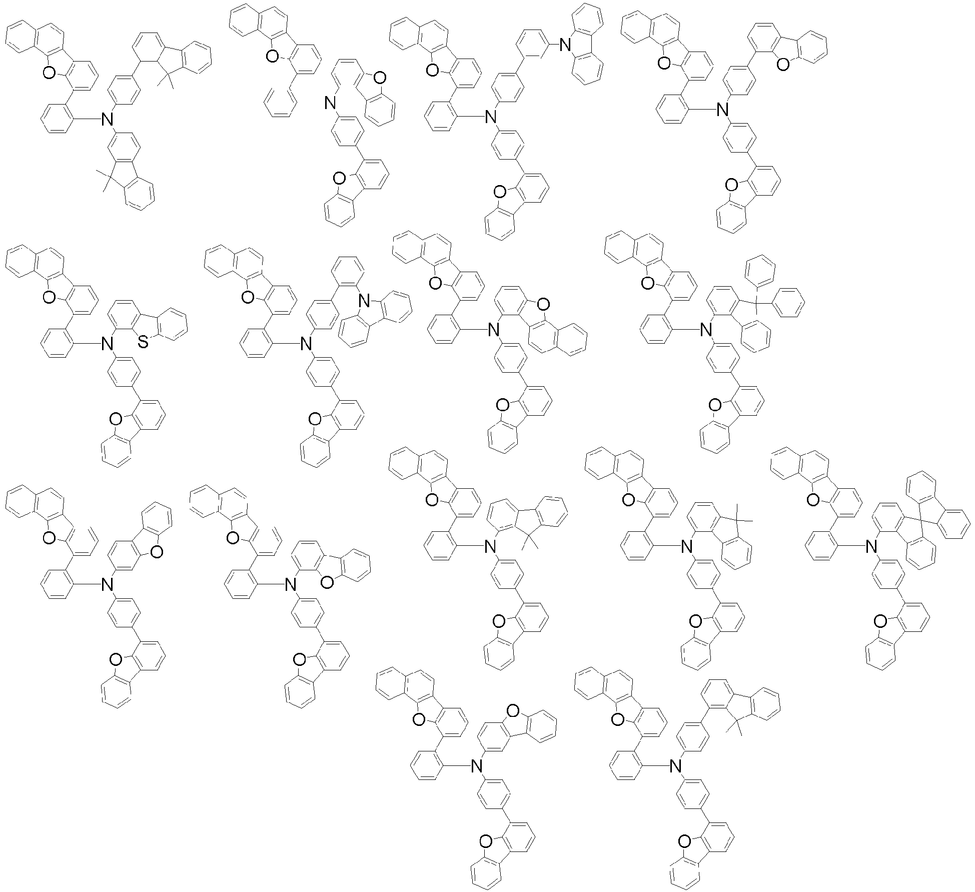

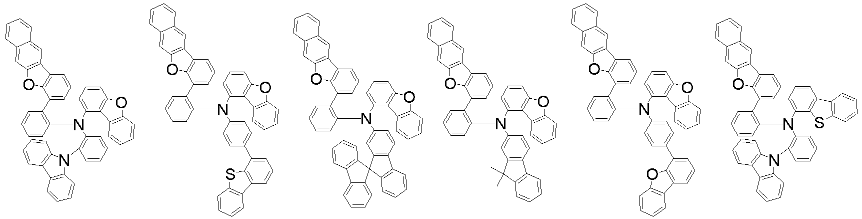

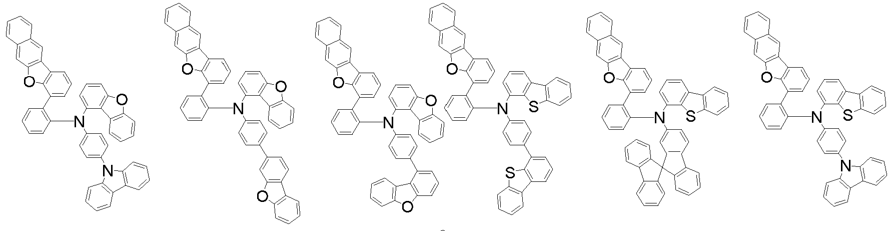

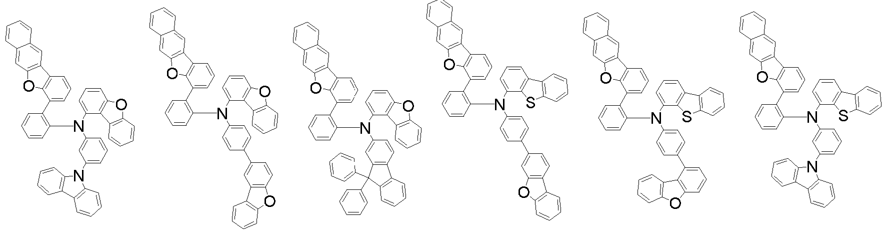

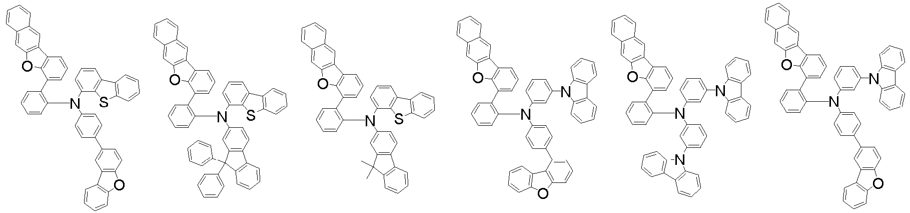

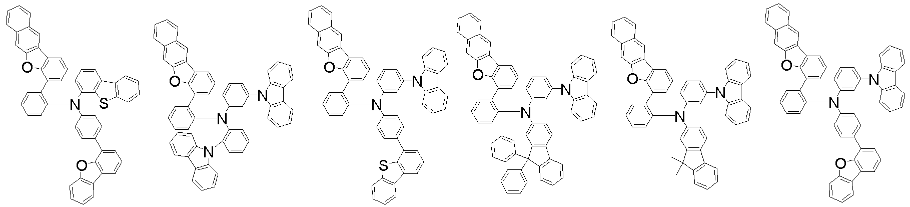

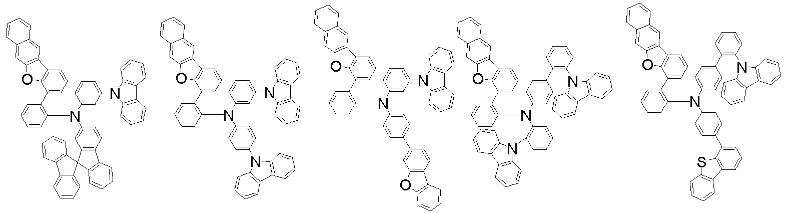

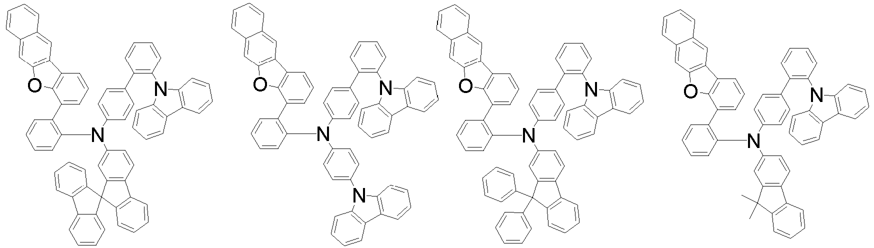

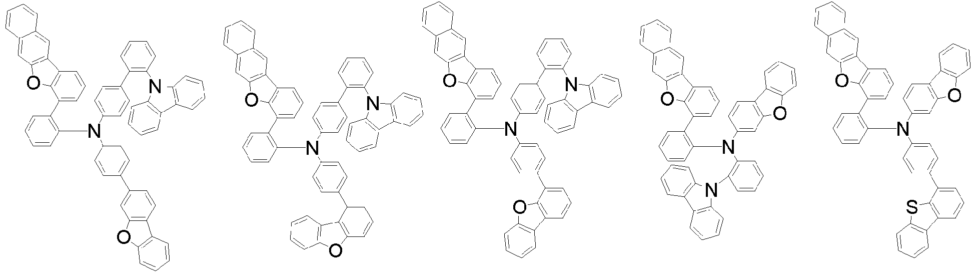

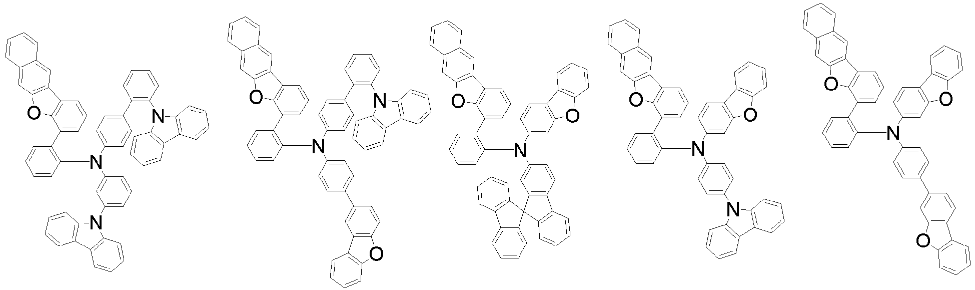

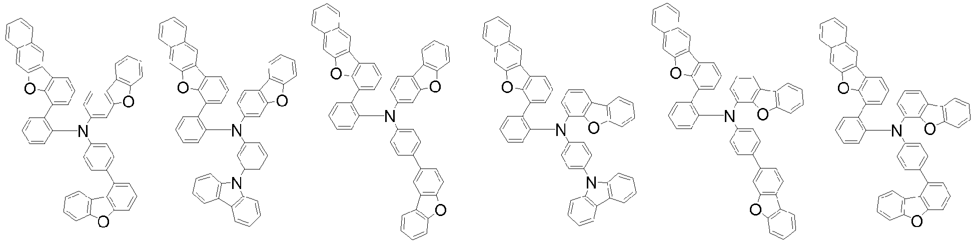

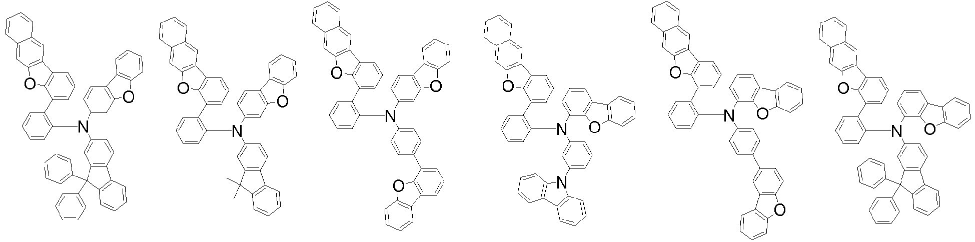

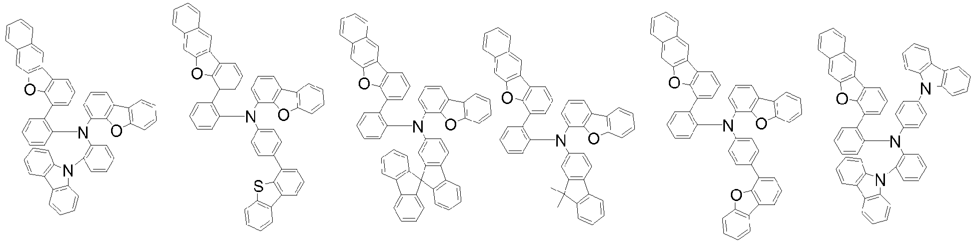

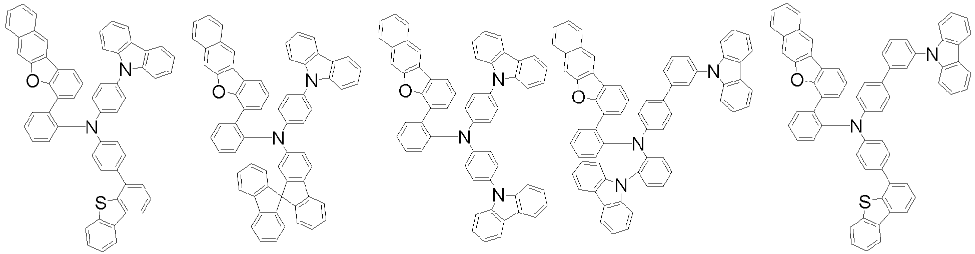

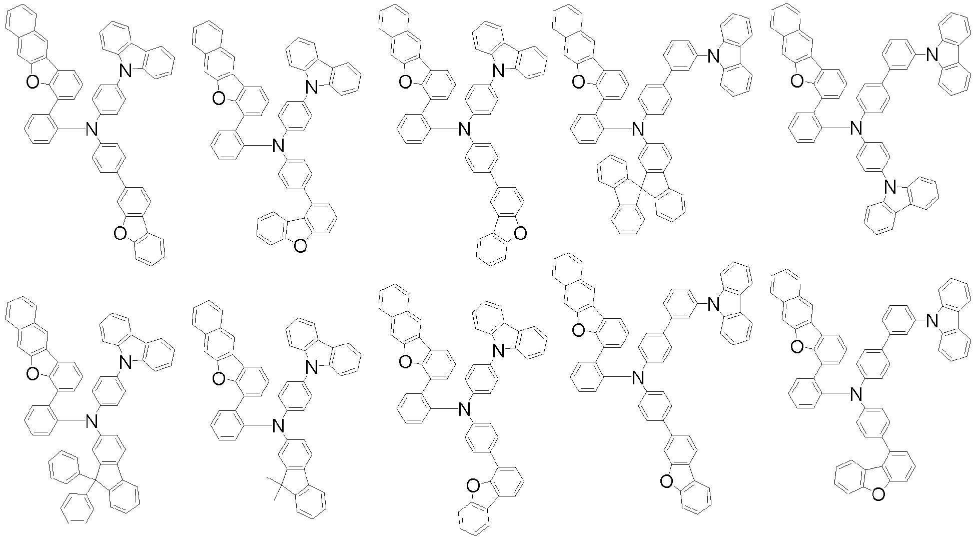

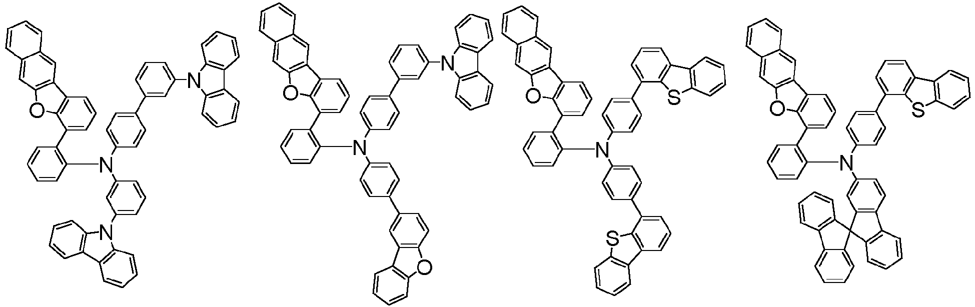

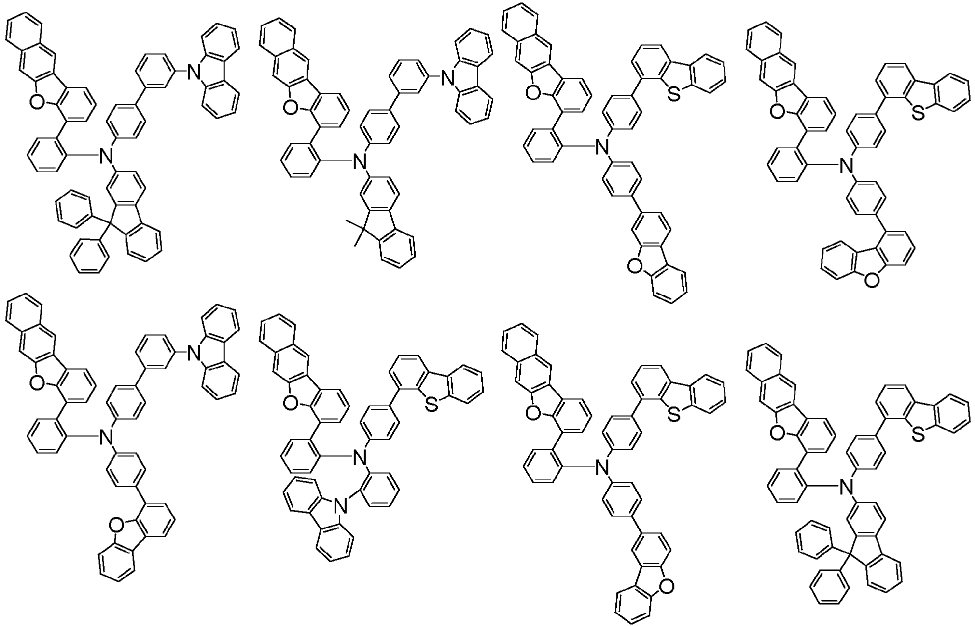

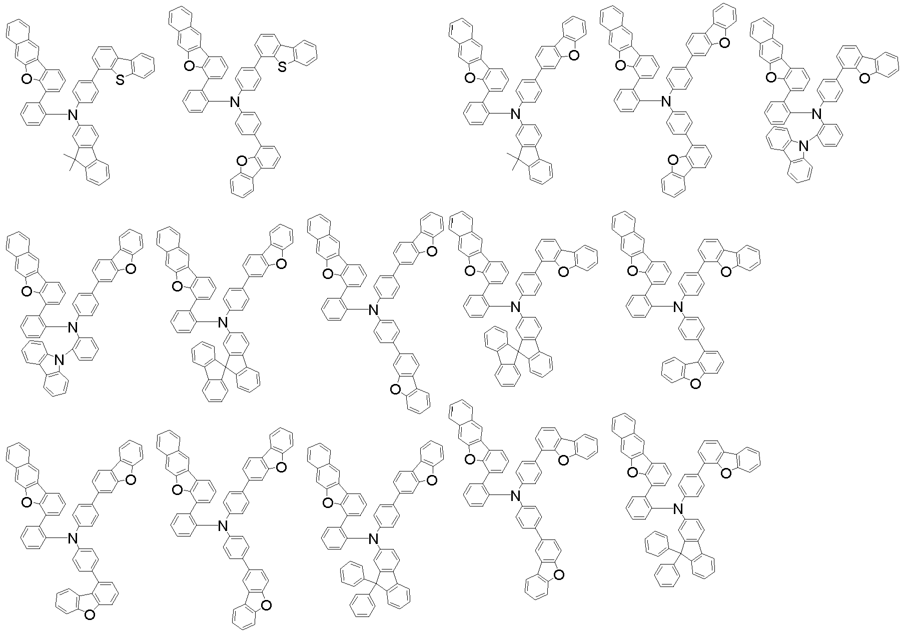

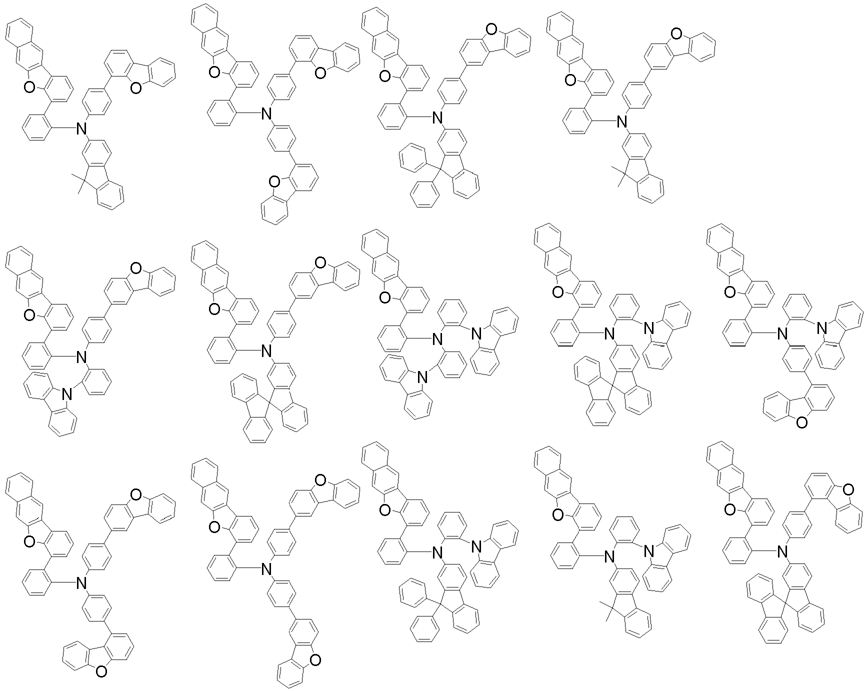

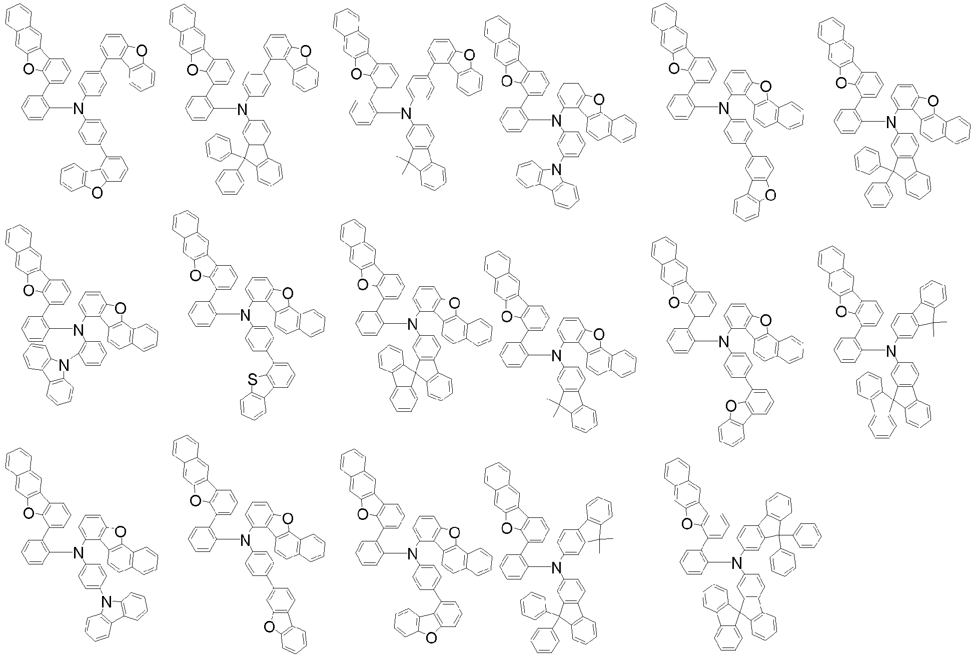

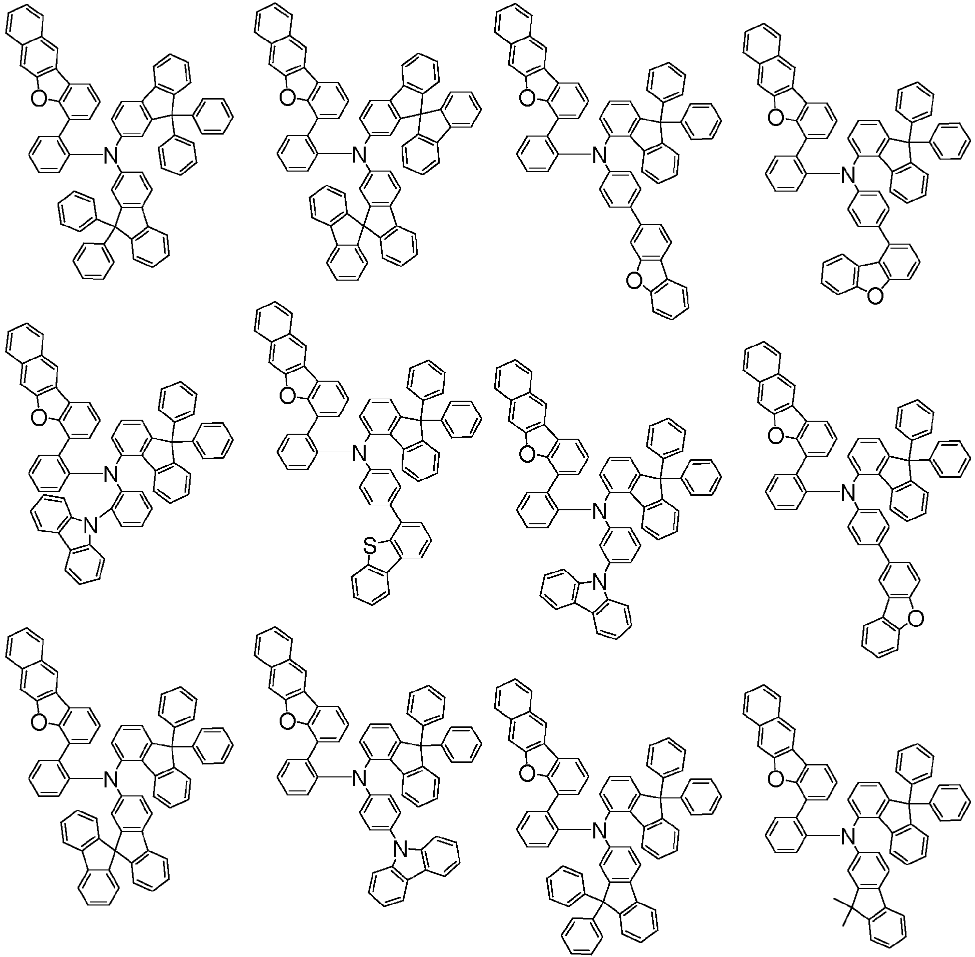

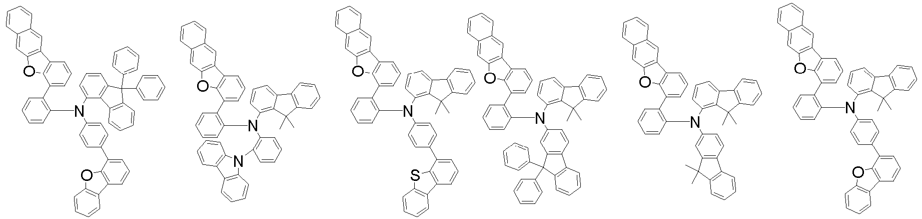

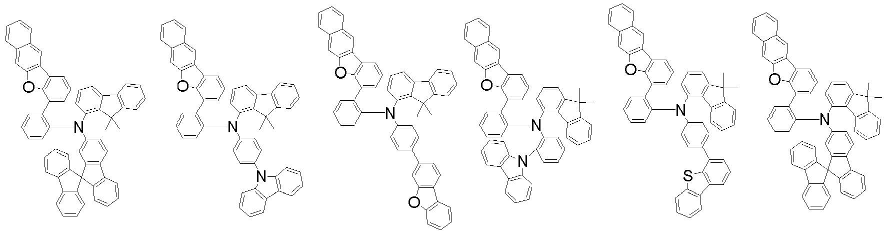

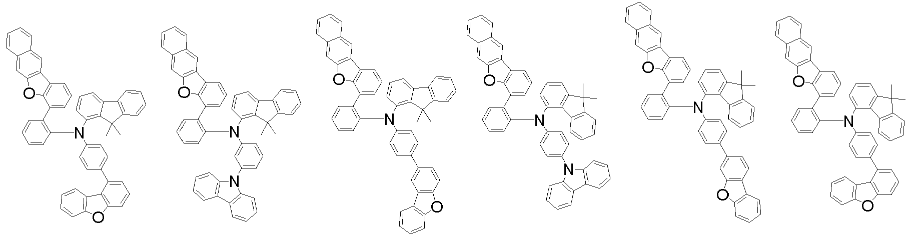

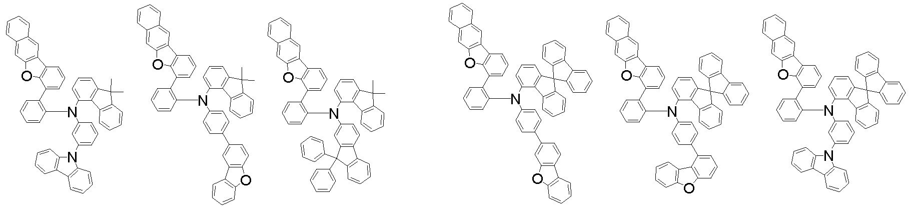

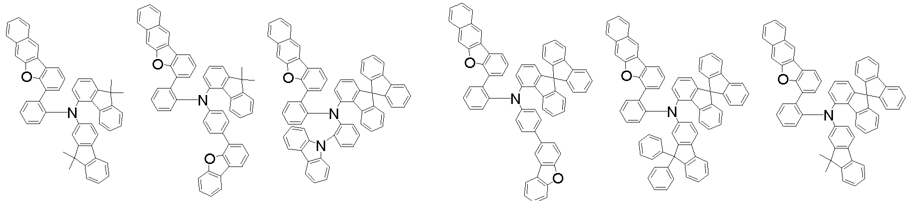

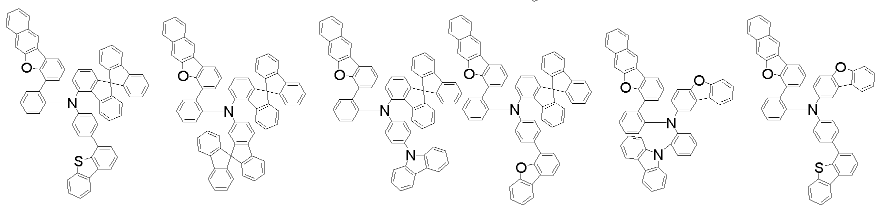

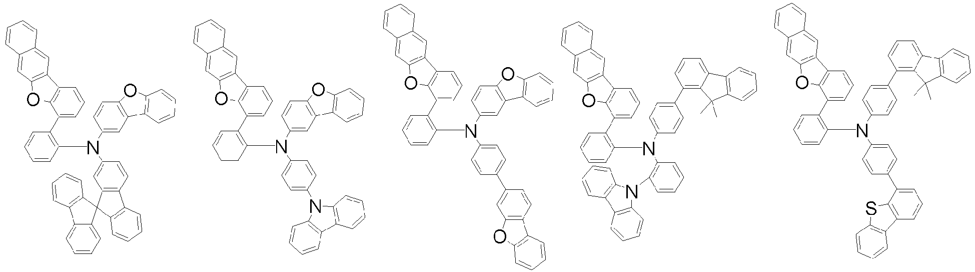

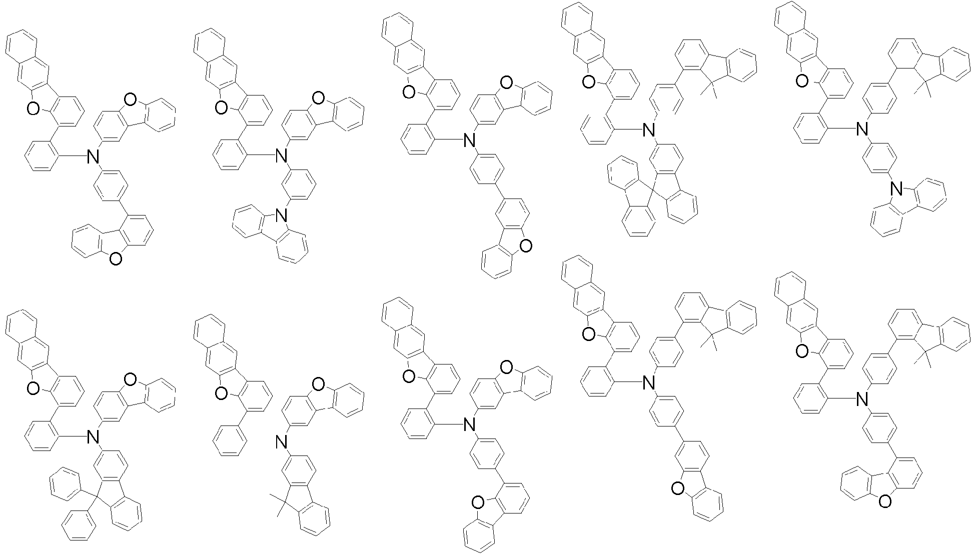

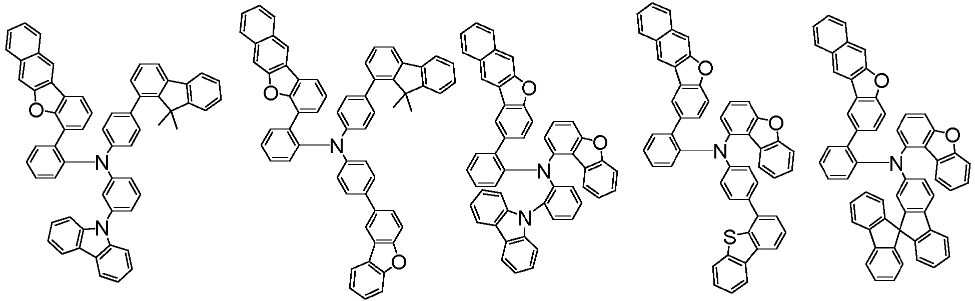

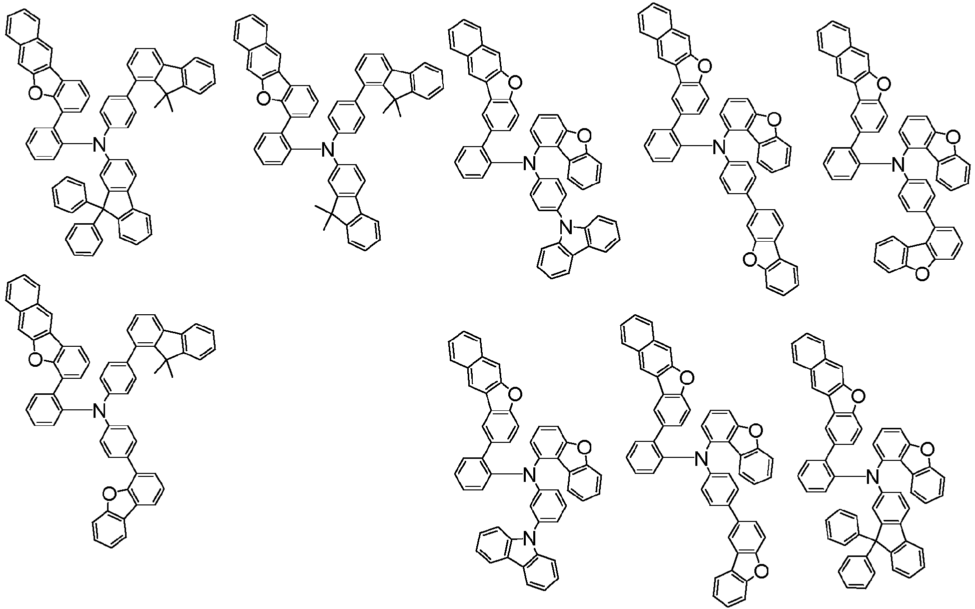

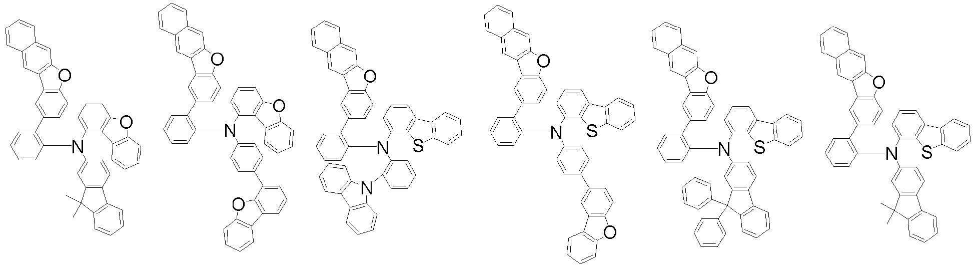

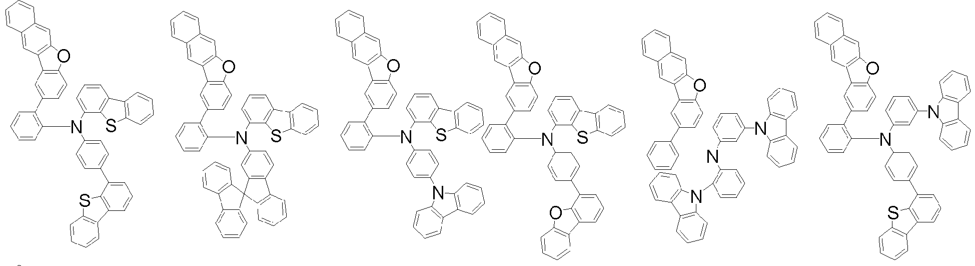

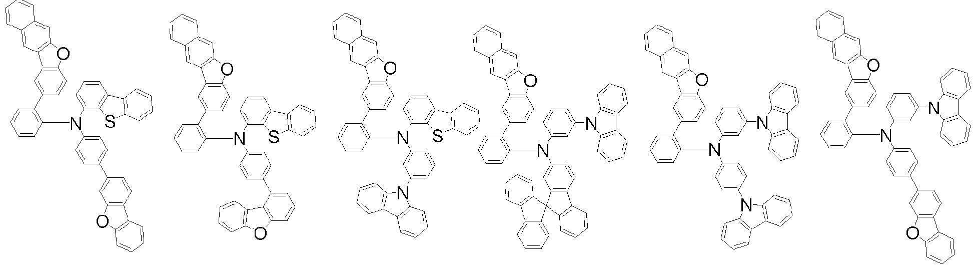

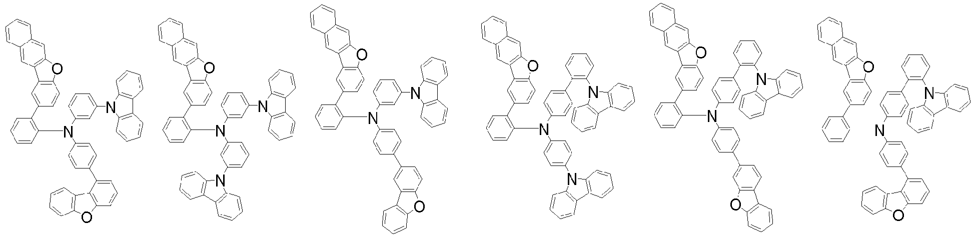

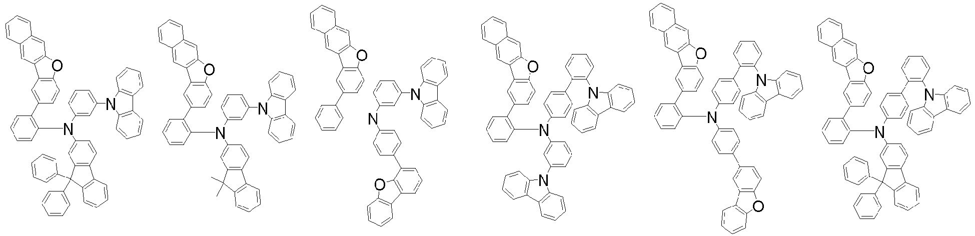

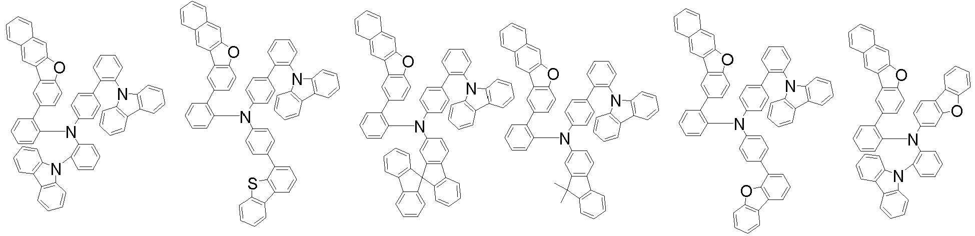

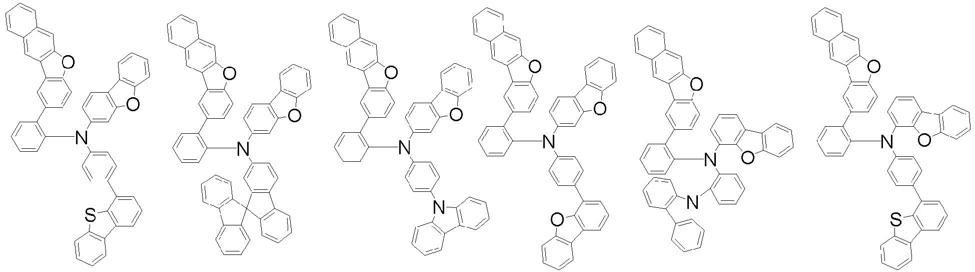

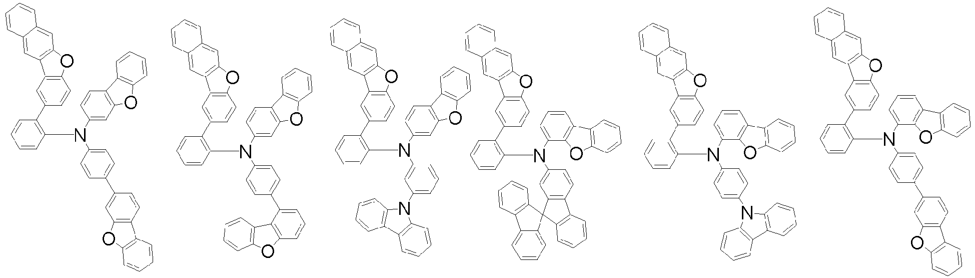

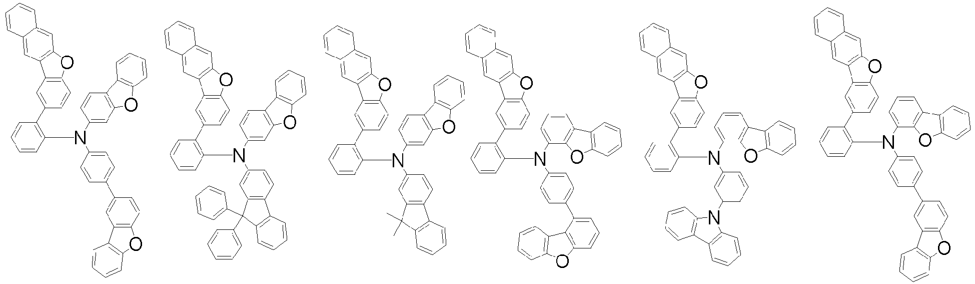

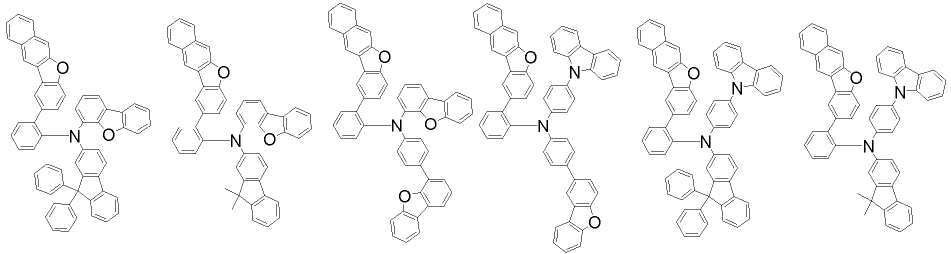

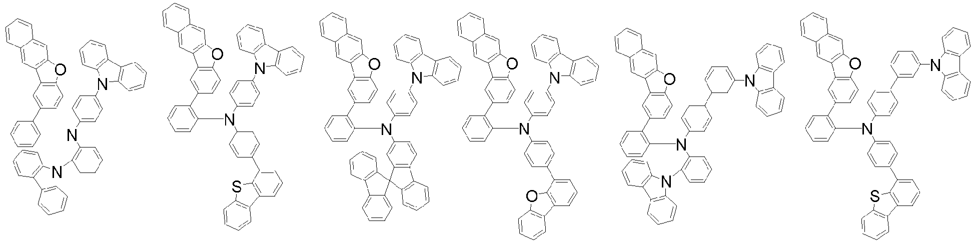

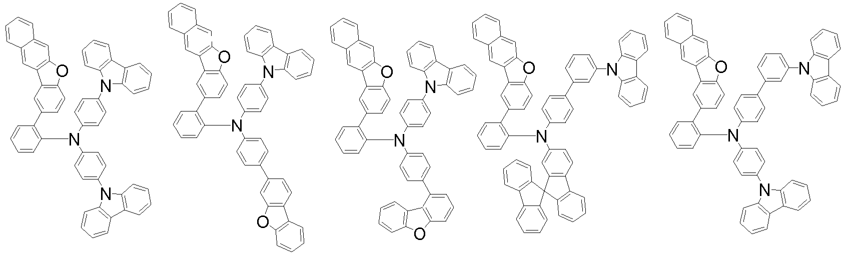

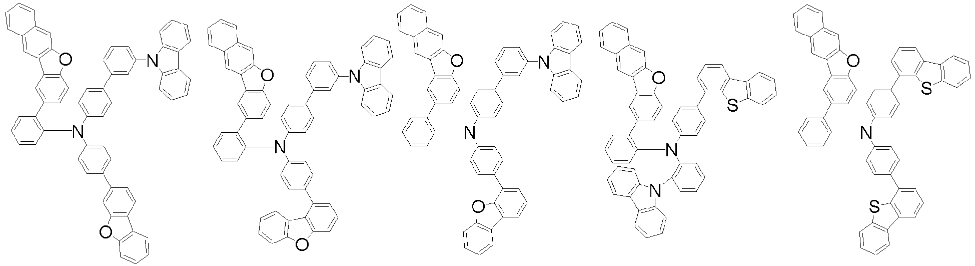

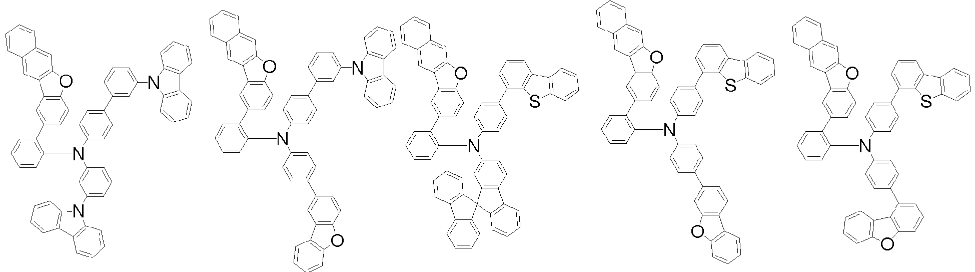

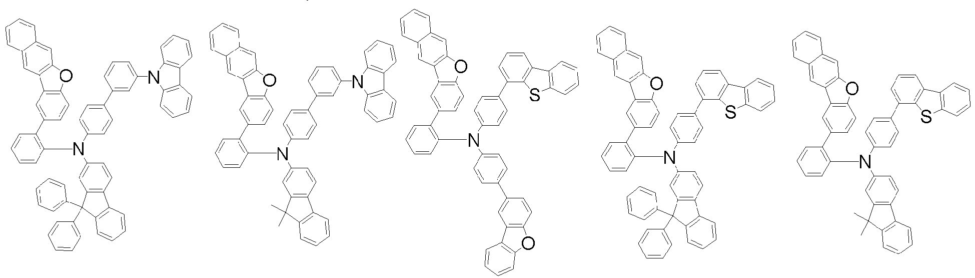

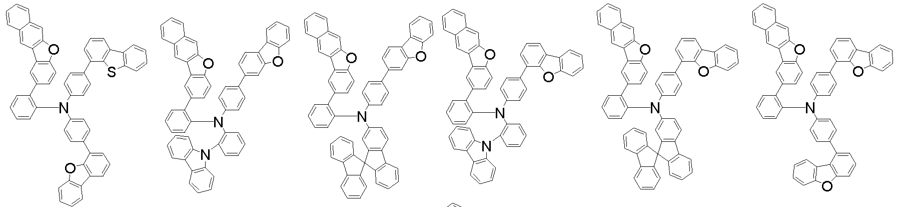

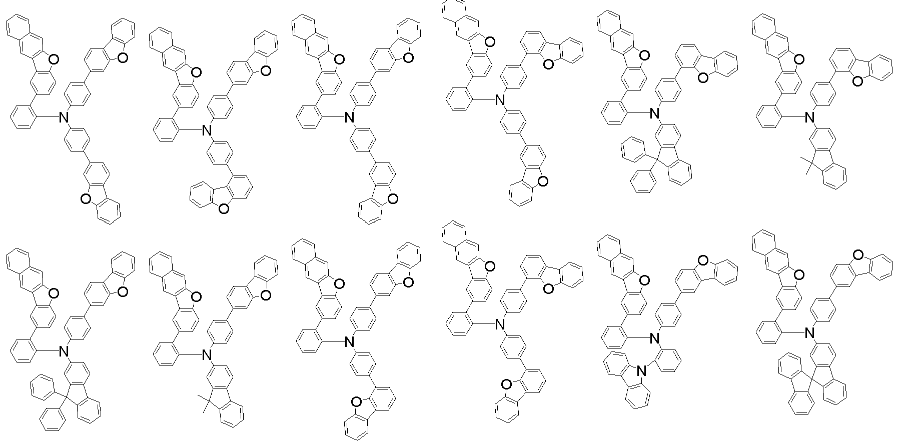

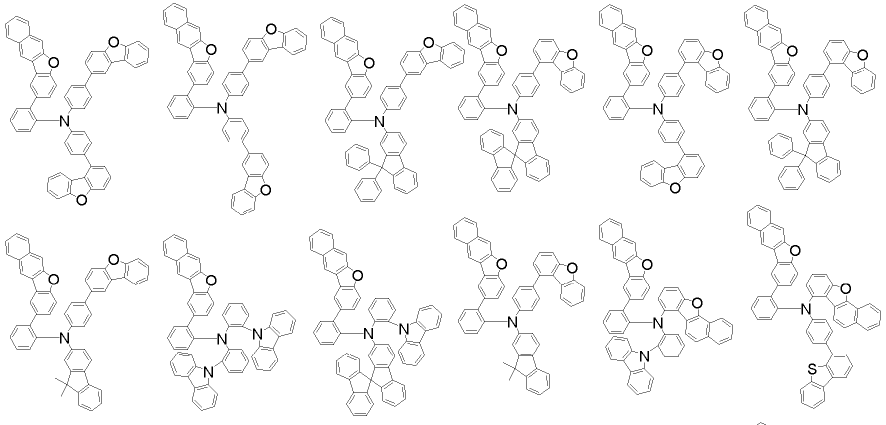

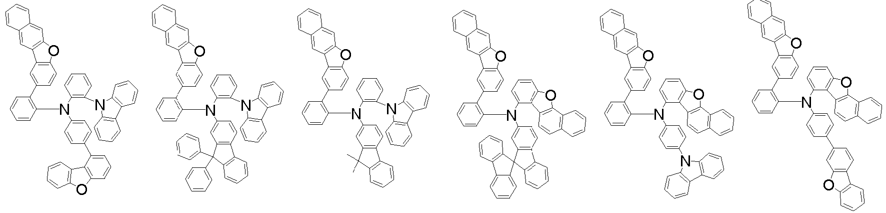

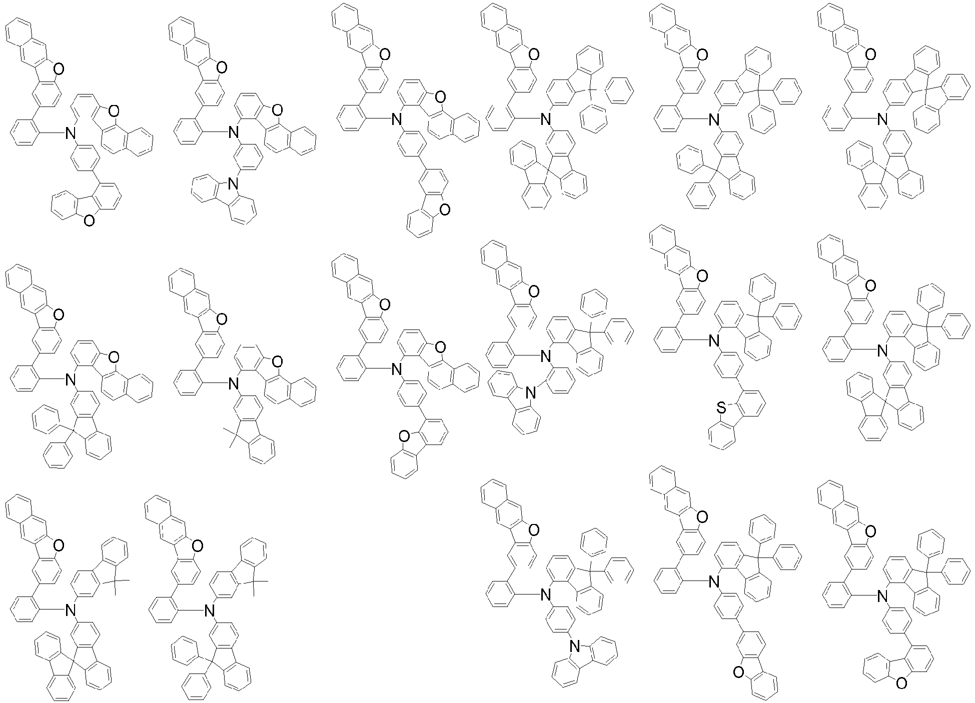

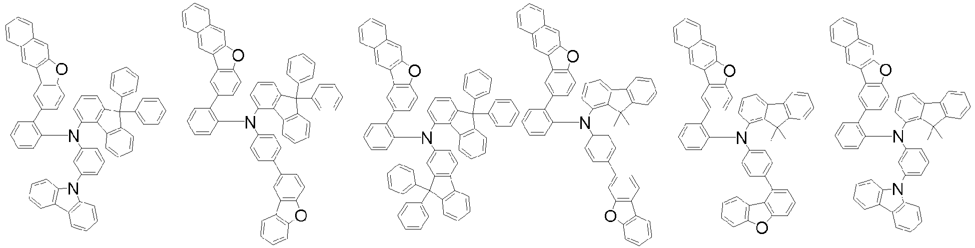

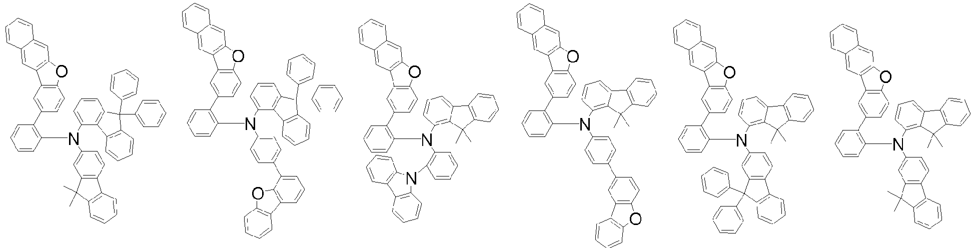

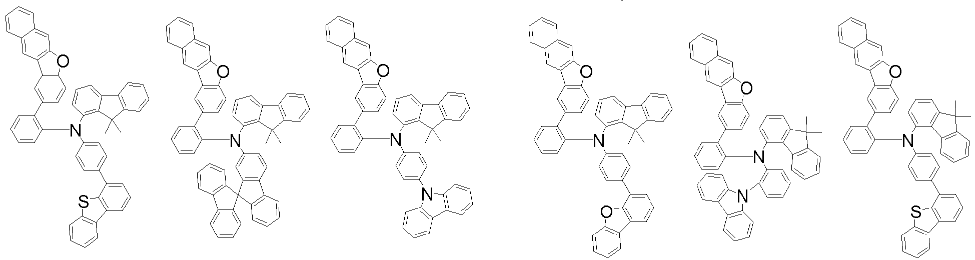

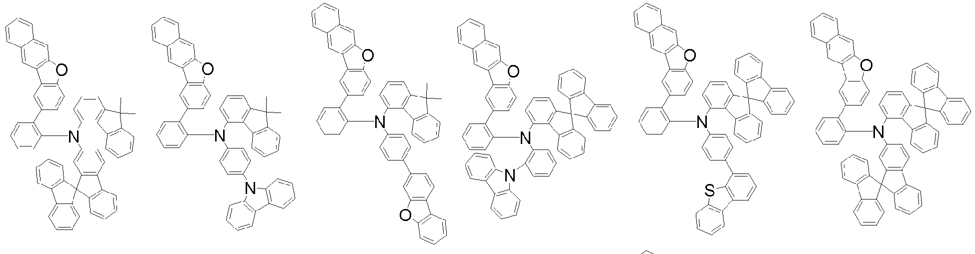

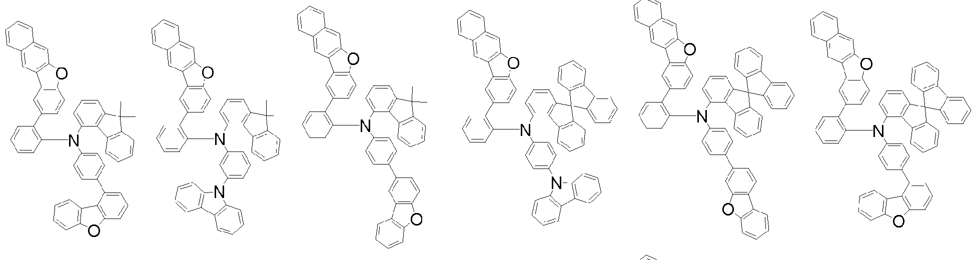

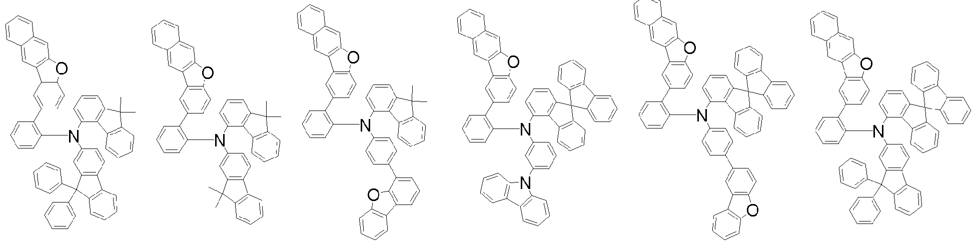

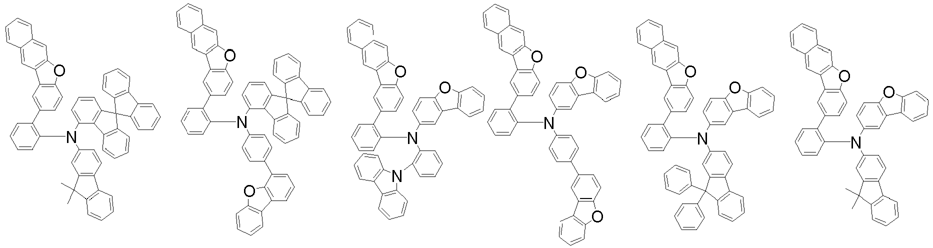

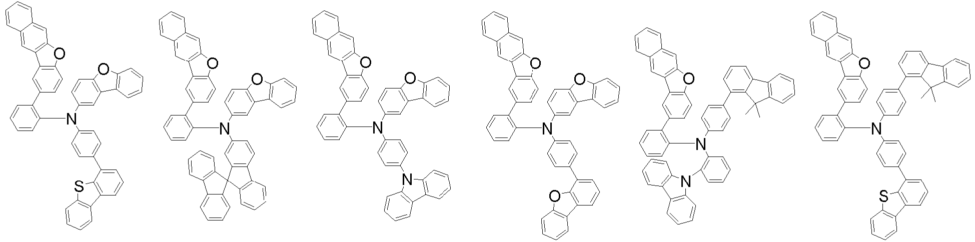

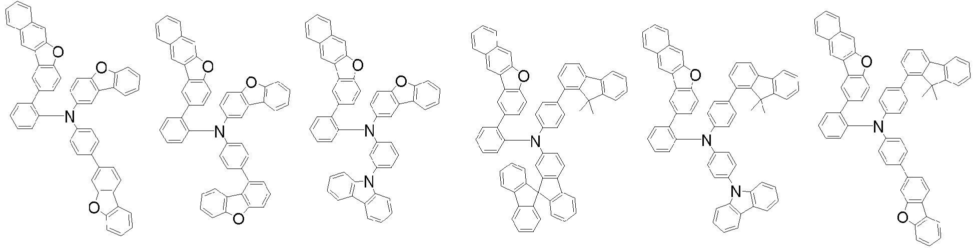

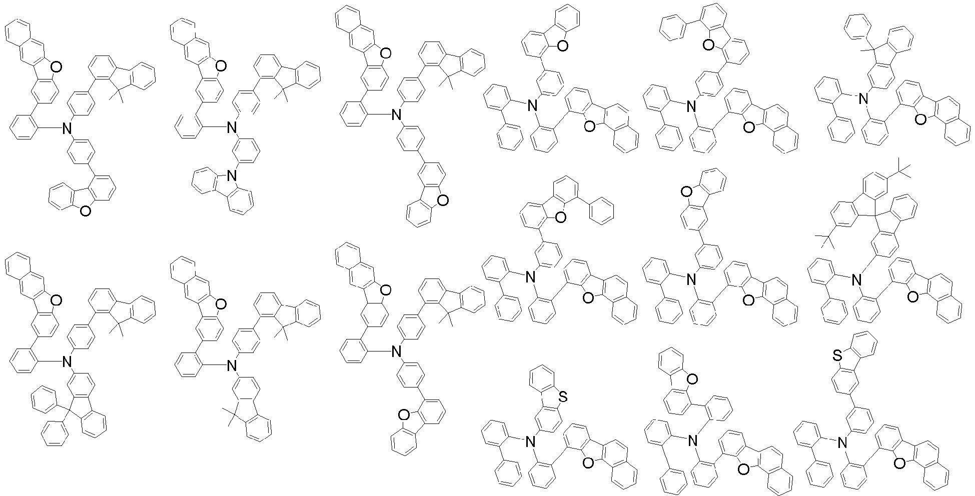

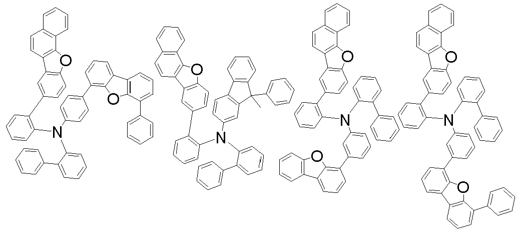

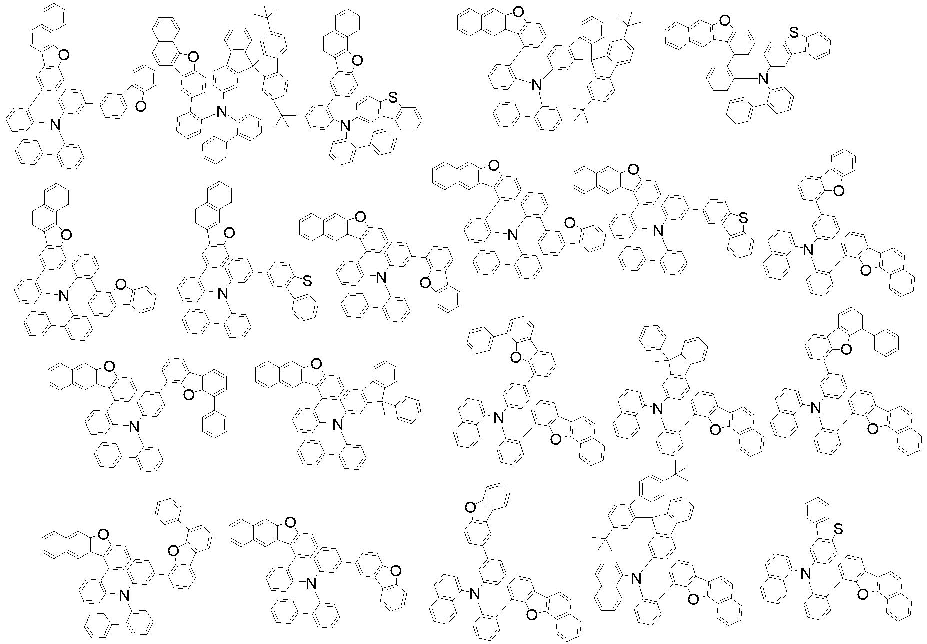

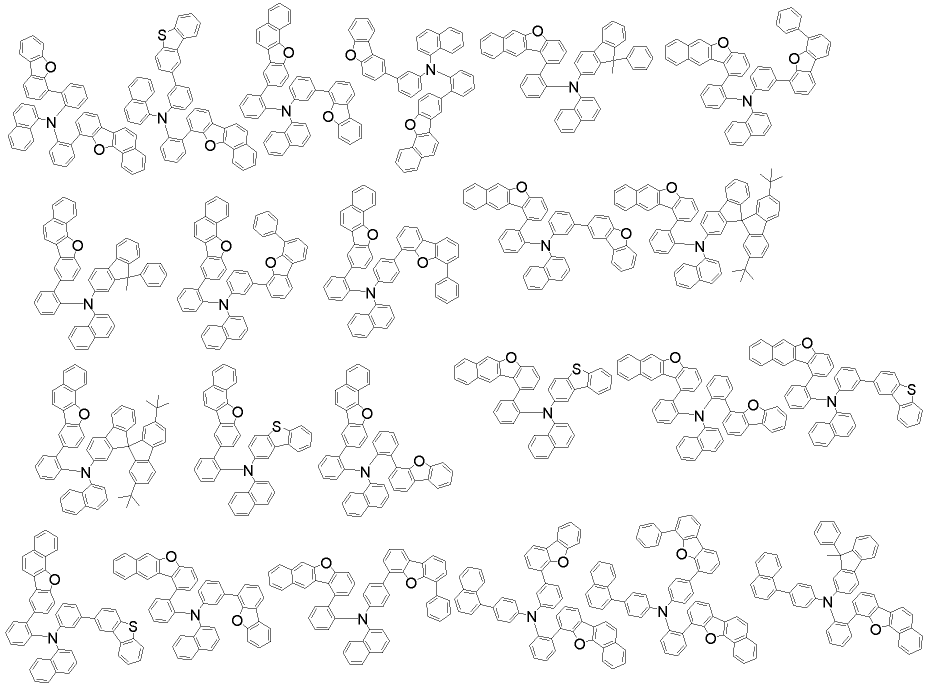

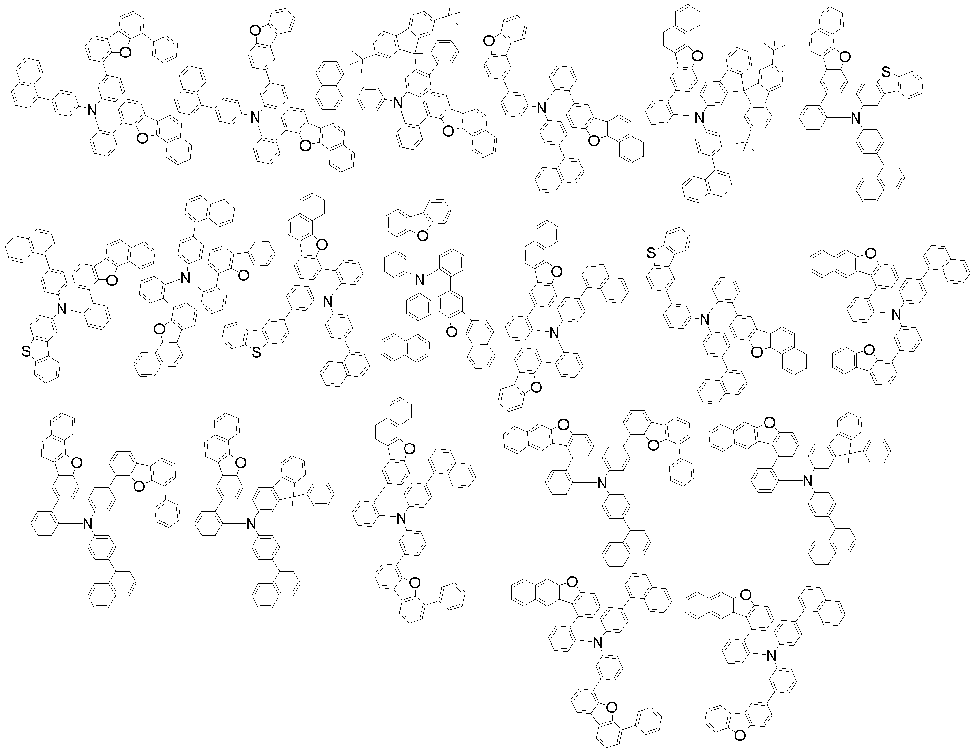

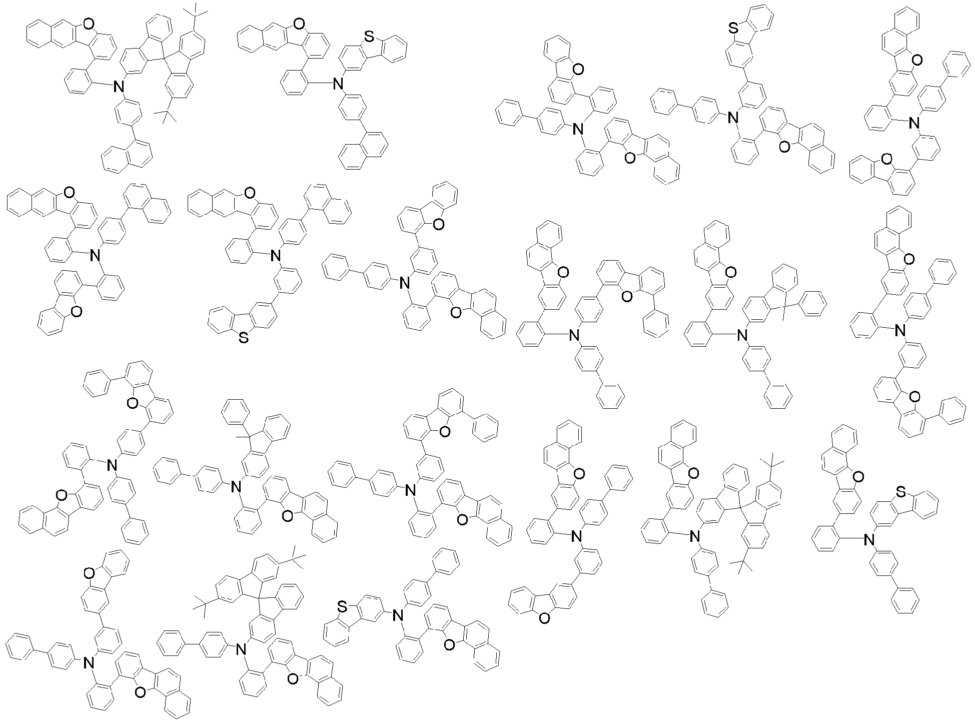

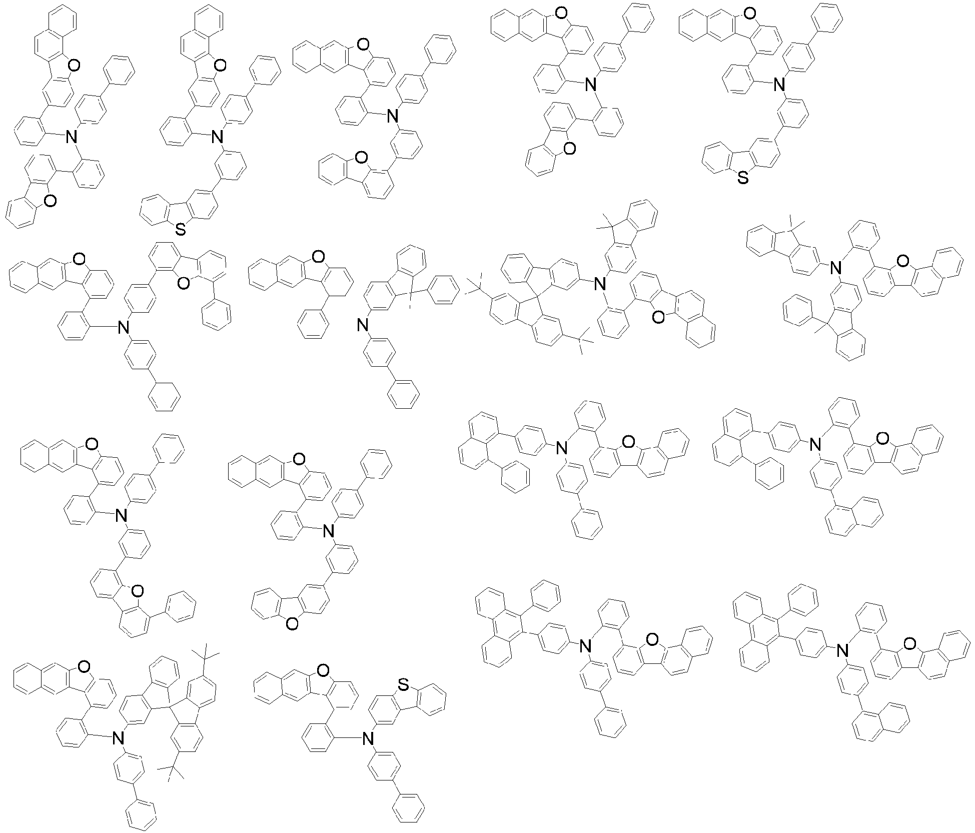

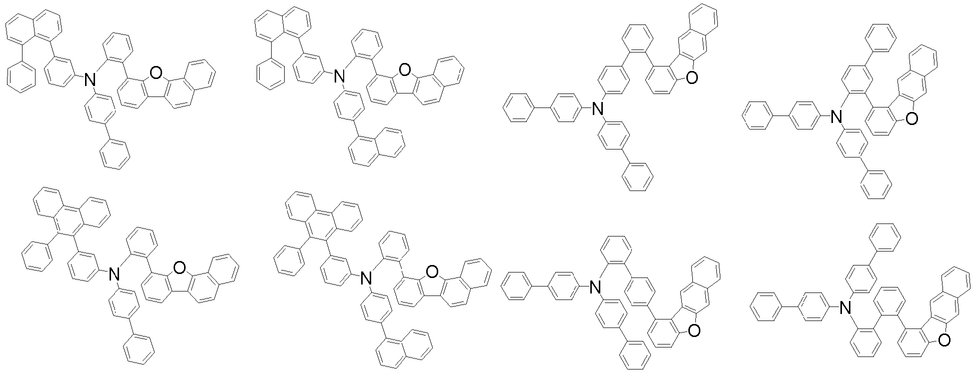

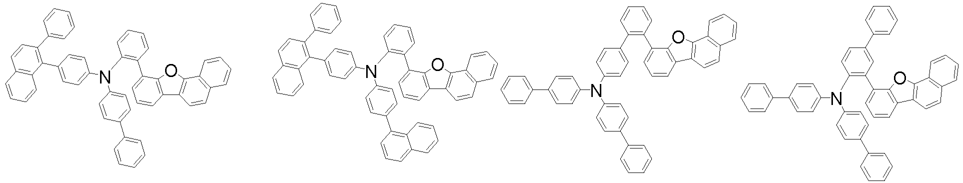

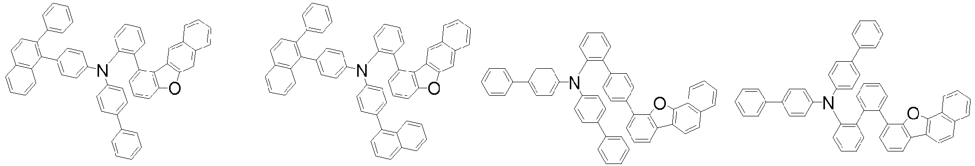

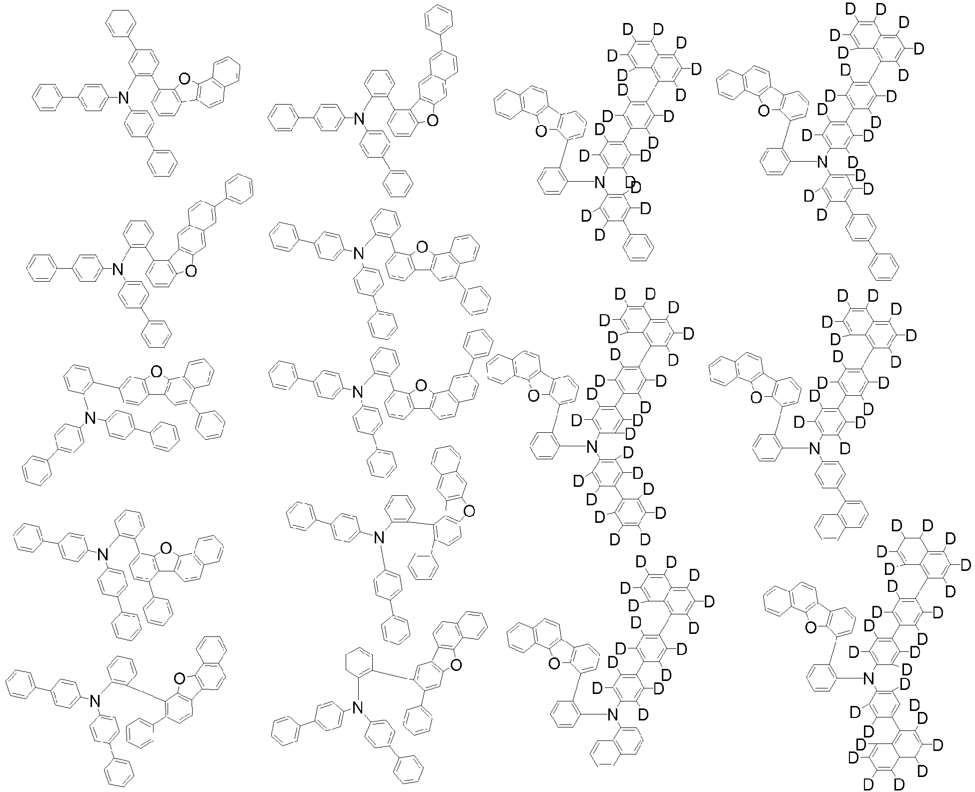

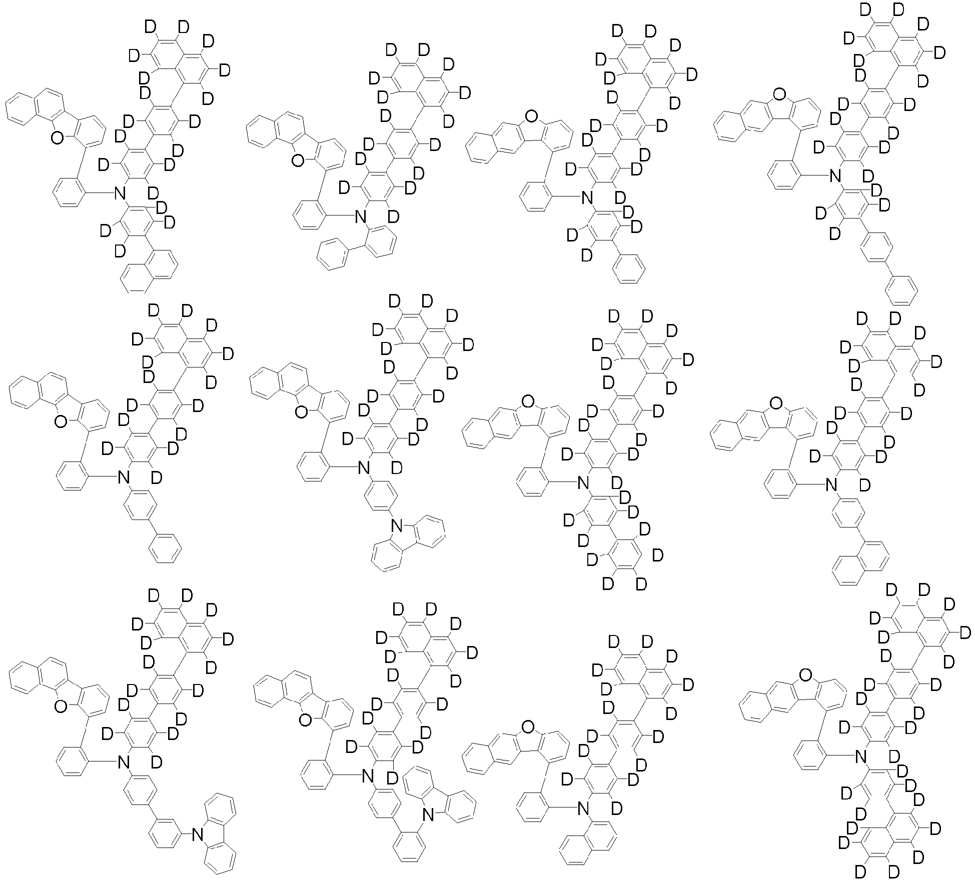

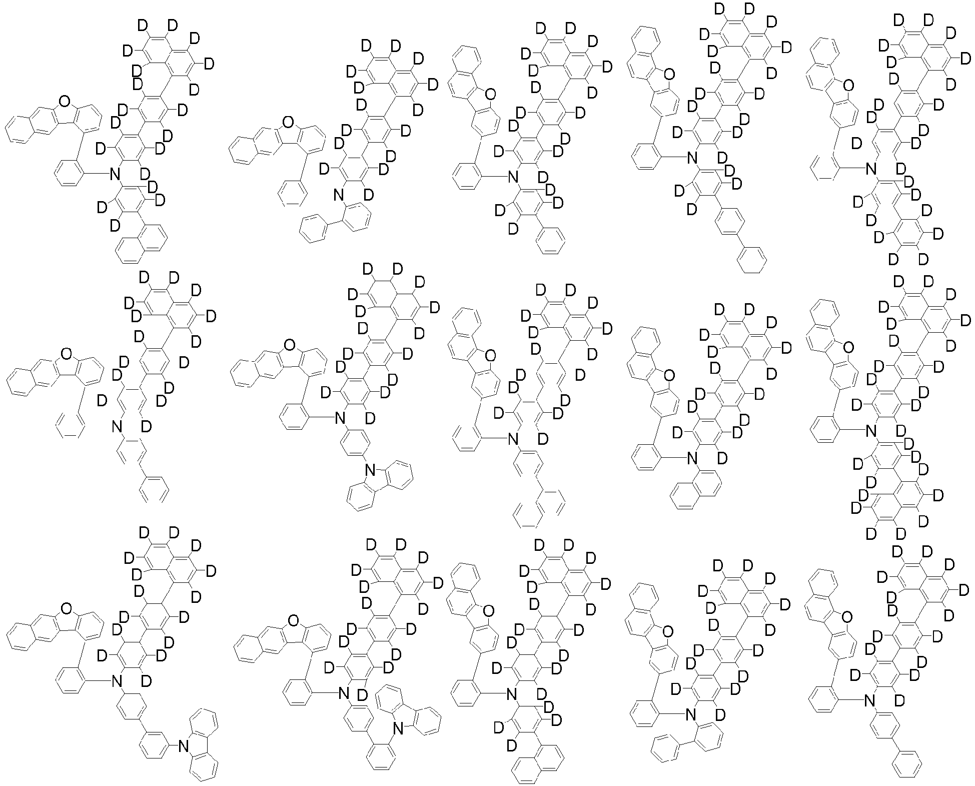

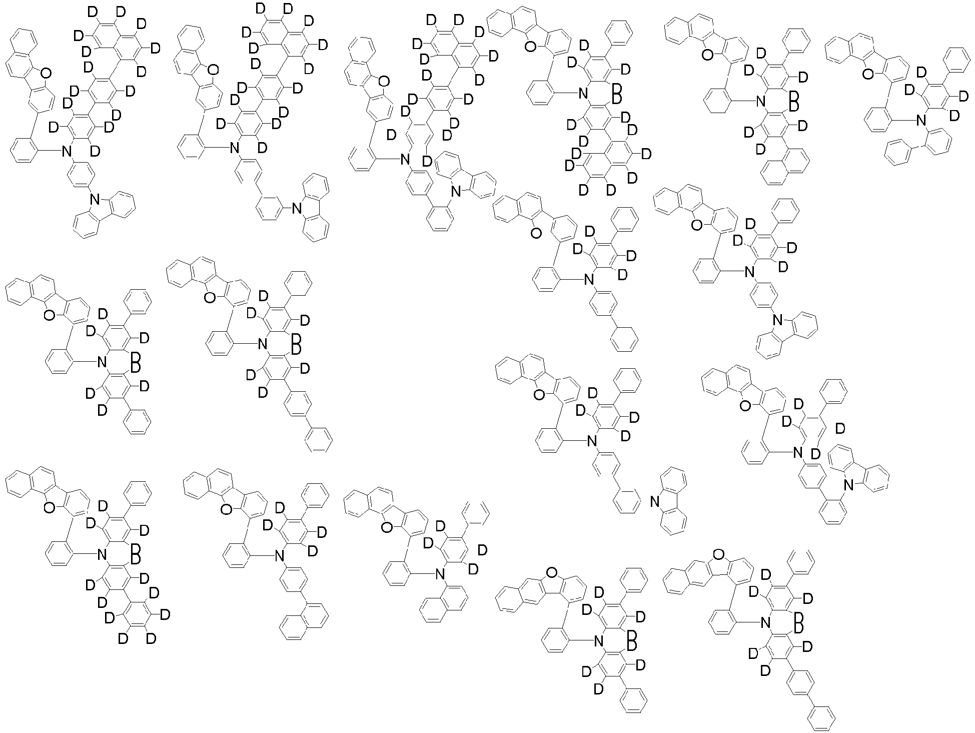

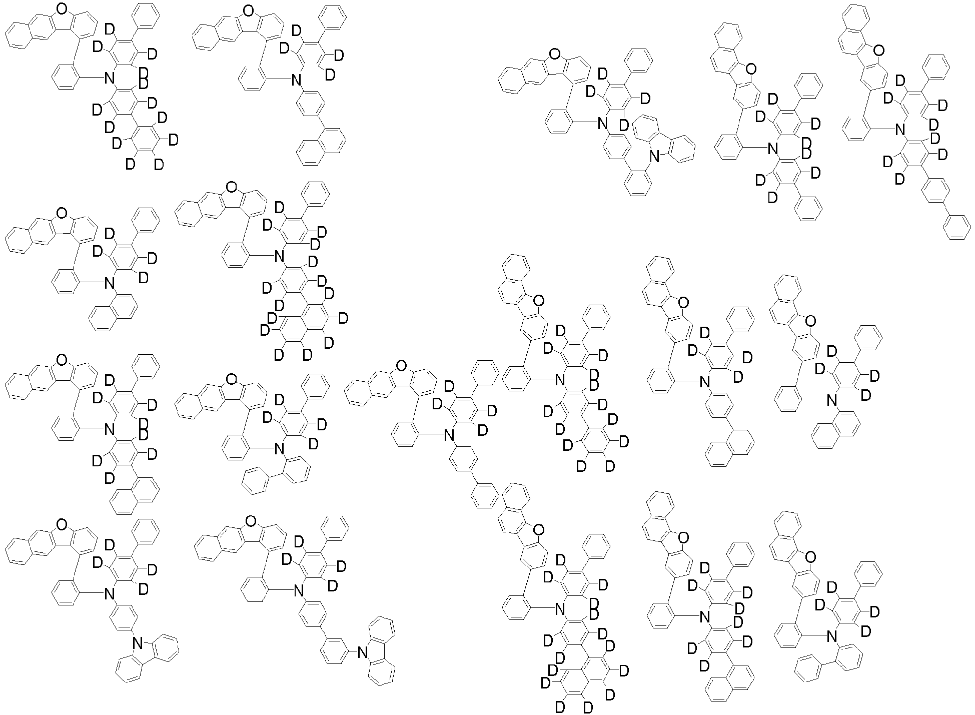

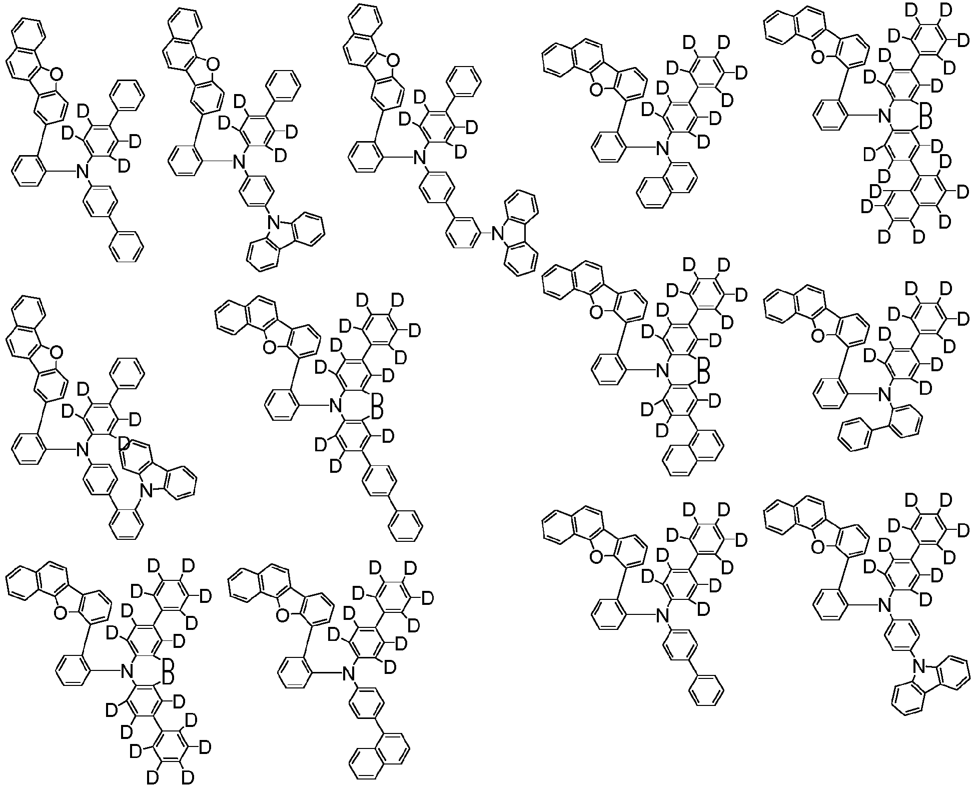

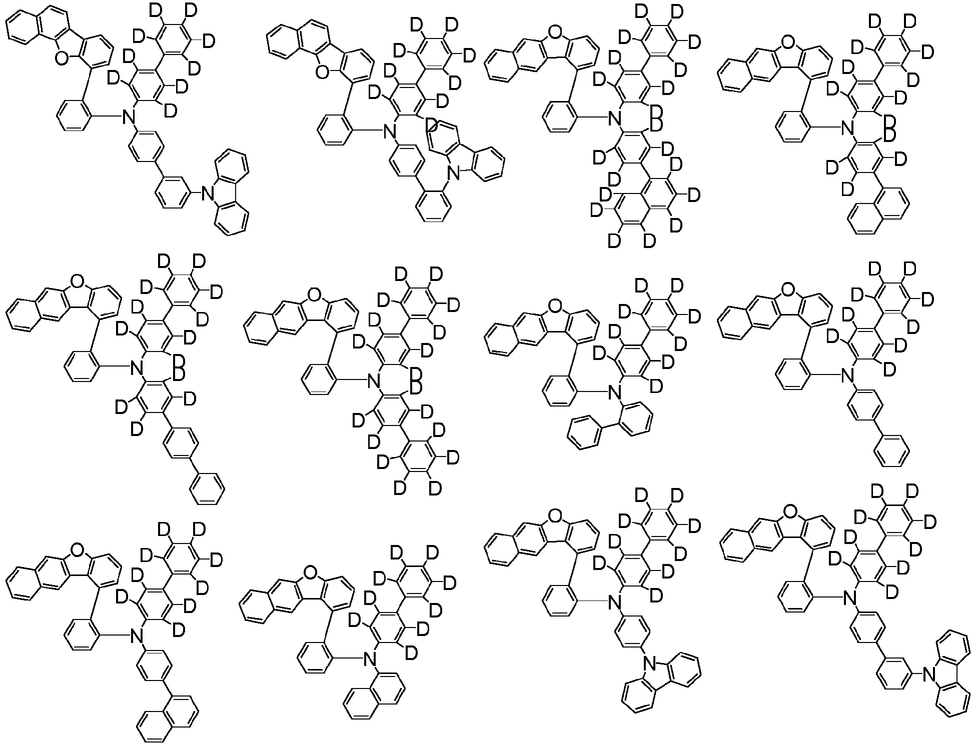

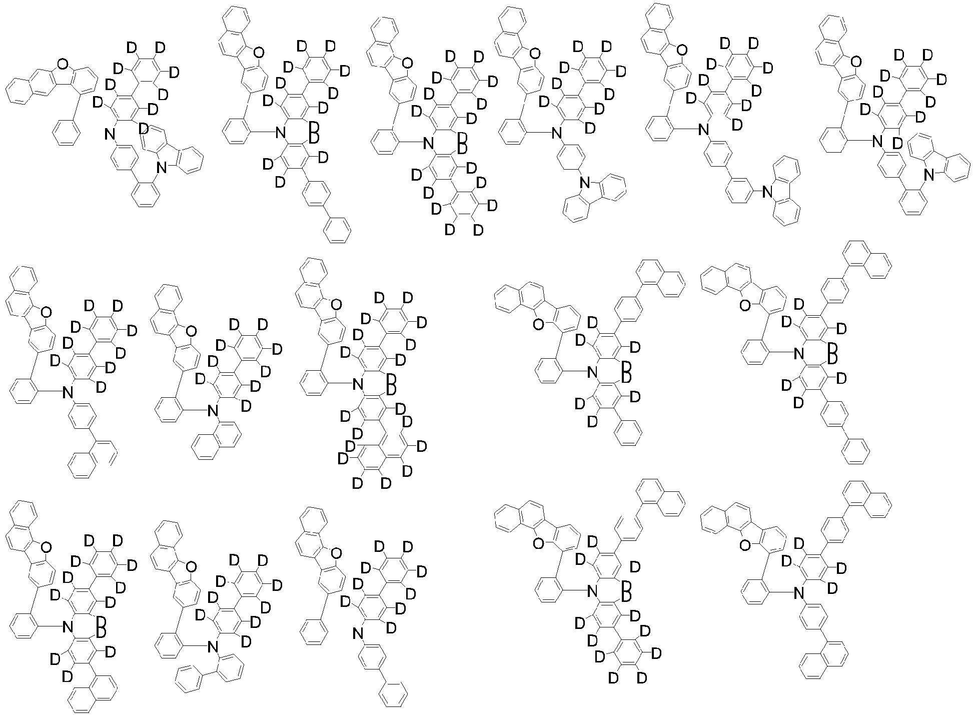

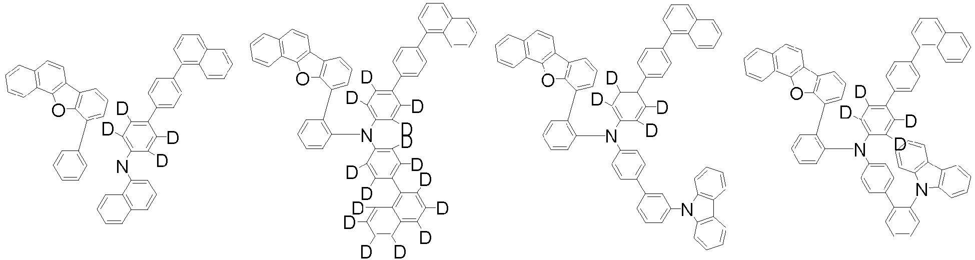

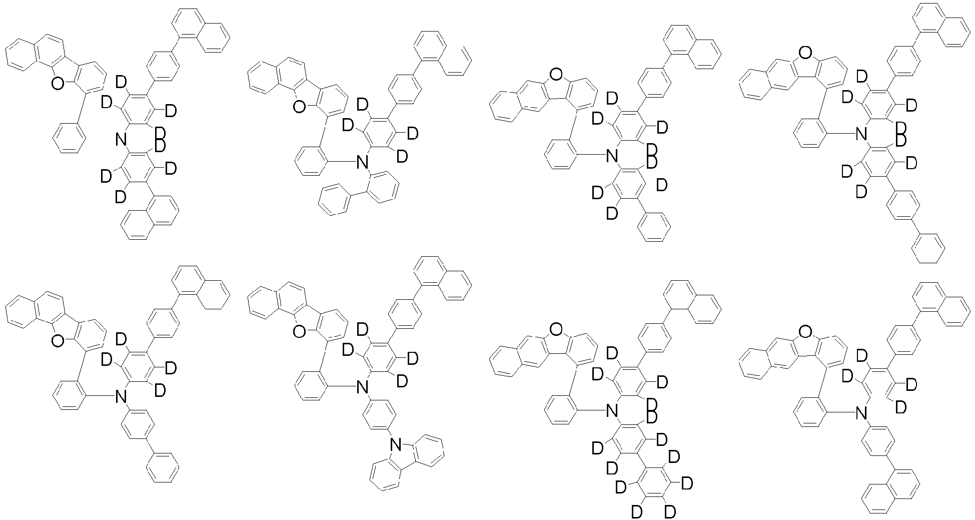

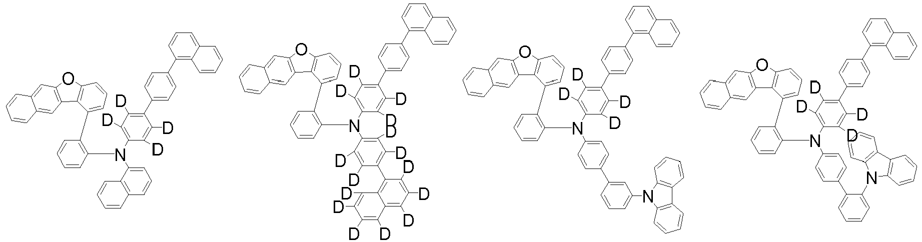

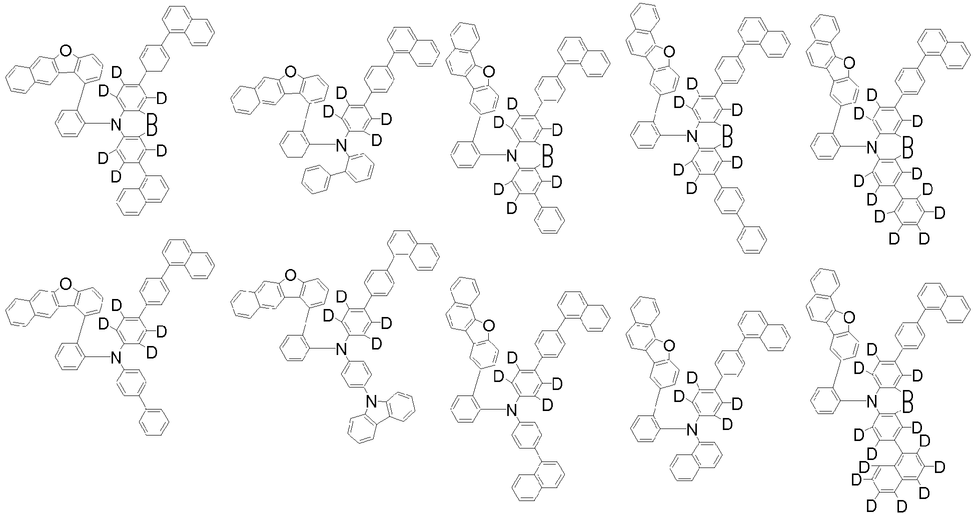

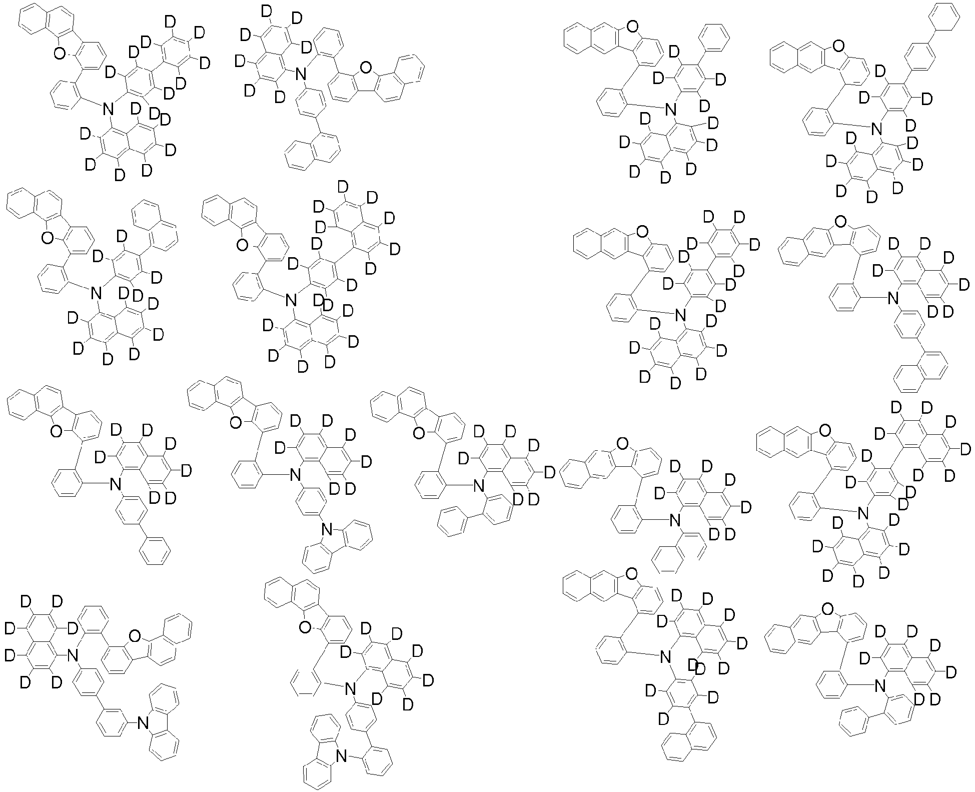

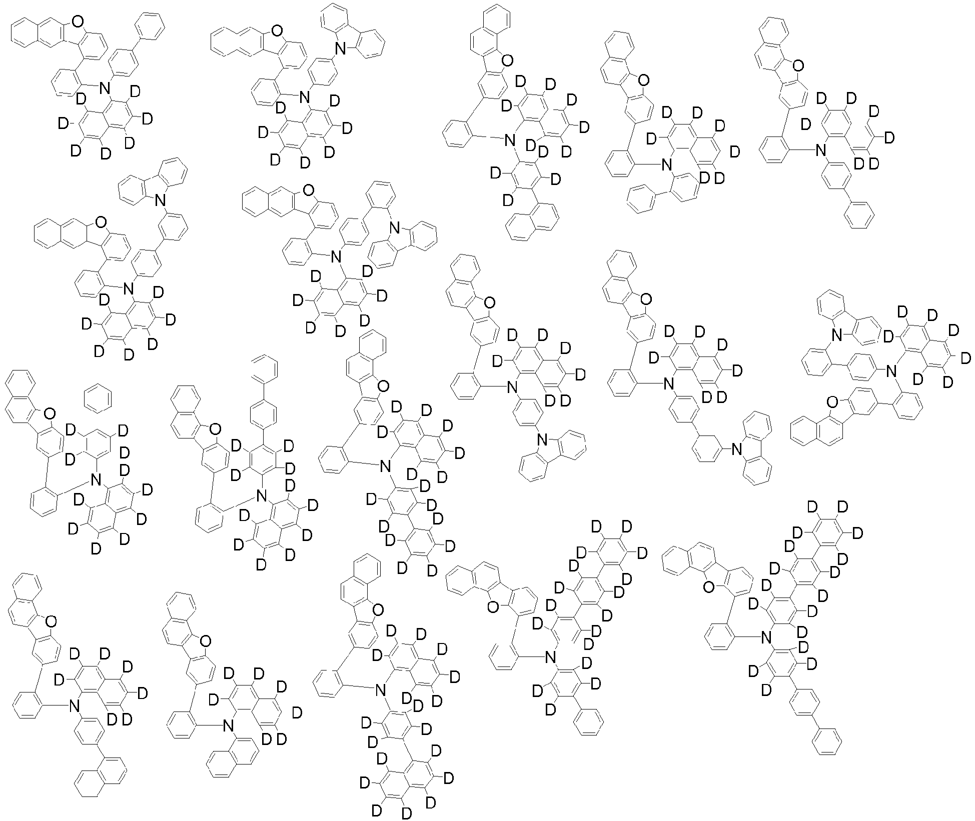

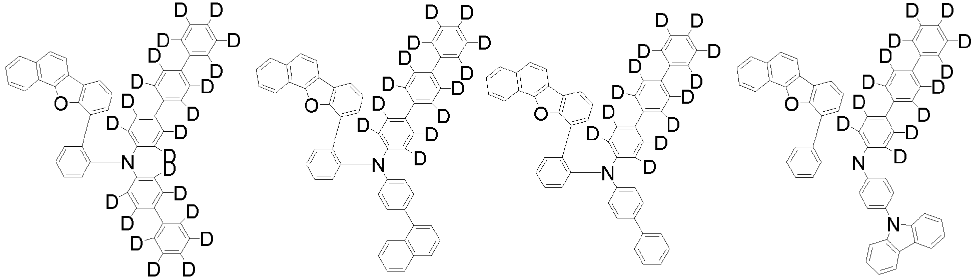

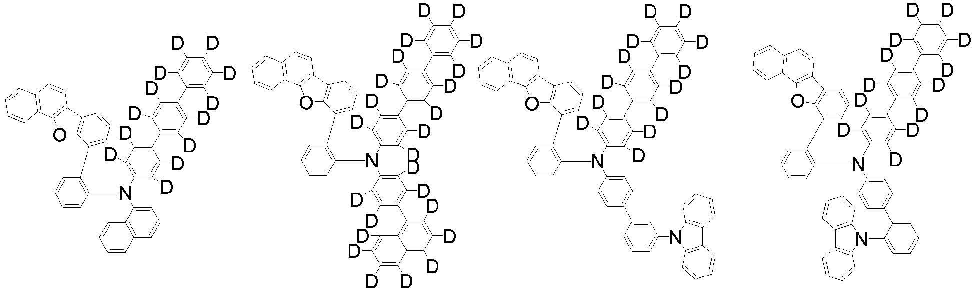

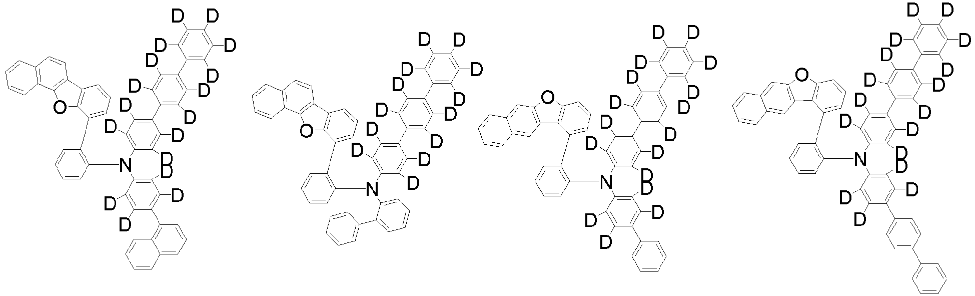

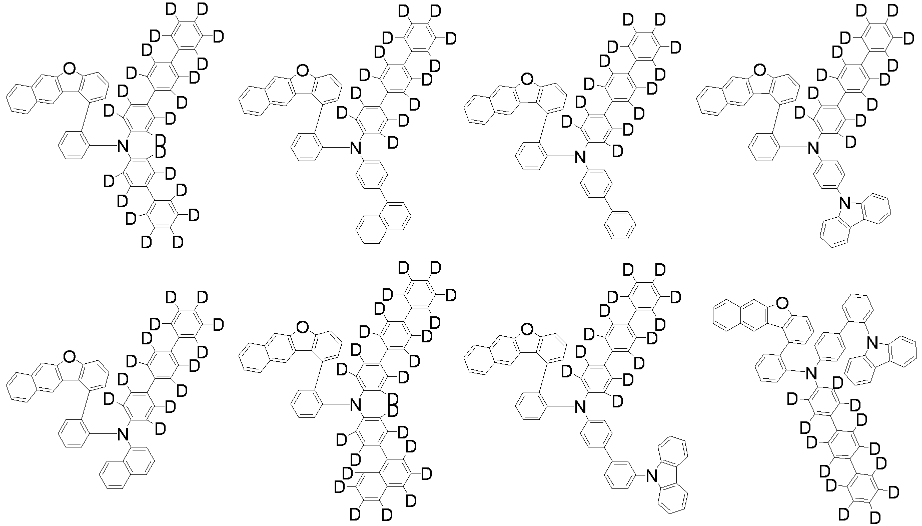

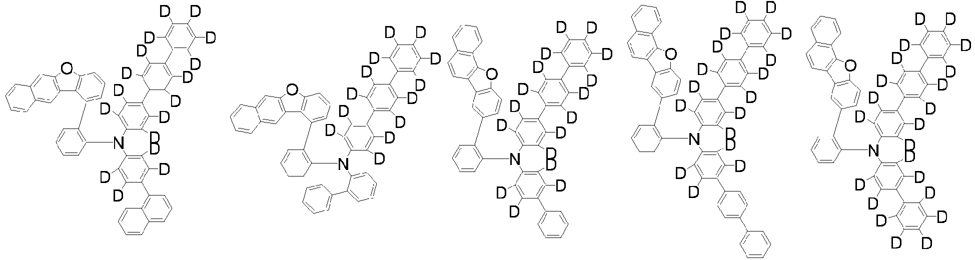

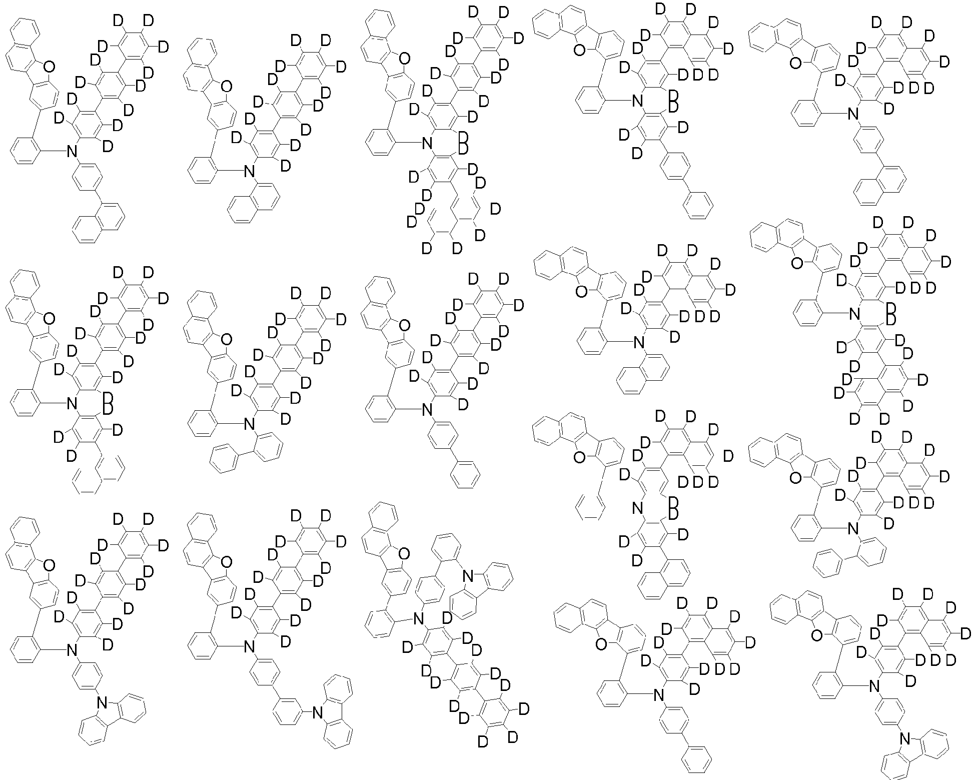

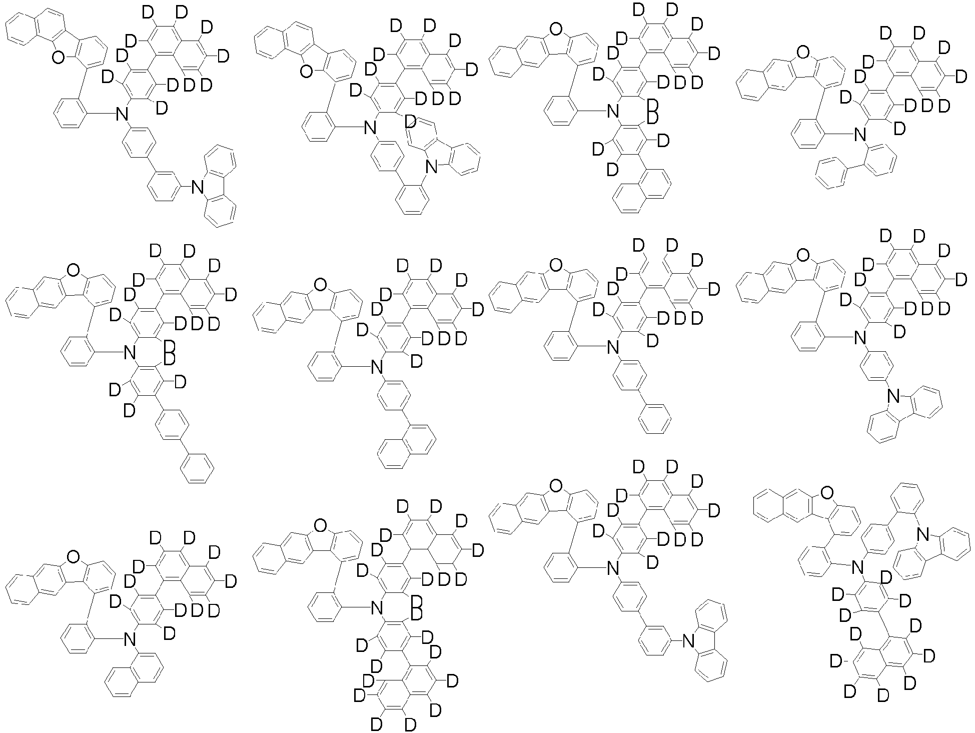

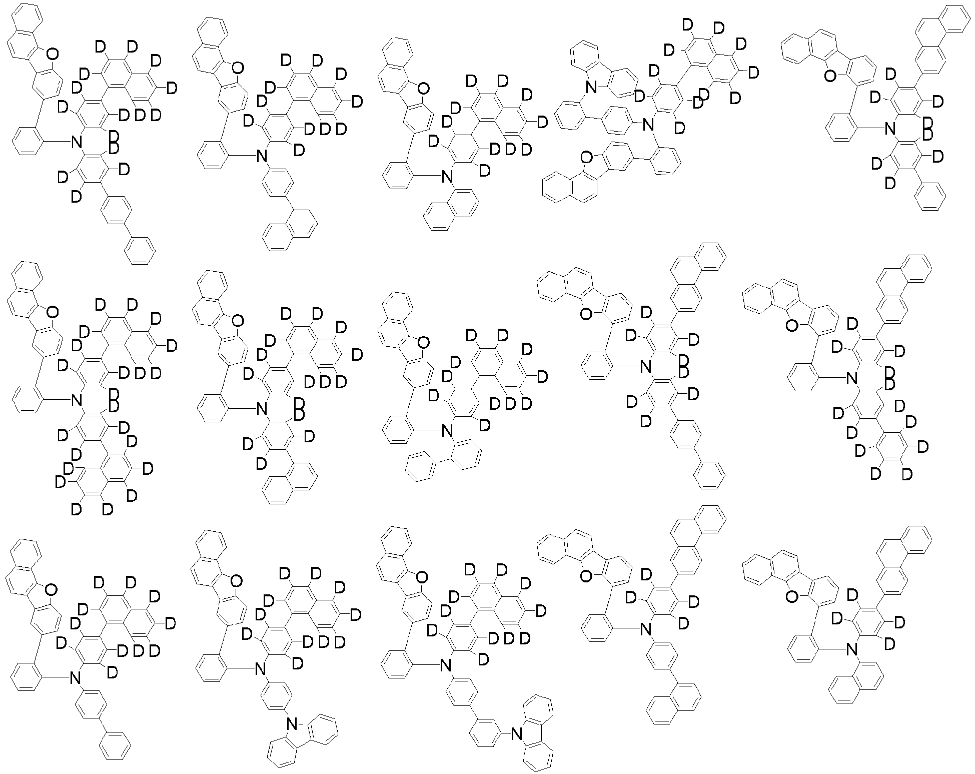

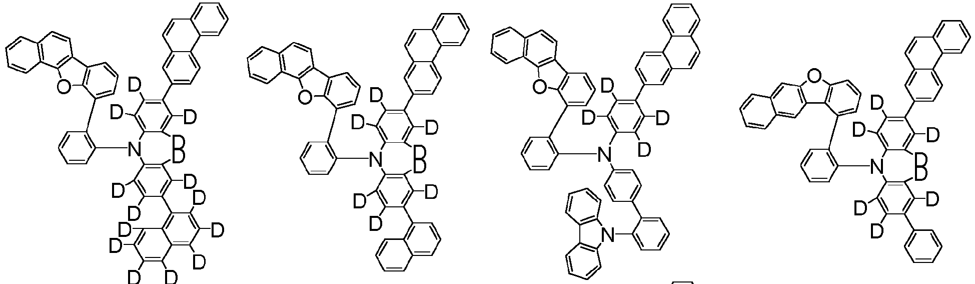

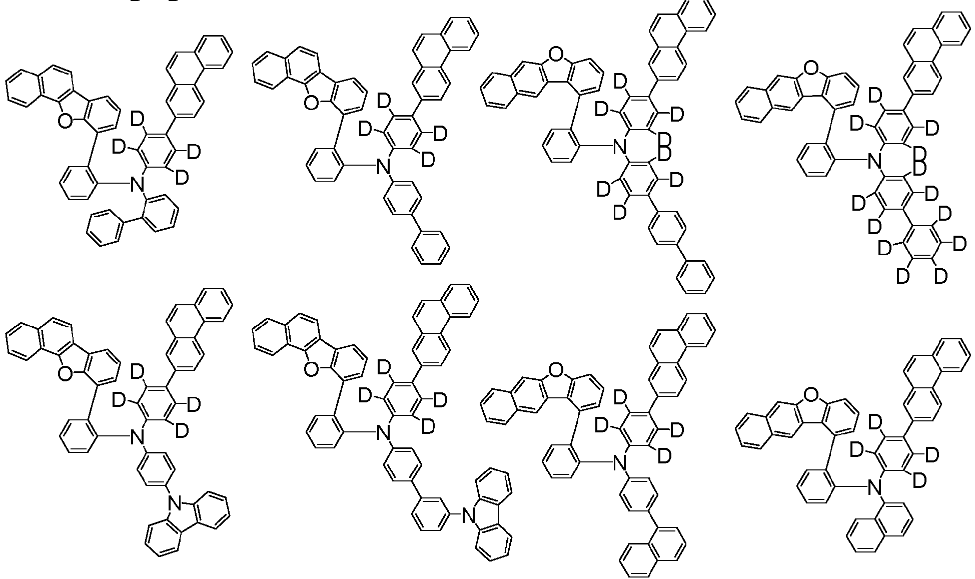

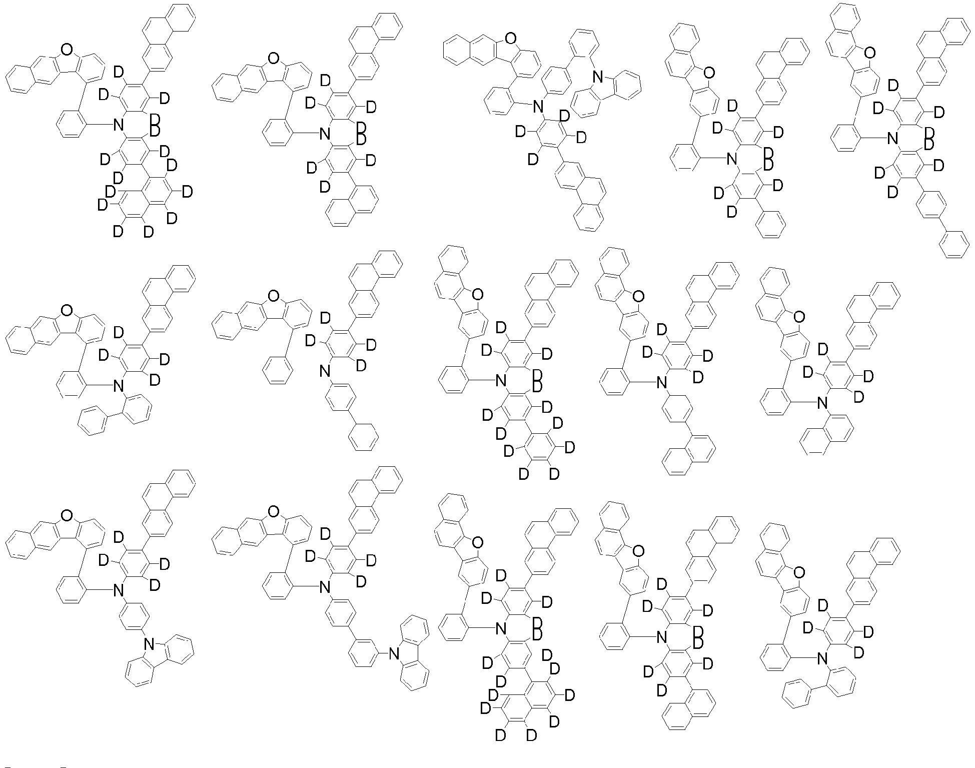

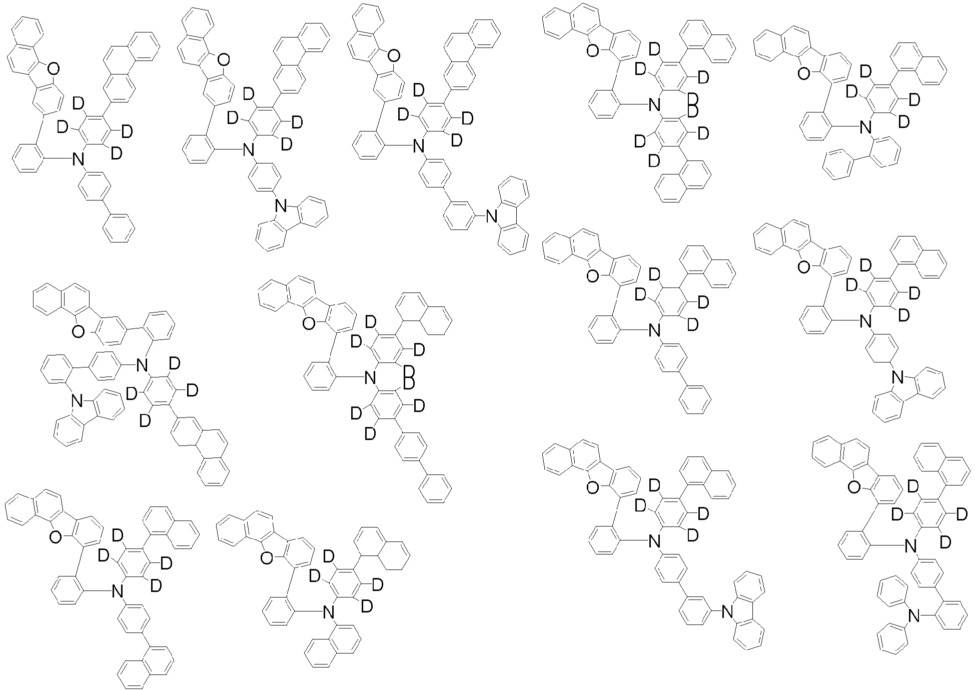

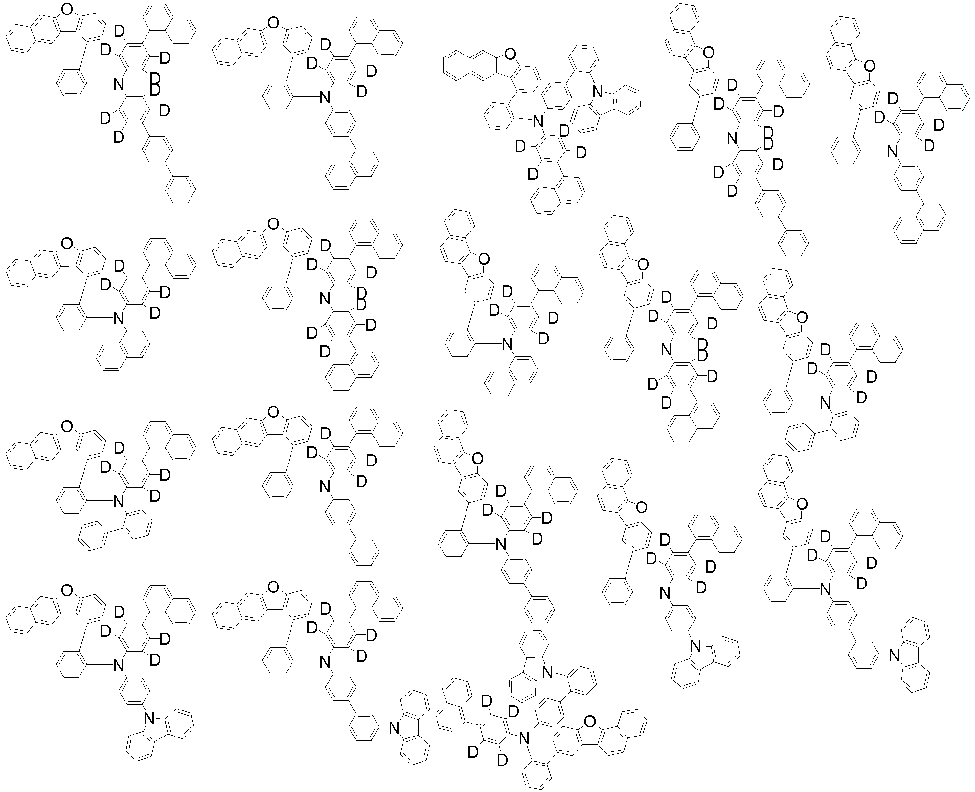

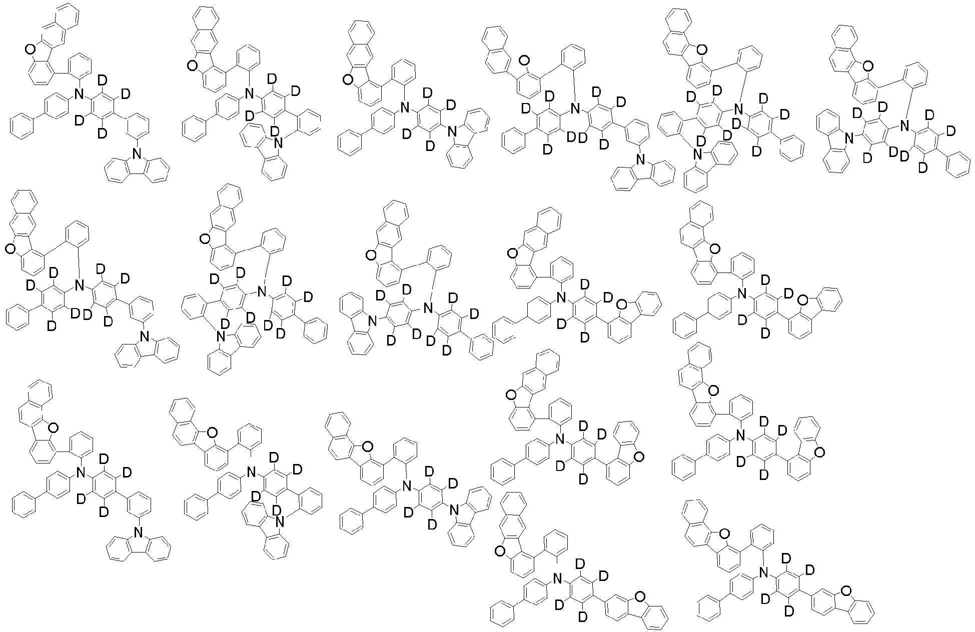

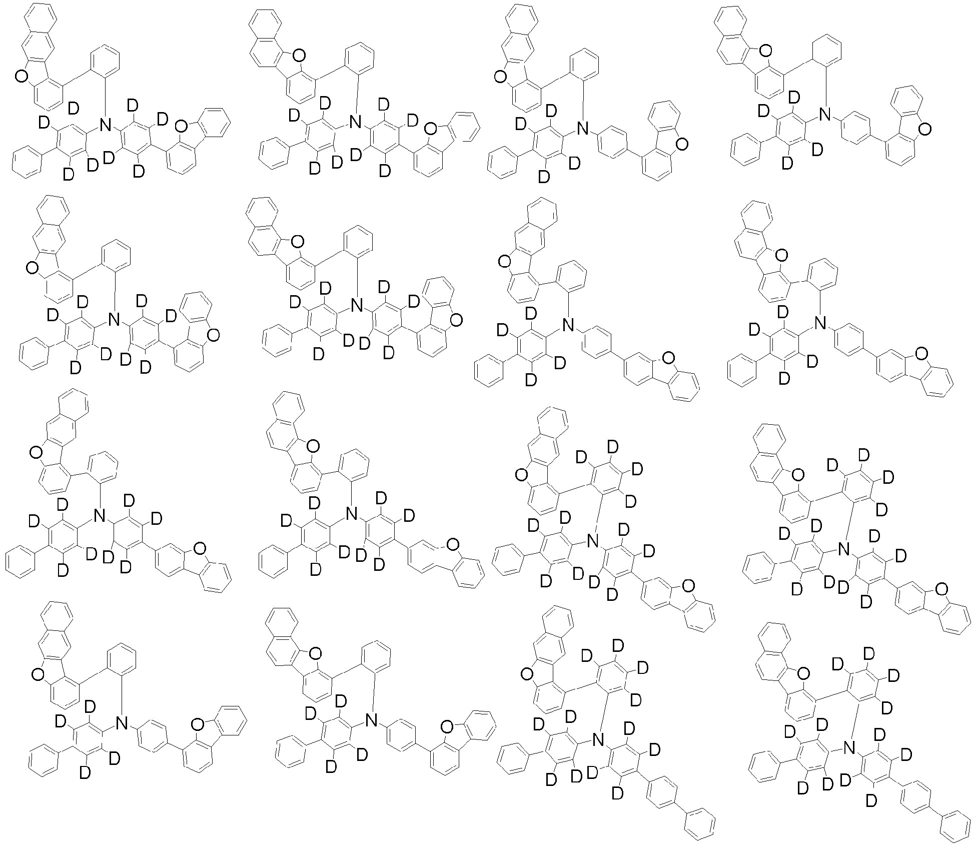

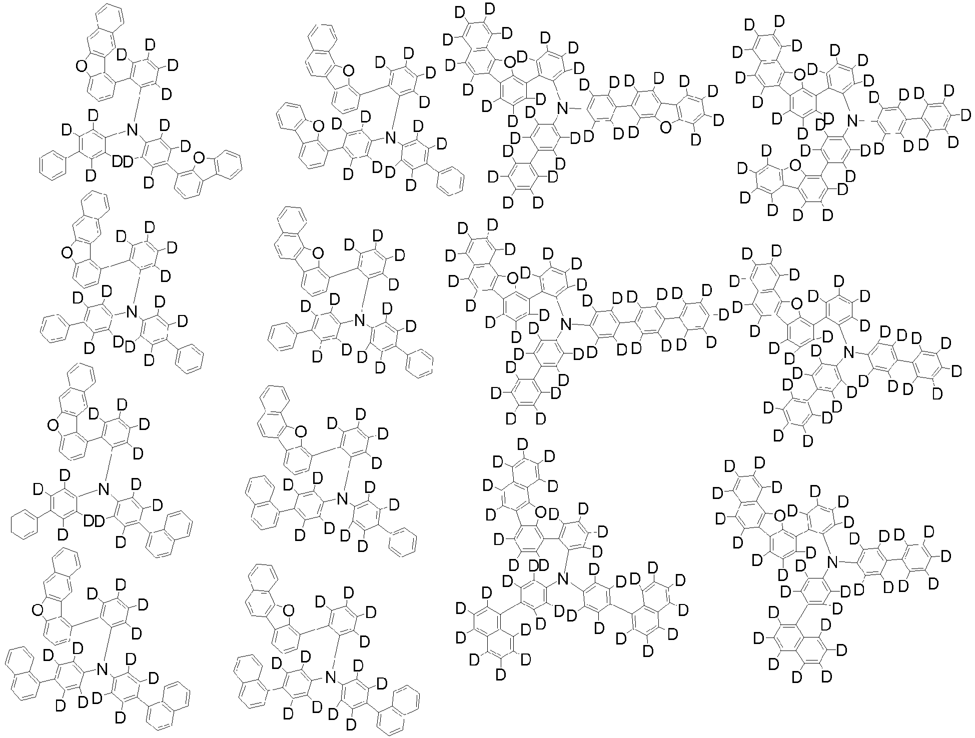

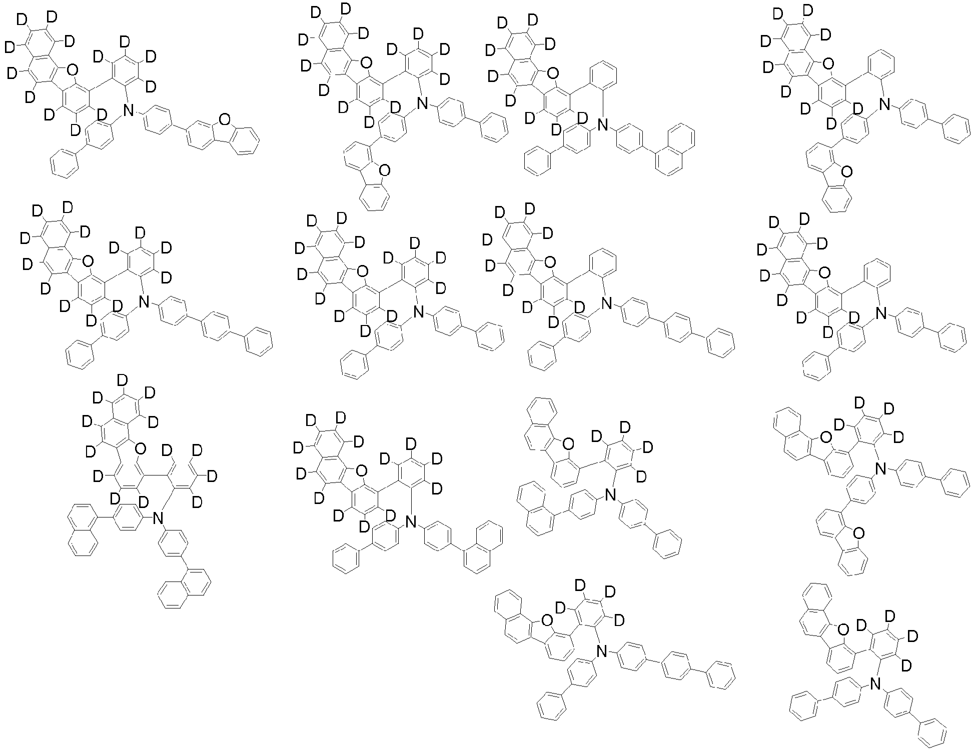

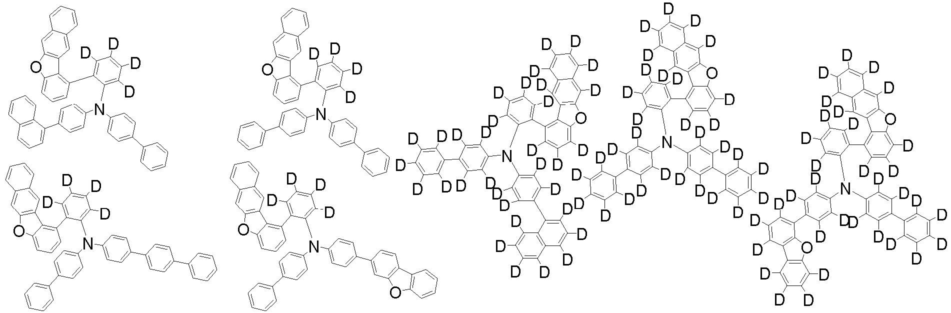

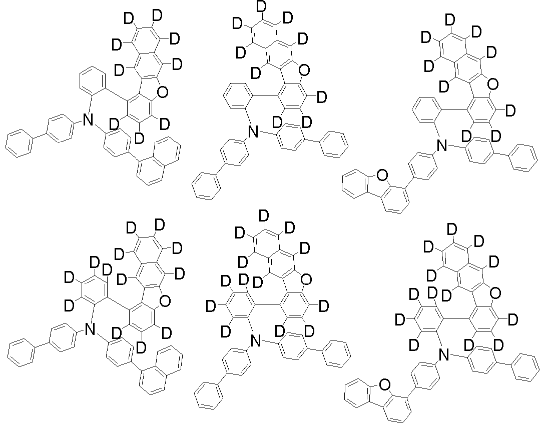

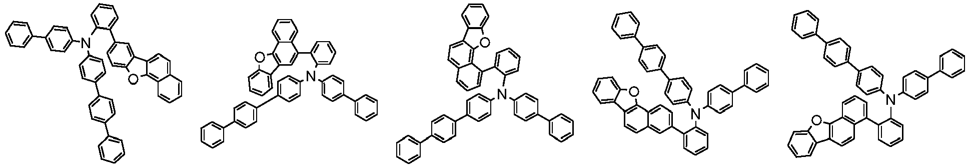

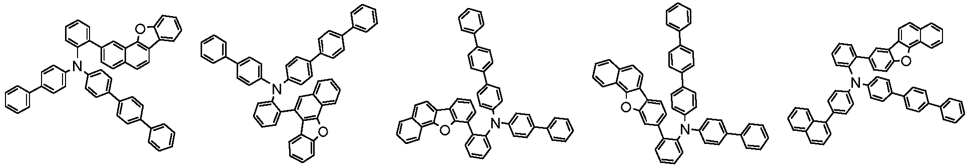

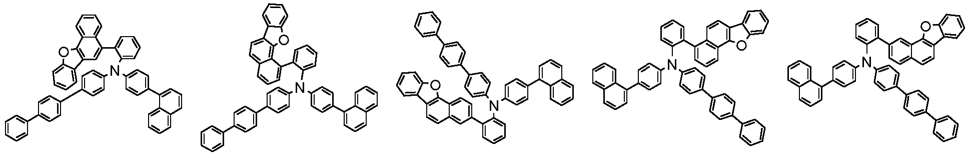

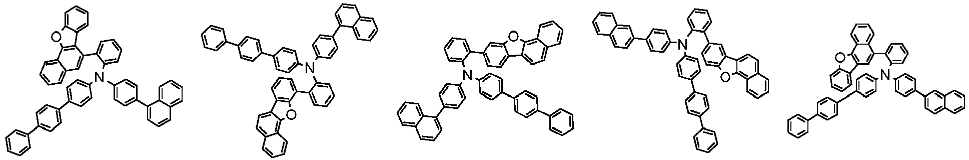

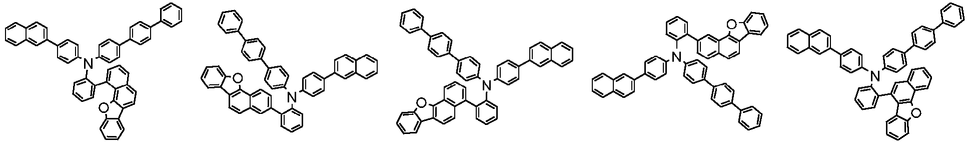

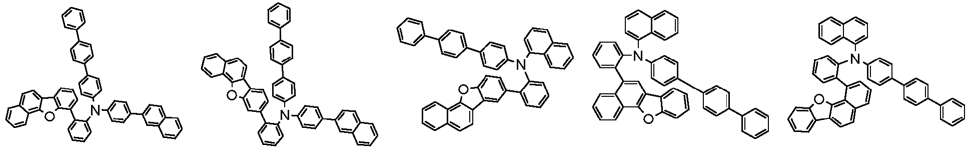

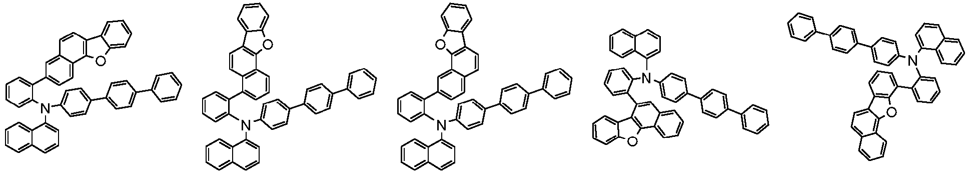

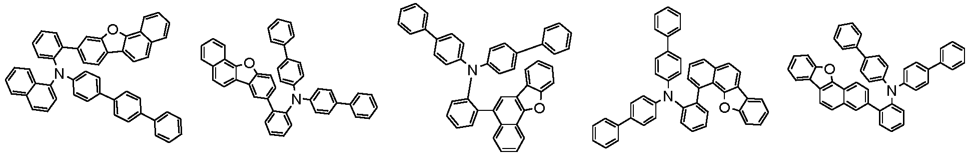

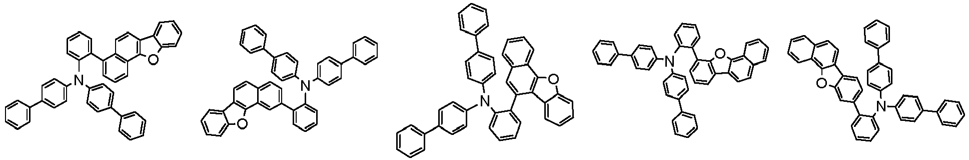

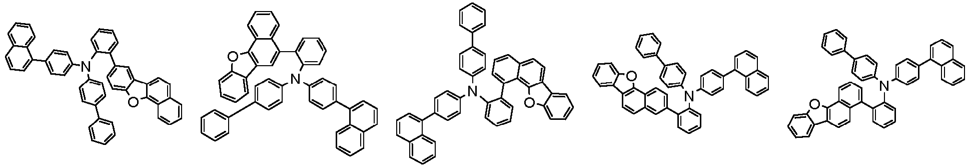

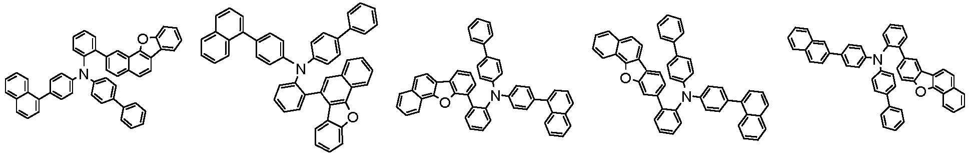

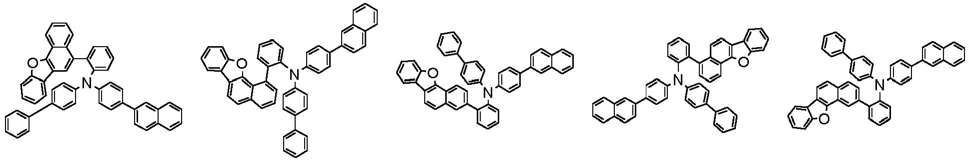

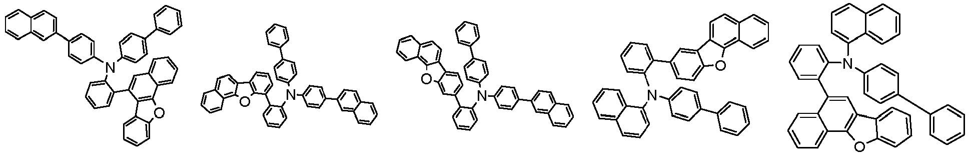

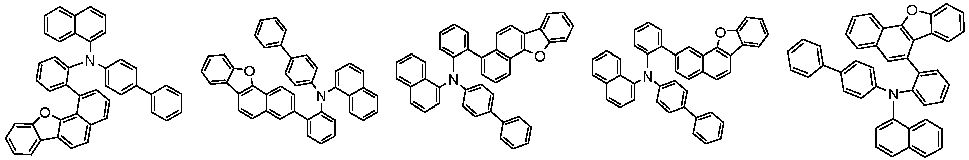

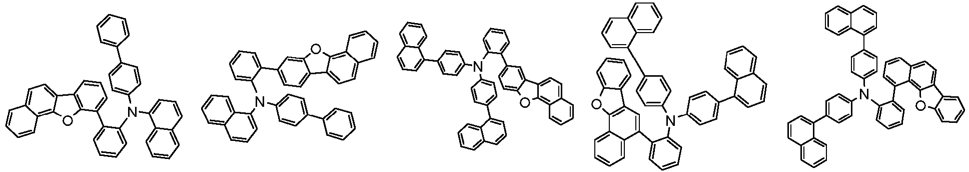

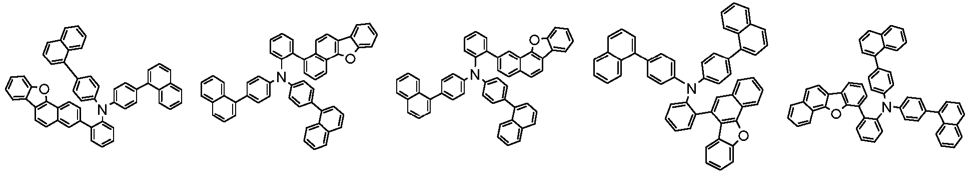

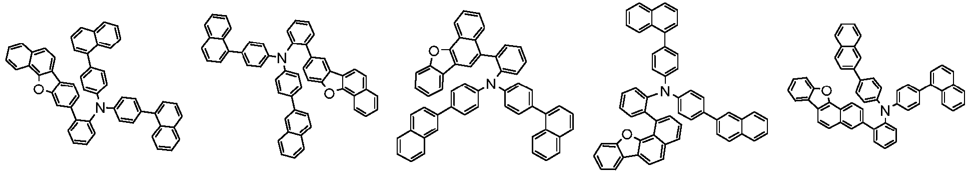

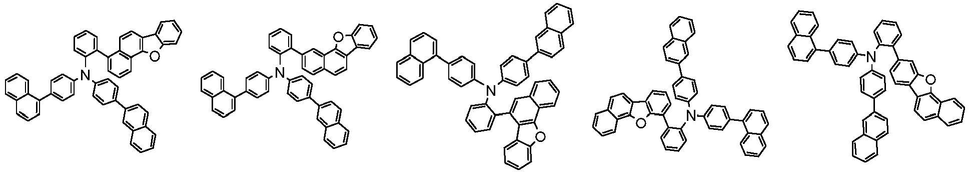

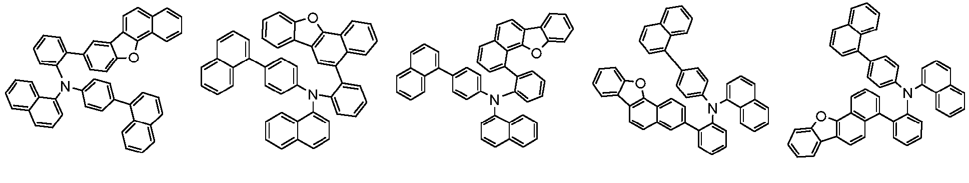

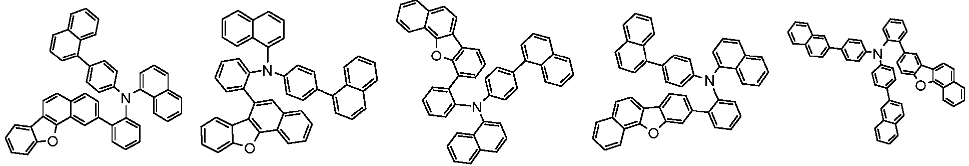

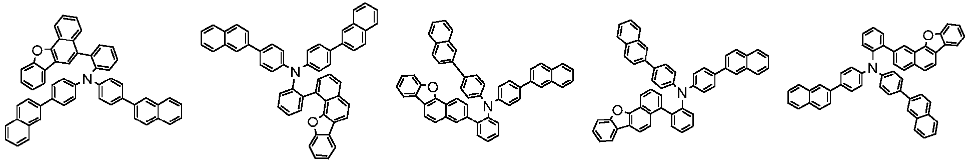

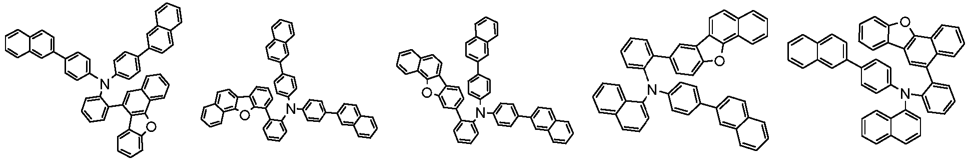

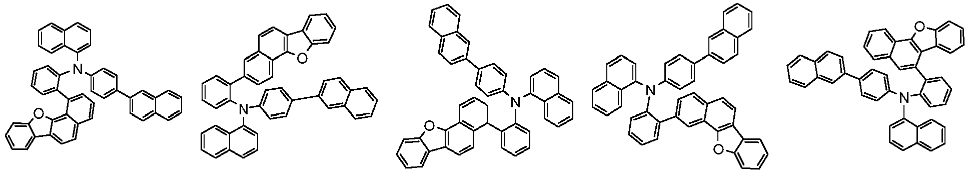

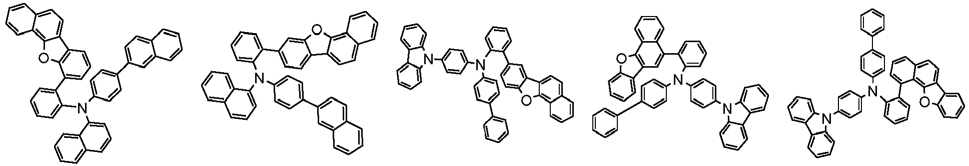

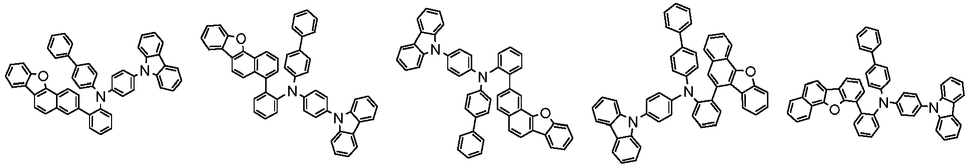

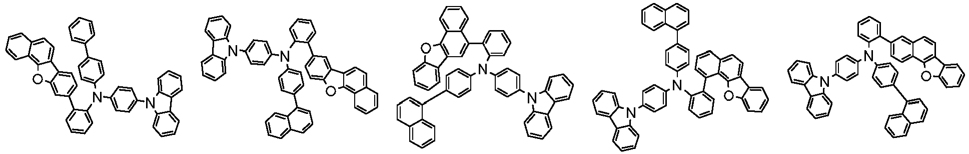

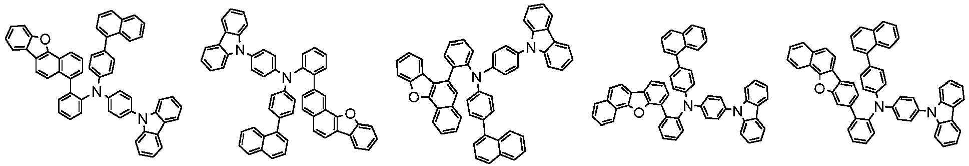

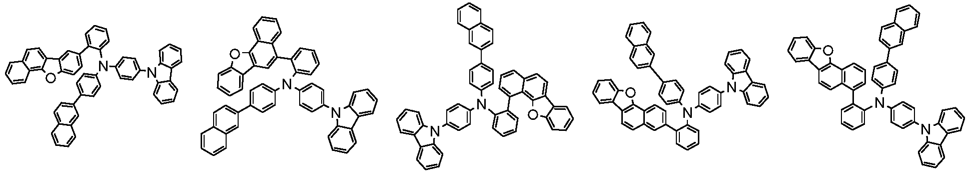

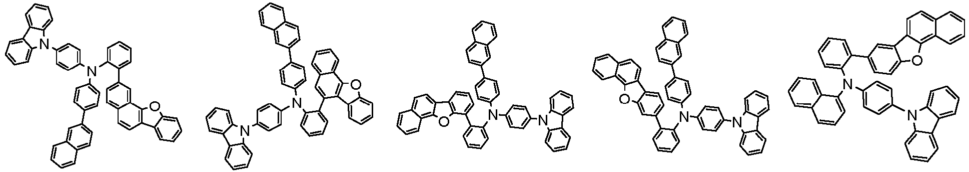

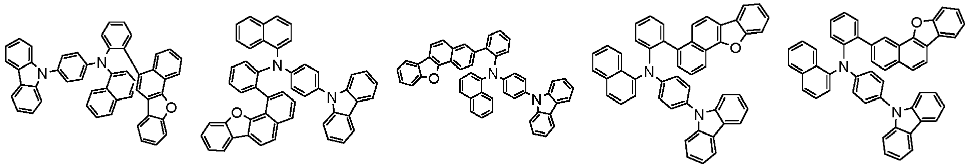

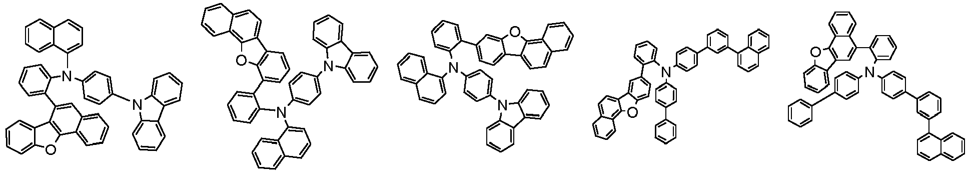

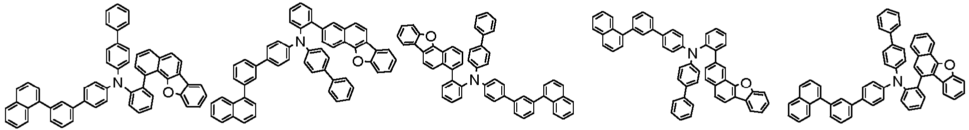

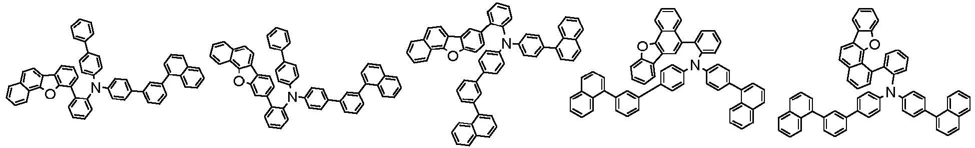

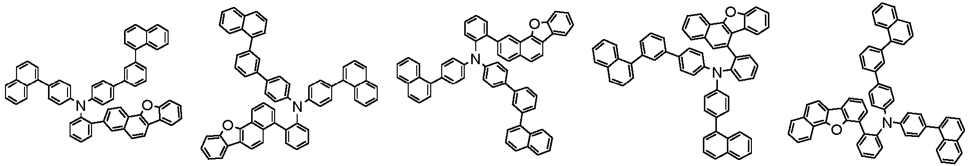

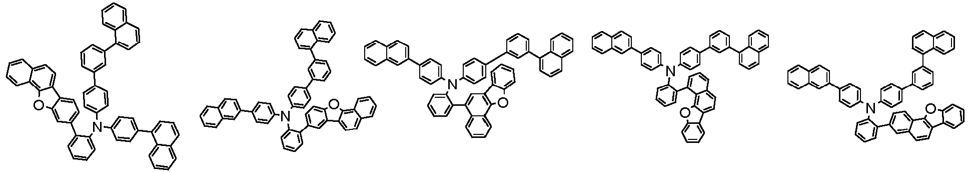

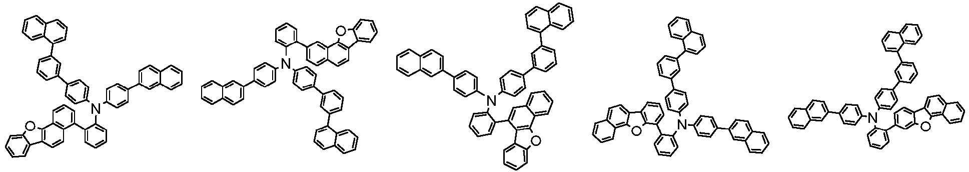

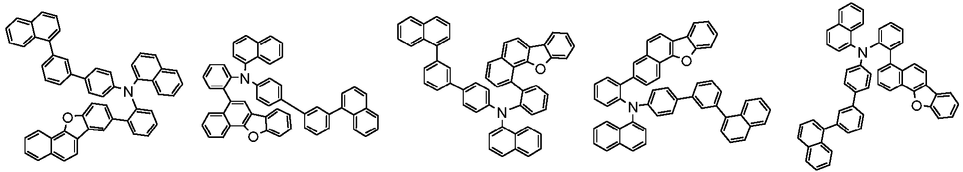

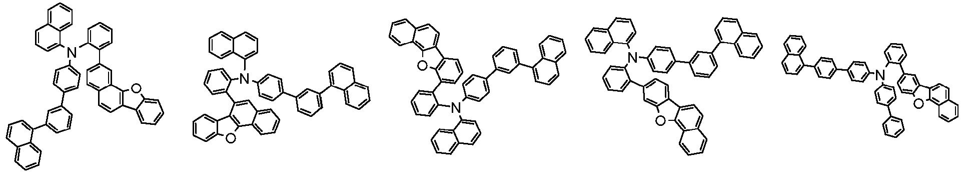

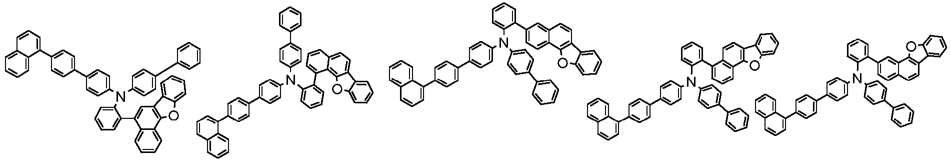

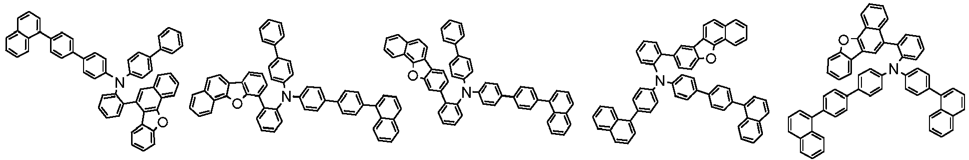

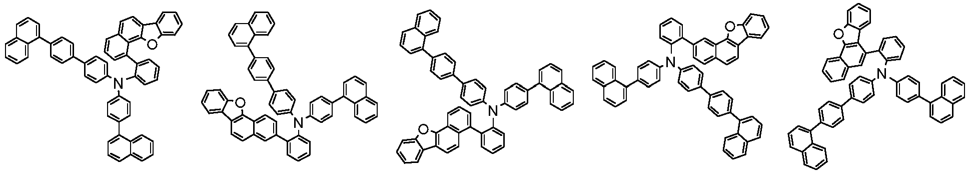

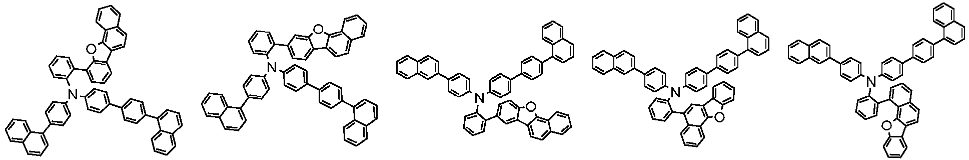

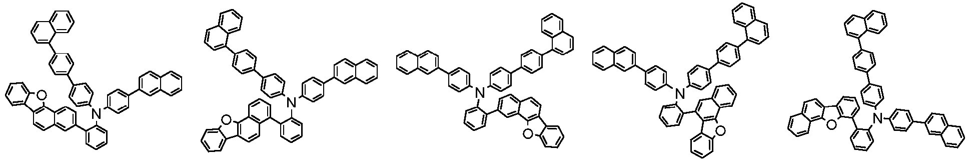

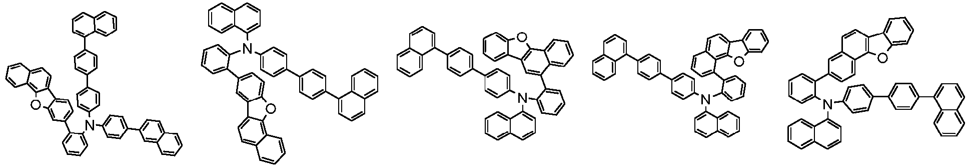

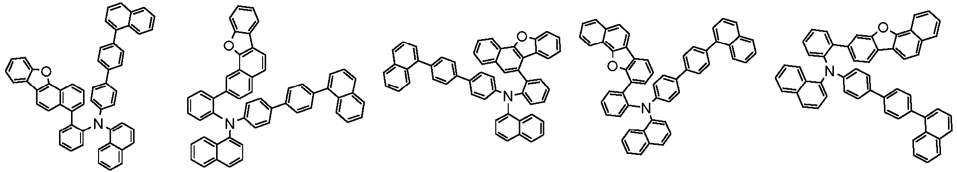

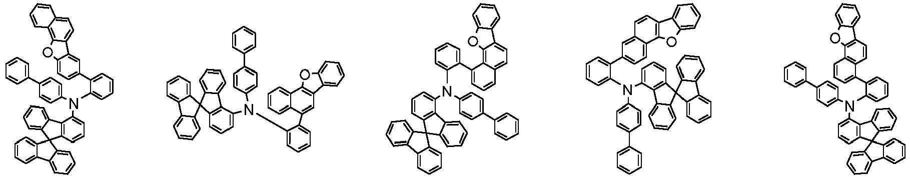

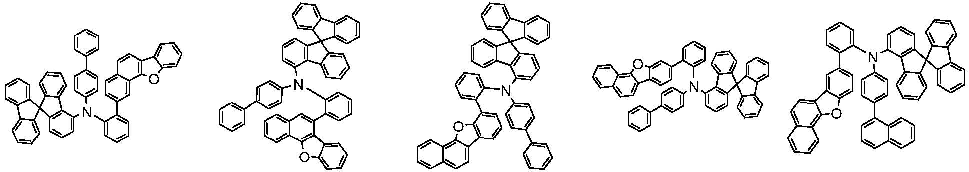

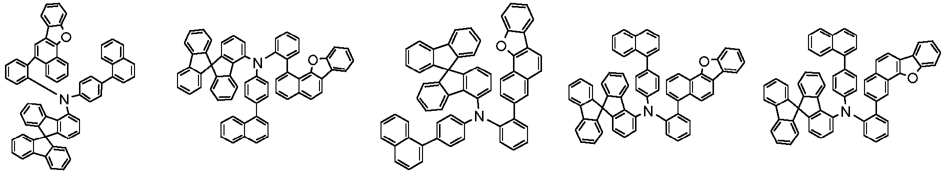

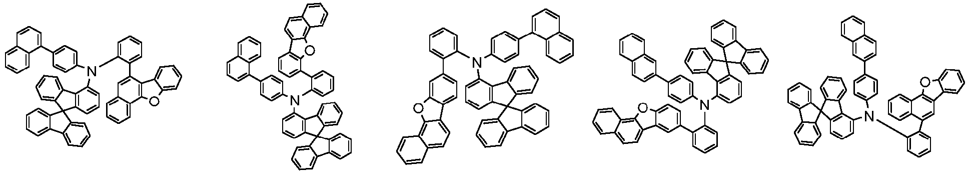

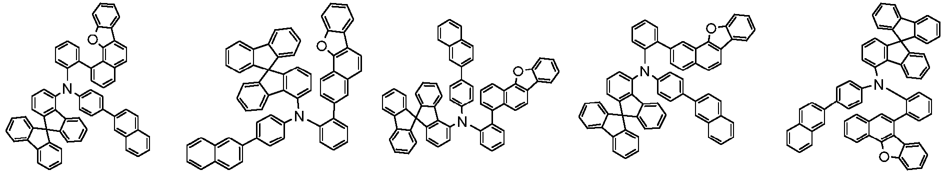

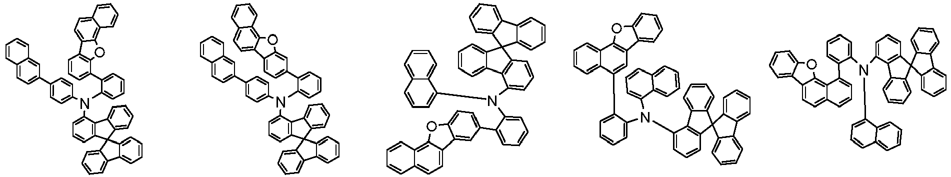

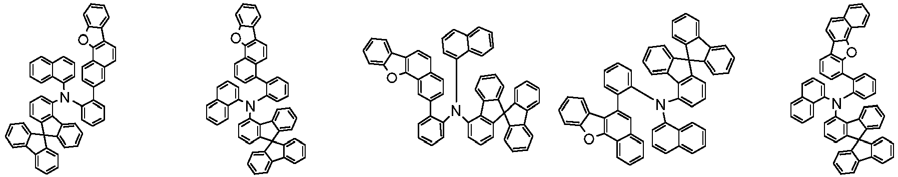

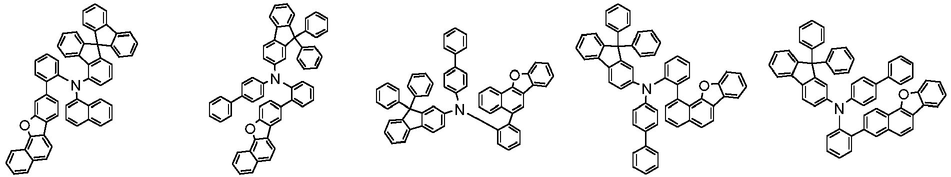

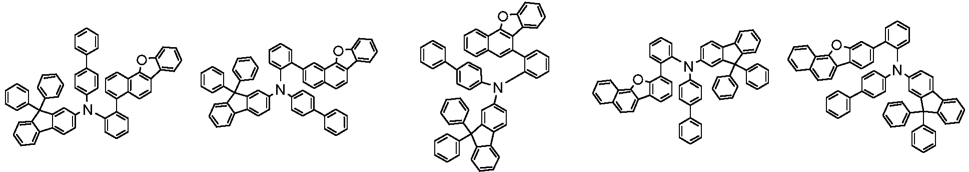

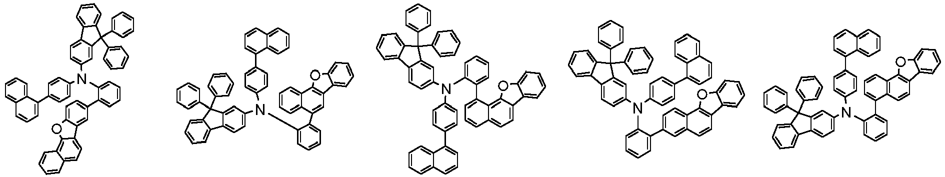

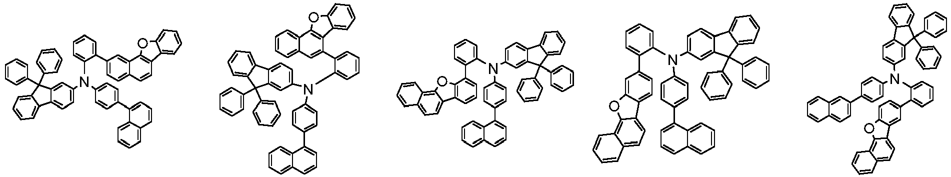

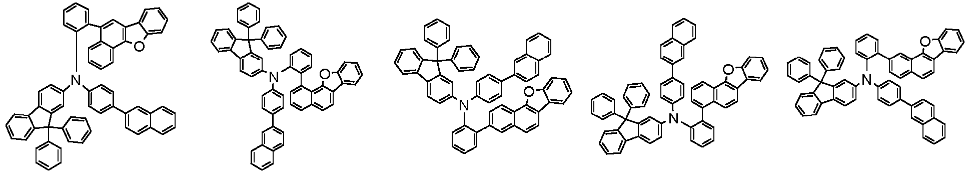

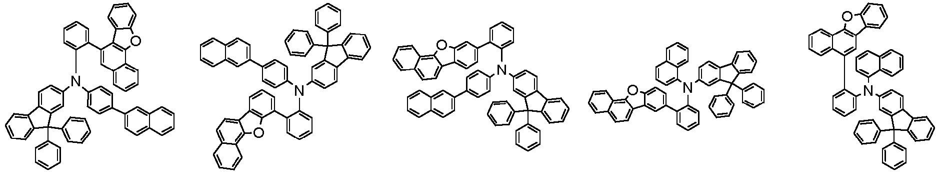

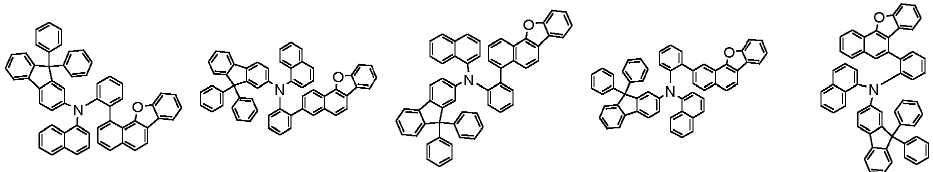

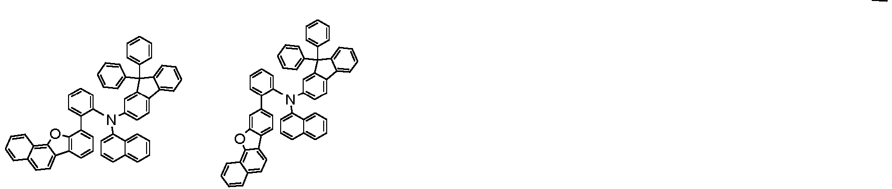

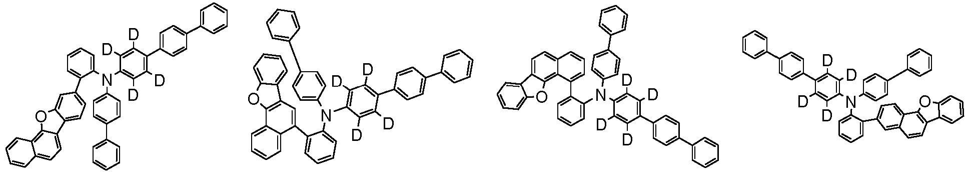

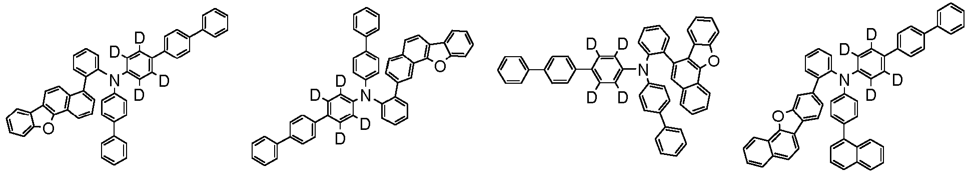

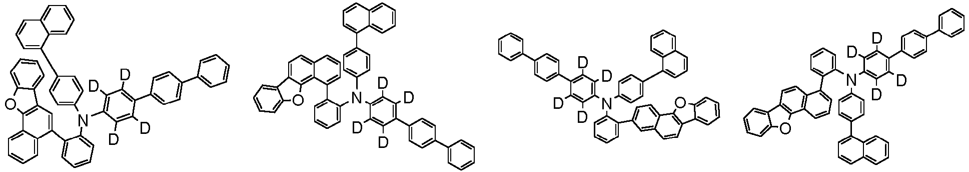

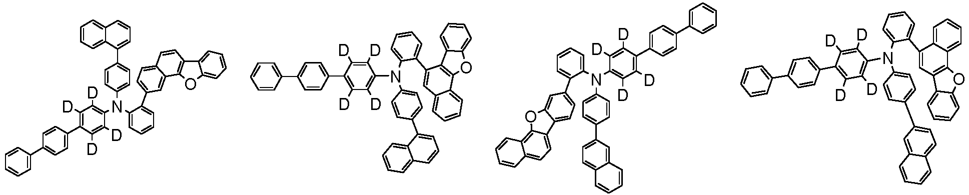

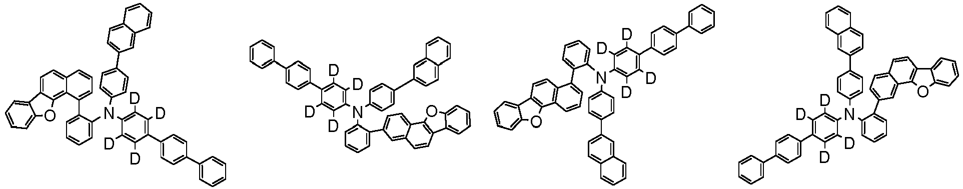

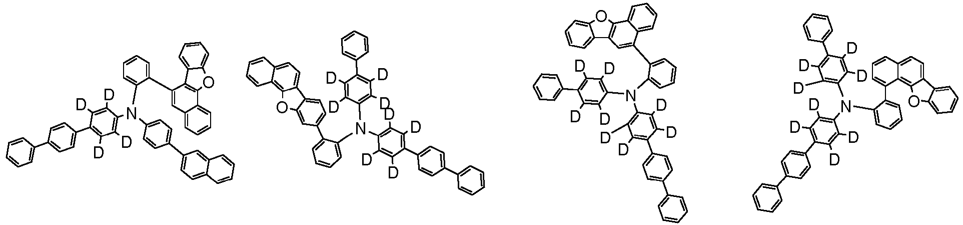

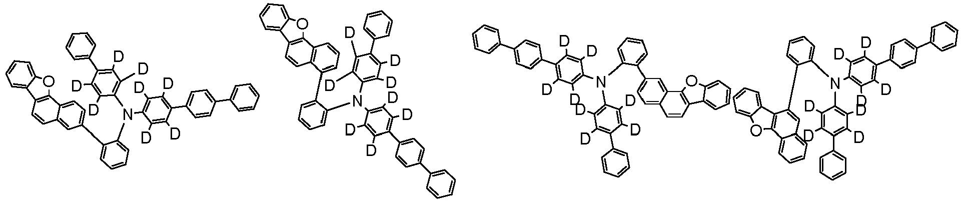

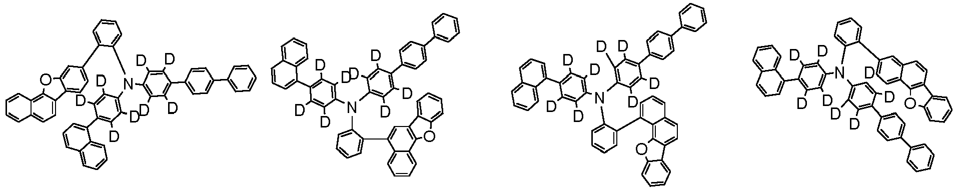

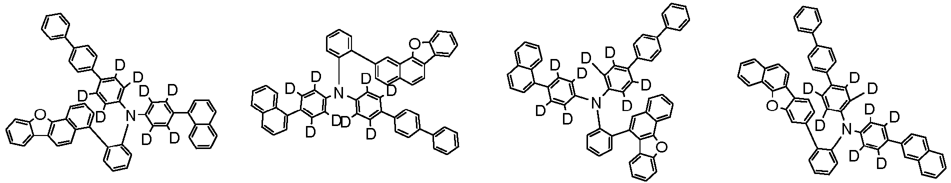

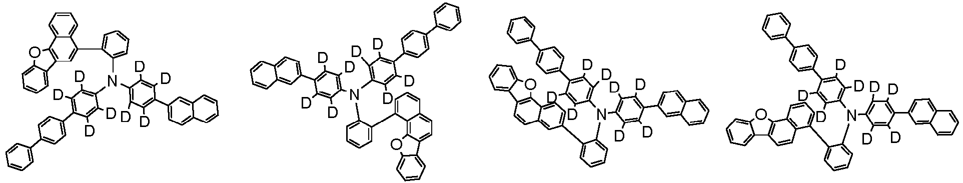

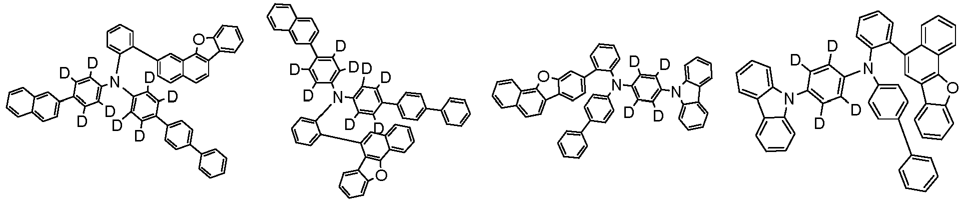

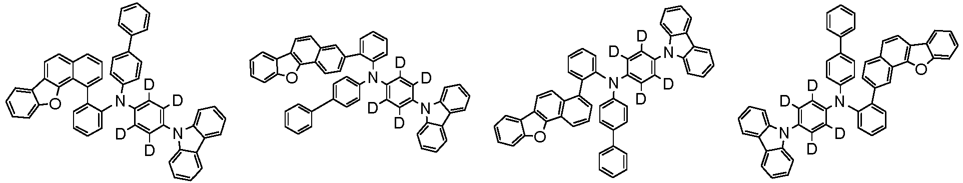

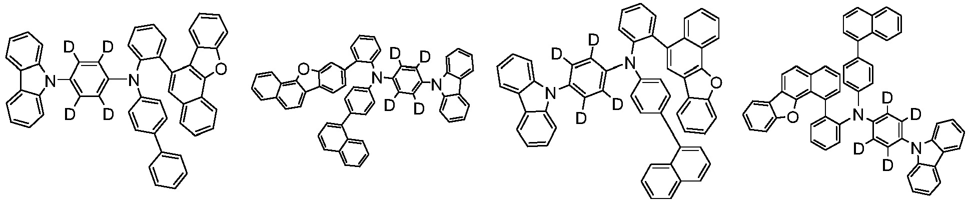

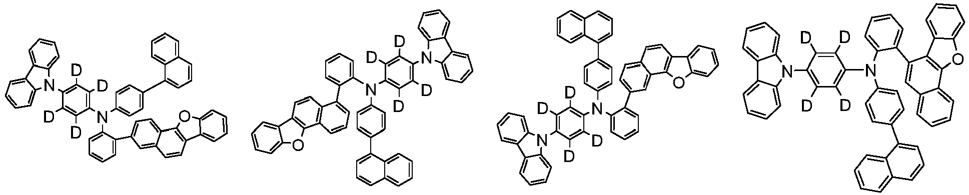

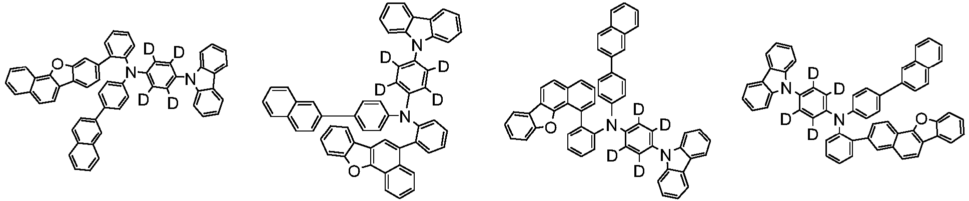

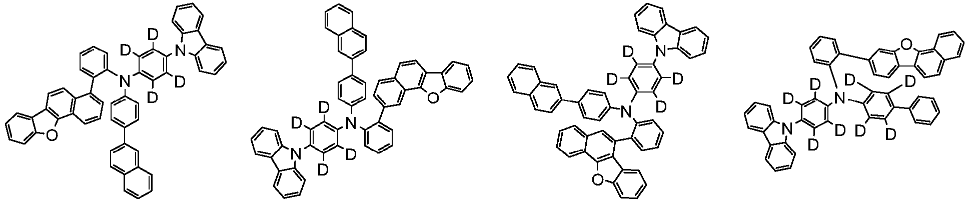

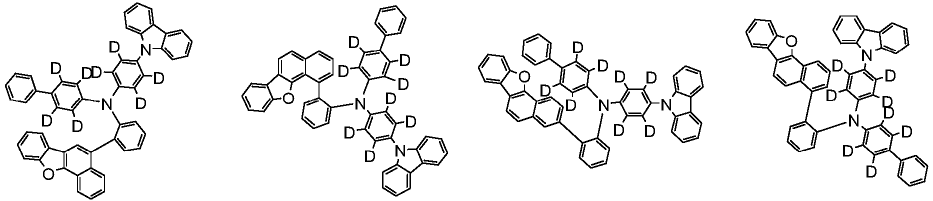

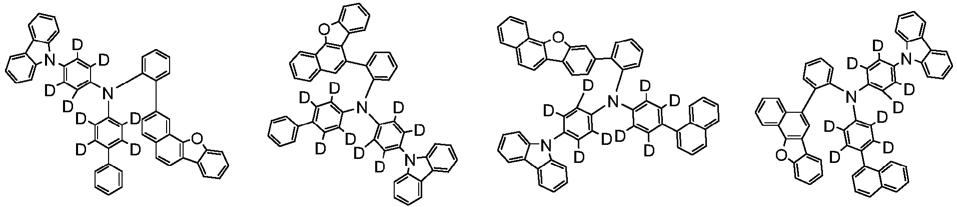

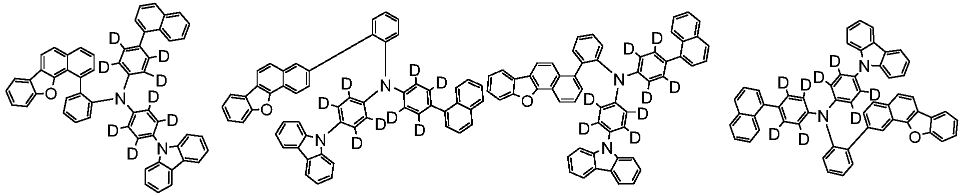

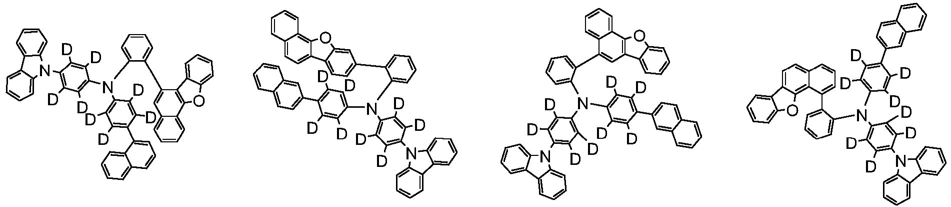

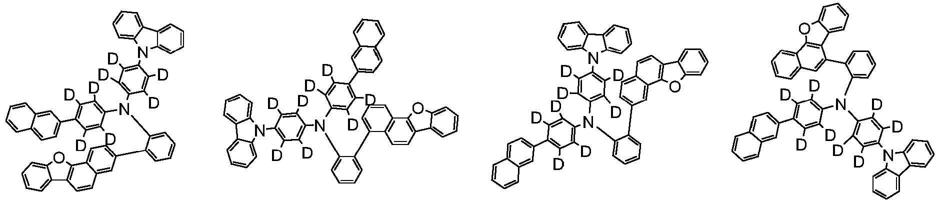

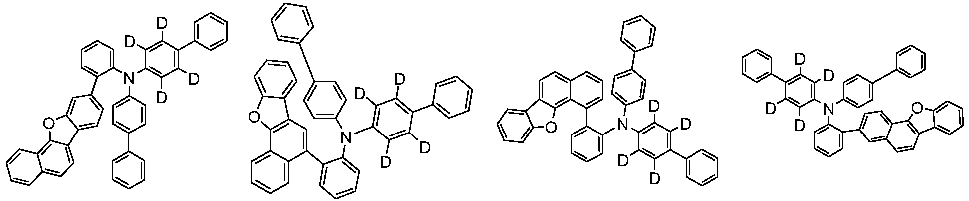

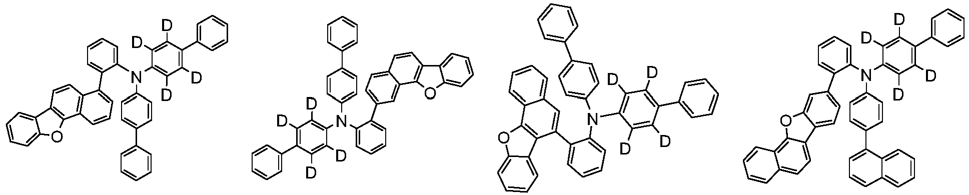

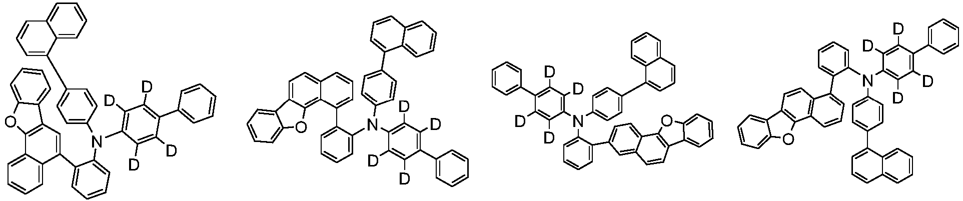

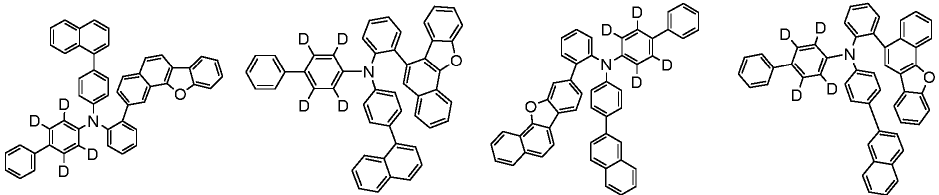

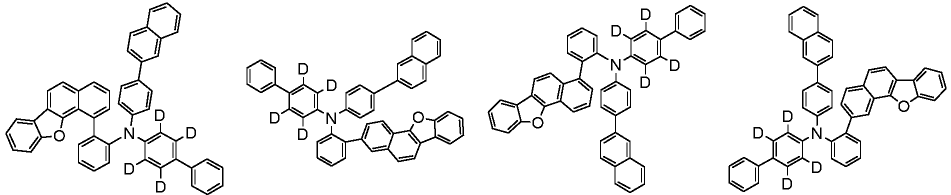

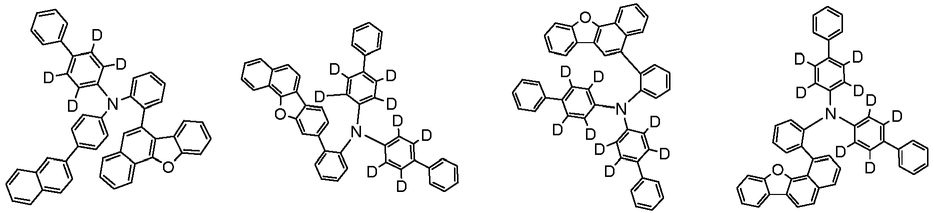

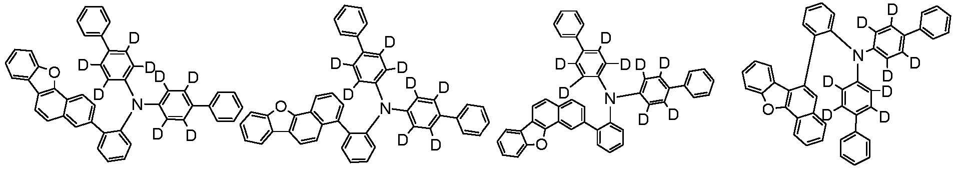

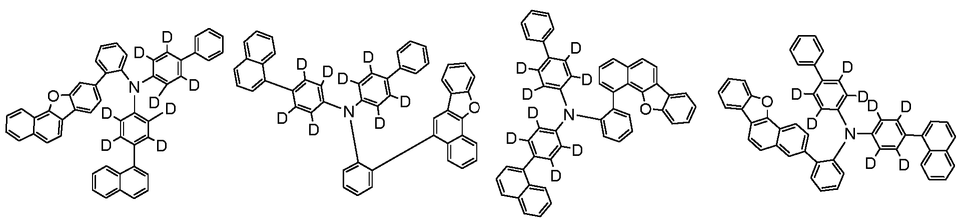

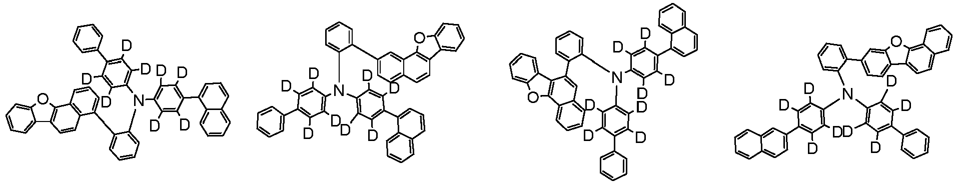

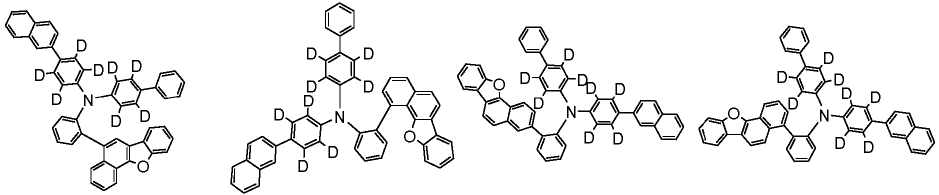

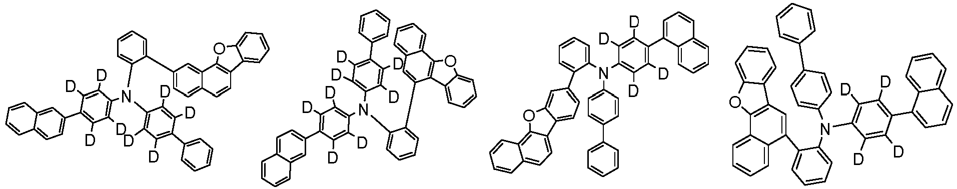

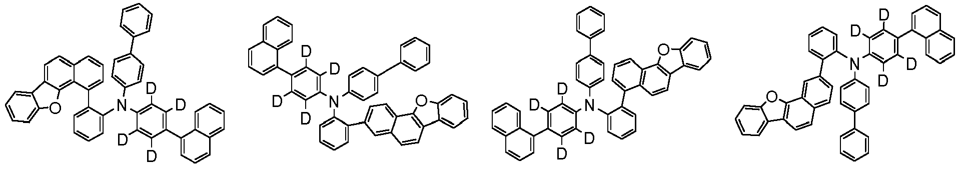

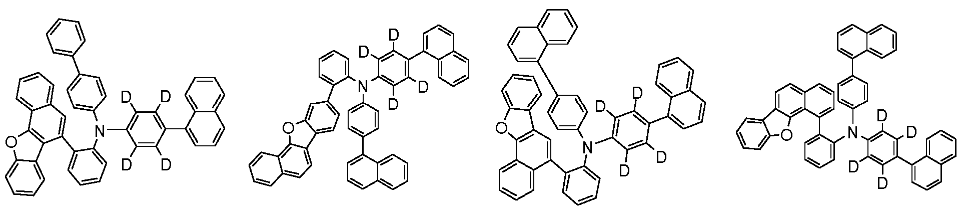

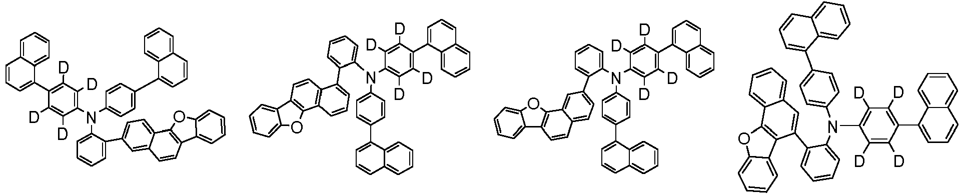

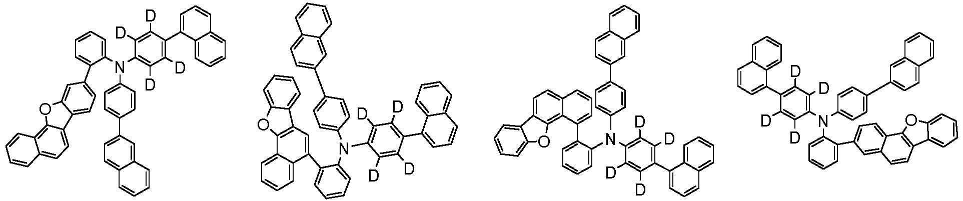

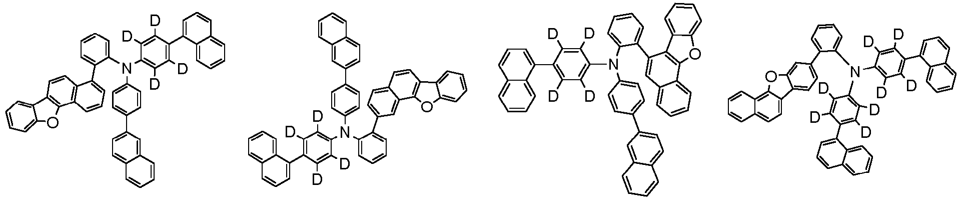

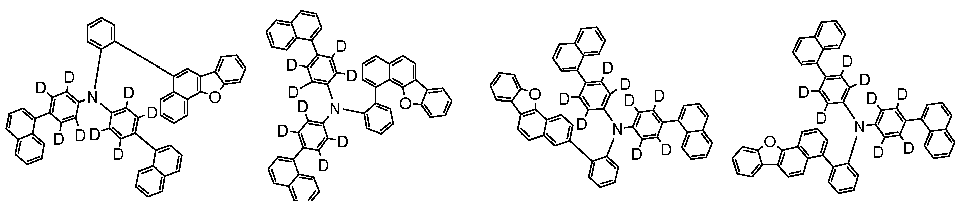

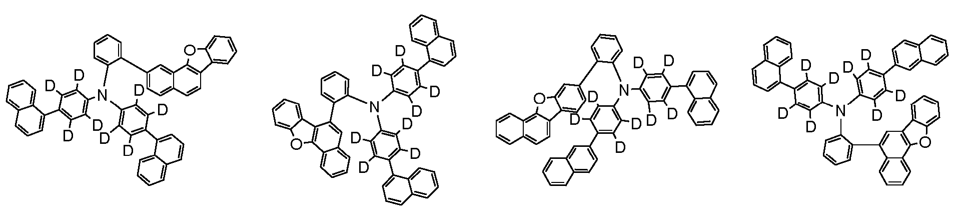

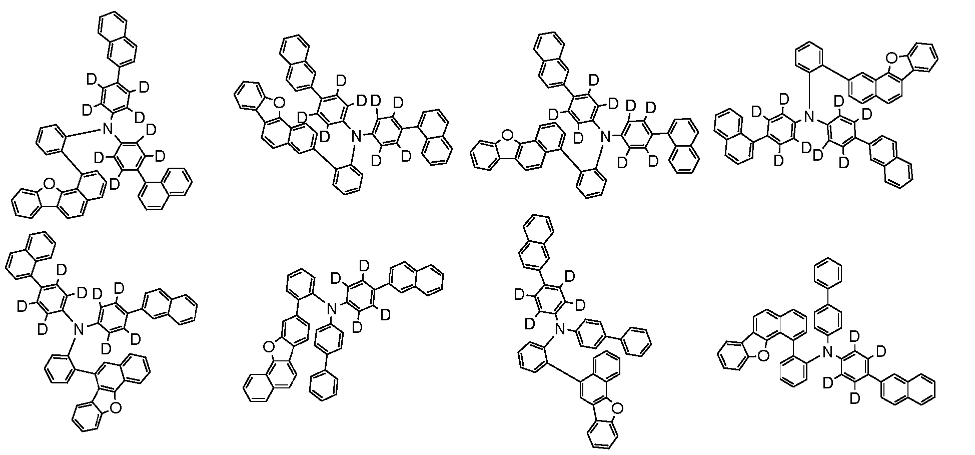

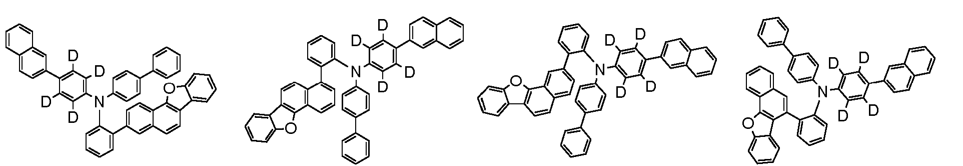

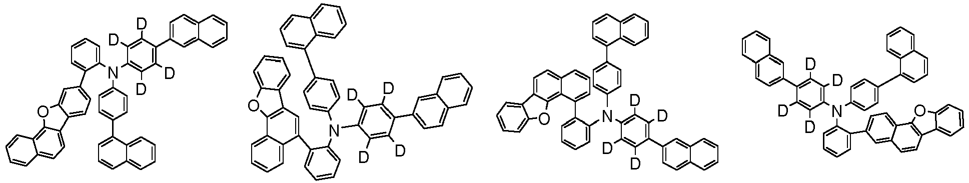

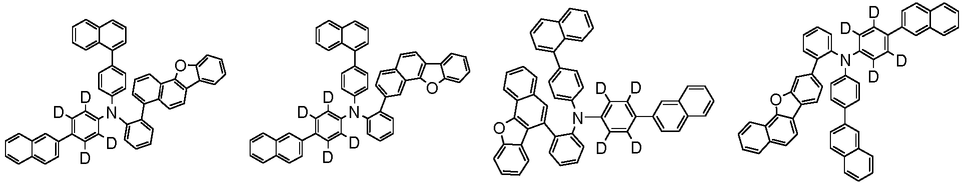

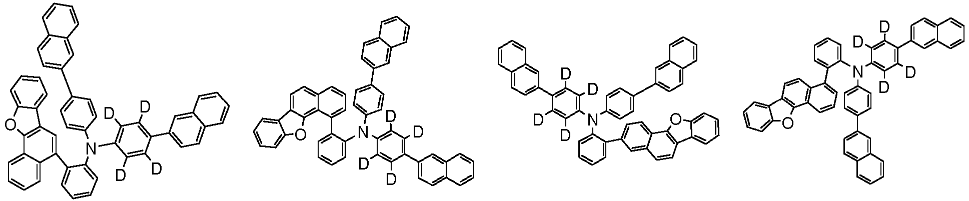

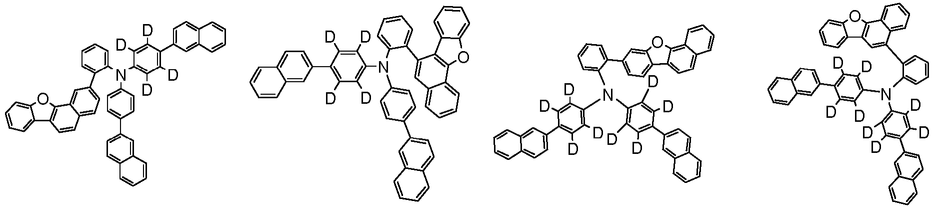

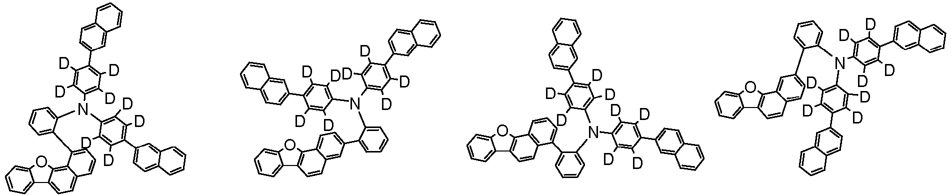

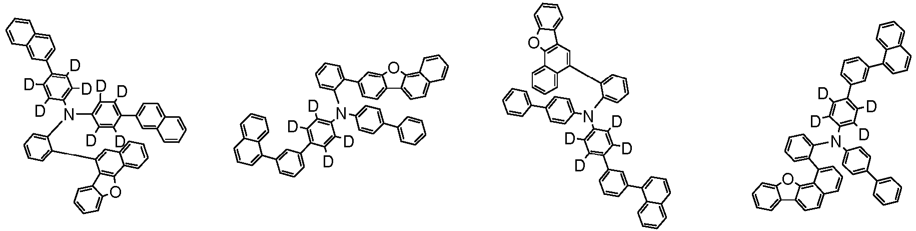

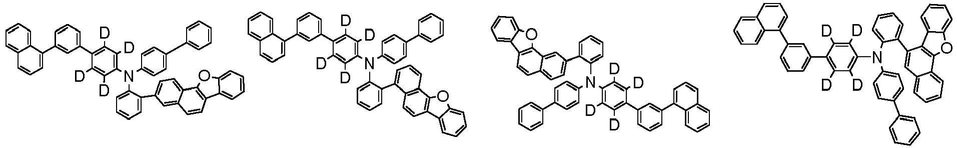

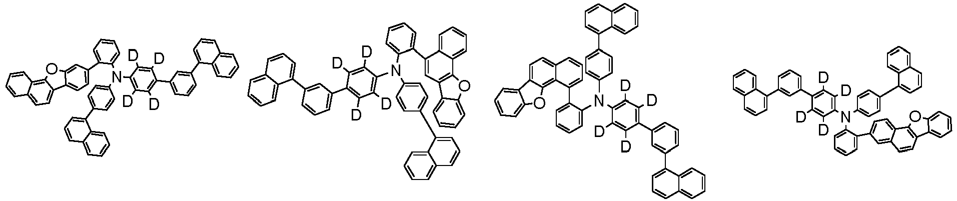

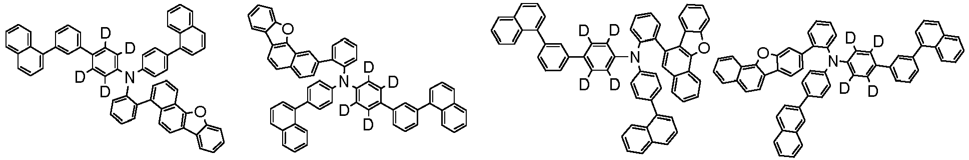

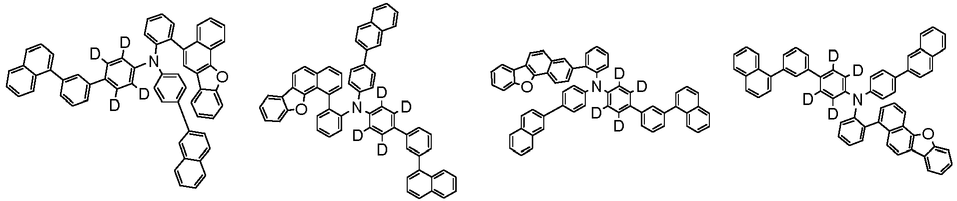

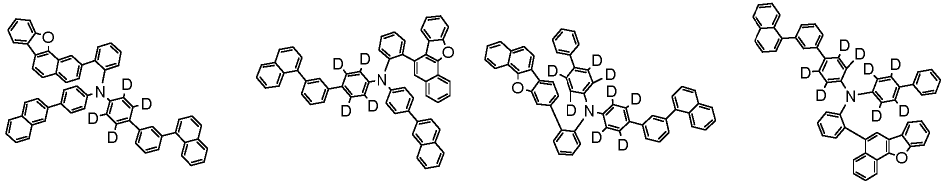

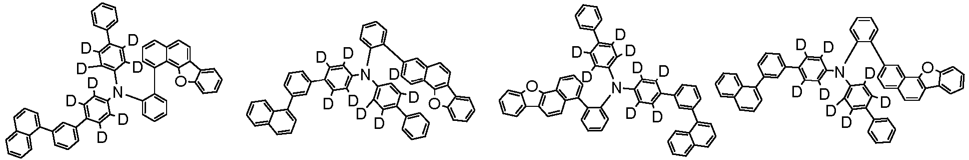

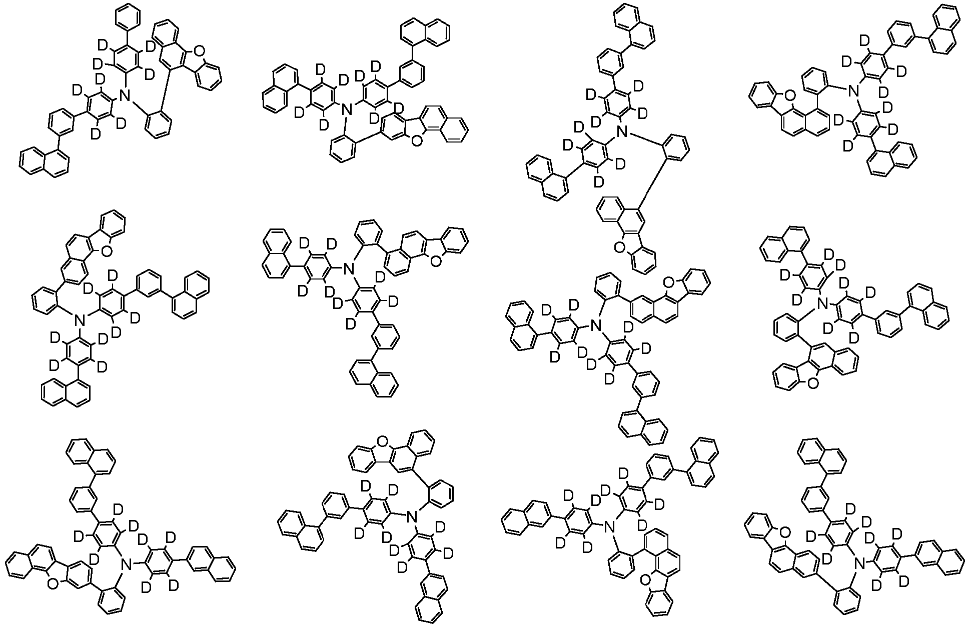

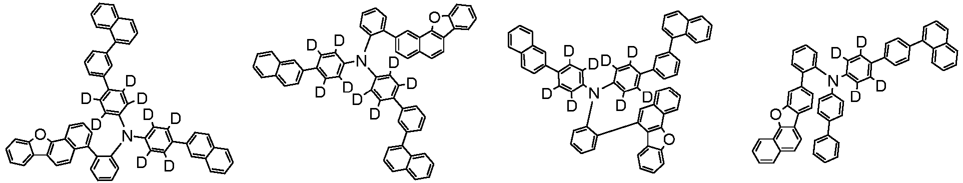

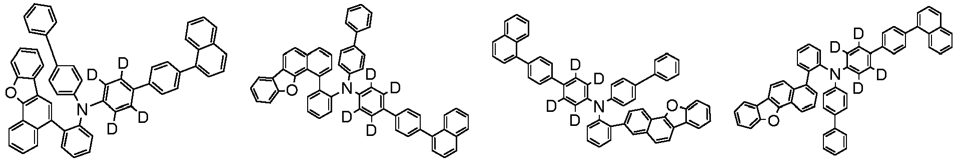

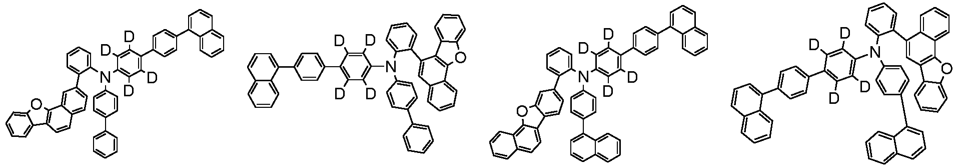

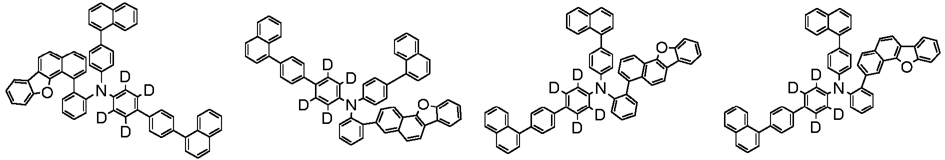

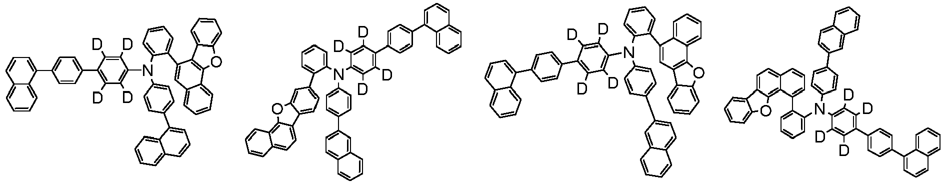

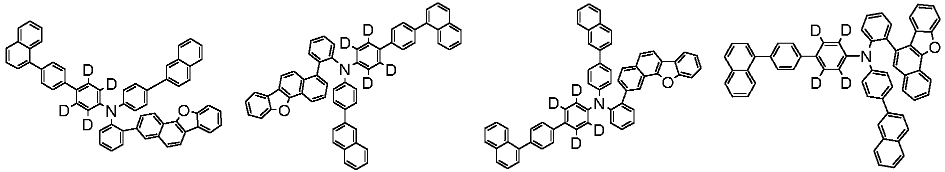

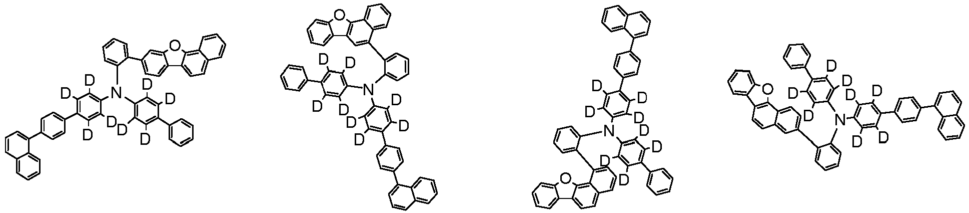

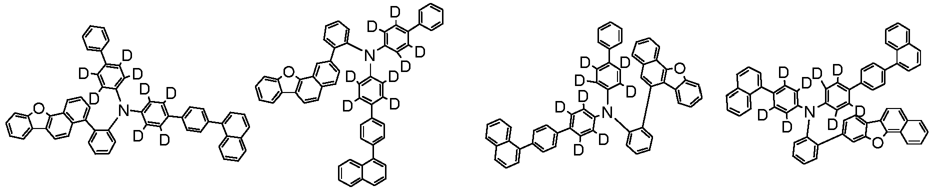

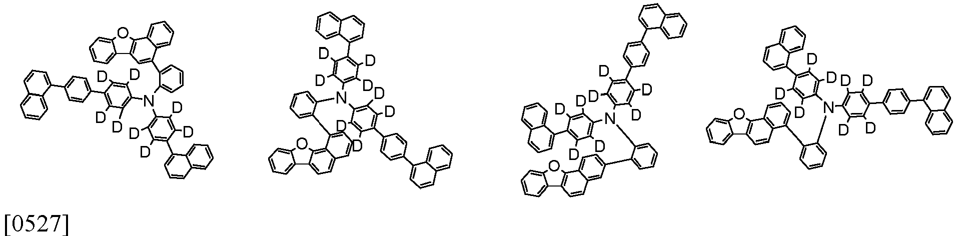

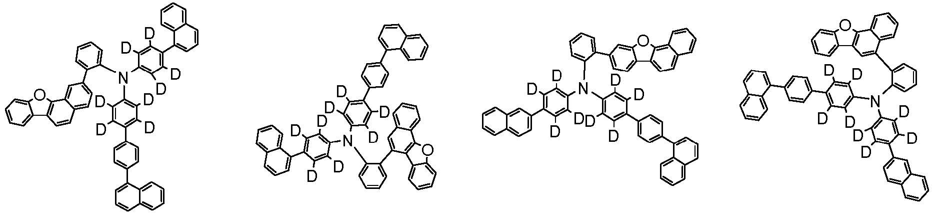

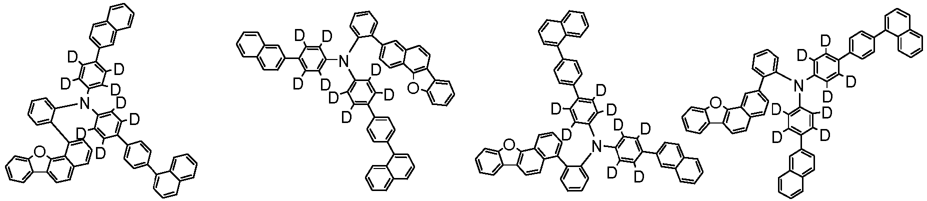

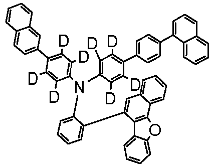

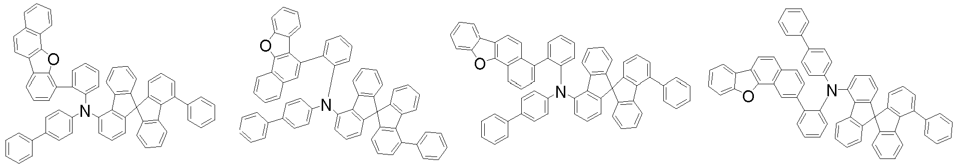

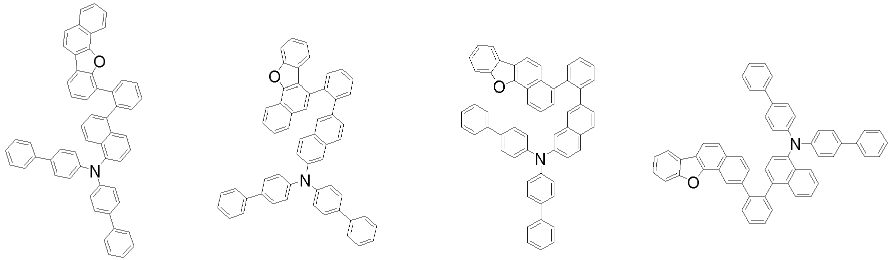

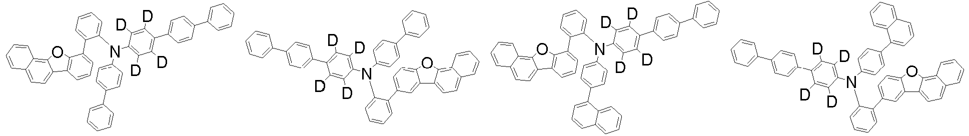

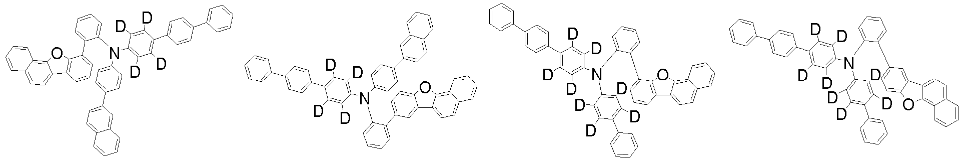

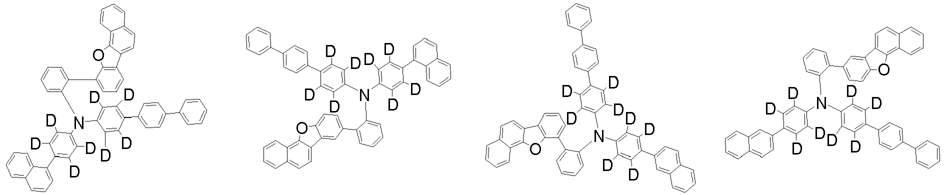

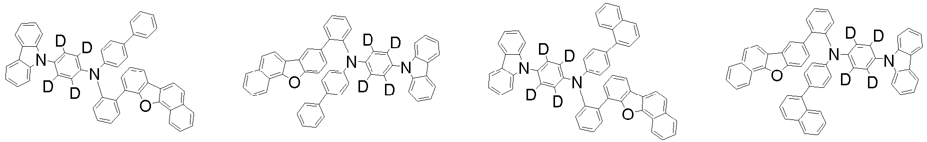

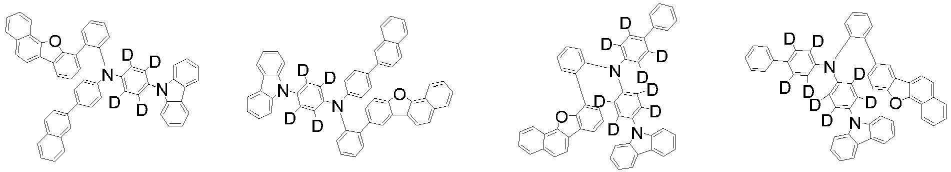

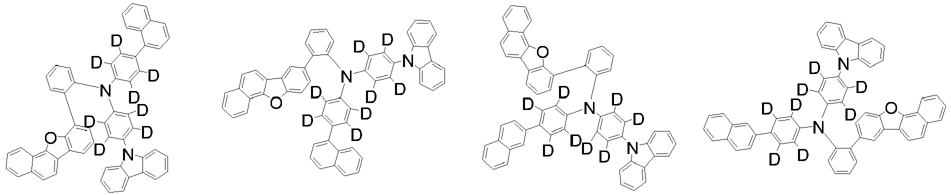

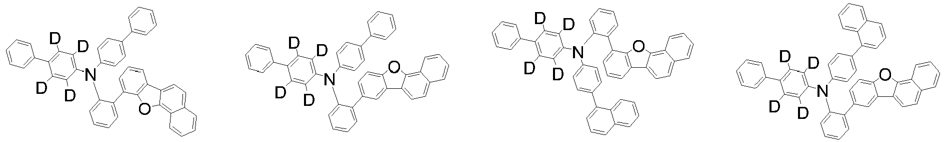

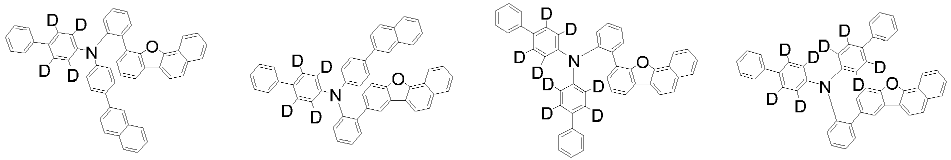

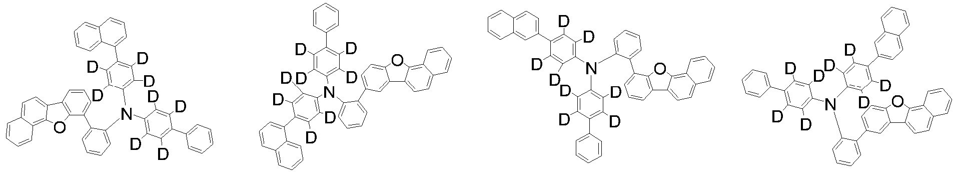

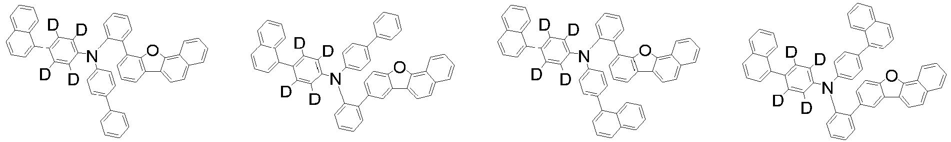

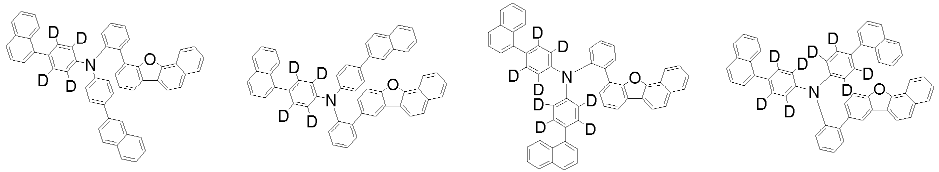

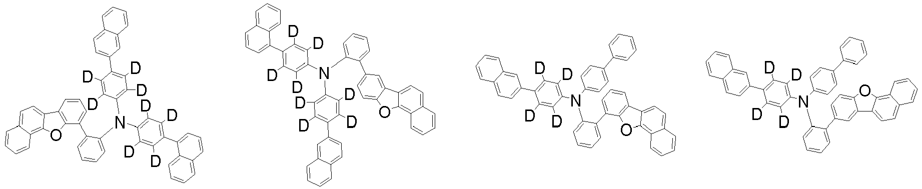

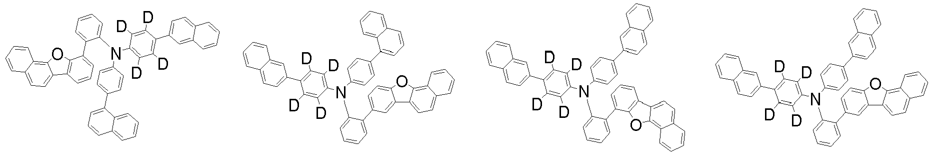

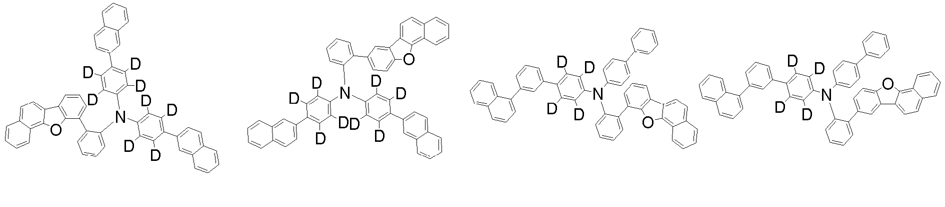

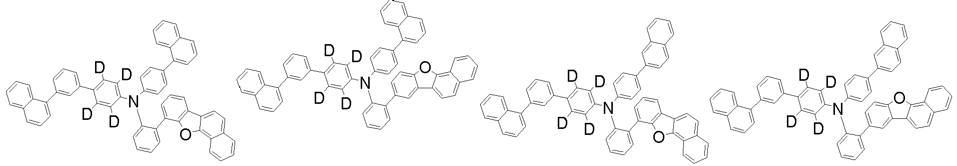

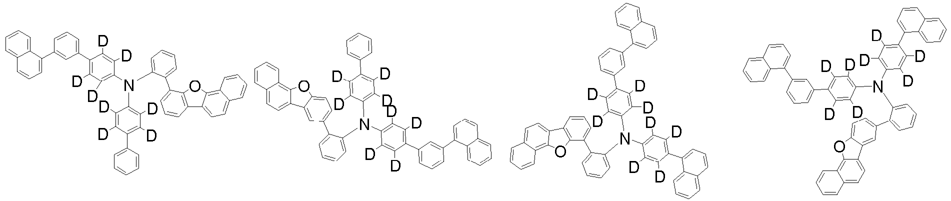

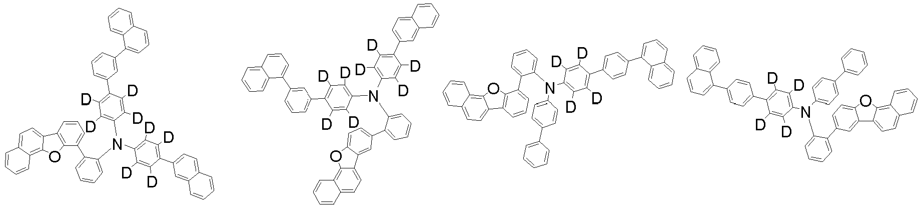

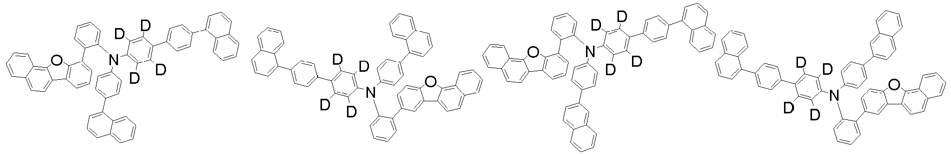

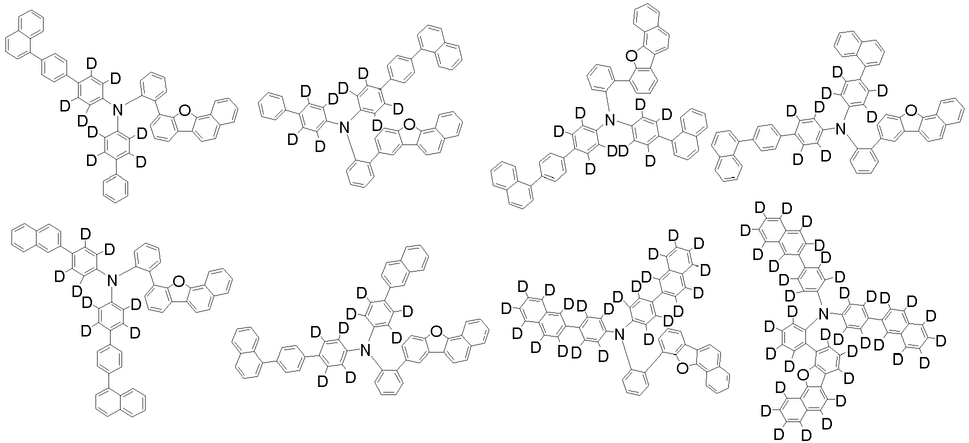

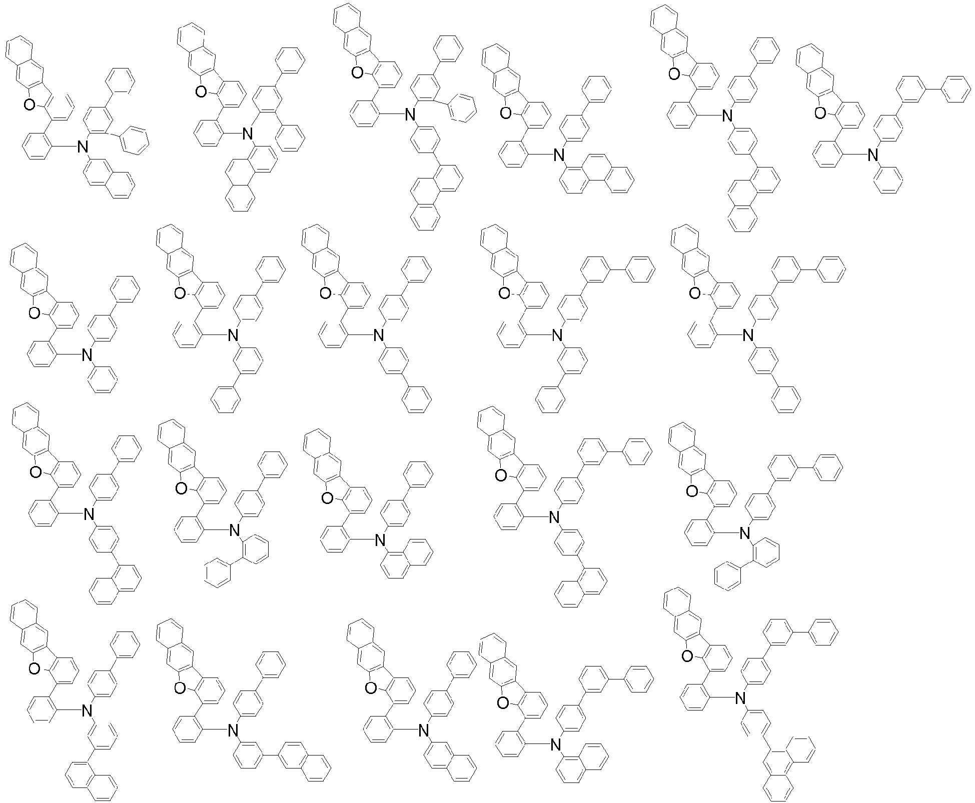

### [Material for Organic EL Devices]

The material for organic EL devices of the present invention contains the inventive compound. The content of the inventive compound in the material for organic EL devices is 1% by mass or more (including 100%), preferably 10% by mass or more (including 100%), more preferably 50% by mass or more (including 100%), further preferably 80% by mass or more (including 100%), and particularly preferably 90% by mass or more (including 100%). The material for organic EL devices of the present invention is useful for production of an organic EL device.

### Organic EL device

The organic EL device of the present invention includes an anode, a cathode, and organic layers disposed between the anode and the cathode. The organic layers include a light emitting layer and at least one of the organic layers contains the inventive compound.

Examples of the organic layer containing the inventive compound include, but not limited to, a hole transporting zone (hole injecting layer, hole transporting layer, electron blocking layer, exciton blocking layer, etc.) provided between the anode and the light emitting layer, the light emitting layer, a space layer, and an electron transporting zone (electron injecting layer, electron transporting layer, hole blocking layer, etc.) provided between the cathode and the light emitting layer. The inventive compound is preferably used as a material for the hole transporting zone or light emitting layer, more preferably as a material for the hole transporting zone, further preferably as a material for the hole injecting layer, hole transporting layer, electron blocking layer, or exciton blocking layer, and particularly preferably as a material for the hole injecting layer or hole transporting layer, of a fluorescent or phosphorescent EL device.

The organic EL device of the present invention may be a fluorescence or phosphorescence emission type monochromatic luminescent device, a fluorescence/phosphorescence hybrid type white luminescent device, a simple type having a single light emitting unit, or a tandem type having two or more light emitting units, and is preferably a fluorescence emission type device. Here, the "light emitting unit" refers to a minimum unit that includes organic layers, at least one of which is a light emitting layer, and that emits light by recombination of injected holes and injected electrons.

An example of a typical device configuration of the simple type organic EL device is the following device configuration.

### (1) Anode/Light Emitting Unit/Cathode

The light emitting unit may be a multilayer type having two or more phosphorescence emitting layers and fluorescence emitting layers, and in this case, a space layer may be provided between the light emitting layers for the purpose of preventing excitons generated in the phosphorescence emitting layers from diffusing into the fluorescence emitting layers. A typical layer configuration of the simple type light emitting unit is shown below. The layers in parentheses are optional.
(a) (hole injecting layer/) hole transporting layer/fluorescence emitting layer/electron transporting layer (/electron injecting layer)
(b) (hole injecting layer/) hole transporting layer/first fluorescence emitting layer/second fluorescence emitting layer/ electron transporting layer (/electron injecting layer)
(c) (hole injecting layer/) hole transporting layer/phosphorescence emitting layer/space layer/fluorescence emitting layer/electron transporting layer (/electron injecting layer)
(d) (hole injecting layer/) hole transporting layer/first phosphorescence emitting layer/second phosphorescence emitting layer/space layer/fluorescence emitting layer/electron transporting layer (/electron injecting layer)
(e) (hole injecting layer/) hole transporting layer/phosphorescence emitting layer/space layer/first fluorescence emitting layer/second fluorescence emitting layer/electron transporting layer (/electron injecting layer)
(f) (hole injecting layer/) hole transporting layer/electron blocking layer/fluorescence emitting layer/electron transporting layer (/electron injecting layer)
(g) (hole injecting layer/) hole transporting layer/exciton blocking layer/fluorescence emitting layer/electron transporting layer (/electron injecting layer)
(h) (hole injecting layer/) first hole transporting layer/second hole transporting layer/fluorescence emitting layer/electron transporting layer (/electron injecting layer)
(i) (hole injecting layer/) first hole transporting layer/second hole transporting layer/fluorescence emitting layer/first electron transporting layer/second electron transporting layer (/electron injecting layer)
(j) (hole injecting layer/) hole transporting layer/fluorescence emitting layer/hole blocking layer/electron transporting layer (/electron injecting layer)
(k) (hole injecting layer/) hole transporting layer/fluorescence emitting layer/exciton blocking layer/electron transporting layer (/electron injecting layer)
(l) (hole injecting layer/) first hole transporting layer/second hole transporting layer/first fluorescence emitting layer/second fluorescence emitting layer/first electron transporting layer/second electron transporting layer (/electron injecting layer)
(m) (hole injecting layer/) first hole transporting layer/second hole transporting layer/third hole transporting layer/first fluorescence emitting layer/second fluorescence emitting layer/first electron transporting layer/second electron transporting layer (/electron injecting layer)
(n) (hole injecting layer/) first hole transporting layer/second hole transporting layer/third hole transporting layer/fluorescence emitting layer/first electron transporting layer/second electron transporting layer (/electron injecting layer)

The phosphorescence or fluorescence emitting layers described above can exhibit luminescent colors different from one another. A specific example of the layer configuration is a layer configuration in the light emitting unit (f) of (hole injecting layer/) hole transporting layer/first phosphorescence emitting layer (red light emission)/second phosphorescence emitting layer (green light emission)/space layer/fluorescence emitting layer (blue light emission)/electron transporting layer.

An electron blocking layer may be appropriately provided between each light emitting layer and a hole transporting layer or a space layer. In addition, a hole blocking layer may be appropriately provided between each light emitting layer and an electron transporting layer. By providing an electron blocking layer or hole blocking layer, electrons or holes can be trapped inside the light emitting layer to increase the probability of recombination of charges in the light emitting layer, thus enhancing the light emitting efficiency.

An example of a typical device configuration of the tandem type organic EL device is the following device configuration.

### (2) Anode/First Light Emitting Unit/Intermediate Layer/Second Light Emitting Unit/Cathode

Here, the first light emitting unit and the second light emitting unit can each be independently selected, for example, from the light emitting units as described above.

The intermediate layer is generally also referred to as intermediate electrode, intermediate conductive layer, charge generating layer, electron withdrawing layer, connection layer, or intermediate insulating layer, and a known material configuration in which electrons are supplied to the first light emitting unit and holes are supplied to the second light emitting unit can be used.

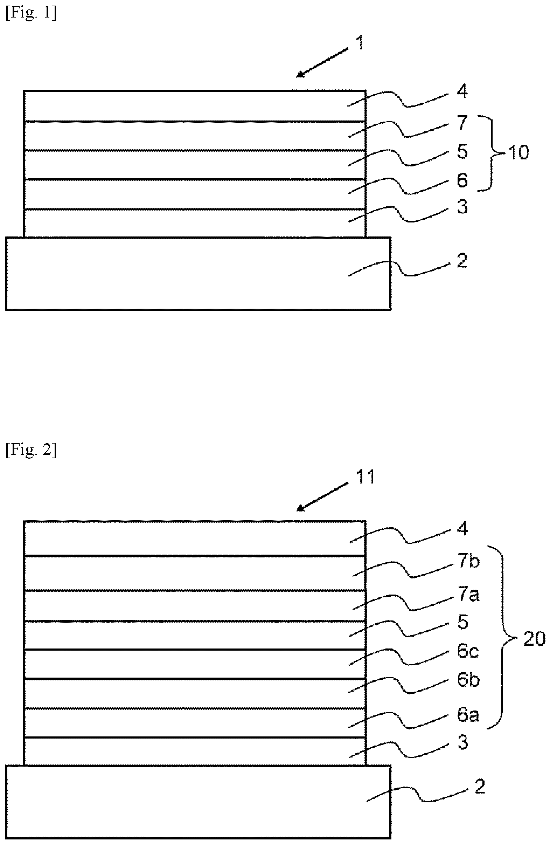

Fig. 1 is a schematic diagram illustrating an example of the configuration of the organic EL device of the present invention. An organic EL device 1 includes a substrate 2, an anode 3, a cathode 4, and a light emitting unit 10 disposed between the anode 3 and the cathode 4. The light emitting unit 10 includes a light emitting layer 5. The organic EL device 1 includes a hole transporting zone 6 (hole injecting layer, hole transporting layer, etc.) between the light emitting layer 5 and the anode 3, and an electron transporting zone 7 (electron injecting layer, electron transporting layer, etc.) between the light emitting layer 5 and the cathode 4. In addition, an electron blocking layer (not shown) may be provided on the anode 3 side of the light emitting layer 5 and a hole blocking layer (not shown) may be provided on the cathode 4 side of the light emitting layer 5. This enables to trap electrons or holes in the light emitting layer 5 to further increase the efficiency of generating excitons in the light emitting layer 5.

Fig. 2 is a schematic diagram illustrating another configuration of the organic EL device of the present invention. An organic EL device 11 includes the substrate 2, the anode 3, the cathode 4, and a light emitting unit 20 disposed between the anode 3 and the cathode 4. The light emitting unit 20 includes the light emitting layer 5. A hole transporting zone disposed between the anode 3 and the light emitting layer 5 is formed of a hole injecting layer 6a, a first hole transporting layer 6b, and a second hole transporting layer 6c. An electron transporting zone disposed between the light emitting layer 5 and the cathode 4 is formed of a first electron transporting layer 7a and a second electron transporting layer 7b.

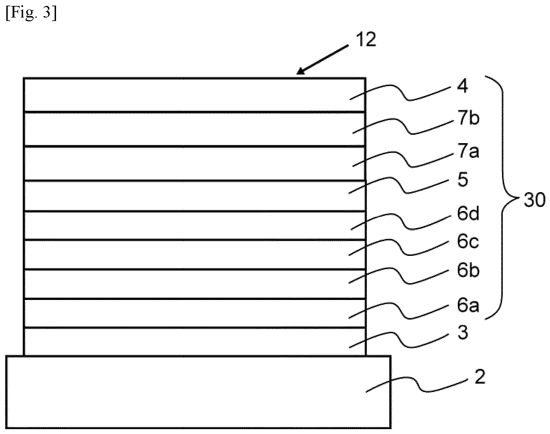

Fig. 3 is a schematic diagram illustrating another configuration of the organic EL device of the present invention. An organic EL device 12 includes the substrate 2, the anode 3, the cathode 4, and a light emitting unit 30 disposed between the anode 3 and the cathode 4. The light emitting unit 30 includes the light emitting layer 5. A hole transporting zone disposed between the anode 3 and the light emitting layer 5 is formed of the hole injecting layer 6a, the first hole transporting layer 6b, the second hole transporting layer 6c, and a third hole transporting layer 6d. An electron transporting zone disposed between the light emitting layer 5 and the cathode 4 is formed of the first electron transporting layer 7a and the second electron transporting layer 7b.

In the present invention, a host combined with a fluorescent dopant material (fluorescence emitting material) is referred to as a fluorescent host, and a host combined with a phosphorescent dopant material is referred to as a phosphorescent host. The fluorescent host and the phosphorescent host are not distinguished only by the molecular structure. In other words, the phosphorescent host means a material that forms a phosphorescence emitting layer containing a phosphorescent dopant, and does not mean that it cannot be used as a material that forms a fluorescence emitting layer. The same applies to the fluorescent host.

### Substrate

The substrate is used as a support of the organic EL device. As the substrate, for example, a plate of glass, quartz, or a plastic can be used. A flexible substrate may be used. An example of the flexible substrate is a plastic substrate of polycarbonate, polyarylate, polyethersulfone, polypropylene, polyester, polyvinyl fluoride, or polyvinyl chloride. An inorganic vapor deposition film can also be used.

### Anode

For the anode formed on the substrate, a metal, an alloy, an electrically conductive compound, a mixture thereof, or the like that has a high work function (specifically 4.0 eV or more) is preferably used. Specific examples thereof include indium oxide-tin oxide (ITO: indium tin oxide), indium oxide-tin oxide containing silicon or silicon oxide, indium oxide-zinc oxide, indium oxide containing tungsten oxide and zinc oxide, and graphene. Other examples include gold (Au), platinum (Pt), nickel (Ni), tungsten (W), chromium (Cr), molybdenum (Mo), iron (Fe), cobalt (Co), cupper (Cu), palladium (Pd), titanium (Ti), and nitride of the metals (for example, titanium nitride).

A film of such a material is generally formed by a spattering method. For example, indium oxide-zinc oxide can be formed by a spattering method by using a target obtained by adding to indium oxide 1 to 10wt% of zinc oxide based on the indium oxide, and indium oxide containing tungsten oxide and zinc oxide can be formed by a spattering method by using a target obtained by adding to indium oxide 0.5 to 5wt% of tungsten oxide and 0.1 to 1wt% of zinc oxide based on the indium oxide. Alternatively, a film of such a material may be produced by a vacuum vapor deposition method, a coating method, an inkjet method, a spin-coating method, or the like.

### Hole transporting zone

As mentioned above, the organic layer may include a hole transporting zone between the anode and the light emitting layer. The hole transporting zone is composed of a hole injecting layer, a hole transporting layer, an electron blocking layer, and the like. Preferably, the hole transporting zone contains the inventive compound. It is preferable that at least one layer of these layers constituting the hole transporting layer contains the inventive compound, and it is particularly preferable that the hole transporting layer contains the inventive compound.

The hole injecting layer formed in contact with the anode is, regardless of the work function of the anode, formed by using a material in which hole injection is easy, and thus a material that is generally used as an electrode material (for example, a metal, an alloy, an electrically conductive compound, or a mixture thereof, or an element belonging to the group 1 or 2 in the periodic table) can be used.

An element belonging to the group 1 or 2 in the periodic table which is a material having a small work function, specifically, an alkali metal, such as lithium (Li) or cesium (Cs), or an alkaline earth metal, such as magnesium (Mg), calcium (Ca), or strontium (Sr), and an alloy containing them (for example, MgAg, AlLi), a rare earth metal, such as europium (Eu) or ytterbium (Yb), or an alloy containing them, or the like, can be used. When the anode is formed using an alkali metal, an alkaline earth metal, or an alloy containing them, a vacuum vapor deposition method or a spattering method can be adopted. When silver paste or the like is used, a coating method, an inkjet method, or the like can be adopted.

### Hole injecting layer

The hole injecting layer is a layer containing a material having a high hole injecting capability (hole injecting material), and is formed between the anode and the light emitting layer, or between a hole transporting layer, if present, and the anode.

As a hole injecting material other than the inventive compound, molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, or the like can be used.

Other examples of a material for the hole injecting layer include aromatic amine compounds, such as 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N- f 4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazol-3-yl)-N-phenylamino]-9-phenylcarbazole (abbreviation: PCzPCA1), 3,6-bis[N-(9-phenylcarbazol-3-yl)-N-phenylamino]-9-phenylcarbazole (abbreviation: PCzPCA2), and 3-[N-(1-naphtyl)-N-(9-phenylcarbazol-3-yl)amino]-9-phenylcarbazole (abbreviation: PCzPCN1), which are low-molecular weight organic compounds.

A high-molecular weight compound (oligomer, dendrimer, polymer, or the like) can also be used. Examples of the high-molecular weight compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). A high-molecular weight compound with an acid, such as poly(3,4-ethylenedioxythiophene)/poly(styrenesulfonic acid) (PEDOT/PSS) or polyaniline/poly(styrenesulfonic acid) (PAni/PSS), added thereto can also be used.

Furthermore, an acceptor material, such as a hexaazatriphenylene (HAT) compound represented by the following formula (K), is also preferably used. 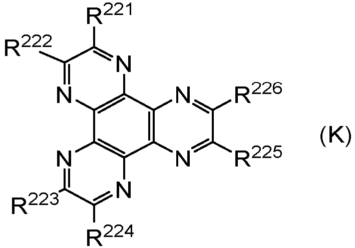 (In the formula, R²²¹ to R²²⁶ each independently represent a cyano group, -CONH₂, a carboxy group, or -COOR²²⁷ (R²²⁷ represents an alkyl group having 1 to 20 carbon atoms or a cycloalkyl group having 3 to 20 carbon atoms). Adjacent two selected from R^{ZZI} and R²²², R²²³and R²²⁴, and R²²⁵and R²²⁶ may be bonded to each other to form a group represented by -CO-O-CO-.)

Examples of R¹²⁷ include a methyl group, an ethyl group, an n-propyl group, an isopropyl group, an n-butyl group, an isobutyl group, a t-butyl group, a cyclopentyl group, and a cyclohexyl group.

### Hole transporting layer

The hole transporting layer is a layer containing a material having a high hole transporting capability (hole transporting material), and is formed between the anode and the light emitting layer or between a hole injecting layer, if present, and the light emitting layer. The inventive compound may be used, for the hole transporting layer, alone or in combination with the following compound.

The hole transporting layer may have a monolayer structure or a multilayer structure including two or more layers. For example, the hole transporting layer may have a two-layer structure including a first hole transporting layer (anode side) and a second hole transporting layer (cathode side). That is, the hole transporting zone may include a first hole transporting layer on the anode side and a second hole transporting layer on the cathode side. Further, the hole transporting layer may have a three-layer structure including, in order from the anode side, a first hole transporting layer, a second hole transporting layer, and a third hole transporting layer. That is, the third hole transporting layer may be arranged between the second hole transporting layer and the light emitting layer.

In an aspect of the present invention, the hole transporting layer of the monolayer structure is preferably adjacent to the light emitting layer, and the hole transporting layer that is the nearest to the cathode in the multilayer structure, for example, the second hole transporting layer in the two-layer structure and the third hole transporting layer in the three-layer structure are preferably adjacent to the light emitting layer. In another embodiment of the present invention, an electron blocking layer as described later or the like may be interposed between the hole transporting layer of the monolayer structure and the light emitting layer or between the hole transporting layer that is the nearest to the light emitting layer in the multilayer structure and the light emitting layer.

When the hole transporting layer has a two-layer structure, at least one of the first hole transporting layer and the second hole transporting layer contains the inventive compound. That is, the inventive compound is contained only in the first hole transporting layer, or only in the second hole transporting layer, or in both the first hole transporting layer and the second hole transporting layer. In an embodiment of the present invention, the inventive compound is preferably contained in the second hole transporting layer. That is, it is preferable that the inventive compound is contained only in the second hole transporting layer, or the inventive compound is contained in the first hole transporting layer and the second hole transporting layer.

When the hole transporting layer has a three-layer structure, at least one of the first to third hole transporting layers contains the inventive compound. That is, the inventive compound is contained only in one layer selected from the first to third hole transporting layers (only in the first hole transporting layer, only in the second hole transporting layer, or only in the third hole transporting layer), only in two layers selected from the first to third hole transporting layers (only in the first hole transporting layer and the second hole transporting layer, only in the first hole transporting layer and the third hole transporting layer, or only in the second hole transporting layer and the third hole transporting layer), or in all of the first to third hole transporting layers.

In an aspect of the present invention, the inventive compound is preferably contained in the third hole transporting layer. That is, it is preferable that the inventive compound is contained only in the third hole transporting layer, or the inventive compound is contained in the third hole transporting layer and one or both of the first hole transporting layer and the second hole transporting layer.

In an embodiment of the present invention, the inventive compound contained in each of the hole transporting layers is preferably a light hydrogen form from the viewpoint of the production cost. The light hydrogen form refers to the inventive compound in which all the hydrogen atoms are a light hydrogen atom.

Accordingly, the present invention includes an organic EL device in which one or both of the first hole transporting layer and the second hole transporting layer (in the case of a two-layer structure), or at least one of the first to third hole transporting layers contain the inventive compound essentially constituted only of light hydrogen forms. "The inventive compound essentially constituted only of light hydrogen forms" means that the content of the light hydrogen form based on the total amount of the inventive compound is 90% by mole or more, preferably 95% by mole or more, and more preferably 99% by mole or more (each including 100%).

As a material for the hole transporting layer other than the inventive compound, for example, an aromatic amine compound, a carbazole derivative, an anthracene derivative can be used.

Examples of the aromatic amine compound include 4,4'-bis[N-(1-naphtyl)-N-phenylamino]biphenyl (abbreviation: NPB) and N,N'-bis(3-methylphenyl)-N,N'-diphenyl-[1,1'-biphenyl]-4,4'-diamine (abbreviation: TPD), 4-phenyl-4'-(9-phenylfluoren-9-yl)triphenylamine ( abbreviation: BAFLP), 4,4'-bis[N-(9,9-dimethylfluoren-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluoren-2-yl)-N-phenylamino]biphenyl (abbreviation: BSPB). The compounds have a hole mobility of 10⁻⁶ cm²/Vs or more.

Examples of the carbazole derivative include 4,4'-di(9-carbazolyl)biphenyl (abbreviation: CBP), 9-[4-(9-carbazolyl)phenyl]-10-phenylanthracene (abbreviation: CzPA), and 9-phenyl-3-[4-(10-phenyl-9-anthryl)phenyl]-9H-carbazole (abbreviation: PCzPA).

Examples of the anthracene derivative include 2-t-butyl-9,10-di(2-naphtyl)anthracene (abbreviation: t-BuDNA), 9,10-di(2-naphtyl)anthracene (abbreviation: DNA), and 9,10-diphenylanthracene (abbreviation: DPAnth).

A high-molecular weight compound, such as poly(N-vinylcarbazole) (abbreviation: PVK) or poly(4-vinyltriphenylamine) (abbreviation: PVTPA), can also be used.

However, compounds other than those mentioned above may be used when they are compounds higher in the hole transporting capability rather than in the electron transporting capability.

In an organic EL device of the present invention having a hole transporting layer having a two-layer structure, the first hole transporting layer preferably contains one or more compounds represented by the following formula (11) or formula (12).

In an organic EL device of the present invention having a hole transporting layer having a three-layer structure, it is preferable that one or both of the first hole transporting layer and the second hole transporting layer contain one or more compounds represented by the following formula (11) or (12).

In an organic EL device of the present invention having a hole transporting layer having an n-layer structure (n is an integer of 4 or more), it is preferable that at least one layer of the first hole transporting layer to the (n-1)th hole transporting layer contains one or more compounds represented by the following formula (11) or formula (12). 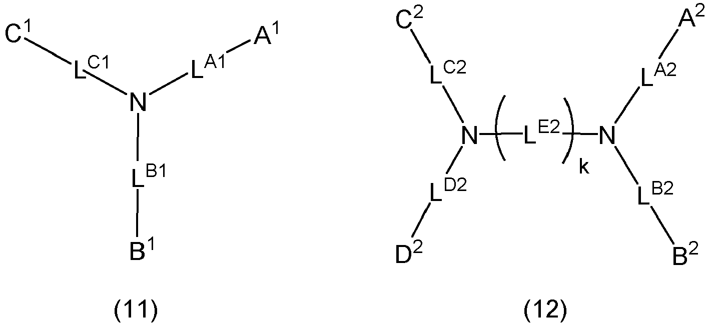 In the above formula (11) and formula (12),
L^{A1}, L^{B1}, L^{C1}, L^{A2}, L^{B2}, L^{C2} and L^{D2} are each independently a single bond, a substituted or unsubstituted arylene group having 6 to 50 ring carbon atoms, or a substituted or unsubstituted divalent heterocyclic group having 5 to 50 ring atoms,
k is 1, 2, 3 or 4,
when k is 1, L^{E2} is a substituted or unsubstituted arylene group having 6 to 50 ring carbon atoms, or a substituted or unsubstituted divalent heterocyclic group having 5 to 50 ring atoms,
when k is 2, 3 or 4, two, three, or four L^{E2}'s are the same as or different from each other,
when k is 2, 3 or 4, a plurality of L^{E2}'s are bonded to each other to form a substituted or unsubstituted monocyclic ring, or are bonded to each other to form a substituted or unsubstituted condensed ring, or are not bonded to each other,
L^{E2} that does not form the monocyclic ring and does not form the condensed ring is a substituted or unsubstituted arylene group having 6 to 50 ring carbon atoms, or a substituted or unsubstituted divalent heterocyclic group having 5 to 50 ring atoms,
A¹, B¹, C¹, A², B², C², and D² are each independently a substituted or unsubstituted aryl group having 6 to 50 ring carbon atoms, a substituted or unsubstituted heterocyclic group having 5 to 50 ring atoms, or -Si(R'₉₀₁)(R'₉₀₂)(R'₉₀₃),
R'₉₀₁, R'₉₀₂ and R'₉₀₃ are each independently a substituted or unsubstituted aryl group having 6 to 50 ring carbon atoms,
in the case where a plurality of groups each represented by R'₉₀₁ exist, the plurality of groups each represented by R'₉₀₁ are the same as or different from each other,
in the case where a plurality of groups each represented by R'₉₀₂ exist, the plurality of groups each represented by R'₉₀₂ are the same as or different from each other,
in the case where a plurality of groups each represented by R'₉₀₃ exist, the plurality of groups each represented by R'₉₀₃ are the same as or different from each other.

In the formulas (11) and (12), A1, B1, C1, A2, B2, C2, and D2 are preferably each independently selected from a substituted or unsubstituted phenylene group, a substituted or unsubstituted biphenyl group, a substituted or unsubstituted terphenyl group, a substituted or unsubstituted naphthyl group, a substituted or unsubstituted fluorenyl group, a substituted or unsubstituted dibensofuranyl group, a substituted or unsubstituted dibenzothiophenyl group, and a substituted or unsubstituted carbazolyl group.

More preferably, in the formula (11), at least one of A1, B1 and C1, and in the formula (12), at least one of A2, B2, C2 and D2 is a substituted or unsubstituted biphenyl group, a substituted or unsubstituted terphenyl group, a substituted or unsubstituted naphthyl group, a substituted or unsubstituted fluorenyl group, a substituted or unsubstituted dibensofuranyl group, or a substituted or unsubstituted dibenzothiophenyl group, a substituted or unsubstituted carbazolyl group.

The fluorenyl group that A1, B1, C1, A2, B2, C2, and D2 might be may have a substituent at site 9, for example, it may be a 9,9-dimethylfluorenyl group, or a 9,9-diphenylfluorenyl group. Further, the substituents at site 9 may form a ring with each other, for example, the substituents at site 9 may form a fluorene skeleton or a xanthene skeleton with each other.

L^{A1}, L^{B1}, L^{C1}, L^{A2}, L^{B2}, L^{C2} and L^{D2} are preferably each independently a single bond or a substituted or unsubstituted arylene group having 6 to 12 ring carbon atoms.

Specific examples of the compounds represented by the formula (11) and the formula (12) include the following compounds.

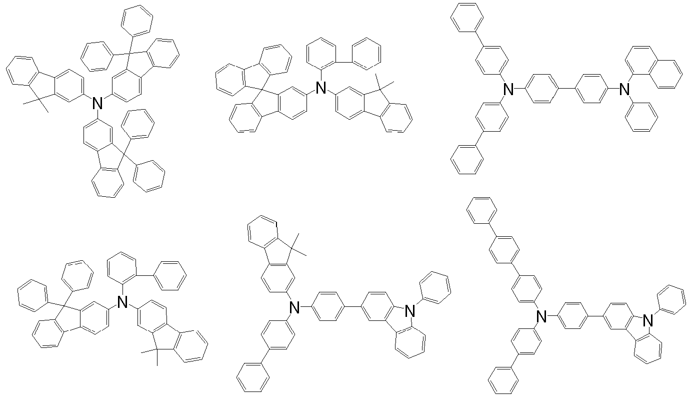

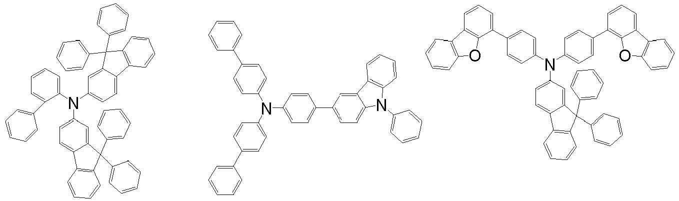

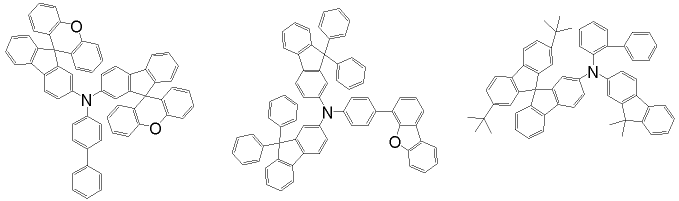

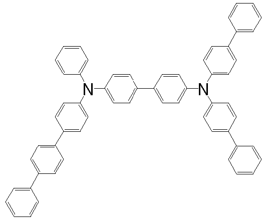

### Dopant material for light emitting layer

The light emitting layer is a layer containing a material having a high light emitting capability (dopant material), and various materials can be used. For example, a fluorescence emitting material or a phosphorescence emitting material can be used as the dopant material. A fluorescence emitting material is a compound that emits light from the singlet excited state, and a phosphorescence emitting material is a compound that emits light from the triplet excited state.

In an aspect of the organic EL device according to the present invention, the light emitting layer is a single layer.

In another aspect of the organic EL device according to the present invention, the light emitting layer includes a first light emitting layer and a second light emitting layer.

As a blue fluorescence emitting material that can be used in the light emitting layer, a pyrene derivative, a styrylamine derivative, a chrysene derivative, a fluoranthene derivative, a fluorene derivative, a diamine derivative, a triarylamine derivative, or the like can be used. Specific examples thereof include N,N'-bis[4-(9H-carbazol-9-yl)phenyl]-N,N'-diphenylstilbene-4,4'-diamine (abbreviation: YGA2S), 4-(9H-carbazol-9-yl)-4'-(10-phenyl-9-anthryl)triphenylamine (abbreviation: YGAPA), and 4-(10-phenyl-9-anthryl)-4'-(9-phenyl-9H-carbazol-3-yl)triphenylamine (abbreviation: PCBAPA).

As a green fluorescence emitting material that can be used in the light emitting layer, an aromatic amine derivative or the like can be used. Specific examples thereof include N-(9,10-diphenyl-2-anthryl)-N,9-diphenyl-9H-carbazole-3-amine (abbreviation: 2PCAPA), N-[9,10-bis(1,1'-biphenyl-2-yl)-2-anthryl]-N,9-diphenyl-9H-carbazole-3-amine (abbreviation: 2PCABPhA), N-(9,10-diphenyl-2-anthryl)-N,N',N'-triphenyl-1,4-phenylenediamine (abbreviation: 2DPAPA), N-[9,10-bis(1,1'-biphenyl-2-yl)-2-anthryl]-N,N',N'-triphenyl-1,4-phenylenediamine (abbreviation: 2DPABPhA), N-[9,10-bis(1,1'-biphenyl-2-yl)]-N-[4-(9H-carbazol-9-yl)phenyl]-N-phenylanthracene-2-amine (abbreviation: 2YGABPhA), and N,N,9-triphenylanthracene-9-amine (abbreviation: DPhAPhA).

As a red fluorescence emitting material that can be used in the light emitting layer, a tetracene derivative, a diamine derivative, or the like can be used. Specific examples thereof include N,N,N',N'-tetrakis(4-methylphenyl)tetracene-5,11-diamine (abbreviation: p-mPhTD) and 7,14-diphenyl-N,N,N',N'-tetrakis(4-methylphenyl)acetonaphtho[1,2-a]fluoranthene-3,10-diamine (abbreviation: p-mPhAFD).

In one embodiment of the present invention, it is preferable that the light emitting layer contains a fluorescence emitting material (fluorescent dopant material).

As a blue phosphorescence emitting material that can be used in the light emitting layer, a metal complex, such as an iridium complex, an osmium complex, or a platinum complex, is used. Specific examples thereof include bis[2-(4',6'-difluorophenyl)pyridinato-N,C2']iridium(III) tetrakis(1-pyrazolyl)borate (abbreviation: FIr6), bis[2-(4',6'-difluorophenyl)pyridinato-N,C2']iridium(III) picolinate (abbreviation: FIrpic), bis[2-(3',5'-bistrifluoromethylphenyl)pyridinato-N,C2']iridium(III) picolinate (abbreviation: Ir(CF3ppy)2(pic)), and bis[2-(4',6'-difluorophenyl)pyridinato-N,C2']iridium(III) acetylacetonate (abbreviation: FIracac).

As a green phosphorescence emitting material that can be used in the light emitting layer, an iridium complex or the like is used. Examples thereof include tris(2-phenylpyridinato-N,C2')iridium(III) (abbreviation: Ir(ppy)3), bis(2-phenylpyridinato-N,C2')iridium(III) acetylacetonate (abbreviation: Ir(ppy)2(acac)), bis(1,2-diphenyl-1H-benzimidazolato)iridium(III) acetylacetonate (abbreviation: Ir(pbi)2(acac)), and bis(benzo[h]quinolinato)iridium(III) acetylacetonate (abbreviation: Ir(bzq)2(acac)).

As a red phosphorescence emitting material that can be used in the light emitting layer, a metal complex, such as an iridium complex, a platinum complex, a terbium complex, or a europium complex, is used. Specific examples thereof include organic metal complexes, such as bis[2-(2'-benzo[4,5-α]thienyl)pyridinato-N,C3']iridium(III) acetylacetonate (abbreviation: Ir(btp)2(acac)), bis(1-phenylisoquinolinato-N,C2')iridium(III) acetylacetonate (abbreviation: Ir(piq)2(acac)), (acetylacetonato)bis[2,3-bis(4-fluorophenyl)quinoxalinato]iridium(III) (abbreviation: Ir(Fdpq)2(acac)), and 2,3,7,8,12,13,17,18-octaethyl-21H,23H-porphyrin platinum(II) (abbreviation: PtOEP).

In addition, a rare earth metal complex, such as tris(acetylacetonate)(monophenanthroline)terbium(III) (abbreviation: Tb(acac)3(Phen)), tris(1,3-diphenyl-1,3-propanedionato)(monophenanthroline)europium(III) (abbreviation: Eu(DBM)3(Phen)), or tris[1-(2-thenoyl)-3,3,3-trifluoroacetonato](monophenanthroline)europium(III) (abbreviation: Eu(TTA)3(Phen)), emits light from rare earth metal ions (electron transition between different multiplicities) and thus, can be used as a phosphorescence emitting material.

### Host material for light emitting layer

The light emitting layer may have a configuration in which such a dopant material as described above is dispersed in another material (host material). A material that has a higher lowest unoccupied molecular orbital level (LUMO level) and a lower highest occupied molecular orbital level (HOMO level) than the dopant material is preferably used.

As the host material, for example,
(1) a metal complex, such as an aluminum complex, a beryllium complex, or a zinc complex,
(2) a heterocyclic compound, such as an oxadiazole derivative, a benzimidazole derivative, or a phenanthroline derivative,
(3) a condensed aromatic compound, such as a carbazole derivative, an anthracene derivative, a phenanthrene derivative, a pyrene derivative, or a chrysene derivative, or
(4) an aromatic amine compound, such as a triarylamine derivative or a condensed polycyclic aromatic amine derivative
is used.

For example, a metal complex, such as tris(8-quinolinolato)aluminum(III) (abbreviation: Alq), tris(4-methyl-8-quinolinolato)aluminum(III) (abbreviation: Almq3), bis(10-hydroxybenzo[h]quinolinato)beryllium(II) (abbreviation: BeBq2), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolato]zinc(II) (abbreviation: ZnPBO), or bis[2-(2-benzothiazolyl)phenolato]zinc(II) (abbreviation: ZnBTZ);
a heterocyclic compound, such as 2-(4-biphenylyl)-5-(4-tert-butylphenyl)-1,3,4-oxadiazole (abbreviation: PBD), 1,3-bis[5-(p-tert-butylphenyl)-1,3,4-oxadiazol-2-yl]benzene (abbreviation: OXD-7), 3-(4-biphenylyl)-4-phenyl-5-(4-tert-butylphenyl)-1,2,4-triazole (abbreviation: TAZ), 2,2',2"-(1,3,5-benzenetriyl)tris(1-phenyl-1H-benzimidazole) (abbreviation: TPBI), bathophenanthroline (abbreviation: BPhen), or bathocuproine (abbreviation: BCP);
a condensed aromatic compound, such as 9-[4-(10-phenyl-9-anthryl)phenyl]-9H-carbazole (abbreviation: CzPA), 3,6-diphenyl-9-[4-(10-phenyl-9-anthryl)phenyl]-9H-carbazole (abbreviation: DPCzPA), 9,10-bis(3,5-diphenylphenyl)anthracene (abbreviation: DPPA), 9,10-di(2-naphtyl)anthracene (abbreviation: DNA), 2-tert-butyl-9,10-di(2-naphtyl)anthracene (abbreviation: t-BuDNA), 9,9'-bianthryl (abbreviation: BANT), 9,9'-(stilbene-3,3'-diyl)diphenanthrene (abbreviation: DPNS), 9,9'-(stilbene-4,4'-diyl)diphenanthrene (abbreviation: DPNS2), 3,3',3"-(benzene-1,3,5-triyl)tripyrene (abbreviation: TPB3), 9,10-diphenylanthracene (abbreviation: DPAnth), or 6,12-dimethoxy-5,11-diphenylchrysene; and
an aromatic amine compound, such as N,N-diphenyl-9-[4-(10-phenyl-9-anthryl)phenyl]-9H-carbazole-3-amine (abbreviation: CzA1PA), 4-(10-phenyl-9-anthryl)triphenylamine (abbreviation: DPhPA), N,9-diphenyl-N-[4-(10-phenyl-9-anthryl)phenyl]-9H-carbazole-3-amine (abbreviation: PCAPA), N,9-diphenyl-N-{4-[4-(10-phenyl-9-anthryl)phenyl]phenyl}-9H-carbazole-3-amine (abbreviation: PCAPBA), N-(9,10-diphenyl-2-anthryl)-N,9-diphenyl-9H-carbazole-3-amine (abbreviation: 2PCAPA), 4,4'-bis[N-(1-naphtyl)-N-phenylamino]biphenyl (abbreviation: NPB or α-NPD), N,N'-bis(3-methylphenyl)-N,N'-diphenyl-[1,1'-biphenyl]-4,4'-diamine (abbreviation: TPD), 4,4'-bis[N-(9,9-dimethylfluoren-2-yl)-N-phenylamino]biphenyl (abbreviation: DFLDPBi), or 4,4'-bis[N-(spiro-9,9'-bifluoren-2-yl)-N-phenylamino]biphenyl (abbreviation: BSPB), can be used. Two or more host materials may be used.

In particular, in the case of the blue fluorescence device, the following anthracene compounds are preferably used as a host material.

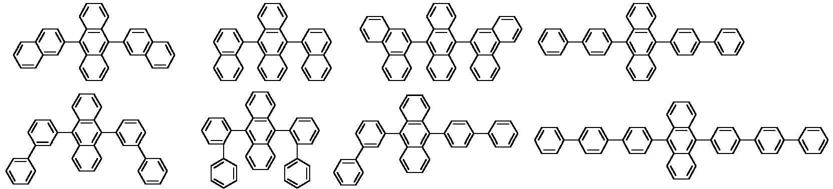

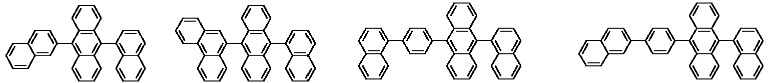

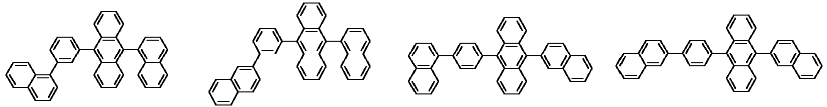

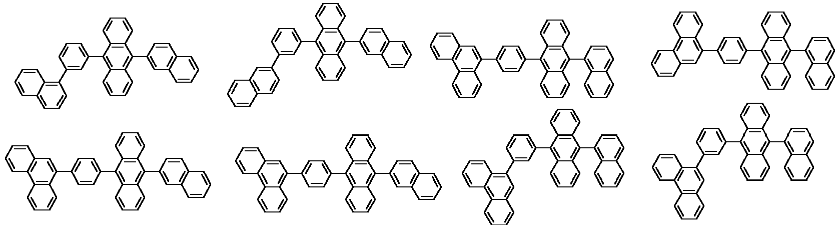

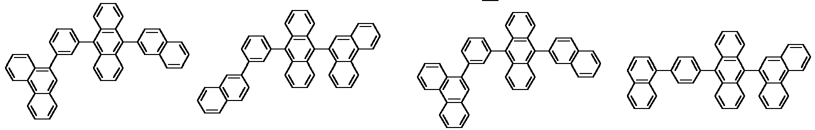

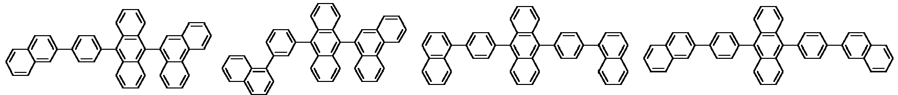

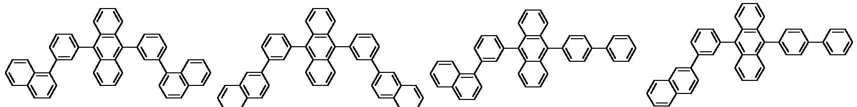

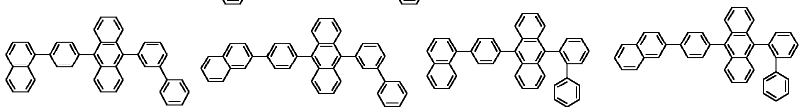

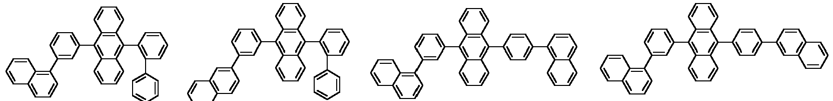

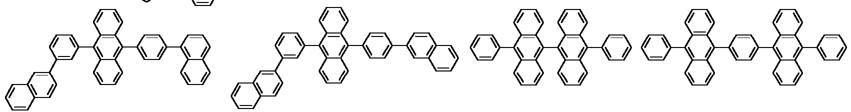

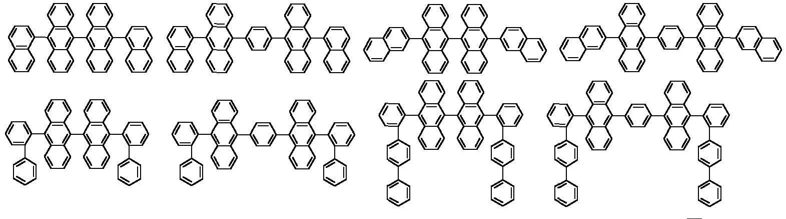

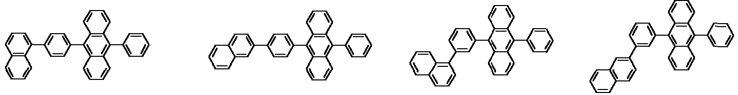

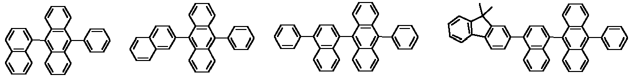

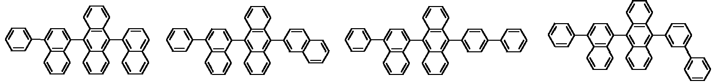

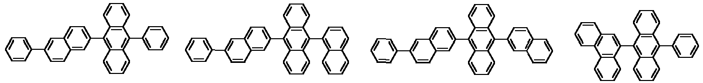

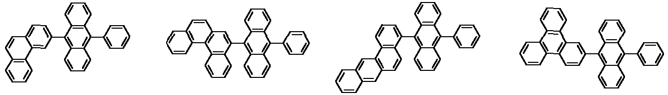

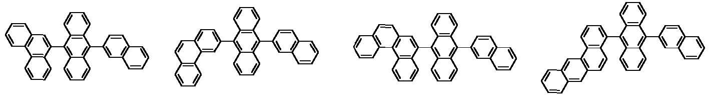

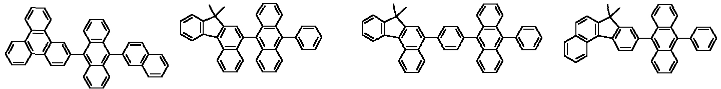

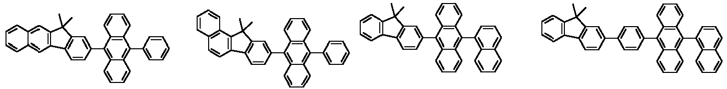

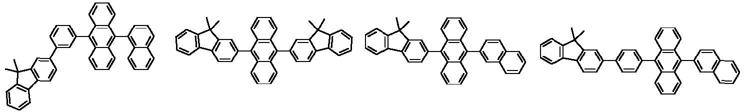

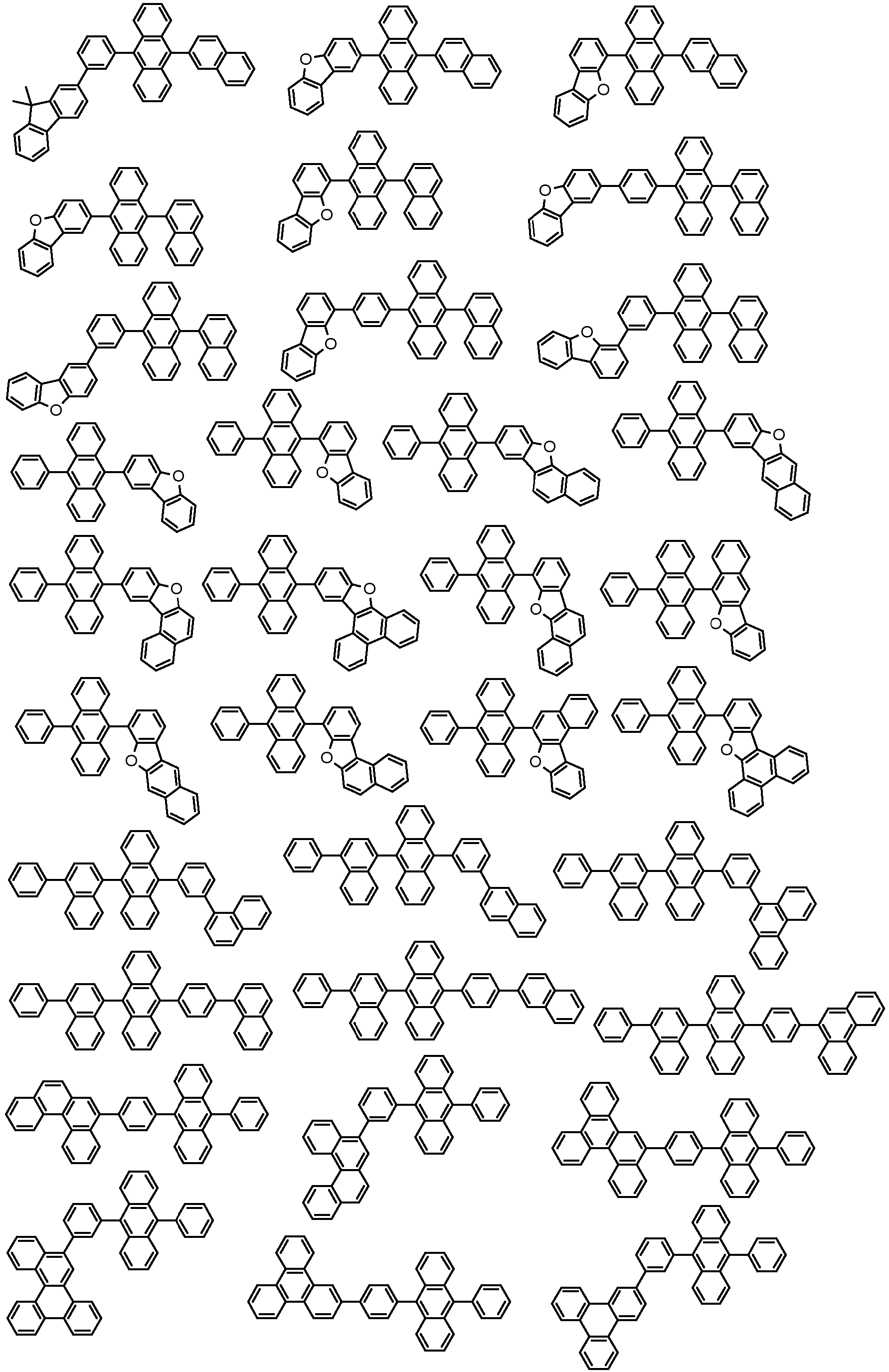

In an aspect of the organic EL device according to the present invention, when the light emitting layer includes a first light emitting layer and a second light emitting layer, at least one of the components constituting the first light emitting layer is different from the components constituting the second light emitting layer. For example, the dopant material contained in the first light emitting layer is different from the dopant material contained in the second light emitting layer, or the host material contained in the first light emitting layer is different from the host material contained in the second light emitting layer.

In the organic EL device of the present invention, the light emitting layer may contain a light emitting compound (hereinafter sometimes simply referred to as a "fluorescent compound") that exhibits fluorescent light emission with a main peak wavelength of 500 nm or less.

A method for measuring the main peak wavelength of a compound is as follows. A 5 µmol/L toluene solution of a compound to be measured is prepared and placed in a quartz cell, and the emission spectrum of the sample (vertical axis: emission intensity, horizontal axis: wavelength) is measured at room temperature (300K). The emission spectrum can be measured using a spectrofluorometer (device name: F-7000) manufactured by Hitachi High-Tech Science Co., Ltd. The emission spectrum measuring device is not limited to the device used herein.

In the emission spectrum, a peak wavelength of an emission spectrum at which the emission intensity is maximum is defined as the main peak wavelength. In the description herein, the main peak wavelength may be referred to as fluorescent light emission main peak wavelength (FL-peak).

### The fluorescent compound may be the dopant material or the host material.

When the light emitting layer is a single layer, only one of the dopant material and the host material may be the fluorescent compound, or both may be the fluorescent compound.

Further, when the light emitting layer includes a first light emitting layer (anode side) and a second light emitting layer (cathode side), only one of the first light emitting layer and the second light emitting layer may contain the fluorescent compound, or both of the light emitting layers may contain the fluorescent compound. When the first light emitting layer contains the fluorescent compound, only one of the dopant material and the host material contained in the first light emitting layer may be the fluorescent compound, or both may be the fluorescent compound. Further, when the second light emitting layer contains the fluorescent compound, only one of the dopant material and the host material contained in the second light emitting layer may be the fluorescent compound, or both may be the fluorescent compound.

### Electron transporting layer

The electron transporting layer is a layer containing a material having a high electron transporting capability (electron transporting material), and is formed between the light emitting layer and the cathode or between an electron injecting layer, if present, and the light emitting layer.

The electron transporting layer may have a monolayer structure or a multilayer structure including two or more layers. For example, the electron transporting layer may have a two-layer structure including a first electron transporting layer (anode side) and a second electron transporting layer (cathode side). In an aspect of the present invention, the electron transporting layer in the monolayer structure is preferably adjacent to the light emitting layer, and the electron transporting layer that is the nearest to the anode in the multilayer configuration, for example, the first electron transporting layer of the two-layer structure, is preferably adjacent to the light emitting layer. In another aspect of the present invention, a hole blocking layer as described later or the like may be interposed between the electron transporting layer of the monolayer structure and the light emitting layer or between the electron transporting layer that is the nearest to the light emitting layer in the multilayer structure and the light emitting layer.

For the electron transporting layer, for example,
(1) a metal complex, such as an aluminum complex, a beryllium complex, or a zinc complex,
(2) a heteroaromatic compound, such as an imidazole derivative, a benzimidazole derivative, an azine derivative, a carbazole derivative, or a phenanthroline derivative, or
(3) a high-molecular weight compound
can be used.

Examples of the metal complex include tris(8-quinolinolato)aluminum(III) (abbreviation: Alq), tris(4-methyl-8-quinolinolato)aluminum (abbreviation: Almq3), bis(10-hydroxybenzo[h]quinolinato)beryllium (abbreviation: BeBq₂), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolato]zinc(II) (abbreviation: ZnPBO), and bis[2-(2-benzothiazolyl)phenolato]zinc(II) (abbreviation: ZnBTZ).

Examples of the heteroaromatic compound include 2-(4-biphenylyl)-5-(4-tert-butylphenyl)-1,3,4-oxadiazole (abbreviation: PBD), 1,3-bis[5-(p-tert-butylphenyl)-1,3,4-oxadiazol-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzoxazol-2-yl)stilbene (abbreviation: BzOs).

Examples of the high-molecular weight compound include poly[(9,9-dihexylfluorene-2,7-diyl)-co-(pyridine-3,5-diyl)] (abbreviation: PF-Py) and poly [(9,9-dioctylfluorene-2,7-diyl)-co-(2,2'-bipyridine-6,6'-diyl)] (abbreviation: PF-BPy).

The materials are materials having an electron mobility of 10⁻⁶ cm²/Vs or more. Materials other than those as mentioned above may be used for the electron transporting layer so long as they are materials higher in the electron transporting capability rather than in the hole transporting capability.

### Electron injecting layer

The electron injecting layer is a layer containing a material having a high electron injecting capability. In the electron injecting layer, an alkali metal, such as lithium (Li) or cesium (Cs), an alkaline earth metal, such as magnesium (Mg), calcium (Ca), or strontium (Sr), a rare earth metal, such as europium (Eu) or ytterbium (Yb), and a compound containing them can be used. Examples of the compound include an alkali metal oxide, an alkali metal halide, an alkali metal-containing organic complex, an alkaline earth metal oxide, an alkaline earth metal halide, an alkaline earth metal-containing organic complex, a rare earth metal oxide, a rare earth metal halide, and a rare earth metal-containing organic complex. In addition, two or more of the compounds can be used in mixture.

Besides, a material in which an alkali metal, an alkaline earth metal, or a compound thereof is contained in a material having electron transporting capability, specifically, a material in which magnesium (Mg) is contained in Alq, or the like, may be used. In this case, electron injection from the cathode can be more efficiently achieved.

Alternatively, a composite material obtained by mixing an organic compound with an electron doner may be used in the electron injecting layer. Such a composite material is excellent in the electron injecting capability and the electron transporting capability because the organic compound receives electrons from the electron doner. In this case, the organic compound is preferably a material excellent in transporting received electrons, and specifically, a material constituting the aforementioned electron transporting layer (such as a metal complex or a heteroaromatic compound) can be used. As the electron donor, a material having an electron donation capability for an organic compound can be used. Specifically, an alkali metal, an alkaline earth metal, and a rare earth metal are preferred, and examples thereof include lithium, cesium, magnesium, calcium, erbium, and ytterbium. An alkali metal oxide or an alkaline earth metal oxide is also preferred, and examples thereof include lithium oxide, calcium oxide, and barium oxide. ALewis base, such as magnesium oxide, can also be used. An organic compound, such as tetrathiafulvalene (abbreviation: TTF), can also be used.

### Cathode

A metal, an alloy, an electrically conductive compound, or a mixture thereof that has a low work function (specifically 3.8 eV or less) is preferably used for the cathode. Specific examples of such a cathode material include elements belonging to the group 1 or 2 of the periodic table, that is, an alkali metal, such as lithium (Li) or cesium (Cs), an alkaline earth metal, such as magnesium (Mg), calcium (Ca), or strontium (Sr), and an alloy containing them (for example, MgAg, AlLi), and a rare earth metal, such as europium (Eu) or ytterbium (Yb), and an alloy containing them.

When the cathode is formed by using an alkali metal, an alkaline earth metal, and an alloy containing them, a vacuum vapor deposition method or a sputtering method can be adopted. When silver paste or the like is used, a coating method, an inkjet method, or the like can be adopted.

By providing the electron injecting layer, the cathode can be formed using various conductive materials, such as Al, Ag, ITO, graphene, and indium oxide-tin oxide containing silicon or silicon oxide, regardless of the magnitude of the work function. A film of such a conductive material can be formed by using a sputtering method, an inkjet method, a spin-coating method, or the like.

### Insulating layer

The organic EL device applies an electric field to an ultrathin film, and thus, pixel defects are likely to occur due to leaks or short-circuiting. In order to prevent this, an insulating layer formed of an insulating thin film layer may be inserted between a pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. A mixture or a laminate of them may also be used.

### Space layer

The space layer is, for example, a layer provided between a fluorescence emitting layer and a phosphorescence emitting layer for the purpose of preventing excitons generated in the phosphorescence emitting layer from diffusing into the fluorescence emitting layer, or adjusting the carrier balance, in the case where the fluorescence emitting layers and the phosphorescence emitting layers are stacked. The space layer can also be provided between two or more phosphorescence emitting layers.

Since the space layer is provided between the light emitting layers, a material having both an electron transporting capability and a hole transporting capability is preferred. In addition, one having a triplet energy of 2.6 eV or more is preferred in order to prevent diffusion of the triplet energy in the adjacent phosphorescence emitting layers. Examples of the material used for the space layer include the same as those used for the hole transporting layer as described above.

### Blocking layer

A blocking layer, such as an electron blocking layer, a hole blocking layer, or an exciton blocking layer, may be provided adjacent to the light emitting layer. The electron blocking layer is a layer that prevents electrons from leaking from the light emitting layer to a hole transporting layer, and the hole blocking layer is a layer that prevents holes from leaking from the light emitting layer to an electron transporting layer. The exciton blocking layer functions to prevent excitons generated in the light emitting layer from diffusing into the surrounding layers to trap the excitons within the light emitting layer.

Each layer of the organic EL device can be formed by a conventionally known vapor deposition method, a coating method, or the like. For example, each layer can be formed by a known technique by a vapor deposition method, such as a vacuum vapor deposition method or a molecular beam vapor deposition method (MBE method), or a coating method using a solution of a compound for forming a layer, such as a dipping method, a spin-coating method, a casting method, a bar-coating method, and a roll-coating method.

The film thickness of each layer is not particularly limited, but is typically 5 nm to 10 µm, and more preferably 10 nm to 0.2 µm because in general, when the film thickness is too small, defects such as pinholes are likely to occur, and conversely, when the film thickness is too large, a high driving voltage is required and the efficiency decreases.

In the organic EL device having a hole transporting layer with a two-layer structure or a three-layer structure of the present invention, a total thickness of the first hole transporting layer and the second hole transporting layer is preferably 30 nm or more and 150 nm or less, and more preferably 40 nm or more and 130 nm or less.

Further, in one aspect of the present invention, the thickness of the second hole transporting layer having a two-layer structure or a three-layer structure is preferably 5 nm or more, more preferably 20 nm or more, further preferably 25 nm or more, and particularly preferably 35 nm or more, and is preferably 100 nm or less.

Further, in one aspect of the present invention, the thickness of the hole transporting layer adjacent to the light emitting layer is preferably 5 nm or more, more preferably 20 nm or more, further preferably 25 nm or more, and particularly preferably 30 nm or more, and is preferably 100 nm or less.

Further, in the organic EL device having a hole transporting layer having a two-layer structure or a three-layer structure according to the present invention, the ratio of a film thickness D2 of the second hole transporting layer to a film thickness D1 of the first hole transporting layer is preferably 0.3 < D2/D1 < 4.0, more preferably 0.5 < D2/D1 < 3.5, and further preferably 0.75 < D2/D1 < 3.0.

Examples of preferred embodiments of the organic EL device of the present invention include
(1) organic EL device having a hole transporting layer with a two-layer structure
   - a first embodiment in which the second hole transporting layer contains the inventive compound and the first hole transporting layer does not contain the inventive compound;
   - a second embodiment in which both the first hole transporting layer and the second hole transporting layer contain the inventive compound;
   - a third embodiment in which the first hole transporting layer contains the inventive compound and the second hole transporting layer does not contain the inventive compound;
(2) organic EL device having a hole transporting layer with a three-layer structure
   - a fourth embodiment in which the first hole transporting layer contains the inventive compound and the second and third hole transporting layers do not contain the inventive compound;
   - a fifth embodiment in which the second hole transporting layer contains the inventive compound and the first and third hole transporting layers do not contain the inventive compound;
   - a sixth embodiment in which the third hole transporting layer contains the inventive compound and the first and second hole transporting layers do not contain the inventive compound;
   - a seventh embodiment in which the first and second hole transporting layers contain the inventive compound and the third hole transporting layer does not contain the inventive compound;
   - an eighth embodiment in which the first and third hole transporting layers contain the inventive compound and the second hole transporting layer does not contain the inventive compound;
   - a tenth embodiment in which the second and third hole transporting layers contain the inventive compound and the first hole transporting layer does not contain the inventive compound;
   - a tenth embodiment in which all of the first to third hole transporting layers contain the inventive compound.

### Electronic instrument

The organic EL device according to one embodiment of the present invention can be used in an electronic instrument, such as a display apparatus and a light emitting apparatus. Examples of the display apparatus include a display component of an organic EL panel module or the like, a television, a mobile phone, a tablet, and a personal computer. Examples of the light emitting apparatus include a lighting or a vehicle lamp.

The organic EL device can be used in an electronic instrument, such as a display component of an organic EL panel module or the like, a display apparatus of a television, a mobile phone, a personal computer, or the like, and a light emitting apparatus of a lighting or a vehicle lamp.

### Examples

The present invention will be described in more detail below by reference to Examples, but the present invention is not to be limited to the following Examples.

### Inventive Compound used for Production of Organic EL Device (I) of Example 1

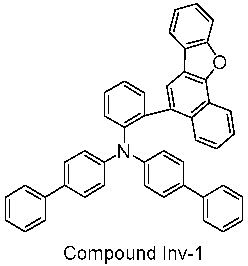

### Comparative Compound used for Production of Organic EL Device (I) of Comparative Example 1

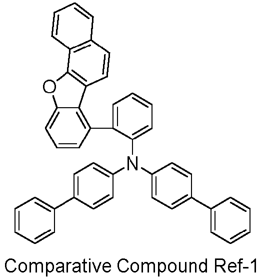

### Other Compounds used for Production of Organic EL Devices (I) of Example 1 and Comparative Example 1

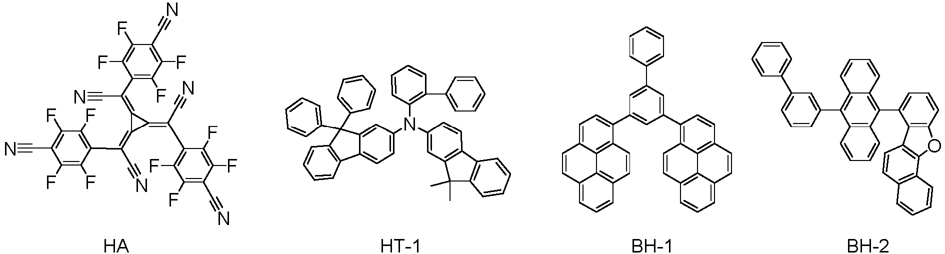

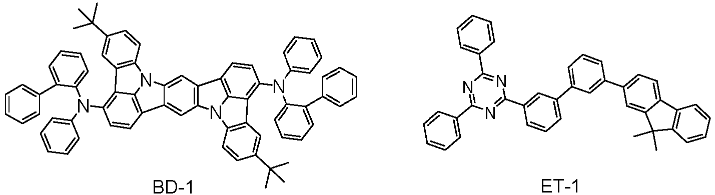

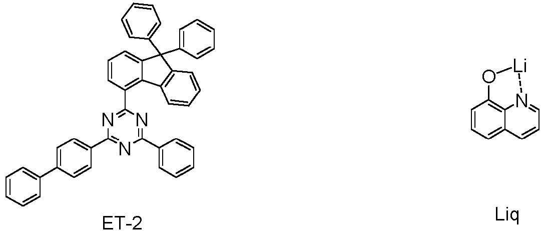

### Production of Organic EL Device (I)

### <Example 1>

A glass substrate of 25 mm × 75 mm × 1.1 mm with an ITO transparent electrode (anode) (manufactured by GEOMATEC Co., Ltd.) was ultrasonically cleaned in isopropyl alcohol for 5 minutes and then subjected to UV ozone cleaning for 30 minutes. The film thickness of the ITO was 130 nm.

The cleaned glass substrate with the ITO transparent electrode was mounted on a substrate holder of a vacuum vapor deposition apparatus, and firstly, Compound HT-1 and Compound HA were vapor co-deposited on the surface having the transparent electrode formed thereon so as to cover the transparent electrode, thus forming a hole injecting layer with a film thickness of 10 nm. The mass ratio of Compound HT-1 and Compound HA (HT-I:HA) was 97:3.

Subsequently, on the hole injecting layer, Compound HT-1 was vapor deposited to form a first hole transporting layer with a film thickness of 40 nm.

Subsequently, on this first hole transporting layer, Compound Inv-1 was vapor deposited to form a second hole transporting layer with a film thickness of 5 nm.

Subsequently, on this second hole transporting layer, Compound BH-1 (host material) and Compound BD-1 (dopant material) were vapor co-deposited to form a first light emitting layer with a film thickness of 10 nm. The mass ratio of Compound BH-1 and Compound BD-1 (BH-1:BD-1) was 99:1.

Subsequently, on this first light emitting layer, Compound BH-2 (host material) and Compound BD-1 (dopant material) were vapor co-deposited to form a second light emitting layer with a film thickness of 10 nm. The mass ratio of Compound BH-2 and Compound BD-1 (BH-2:BD-1) was 99:1.

Subsequently, on this light emitting layer, Compound ET-1 was vapor deposited to form a first electron transporting layer with a film thickness of 5 nm.

Subsequently, on this first electron transporting layer, Compound ET-2 and Liq were vapor co-deposited to form a second electron transporting layer with a film thickness of 25 nm. The mass ratio of Compound ET-2 and Liq (ET-2:Liq) was 50:50.

Subsequently, on this second electron transporting layer, Yb was vapor deposited to form an electron injecting electrode with a film thickness of 1 nm.

Then, on this electron injecting electrode, metal Al was vapor deposited to form a metal cathode with a film thickness of 50 nm.

The layer configuration of the organic EL device (I) of Example 1 thus obtained was as follows.

ITO (130)/(HT-1:HA=97:3) (10)/HT-1 (40)/Inv-1 (5)/(BH-1:BD-1=99:1) (10)/(BH-2:BD-I =99: 1) (10)/ET-1 (5)/(ET-2:Liq=50:50) (25)/Yb (1)/Al (50)

In the layer configuration, the numerals in parentheses each indicate the film thickness (nm), and the ratios are each a mass ratio.

### <Comparative Example 1>

Each organic EL device (I) was produced in the same manner as in Example 1 except for using a comparative compound Ref-1 instead of the inventive compound Inv-1.

### Evaluation of Organic EL Device (I)

### (1) 95% Lifetime (LT95)

The resulting organic EL device (I) was driven with a constant direct current at a current density of 50 mA/cm², and the period of time until the luminance was reduced to 95% of the initial luminance was measured, and was defined as 95% lifetime (LT95).

The result is shown in Table 1.

**Table 1**

| | Material of second hole transporting layer | LT95(h) at 50mA/cm² |
|---|---|---|
| Example 1 | Compound Inv-1 | 97 |
| Comparative Example 1 | Comparative Compound Ref-1 | 85 |

As apparent from the results in Table 1, the Compound Inv-1 provides an organic EL device with a longer lifetime than that of the Comparative Compound Ref-1.

### Inventive Compounds used for Production of Organic EL Device (II) of Examples 2 to 11

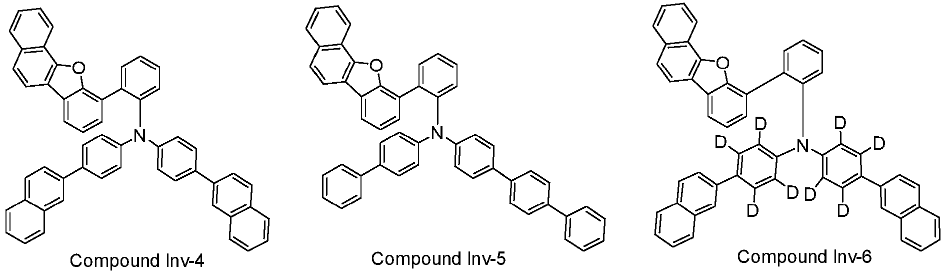

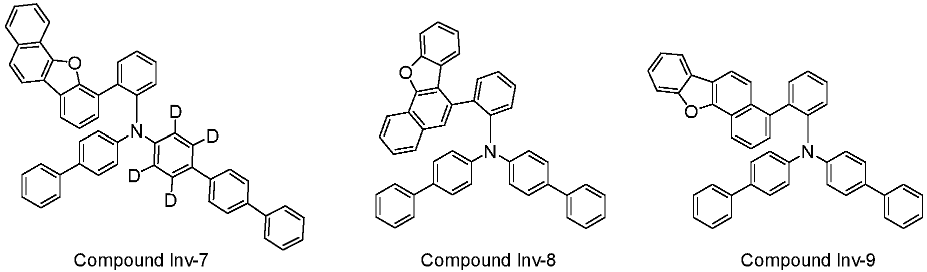

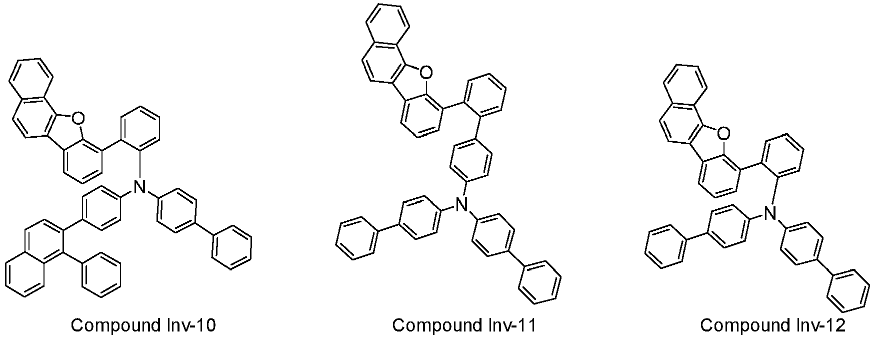

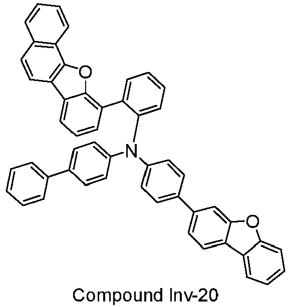

### Comparative Compound used for Production of Organic EL Device (II) of Comparative Example 2

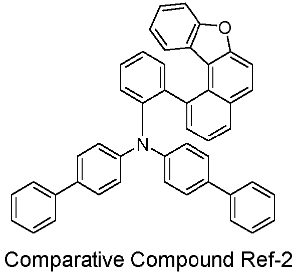

### Other Compounds used for Production of Organic EL Device (II) of Examples 2 to 11 and Comparative Example 2

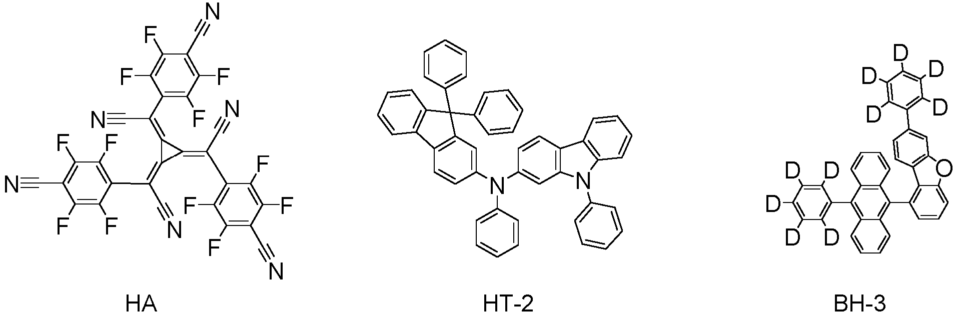

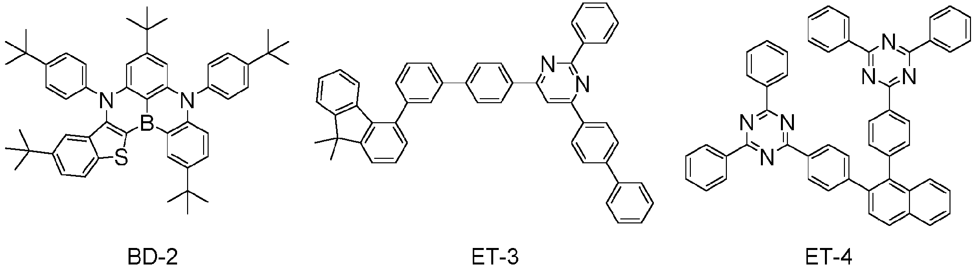

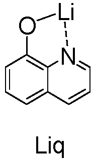

### Production of Organic EL Device (II)

### <Example 2>

A glass substrate of 25 mm × 75 mm × 1.1 mm with an ITO transparent electrode (anode) (manufactured by GEOMATEC Co., Ltd.) was ultrasonically cleaned in isopropyl alcohol for 5 minutes and then subjected to UV ozone cleaning for 30 minutes. The film thickness of the ITO was 130 nm.

The cleaned glass substrate with the ITO transparent electrode was mounted on a substrate holder of a vacuum vapor deposition apparatus, and firstly, Compound HT-2 and Compound HA were vapor co-deposited on the surface having the transparent electrode formed thereon so as to cover the transparent electrode, thus forming a hole injecting layer with a film thickness of 10 nm. The mass ratio of Compound HT-2 and Compound HA (HT-2:HA) was 97:3.

Subsequently, on the hole injecting layer, Compound HT-2 was vapor deposited to form a first hole transporting layer with a film thickness of 85 nm.

Subsequently, on this first hole transporting layer, Compound Inv-4 was vapor deposited to form a second hole transporting layer with a film thickness of 5 nm.

Subsequently, on this second hole transporting layer, Compound BH-3 (host material) and Compound BD-2 (dopant material) were vapor co-deposited to form a first light emitting layer with a film thickness of 20 nm. The mass ratio of Compound BH-3 and Compound BD-2 (BH-3:BD-2) was 99:1.

Subsequently, on this light emitting layer, Compound ET-3 was vapor deposited to form a first electron transporting layer with a film thickness of 5 nm.

Subsequently, on this first electron transporting layer, Compound ET-4 and Liq were vapor co-deposited to form a second electron transporting layer with a film thickness of 31 nm. The mass ratio of Compound ET-4 and Liq (ET-4:Liq) was 50:50.

Subsequently, on this second electron transporting layer, Liq was vapor deposited to form an electron injecting electrode with a film thickness of 1 nm.

Then, on this electron injecting electrode, metal Al was vapor deposited to form a metal cathode with a film thickness of 80 nm.

The layer configuration of the organic EL device (II) of Example 2 thus obtained was as follows.

ITO (130)/(HT-2:HA=97:3) (10)/HT-2 (85)/Inv-4 (5)/(BH-3:BD-2=99:1) (20)/ET-3 (5)/(ET-4:Liq=50:50) (31)/Liq (1)/Al (80)

In the layer configuration, the numerals in parentheses each indicate the film thickness (nm), and the ratios are each a mass ratio.

### <Examples 3 to 11 and Comparative Example 2>

An organic EL device (II) was produced in the same manner as in Example 2 except for using the compounds shown in Table 2 instead of the Compound Inv-2.

### Evaluation of Organic EL Device (II)

The 95% lifetime (LT95) of the resulting organic EL device (II) was measured in the same manner as the evaluation of the organic EL device (I). The results are shown in Table 2.

**Table 2**

| | Material of second hole transporting layer | LT95(h) at 50mA/cm² |
|---|---|---|
| Example 2 | Compound Inv-4 | 198 |
| Example 3 | Compound Inv-5 | 160 |
| Example 4 | Compound Inv-6 | 206 |
| Example 5 | Compound Inv-7 | 178 |
| Example 6 | Compound Inv-8 | 142 |
| Example 7 | Compound Inv-9 | 145 |
| Example 8 | Compound Inv-10 | 167 |
| Example 9 | Compound Inv-11 | 137 |
| Example 10 | Compound Inv-12 | 151 |
| Example 11 | Compound Inv-20 | 166 |
| Comparative Example 2 | Comparative Compound Ref-2 | 70 |

As apparent from the results in Table 2, the Compounds Inv-4 to 12 and 20 provide an organic EL device with a longer lifetime than that of the Comparative Compound Ref-2.

### Inventive Compounds used for Production of Organic EL Device (III) of Examples 12 to 18

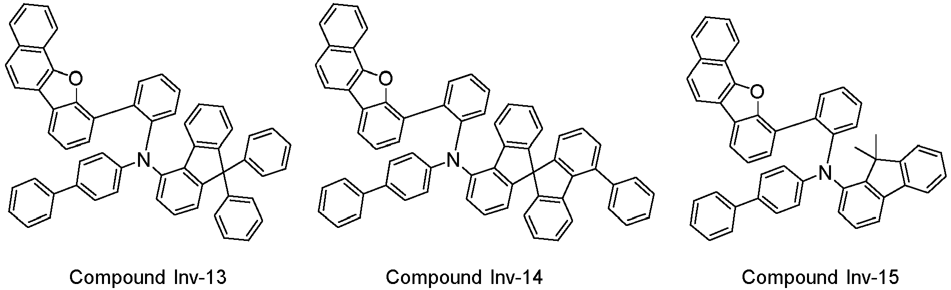

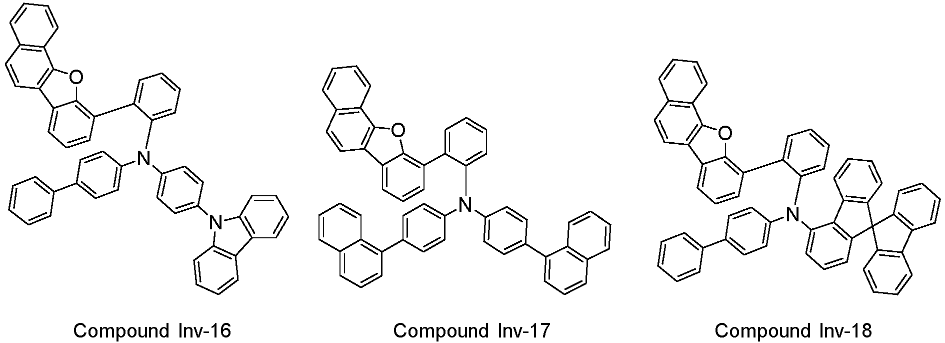

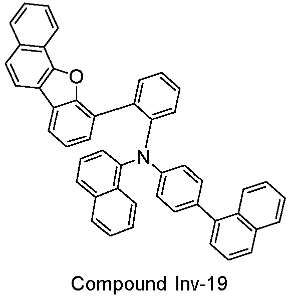

### Comparative Compound used for Production of Organic EL Device (III) of Comparative Example 3

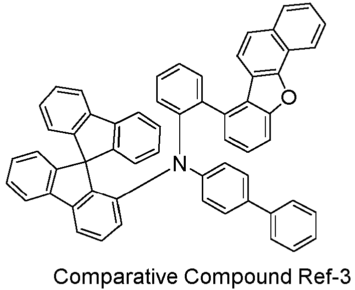

### Other Compounds used for Production of Organic EL Device (III) of Examples 12 to 18 and Comparative Example 3

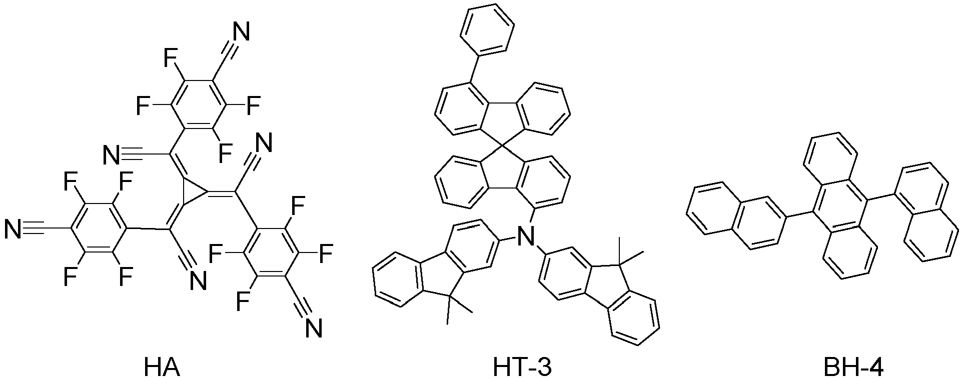

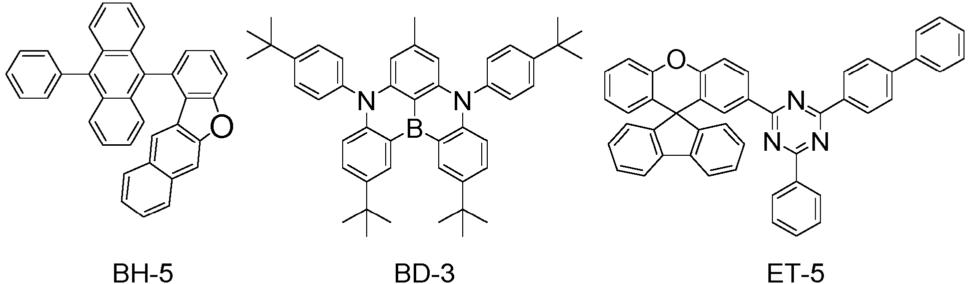

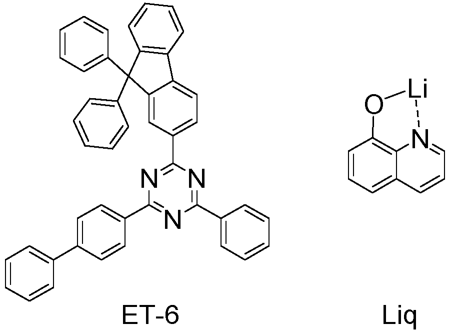

### Production of Organic EL Device (III)

### <Example 12>

A glass substrate of 25 mm × 75 mm × 1.1 mm with an ITO transparent electrode (anode) (manufactured by GEOMATEC Co., Ltd.) was ultrasonically cleaned in isopropyl alcohol for 5 minutes and then subjected to UV ozone cleaning for 30 minutes. The film thickness of the ITO was 130 nm.

The cleaned glass substrate with the ITO transparent electrode was mounted on a substrate holder of a vacuum vapor deposition apparatus, and firstly, Compound HT-3 and Compound HA were vapor co-deposited on the surface having the transparent electrode formed thereon so as to cover the transparent electrode, thus forming a hole injecting layer with a film thickness of 10 nm. The mass ratio of Compound HT-3 and Compound HA (HT-3:HA) was 97:3.

Subsequently, on the hole injecting layer, Compound HT-3 was vapor deposited to form a first hole transporting layer with a film thickness of 80 nm.

Subsequently, on this first hole transporting layer, Compound Inv-13 was vapor deposited to form a second hole transporting layer with a film thickness of 7.5 nm.

Subsequently, on this second hole transporting layer, a film of BH (host material):BD-3 (dopant material) with a film thickness of 20 nm was formed. This BH:BD-3 film functions as a light emitting layer. The BH [Compounds BH-4 and BH-5 (both host materials)] contained in the light emitting layer have a mass ratio of 3:2, and the concentration of BD-3 is 2% by mass with respect to the entire light emitting layer.

Subsequently, on this light emitting layer, Compound ET-5 was vapor deposited to form a first electron transporting layer with a film thickness of 5 nm.

Subsequently, on this first electron transporting layer, Compound ET-6 and Liq were vapor co-deposited to form a second electron transporting layer with a film thickness of 25 nm. The mass ratio of Compound ET-6 and Liq (ET-6:Liq) was 67:33.

Subsequently, on this second electron transporting layer, Yb was vapor deposited to form an electron injecting electrode with a film thickness of 1 nm.

Then, on this electron injecting electrode, metal Al was vapor deposited to form a metal cathode with a film thickness of 80 nm.

The layer configuration of the organic EL device (III) of Example 12 thus obtained was as follows.

ITO (130)/(HT-3:HA=97:3) (10)/HT-3 (80)/Inv-13 (7.5)/(BH-4:BH-5:BD-3=60:40:2 (20)/ET-5 (5)/ET-6:Liq=67:33) (25)/Yb (1)/Al (80)

In the layer configuration, the numerals in parentheses each indicate the film thickness (nm), and the ratios are each a mass ratio.

### <Examples 13 to 18 and Comparative Example 3>

An organic EL device (III) was produced in the same manner as in Example 12 except for using the compounds shown in Table 3 instead of the Compound Inv-13.

### Evaluation of Organic EL Device (III)

The 95% lifetime (LT95) of the resulting organic EL device (III) was measured in the same manner as the evaluation of the organic EL device (I). The results are shown in Table 3.

**Table 3**

| | Material of second hole transporting layer | LT95(h) at 50mA/cm² |
|---|---|---|
| Example 12 | Compound Inv-13 | 96 |
| Example 13 | Compound Inv-14 | 108 |
| Example 14 | Compound Inv-15 | 88 |
| Example 15 | Compound Inv-16 | 94 |
| Example 16 | Compound Inv-17 | 115 |
| Example 17 | Compound Inv-18 | 103 |
| Example 18 | Compound Inv-19 | 79 |
| Comparative Example 3 | Comparative Compound Ref-3 | 64 |

As apparent from the results in Table 3, the Compounds Inv-13 to 19 provide an organic EL device with a longer lifetime than that of the Comparative Compound Ref-3.

### Inventive Compounds Synthesized in Synthetic Examples

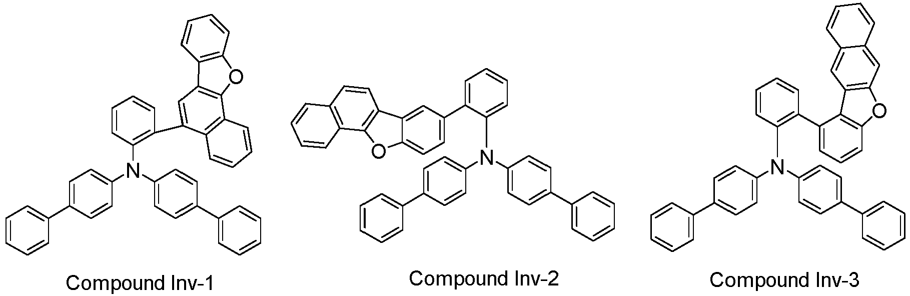

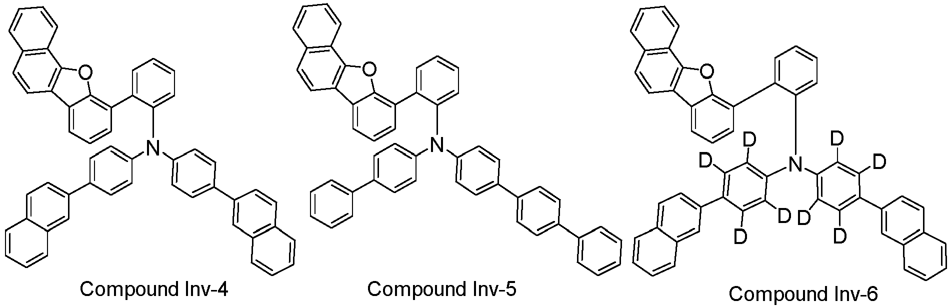

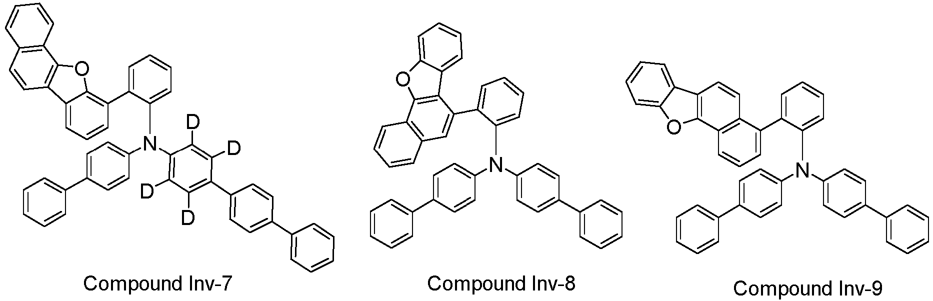

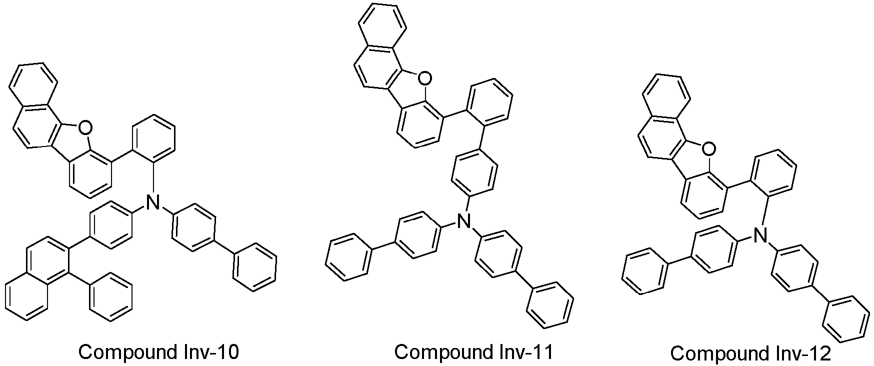

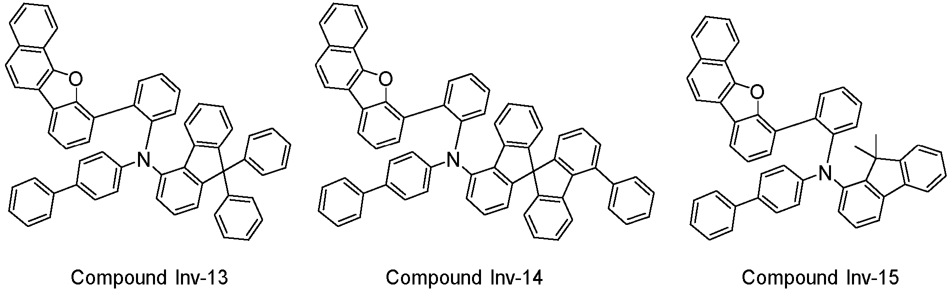

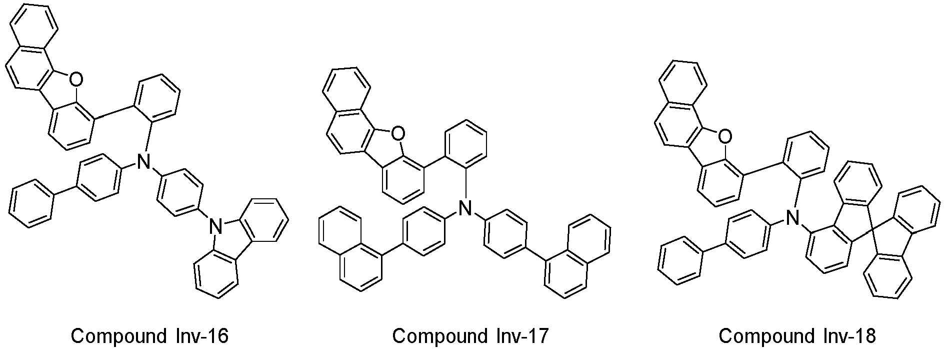

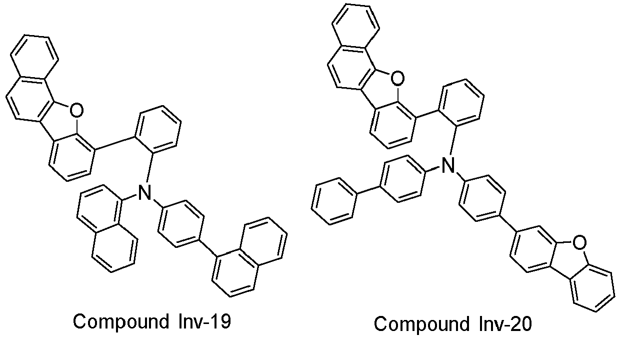

### Intermediate Synthetic Example 1: Synthesis of Benzo[b]naphtho[2,1-d]furan-4-yl trifluoromethanesulfonate

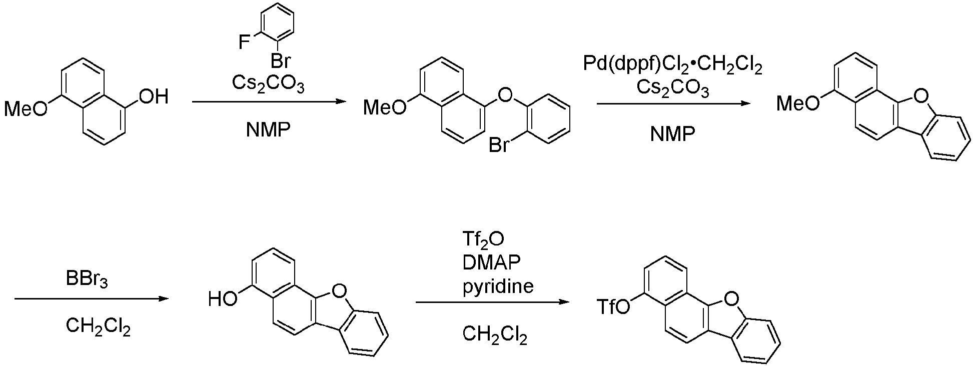

In an argon atmosphere, a mixture of 10.0 g (57.4 mmol) of 5-methoxy-1-naphthol, 50.2 g (287 mmol) of 1-bromo-2-fluorobenzene, 28.1 g (86.0 mmol) of cesium carbonate, and 287 mL of N-methyl-2-pyrrolidone was stirred at 145°C for 12 hours. After cooling to room temperature and adding water thereto, the reaction solution was extracted with toluene, and concentrated under reduced pressure. The resulting residue was purified by silica gel column chromatography to obtain 14.7 g of 1-(2-bromophenoxy)-5-methoxynaphthalene as a colorless liquid. The yield was 78%.

In an argon atmosphere, a mixture of 14.7 g (44.8 mmol) of the resulting 1-(2-bromophenoxy)-5-methoxynaphthalene, 1.83 g (2.24 mmol) of a [1,1'-bis(diphenylphosphino)ferrocene]palladium(II) dichloride dichloromethane adduct, 21.9g (67.2mmol) of cesium carbonate, and 224 mL of N-methyl-2-pyrrolidone was stirred at 130°C for 5 hours. After cooling to room temperature and adding water thereto, the reaction solution was extracted with toluene, and concentrated under reduced pressure. The resulting residue was purified by silica gel column chromatography to obtain 8.0 g of 4-methoxybenzo[b]naphtho[2,1-d]furan as a white solid. The yield was 72%.

In an argon atmosphere, after cooling a mixture of 2.48 g (10.0 mmol) of the resulting 4-methoxybenzo[b]naphtho[2,1-d]furan and 100 mL of dichloromethane to 0°C, 20 mL of 1.0 mol/L boron tribromide dichloromethane solution was added thereto, and the mixture was stirred at room temperature for 5 hours. After cooling to -78°C and adding water thereto, the reaction solution was extracted with dichloromethane, and concentrated under reduced pressure. The resulting residue was purified by silica gel column chromatography to obtain 2.13 g of benzo[b]naphtho[2,1-d]furan-4-ol as a white solid. The yield was 91%.

In an argon atmosphere, a mixture of 1.17 g (5.0 mmol) of the resulting benzo[b]naphtho[2,1-d]furan-4-ol, 0.061 g (0.50 mmol) of N,N-dimethyl-4-aminopyridine, 10.0 mL (125 mmol) of pyridine, 1.01 mL (6.00 mmol) of trifluoromethanesulfonic anhydride, and 25 mL of dichloromethane was stirred at 0°C for 5 hours. After adding water thereto, the reaction solution was extracted with dichloromethane, and concentrated under reduced pressure. The resulting residue was purified by silica gel column chromatography to obtain 1.48 g of benzo[b]naphtho[2,1-d]furan-4-yl trifluoromethanesulfonate as a white solid. The yield was 81%.

### Intermediate Synthetic Example 2: Synthesis of Intermediate A-1

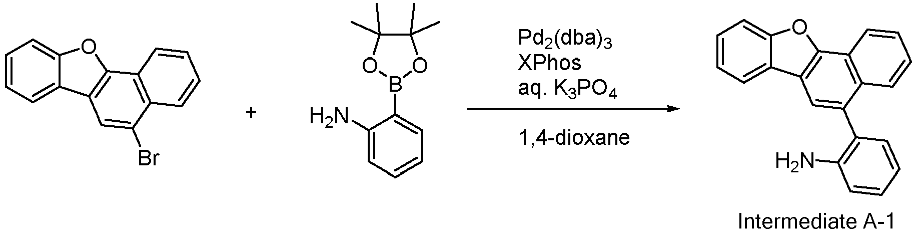

In an argon atmosphere, a mixture of 6.66 g (22.4 mmol) of 5-bromobenzo[b]naphtho[2,1-d]furan (raw material 1), 5.21 g (23.8 mmol) of 2-(4,4,5,5-tetramethyl-1,3, 2-dioxaborolan-2-yl)aniline (raw material 2), 0.205 g (0.224 mmol) of tris(dibenzylideneacetone)dipalladium(0), 0.427 g (0.897 mmol) of 2-dicyclohexylphosphino-2',4',6'-triisopropylbiphenyl (XPhos), 33.6 mL (67.2 mmol) of 2M aqueous potassium phosphate solution, and 149 mL of 1,4-dioxane was stirred at 110°C for 7 hours. The reaction solution was cooled to room temperature and concentrated under reduced pressure. The resulting residue was purified by silica gel column chromatography to obtain 6.01 g of Intermediate A-1 as a white solid. The yield was 87%.

### Intermediate Synthetic Examples 3 and 4: Synthesis of Intermediates A-2 and A-3

Intermediates A-2 and A-3 were obtained in the same manner as in Intermediate Synthetic Example 2 except for changing the raw materials 1 and 2 to those shown in Table 4. The respective yields of Intermediates A-2 and A-3 are shown in Table 4.

**Table 4**

| | Raw material 1 | Raw material 2 | Intermediate | Yield (%) |
|---|---|---|---|---|
| Synthetic Example 2 | 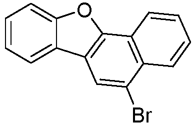 | 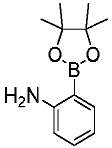 | 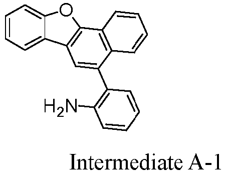 | 87 |
| Synthetic Example 3 | 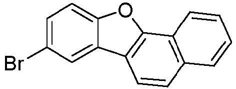 | 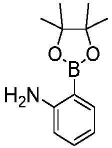 | 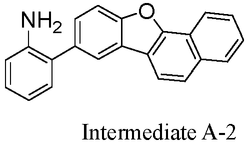 | 78 |
| Synthetic Example 4 | 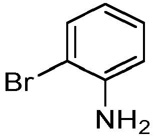 | 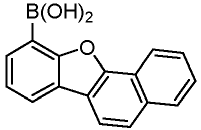 | 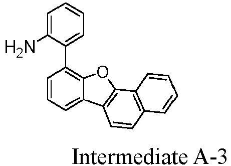 | 70 |

### Intermediate Synthetic Example 5: Synthesis of Intermediate B-1

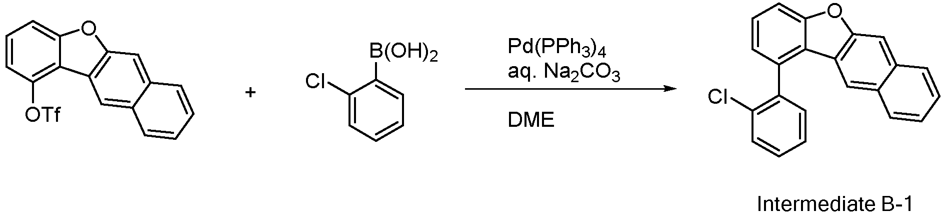

In an argon atmosphere, a mixture of 11.0 g (30.0 mmol) of 5-bromobenzo[b]naphtho[2,1-d]furan (raw material 1), 5.16 g (33.0 mmol) of 2-chlorophenylboronic acid (raw material 2), 1.04 g (0.90 mmol) of tetrakis(triphenylphosphine)palladium (0), 37.5 mL (75.0 mmol) of 2M aqueous sodium carbonate solution, and 100 mL of DME was refluxed at boiling point for 5 hours. The reaction solution was cooled to room temperature and concentrated under reduced pressure. The resulting residue was purified by silica gel column chromatography to obtain 8.15 g of Intermediate B-1 as a white solid. The yield was 83%.

### Intermediate Synthetic Examples 6 to 9: Synthesis of Intermediates B-2 to B-5

Intermediates B-2 to B-5 were obtained in the same manner as in Intermediate Synthetic Example 5 except for changing the raw material 1 and 2 to those shown in Table 5. The respective yields of Intermediates B-2 to B-5 are shown in Table 5.

**Table 5**

| | Raw material 1 | Raw material 2 | Intermediate | Yield (%) |
|---|---|---|---|---|
| Synthetic Example 5 | 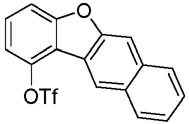 | 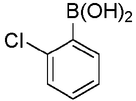 | 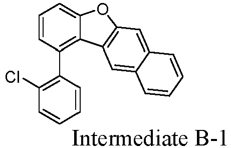 | 83 |
| Synthetic Example 6 | 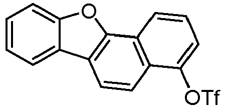 | 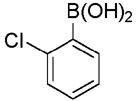 | 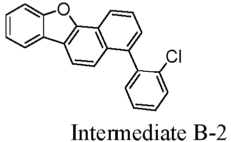 | 75 |
| Synthetic Example 7 | 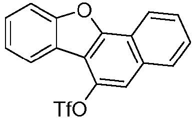 | 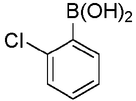 | 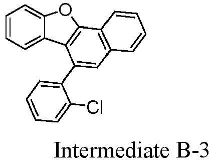 | 69 |
| Synthetic Example 8 | 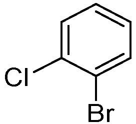 | 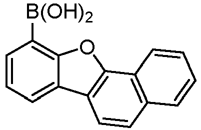 | 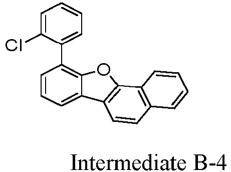 | 86 |
| Synthetic Example 9 | 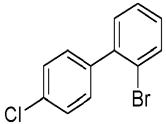 | 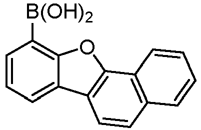 | 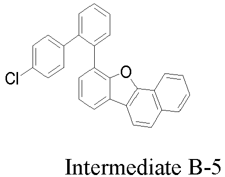 | 74 |

### Intermediate Synthetic Example 10: Synthesis of Intermediate C-1

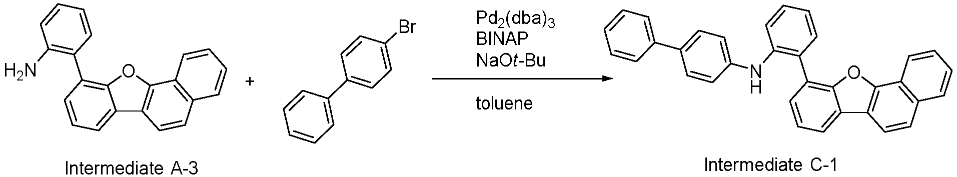

In an argon atmosphere, a mixture of 21.5 g (69.5 mmol) of Intermediate A-3 (raw material 1), 16.2 g (69.5 mmol) of 4-bromobiphenyl (raw material 2), 1.27 g (1.39 mmol) of tris(dibenzylideneacetone)dipalladium(0), 1.73 g (2.78 mmol) of BINAP, 7.35 g (76.0 mmol) of sodium-t-butoxide, and 460 mL of toluene was refluxed at boiling point for 7 hours. The reaction solution was cooled to room temperature and then concentrated under reduced pressure. The resulting residue was purified by silica gel column chromatography and recrystallization to obtain 26.2 g of Intermediate C-1 as a white solid. The yield was 82%.

### Intermediate Synthetic Examples 11 to 14: Synthesis of Intermediates C-2 to C-5

Intermediates C-2 to C-5 were obtained in the same manner as in Intermediate Synthetic Example 10 except for changing the raw materials 1 and 2 to those shown in Table 6. The respective yields of Intermediates C-2 to C-5 are shown in Table 5.

**Table 6**

| | Raw material 1 | Raw material 2 | Intermediate | Yield (%) |
|---|---|---|---|---|
| Synthetic Example 10 | 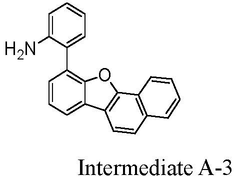 | 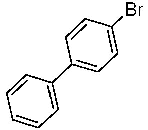 | 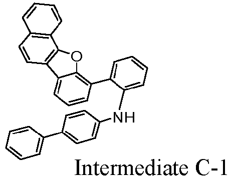 | 82 |
| Synthetic Example 11 | 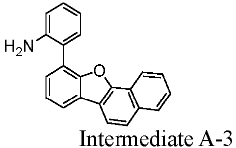 | 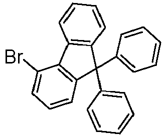 | 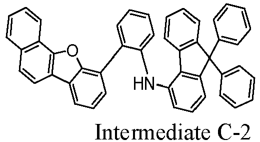 | 77 |
| Synthetic Example 12 | 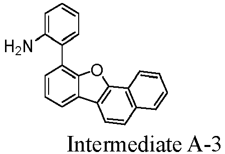 | 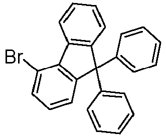 | 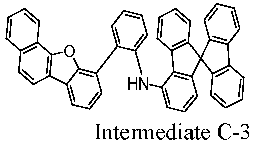 | 69 |
| Synthetic Example 13 | 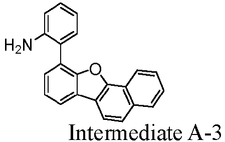 | 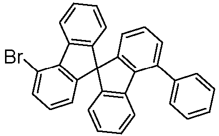 | 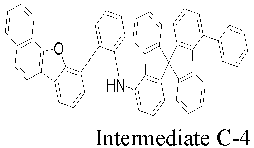 | 65 |
| Synthetic Example 14 | 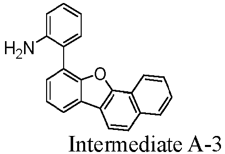 | 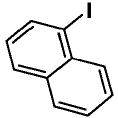 | 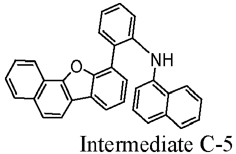 | 72 |

### Synthetic Example 1-1: Synthesis of Inventive Compound Inv-1

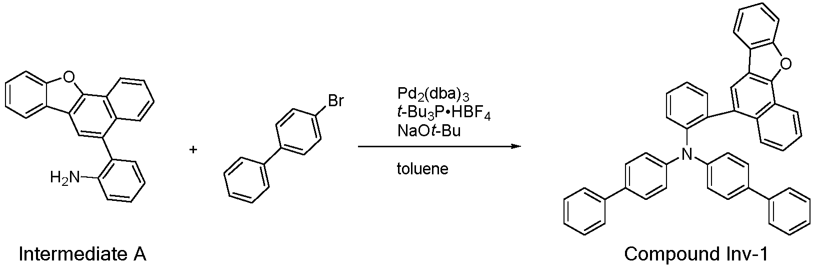

In an argon atmosphere, a mixture of 3.09 g (10.0 mmol) of Intermediate A-1 (Intermediate 1), 4.90 g (21.0 mmol) of 4-bromobiphenyl (Intermediate 2), 0.366 g (0.400 mmol) of tris(dibenzylideneacetone)dipalladium(0), 0.464 g (1.60 mmol) of tri-tert-butylphosphonium tetrafluoroborate, 2.69 g (28.0 mmol) of sodium-t-butoxide, and 100 mL of toluene was refluxed at boiling point for 7 hours. The reaction solution was cooled to room temperature and then concentrated under reduced pressure. The resulting residue was purified by silica gel column chromatography and recrystallization to obtain 3.87 g of a white solid. The yield was 63%.

The resulting substance was revealed as Compound Inv-1 by mass spectrometry, showing m/e=614 with respect to the molecular weight of 613.76.

### Synthetic Examples 1-2 and 1-3: Synthesis of Inventive Compounds Inv-2 and Inv-3

Inventive Compounds Inv-2 and Inv-12 were obtained in the same manner as in Synthetic Example 1-1 except for changing the Intermediates 1 and 2 to those shown in Table 7. The respective yields of the Inventive Compounds Inv-2 and Inv-12 are shown in Table 7.

**Table 7**

| | Intermediate 1 | Intermediate 2 | Inventive compound | Yield (%) |
|---|---|---|---|---|
| Synthetic Example 1-1 | 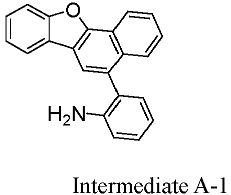 | 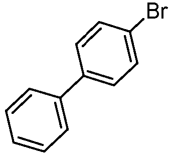 | 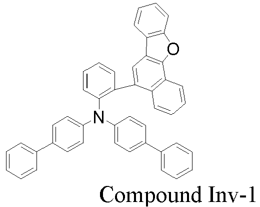 | 63 |
| Synthetic Example 1-2 | 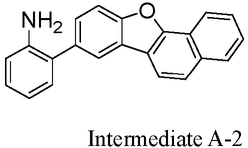 | 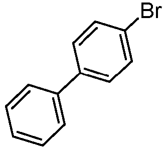 | 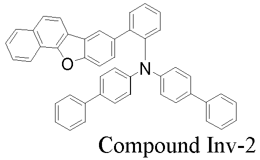 | 67 |
| Synthetic Example 1-3 | 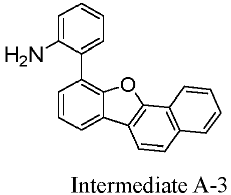 | 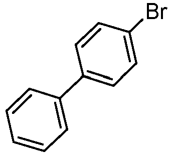 | 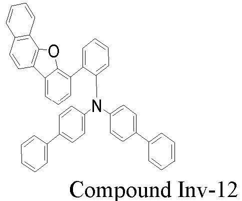 | 73 |

### Synthetic Example 2-1: Synthesis of Inventive Compound Inv-3

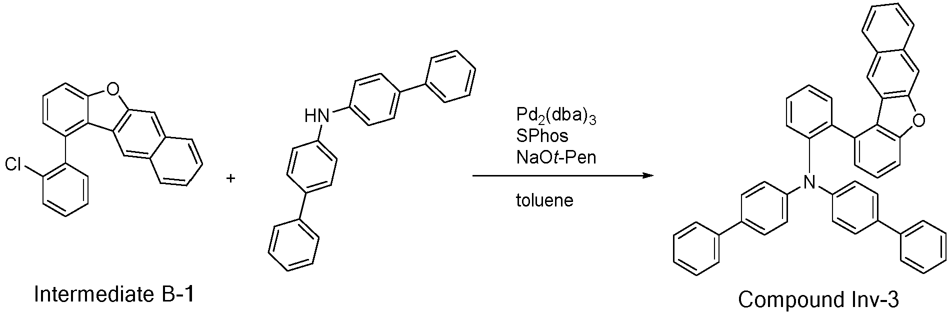

In an argon atmosphere, a mixture of 3.45 g (10.5 mmol) of Intermediate B-1 (Intermediate 1), 3.21 g (21.0 mmol) of N-[1,1'-biphenyl]-4-yl[1,1'-biphenyl]-4-amine (Intermediate 2), 0.366 g (0.400 mmol) of tris(dibenzylideneacetone)dipalladium(0), 0.328 g (0.800 mmol) of 2-dicyclohexylphosphino-2',6'-dimethoxybiphenyl (SPhos), 4.2 mL of sodium t-pentoxide (40% toluene solution), and 67 mL of toluene was refluxed at boiling point for 7 hours. The reaction solution was cooled to room temperature and then concentrated under reduced pressure. The resulting residue was purified by silica gel column chromatography and recrystallization to obtain 4.16 g of a white solid. The yield was 68%.

The resulting substance was revealed as Compound Inv-3 by mass spectrometry, showing m/e=614 with respect to the molecular weight of 613.76.

### Synthetic Examples 2-2 to 2-17: Synthesis of Inventive Compounds Inv-4 to Inv-11 and Inv-13 to Inv-20

Inventive Compounds Inv-4 to Inv-11 and Inv-13 to Inv-20 were obtained in the same manner as in Synthetic Example 2-1 except for changing the Intermediates 1 and 2 to those shown in Tables 8 to 10. The respective yields of the Inventive Compounds Inv-4 to Inv-11 and Inv-13 to Inv-20 are shown in Tables 8 to 10.

**Table 8**

| | Intermediate 1 | Intermediate 2 | Compound | Yield (%) |
|---|---|---|---|---|
| Synthetic Example 2-1 | 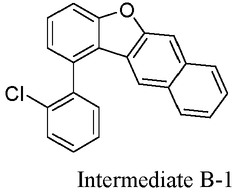 | 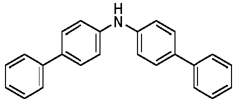 | 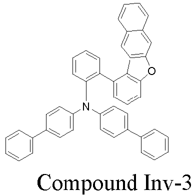 | 68 |
| Synthetic Example 2-2 | 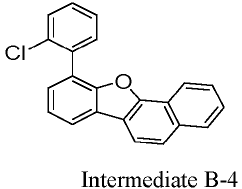 | 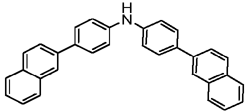 | 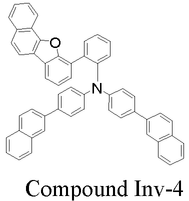 | 61 |
| Synthetic Example 2-3 | 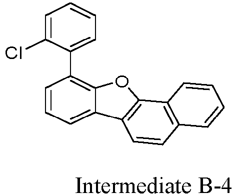 | 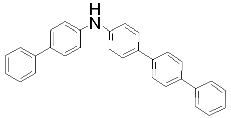 | 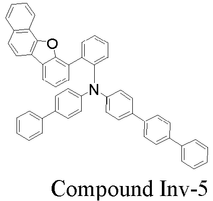 | 70 |
| Synthetic Example 2-4 | 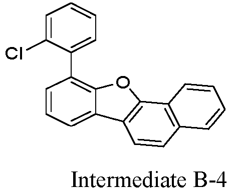 | 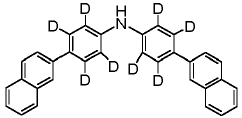 | 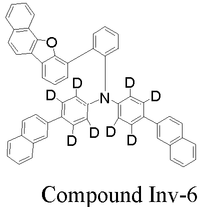 | 78 |
| Synthetic Example 2-5 | 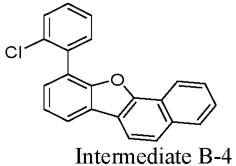 | 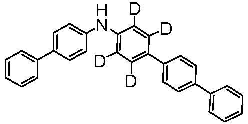 | 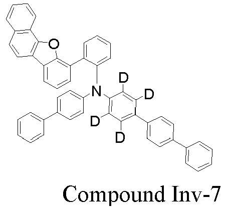 | 70 |
| Synthetic Example 2-6 | 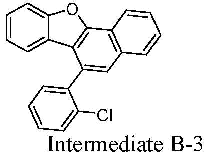 | 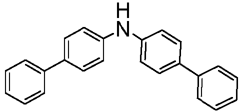 | 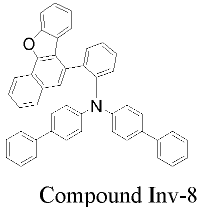 | 68 |

**Table 9**

| | Intermediate 1 | Intermediate 2 | Compound | Yield (%) |
|---|---|---|---|---|
| Synthetic Example 2-7 | 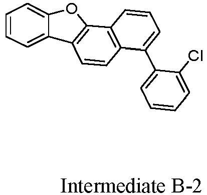 | 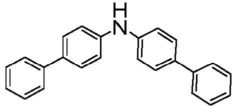 | 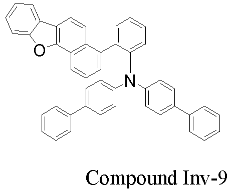 | 64 |
| Synthetic Example 2-8 | 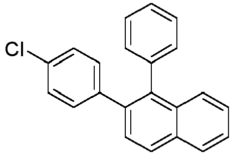 | 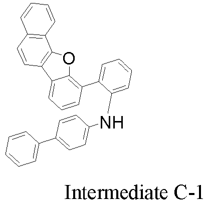 | 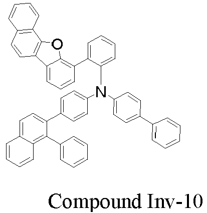 | 73 |
| Synthetic Example 2-9 | 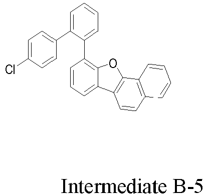 | 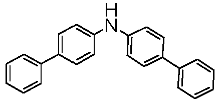 | 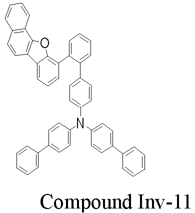 | 69 |
| Synthetic Example 2-10 | 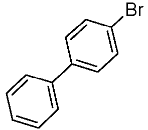 | 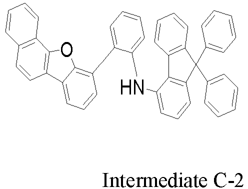 | 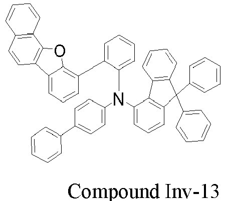 | 64 |
| Synthetic Example 2-11 | 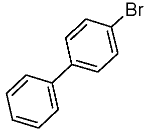 | 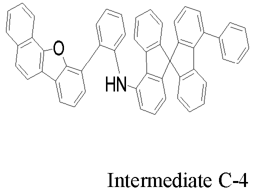 | 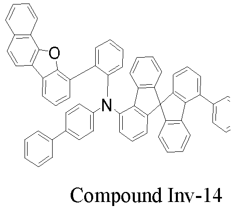 | 71 |
| Synthetic Example 2-12 | 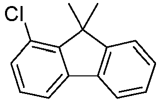 | 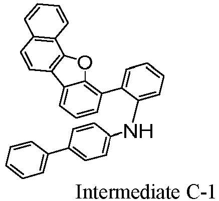 | 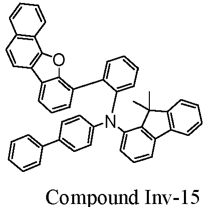 | 58 |

**Table 10**

| | Intermediate 1 | Intermediate 2 | Compound | Yield (%) |
|---|---|---|---|---|
| Syntheti c Example 2-13 | 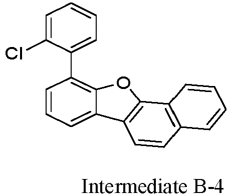 | 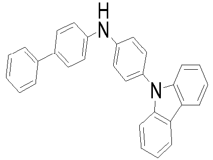 | 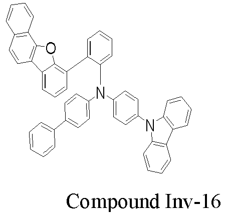 | 70 |
| Syntheti c Example 2-14 | 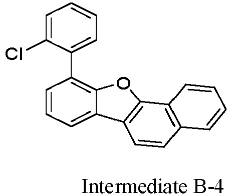 | 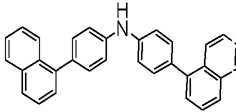 | 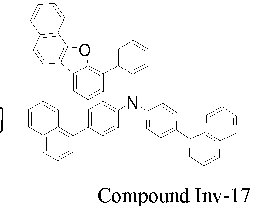 | 75 |
| Syntheti c Example 2-15 | 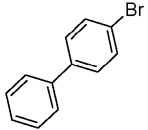 | 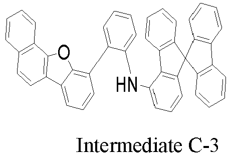 | 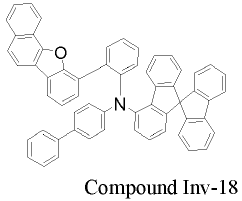 | 67 |
| Syntheti c Example 2-16 | 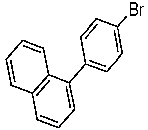 | 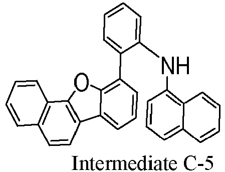 | 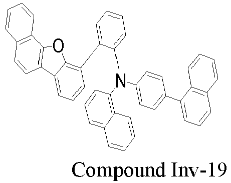 | 80 |
| Syntheti c Example 2-17 | 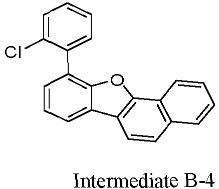 | 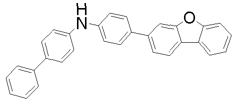 | 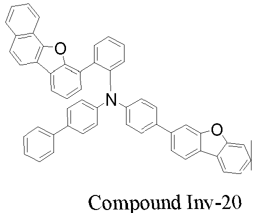 | 76 |

### Reference Signs List

1, 11, 12: Organic EL device
2: Substrate
3: Anode
4: Cathode
5: Light emitting layer
5a: First light emitting layer
5b: Second light emitting layer
6: Hole transporting zone (hole transporting layer)
6a: Hole injecting layer
6b: First hole transporting layer
6c: Second hole transporting layer
6d: Third hole transporting layer
7: Electron transporting zone (Electron transporting layer)
7a: First electron transporting layer
7b: Second electron transporting layer
10, 20, 30: Light emitting unit

## Claims

1. A compound represented by the following formula (1): 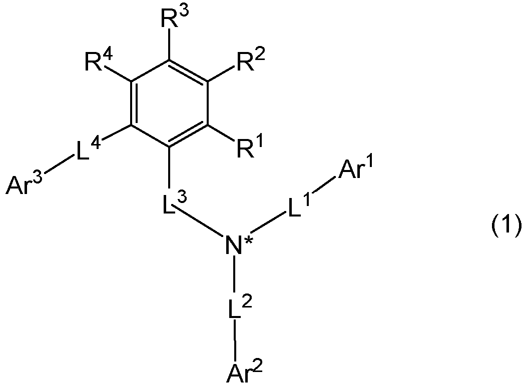 wherein
N* is a central nitrogen atom,
R¹ to R⁴ are each independently a hydrogen atom, an unsubstituted alkyl group having 1 to 6 carbon atoms, or an unsubstituted aryl group having 6 to 12 ring carbon atoms,
adjacent two selected from R¹ to R⁴ are not bonded to each other, thus forming no ring,
L¹ to L⁴ are each independently a single bond, an unsubstituted arylene group having 6 to 30 ring carbon atoms or an unsubstituted divalent heterocyclic group having 5 to 30 ring atoms,
Ar¹ and Ar² are each independently a substituted or unsubstituted aryl group having 6 to 30 ring carbon atoms constituted only of 6-membered rings, a substituted or unsubstituted heterocyclic group having 5 to 30 ring atoms, or a group represented by the following formula (a): 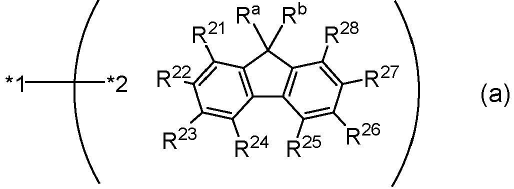 wherein
* 1 is a bonding site to L¹ or L²,
R^{a} and R^{b} are each independently a hydrogen atom, a substituted or unsubstituted alkyl group having 1 to 30 carbon atoms, a substituted or unsubstituted aryl group having 6 to 30 ring carbon atoms, or a substituted or unsubstituted heterocyclic group having 5 to 30 ring atoms,
R^{a} and R^{b} may be bonded to each other to form a substituted or unsubstituted ring, or may not be bonded to each other, thus forming no ring,
one selected from R²¹ to R²⁸ is a single bond bonded to *2, and R²¹ to R²⁸ that are not a single bond bonded to *2 are each independently a hydrogen atom, a substituted or unsubstituted alkyl group having 1 to 6 carbon atoms, a substituted or unsubstituted aryl group having 6 to 12 ring carbon atoms, or a substituted or unsubstituted heterocyclic group having 5 to 13 ring atoms,
adjacent two selected from R²¹ to R²⁸ that are not the single bond may be bonded to each other to form one or more unsubstituted benzene rings, or may not be bonded to each other, thus forming no ring,
provided that when R^{a} and R^{b} are bonded to each other to form a substituted or unsubstituted spirofluorene ring with a carbon atom at site 9 of a fluorene skeleton, one selected from R²² to R²⁷ is a single bond bonded to *2,
when all of L¹ to L⁴ are single bonds, Ar¹ and Ar² are groups represented by the formula (a), and R²² or R²⁷ is a single bond bonded to *2, at least one selected from two R^{a}'s and two R^{b}'s is a substituted or unsubstituted aryl group having 6 to 30 ring carbon atoms;
Ar³ is a group represented by the following formula (x): 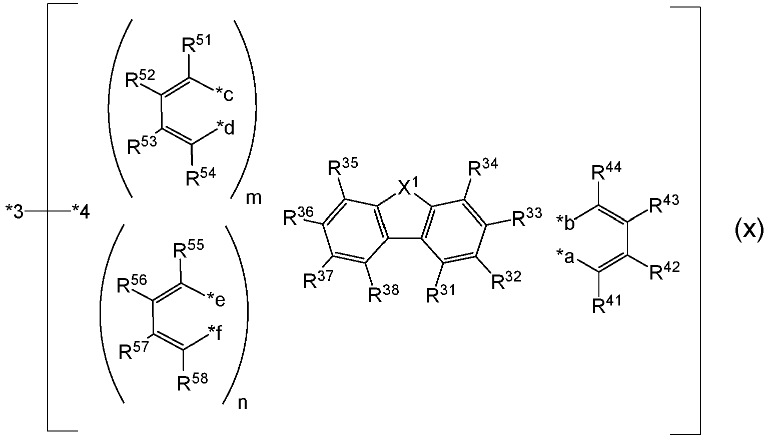 wherein
*3 is a bonding site to L⁴,
R³¹ to R³⁸, R⁴¹ to R⁴⁴, and R⁵¹ to R⁵⁸ are each independently a hydrogen atom, a halogen atom, a cyano group, a nitro group, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by - Si(R₉₀₁)(R₉₀₂)(R₉₀₃), a group represented by -O-(R₉₀₄), a group represented by -S-(R₉₀₅), a group represented by -N(R₉₀₆)(R₉₀₇), a substituted or unsubstituted aryl group having 6 to 50 ring carbon atoms, or a substituted or unsubstituted heterocyclic group having 5 to 50 ring atoms,
R⁹⁰¹ to R⁹⁰⁷ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms,
in the case where two or more groups represented by R⁹⁰¹ exist, the two or more groups each represented by R⁹⁰¹ are the same as or different from each other,
in the case where two or more groups represented by R₉₀₂ exist, the two or more groups each represented by R₉₀₂ are the same as or different from each other,
in the case where two or more groups represented by R₉₀₃ exist, the two or more groups each represented by R₉₀₃ are the same as or different from each other,
in the case where two or more groups represented by R₉₀₄ exist, the two or more groups each represented by R₉₀₄ are the same as or different from each other,
in the case where two or more groups represented by R₉₀₅ exist, the two or more groups each represented by R₉₀₅ are the same as or different from each other,
in the case where two or more groups represented by R₉₀₆ exist, the two or more groups each represented by R₉₀₆ are the same as or different from each other,
in the case where two or more groups represented by R₉₀₇ exist, the two or more groups each represented by R₉₀₇ are the same as or different from each other,
m is 0 or 1, and n is 0 or 1,
when m and n are 0, one of R³¹ and R³², R³² and R³³, or R³³ and R³⁴ is a single bond bonded to *a, and the other is a single bond bonded to *b,
one selected from R³¹ to R³⁸ that are not a single bond bonded to *a and *b, and R⁴¹ to R⁴⁴ is a single bond bonded to *4,
when m is 1 and n is 0, one of R³¹ and R³², R³² and R³³, or R³³ and R³⁴ is a single bond bonded to *a, and the other is a single bond bonded to *b,
one of R³⁵ and R³⁶, R³⁶ and R³⁷, or R³⁷ and R³⁸ is a single bond bonded to *c, and the other is a single bond bonded to *d,
one selected from R³¹ to R³⁴ that are not a single bond bonded to *a and *b, R³⁵ to R³⁸ that are not a single bond bonded to *c and *d, R⁴¹ to R⁴⁴, and R⁵¹ to R⁵⁴ is a single bond bonded to *4,
when m is 0 and n is 1, one of R³¹ and R³², R³² and R³³, or R³³ and R³⁴ is a single bond bonded to *a, and the other is a single bond bonded to *b,
one of R³⁵ and R³⁶, R³⁶ and R³⁷, or R³⁷ and R³⁸ is a single bond bonded to *e, and the other is a single bond bonded to *f,
one selected from R³¹ to R³⁴ that are not a single bond bonded to *a and *b, R³⁵ to R³⁸ that are not a single bond bonded to *e and *f, R⁴¹ to R⁴⁴, and R⁵⁵ to R⁵⁸ is a single bond bonded to *4,
when m is 1 and n is 1, one of R³¹ and R³², R³² and R³³, or R³³ and R³⁴ is a single bond bonded to *a, and the other is a single bond bonded to *b,
one in any one group of three groups, R³⁵ and R³⁶, R³⁶ and R³⁷, and R³⁷ and R³⁸, is a single bond bonded to *c, and the other is a single bond bonded to *d,
one in any one group of the remaining two groups of R³⁵ and R³⁶, R³⁶ and R³⁷, or R³⁷ and R³⁸, is a single bond bonded to *e, and the other is a single bond bonded to *f,
one selected from R³¹ to R³⁴ that are not a single bond bonded to *a and *b, R³⁵ to R³⁸ that are not a single bond bonded to *c to *f, R⁴¹ to R⁴⁴, and R⁵¹ to R⁵⁸ is a single bond bonded to *4,
provided that when L³ and L⁴ are a single bond, m and n are 0, R³³ is a single bond bonded to *a, and R³⁴ is a single bond bonded to *b, or R³⁴ is a single bond bonded to *a and R³³ is a single bond bonded to *b, one selected from R³¹, R³², R³⁵ to R³⁷, and R⁴¹ to R⁴⁴ is a single bond bonded to *4,
when L³ and L⁴ are a single bond, m and n are 0, R³¹ is a single bond bonded to *a, and R³² is a single bond bonded to *b, one selected from R³³ to R³⁸ and R⁴² to R⁴⁴ is a single bond bonded to *4,
when L³ and L⁴ are a single bond, m and n are 0, R³² is a single bond bonded to *a, and R³¹ is a single bond bonded to *b, one selected from R³³ to R³⁸ and R⁴¹ to R⁴³ is a single bond bonded to *4;
X¹ is an oxygen atom or a sulfur atom.

2. The compound according to claim 1, wherein X¹ is an oxygen atom.

3. The compound according to claim 1 or 2, wherein m is 0.

4. The compound according to any one of claims 1 to 3, wherein n is 0.

5. The compound according to any one of claims 1 to 4, wherein Ar¹ and Ar² are each independently a substituted or unsubstituted aryl group having 6 to 30 ring carbon atoms constituted only of 6-membered rings, or a substituted or unsubstituted heterocyclic group having 5 to 30 ring atoms.

6. The compound according to any one of claims 1 to 5, wherein L⁴ is a single bond.

7. The compound according to any one of claims 1 to 6, wherein L³ is a single bond.

8. The compound according to any one of claims 1 to 7, wherein Ar¹ and Ar² are each independently a group represented by any one of the following formulas (1a) to (1e): 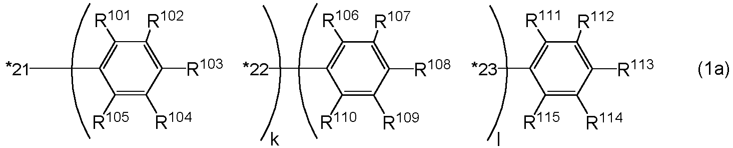 wherein
*21 is a bonding site to L¹ or L²,
R¹⁰¹ to R¹⁰⁵ and R¹⁰⁶ to R¹¹⁰ are each independently a hydrogen atom, an unsubstituted alkyl group having 1 to 6 carbon atoms, or an unsubstituted aryl group having 6 to 12 ring carbon atoms, provided that one selected from R¹⁰¹ to R¹⁰⁵ is a single bond bonded to *22, and one selected from R¹⁰⁶ to R¹¹⁰ is a single bond bonded to *23,
adjacent two selected from R¹⁰¹ to R¹⁰⁵ that are not the single bond are not bonded to each other, thus forming no ring,
adjacent two selected from R¹⁰⁶ to R¹¹⁰ that are not the single bond are not bonded to each other, thus forming no ring,
k is 0 or 1, 1 is 0 or 1,
k + 1 is an integer of 0 to 2,
R¹¹¹ to R¹¹⁵ are each independently a hydrogen atom, a substituted or unsubstituted alkyl group having 1 to 6 carbon atoms, a substituted or unsubstituted aryl group having 6 to 12 ring carbon atoms, or a substituted or unsubstituted heterocyclic group having 5 to 13 ring atoms,
adjacent two selected from R¹¹¹ to R¹¹⁵ may be bonded to each other to form one or more unsubstituted benzene rings, or may not be bonded to each other, thus forming no ring; 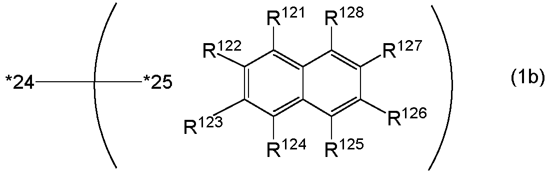 wherein
*24 is a bonding site to L¹ or L²,
R¹²¹ to R¹²⁸ are each independently a hydrogen atom, a substituted or unsubstituted alkyl group having 1 to 6 carbon atoms, or a substituted or unsubstituted aryl group having 6 to 12 ring carbon atoms,
provided that one selected from R¹²¹ to R¹²⁸ is a single bond bonded to *25, and adjacent two selected from R¹²¹ to R¹²⁸ that are not the single bond are not bonded to each other, thus forming no ring; 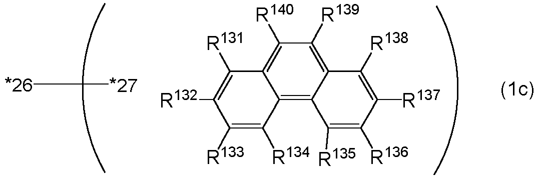 wherein
*26 is a bonding site to L¹ or L²,
R¹³¹ to R¹⁴⁰ are each independently a hydrogen atom, a substituted or unsubstituted alkyl group having 1 to 6 carbon atoms, or a substituted or unsubstituted aryl group having 6 to 12 ring carbon atoms,
provided that one selected from R¹³¹ to R¹⁴⁰ is a single bond bonded to *27, and adjacent two selected from R¹³¹ to R¹⁴⁰ that are not the single bond are not bonded to each other, thus forming no ring; 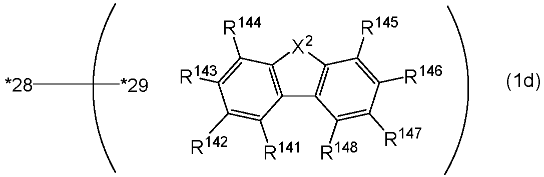 wherein
*28 is a bonding site to L¹ or L²,
X² is an oxygen atom, a sulfur atom, or NR^{A},
R^{A} is a substituted or unsubstituted alkyl group having 1 to 6 carbon atoms or a substituted or unsubstituted aryl group having 6 to 12 ring carbon atoms,
R¹⁴¹ to R¹⁴⁸ are each independently a hydrogen atom, a substituted or unsubstituted alkyl group having 1 to 6 carbon atoms, a substituted or unsubstituted aryl group having 6 to 12 ring carbon atoms, or a substituted or unsubstituted heterocyclic group having 5 to 13 ring atoms, provided that one selected from R¹⁴¹ to R¹⁴⁸ and R^{A} is a single bond bonded to *29,
at least one adjacent two selected from R¹⁴¹ to R¹⁴⁸ that are not the single bond may be bonded to each other to form one or more unsubstituted benzene rings, or may not be bonded to each other, thus forming no ring; 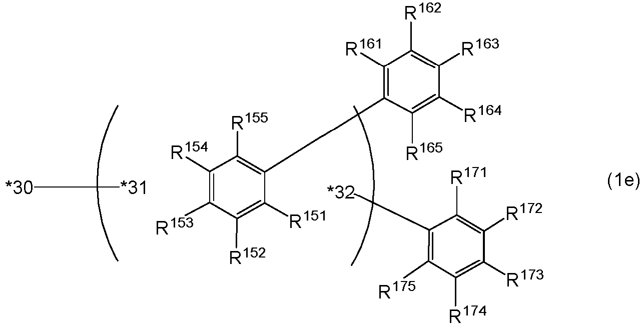 wherein
*30 is a bonding site to L¹ or L²,
R¹⁵¹ to R¹⁵⁵ are each independently a hydrogen atom, an unsubstituted alkyl group having 1 to 6 carbon atoms, or an unsubstituted phenyl group,
provided that one selected from R¹⁵¹ to R¹⁵⁵ is a single bond bonded to *31, and another one selected from R¹⁵¹ to R¹⁵⁵ is a single bond bonded to *32,
adjacent two selected from R¹⁵¹ to R¹⁵⁵ that are neither a single bond bonded to *31 nor a single bond bonded to *32 are not bonded to each other, thus forming no ring,
R¹⁶¹ to R¹⁶⁵ and R¹⁷¹ to R¹⁷⁵ are each independently a hydrogen atom or an unsubstituted alkyl group having 1 to 6 carbon atoms,
provided that at least one adjacent two selected from R¹⁶¹ to R¹⁶⁵ may be bonded to each other to form one or more unsubstituted benzene rings, or may not be bonded to each other, thus forming no ring,
at least one adjacent two selected from R¹⁷¹ to R¹⁷⁵ may be bonded to each other to form one or more unsubstituted benzene rings, or may not be bonded to each other, thus forming no ring.

9. The compound according to claim 8, wherein Ar¹ and Ar² are a group represented by any one of the formulas (1a), (1b) and (1d).

10. The compound according to any one of claims 1 to 9, wherein R³² or R³⁷ is a single bond bonded to *4.

11. The compound according to any one of claims 1 to 10, wherein R¹ to R⁴ is a hydrogen atom.

12. The compound according to any one of claims 1 to 11, comprising at least one deuterium atom.

13. A material for organic electroluminescent devices, the material comprising the compound according to any one of claims 1 to 12.

14. A hole transporting layer material comprising the compound according to any one of claims 1 to 12.

15. An organic electroluminescent device comprising a cathode, an anode, and an organic layer between the cathode and the anode, the organic layer including a light emitting layer, at least one layer of the organic layer containing the compound according to any one of claims 1 to 12.

16. The organic electroluminescent device according to claim 15, wherein the organic layer comprises a hole transporting zone between the anode and the light emitting layer, the hole transporting zone containing the compound.

17. The organic electroluminescent device according to claim 16, wherein the hole transporting zone comprises a first hole transporting layer on an anode side and a second hole transporting layer on a cathode side, one of the first hole transporting layer and the second hole transporting layer, or both of the first hole transporting layer and the second hole transporting layer containing the compound.

18. The organic electroluminescent device according to claim 17, wherein the light emitting layer and the second hole transporting layer are in direct contact with each other.

19. The organic electroluminescent device according to claim 17 or 18, wherein a total thickness of the first hole transporting layer and the second hole transporting layer is 30 nm or more and 150 nm or less.

20. The organic electroluminescent device according to any one of claims 15 to 19, wherein the light emitting layer is a single layer.

21. The organic electroluminescent device according to any one of claims 15 to 20, wherein the light emitting layer comprises a light emitting compound that shows fluorescence emission with a main peak wavelength of 500 nm or less.

22. The organic electroluminescent device according to any one of claims 15 to 21, wherein the light emitting layer comprises a phosphorescent dopant material.

23. An electronic instrument comprising the organic electroluminescent device according to any one of claims 15 to 22.
